(19) Europäisches Patentamt
European Patent Office
Office européen des brevets

(11) EP 3 710 576 B1

(12) EUROPEAN PATENT SPECIFICATION

(45) Date of publication and mention of the grant of the patent:
**02.10.2024 Bulletin 2024/40**

(21) Application number: **18827296.7**

(22) Date of filing: **19.11.2018**

(51) International Patent Classification (IPC):
***C12N 5/0783*** *(2010.01)* ***A61K 35/17*** *(2015.01)*
***A61K 39/00*** *(2006.01)* ***A61P 35/00*** *(2006.01)*

(52) Cooperative Patent Classification (CPC):
**C12N 5/0636; A61K 39/4611; A61K 39/4644;**
A61K 2039/852; A61K 2039/86; A61K 2039/876;
A61K 2039/892; A61K 2239/54; A61K 2239/55;
A61K 2239/57; A61K 2239/59; C12N 2501/2302;
C12N 2501/2315; C12N 2501/2321;
C12N 2501/515; (Cont.)

(86) International application number:
**PCT/US2018/061865**

(87) International publication number:
**WO 2019/100023 (23.05.2019 Gazette 2019/21)**

# (54) TIL EXPANSION FROM FINE NEEDLE ASPIRATES AND SMALL BIOPSIES

TIL-EXPANSION VON FEINNADELASPIRATEN UND KLEINEN BIOPSIEN

EXPANSION DE TIL À PARTIR DE PRODUITS D'ASPIRATION D'AIGUILLE FINE ET DE PETITES BIOPSIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.11.2017 US 201762588044 P**
**24.01.2018 US 201862621515 P**
**05.11.2018 US 201862756038 P**

(43) Date of publication of application:
**23.09.2020 Bulletin 2020/39**

(73) Proprietor: **Iovance Biotherapeutics, Inc.**
**San Carlos, CA 94070 (US)**

(72) Inventors:
- **SIMPSON-ABELSON, Michelle**
**Lithia,FL 33547 (US)**
- **CHARTIER-COURTAUD, Cecile**
**Palo Alto,CA 94301 (US)**

(74) Representative: **Secerna LLP**
**The Old Fire Station**
**18 Clifford Street**
**York YO1 9RD (GB)**

(56) References cited:
**WO-A1-2018/182817 US-A1- 2014 030 806**
**US-A1- 2017 224 734**

- **DONIA M ET AL: "Characterization and Comparison of 'Standard' and 'Young' Tumour-Infiltrating Lymphocytes for Adoptive Cell Therapy at a Danish Translational Research Institution", SCANDINAVIAN JOURNAL OF IMMUNOLOGY, vol. 75, no. 2, February 2012 (2012-02-01), pages 157 - 167, XP055560924, ISSN: 0300-9475, DOI: 10.1111/j.1365-3083.2011.02640.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BESSER M J ET AL: "Minimally cultured or selected autologous tumor-infiltrating lymphocytes after a lympho-depleting chemotherapy regimen in metastatic melanoma patients", JOURNAL OF IMMUNOTHERAPY, vol. 32, no. 4, May 2009 (2009-05-01), pages 415 - 423, XP009511400, ISSN: 1537-4513, DOI: 10.1097/CJI.0B013E31819C8BDA**
- **TRAN K Q ET AL: "Minimally Cultured Tumor-infiltrating Lymphocytes Display Optimal Characteristics for Adoptive Cell Therapy", JOURNAL OF IMMUNOTHERAPY, vol. 31, no. 8, October 2008 (2008-10-01), pages 742 - 751, XP055031158, ISSN: 1524-9557, DOI: 10.1097/CJI.0b013e31818403d5**
- **JIN J ET AL: "Simplified method of the growth of human tumor infiltrating lymphocytes in gas-permeable flasks to numbers needed for patient treatment", JOURNAL OF IMMUNOTHERAPY, vol. 35, no. 3, April 2012 (2012-04-01), pages 283 - 292, XP008153078, ISSN: 1537-4513, DOI: 10.1097/CJI.0B013E31824E801F**
- **DUDLEY M E ET AL: "Generation of tumor-infiltrating lymphocyte cultures for use in adoptive transfer therapy for melanoma patients", JOURNAL OF IMMUNOTHERAPY, vol. 26, no. 4, July 2003 (2003-07-01), pages 332 - 342, XP009089890, ISSN: 1524-9557**


Remarks:

The complete document including Reference Table(s) and the Sequence Listing(s) can be downloaded from the EPO website

(52) Cooperative Patent Classification (CPC): (Cont.)
C12N 2501/599; C12N 2502/11

## Description

## BACKGROUND OF THE INVENTION

Treatment of bulky, refractory cancers using adoptive transfer of tumor infiltrating lymphocytes (TILs) represents a powerful approach to therapy for patients with poor prognoses. Gattinoni, et al., Nat. Rev. Immunol. 2006, 6, 383-393. A large number of TILs are required for successful immunotherapy, and a robust and reliable process is needed for commercialization. This has been a challenge to achieve because of technical, logistical, and regulatory issues with cell expansion. IL-2-based TIL expansion followed by a "rapid expansion process" (REP) has become a preferred method for TIL expansion because of its speed and efficiency. Dudley, et al., Science 2002, 298, 850-54; Dudley, et al., J. Clin. Oncol. 2005, 23, 2346-57; Dudley, et al., J. Clin. Oncol. 2008, 26, 5233-39; Riddell, et al., Science 1992, 257, 238-41; Dudley, et al., J. Immunother. 2003, 26, 332-42. REP can result in a 1,000-fold expansion of TILs over a 14-day period, although it requires a large excess *(e.g.,* 200-fold) of irradiated allogeneic peripheral blood mononuclear cells (PBMCs, also known as mononuclear cells (MNCs)), often from multiple donors, as feeder cells, as well as anti-CD3 antibody (OKT3) and high doses of IL-2. Dudley, et al., J. Immunother. 2003, 26, 332-42. TILs that have undergone an REP procedure have produced successful adoptive cell therapy following host immunosuppression in patients with melanoma. Current infusion acceptance parameters rely on readouts of the composition of TILs (*e.g.,* CD28, CD8, or CD4 positivity) and on fold expansion and viability of the REP product.

Current TIL manufacturing processes are limited by how the TILs are obtained from the patient. In some cases, where the tumor is sufficiently small or in a location in which tumor resection is not feasible, there remains a need for additional methods of obtaining TILs for expansion and treatment. The present invention meets this need by providing methods for expanding TILs from a fine needle aspirate (FNA) or a core needle biopsy, which contain low numbers of TILs, and using these expanded TILs in treatment methods.

## BRIEF SUMMARY OF THE INVENTION

The present invention provides improved and/or shortened methods for expanding TILs isolated from a fine needle aspirate or a core biopsy and producing therapeutic populations of TILs as defined in the appended claims.

The present invention provides a method for expanding tumor infiltrating lymphocytes (TILs) into a therapeutic population of TILs comprising:

(i) performing a first expansion by culturing a first population of TILs from at least one fine needle aspirate (FNA) or at least one small biopsy from a tumor in a patient in a cell culture medium comprising IL-2 to produce a second population of TILs, wherein the cell culture medium is supplemented with OKT-3 at any one of days 1-3, wherein the first expansion is performed for 3 days to 12 days in order to obtain the second population of TILs, wherein the second population of TILs comprises at least $5 \times 10^7$ TILs by 3 days to 12 days when the first population of TILs is from a small biopsy; and

(ii) performing a second expansion by supplementing the cell culture medium of the second population of TILs with additional IL-2, OKT-3, and antigen presenting cells (APCs), to produce a third population of TILs, wherein the third population of TILs is at least 25-fold greater in number than the second population of TILs, and wherein the second expansion is performed for 3 days to 12 days in order to obtain the third population of TILs, wherein the third population of TILs is a therapeutic population of TILs.

In some embodiments, the cell culture medium comprising IL-2 in step (i) further comprises OKT-3 and is not optionally supplemented with OKT-3 at any one of days 1-3, wherein step (i) is a priming first expansion step and step (ii) is a rapid second expansion.

The method according to claim 1 or 2, wherein after step (ii), the cells are removed from the cell culture and cryopreserved in a storage medium.

In some embodiments, steps (i) through (ii) are performed within a period of 17 days to 24 days.

In some embodiments, steps (i) through (ii) are performed within a period of 18 days to 22 days.

In some embodiments, steps (i) through (ii) are performed within a period of 20 days to 22 days.

In some embodiments, steps (i) through (ii) are performed within 22 days.

In some embodiments, the cells in the therapeutic population of TILs from step (ii) express CD4, CD8, and TCR $\alpha$ $\beta$ at levels similar to freshly harvested cells.

In some embodiments, the APCs are peripheral blood mononuclear cells (PBMCs).

In some embodiments, the PBMCs are added to the cell culture in step (i) on any of days 3 through 12 after initiation of the culture in step (i) and/or in step (ii) on any of days 11 through 14 after initiation of the culture in step (i).

In some embodiments, the third population of TILs comprises an increased subpopulation of effector T cells and/or central memory T cells relative to the second population of TILs, wherein the effector T cells and/or central memory T cells in the therapeutic population of TILs in step (ii) exhibit one or more characteristics selected from the group consisting of expression of CD27, expression of CD28, longer telomeres, increased CD57 expression, and decreased CD56 expression, relative to effector T cells and/or central memory T cells in the second population of cells.

In some embodiments, the effector T cells and/or central memory T cells exhibit increased CD57 expression and decreased CD56 expression.

In some embodiments, the APCs are artificial APCs (aAPCs) or autologous APCs.

In some embodiments, the therapeutic population of TILs are for infusion into a patient.

In some embodiments, the first expansion in step (i) is performed by further supplementing the cell culture medium of the second population of TILs with OKT-3, IL-15, OX40 agonistic antibody and/or 4-1BB agonistic antibody.

In some embodiments, the second expansion in step (ii) is performed by further supplementing the cell culture medium of the second population of TILs with IL-15, OX40 agonistic antibody and/or 4-1BB agonistic antibody.

In some embodiments, the FNA in step (i) comprises at least 400,000 TILs.

In some embodiments, the small biopsy is obtained from a tumor selected from the group consisting of pancreatic, melanoma, breast, and ovarian.

In some embodiments, the FNA is obtained from a tumor selected from the group consisting of lung, melanoma, head and neck, cervical, ovarian, pancreatic, glioblastoma, colorectal, and sarcoma.

In some embodiments, the lung tumor is a non-small cell lung carcinoma (NSCLC), and optionally wherein the patient has previously undergone surgical treatment.

In some embodiments, the TILs in step (i) are obtained from a FNA.

In some embodiments, the FNA is obtained using a 25-18 gauge needle.

In some embodiments, the TILs in step (i) are obtained from a small biopsy.

In some embodiments, the small biopsy is obtained using a 16-11 gauge needle.

In some embodiments, step (ii) is repeated one to four times in order to obtain sufficient TILs in the therapeutic population of TILs for a therapeutically effective dosage of the TILs.

In some embodiments, the number of TILs sufficient for a therapeutically effective dosage is from $2.3\times10^{10}$ to $13.7\times10^{10}$.

In some embodiments, the present invention provides a method for expanding tumor infiltrating lymphocytes (TILs) into a therapeutic population of TILs comprising:

(i) performing a first expansion by culturing a first population of TILs from a fine needle aspirate (FNA) or a small biopsy from a tumor in a patient in a cell culture medium comprising IL-2 to obtain a second population of TILs, wherein the cell culture medium is supplemented with OKT-3 at any one of days 1-3, wherein the first expansion is performed for 3 days to 12 days in order to obtain the second population of TILs, wherein the second population of TILs comprises at least $5 \times 10^7$ TILs by 3 days to 12 days when the first population of TILs is from a small biopsy; and

(ii) performing a second expansion by supplementing the cell culture medium of the second population of TILs with additional IL-2, OKT-3, and antigen presenting cells (APCs) to obtain a third population of TILs, wherein the third population of TILs is at least 25-fold greater in number than the second population of TILs, and wherein the second expansion is performed for 3 days to 12 days in order to obtain the third population of TILs, wherein the third population of TILs is a therapeutic population of TILs.

In some embodiments, the cell culture medium comprising IL-2 in step (i) further comprises OKT-3 and is not optionally supplemented with OKT-3 at any one of days 1-3, wherein step (i) is a priming first expansion step and step (ii) is a rapid second expansion step.

In some embodiments, the APCs are artificial APCs (aAPCs) or autologous APCs.

In some embodiments, the therapeutic population of TILs are for infusion into a patient.

In some embodiments, the first expansion in step (i) is performed by further supplementing the cell culture medium of the second population of TILs with OKT-3, IL-15, OX40 agonistic antibody and/or 4-1BB agonistic antibody.

In some embodiments, the second expansion in step (ii) is performed by further supplementing the cell culture medium of the second population of TILs with IL-15, OX40 agonistic antibody and/or 4-1BB agonistic antibody.

In some embodiments, which comprises an additional second expansion in step (ii) which is performed by further supplementing the cell culture medium of the third population of TILs with IL-15, OX40 agonistic antibody and/or 4-1BB agonistic antibody.

In some embodiments, the FNA in step (i) comprises at least 400,000 TILs.

In some embodiments, the small biopsy is obtained from a tumor selected from the group consisting of pancreatic, melanoma, breast, and ovarian.

In some embodiments, the FNA is obtained from a tumor selected from the group consisting of lung, melanoma, head and neck, cervical, ovarian, pancreatic, glioblastoma, colorectal, and sarcoma.
In some embodiments, the lung tumor is a non-small cell lung carcinoma (NSCLC), and optionally wherein the patient has previously undergone surgical treatment.
In some embodiments, the first population of TILs in step (i) are obtained from a FNA.
In some embodiments, the FNA is obtained using a 25-18 gauge needle.
In some embodiments, the TILs in step (i) are obtained from a small biopsy.
In some embodiments, the small biopsy is obtained using a 16-11 gauge needle.
In some embodiments, step (ii) is repeated one to four times in order to obtain sufficient TILs in the therapeutic population of TILs for a therapeutically effective dosage of the TILs.
In some embodiments, the number of TILs sufficient for a therapeutically effective dosage is from $2.3\times10^{10}$ to $13.7\times10^{10}$.
In some embodiments, the third population of TILs comprises an increased subpopulation of effector T cells and/or central memory T cells relative to the second population of TILs, wherein the effector T cells and/or central memory T cells exhibit one or more characteristics selected from the group consisting of expression of CD27, expression of CD28, longer telomeres, increased CD57 expression, and decreased CD56 expression, relative to effector T cells and/or central memory T cells in the second population of cells.
In some embodiments, the effector T cells and/or central memory T cells exhibit increased CD57 expression and decreased CD56 expression.

OTHER SUBJECT MATTER

Disclosed herein but not claimed, the cells are thawed prior to performing step (iv).
Disclosed herein but not claimed, step (iv) is repeated one to four times in order to obtain sufficient TILs in the therapeutic population of TILs for a therapeutically effective dosage of the TILs.
Disclosed herein but not claimed, the cells from the cell culture medium in step (ii) are removed and cryopreserved in a storage medium prior to step (iii).
Disclosed herein but not claimed, the cells are thawed prior to step (iii).
Disclosed herein but not claimed is a method for treating a subject with cancer comprising administering expanded tumor infiltrating lymphocytes (TILs) comprising:

(i) obtaining a first population of TILs from a fine needle aspirate (FNA) or a small biopsy obtained from a tumor in a patient;

(ii) performing a first expansion by culturing the first population of TILs in a cell culture medium comprising IL-2 to produce a second population of TILs, wherein the cell culture medium is supplemented with OKT-3 at any one of days 1-3, wherein the first expansion is performed for about 3 days to about 12 days in order to obtain the second population of TILs, wherein the second population of TILs comprises at least $5 \times 10^7$ TILs by about 3 days to about 12 days when the first population of TILs is from a small biopsy;

(iii) performing a second expansion by supplementing the cell culture medium of the second population of TILs with additional IL-2, OKT-3, and antigen presenting cells (APCs), to produce a third population of TILs, wherein the third population of TILs is at least 25-fold greater in number than the second population of TILs, and wherein the second expansion is performed for about 3 days to about 12 days in order to obtain the third population of TILs, wherein the third population of TILs is a therapeutic population of TILs; and

(iv) administering a therapeutically effective dosage of the third population of TILs to the patient.

In some methods disclosed herein, the cell culture medium comprising IL-2 in step (ii) further comprises OKT-3 and is not optionally supplemented with OKT-3 at any one of days 1-3, and wherein step (i) is a priming first expansion step and step (ii) is a rapid second expansion step.
In some methods disclosed herein, after step (ii) the cells are removed from the cell culture medium and cryopreserved in a storage medium prior to step (iv).
In some methods disclosed herein, the cells are thawed prior to step (iv).
In some methods disclosed herein, step (iii) is repeated one to four times in order to obtain sufficient TILs in the therapeutic population of TILs for a therapeutically effective dosage of the TILs.
In some methods disclosed herein, the APCs are artificial APCs (aAPCs) or autologous APCs.
In some methods disclosed herein, the first expansion in step (ii) is performed by further supplementing the cell culture medium of the second population of TILs with OKT-3, IL-15, OX40 agonistic antibody and/or 4-1BB agonistic antibody.

In some methods disclosed herein, the second expansion in step (iii) is performed by further supplementing the cell culture medium of the second population of TILs with IL-15, OX40 agonistic antibody and/or 4-1BB agonistic antibody.

In some methods disclosed herein, the FNA in step (i) comprises at least 400,000 TILs.

In some methods disclosed herein, the FNA is obtained from a tumor selected from the group consisting of lung, melanoma, head and neck, cervical, ovarian, pancreatic, glioblastoma, colorectal, and sarcoma.

In some methods disclosed herein,, the lung tumor is a non-small cell lung carcinoma (NSCLC), and optionally wherein the subject has previously undergone surgical treatment.

In some methods disclosed herein, the TILs in step (i) are obtained from a FNA.

In some methods disclosed herein, the FNA is obtained using a 25-18 gauge needle.

In some methods disclosed herein, the TILs in step (i) are obtained from a small biopsy.

In some methods disclosed herein, the small biopsy is obtained using a 16-11 gauge needle.

In some methods disclosed herein, step (iii) is repeated one to four times in order to obtain sufficient TILs in the therapeutic population of TILs for a therapeutically effective dosage of the TILs.

In some methods disclosed herein, the number of TILs sufficient for a therapeutically effective dosage is from about $2.3\times10^{10}$ to about $13.7\times10^{10}$.

In some methods disclosed herein, the third population of TILs comprises an increased subpopulation of effector T cells and/or central memory T cells relative to the second population of TILs, wherein the effector T cells and/or central memory T cells exhibit one or more characteristics selected from the group consisting of expression of CD27, expression of CD28, longer telomeres, increased CD57 expression, and decreased CD56 expression, relative to effector T cells and/or central memory T cells in the third population of cells.

In some methods disclosed herein, the effector T cells and/or central memory T cells exhibit increased CD57 expression and decreased CD56 expression.

In some methods disclosed herein, the cancer is selected from the group consisting of melanoma, cervical cancer, head and neck cancer, glioblastoma, ovarian cancer, sarcoma, pancreatic cancer, bladder cancer, breast cancer, triple negative breast cancer, and non-small cell lung carcinoma.

Also disclosed herein but not claimed is a method for treating a subject with cancer comprising administering expanded tumor infiltrating lymphocytes (TILs) comprising:

(i) performing a first expansion by culturing a first population of TILs from a fine needle aspirate (FNA) or a small biopsy from a tumor in a patient in a cell culture medium comprising IL-2 to obtain a second population of TILs, wherein the cell culture medium is supplemented with OKT-3 at any one of days 1-3, wherein the first expansion is performed for about 3 days to about 12 days in order to obtain the second population of TILs, wherein the second population of TILs comprises at least $5 \times 10^7$ TILs by about 3 days to about 12 days when the first population of TILs is from a small biopsy;

(ii) performing a second expansion by supplementing the cell culture medium of the second population of TILs with additional IL-2, OKT-3, and antigen presenting cells (APCs) to obtain a third population of TILs, wherein the third population of TILs is at least 25-fold greater in number than the second population of TILs, and wherein the second expansion is performed for about 3 days to about 12 days in order to obtain the third population of TILs, wherein the third population of TILs is a therapeutic population of TILs; and

(iii) administering a therapeutically effective dosage of the therapeutic population of TILs to the patient.

In some methods disclosed herein, the cell culture medium comprising IL-2 in step (ii) further comprises OKT-3 and is not optionally supplemented with OKT-3 at any one of days 1-3, and wherein step (i) is a priming first expansion step and step (ii) is a rapid second expansion step.

In some methods disclosed herein, the cells from the cell culture medium in step (ii) are removed and cryopreserved in a storage medium prior step (iii).

In some methods disclosed herein, the cells are thawed prior to step (iii).

In some methods disclosed herein, the APCs are artificial APCs (aAPCs) or autologous APCs.

In some methods disclosed herein, the APCs are peripheral blood mononuclear cells (PBMCs).

In some methods disclosed herein, the therapeutic population of TILs are infused into a patient.

In some methods disclosed herein, the first expansion in step (i) is performed by further supplementing the cell culture medium of the second population of TILs with OKT-3, IL-15, OX40 agonistic antibody and/or 4-1BB agonistic antibody.

In some methods disclosed herein, the second expansion in step (iii) is performed by further supplementing the cell culture medium of the second population of TILs with IL-15, OX40 agonistic antibody and/or 4-1BB agonistic antibody.

In some methods disclosed herein, the FNA in step (i) comprises at least 400,000 TILs.

In some methods disclosed herein, the small biopsy is obtained from a tumor selected from the group consisting of pancreatic, melanoma, breast, and ovarian.

In some methods disclosed herein, the FNA is obtained from a tumor selected from the group consisting of lung, melanoma, head and neck, cervical, ovarian, pancreatic, glioblastoma, colorectal, and sarcoma.

In some methods disclosed herein, the lung tumor is a non-small cell lung carcinoma (NSCLC), and optionally wherein the subject has previously undergone surgical treatment.

In some methods disclosed herein, the TILs in step (i) are obtained from a FNA.

In some methods disclosed herein, the FNA is obtained using a 25-18 gauge needle.

In some methods disclosed herein, the TILs in step (i) are obtained from a small biopsy.

In some methods disclosed herein, the small biopsy is obtained using a 16-11 gauge needle.

In some methods disclosed herein, step (ii) is repeated one to four times in order to obtain sufficient TILs in the therapeutic population of TILs for a therapeutically effective dosage of the TILs.

In some methods disclosed herein, the number of TILs sufficient for a therapeutically effective dosage is from about $2.3\times10^{10}$ to about $13.7\times10^{10}$.

In some methods disclosed herein, the third population of TILs comprises an increased subpopulation of effector T cells and/or central memory T cells relative to the second population of TILs, wherein the effector T cells and/or central memory T cells exhibit one or more characteristics selected from the group consisting of expression of CD27, expression of CD28, longer telomeres, increased CD57 expression, and decreased CD56 expression, relative to effector T cells and/or central memory T cells in the third population of cells.

In some methods disclosed herein, the effector T cells and/or central memory T cells exhibit increased CD57 expression and decreased CD56 expression.

Also disclosed herein but not claimed is a method for expanding tumor infiltrating lymphocytes (TILs) into a therapeutic population of TILs comprising:

(a) obtaining a first population of TILs from a fine needle aspirate (FNA) or a small biopsy obtained from a tumor in a patient;
(b) adding the first population into a closed system;
(c) performing a first expansion by culturing the first population of TILs in a cell culture medium comprising IL-2 to produce a second population of TILs, wherein the cell culture medium is supplemented with OKT-3 at any one of days 1-3, wherein the first expansion is performed for about 3 days to about 12 days in order to obtain the second population of TILs, wherein the second population of TILs comprises at least $5 \times 10^7$ TILs by about 3 days to about 12 days when the first population of TILs is from a small biopsy, wherein the first expansion is performed in a closed container providing a first gas-permeable surface area, wherein the first expansion is performed for about 3 days to about 12 days to obtain the second population of TILs, wherein the second population of TILs is at least 25-fold greater in number than the first population of TILs, and wherein the transition from step (b) to step (c) occurs without opening the system;
(d) performing a second expansion by supplementing the cell culture medium of the second population of TILs with additional IL-2, OKT-3, and antigen presenting cells (APCs), to produce a third population of TILs, wherein the second expansion is performed for about 3 days to about 12 days to obtain the third population of TILs, wherein the third population of TILs is a therapeutic population of TILs, wherein the second expansion is performed in a closed container providing a second gas-permeable surface area, and wherein the transition from step (c) to step (d) occurs without opening the system;
(e) harvesting the therapeutic population of TILs obtained from step (d), wherein the transition from step (d) to step (e) occurs without opening the system; and
(f) transferring the harvested TIL population from step (e) to an infusion bag, wherein the transfer from step (e) to (f) occurs without opening the system.

In some methods disclosed herein, the cell culture medium comprising IL-2 in step (ii) further comprises OKT-3 and is not optionally supplemented with OKT-3 at any one of days 1-3, and wherein step (i) is a priming first expansion step and step (ii) is a rapid second expansion step.

In some methods disclosed herein, the method further comprises the step of cryopreserving the infusion bag comprising the harvested TIL population in step (f) using a cryopreservation process.

In some methods disclosed herein, the cryopreservation process is performed using a 1:1 ratio of harvested TIL population to CS10 media.

In some methods disclosed herein, the APCs are peripheral blood mononuclear cells (PBMCs).

In some methods disclosed herein, the PBMCs are irradiated and allogeneic.

In some methods disclosed herein, the PBMCs are added to the cell culture on any of days 3 through 12 in step (c) and/or any of days 3 through 12 in step (d).

In some methods disclosed herein, the antigen-presenting cells are artificial antigen-presenting cells (aAPCs) or autologous APCs.

In some methods disclosed herein, the therapeutic population of TILs are infused into a patient.

In some methods disclosed herein, the first expansion in step (c) is performed by further supplementing the cell culture medium of the second population of TILs with OKT-3, IL-15, OX40 agonistic antibody and/or 4-1BB agonistic antibody.

In some methods disclosed herein, the second expansion in step (d) is performed by further supplementing the cell culture medium of the second population of TILs with IL-15, OX40 agonistic antibody and/or 4-1BB agonistic antibody.

In some methods disclosed herein, the FNA in step (a) comprises at least 400,000 TILs.

In some methods disclosed herein, the small biopsy is obtained from a tumor selected from the group consisting of pancreatic, melanoma, breast and ovarian.

In some methods disclosed herein, the FNA is obtained from a tumor selected from the group consisting of lung, melanoma, head and neck, cervical, ovarian, pancreatic, glioblastoma, colorectal, and sarcoma.

In some methods disclosed herein, the lung tumor is a non-small cell lung carcinoma (NSCLC), and optionally wherein the subject has previously undergone surgical treatment.

In some methods disclosed herein, the TILs in step (a) are obtained from a FNA.

In some methods disclosed herein, the FNA is obtained using a 25-18 gauge needle.

In some methods disclosed herein, the TILs in step (a) are obtained from a small biopsy.

In some methods disclosed herein, the small biopsy is obtained using a 16-11 gauge needle.

In some methods disclosed herein, the harvesting in step (e) is performing using a LOVO cell processing system.

In some methods disclosed herein, the cell culture medium is provided in a container selected from the group consisting of a G-container and a Xuri cellbag.

In some methods disclosed herein, the infusion bag in step (f) is a hypothermosol infusion bag.

In some methods disclosed herein, steps (a) through (f) are performed within a period of about 17 days to about 24 days.

In some methods disclosed herein, steps (a) through (f) are performed within a period of about 18 days to about 22 days.

In some methods disclosed herein, steps (a) through (f) are performed within a period of about 20 days to about 22 days.

In some methods disclosed herein, steps (a) through (f) are performed in 22 days or less.

In some methods disclosed herein, steps (a) through (f) and cryopreservation are performed in 22 days or less.

In some methods disclosed herein, the therapeutic population of TILs harvested in step (e) comprises sufficient TILs for a therapeutically effective dosage of the TILs.

In some methods disclosed herein, the number of TILs sufficient for a therapeutically effective dosage is from about $2.3\times10^{10}$ to about $13.7\times10^{10}$.

In some methods disclosed herein, steps (b) through (e) are performed in a single container, wherein performing steps (b) through (e) in a single container results in an increase in TIL yield per resected tumor as compared to performing steps (b) through (e) in more than one container.

In some methods disclosed herein, the antigen-presenting cells are added to the TILs during the second period in step (d) without opening the system.

In some methods disclosed herein, the third population of TILs comprises an increased subpopulation of effector T cells and/or central memory T cells relative to the second population of TILs, wherein the effector T cells and/or central memory T cells in the therapeutic population of TILs exhibit one or more characteristics selected from the group consisting of expressing CD27+, expressing CD28+, longer telomeres, increased CD57 expression, and decreased CD56 expression relative to effector T cells, and/or central memory T cells obtained from the second population of cells.

In some methods disclosed herein, the effector T cells and/or central memory T cells obtained from the third population of TILs exhibit increased CD57 expression and decreased CD56 expression relative to effector T cells and/or central memory T cells obtained from the second population of cells.

In some methods disclosed herein, the risk of microbial contamination is reduced as compared to an open system.

In some methods disclosed herein, the TILs from step (g) are infused into a patient.

Also disclosed herein but not claimed is a method for treating a subject with cancer, the method comprising administering expanded tumor infiltrating lymphocytes (TILs) comprising:

(a) obtaining a first population of TILs from a fine needle aspirate (FNA) or a small biopsy from a tumor resected from a subject;
(b) adding the first population into a closed system;
(c) performing a first expansion by culturing the first population of TILs in a cell culture medium comprising IL-2 to produce a second population of TILs, wherein the cell culture medium is supplemented with OKT-3 at any one of days 1-3, wherein the first expansion is performed for about 3 days to about 12 days in order to obtain the second population of TILs, wherein the second population of TILs comprises at least $5 \times 10^7$ TILs by about 3 days to about 12 days when the first population of TILs is from a small biopsy, wherein the first expansion is performed in a closed container providing a first gas-permeable surface area, wherein the first expansion is performed for about 3-14 days to obtain the second population of TILs, wherein the second population of TILs is at least 25-fold greater in number than the first population of TILs, and wherein the transition from step (b) to step (c) occurs without opening the system;
(d) performing a second expansion by supplementing the cell culture medium of the second population of TILs with additional IL-2, OKT-3, and antigen presenting cells (APCs), to produce a third population of TILs, wherein the second expansion is performed for about 3 days to about 12 days to obtain the third population of TILs, wherein the third population of TILs is a therapeutic population of TILs, wherein the second expansion is performed in a closed container providing a second gas-permeable surface area, and wherein the transition from step (c) to step (d) occurs without opening the system;
(e) harvesting the therapeutic population of TILs obtained from step (d), wherein the transition from step (d) to step (e) occurs without opening the system; and
(f) transferring the harvested TIL population from step (e) to an infusion bag, wherein the transfer from step (e) to (f) occurs without opening the system;
(g) optionally cryopreserving the infusion bag comprising the harvested TIL population from step (f) using a cryopreservation process; and
(h) administering a therapeutically effective dosage of the third population of TILs from the infusion bag in step (g) to the patient.

In some methods disclosed herein, the therapeutic population of TILs harvested in step (e) comprises sufficient TILs for administering a therapeutically effective dosage of the TILs in step (h).

In some methods disclosed herein, the cell culture medium comprising IL-2 in step (ii) further comprises OKT-3 and is not optionally supplemented with OKT-3 at any one of days 1-3, and wherein step (i) is a priming first expansion step and step (ii) is a rapid second expansion step.

In some methods disclosed herein, the APCs are artificial APCs (aAPCs) or autologous APCs.

In some methods disclosed herein, the therapeutic population of TILs are infused into a patient.

In some methods disclosed herein, the first expansion in step (c) is performed by further supplementing the cell culture medium of the second population of TILs with OKT-3, IL-15, OX40 agonistic antibody and/or 4-1BB agonistic antibody.

In some methods disclosed herein, the second expansion in step (d) is performed by further supplementing the cell culture medium of the second population of TILs with OKT-3, IL-15, OX40 agonistic antibody and/or 4-1BB agonistic antibody.

In some methods disclosed herein, the FNA in step (a) comprises at least 400,000 TILs.

In some methods disclosed herein, the small biopsy is obtained from a tumor selected from the group consisting of pancreatic, melanoma, breast and ovarian.

In some methods disclosed herein, the FNA is obtained from a tumor selected from the group consisting of lung, melanoma, head and neck, cervical, ovarian, pancreatic, glioblastoma, colorectal, and sarcoma.

In some methods disclosed herein, the lung tumor is a non-small cell lung carcinoma (NSCLC), and optionally wherein the subject has previously undergone surgical treatment.

In some methods disclosed herein, the TILs in step (i) are obtained from a FNA.

In some methods disclosed herein, the FNA is obtained using a 25-18 gauge needle.

In some methods disclosed herein, the TILs in step (i) are obtained from a small biopsy.

In some methods disclosed herein, the small biopsy is obtained using a 16-11 gauge needle.

In some methods disclosed herein, the number of TILs sufficient for administering a therapeutically effective dosage in step (h) is from about $2.3 \times 10^{10}$ to about $13.7 \times 10^{10}$.

In some methods disclosed herein, the antigen presenting cells (APCs) are PBMCs.

In some methods disclosed herein, the PBMCs are added to the cell culture on any of days 3 through 12 in step (c) and/or any of days 3 through 12 in step (d).

In some methods disclosed herein, prior to administering a therapeutically effective dosage of TIL cells in step (h), a non-myeloablative lymphodepletion regimen has been administered to the patient.

In some methods disclosed herein, the non-myeloablative lymphodepletion regimen comprises the steps of administration of cyclophosphamide at a dose of 60 mg/$m^2$/day for two days followed by administration of fludarabine at a dose of 25 mg/$m^2$/day for five days.

In some methods disclosed herein, the method further comprises the step of treating the patient with a high-dose IL-2 regimen starting on the day after administration of the TIL cells to the patient in step (h).

In some methods disclosed herein, the high-dose IL-2 regimen comprises 600,000 or 720,000 IU/kg administered as a 15-minute bolus intravenous infusion every eight hours until tolerance.

In some methods disclosed herein, the third population of TILs comprises an increased subpopulation of effector T cells and/or central memory T cells relative to the second population of TILs, wherein the effector T cells and/or central memory T cells in the therapeutic population of TILs exhibit one or more characteristics selected from the group consisting of expressing CD27+, expressing CD28+, longer telomeres, increased CD57 expression, and decreased CD56 expression relative to effector T cells, and/or central memory T cells obtained from the second population of cells.

In some methods disclosed herein, the effector T cells and/or central memory T cells in the therapeutic population of TILs exhibit increased CD57 expression and decreased CD56 expression relative to effector T cells and/or central memory T cells obtained from the second population of cells.

Also disclosed herein but not claimed is a method for expanding tumor infiltrating lymphocytes (TILs) into a therapeutic population of TILs comprising:

(a) obtaining a first population of TILs from a fine needle aspirate (FNA), a small biopsy, a core biopsy, or a small biopsy from a tumor in a patient;

(b) performing a priming first expansion by culturing the first population of TILs in a cell culture medium comprising IL-2, OKT-3, and antigen presenting cells (APCs) to produce a second population of TILs, wherein the priming first expansion is performed for a first period of about 1 to 14 days in a container comprising a first gas-permeable surface area to obtain the second population of TILs, wherein the second population of TILs is greater in number than the first population of TILs;

(c) performing a rapid second expansion by supplementing the cell culture medium of the second population of TILs with additional IL-2, OKT-3, and APCs, to produce a third population of TILs, wherein the rapid second expansion is performed for a second period of about 1 to about 14 days to obtain the third population of TILs, wherein the third population of TILs is a therapeutic population of TILs; and

(d) harvesting the therapeutic population of TILs from step (c).

In some methods disclosed herein, the first period is about 6 to 12 days.

In some methods disclosed herein, the second period is about 6 to 12 days.

In some methods disclosed herein, the first period is selected from the group consisting of 5 days, 6 days, 7 days, 8 days, 9 days, 10 days, 11 days, or 12 days.

In some methods disclosed herein, the second period is selected from the group consisting of 5 days, 6 days, 7 days, 8 days, 9 days, 10 days, 11 days, or 12 days.

In some methods disclosed herein, the APCs are peripheral blood mononuclear cells (PBMCs).

In some methods disclosed herein, the ratio of APCs used in Step (b) to the APCs used in Step (c) is about 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, 4:1, 4.1:1, 4.2:1, 4.3:1, 4.4:1, 4.5:1, 4.6:1, 4.7:1, 4.8:1, 4.9:1, or 5:1, or preferably about 1 to 2.

In some methods disclosed herein, the first population of TILs is obtained from a core biopsy.

In some methods disclosed herein, the core biopsy is obtained from a tumor selected from the group consisting of a melanoma tumor, an ovarian cancer tumor, a cervical cancer tumor, a non-small-cell lung cancer (NSCLC) tumor, a lung cancer tumor, a bladder cancer tumor, a breast cancer tumor, a tumor from a cancer caused by human papilloma virus, a head and neck cancer (including head and neck squamous cell carcinoma (HNSCC)) tumor, a glioblastoma tumor, a gastrointestinal cancer tumor, and a renal cancer tumor.

The present disclosure also provides compositions comprising TILs expanded using the methods as described herein.

In some compositions disclosed herein, the composition further comprises a cyropreservant.

In some compositions disclosed herein, the cryopreservant comprises dimethylsulfoxide.

In some compositions disclosed herein, the composition further comprises a cyropreservant and an isotonic agent.

In some compositions disclosed herein, the composition further comprises a cyropreservant comprising dimethylsulfoxide and an isotonic agent comprising sodium chloride, sodium gluconate, and sodium acetate.

In some compositions disclosed herein, the composition further comprises a cyropreservant comprising dimethylsulfoxide and dextran 40 and an isotonic agent comprising sodium chloride, sodium gluconate, and sodium acetate.

In some compositions disclosed herein, the composition comprising the TILs is provided in a sterile infusion bag.

## BRIEF DESCRIPTION OF THE DRAWINGS

**Figure 1:** Exemplary Process 2A chart providing an overview of Steps A through F.

**Figure 2:** Process Flow Chart of Process 2A.

**Figure 3:** Shows a diagram of an embodiment of a cryopreserved TIL exemplary manufacturing process (~22 days).

**Figure 4:** Shows a diagram of an embodiment of process 2A, a 22-day process for TIL manufacturing.

**Figure 5:** Comparison table of Steps A through F from exemplary embodiments of process 1C and process 2A.

**Figure 6:** Detailed comparison of an embodiment of process 1C and an embodiment of process 2A.

**Figure 7:** Exemplary Small Biopsy chart providing an overview of Steps A through F of the Small Biopsy Expansion process.

**Figure 8:** Image of viable cells expanded from core biopsies taken from a patient with pancreatic cancer (P7015). A) Core biopsies treated with IL-2, and B) core biopsies treated with a combination of IL-2, IL-15, and IL-21.

**Figure 9:** FACS analysis of TILs expanded from pancreatic core biopsies. A) T cells expanded from core biopsies treated with IL-2 are CD4+ and CD8+. B) CD8+ T cells expanded from core biopsies treated with IL-2 are CD107a+. C) T cells expanded from core biopsies treated with a combination of IL-2, IL-15, and IL-21 are CD4+ and CD8+. D) CD8+ T cells expanded from core biopsies treated with a combination of IL-2, IL-15, and IL-21 are CD107a+.

**Figure 10:** FACS analysis of TILs expanded from fine needle aspirates from a patient with cervical carcinoma (C2005). A) Fine needle aspirates treated with IL-2, and B) fine needle aspirates treated with a combination of IL-2, IL-15, and IL-21.

**Figure 11:** FACS analysis of TILs expanded from fine needle aspirates from a patient with lung carcinoma (L4032). A) Fine needle aspirates treated with IL-2, and B) fine needle aspirates treated with a combination of IL-2, IL-15, and IL-21. C) As a control, lung tumor fragments were cultured with IL-2 and expanded to produce CD4+ and CD8+ T cells.

**Figure 12:** TILs from the FNA sample from a patient with lung carcinoma (L4032) were further expanding using a Rapid Expansion Protocol. A) Total cell counts after rapid expansion of TILs expanded from aspirates that were initially treated with either IL-2 alone, a combination of IL-2, IL-15, and IL-21, or initially a combination of IL-2, IL-15, and IL-21 and then IL-2 alone. B) CD4+ and CD8+ T cells were present in the expanded TIL population treated with Il-2.

**Figure 13:** FACS analysis of TILs expanded from fine needle aspirates from a patient with lung carcinoma (L4033). A) T cells from fine needle aspirates treated with IL-2, and B) as a control, T cells from lung tumor fragments cultured with IL-2.

**Figure 14:** TILs expanded from fine needle aspirates from a patient with lung carcinoma (L4033) were further expanded using a Rapid Expansion Protocol. A) Total cell counts after rapid expansion of TILs obtained from fine needle aspirates treated with IL-2.

**Figure 15:** TILs from fine needle aspirates from ovarian tumors taken from patients with ovarian carcinoma (OV8011, OV8012, and OV8013). Total cell counts after culturing the fine needle aspirates with IL-2 or a combination of IL-2, IL-15, and IL-21.

**Figure 16:** FACS analysis of TILs expanded from fine needle aspirates from a patient with melanoma (M1101). A) T cells from fine needle aspirates treated with IL-2, and B) as a control, T cells from lung tumor fragments cultured with IL-2.

**Figure 17:** Phenotypic and functional status of TILs derived from pancreatic tumor sample obtained by core biopsies. A) Results from pancreatic tumors cultured in culture medium with IL-2 or culture medium with IL-2 and cell culture additives. B and C) Results of CD107a expression in CD4+ cells and CD8+ cells in different culture mediums (IL-2 supplemented culture medium versus additive supplemented culture medium) after stimulation with phorbol 12-myristate 13-acetate (PMA).

**Figure 18:** Summary table of results from core biopsies and fine needle aspirates. A) Results from pancreatic cancer and lung cancer samples. B) Results from colorectal cancer, ovarian cancer, cervical cancer, and melanoma samples.

**Figure 19:** Phenotypic and functional status of TILs derived from melanoma samples obtained by fine needle aspirate biopsies. A) Total cell counts of cells cultures after 0 days, 11 days, 18 days, and 21 days. B) Phenotypic analysis of cells derived from fragments and aspirates. B) Functional analysis (CD107a expression) of cells derived from fragments and aspirates after stimulation with PMA.

**Figure 20:** Summary of data showing TIL can be expanded from the Pancreatic Cancer Core Biopsies obtained from UPMC (University of Pittsburgh Medical Center).

**Figure 21:** Data showing general phenotyping of TIL derived from pancreatic tumors.

**Figure 22:** TIL isolated from the pre-REP and REP of Pancreatic Cores are Functional as determined by IFNγ and CD107a.

**Figure 23:** An increase in CD3+/NK cell population was observed in low expanded Pre-REP lung TIL.

**Figure 24:** CD4/8 ratio is higher than 1 in majority of Pre-REP lung TIL.

**Figure 25:** Comparable fold expansion during REP was observed in both high and low yield.

**Figure 26:** Data regarding Sarcoma (UPMC 58).

**Figure 27:** Data regarding Cervical cancer (UPMC45). Pre-REP TIL were expanded from 4 fragments in G-REX 10 for 21 days.

**Figure 28:** Data regarding ES 19001 (UPMC46). Pre-REP TIL were expanded from 4 fragments in G-REX 10 for 21 days.

**Figure 29:** Data regarding Hard Palate (UPMC 67) and Oral Cancer (UPMC 68). Pre-REP TIL were expanded from 4 fragments in G-REX 10 for 21 days.

**Figure 30:** Summary of data showing TIL can be expanded from the Pancreatic Cancer Core Biopsies provided by UPMC.

**Figure 31:** Pancreatic Core Biopsies: TIL can be expanded from Core Biopsies from Pancreatic Cancer. P7015 Experimental Summary and Results: One core was placed into one well of a 24 well plate for a total of 5 wells. Two cores were placed into two wells, however there was little to no growth in these wells. Three wells were treated with 1) IL-2 and two wells with 2) IL-2/IL-15/IL-21.

**Figure 32:** Data showing that the majority of cells expanded from pancreatic cores were CD4 and CD8+ T cells, and were functional as determined by CD107a expression (P7015). P7015 Experimental Results: The majority of cells expanded from the pancreatic core biopsies are T cells (approximately 80% were CD4+ and CD8+). Stimulation with PMA/I demonstrated the ability of the CD8+ to degranulate as indicated by expression of CD107a+, thereby suggesting that these cells are functional. The % of CD8+ expressing CD107a+ was higher in the IL-2/IL-15/IL-21 triple cocktail condition, compared to IL-2 alone.

**Figure 33:** Pancreatic Core Biopsies: TIL can be expanded from Core Biopsies from Pancreatic Cancer. P7028 and P7031 pre-REP Experimental Summary and Results: 11 core biopsies were place into a G-Rex 10 with the triple cocktail (IL-2/IL-15/IL-21). P7028 TIL were assessed at Day 12 and kept in culture until Day 19. Cell counts: Day 12= 1.61e6 viable cells; Day 21 3.49e7 viable cells. P7028 TIL were assessed at Day 12 and kept in culture until Day 27. Cell counts: Day 12= 1.52e5 viable cells; Day 27: 1.14e7 viable cells.

**Figure 34:** Pancreatic Core Biopsies: TIL can be expanded from Core Biopsies and fragments treated with IL-2/IL-15/IL-21 from Pancreatic Cancer Day of harvest for TIL derived from fragments ranged from Day 11-21. Day of harvest for TIL derived from cores ranged from Day 21-28.

**Figure 35:** Phenotypic Characterization of TIL derived from cores and tumors biopsies. Day of harvest for TIL derived from fragments ranged from Day 11-21. Day of harvest for TIL derived from cores ranged from Day 21-28.

**Figure 36:** Phenotypic Characterization of TIL derived from cores and tumors biopsies.

**Figure 37:** TIL from core biopsies can express CD107a and secrete IFNγ similarly to TIL isolated from fragments.

**Figure 38:** A) Exemplary process for core biopsy expansion procedure. 1-2 core biopsies (3 pancreases, 4 melanoma, 1 breast and 1 ovarian) were subjected to an exemplary process that included the addition of OKT3 + feeders at Day 3. Pancreatic cores were expanded +/- IL-15 and IL-21 (REP1 & REP2; *i.e.,* first expansion and second expansion). Ovarian cores were treated +/- OX40 (BMS) (n=1). All TIL were subjected to a second REP (second expansion). B) Exemplary process for core biopsy expansion procedure. 1-2 core biopsies are subjected to a process that includes the addition of OKT3 + feeders at Day 3

**Figure 39:** Summary table of pancreatic core biopsy expansion procedure.

**Figure 40:** Data regarding cell number for pancreatic core biopsy expansions.

**Figure 41:** Summary table of melanoma core biopsy expansion procedure.

**Figure 42:** Data regarding cell number for melanoma core biopsy expansions.

**Figure 43:** Phenotypic expression of the exhaustion and activation markers are similar between REP1 and REP2 (*i.e.,* first expansion and second expansion).

**Figure 44:** The addition of OKT3 + feeders to cores at Day 3 does not significantly alter the phenotype of TIL compared to Gen2 (*i.e.,* process 2A) derived TIL from fragments.

**Figure 45:** Summary table of breast and ovarian core biopsy expansion procedure.

**Figure 46:** Data regarding cell number for breast (left) and ovarian (right panel) core biopsy expansions.

**Figure 47:** Experiment showing that TILs can be expanded from Core Biopsies from Pancreatic Cancer. P7028 and P7031 pre-REP Experimental Summary and Results. Core biopsies were place into a G-Rex 10 with the triple cocktail (IL-2/IL-15/IL-21). P7028 TIL were assessed at Day 12 and kept in culture until Day 19. Cell counts: Day 12= $1.61\times10^6$ viable cells; Day 21 $3.49\times10^7$ viable cells. P7030 TIL were assessed at Day 12 and kept in culture until Day 27. Cell counts: Day 12= $1.52\times10^5$ viable cells; Day 27: $1.14\times10^7$ viable cells.

**Figure 48:** TILs can be expanded from Core Biopsies and fragments treated with IL-2/IL-15/IL-21 from Pancreatic Cancer. Graphs showing cell expansion numbers for fragments and cores. Day of harvest for TIL derived from fragments ranged from Day 11-21. Day of harvest for TIL derived from cores ranged from Day 21-28.

**Figure 49:** TIL derived from cores and fragments are phenotypically similar. Day of harvest for TIL derived from fragments ranged from Day 11-21. Day of harvest for TIL derived from cores ranged from Day 21-28.

**Figure 50:** The "exhaustion markers" are differentially expressed when comparing TIL from cores and fragments.

**Figure 51:** TIL from core biopsies can express CD107a and secrete IFNγ similarly to TIL isolated from fragments.

**Figure 52:** The TC (triple cocktail; IL-2/IL-15/IL-21) does not significantly alter the total cell count in pancreatic cores in REP2 (second expansion), regardless of the initial culture conditions (REP1; first expansion).

**Figure 53:** The TC (triple cocktail; IL-2/IL-15/IL-21) does not significantly alter the CD4/CD8 ratio or the Memory subsets in REP2 (second expansion) in pancreatic cores.

**Figure 54:** CD107a is similar in the CD4 and CD8 populations from REP2 (second expansion) in all treatment conditions in pancreatic cores.

**Figure 55:** IFNγ secretion is similar regardless of REP2 (second expansion) treatment condition in pancreatic cores.

**Figure 56:** The addition of OKT3 & feeders at Day 3 (REP1; first expansion) does not significantly alter phenotypic expression, in comparison to pre-REP conditions in pancreatic cores.

**Figure 57:** The addition of OKT3 & feeders at Day 3 (REP1; first expansion) does not significantly alter phenotypic expression of the "exhaustion markers" in pancreatic cores.

**Figure 58:** The addition of OKT3 & feeders at Day 3 (REP1; first expansion) does not significantly alter phenotypic expression of the activation markers in pancreatic cores.

**Figure 59:** CD107a is similar in the CD4 and CD8 populations in the pre-REP, REP1 (first expansion) and REP2 (second expansion) in pancreatic cores.

**Figure 60:** IFNγ secretion is reduced in REP2 (second expansion), compared to REP1 (first expansion) in pancreatic cores.

**Figure 61:** TCM (central memory T-cells) population is increased and the TEM (effector memory T-cells) decreased in REP2 (second expansion), but CD27 and CD28 are unaltered in melanoma. TEMRA: terminally differentiated effector memory cells re-expressing CD45RA. TSCM: Stem memory T cells.

**Figure 62:** IFNγ and CD107a are similar in melanoma core REP1 (first expansion) and REP2 (second expansion).

**Figure 63:** Data showing CD4 and CD8 phenotypic markers in melanoma fragments versus melanoma cores.

**Figure 64:** Data showing CD4 and CD8 phenotypic markers in melanoma fragments versus melanoma cores.

**Figure 65:** Data showing CD107a level in CD4 and CD8 in melanoma fragments versus melanoma cores.

**Figure 66:** Treatment with OX40 enhanced the percentage of CD8+ cells in the ovarian cores.

**Figure 67:** Similar CD107a expression and IFNγ secretion in ovarian cores treated with OX40.

**Figure 68:** Enhanced TCM and differential expression of exhaustion and activation markers in REP1 (first expansion) and REP2 (second expansion) breast cores.

**Figure 69:** CD107a and IFNγ is similar in REP1 (first expansion) and REP2 (second expansion) of breast cores.

**Figure 70:** Schematic of an exemplary tumor core biopsy TIL expansion procedure.

**Figure 71:** TIL derived from pancreatic cores treated with OKT3 + feeders secrete IFNγ with restimulation.

**Figure 72:** TIL derived from melanoma cores express CD107a and secrete IFNγ upon restimulation.

**Figure 73:** Expanding TIL from Breast and Ovarian Core Biopsies.

**Figure 74:** The final product phenotype of TIL derived from breast cores treated with OKT3+feeders during REP1 were similar to cores treated with IL-2 alone during REP1.

**Figure 75:** The final product phenotype of TIL derived from breast cores treated with OKT3+feeders during REP1 were similar to cores treated with IL-2 alone during REP1.

**Figure 76:** Enhanced IFNγ secretion expression in breast cores treated with OKT3+feeders during REP1 compared to cores treated with IL-2 alone during REP1.

**Figure 77:** Treatment with OX40 enhanced the percentage of CD8+ cells in the ovarian cores in REP2.

**Figure 78:** Treatment with OX40 during both REPs altered the expression of PD-1, KLRG1 and CD25 in REP2.

**Figure 79:** IFNγ secretion in ovarian cores treated with OX40 is slightly higher in the final product (REP2).

**Figure 80:** CD107a expression in ovarian cores treated with OX40.

**Figure 81:** Treatment with OX40 through 2 REPs altered the expression of markers associated with T cell "youth".

**Figure 82:** Treatment with OX40 during both REPs slightly altered the expression altered the expression of PD-1, KLRG1 and CD25 in REP2.

Figure 83: Differential expression of exhaustion and activation markers in REP1 and REP 2 breast cores.

Figure 84: CD107a and IFNγ is similar in REP1 and REP2 of breast cores.

Figure 85A-85B: A) Shows a comparison between the 2A process (approximately 22-day process) and an embodiment of the Small Biopsy Gen 3 process for TIL manufacturing (approximately 17-days to 24-days process). B) Exemplary Process Gen3 chart providing an overview of Steps A through F (approximately 17-days to 24-days process).

Figure 86: Provides an experimental flow chart for comparability between GEN 2 (process 2A) versus GEN 3.

Figure 84A-87C: A) L4054 - Phenotypic characterization on TIL product on Gen 2 and Gen 3 process. B) L4055- Phenotypic characterization on TIL product on Gen 2 and Gen 3 process. C) M1085T-Phenotypic characterization on TIL product on Gen 2 and Gen 3 process.

Figure 88A-88C: A) L4054 - Memory markers analysis on TIL product from the Gen 2 and Gen 3 processes. B) L4055 - Memory markers analysis on TIL product from the Gen 2 and Gen 3 processes. C) M1085T- Memory markers analysis on TIL product from the Gen 2 and Gen 3 processes.

Figure 89: L4054 Activation and exhaustion markers (A) Gated on CD4+, (B) Gated on CD8+.

Figure 90: L4055 Activation and exhaustion markers (A) Gated on CD4+, (B) Gated on CD8+.

Figure 91: IFNγ production (pg/mL): (A) L4054, (B) L4055, and (C) M1085T for the Gen 2 and Gen 3 processes: Each bar represented here is mean + SEM for IFNγ levels of stimulated, unstimulated, and media control. Optical density measured at 450 nm.

Figure 92: ELISA analysis of IL-2 concentration in cell culture supernatant: (A) L4054 and (B) L4055. Each bar represented here is mean + SEM for IL-2 levels on spent media. Optical density measured at 450 nm.

**Figure 93:** Quantification of glucose and lactate (g/L) in spent media: (A) Glucose and (B) Lactate: In the two tumor lines, and in both processes, a decrease in glucose was observed throughout the REP expansion. Conversely, as expected, an increase in lactate was observed. Both the decrease in glucose and the increase in lactate were comparable between the Gen 2 and Gen 3 processes.

**Figure 94: A)** Quantification of L-glutamine in spent media for L4054 and L4055. **B)** Quantification of Glutamax in spent media for L4054 and L4055. C) Quantification of ammonia in spent media for L4054 and L4055.

**Figure 95: Telomere length analysis:** The relative telomere length (RTL) value indicates that the average telomere fluorescence per chromosome/genome in Gen 2 and Gen 3 process of the telomere fluorescence per chromosome/genome in the control cells line (1301 Leukemia cell line) using DAKO kit.

**Figure 96:** Unique CDR3 sequence analysis for TIL final product on L4054 and L4055 under Gen 2 and Gen 3 process. Columns show the number of unique TCR B clonotypes identified from $1 \times 10^6$ cells collected on Harvest Day Gen 2 *(e.g.,* day 22) and Gen 3 process *(e.g.,* day 14-16). Gen 3 shows higher clonal diversity compared to Gen 2 based on the number of unique peptide CDRs within the sample.

**Figure 97:** Frequency of unique CDR3 sequences on L4054 IL harvested final cell product (Gen 2 *(e.g.,* day 22) and Gen 3 process *(e.g.,* day 14-16)).

**Figure 98:** Frequency of unique CDR3 sequences on L4055 TIL harvested final cell product (Gen 2 *(e.g.,* day 22) and Gen 3 process *(e.g.,* day 14-16)).

**Figure 99:** Diversity Index for TIL final product on L4054 and L4055 under Gen 2 and Gen 3 process. Shanon entropy diversity index is a more reliable and common metric for comparison. Gen 3 L4054 and L4055 showed a slightly higher diversity than Gen 2.

**Figure 100:** Raw data for cell counts Day 7-Gen 3 REP initiation presented in Table 37 (see Example 14 below).

**Figure 101:** Raw data for cell counts Day 11-Gen 2 REP initiation and Gen 3 Scale Up presented in Table 37 (see Example 14 below).

**Figure 102:** Raw data for cell counts Day 16-Gen 2 Scale Up and Gen 3 Harvest *(e.g.,* day 16) presented in Table 38 (see Example 14 below).

**Figure 103:** Raw data for cell counts Day 22-Gen 2 Harvest *(e.g.,* day 22) presented in Table 38 (see Example 14 below). For L4054 Gen 2, post LOVO count was extrapolated to 4 flasks, because was the total number of the study. 1 flask was contaminated, and the extrapolation was done for total = $6.67 \times 10^{10}$.

**Figure 104:** Raw data for flow cytometry results depicted in Figs. 87A, 87A, and 87B.

**Figure 105:** Raw data for flow cytometry results depicted in Figs. 87C and 87C.

**Figure 106:** Raw data for flow cytometry results depicted in Figs. 89 and 90.

**Figure 107:** Raw data for IFNγ production assay results for L4054 samples depicted in Fig. 91.

**Figure 108:** Raw data for IFNγ production assay results for L4055 samples depicted in Fig. 91.

**Figure 109:** Raw data for IFNγ production assay results for M1085T samples depicted in Fig. 91.

**Figure 110:** Raw data for IL-2 ELISA assay results depicted in Fig. 92.

**Figure 111:** Raw data for the metabolic substrate and metabolic analysis results presented in Figs. 93 and 94.

**Figure 112:** Raw data for the relative telomere length anaylsis results presented in Fig. 95.

**Figure 113:** Raw data for the unique CD3 sequence and clonal diversity analyses results presented in Figs. 96 and 99.

**Figure 114:** Shows a comparison between various Gen 2 (2A process) and the Gen 3.1 process embodiment.

**Figure 115:** Table describing various features of embodiments of the Gen 2, Gen 2.1 and Gen 3.0 process.

**Figure 116:** Overview of the media conditions for an embodiment of the Gen 3 process, referred to as Gen 3.1

**Figure 117:** Table describing various features of embodiments of the Gen 2, Gen 2.1 and Gen 3.0 process.

**Figure 118:** Table comparing various features of embodiments of the Gen 2 and Gen 3.0 processes.

**Figure 119:** Table providing media uses in the various embodiments of the described expansion processes.

**Figure 120:** Phenotype comparison: Gen 3.0 and Gen 3.1 embodiments of the process showed comparable CD28, CD27 and CD57 expression.

**Figure 121:** Higher production of IFNγ on Gen 3 final product. IFNγ analysis (by ELISA) was assessed in the culture frozen supernatant to compared both processes. For each tumor overnight stimulation with coated anti -CD3 plate, using fresh TIL product on each Gen 2 *(e.g.,* day 22) and Gen 3 process *(e.g.,* day 16). Each bar represents here are IFNylevels of stimulated, unstimulated and media control.

**Figure 122:** Top: Unique CDR3 sequence analysis for TIL final product: Columns show the number of unique TCR B clonotypes identified from $1 \times 10^6$ cells collected on Gen *2 (e.g.,* day 22) and Gen 3 process *(e.g.,* day 14-16). Gen 3 shows higher clonal diversity compared to Gen 2 based on the number of unique peptide CDRs within the sample. Bottom: Diversity Index for TIL final product: Shanon entropy diversity index is a more reliable a common metric for comparison. Gen 3 showed a slightly higher diversity than Gen 2.

**Figure 123:** 199 sequences are shared between Gen 3 and Gen 2 final product, corresponding to 97.07% of top 80% of unique CDR3 sequences from Gen 2 shared with Gen 3 final product.

**Figure 124:** 1833 sequences are shared between Gen 3 and Gen 2 final product, corresponding to 99.45% of top 80% of unique CDR3 sequences from Gen 2 shared with Gen 3 final product.

**Figure 125:** Schematic of an exemplary embodiment of the Gen 3 process (a 16-day process).

**Figure 126:** Schematic of an exemplary embodiment of a method for expanding TILS from hematopoietic malignancies using Gen 3 expansion platform.

**Figure 127:** Provides data showing the addition of OKT3 + feeders to cores at Day 3 did not significantly alter the phenotype of TIL compared to Gen2, indicating the TILs were healthy TILs.

**Figure 128:** Provides data showing expression of exhaustion and activation markers by melanoma core-derived TIL followed similar trends as that by pancreatic core-derived TIL. There were no differences observed in the phenotypic expression between IL-2 and the triple cocktail, in REP1 and REP2.

**Figure 129:** Provides data regarding expanding TIL from lung core biopsies.

**Figure 130:** Provides data showing that similar phenotypic profiles are observed in Day 0 versus Day 3 (OKT3 + feeders) treated lung cores.

**Figure 131:** Provides data showing enhanced phenotypic expression of activation and resident T cell markers in cores treated with OKT3 and feeders at Day 3.

**Figure 132:** TIL from lung cores are functional as determined by IFNγ secretion and CD107a mobilization in response to non-specific stimulation.

**Figure 133:** The CD8+ TIL from Day 3 treated lung cores are oligoclonal compared to the Day 0 treated TIL.

## BRIEF DESCRIPTION OF THE SEQUENCE LISTING

SEQ ID NO:1 is the amino acid sequence of the heavy chain of muromonab.

SEQ ID NO:2 is the amino acid sequence of the light chain of muromonab.

SEQ ID NO:3 is the amino acid sequence of a recombinant human IL-2 protein.

SEQ ID NO:4 is the amino acid sequence of aldesleukin.

SEQ ID NO:5 is the amino acid sequence of a recombinant human IL-4 protein.

SEQ ID NO:6 is the amino acid sequence of a recombinant human IL-7 protein.

SEQ ID NO:7 is the amino acid sequence of a recombinant human IL-15 protein.

SEQ ID NO:8 is the amino acid sequence of a recombinant human IL-21 protein.

SEQ ID NO:9 is the amino acid sequence of human 4-1BB.

SEQ ID NO: 10 is the amino acid sequence of murine 4-1BB.

SEQ ID NO:11 is the heavy chain for the 4-1BB agonist monoclonal antibody utomilumab (PF-05082566).

SEQ ID NO:12 is the light chain for the 4-1BB agonist monoclonal antibody utomilumab (PF-05082566).

SEQ ID NO:13 is the heavy chain variable region (VH) for the 4-1BB agonist monoclonal antibody utomilumab (PF-05082566).

SEQ ID NO:14 is the light chain variable region (VL) for the 4-1BB agonist monoclonal antibody utomilumab (PF-05082566).

SEQ ID NO:15 is the heavy chain CDR1 for the 4-1BB agonist monoclonal antibody utomilumab (PF-05082566).

SEQ ID NO:16 is the heavy chain CDR2 for the 4-1BB agonist monoclonal antibody utomilumab (PF-05082566).

SEQ ID NO:17 is the heavy chain CDR3 for the 4-1BB agonist monoclonal antibody utomilumab (PF-05082566).

SEQ ID NO:18 is the light chain CDR1 for the 4-1BB agonist monoclonal antibody utomilumab (PF-05082566).

SEQ ID NO:19 is the light chain CDR2 for the 4-1BB agonist monoclonal antibody utomilumab (PF-05082566).

SEQ ID NO:20 is the light chain CDR3 for the 4-1BB agonist monoclonal antibody utomilumab (PF-05082566).

SEQ ID NO:21 is the heavy chain for the 4-1BB agonist monoclonal antibody urelumab (BMS-663513).

SEQ ID NO:22 is the light chain for the 4-1BB agonist monoclonal antibody urelumab (BMS-663513).

SEQ ID NO:23 is the heavy chain variable region (VH) for the 4-1BB agonist monoclonal antibody urelumab (BMS-663513).

SEQ ID NO:24 is the light chain variable region (VL) for the 4-1BB agonist monoclonal antibody urelumab (BMS-663513).

SEQ ID NO:25 is the heavy chain CDR1 for the 4-1BB agonist monoclonal antibody urelumab (BMS-663513).

SEQ ID NO:26 is the heavy chain CDR2 for the 4-1BB agonist monoclonal antibody urelumab (BMS-663513).

SEQ ID NO:27 is the heavy chain CDR3 for the 4-1BB agonist monoclonal antibody urelumab (BMS-663513).

SEQ ID NO:28 is the light chain CDR1 for the 4-1BB agonist monoclonal antibody urelumab (BMS-663513).

SEQ ID NO:29 is the light chain CDR2 for the 4-1BB agonist monoclonal antibody urelumab (BMS-663513).

SEQ ID NO:30 is the light chain CDR3 for the 4-1BB agonist monoclonal antibody urelumab (BMS-663513).

SEQ ID NO:31 is an Fc domain for a TNFRSF agonist fusion protein.

SEQ ID NO:32 is a linker for a TNFRSF agonist fusion protein.

SEQ ID NO:33 is a linker for a TNFRSF agonist fusion protein.

SEQ ID NO:34 is a linker for a TNFRSF agonist fusion protein.

SEQ ID NO:35 is a linker for a TNFRSF agonist fusion protein.

SEQ ID NO:36 is a linker for a TNFRSF agonist fusion protein.

SEQ ID NO:37 is a linker for a TNFRSF agonist fusion protein.

SEQ ID NO:38 is a linker for a TNFRSF agonist fusion protein.

SEQ ID NO:39 is a linker for a TNFRSF agonist fusion protein.

SEQ ID NO:40 is a linker for a TNFRSF agonist fusion protein.

SEQ ID NO:41 is a linker for a TNFRSF agonist fusion protein.

SEQ ID NO:42 is an Fc domain for a TNFRSF agonist fusion protein.

SEQ ID NO:43 is a linker for a TNFRSF agonist fusion protein.

SEQ ID NO:44 is a linker for a TNFRSF agonist fusion protein.

SEQ ID NO:45 is a linker for a TNFRSF agonist fusion protein.

SEQ ID NO:46 is a 4-1BB ligand (4-1BBL) amino acid sequence.

SEQ ID NO:47 is a soluble portion of 4-1BBL polypeptide.

SEQ ID NO:48 is a heavy chain variable region (VH) for the 4-1BB agonist antibody 4B4-1-1 version 1.

SEQ ID NO:49 is a light chain variable region (VL) for the 4-1BB agonist antibody 4B4-1-1 version 1.

SEQ ID NO:50 is a heavy chain variable region (VH) for the 4-1BB agonist antibody 4B4-1-1 version 2.

SEQ ID NO:51 is a light chain variable region (VL) for the 4-1BB agonist antibody 4B4-1-1 version 2.

SEQ ID NO:52 is a heavy chain variable region (VH) for the 4-1BB agonist antibody H39E3-2.

SEQ ID NO:53 is a light chain variable region (VL) for the 4-1BB agonist antibody H39E3-2.

SEQ ID NO:54 is the amino acid sequence of human OX40.

SEQ ID NO:55 is the amino acid sequence of murine OX40.

SEQ ID NO:56 is the heavy chain for the OX40 agonist monoclonal antibody tavolixizumab (MEDI-0562).

SEQ ID NO:57 is the light chain for the OX40 agonist monoclonal antibody tavolixizumab (MEDI-0562).

SEQ ID NO:58 is the heavy chain variable region (VH) for the OX40 agonist monoclonal antibody tavolixizumab (MEDI-0562).

SEQ ID NO:59 is the light chain variable region (VL) for the OX40 agonist monoclonal antibody tavolixizumab (MEDI-0562).

SEQ ID NO:60 is the heavy chain CDR1 for the OX40 agonist monoclonal antibody tavolixizumab (MEDI-0562).

SEQ ID NO:61 is the heavy chain CDR2 for the OX40 agonist monoclonal antibody tavolixizumab (MEDI-0562).

SEQ ID NO:62 is the heavy chain CDR3 for the OX40 agonist monoclonal antibody tavolixizumab (MEDI-0562).

SEQ ID NO:63 is the light chain CDR1 for the OX40 agonist monoclonal antibody tavolixizumab (MEDI-0562).

SEQ ID NO:64 is the light chain CDR2 for the OX40 agonist monoclonal antibody tavolixizumab (MEDI-0562).

SEQ ID NO:65 is the light chain CDR3 for the OX40 agonist monoclonal antibody tavolixizumab (MEDI-0562).

SEQ ID NO:66 is the heavy chain for the OX40 agonist monoclonal antibody 11D4.

SEQ ID NO:67 is the light chain for the OX40 agonist monoclonal antibody 11D4.

SEQ ID NO:68 is the heavy chain variable region (VH) for the OX40 agonist monoclonal antibody 11D4.

SEQ ID NO:69 is the light chain variable region (VL) for the OX40 agonist monoclonal antibody 11D4.

SEQ ID NO:70 is the heavy chain CDR1 for the OX40 agonist monoclonal antibody 11D4.

SEQ ID NO:71 is the heavy chain CDR2 for the OX40 agonist monoclonal antibody 11D4.

SEQ ID NO:72 is the heavy chain CDR3 for the OX40 agonist monoclonal antibody 11D4.

SEQ ID NO:73 is the light chain CDR1 for the OX40 agonist monoclonal antibody 11D4.

SEQ ID NO:74 is the light chain CDR2 for the OX40 agonist monoclonal antibody 11D4.

SEQ ID NO:75 is the light chain CDR3 for the OX40 agonist monoclonal antibody 11D4.

SEQ ID NO:76 is the heavy chain for the OX40 agonist monoclonal antibody 18D8.

SEQ ID NO:77 is the light chain for the OX40 agonist monoclonal antibody 18D8.

SEQ ID NO:78 is the heavy chain variable region (VH) for the OX40 agonist monoclonal antibody 18D8.

SEQ ID NO:79 is the light chain variable region (VL) for the OX40 agonist monoclonal antibody 18D8.

SEQ ID NO:80 is the heavy chain CDR1 for the OX40 agonist monoclonal antibody 18D8.

SEQ ID NO:81 is the heavy chain CDR2 for the OX40 agonist monoclonal antibody 18D8.

SEQ ID NO:82 is the heavy chain CDR3 for the OX40 agonist monoclonal antibody 18D8.

SEQ ID NO:83 is the light chain CDR1 for the OX40 agonist monoclonal antibody 18D8.

SEQ ID NO:84 is the light chain CDR2 for the OX40 agonist monoclonal antibody 18D8.

SEQ ID NO:85 is the light chain CDR3 for the OX40 agonist monoclonal antibody 18D8.

SEQ ID NO:86 is the heavy chain variable region (VH) for the OX40 agonist monoclonal antibody Hu119-122.

SEQ ID NO:87 is the light chain variable region (VL) for the OX40 agonist monoclonal antibody Hu119-122.

SEQ ID NO:88 is the heavy chain CDR1 for the OX40 agonist monoclonal antibody Hu119-122.

SEQ ID NO:89 is the heavy chain CDR2 for the OX40 agonist monoclonal antibody Hu119-122.

SEQ ID NO:90 is the heavy chain CDR3 for the OX40 agonist monoclonal antibody Hu119-122.

SEQ ID NO:91 is the light chain CDR1 for the OX40 agonist monoclonal antibody Hu119-122.

SEQ ID NO:92 is the light chain CDR2 for the OX40 agonist monoclonal antibody Hu119-122.

SEQ ID NO:93 is the light chain CDR3 for the OX40 agonist monoclonal antibody Hu119-122.

SEQ ID NO:94 is the heavy chain variable region (VH) for the OX40 agonist monoclonal antibody Hu106-222.

SEQ ID NO:95 is the light chain variable region (VL) for the OX40 agonist monoclonal antibody Hu106-222.

SEQ ID NO:96 is the heavy chain CDR1 for the OX40 agonist monoclonal antibody Hu106-222.

SEQ ID NO:97 is the heavy chain CDR2 for the OX40 agonist monoclonal antibody Hu 106-222.

SEQ ID NO:98 is the heavy chain CDR3 for the OX40 agonist monoclonal antibody Hu106-222.

SEQ ID NO:99 is the light chain CDR1 for the OX40 agonist monoclonal antibody Hu106-222.

SEQ ID NO:100 is the light chain CDR2 for the OX40 agonist monoclonal antibody Hu 106-222.

SEQ ID NO:101 is the light chain CDR3 for the OX40 agonist monoclonal antibody Hu106-222.

SEQ ID NO:102 is an OX40 ligand (OX40L) amino acid sequence.

SEQ ID NO:103 is a soluble portion of OX40L polypeptide.

SEQ ID NO:104 is an alternative soluble portion of OX40L polypeptide.

SEQ ID NO:105 is the heavy chain variable region (VH) for the OX40 agonist monoclonal antibody 008.

SEQ ID NO:106 is the light chain variable region (VL) for the OX40 agonist monoclonal antibody 008.

SEQ ID NO:107 is the heavy chain variable region (VH) for the OX40 agonist monoclonal antibody 011.

SEQ ID NO:108 is the light chain variable region (VL) for the OX40 agonist monoclonal antibody 011.

SEQ ID NO:109 is the heavy chain variable region (VH) for the OX40 agonist monoclonal antibody 021.

SEQ ID NO:110 is the light chain variable region (VL) for the OX40 agonist monoclonal antibody 021.

SEQ ID NO:111 is the heavy chain variable region (VH) for the OX40 agonist monoclonal antibody 023.

SEQ ID NO:112 is the light chain variable region (VL) for the OX40 agonist monoclonal antibody 023.

SEQ ID NO:113 is the heavy chain variable region (VH) for an OX40 agonist monoclonal antibody.

SEQ ID NO:114 is the light chain variable region (VL) for an OX40 agonist monoclonal antibody.

SEQ ID NO:115 is the heavy chain variable region (VH) for an OX40 agonist monoclonal antibody.

SEQ ID NO:116 is the light chain variable region (VL) for an OX40 agonist monoclonal antibody.

SEQ ID NO:117 is the heavy chain variable region (VH) for a humanized OX40 agonist monoclonal antibody.

SEQ ID NO:118 is the heavy chain variable region (VH) for a humanized OX40 agonist monoclonal antibody.

SEQ ID NO:119 is the light chain variable region (VL) for a humanized OX40 agonist monoclonal antibody.

SEQ ID NO:120 is the light chain variable region (VL) for a humanized OX40 agonist monoclonal antibody.

SEQ ID NO:121 is the heavy chain variable region (VH) for a humanized OX40 agonist monoclonal antibody.

SEQ ID NO:122 is the heavy chain variable region (VH) for a humanized OX40 agonist monoclonal antibody.

SEQ ID NO:123 is the light chain variable region (VL) for a humanized OX40 agonist monoclonal antibody.

SEQ ID NO:124 is the light chain variable region (VL) for a humanized OX40 agonist monoclonal antibody.

SEQ ID NO:125 is the heavy chain variable region (VH) for an OX40 agonist monoclonal antibody.

SEQ ID NO:126 is the light chain variable region (VL) for an OX40 agonist monoclonal antibody.

## DETAILED DESCRIPTION OF THE INVENTION

### I. Introduction

Adoptive cell therapy utilizing TILs cultured *ex vivo* by the Rapid Expansion Protocol (REP) has produced successful adoptive cell therapy following host immunosuppression in patients with melanoma. Current infusion acceptance parameters rely on readouts of the composition of TILs *(e.g.,* CD28, CD8, or CD4 positivity) and on the numerical folds of expansion and viability of the REP product.

Current REP protocols give little insight into the health of the TIL that will be infused into the patient. T cells undergo a profound metabolic shift during the course of their maturation from naive to effector T cells (see Chang, et al., Nat. Immunol. 2016, 17, 364, and in particular for the discussion and markers of anaerobic and aerobic metabolism). For example, naive T cells rely on mitochondrial respiration to produce ATP, while mature, healthy effector T cells such as TIL are highly glycolytic, relying on aerobic glycolysis to provide the bioenergetics substrates they require for proliferation, migration, activation, and anti-tumor efficacy.

Previous papers report that limiting glycolysis and promoting mitochondrial metabolism in TILs prior to transfer is desirable as cells that are relying heavily on glycolysis will suffer nutrient deprivation upon adoptive transfer which results in a majority of the transferred cells dying. Thus, the art teaches that promoting mitochondrial metabolism might promote *in vivo* longevity and in fact suggests using inhibitors of glycolysis before induction of the immune response. See Chang et al. (Chang, et al., Nat. Immunol. 2016, 17(364).

Also disclosed herein but not claimed are methods for evaluating and quantifying this increase in metabolic health. Thus, the disclosure provides methods of assaying the relative health of a TIL population using one or more general evaluations of metabolism, including, but not limited to, rates and amounts of glycolysis, oxidative phosphorylation, spare respiratory capacity (SRC), and glycolytic reserve.

Furthermore, also disclosed herein but not claimed are methods for evaluating and quantifying this increase in metabolic health. Thus, the disclosure provides methods of assaying the relative health of a TIL population using one or more general evaluations of metabolism, including, but not limited to, rates and amounts of glycolysis, oxidative phosphorylation, spare respiratory capacity (SRC), and glycolytic reserve.

In addition, optional additional evaluations include, but are not limited to, ATP production, mitochondrial mass and glucose uptake.

### II. Definitions

The term *"in vivo"* refers to an event that takes place in a subject's body.

The term *"in vitro"* refers to an event that takes places outside of a subject's body. *In vitro* assays encompass cell-based assays in which cells alive or dead are employed and may also encompass a cell-free assay in which no intact cells are employed.

The term "rapid expansion" means an increase in the number of antigen-specific TILs of at least about 3-fold (or 4-, 5-, 6-, 7-, 8-, or 9-fold) over a period of a week, more preferably at least about 10-fold (or 20-, 30-, 40-, 50-, 60-, 70-, 80-, or 90-fold) over a period of a week, or most preferably at least about 100-fold over a period of a week. A number of rapid expansion protocols are outlined below.

By "tumor infiltrating lymphocytes" or "TILs" herein is meant a population of cells originally obtained as white blood cells that have left the bloodstream of a subject and migrated into a tumor. TILs include, but are not limited to, $CD8^+$ cytotoxic T cells (lymphocytes), Th1 and Th17 $CD4^+$ T cells, natural killer cells, dendritic cells and M1 macrophages. TILs include both primary and secondary TILs. "Primary TILs" are those that are obtained from patient tissue samples as outlined herein (sometimes referred to as "freshly harvested"), and "secondary TILs" are any TIL cell populations that have been expanded or proliferated as discussed herein, including, but not limited to bulk TILs and expanded TILs ("REP TILs" or "post-REP TILs").

By "population of cells" (including TILs) herein is meant a number of cells that share common traits. In general, populations generally range from $1 \times 10^6$ to $1 \times 10^{10}$ in number, with different TIL populations comprising different numbers. For example, initial growth of primary TILs in the presence of IL-2 results in a population of bulk TILs of roughly $1 \times 10^8$ cells. REP expansion is generally done to provide populations of $1.5 \times 10^9$ to $1.5 \times 10^{10}$ cells for infusion.

By "small biopsy" herein is meant a biopsy that effects the removal of a volume of tissue from a tumor that is typically less, and in some embodiments substantially less, than the volume of the tumor. Small biopsy includes core biopsies, punch biopsies and the like. Any small biopsy is included, for example any biopsy with a volume less than about 1 $mm^3$, 2 $mm^3$, about 5 $mm^3$, about 10 $mm^3$, about 25 $mm^3$, about 50 $mm^3$, or about 100 $mm^3$ or with a cross-sectional area of less than about 1 $mm^2$, 2 $mm^2$, about 5 $mm^2$, about 10 $mm^2$, about 25 $mm^2$, about 50 $mm^2$, or about 100 $mm^2$. A small biopsy can also include multiple biopsies taken from the same or multiple locations in the body, including from different cancerous lesions in metastatic disease.

By "cryopreserved TILs" herein is meant that TILs, either primary, bulk, or expanded (REP TILs), are treated and stored in the range of about -150°C to -60°C. General methods for cryopreservation are also described elsewhere herein, including in the Examples. For clarity, "cryopreserved TILs" are distinguishable from frozen tissue samples which may be used as a source of primary TILs.

By "thawed cryopreserved TILs" herein is meant a population of TILs that was previously cryopreserved and then treated to return to room temperature or higher, including but not limited to cell culture temperatures or temperatures wherein TILs may be administered to a patient.

TILs can generally be defined either biochemically, using cell surface markers, or functionally, by their ability to infiltrate tumors and effect treatment. TILs can be generally categorized by expressing one or more of the following biomarkers: CD4, CD8, TCR $\alpha\beta$, CD27, CD28, CD56, CCR7, CD45Ra, CD95, PD-1, and CD25. Additionally and alternatively, TILs can be functionally defined by their ability to infiltrate solid tumors upon reintroduction into a patient.

The term "cryopreservation media" or "cryopreservation medium" refers to any medium that can be used for cryopreservation of cells. Such media can include media comprising 7% to 10% DMSO. Exemplary media include CryoStor CS10, Hyperthermasol, as well as combinations thereof. The term "CS10" refers to a cryopreservation medium which is obtained from Stemcell Technologies or from Biolife Solutions. The CS10 medium may be referred to by the trade name "CryoStor® CS10". The CS10 medium is a serum-free, animal component-free medium which comprises DMSO.

By "population of cells" (including TILs) herein is meant a number of cells that share common traits. In general, populations generally range from $1 \times 10^6$ to $1 \times 10^{10}$ in number, with different TIL populations comprising different numbers. For example, initial growth of primary TILs in the presence of IL-2 results in a population of bulk TILs of roughly $1 \times 10^8$ cells. REP expansion is generally done to provide populations of $1.5 \times 10^9$ to $1.5 \times 10^{10}$ cells for infusion.

The term "central memory T cell" refers to a subset of T cells that in the human are CD45R0+ and constitutively express CCR7 ($CCR7^{hi}$) and CD62L ($CD62^{hi}$). The surface phenotype of central memory T cells also includes TCR, CD3, CD127 (IL-7R), and IL-15R. Transcription factors for central memory T cells include BCL-6, BCL-6B, MBD2, and BMI1. Central memory T cells primarily secret IL-2 and CD40L as effector molecules after TCR triggering. Central memory T cells are predominant in the CD4 compartment in blood, and in the human are proportionally enriched in lymph nodes and tonsils.

The term "effector memory T cell" refers to a subset of human or mammalian T cells that, like central memory T cells, are CD45R0+, but have lost the constitutive expression of CCR7 ($CCR7^{lo}$) and are heterogeneous or low for CD62L expression ($CD62L^{lo}$). The surface phenotype of central memory T cells also includes TCR, CD3, CD127 (IL-7R), and IL-15R. Transcription factors for central memory T cells include BLIMP1. Effector memory T cells rapidly secret high levels of inflammatory cytokines following antigenic stimulation, including interferon-$\gamma$, IL-4, and IL-5. Effector memory T cells are predominant in the CD8 compartment in blood, and in the human are proportionally enriched in the lung, liver, and gut. CD8+ effector memory T cells carry large amounts of perforin. The term "closed system" refers to a system that is closed to the outside environment. Any closed system appropriate for cell culture methods can be employed with the methods of the present invention. Closed systems include, for example, but are not limited to closed G-containers. Once a tumor segment is added to the closed system, the system is no opened to the outside environment until the TILs are ready to be administered to the patient.

The terms "fragmenting," "fragment," and "fragmented," as used herein to describe processes for disrupting a tumor, includes mechanical fragmentation methods such as crushing, slicing, dividing, and morcellating tumor tissue as well as any other method for disrupting the physical structure of tumor tissue.

The terms "peripheral blood mononuclear cells" and "PBMCs" refers to a peripheral blood cell having a round nucleus, including lymphocytes (T cells, B cells, NK cells) and monocytes. When used as an antigen-presenting cell (PBMCs are a type of antigen-presenting cell), the peripheral blood mononuclear cells are irradiated allogeneic peripheral blood mononuclear cells.

The terms "peripheral blood lymphocytes" and "PBLs" refers to T-cells expanded from peripheral blood. In some embodiments, PBLs are separated from whole blood or apheresis product from a donor. In some embodiments, PBLs are separated from whole blood or apheresis product from a donor by positive or negative selection of a T cell phenotype, such as the T cell phenotype of CD3+ CD45+.

The term "anti-CD3 antibody" refers to an antibody or variant thereof, *e.g.,* a monoclonal antibody and including human, humanized, chimeric or murine antibodies which are directed against the CD3 receptor in the T cell antigen receptor of mature T cells. Anti-CD3 antibodies include OKT-3, also known as muromonab. Other anti-CD3 antibodies include, for example, otelixizumab, teplizumab, and visilizumab.

The term "OKT-3" (also referred to herein as "OKT3") refers to a monoclonal antibody or biosimilar or variant thereof, including human, humanized, chimeric, or murine antibodies, directed against the CD3 receptor in the T cell antigen receptor of mature T cells, and includes commercially-available forms such as OKT-3 (30 ng/mL, MACS GMP CD3 pure, Miltenyi Biotech, Inc., San Diego, CA, USA) and muromonab or variants, conservative amino acid substitutions, glycoforms, or biosimilars thereof. The amino acid sequences of the heavy and light chains of muromonab are given in Table 1 (SEQ ID NO: 1 and SEQ ID NO:2). A hybridoma capable of producing OKT-3 is deposited with the American Type Culture Collection and assigned the ATCC accession number CRL 8001. A hybridoma capable of producing OKT-3 is also deposited with European Collection of Authenticated Cell Cultures (ECACC) and assigned Catalogue No. 86022706.

TABLE 1. Amino acid sequences of muromonab.

| Identifier | Sequence (One-Letter Amino Acid Symbols) |
|---|---|
| SEQ ID NO:1<br>Muromonab heavy chain | QVQLQQSGAE LARPGASVKM SCKASGYTFT RYTMHWVKQR PGQGLEWIGY INPSRGYTNY 60<br>NQKFKDKATL TTDKSSSTAY MQLSSLTSED SAVYYCARYY DDHYCLDYWG QGTTLTVSSA 120<br>KTTAPSVYPL APVCGGTTGS SVTLGCLVKG YFPEPVTLTW NSGSLSSGVH TFPAVLQSDL 180<br>YTLSSSVTVT SSTWPSQSIT CNVAHPASST KVDKKIEPRP KSCDKTHTCP PCPAPELLGG 240<br>PSVFLFPPKP KDTLMISRTP EVTCVVVDVS HEDPEVKFNW YVDGVEVHNA KTKPREEQYN 300<br>STYRVVSVLT VLHQDWLNGK EYKCKVSNKA LPAPIEKTIS KAKGQPREPQ VYTLPPSRDE 360<br>LTKNQVSLTC LVKGFYPSDI AVEWESNGQP ENNYKTTPPV LDSDGSFFLY SKLTVDKSRW 420<br>QQGNVFSCSV MHEALHNHYT QKSLSLSPGK 450 |
| SEQ ID NO:2<br>Muromonab light chain | QIVLTQSPAI MSASPGEKVT MTCSASSSVS YMNWYQQKSG TSPKRWIYDT SKLASGVPAH 60<br>FRGSGSGTSY SLTISGMEAE DAATYYCQQW SSNPFTFGSG TKLEINRADT APTVSIFPPS 120<br>SEQLTSGGAS VVCFLNNFYP KDINVKWKID GSERQNGVLN SWTDQDSKDS TYSMSSTLTL 180<br>TKDEYERHNS YTCEATHKTS TSPIVKSFNR NEC 213 |

The term "IL-2" (also referred to herein as "IL2") refers to the T cell growth factor known as interleukin-2, and includes all forms of IL-2 including human and mammalian forms, conservative amino acid substitutions, glycoforms, biosimilars, and variants thereof. IL-2 is described, *e.g.,* in Nelson, J. Immunol. 2004, 172, 3983-88 and Malek, Annu. Rev. Immunol. 2008, 26, 453-79. The amino acid sequence of recombinant human IL-2 suitable for use in the invention is given in Table 2 (SEQ ID NO:3). For example, the term IL-2 encompasses human, recombinant forms of IL-2 such as aldesleukin (PROLEUKIN, available commercially from multiple suppliers in 22 million IU per single use vials), as well as the form of recombinant IL-2 commercially supplied by CellGenix, Inc., Portsmouth, NH, USA (CELLGRO GMP) or ProSpec-Tany TechnoGene Ltd., East Brunswick, NJ, USA (Cat. No. CYT-209-b) and other commercial equivalents from other vendors. Aldesleukin (desalanyl-1, serine-125 human IL-2) is a nonglycosylated human recombinant form of IL-2 with a molecular weight of approximately 15 kDa. The amino acid sequence of aldesleukin suitable for use in the invention is given in Table 2 (SEQ ID NO:4). The term IL-2 also encompasses pegylated forms of IL-2, as described herein, including the pegylated IL2 prodrug NKTR-214, available from Nektar Therapeutics, South San Francisco, CA, USA. NKTR-214 and pegylated IL-2 suitable for use in the invention is described in U.S. Patent Application Publication No. US 2014/0328791 A1 and International Patent Application Publication No. WO 2012/065086 A1. Alternative forms of conjugated IL-2 suitable for use in the invention are described in U.S. Patent Nos. 4,766,106, 5,206,344, 5,089,261 and 4902,502. Formulations of IL-2 suitable for use in the invention are described in U.S. Patent No. 6,706,289

TABLE 2. Amino acid sequences of interleukins.

| Identifier | Sequence (One-Letter Amino Acid Symbols) |
|---|---|
| SEQ ID NO:3 recombinant human IL-2 (rhIL-2) | MAPTSSSTKK TQLQLEHLLL DLQMILNGIN NYKNPKLTRM LTFKFYMPKK ATELKHLQCL 60<br>EEELKPLEEV LNLAQSKNFH LRPRDLISNI NVIVLELKGS ETTFMCEYAD ETATIVEFLN 120<br>RWITFCQSII STLT 134 |
| SEQ ID NO:4 Aldesleukin | PTSSSTKKTQ LQLEHLLLDL QMILNGINNY KNPKLTRMLT FKFYMPKKAT ELKHLQCLEE 60<br>ELKPLEEVLN LAQSKNFHLR PRDLISNINV IVLELKGSET TFMCEYADET ATIVEFLNRW 120<br>ITFSQSIIST LT 132 |
| SEQ ID NO:5 recombinant human IL-4 (rhIL-4) | MHKCDITLQE IIKTLNSLTE QKTLCTELTV TDIFAASKNT TEKETFCRAA TVLRQFYSHH 60<br>EKDTRCLGAT AQQFHRHKQL IRFLKRLDRN LWGLAGLNSC PVKEANQSTL ENFLERLKTI 120<br>MREKYSKCSS 130 |
| SEQ ID NO:6 recombinant human IL-7 (rhIL-7) | MDCDIEGKDG KQYESVLMVS IDQLLDSMKE IGSNCLNNEF NFFKRHICDA NKEGMFLFRA 60<br>ARKLRQFLKM NSTGDFDLHL LKVSEGTTIL LNCTGQVKGR KPAALGEAQP TKSLEENKSL 120<br>KEQKKLNDLC FLKRLLQEIK TCWNKILMGT KEH 153 |
| SEQ ID NO:7 recombinant human IL-15 (rhIL-15) | MNWVNVISDL KKIEDLIQSM HIDATLYTES DVHPSCKVTA MKCFLLELQV ISLESGDASI 60<br>HDTVENLIIL ANNSLSSNGN VTESGCKECE ELEEKNIKEF LQSFVHIVQM FINTS 115 |
| SEQ ID NO:8 recombinant human IL-21 (rhIL-21) | MQDRHMIRMR QLIDIVDQLK NYVNDLVPEF LPAPEDVETN CEWSAFSCFQ KAQLKSANTG 60<br>NNERIINVSI KKLKRKPPST NAGRRQKHRL TCPSCDSYEK KPPKEFLERF KSLLQKMIHQ 120<br>HLSSRTHGSE DS 132 |

The term "IL-4" (also referred to herein as "IL4") refers to the cytokine known as interleukin 4, which is produced by Th2 T cells and by eosinophils, basophils, and mast cells. IL-4 regulates the differentiation of naive helper T cells (Th0 cells) to Th2 T cells. Steinke and Borish, Respir. Res. 2001, 2, 66-70. Upon activation by IL-4, Th2 T cells subsequently produce additional IL-4 in a positive feedback loop. IL-4 also stimulates B cell proliferation and class II MHC expression, and induces class switching to IgE and IgGi expression from B cells. Recombinant human IL-4 suitable for use in the invention is commercially available from multiple suppliers, including ProSpec-Tany TechnoGene Ltd., East Brunswick, NJ, USA (Cat. No. CYT-211) and ThermoFisher Scientific, Inc., Waltham, MA, USA (human IL-15 recombinant protein, Cat. No. Gibco CTP0043). The amino acid sequence of recombinant human IL-4 suitable for use in the invention is given in Table 2 (SEQ ID NO:5).

The term "IL-7" (also referred to herein as "IL7") refers to a glycosylated tissue-derived cytokine known as interleukin 7, which may be obtained from stromal and epithelial cells, as well as from dendritic cells. Fry and Mackall, Blood 2002, 99, 3892-904. IL-7 can stimulate the development of T cells. IL-7 binds to the IL-7 receptor, a heterodimer consisting of IL-7 receptor alpha and common gamma chain receptor, which in a series of signals important for T cell development within the thymus and survival within the periphery. Recombinant human IL-4 suitable for use in the invention is commercially available from multiple suppliers, including ProSpec-Tany TechnoGene Ltd., East Brunswick, NJ, USA (Cat. No. CYT-254) and ThermoFisher Scientific, Inc., Waltham, MA, USA (human IL-15 recombinant protein, Cat. No. Gibco PHC0071). The amino acid sequence of recombinant human IL-7 suitable for use in the invention is given in Table 2 (SEQ ID NO:6).

The term "IL-15" (also referred to herein as "IL15") refers to the T cell growth factor known as interleukin-15, and includes all forms of IL-2 including human and mammalian forms, conservative amino acid substitutions, glycoforms, biosimilars, and variants thereof. IL-15 is described, *e.g.,* in Fehniger and Caligiuri, Blood 2001, 97, 14-32. IL-15 shares $\beta$ and $\gamma$ signaling receptor subunits with IL-2. Recombinant human IL-15 is a single, non-glycosylated polypeptide chain containing 114 amino acids (and an N-terminal methionine) with a molecular mass of 12.8 kDa. Recombinant human IL-15 is commercially available from multiple suppliers, including ProSpec-Tany TechnoGene Ltd., East Brunswick, NJ, USA (Cat. No. CYT-230-b) and ThermoFisher Scientific, Inc., Waltham, MA, USA (human IL-15 recombinant protein, Cat. No. 34-8159-82). The amino acid sequence of recombinant human IL-15 suitable for use in the invention is given in Table 2 (SEQ ID NO:7).

The term "IL-21" (also referred to herein as "IL21") refers to the pleiotropic cytokine protein known as interleukin-21, and includes all forms of IL-21 including human and mammalian forms, conservative amino acid substitutions, glycoforms, biosimilars, and variants thereof. IL-21 is described, *e.g.,* in Spolski and Leonard, Nat. Rev. Drug. Disc. 2014, 13, 379-95. IL-21 is primarily produced by natural killer T cells and activated human $CD4^+$ T cells. Recombinant human IL-21 is a single, non-glycosylated polypeptide chain containing 132 amino acids with a molecular mass of 15.4 kDa. Recombinant human IL-21 is commercially available from multiple suppliers, including ProSpec-Tany TechnoGene Ltd., East Brunswick, NJ, USA (Cat. No. CYT-408-b) and ThermoFisher Scientific, Inc., Waltham, MA, USA (human IL-21 recombinant protein, Cat. No. 14-8219-80). The amino acid sequence of recombinant human IL-21 suitable for use in the invention is given in Table 2 (SEQ ID NO:8).

When "an anti-tumor effective amount", "a tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the genetically modified cytotoxic lymphocytes described herein may be administered at a dosage of $10^4$ to $10^{11}$ cells/kg body weight (*e.g.,* $10^5$ to $10^6$, $10^5$ to $10^{10}$, $10^5$ to $10^{11}$, $10^6$ to $10^{10}$, $10^6$ to $10^{11}$ ,$10^7$ to $10^{11}$, $10^7$ to $10^{10}$, $10^8$ to $10^{11}$, $10^8$ to $10^{10}$, $10^9$ to $10^{11}$, or $10^9$ to $10^{10}$ cells/kg body weight), including all integer values within those ranges. Genetically modified cytotoxic lymphocytes compositions may also be administered multiple times at these dosages. The genetically modified cytotoxic lymphocytes can be administered by using infusion techniques that are commonly known in immunotherapy (see, *e.g.,* Rosenberg et al., New Eng. J. of Med. 319: 1676, 1988). The optimal dosage and treatment regime for a particular patient can readily be determined by one skilled in the art of medicine by monitoring the patient for signs of disease and adjusting the treatment accordingly.

The term "hematological malignancy," "hematologic malignancy" or terms of correlative meaning refer to mammalian cancers and tumors of the hematopoietic and lymphoid tissues, including but not limited to tissues of the blood, bone marrow, lymph nodes, and lymphatic system. Hematological malignancies are also referred to as "liquid tumors." Hematological malignancies include, but are not limited to, acute lymphoblastic leukemia (ALL), chronic lymphocytic lymphoma (CLL), small lymphocytic lymphoma (SLL), acute myelogenous leukemia (AML), chronic myelogenous leukemia (CML), acute monocytic leukemia (AMoL), Hodgkin's lymphoma, and non-Hodgkin's lymphomas. The term "B cell hematological malignancy" refers to hematological malignancies that affect B cells.

The term "solid tumor" refers to an abnormal mass of tissue that usually does not contain cysts or liquid areas. Solid tumors may be benign or malignant. The term "solid tumor cancer refers to malignant, neoplastic, or cancerous solid tumors. Solid tumor cancers include, but are not limited to, sarcomas, carcinomas, and lymphomas, such as cancers of the lung, breast, prostate, colon, rectum, and bladder. The tissue structure of solid tumors includes interdependent tissue compartments including the parenchyma (cancer cells) and the supporting stromal cells in which the cancer cells are dispersed and which may provide a supporting microenvironment.

The term "fine needle aspirate" or FNA refers to a type of biopsy procedure that can be employed for sampling or diagnostic procedures, including tumor sampling, in which a sample is taken but the tumor is not removed or resected. In fine needle aspiration, a hollow needle, for example 25-18 gauge, is inserted into the tumor or into an area containing the tumor and fluid and cells (including tissue) are obtained for further analysis or expansion, as described herein. With an FNA, the cells are removed without preserving the histological architecture of the tissue cells. An FNA can comprise TILs. In some instances, a fine needle aspiration biopsy is performed using an ultrasound-guided fine needle aspiration biopsy needle. FNA needles are commercially available from Becton Dickinson, Covidien, and the like.

The term "core biopsy" or "core needle biopsy" refers to a type of biopsy procedure that can be employed for sampling or diagnostic procedures, including tumor sampling, in which a sample is taken but the tumor is not removed or resected. In a core biopsy, a hollow needle, example 16-11 gauge, is inserted into the tumor or into an area containing the tumor and fluid and cells (including tissue) are obtained for further analysis or expansion, as described herein. With a core biopsy, the cells can be removed with some preservation of the histological architecture of the tissue cells, given the larger needle size as compared to a FNA. The core biopsy needle is generally of a gauge size that is able to preserve at least some portion of the histological architecture of the tumor. A core biopsy can comprise TILs. In some instances, a core needle biopsy is performed using a biopsy instrument, a vacuum-assisted core-needle biopsy instrument, a steretactically guided core-needle biopsy instrument, an ultrasound-guided core-needle biopsy instrument, an MRI-guided core-needle biopsy instrument commercially available from Bard Medical, Becton Dickinson, and the like.

The term "liquid tumor" refers to an abnormal mass of cells that is fluid in nature. Liquid tumor cancers include, but are not limited to, leukemias, myelomas, and lymphomas, as well as other hematological malignancies. TILs obtained from liquid tumors may also be referred to herein as marrow infiltrating lymphocytes (MILs). TILs obtained from liquid tumors, including liquid tumors circulating in peripheral blood, may also be referred to herein as PBLs. The terms MIL, TIL, and PBL are used interchangeably herein and differ only based on the tissue type from which the cells are derived.

The term "microenvironment," as used herein, may refer to the solid or hematological tumor microenvironment as a whole or to an individual subset of cells within the microenvironment. The tumor microenvironment, as used herein, refers to a complex mixture of "cells, soluble factors, signaling molecules, extracellular matrices, and mechanical cues that promote neoplastic transformation, support tumor growth and invasion, protect the tumor from host immunity, foster therapeutic resistance, and provide niches for dominant metastases to thrive," as described in Swartz, et al., Cancer Res., 2012, 72, 2473. Although tumors express antigens that should be recognized by T cells, tumor clearance by the immune system is rare because of immune suppression by the microenvironment.

Also disclosed herein but not claimed is a method of treating a cancer with a population of remnant TILs (rTILs), wherein a patient is pre-treated with non-myeloablative chemotherapy prior to an infusion of rTILs according to the invention. In some methods disclosed herein, the population of rTILs may be provided with a population of normal emigrant TILs (eTILs), wherein a patient is pre-treated with nonmyeloablative chemotherapy prior to an infusion of rTILs and eTILs according to the invention. In some methods disclosed herein, the non-myeloablative chemotherapy is cyclophosphamide 60 mg/kg/d for 2 days (days 27 and 26 prior to rTIL infusion) and fludarabine 25 mg/$m^2$/d for 5 days (days 27 to 23 prior to rTIL infusion). In some methods disclosed herein, after non-myeloablative chemotherapy and rTIL infusion (at day 0) according to the invention, the patient receives an intravenous infusion of IL-2 intravenously at 720,000 IU/kg every 8 hours to physiologic tolerance.

Experimental findings indicate that lymphodepletion prior to adoptive transfer of tumor-specific T lymphocytes plays a key role in enhancing treatment efficacy by eliminating regulatory T cells and competing elements of the immune system ("cytokine sinks"). Accordingly, a lymphodepletion step (sometimes also referred to as "immunosuppressive conditioning") on the patient may be utilized prior to the introduction of the rTILs of the invention.

The terms "co-administration," "co-administering," "administered in combination with," "administering in combination with," "simultaneous," and "concurrent," as used herein, encompass administration of two or more active pharmaceutical ingredients (in a preferred embodiment of the present invention, for example, at least one potassium channel agonist in combination with a plurality of TILs) to a subject so that both active pharmaceutical ingredients and/or their metabolites are present in the subject at the same time. Co-administration includes simultaneous administration in separate compositions, administration at different times in separate compositions, or administration in a composition in which two or more active pharmaceutical ingredients are present. Simultaneous administration in separate compositions and administration in a composition in which both agents are present are preferred.

The term "effective amount" or "therapeutically effective amount" refers to that amount of a compound or combination of compounds as described herein that is sufficient to effect the intended application including, but not limited to, disease treatment. A therapeutically effective amount may vary depending upon the intended application (*in vitro* or *in vivo*), or the subject and disease condition being treated *(e.g.,* the weight, age and gender of the subject), the severity of the disease condition, or the manner of administration. The term also applies to a dose that will induce a particular response in target cells *(e.g.,* the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

The terms "treatment", "treating", "treat", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment", as used herein, covers any treatment of a disease in a mammal, particularly in a human, and includes: (a) preventing the disease from occurring in a subject which may be predisposed to the disease but has not yet been diagnosed as having it; (b) inhibiting the disease, *i.e.,* arresting its development or progression; and (c) relieving the disease, *i.e.,* causing regression of the disease and/or relieving one or more disease symptoms. "Treatment" is also meant to encompass delivery of an agent in order to provide for a pharmacologic effect, even in the absence of a disease or condition. For example, "treatment" encompasses delivery of a composition that can elicit an immune response or confer immunity in the absence of a disease condition, *e.g.,* in the case of a vaccine.

The term "heterologous" when used with reference to portions of a nucleic acid or protein indicates that the nucleic acid or protein comprises two or more subsequences that are not found in the same relationship to each other in nature. For instance, the nucleic acid is typically recombinantly produced, having two or more sequences from unrelated genes arranged to make a new functional nucleic acid, *e.g.,* a promoter from one source and a coding region from another source, or coding regions from different sources. Similarly, a heterologous protein indicates that the protein comprises two or more subsequences that are not found in the same relationship to each other in nature *(e.g.,* a fusion protein).

The terms "sequence identity," "percent identity," and "sequence percent identity" (or synonyms thereof, *e.g.,* "99% identical") in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Suitable programs to determine percent sequence identity include for example the BLAST suite of programs available from the U.S. Government's National Center for Biotechnology Information BLAST web site. Comparisons between two sequences can be carried using either the BLASTN or BLASTP algorithm. BLASTN is used to compare nucleic acid sequences, while BLASTP is used to compare amino acid sequences. ALIGN, ALIGN-2 (Genentech, South San Francisco, California) or MegAlign, available from DNASTAR, are additional publicly available software programs that can be used to align sequences. One skilled in the art can determine appropriate parameters for maximal alignment by particular alignment software. In certain embodiments, the default parameters of the alignment software are used.

As used herein, the term "variant" encompasses but is not limited to antibodies or fusion proteins which comprise an amino acid sequence which differs from the amino acid sequence of a reference antibody by way of one or more substitutions, deletions and/or additions at certain positions within or adjacent to the amino acid sequence of the reference antibody. The variant may comprise one or more conservative substitutions in its amino acid sequence as compared to the amino acid sequence of a reference antibody. Conservative substitutions may involve, *e.g.,* the substitution of similarly charged or uncharged amino acids. The variant retains the ability to specifically bind to the antigen of the reference antibody. The term variant also includes pegylated antibodies or proteins.

The term *"in vivo"* refers to an event that takes place in a subject's body.

The term *"in vitro"* refers to an event that takes places outside of a subject's body. *In vitro* assays encompass cell-based assays in which cells alive or dead are employed and may also encompass a cell-free assay in which no intact cells are employed.

The term "rapid expansion" means an increase in the number of antigen-specific TILs of at least about 3-fold (or 4-, 5-, 6-, 7-, 8-, or 9-fold) over a period of a week, more preferably at least about 10-fold (or 20-, 30-, 40-, 50-, 60-, 70-, 80-, or 90-fold) over a period of a week, or most preferably at least about 100-fold over a period of a week. A number of rapid expansion protocols are outlined below.

By "tumor infiltrating lymphocytes" or "TILs" herein is meant a population of cells originally obtained as white blood cells that have left the bloodstream of a subject and migrated into a tumor. TILs include, but are not limited to, $CD8^+$ cytotoxic T cells (lymphocytes), Th1 and Th17 $CD4^+$ T cells, natural killer cells, dendritic cells and M1 macrophages. TILs include both primary and secondary TILs. "Primary TILs" are those that are obtained from patient tissue samples as outlined herein (sometimes referred to as "freshly harvested"), and "secondary TILs" are any TIL cell populations that have been expanded or proliferated as discussed herein, including, but not limited to bulk TILs, expanded TILs ("REP TILs") as well as "reREP TILs" as discussed herein. reREP TILs can include for example second expansion TILs or second additional expansion TILs (such as, for example, those described in Step D of Figure 7, including TILs referred to as reREP TILs).

TILs can generally be defined either biochemically, using cell surface markers, or functionally, by their ability to infiltrate tumors and effect treatment. TILs can be generally categorized by expressing one or more of the following biomarkers: CD4, CD8, TCR $\alpha\beta$, CD27, CD28, CD56, CCR7, CD45Ra, CD95, PD-1, and CD25. Additionally, and alternatively, TILs can be functionally defined by their ability to infiltrate solid tumors upon reintroduction into a patient. TILS may further be characterized by potency - for example, TILS may be considered potent if, for example, interferon (IFN) release is greater than about 50 pg/mL, greater than about 100 pg/mL, greater than about 150 pg/mL, or greater than about 200 pg/mL.

By "population of cells" (including TILs) herein is meant a number of cells that share common traits. In general, populations generally range from $1 \times 10^6$ to $1 \times 10^{10}$ in number, with different TIL populations comprising different numbers. For example, initial growth of primary TILs in the presence of IL-2 results in a population of bulk TILs of roughly $1 \times 10^8$ cells. REP expansion is generally done to provide populations of $1.5 \times 10^9$ to $1.5 \times 10^{10}$ cells for infusion.

The term "pharmaceutically acceptable salt" refers to salts derived from a variety of organic and inorganic counter ions known in the art. Pharmaceutically acceptable acid addition salts can be formed with inorganic acids and organic acids. Preferred inorganic acids from which salts can be derived include, for example, hydrochloric acid, hydrobromic acid, sulfuric acid, nitric acid and phosphoric acid. Preferred organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese and aluminum. Organic bases from which salts can be derived include, for example, primary, secondary, and tertiary amines, substituted amines including naturally occurring substituted amines, cyclic amines and basic ion exchange resins. Specific examples include isopropylamine, trimethylamine, diethylamine, triethylamine, tripropylamine, and ethanolamine. Also disclosed herein, the pharmaceutically acceptable base addition salt can be chosen from ammonium, potassium, sodium, calcium, and magnesium salts. The term "cocrystal" refers to a molecular complex derived from a number of cocrystal formers known in the art. Unlike a salt, a cocrystal typically does not involve hydrogen transfer between the cocrystal and the drug, and instead involves intermolecular interactions, such as hydrogen bonding, aromatic ring stacking, or dispersive forces, between the cocrystal former and the drug in the crystal structure.

The terms "pharmaceutically acceptable carrier" or "pharmaceutically acceptable excipient" are intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically acceptable carrier or pharmaceutically acceptable excipient is incompatible with the active pharmaceutical ingredient, its use in the therapeutic compositions of the invention is contemplated. Additional active pharmaceutical ingredients, such as other drugs, can also be incorporated into the described compositions and methods.

The terms "about" and "approximately" mean within a statistically meaningful range of a value. Such a range can be within an order of magnitude, preferably within 50%, more preferably within 20%, more preferably still within 10%, and even more preferably within 5% of a given value or range. The allowable variation encompassed by the terms "about" or "approximately" depends on the particular system under study, and can be readily appreciated by one of ordinary skill in the art. Moreover, as used herein, the terms "about" and "approximately" mean that dimensions, sizes, formulations, parameters, shapes and other quantities and characteristics are not and need not be exact, but may be approximate and/or larger or smaller, as desired, reflecting tolerances, conversion factors, rounding off, measurement error and the like, and other factors known to those of skill in the art. In general, a dimension, size, formulation, parameter, shape or other quantity or characteristic is "about" or "approximate" whether or not expressly stated to be such. It is noted that embodiments of very different sizes, shapes and dimensions may employ the described arrangements.

The transitional terms "comprising," "consisting essentially of," and "consisting of," when used in the appended claims, in original and amended form, define the claim scope with respect to what unrecited additional claim elements or steps, if any, are excluded from the scope of the claim(s). The term "comprising" is intended to be inclusive or open-ended and does not exclude any additional, unrecited element, method, step or material. The term "consisting of" excludes any element, step or material other than those specified in the claim and, in the latter instance, impurities ordinary associated with the specified material(s). The term "consisting essentially of" limits the scope of a claim to the specified elements, steps or material(s) and those that do not materially affect the basic and novel characteristic(s) of the claimed invention. All compositions, methods, and kits described herein that embody the present invention can, in alternate embodiments, be more specifically defined by any of the transitional terms "comprising," "consisting essentially of," and "consisting of."

### III. TIL Manufacturing Processes - Process 2A (Gen 2 Processes)

In some embodiments, the invention provides the method of any of GEN 2 processes (*e.g.* process 2A processes) modified to use a small biopsy, core biopsy or fine needle aspirate as the source of T cells for expansion in the first expansion of any such GEN 2 process, wherein (1) the duration of the first expansion is lengthened to achieve the desired TIL cell count in the population of TILs harvested after the second expansion of such GEN 2 process or (2) the duration of the second expansion of such GEN 2 process is lengthened to achieve the desired TIL cell count in the population of TILs harvested after the second expansion or (3) the duration of the first expansion is lengthened and the duration of the second expansion of such GEN 2 process is lengthened to achieve the desired TIL cell count in the population of TILs harvested after the second expansion.

Also disclosed herein is a population of TILs made by the method of any of GEN 2 processes (*e.g.* process 2A processes) modified to use a small biopsy, core biopsy or fine needle aspirate as the source of T cells for expansion in the first expansion of any such GEN 2 process, wherein (1) the duration of the first expansion is lengthened to achieve the desired TIL cell count in the population of TILs harvested after the second expansion of such GEN 2 process or (2) the duration of the second expansion of such GEN 2 process is lengthened to achieve the desired TIL cell count in the population of TILs harvested after the second expansion or (3) the duration of the first expansion is lengthened and the duration of the the second expansion of such GEN 2 process is lengthened to achieve the desired TIL cell count in the population of TILs harvested after the second expansion.

Exemplary TIL processes known as process 2A and the small biopsy process containing some of these features is depicted in Figure 7, and some of the advantages of this embodiment of the present invention over process 1C are described in Figures 5 and 6. An embodiment of process 2A is shown Figure 1. Moreover, an overview as well as a comparison to process 2A with regard to the exemplary core biopsy process is provide in Figure 7. Process 2A (Gen 2), methods of treatment using TILs from process 2A, and compositions of TILs prepared by process 2A can be used in conjunction with small biopsy/core biopsy with durations of expansion periods adjusted as needed to achieve necessary cell counts, as provided herein below and throughout the present application.

As discussed herein, the present disclosure includes a step relating to the restimulation of cryopreserved TILs to increase their metabolic activity and thus relative health prior to transplant into a patient, and methods of testing said metabolic health. As generally outlined herein, TILs are generally taken from a patient sample and manipulated to expand their number prior to transplant into a patient. Also disclosed herein but not claimed, the TILs may be optionally genetically manipulated as discussed below.

In some embodiments, the TILs may be cryopreserved. Once thawed, they may also be restimulated to increase their metabolism prior to infusion into a patient.

Also disclosed herein but not claimed, the first expansion (including processes referred to as the pre-REP as well as processes shown in Figure 7 as Step A) is 1-19 days and the second expansion (including processes referred to as the REP as well as processes shown in Figure 7 as Step B) is shorted to 11-14 days, as discussed in detail below as well as in the examples and figures. In some methods disclosed herein, the first expansion (for example, an expansion described as Step B in Figure 7) is split into two periods of 1-2 days and 3-19, or in some cases 1-2 days and 3-10 days and the second expansion ( for example, an expansion as described in Step D in Figure 7) is 11-14 days, as discussed in the Examples and shown in Figures 4, 5, and 7. In some methods disclosed herein, the combination of the first expansion and second expansion (for example, expansions described as Step B and Step D in Figure 7) is shortened to 22 days, as discussed in detail below and in the examples and figures.

The "Step" Designations A, B, C, *etc.,* below are in reference to Figure 7. The ordering of the Steps below and in Figure 7 is exemplary and any combination or order of steps, as well as additional steps, repetition of steps, and/or omission of steps is contemplated by the present application and the methods disclosed herein.

### A. STEP A: Obtain Patient tumor sample

In general, TILs are initially obtained from a patient tumor sample ("primary TILs") obtained by a core biopsy or similar procedure and then expanded into a larger population for further manipulation as described herein, optionally cryopreserved, and optionally evaluated for phenotype and metabolic parameters.

A patient tumor sample may be obtained using methods known in the art, generally via small biopsy, core biopsy, needle biopsy or other means for obtaining a sample that contains a mixture of tumor and TIL cells. In general, the tumor sample may be from any solid tumor, including primary tumors, invasive tumors or metastatic tumors. The tumor sample may also be a liquid tumor, such as a tumor obtained from a hematological malignancy. In some embodiments, the sample can be from multiple small tumor samples or biopsies. In some embodiments, the sample can comprise multiple tumor samples from a single tumor from the same patient. In some embodiments, the sample can comprise multiple tumor samples from one, two, three, or four tumors from the same patient. In some embodiments, the sample can comprise multiple tumor samples from multiple tumors from the same patient. The solid tumor may be of any cancer type, including, but not limited to, breast, pancreatic, prostate, colorectal, lung, brain, renal, stomach, and skin (including but not limited to squamous cell carcinoma, basal cell carcinoma, and melanoma). In some embodiments, the cancer is selected from cervical cancer, head and neck cancer (including, for example, head and neck squamous cell carcinoma (HNSCC)), glioblastoma (GBM), gastrointestinal cancer, ovarian cancer, sarcoma, pancreatic cancer, bladder cancer, breast cancer, triple negative breast cancer, and non-small cell lung carcinoma (NSCLC). In some embodiments, useful TILs are obtained from malignant melanoma tumors, as these have been reported to have particularly high levels of TILs.

In general, the cell suspension obtained from the tumor core or fragment is called a "primary cell population" or a "freshly obtained" or a "freshly isolated" cell population. In certain embodiments, the freshly obtained cell population of TILs is exposed to a cell culture medium comprising antigen presenting cells, IL-2 and OKT-3.

If the tumor is metastatic and the primary lesion has been efficiently treated/removed in the past, removal of one of the metastatic lesions may be needed. The least invasive approach may be to remove a skin lesion, or a lymph node on the neck or axillary area when available. A skin lesion may be removed or small biopsy thereof may be removed. A lymph node or small biopsy thereof may be removed. A lung or liver metastatic lesion, or an intra-abdominal or thoracic lymph node or small biopsy can thereof can be employed.

In some embodiments, the tumor is a melanoma. In some embodiments, the small biopsy for a melanoma comprises a mole or portion thereof.

In some embodiments, the small biopsy is a punch biopsy. In some embodiments, the punch biopsy is previously obtained with a circular blade pressed into the skin. In some embodiments, the punch biopsy is previously obtained with a circular blade pressed into the skin. around a suspicious mole. In some embodiments, the punch biopsy is previously obtained with a circular blade pressed into the skin, and a round piece of skin is removed. In some embodiments, the small biopsy is a punch biopsy and round portion of the tumor has been previously removed.

In some embodiments, the small biopsy is an excisional biopsy. In some embodiments, the small biopsy is an excisional biopsy and the entire mole or growth is removed. In some embodiments, the small biopsy is an excisional biopsy and the entire mole or growth has been previously removed along with a small border of normal-appearing skin.

In some embodiments, the small biopsy is an incisional biopsy. In some embodiments, the small biopsy is an incisional biopsy and only the most irregular part of a mole or growth is taken. In some embodiments, the small biopsy is an incisional biopsy and the incisional biopsy is used when other techniques can’t be completed, such as if a suspicious mole is very large.

In some embodiments, the small biopsy is a lung biopsy. In some embodiments, the small biopsy is previously obtained by bronchoscopy. Generally, bronchoscopy, the patient is put under anesthesia, and a small tool goes through the nose or mouth, down the throat, and into the bronchial passages, where small tools are used to remove some tissue. In some embodiments, where the tumor or growth cannot be reached via bronchoscopy, a transthoracic needle biopsy can be employed. Generally, for a transthoracic needle biopsy, the patient is also under anesthesia and a needle is inserted through the skin directly into the suspicious spot to remove a small sample of tissue. In some embodiments, a transthoracic needle biopsy may require interventional radiology (for example, the use of x-rays or CT scan to guide the needle). In some embodiments, the small biopsy is previously obtained by needle biopsy. In some embodiments, the small biopsy is previously obtained endoscopic ultrasound (for example, an endoscope with a light and is placed through the mouth into the esophagus). In some embodiments, the small biopsy is previously obtained surgically.

In some embodiments, the small biopsy is a head and neck biopsy. In some embodiments, the small biopsy is an incisional biopsy. The small biopsy may be an incisional biopsy, wherein a small piece of tissue is cut from an abnormal-looking area. If the abnormal region is easily accessed, the sample may be taken without hospitalization. If the tumor is deeper inside the mouth or throat, the biopsy may need to be done in an operating room, with general anesthesia. In some embodiments, the small biopsy is an excisional biopsy. The small biopsy may be an excisional biopsy, wherein the whole area is removed. In some embodiments, the small biopsy is a fine needle aspiration (FNA). In some embodiments, the small biopsy is a fine needle aspiration (FNA), wherein a very thin needle attached to a syringe may be used to extract (aspirate) cells from a tumor or lump. In some embodiments, the small biopsy is a punch biopsy. In some embodiments, the small biopsy is a punch biopsy, wherein punch forceps may be used to remove a piece of the suspicious area.

In some embodiments, the small biopsy is a cervical biopsy. The small biopsy may be obtained via colposcopy. Generally, colposcopy methods employ the use of a lighted magnifying instrument attached to magnifying binoculars (a colposcope) which is then used to biopsy a small section of the surface of the cervix. In some embodiments, the small biopsy is a conization/cone biopsy. In some embodiments, the small biopsy is a conization/cone biopsy, wherein previously an outpatient surgery may be needed to remove a larger piece of tissue from the cervix. The cone biopsy, in addition to helping to confirm a diagnosis, can serve as an initial treatment.

The term "solid tumor" refers to an abnormal mass of tissue that usually does not contain cysts or liquid areas. Solid tumors may be benign or malignant. The term "solid tumor cancer refers to malignant, neoplastic, or cancerous solid tumors. Solid tumor cancers include, but are not limited to, sarcomas, carcinomas, and lymphomas, such as cancers of the lung, breast, triple negative breast cancer, prostate, colon, rectum, and bladder. In some embodiments, the cancer is selected from cervical cancer, head and neck cancer, glioblastoma, ovarian cancer, sarcoma, pancreatic cancer, bladder cancer, breast cancer, triple negative breast cancer, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

In some embodiments, the sample from the tumor is obtained as a fine needle aspirate (FNA), a core biopsy, a small biopsy (including, for example, a punch biopsy). In some embodiments, sample is placed first into a G-Rex 10. In some embodiments, sample is placed first into a G-Rex 10 when there are 1 or 2 core biopsy and/or small biopsy samples. In some embodiments, sample is placed first into a G-Rex 100 when there are 3, 4, 5, 6, 8, 9, or 10 or more core biopsy and/or small biopsy samples. In some embodiments, sample is placed first into a G-Rex 500 when there are 3, 4, 5, 6, 8, 9, or 10 or more core biopsy and/or small biopsy samples.

The FNA can be obtained from a tumor selected from the group consisting of lung, melanoma, head and neck, cervical, ovarian, pancreatic, glioblastoma, colorectal, and sarcoma. In some embodiments, the FNA is obtained from a lung tumor, such as a lung tumor from a patient with non-small cell lung cancer (NSCLC). In some cases, the patient with NSCLC has previously undergone a surgical treatment.

TILs described herein can be obtained from an FNA sample. In some cases, the FNA sample is obtained or isolated from the patient using a fine gauge needle ranging from an 18 gauge needle to a 25 gauge needle. The fine gauge needle can be 18 gauge, 19 gauge, 20 gauge, 21 gauge, 22 gauge, 23 gauge, 24 gauge, or 25 gauge. In some embodiments, the FNA sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In some cases, the TILs described herein are obtained from a core biopsy sample. In some cases, the core biopsy sample is obtained or isolated from the patient using a surgical or medical needle ranging from an 11 gauge needle to a 16 gauge needle. The needle can be 11 gauge, 12 gauge, 13 gauge, 14 gauge, 15 gauge, or 16 gauge. In some embodiments, the core biopsy sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In general, the harvested cell suspension is called a "primary cell population" or a "freshly harvested" cell population.

In some embodiments, the TILs are not obtained from tumor digests. In some embodiments, the solid tumor cores are not fragmented.

In some embodiments, the TILs are obtained from tumor digests. In some embodiments, tumor digests were generated by incubation in enzyme media, for example but not limited to RPMI 1640, 2mM GlutaMAX, 10 mg/mL gentamicin, 30 U/mL DNase, and 1.0 mg/mL collagenase, followed by mechanical dissociation (GentleMACS, Miltenyi Biotec, Auburn, CA). After placing the tumor in enzyme media, the tumor can be mechanically dissociated for approximately 1 minute. The solution can then be incubated for 30 minutes at 37 °C in 5% $CO_2$ and it then mechanically disrupted again for approximately 1 minute. After being incubated again for 30 minutes at 37 °C in 5% $CO_2$, the tumor can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% $CO_2$. In some embodiments, at the end of the final incubation if the cell suspension contained a large number of red blood cells or dead cells, a density gradient separation using Ficoll can be performed to remove these cells.

In some embodiments, the harvested cell suspension prior to the first expansion step is called a "primary cell population" or a "freshly harvested" cell population.

In some embodiments, cells can be optionally frozen after sample harvest and stored frozen prior to entry into the expansion described in Step B, which is described in further detail below, as well as exemplified in Figure 7.

**B. STEP B: First Expansion**

In some embodiments, the present methods provide for obtaining young TILs, which are capable of increased replication cycles upon administration to a subject/patient and as such may provide additional therapeutic benefits over older TILs (*i.e.,* TILs which have further undergone more rounds of replication prior to administration to a subject/patient). Features of young TILs have been described in the literature, for example Donia, et al., Scandinavian Journal of Immunology, 75:157-167 (2012); Dudley et al., Clin Cancer Res, 16:6122-6131 (2010); Huang et al., J Immunother, 28(3):258-267 (2005); Besser et al., Clin Cancer Res, 19(17):OF1-OF9 (2013); Besser et al., J Immunother 32:415-423 (2009); Robbins, et al., J Immunol 2004; 173:7125-7130; Shen et al., J Immunother, 30:123-129 (2007); Zhou, et al., J Immunother, 28:53-62 (2005); and Tran, et al., J Immunother, 31:742-751 (2008).

The diverse antigen receptors of T and B lymphocytes are produced by somatic recombination of a limited, but large number of gene segments. These gene segments: V (variable), D (diversity), J (joining), and C (constant), determine the binding specificity and downstream applications of immunoglobulins and T-cell receptors (TCRs). The present invention provides a method for generating TILs which exhibit and increase the T-cell repertoire diversity. In some embodiments, the TILs obtained by the present method exhibit an increase in the T-cell repertoire diversity. In some embodiments, the TILs obtained by the present method exhibit an increase in the T-cell repertoire diversity as compared to freshly harvested TILs and/or TILs prepared using other methods than those provide herein including for example, methods other than those embodied in Figure 1. In some embodiments, the TILs obtained by the present method exhibit an increase in the T-cell repertoire diversity as compared to freshly harvested TILs and/or TILs prepared using methods referred to as process 1C, as exemplified in Figure 3 and/or Figure 4. In some embodiments, the TILs obtained in the first expansion exhibit an increase in the T-cell repertoire diversity. In some embodiments, the increase in diversity is an increase in the immunoglobulin diversity and/or the T-cell receptor diversity. In some embodiments, the diversity is in the immunoglobulin is in the immunoglobulin heavy chain. In some embodiments, the diversity is in the immunoglobulin is in the immunoglobulin light chain. In some embodiments, the diversity is in the T-cell receptor. In some embodiments, the diversity is in one of the T-cell receptors selected from the group consisting of alpha, beta, gamma, and delta receptors. In some embodiments, there is an increase in the expression of T-cell receptor (TCR) alpha and/or beta. In some embodiments, there is an increase in the expression of T-cell receptor (TCR) alpha. In some embodiments, there is an increase in the expression of T-cell receptor (TCR) beta. In some embodiments, there is an increase in the expression of TCRab (*i.e.,* TCR$\alpha/\beta$).

After obtaining the small biopsy, core biopsy or fine needle aspirate tumor fragment or fragments (which may be referred to as "cores" or "fragments"), for example such as described in Step A of Figure 7, the resulting cells are cultured in serum containing IL-2 under conditions that favor the growth of TILs over tumor and other cells. In some embodiments, the tumor digests are incubated in 2 mL wells in media comprising inactivated human AB serum with 6000 IU/mL of IL-2. This primary cell population is cultured for a period of days, generally from 3 to 14 days, resulting in a bulk TIL population, generally about $1 \times 10^8$ bulk TIL cells. In some embodiments, IL-2 is included during days 1-2 of the first expansion and OKT3 and feeders are added at day 3 of the first expansion. In some embodiments, the OKT3 and feeders are included for days 3-12. In some embodiments, the OKT3 and feeders are included for days 3-11. In some embodiments, the OKT3 and feeders are included for days 3-10. In some embodiments, the OKT3 and feeders are included for days 3-9. In a preferred embodiment, expansion of TILs may be performed using an initial bulk TIL expansion step (for example such as those described in Step B of Figure 7, which can include processes referred to as pre-REP) as described below and herein, followed by a second expansions (Step D, including processes referred to as rapid expansion protocol (REP) steps) as described below under Step D and herein, followed by optional cryopreservation, and followed by a second Step D (including processes referred to as restimulation REP steps) as described below and herein. The TILs obtained from this process may be optionally characterized for phenotypic characteristics and metabolic parameters as described herein.

In embodiments where TIL cultures are initiated in 24-well plates, for example, using Costar 24-well cell culture cluster, flat bottom (Corning Incorporated, Corning, NY, each well can be seeded with 1 × $10^6$ small biopsy, core biopsy, or fine needle aspirate tumor cells or one small biopsy, core biopsy, or fine needle aspirate tumor fragment in 2 mL of complete medium (CM) with IL-2 (6000 IU/mL; Chiron Corp., Emeryville, CA).

In some embodiments, the first expansion culture medium is referred to as "CM", an abbreviation for culture media. In some embodiments, CM for Step B consists of RPMI 1640 with GlutaMAX, supplemented with 10% human AB serum, 25 mM Hepes, and 10 mg/mL gentamicin. In embodiments where cultures are initiated in gas-permeable flasks with a 40 mL capacity and a 10 $cm^2$ gas-permeable silicon bottom (for example, G-Rex10; Wilson Wolf Manufacturing, New Brighton, MN) (Fig. 1), each flask was loaded with 10-40 × $10^6$ viable small biopsy, core biopsy, or fine needle aspirate tumor cells or 5-30 small biopsy, core biopsy or fine needle aspirate tumor fragments in 10-40 mL of CM with IL-2. Both the G-Rex10 and 24-well plates were incubated in a humidified incubator at 37°C in 5% $CO_2$ and 5 days after culture initiation, half the media was removed and replaced with fresh CM and IL-2 and after day 5, half the media was changed every 2-3 days.

After harvest of the small biopsy, core biopsy or fine needle aspirate tumor fragments, the resulting cells *(i.e.,* fragments) are cultured in serum containing IL-2 under conditions that favor the growth of TILs over tumor and other cells. In some embodiments, the tumor fragments are incubated in 2 mL wells in media comprising inactivated human AB serum (or, in some cases, as outlined herein, in the presence of aAPC cell population) with 6000 IU/mL of IL-2. This primary cell population is cultured for a period of days, generally from 10 to 14 days, resulting in a bulk TIL population, generally about 1 × $10^8$ bulk TIL cells. In some embodiments, the growth media during the first expansion comprises IL-2 or a variant thereof. In some embodiments, the IL is recombinant human IL-2 (rhIL-2). In some embodiments the IL-2 stock solution has a specific activity of 20-30 × $10^6$ IU/mg for a 1 mg vial. In some embodiments the IL-2 stock solution has a specific activity of 20-30 × $10^6$ IU/mg for a 1 mg vial. In some embodiments the IL-2 stock solution has a specific activity of 25 × $10^6$ IU/mg for a 1 mg vial. In some embodiments the IL-2 stock solution has a specific activity of 30 × $10^6$ IU/mg for a 1 mg vial. In some embodiments, the IL- 2 stock solution has a final concentration of 4-8 × $10^6$ IU/mg of IL-2. In some embodiments, the IL- 2 stock solution has a final concentration of 5-7 × $10^6$ IU/mg of IL-2. In some embodiments, the IL-2 stock solution has a final concentration of 6 × $10^6$ IU/mg of IL-2. In some embodiments, the IL-2 stock solution is prepared as described in Example 4. In some embodiments, first expansion culture media comprises about 10,000 IU/mL of IL-2, about 9,000 IU/mL of IL-2, about 8,000 IU/mL of IL-2, about 7,000 IU/mL of IL-2, about 6000 IU/mL of IL-2 or about 5,000 IU/mL of IL-2. In some embodiments, first expansion culture media comprises about 9,000 IU/mL of IL-2, to about 5,000 IU/mL of IL-2. In some embodiments, first expansion culture media comprises about 8,000 IU/mL of IL-2, to about 6,000 IU/mL of IL-2. In some embodiments, first expansion culture media comprises about 7,000 IU/mL of IL-2, to about 6,000 IU/mL of IL-2. In some embodiments, first expansion culture media comprises about 6,000 IU/mL of IL-2. In an embodiment, the cell culture medium further comprises IL-2. In some embodiments, the cell culture medium comprises about 3000 IU/mL of IL-2. In an embodiment, the cell culture medium further comprises IL-2. In a preferred embodiment, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or about 8000 IU/mL of IL-2.

In some embodiments, the first expansion is performed by further supplementing the cell culture medium of the second population of TILs with OKT-3, IL-15, OX40 agonistic antibody and/or 4-1BB agonistic antibody. For instance, the cell culture medium of the second population of TILs is supplemented with OKT3, IL-15, an OX40 agonistic antibody, a 4-1BB agonistic antibody, a combination of OKT3 and IL-15, a combination of OKT3 and an OX40 agonistic antibody, a combination of OKT3 and a 4-1BB agonistic antibody, a combination of IL-15 and an OX40 agonistic antibody, a combination of IL-15, and a 4-1BB agonistic antibody, a combination of an OX40 agonistic antibody and a 4-1BB agonistic antibody, a combination of OKT3, IL-15, and an OX40 agonistic antibody, a combination of OKT3, IL-15, and a 4-1BB agonistic antibody, a combination of OKT3, an OX40 agonistic antibody, and a 4-1BB agonistic antibody, a combination of IL-15, an OX40 agonistic antibody, and a 4-1BB agonistic antibody, a combination of OKT3, IL-15, an OX40 agonistic antibody, and a 4-1BB agonistic antibody, and any combination thereof. In some embodiments, IL-15 is not included.

After obtaining the small biopsy, core biopsy or fine needle aspirate tumor fragments, for example such as described in Step A of Figure 7A, the resulting cells are cultured in serum containing IL-2 under conditions that favor the growth of TILs over tumor and other cells. In some embodiments, the tumor fragments are incubated in 2 mL wells in media comprising inactivated human AB serum with 6000 IU/mL of IL-2. This primary cell population is cultured for a period of days, generally from 1 to 19 days, resulting in a TIL population of at least 5 × $10^7$ (*i.e.,* 50 million) TIL cells by at least day 11 to day 19. This primary cell population may be cultured for a period of 1 to 18 days, resulting in a bulk TIL population of at least 5 × $10^7$ (*i.e.,* 50 million) TIL cells by at least day 18. This primary cell population may be cultured for a period of 1 to 17 days, resulting in a bulk TIL population of at least 5 × $10^7$ (*i.e.,* 50 million) TIL cells by at least day 17. This primary cell population may be cultured for a period of 1 to 16 days, resulting in a bulk TIL population of at least 5 × $10^7$ *(i.e.,* 50 million) TIL cells by at least day 16. This primary cell population may be cultured for a period of 1 to 15 days, resulting in a bulk TIL population of at least 5 × $10^7$ *(i.e.,* 50 million) TIL cells by at least day 15. This primary cell population may be cultured for a period of 1 to 14 days, resulting in a bulk TIL population of at least 5 × $10^7$ (*i.e.,* 50 million) TIL cells by at least day 14. This primary cell population may be cultured for a period of 1 to 13 days, resulting in a bulk TIL population of at least 5 × $10^7$ *(i.e.,* 50 million) TIL cells by at least day 13. This primary cell population may be cultured for a period of 1 to 12 days, resulting in a bulk TIL population of at least 5 × $10^7$ *(i.e.,* 50 million) TIL cells by at least day 12. This primary cell population may be cultured for a period of 1 to 11 days, resulting in a bulk TIL population of at least 5 × $10^7$ (*i.e.,* 50 million) TIL cells by at least day 11. TIL population may be at least about 5 × $10^7$ *(i.e.,* 50 million) TIL cells by the end of the first expansion, step (for example such as those described in Step B of Figure 7, which can include processes referred to as pre-REP).

In a preferred embodiment, expansion of TILs may be performed using an initial bulk TIL expansion step (for example such as those described in Step B of Figure7, which can include processes referred to as pre-REP) as described below and herein, followed by a second expansion (Step D, including processes referred to as rapid expansion protocol (REP) steps) as described below under Step D and herein, followed by optional cryopreservation, and followed by a second Step D (including processes referred to as restimulation REP steps) as described below and herein. The TILs obtained from this process may be optionally characterized for phenotypic characteristics and metabolic parameters as described herein.

In some embodiments, the first expansion culture medium is referred to as "CM", an abbreviation for culture media. In some embodiments, CM for Step B consists of RPMI 1640 with GlutaMAX, supplemented with 10% human AB serum, 25 mM Hepes, and 10 mg/mL gentamicin. In embodiments where cultures are initiated in gas-permeable flasks with a 40 mL capacity and a 10 $cm^2$ gas-permeable silicon bottom (for example, G-Rex10; Wilson Wolf Manufacturing, New Brighton, MN), each flask was loaded with 1-2 biopsy cores in 10-40 mL of CM with IL-2. Both the G-Rex10 and 24-well plates were incubated in a humidified incubator at 37°C in 5% $CO_2$ and 5 days after culture initiation, half the media was removed and replaced with fresh CM and IL-2 and after day 2, half the media was changed every 2-3 days. In some embodiments, when the core biopsy is from an ovarian tumor *(e.g.,* of ovarian origin), cores are incubated in CM and IL-2 for 1-2 days. In some embodiments, when the core biopsy is from a pancreatic tumor cells *(e.g.,* of pancreatic origin), cores are incubated in CM and IL-2/IL-15/IL-21 for 1-2 days. In some embodiments, at day 3, OKT3 and feeder cells are added to the cores. In some embodiments, at day 3, 30 ng OKT3 and $10^6$ feeder cells are added to the cores.

The small biopsy fragments or cores are cultured in serum containing IL-2 under conditions that favor the growth of TILs over tumor and other cells. In some embodiments, the small biopsy fragments or cores are incubated in 2 mL wells in media comprising inactivated human AB serum (or, in some cases, as outlined herein, in the presence of aAPC cell population) with 6000 IU/mL of IL-2. This primary cell population is cultured for a period of days, generally from 1 to 19 days, resulting in a bulk TIL population, of at least about 5×$10^7$ TIL cells at day 11. In some embodiments, the growth media during the first expansion comprises IL-2 or a variant thereof. In some embodiments, the IL is recombinant human IL-2 (rhIL-2). In some embodiments the IL-2 stock solution has a specific activity of 20-30×$10^6$ IU/mg for a 1 mg vial. In some embodiments the IL-2 stock solution has a specific activity of 20×$10^6$ IU/mg for a 1 mg vial. In some embodiments the IL-2 stock solution has a specific activity of 25×$10^6$ IU/mg for a 1 mg vial. In some embodiments the IL-2 stock solution has a specific activity of 30×$10^6$ IU/mg for a 1 mg vial. In some embodiments, the IL- 2 stock solution has a final concentration of 4-8×$10^6$ IU/mg of IL-2. In some embodiments, the IL- 2 stock solution has a final concentration of 5-7×$10^6$ IU/mg of IL-2. In some embodiments, the IL- 2 stock solution has a final concentration of 6×$10^6$ IU/mg of IL-2. In some embodiments, the IL-2 stock solution is prepare as described in Example E. In some embodiments, the first expansion culture media comprises about 10,000 IU/mL of IL-2, about 9,000 IU/mL of IL-2, about 8,000 IU/mL of IL-2, about 7,000 IU/mL of IL-2, about 6000 IU/mL of IL-2 or about 5,000 IU/mL of IL-2. In some embodiments, the first expansion culture media comprises about 9,000 IU/mL of IL-2 to about 5,000 IU/mL of IL-2. In some embodiments, the first expansion culture media comprises about 8,000 IU/mL of IL-2 to about 6,000 IU/mL of IL-2. In some embodiments, the first expansion culture media comprises about 7,000 IU/mL of IL-2 to about 6,000 IU/mL of IL-2. In some embodiments, the first expansion culture media comprises about 6,000 IU/mL of IL-2. In an embodiment, the cell culture medium further comprises IL-2. In some embodiments, the cell culture medium comprises about 3000 IU/mL of IL-2. In an embodiment, the cell culture medium further comprises IL-2. In a preferred embodiment, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or about 8000 IU/mL of IL-2.

In some embodiments, first expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15. In some embodiments, the first expansion culture media comprises about 500 IU/mL of IL-15 to about 100 IU/mL of IL-15. In some embodiments, the first expansion culture media comprises about 400 IU/mL of IL-15 to about 100 IU/mL of IL-15. In some embodiments, the first expansion culture media comprises about 300 IU/mL of IL-15 to about 100 IU/mL of IL-15. In some embodiments, the first expansion culture media comprises about 200 IU/mL of IL-15. In some embodiments, the cell culture medium comprises about 180 IU/mL of IL-15. In an embodiment, the cell culture medium further comprises IL-15. In a preferred embodiment, the cell culture medium comprises about 180 IU/mL of IL-15. In some embodiments, IL-15 is included when the cores are from a pancreatic tumor *(e.g.,* of pancreatic origin).

In some embodiments, first expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 IU/mL of IL-21, or about 0.5 IU/mL of IL-21. In some embodiments, the first expansion culture media comprises about 20 IU/mL of IL-21 to about 0.5 IU/mL of IL-21. In some embodiments, the first expansion culture media comprises about 15 IU/mL of IL-21 to about 0.5 IU/mL of IL-21. In some embodiments, the first expansion culture media comprises about 12 IU/mL of IL-21 to about 0.5 IU/mL of IL-21. In some embodiments, the first expansion culture media comprises about 10 IU/mL of IL-21 to about 0.5 IU/mL of IL-21. In some embodiments, the first expansion culture media comprises about 5 IU/mL of IL-21 to about 1 IU/mL of IL-21. In some embodiments, the first expansion culture media comprises about 2 IU/mL of IL-21. In some embodiments, the cell culture medium comprises about 1 IU/mL of IL-21. In some embodiments, the cell culture medium comprises about 0.5 IU/mL of IL-21. In an embodiment, the cell culture medium further comprises IL-21. In a preferred embodiment, the cell culture medium comprises about 1 IU/mL of IL-21. In some embodiments, IL-21 is included when the cores are from a pancreatic tumor *(e.g.,* of pancreatic origin).

In an embodiment, the cell culture medium comprises OKT-3 antibody. In some embodiments, the cell culture medium comprises about 30 ng/mL of OKT-3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 μg/mL of OKT-3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT-3 antibody. In some embodiments, the cell culture medium does not comprise OKT-3 antibody. In some embodiments, the OKT-3 antibody is muromonab.

TABLE 3: Amino acid sequences of muromonab (exemplary OKT-3 antibody)

| Identifier | Sequence (One-Letter Amino Acid Symbols) |
|---|---|
| SEQ ID NO:1 Muromonab heavy chain | QVQLQQSGAE LARPGASVKM SCKASGYTFT RYTMHWVKQR PGQGLEWIGY INPSRGYTNY 60<br>NQKFKDKATL TTDKSSSTAY MQLSSLTSED SAVYYCARYY DDHYCLDYWG QGTTLTVSSA 120<br>KTTAPSVYPL APVCGGTTGS SVTLGCLVKG YFPEPVTLTW NSGSLSSGVH TFPAVLQSDL 180<br>YTLSSSVTVT SSTWPSQSIT CNVAHPASST KVDKKIEPRP KSCDKTHTCP PCPAPELLGG 240<br>PSVFLFPPKP KDTLMISRTP EVTCVVVDVS HEDPEVKFNW YVDGVEVHNA KTKPREEQYN 300<br>STYRVVSVLT VLHQDWLNGK EYKCKVSNKA LPAPIEKTIS KAKGQPREPQ VYTLPPSRDE 360<br>LTKNQVSLTC LVKGFYPSDI AVEWESNGQP ENNYKTTPPV LDSDGSFFLY SKLTVDKSRW 420<br>QQGNVFSCSV MHEALHNHYT QKSLSLSPGK 450 |
| SEQ ID NO:2 Muromonab light chain | QIVLTQSPAI MSASPGEKVT MTCSASSSVS YMNWYQQKSG TSPKRWIYDT SKLASGVPAH 60<br>FRGSGSGTSY SLTISGMEAE DAATYYCQQW SSNPFTFGSG TKLEINRADT APTVSIFPPS 120<br>SEQLTSGGAS VVCFLNNFYP KDINVKWKID GSERQNGVLN SWTDQDSKDS TYSMSSTLTL 180<br>TKDEYERHNS YTCEATHKTS TSPIVKSFNR NEC 213 |

In some embodiments, the cell culture medium comprises one or more TNFRSF agonists in a cell culture medium. In some embodiments, the TNFRSF agonist comprises a 4-1BB agonist. In some embodiments, the TNFRSF agonist is a 4-1BB agonist, and the 4-1BB agonist is selected from the group consisting of urelumab, utomilumab, EU-101, a fusion protein, and fragments, derivatives, variants, biosimilars, and combinations thereof. In some embodiments, the TNFRSF agonist is added at a concentration sufficient to achieve a concentration in the cell culture medium of between 0.1 μg/mL and 100 μg/mL. In some embodiments, the TNFRSF agonist is added at a concentration sufficient to achieve a concentration in the cell culture medium of between 20 μg/mL and 40 μg/mL.

In some embodiments, in addition to one or more TNFRSF agonists, the cell culture medium further comprises IL-2 at an initial concentration of about 3000 IU/mL and OKT-3 antibody at an initial concentration of about 30 ng/mL, and wherein the one or more TNFRSF agonists comprises a 4-1BB agonist.

In some embodiments, the first expansion culture medium is referred to as "CM", an abbreviation for culture media. In some embodiments, it is referred to as CM1 (culture medium 1). In some embodiments, CM consists of RPMI 1640 with GlutaMAX, supplemented with 10% human AB serum, 25 mM Hepes, and 10 mg/mL gentamicin. In embodiments where cultures are initiated in gas-permeable flasks with a 40 mL capacity and a 10 $cm^2$ gas-permeable silicon bottom (for example, G-Rex10; Wilson Wolf Manufacturing, New Brighton, MN). In some embodiments, the CM is the CM1 described in the Examples, *see,* Example 1 In some embodiments, the first expansion occurs in an initial cell culture medium or a first cell culture medium. In some embodiments, the initial cell culture medium or the first cell culture medium comprises IL-2.

The first expansion (including processes such as for example those described in Step B of Figure 7, which can include those sometimes referred to as the pre-REP) process may be about 1-19 days, a time period sufficient to obtain $5 \times 10^7$ *(i.e.,* 50 million) TILs, as discussed in the examples and figures. The first expansion (including processes such as for example those described in Step B of Figure 7, which can include those sometimes referred to as the pre-REP) may be divided into two phases, 1-2 days and 3 to 19 days, as provided in Figure 7 and 38. The first expansion of Step B may be divided into two phases, 1-2 days (cultured with IL-2 or IL2/IL-15/IL-21) and 3 to 19 days (cultured with OKT3 and feeders), as provided in Figure 7 and 38.

In some embodiments, the first TIL expansion can proceed for 3 days, 4 days, 5 days, 6 days, 7 days, 8 days, 9 days, 10 days, 11 days, 12 days, in order to obtain at least $5 \times 10^7$ *(i.e.,* 50 million) TILs. In some methods disclosed herein, the first TIL expansion can proceed for 1 days to 19 days, in order to obtain at least $5 \times 10^7$ *(i.e.,* 50 million) TILs. In some methods disclosed herein, the first TIL expansion can proceed for 2 days to 19 days, in order to obtain at least $5 \times 10^7$ *(i.e.,* 50 million) TILs. In some methods disclosed herein, the first TIL expansion can proceed for 3 days to 19 days, in order to obtain at least $5 \times 10^7$ (*i.e.,* 50 million) TILs. In some methods disclosed herein, the first TIL expansion can proceed for 6 days to 19 days, in order to obtain at least $5 \times 10^7$ (*i.e.,* 50 million) TILs. In some methods disclosed herein, the first TIL expansion can proceed for 7 days to 19 days, in order to obtain at least $5 \times 10^7$ *(i.e.,* 50 million) TILs. In some methods disclosed herein, the first TIL expansion can proceed for 8 days to 19 days, in order to obtain at least $5 \times 10^7$ *(i.e.,* 50 million) TILs. In some methods disclosed herein, the first TIL expansion can proceed for 9 days to 19 days, in order to obtain at least $5 \times 10^7$ (*i.e.,* 50 million) TILs. In some methods disclosed herein, the first TIL expansion can proceed for 10 days to 19 days, in order to obtain at least $5 \times 10^7$ (*i.e.,* 50 million) TILs. In some methods disclosed herein, the first TIL expansion can proceed for 11 days to 19 days, in order to obtain at least $5 \times 10^7$ *(i.e.,* 50 million) TILs. In some methods disclosed herein, the first TIL expansion can proceed for 12 days to 19 days, in order to obtain at least $5 \times 10^7$ (*i.e.,* 50 million) TILs. In some methods disclosed herein, the first TIL expansion can proceed for 13 days to 19 days, in order to obtain at least $5 \times 10^7$ *(i.e.,* 50 million) TILs. In some methods disclosed herein, the first TIL expansion can proceed for 16 days, in order to obtain at least $5 \times 10^7$ *(i.e.,* 50 million) TILs. In some methods disclosed herein, the first TIL expansion can proceed for 17 days, in order to obtain at least $5 \times 10^7$ *(i.e.,* 50 million) TILs. In some methods disclosed herein, the first TIL expansion can proceed for 18 days, in order to obtain at least $5 \times 10^7$ (*i.e.,* 50 million) TILs. In some methods disclosed herein, the first TIL expansion can proceed for 19 days, in order to obtain at least $5 \times 10^7$ *(i.e.,* 50 million) TILs. In some embodiments, the small biopsy *(e.g.,* the core biopsy) is removed from the culture at about day 1, 2, or 3. In some embodiments, the small biopsy *(e.g.,* the core biopsy) is removed from the culture at day 3.

In some embodiments, a combination of IL-2, IL-7, IL-15, and/or IL-21 are employed as a combination during the first expansion. In some embodiments, IL-2, IL-7, IL-15, and/or IL-21 as well as any combinations thereof can be included during the first expansion, including for example during a Step B processes according to Figure 7, as well as described herein. In some embodiments, a combination of IL-2, IL-15, and IL-21 are employed as a combination during the first expansion. In some embodiments, IL-2, IL-15, and IL-21 as well as any combinations thereof can be included during Step B processes according to Figure 7 and as described herein. In some embodiments, IL-15 and/or IL-21 is added at day 1. In some embodiments, IL-15 and/or IL-21 is added at day 1 if the core biopsy is from a pancreatic tumor *(e.g.,* of pancreatic origin). In some embodiments, IL-15 is not included. In some embodiments, IL-21 is not included. In some embodiments, neither IL-15 nor IL-21 is not included.

In some embodiments, the first expansion, for example, Step B according to Figure 7, is performed in a closed system bioreactor. In some embodiments, a closed system is employed for the TIL expansion, as described herein. In some embodiments, a single bioreactor is employed. In some embodiments, the single bioreactor employed is for example a GREX-10 or a GREX-100. In some embodiments, the closed system bioreactor is a single bioreactor.

In some embodiments, the first TIL expansion generates at least $50 \times 10^6$ TILs, *e.g.,* $50 \times 106$ (*i.e.,* $5 \times 10^7$), $55 \times 10^6$, $60 \times 10^6$, $65 \times 10^6$, $70 \times 10^6$, $75 \times 10^6$, $80 \times 10^6$, $85 \times 10^6$, $90 \times 10^6$, $95 \times 10^6$, $100 \times 10^6$, $105 \times 10^6$, $110 \times 10^6$, $115 \times 10^6$, $120 \times 10^6$, $125 \times 10^6$, $150 \times 10^6$, $200 \times 10^6$, $300 \times 10^6$, $400 \times 10^6$, or more.

### C. STEP C: First Expansion to Second Expansion Transition

In some cases, the bulk TIL population obtained from the first expansion, including for example the TIL population obtained from for example, Step B as indicated in Figure 7, can be cryopreserved immediately, using the protocols discussed herein below. Alternatively, the TIL population obtained from the first expansion, referred to as the second TIL population, can be subjected to a second expansion (which can include expansions sometimes referred to as REP) and then cryopreserved as discussed below. Similarly, in the case where genetically modified TILs will be used in therapy, the first TIL population (sometimes referred to as the bulk TIL population) or the second TIL population (which can in some embodiments include populations referred to as the REP TIL populations) can be subjected to genetic modifications for suitable treatments prior to expansion or after the first expansion and prior to the second expansion.

In some embodiments, the TILs obtained from the first expansion (for example, from Step B as indicated in Figure 7) are stored until phenotyped for selection. In some embodiments, the TILs obtained from the first expansion (for example, from Step B as indicated in Figure 7) are not stored and proceed directly to the second expansion. In some embodiments, the TILs obtained from the first expansion are not cryopreserved after the first expansion and prior to the second expansion. In some embodiments, the transition from the first expansion to the second expansion occurs at about 3 days, 4, days, 5 days, 6 days, 7 days, 8 days, 9 days, 10 days, 11 days, 12 days, 13 days, 14 days, 15 days, 16 days, 17 days, 18 days, or 19 days from when the cores are added to the culture medium for the first expansion. In some embodiments, the transition from the first expansion to the second expansion occurs at about 3 days to 19 days from when the cores are added to the culture medium for the first expansion. In some embodiments, the transition from the first expansion to the second expansion occurs at about 4 days to 19 days from when the cores are added to the culture medium for the first expansion. In some embodiments, the transition from the first expansion to the second expansion occurs at about 4 days to 19 days from when the cores are added to the culture medium for the first expansion. In some embodiments, the transition from the first expansion to the second expansion occurs at about 7 days to 19 days from when the cores are added to the culture medium for the first expansion. In some embodiments, the transition from the first expansion to the second expansion occurs at about 10 days to 19 days from when the cores are added to the culture medium for the first expansion. In some embodiments, the transition from the first expansion to the second expansion occurs at about 11 days to 19 days from when the cores are added to the culture medium for the first expansion. In some embodiments, the transition from the first expansion to the second expansion occurs at about 12 days to 19 days from when the cores are added to the culture medium for the first expansion. In some embodiments, the transition from the first expansion to the second expansion occurs at about 13 days to 19 days from when the cores are added to the culture medium for the first expansion. In some embodiments, the transition from the first expansion to the second expansion occurs at about 14 days to 19 days from when the cores are added to the culture medium for the first expansion. In some embodiments, the transition from the first expansion to the second expansion occurs at about 15 days to 19 days from when the cores are added to the culture medium for the first expansion. In some embodiments, the transition from the first expansion to the second expansion occurs at about 16 days to 19 days from when the cores are added to the culture medium for the first expansion. In some embodiments, the transition from the first expansion to the second expansion occurs at about 17 days to 19 days from when the cores are added to the culture medium for the first expansion. In some embodiments, the transition from the first expansion to the second expansion occurs at about 18 days to 19 days from when the cores are added to the culture medium for the first expansion. In some embodiments, the transition from the first expansion to the second expansion occurs at about 10 days from when the cores are added to the culture medium for the first expansion. In some embodiments, the transition from the first expansion to the second expansion occurs at about 11 days from when the cores are added to the culture medium for the first expansion. In some embodiments, the transition from the first expansion to the second expansion occurs at about 12 days from when the cores are added to the culture medium for the first expansion. In some embodiments, the transition from the first expansion to the second expansion occurs at about 13 days from when the cores are added to the culture medium for the first expansion. In some embodiments, the transition from the first expansion to the second expansion occurs at about 14 days from when the cores are added to the culture medium for the first expansion. In some embodiments, the transition from the first expansion to the second expansion occurs at about 15 days from when the cores are added to the culture medium for the first expansion. In some embodiments, the transition from the first expansion to the second expansion occurs at about 16 days from when the cores are added to the culture medium for the first expansion. In some embodiments, the transition from the first expansion to the second expansion occurs at about 17 days from when the cores are added to the culture medium for the first expansion. In some embodiments, the transition from the first expansion to the second expansion occurs at about 18 days from when the cores are added to the culture medium for the first expansion. In some embodiments, the transition from the first expansion to the second expansion occurs at about 19 days from when the cores are added to the culture medium for the first expansion.

In some embodiments, the TILs are not stored after the first expansion and prior to the second expansion, and the TILs proceed directly to the second expansion (for example, in some embodiments, there is no storage during the transition from Step B to Step D as shown in Figure 7). In some embodiments, the transition occurs in closed system, as described herein. In some embodiments, the TILs from the first expansion, the second population of TILs, proceeds directly into the second expansion with no transition period.

In some embodiments, the transition from the first expansion to the second expansion, for example, Step C according to Figure 7, is performed in a closed system bioreactor. In some embodiments, a closed system is employed for the TIL expansion, as described herein. In some embodiments, a single bioreactor is employed. In some embodiments, the single bioreactor employed is for example a GREX-10 or a GREX-100. In some embodiments, the closed system bioreactor is a single bioreactor.

**D. STEP D: Second Expansion**

In some embodiments, the TIL cell population is expanded in number after harvest and initial bulk processing for example, after Step A and Step B, and the transition referred to as Step C, as indicated in Figure 7). This further expansion is referred to herein as the second expansion, which can include expansion processes generally referred to in the art as a rapid expansion process (REP; as well as processes as indicated in Step D of Figure 7). The second expansion is generally accomplished using a culture media comprising a number of components, including feeder cells, a cytokine source, and an anti-CD3 antibody, in a gas-permeable container.

In some embodiments, the second expansion or second TIL expansion (which can include expansions sometimes referred to as REP; as well as processes as indicated in Step D of Figure 7) of TIL can be performed using any TIL flasks or containers known by those of skill in the art. In some embodiments, the second TIL expansion can proceed for 10 days, 11 days or 12 days.

In an embodiment, the second expansion can be performed in a gas permeable container using the methods of the present disclosure (including for example, expansions referred to as REP; as well as processes as indicated in Step D of Figure 7). For example, TILs can be rapidly expanded using non-specific T-cell receptor stimulation in the presence of interleukin-2 (IL-2) or interleukin-15 (IL-15). The non-specific T-cell receptor stimulus can include, for example, about 30 ng/mL of OKT3, a mouse monoclonal anti-CD3 antibody (commercially available from Ortho-McNeil, Raritan, NJ or Miltenyi Biotech, Auburn, CA). TILs can be expanded to induce further stimulation of the TILs *in vitro* by including one or more antigens during the second expansion, including antigenic portions thereof, such as epitope(s), of the cancer, which can be optionally expressed from a vector, such as a human leukocyte antigen A2 (HLA-A2) binding peptide, *e.g.,* 0.3 $\mu$M MART-1 :26-35 (27 L) or gpl 00:209-217 (210M), optionally in the presence of a T-cell growth factor, such as 300 IU/mL IL-2 or IL-15. Other suitable antigens may include, *e.g.,* NY-ESO-1, TRP-1, TRP-2, tyrosinase cancer antigen, MAGE-A3, SSX-2, and VEGFR2, or antigenic portions thereof. TIL may also be rapidly expanded by re-stimulation with the same antigen(s) of the cancer pulsed onto HLA-A2-expressing antigen-presenting cells. Alternatively, the TILs can be further re-stimulated with, e.g., example, irradiated, autologous lymphocytes or with irradiated HLA-A2+ allogeneic lymphocytes and IL-2. In some methods disclosed herein, the re-stimulation occurs as part of the second expansion. In some embodiments, the second expansion occurs in the presence of irradiated, autologous lymphocytes or with irradiated HLA-A2+ allogeneic lymphocytes and IL-2. In some embodiments, $13 \times 10^6$ feeder cells are added during the second expansion. In some embodiments, $13 \times 10^6$ feeder cells are added at the start of second expansion.

In an embodiment, the cell culture medium further comprises IL-2. In some embodiments, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or between 8000 IU/mL of IL-2.

In an embodiment, the cell culture medium comprises OKT3 antibody. In some embodiments, the cell culture medium comprises about 30 ng/mL of OKT3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 $\mu$g/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, a combination of IL-2, IL-7, IL-15, and/or IL-21 are employed as a combination during the second expansion. In some embodiments, IL-2, IL-7, IL-15, and/or IL-21 as well as any combinations thereof can be included during the second expansion, including for example during a Step D processes according to Figure 7, as well as described herein. In some embodiments, a combination of IL-2, IL-15, and IL-21 are employed as a combination during the second expansion. In some embodiments, IL-2, IL-15, and IL-21 as well as any combinations thereof can be included during Step D processes according to Figure 7 and as described herein.

In some embodiments, the second expansion can be conducted in a supplemented cell culture medium comprising IL-2, OKT-3, and antigen-presenting feeder cells. In some embodiments, the second expansion occurs in a supplemented cell culture medium. In some embodiments, the supplemented cell culture medium comprises IL-2, OKT-3, and antigen-presenting feeder cells. In some embodiments, the second cell culture medium comprises IL-2, OKT-3, and antigen-presenting cells (APCs; also referred to herein as antigen-presenting feeder cells, feeder cells or feeders). In some embodiments, the second expansion occurs in a cell culture medium comprising IL-2, OKT-3, and antigen-presenting feeder cells (*i.e.,* antigen presenting cells).

In some embodiments, the second expansion is performed by further supplementing the cell culture medium of the population of TILs in the second expansion with IL-15, OX40 agonistic antibody and/or 4-1BB agonistic antibody (sometimes referred to as the second population of TILs). For instance, the cell culture medium of the population of TILs in the second expansion is supplemented with IL-15, an OX40 agonistic antibody, a 4-1BB agonistic antibody, a combination of IL-15 and an OX40 agonistic antibody, a combination of IL-15, and a 4-1BB agonistic antibody, a combination of an OX40 agonistic antibody and a 4-1BB agonistic antibody, a combination of IL-15, an OX40 agonistic antibody, and a 4-1BB agonistic antibody, and any combinations thereof. In some embodiments, OX40 is included when the cores are from an ovarian tumor *(e.g.,* of ovarian origin). In some embodiments, OKT-3 is added during the first and/or second expansion. In some embodiments, OKT-3 is added at day-3, along with the antigen-presenting feeder cells (APCs). In some embodiments, when the sample is from an FNA, OKT-3 is added at day-1, along with the antigen-presenting feeder cells (APCs).

In some embodiments the antigen-presenting feeder cells (APCs) are PBMCs. In an embodiment, the ratio of TILs to PBMCs and/or antigen-presenting cells in the rapid expansion and/or the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to PBMCs in the rapid expansion and/or the second expansion is between 1 to 50 and 1 to 300. In an embodiment, the ratio of TILs to PBMCs in the rapid expansion and/or the second expansion is between 1 to 100 and 1 to 200.

In an embodiment, REP and/or the second expansion is performed in flasks with the bulk TILs being mixed with a 100- or 200-fold excess of inactivated feeder cells, 30 mg/mL OKT3 anti-CD3 antibody and 3000 IU/mL IL-2 in 150 ml media. Media replacement is done (generally 2/3 media replacement via respiration with fresh media) until the cells are transferred to an alternative growth chamber. Alternative growth chambers include GREX flasks and gas permeable containers as more fully discussed below.

The second expansion (which can include processes referred to as the REP process) may be 11-14 days, as discussed in the examples and figures. In some embodiments, the second expansion is 11 days. In some embodiments, the second expansion is 12 days.

In an embodiment, REP and/or the second expansion may be performed using T-175 flasks and gas permeable bags as previously described (Tran, et al., J. Immunother. 2008, 31, 742-51; Dudley, et al., J. Immunother. 2003, 26, 332-42) or gas permeable cultureware (G-Rex flasks). In some embodiments, the second expansion (including expansions referred to as rapid expansions) is performed in T-175 flasks, and about $1 \times 10^6$ TILs suspended in 150 mL of media may be added to each T-175 flask. The TILs may be cultured in a 1 to 1 mixture of CM and AIM-V medium, supplemented with 3000 IU per mL of IL-2 and 30 ng per ml of anti-CD3. The T-175 flasks may be incubated at 37° C in 5% $CO_2$. Half the media may be exchanged on day 5 using 50/50 medium with 3000 IU per mL of IL-2. In some embodiments, on day 7 cells from two T-175 flasks may be combined in a 3 L bag and 300 mL of AIM V with 5% human AB serum and 3000 IU per mL of IL-2 was added to the 300 ml of TIL suspension. The number of cells in each bag was counted every day or two and fresh media was added to keep the cell count between 0.5 and $2.0 \times 10^6$ cells/mL.

In an embodiment, the second expansion (which can include expansions referred to as REP, as well as those referred to in Step D of Figure 7) may be performed in 500 mL capacity gas permeable flasks with 100 cm gas-permeable silicon bottoms (G-Rex 100, commercially available from Wilson Wolf Manufacturing Corporation, New Brighton, MN, USA), $5 \times 10^6$ or $10 \times 10^6$ TIL may be cultured with PBMCs in 400 mL of 50/50 medium, supplemented with 5% human AB serum, 3000 IU per mL of IL-2 and 30 ng per ml of anti-CD3 (OKT3). The G-Rex 100 flasks may be incubated at 37°C in 5% $CO_2$. On day 5, 250 mL of supernatant may be removed and placed into centrifuge bottles and centrifuged at 1500 rpm (491 $\times$ g) for 10 minutes. The TIL pellets may be re-suspended with 150 mL of fresh medium with 5% human AB serum, 3000 IU per mL of IL-2, and added back to the original G-Rex 100 flasks. When TILs are expanded serially in G-Rex 100 flasks, on day 7 the TIL in each G-Rex 100 may be suspended in the 300 mL of media present in each flask and the cell suspension may be divided into 3-100 mL aliquots that may be used to seed 3 G-Rex 100 flasks. Then 150 mL of AIM-V with 5% human AB serum and 3000 IU per mL of IL-2 may be added to each flask. The G-Rex 100 flasks may be incubated at 37° C in 5% $CO_2$ and after 4 days 150 mL of AIM-V with 3000 IU per mL of IL-2 may be added to each G-Rex 100 flask. The cells may be harvested on day 11 of culture. The cells may be harvested on day 12 of culture. In some embodiments, cultures are expanded into a G-Rex 500.

In an embodiment, the second expansion (including expansions referred to as REP) is performed in flasks with the bulk TILs being mixed with a 100- or 200-fold excess of inactivated feeder cells, 30 mg/mL OKT3 anti-CD3 antibody and 3000 IU/mL IL-2 in 150 ml media. In some embodiments, media replacement is done until the cells are transferred to an alternative growth chamber. In some embodiments, 2/3 of the media is replaced by aspiration of spent media and replacement with an equivalent volumne of fresh media. In some embodiments, alternative growth chambers include GRex flasks and gas permeable containers as more fully discussed below.

In an embodiment, the second expansion (including expansions referred to as REP) is performed and further comprises a step wherein TILs are selected for superior tumor reactivity. Any selection method known in the art may be used. For example, the methods described in U.S. Patent Application Publication No. 2016/0010058 A1, may be used for selection of TILs for superior tumor reactivity.

Optionally, a cell viability assay can be performed after the second expansion (including expansions referred to as the REP expansion), using standard assays known in the art. For example, a trypan blue exclusion assay can be done on a sample of the bulk TILs, which selectively labels dead cells and allows a viability assessment. In some embodiments, TIL samples can be counted and viability determined using a Cellometer K2 automated cell counter (Nexcelom Bioscience, Lawrence, MA). In some embodiments, viability is determined according to the Cellometer K2 Image Cytometer Automatic Cell Counter protocol.

In some embodiments, the second expansion (including expansions referred to as REP) of TIL can be performed using T-175 flasks and gas-permeable bags as previously described (Tran KQ, Zhou J, Durflinger KH, et al., 2008, J Immunother., 31:742-751, and Dudley ME, Wunderlich JR, Shelton TE, et al. 2003, J Immunother., 26:332-342) or gas-permeable G-Rex flasks. In some embodiments, the second expansion is performed using flasks. In some embodiments, the second expansion is performed using gas-permeable G-Rex flasks. In some embodiments, the second expansion is performed in T-175 flasks, and about $1 \times 10^6$ TIL are suspended in about 150 mL of media and this is added to each T-175 flask. The TIL are cultured with irradiated (50 Gy) allogeneic PBMC as "feeder" cells at a ratio of 1 to 100 and the cells were cultured in a 1 to 1 mixture of CM and AIM-V medium (50/50 medium), supplemented with 3000 IU/mL of IL-2 and 30 ng/mL of anti-CD3. The T-175 flasks are incubated at 37°C in 5% $CO_2$. In some embodiments, half the media is changed on day 5 using 50/50 medium with 3000 IU/mL of IL-2. In some embodiments, on day 7, cells from 2 T-175 flasks are combined in a 3 L bag and 300 mL of AIM-V with 5% human AB serum and 3000 IU/mL of IL-2 is added to the 300 mL of TIL suspension. The number of cells in each bag can be counted every day or two and fresh media can be added to keep the cell count between about 0.5 and about $2.0 \times 10^6$ cells/mL. In some embodiments, cultures are expanded into a G-Rex 500.

In some embodiments, the second expansion (including expansions referred to as REP) are performed in 500 mL capacity flasks with 100 $cm^2$ gas-permeable silicon bottoms (G-Rex100, Wilson Wolf) (Fig. 1), about $5 \times 10^6$ or $10 \times 10^6$ TIL are cultured with irradiated allogeneic PBMC at a ratio of 1 to 100 in 400 mL of 50/50 medium, supplemented with 3000 IU/mL of IL-2 and 30 ng/mL of anti-CD3. The G-Rex100 flasks are incubated at 37°C in 5% $CO_2$. In some embodiments, on day 5, 250 mL of supernatant is removed and placed into centrifuge bottles and centrifuged at 1500 rpm ($491 \times g$) for 10 minutes. The TIL pellets can then be resuspended with 150 mL of fresh 50/50 medium with 3000 IU/ mL of IL-2 and added back to the original G-Rex100 flasks. In embodiments where TILs are expanded serially in G-Rex100 flasks, on day 7 the TIL in each G-Rex100 are suspended in the 300 mL of media present in each flask and the cell suspension was divided into three 100 mL aliquots that are used to seed 3 G-Rex100 flasks. Then 150 mL of AIM-V with 5% human AB serum and 3000 IU/mL of IL-2 is added to each flask. The G-Rex100 flasks are incubated at 37°C in 5% $CO_2$ and after 4 days 150 mL of AIM-V with 3000 IU/mL of IL-2 is added to each G-Rex100 flask. The cells are harvested on day 14 of culture. In some embodiments, cultures are expanded into a G-Rex 500.

In some embodiments, the second expansion culture medium *(e.g.,* sometimes referred to as CM2 or the second cell culture medium), comprises IL-2, OKT-3, as well as the antigen-presenting feeder cells (APCs), as discussed in more detail below.

In some embodiments, the second expansion, for example, Step D according to Figure 7, is performed in a closed system bioreactor. In some embodiments, a closed system is employed for the TIL expansion, as described herein. In some embodiments, a single bioreactor is employed. In some embodiments, the single bioreactor employed is for example a GREX-10 or a GREX-100. In some embodiments, the closed system bioreactor is a single bioreactor.

1. Feeder Cells and Antigen Presenting Cells

In an embodiment, the second expansion procedures described herein (for example including expansion such as those described in Step D from Figure 7, as well as those referred to as REP) require an excess of feeder cells during REP TIL expansion and/or during the second expansion. In many embodiments, the feeder cells are peripheral blood mononuclear cells (PBMCs) obtained from standard whole blood units from healthy blood donors. The PBMCs are obtained using standard methods such as Ficoll-Paque gradient separation.

In general, the allogenic PBMCs are inactivated, either via irradiation or heat treatment, and used in the REP procedures, as described in the examples, in particular Example 4, which provides an exemplary protocol for evaluating the replication incompetence of irradiate allogeneic PBMCs.

In some embodiments, PBMCs are considered replication incompetent and accepted for use in the TIL expansion procedures described herein if the total number of viable cells on day 14 is less than the initial viable cell number put into culture on day 0 of the REP and/or day 0 of the second expansion (*i.e.*, the start day of the second expansion). See, for example, Examples 3 and/or 4.

In some embodiments, PBMCs are considered replication incompetent and accepted for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion (*i.e.*, the start day of the second expansion). In some embodiments, the PBMCs are cultured in the presence of 30 ng/mL OKT3 antibody and 3000 IU/mL IL-2. See, for example, Examples 3 and/or 4.

In some embodiments, PBMCs are considered replication incompetent and accepted for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion (*i.e.*, the start day of the second expansion). In some embodiments, the PBMCs are cultured in the presence of 5-60 ng/mL OKT3 antibody and 1000-6000 IU/mL IL-2. In some embodiments, the PBMCs are cultured in the presence of 10-50 ng/mL OKT3 antibody and 2000-5000 IU/mL IL-2. In some embodiments, the PBMCs are cultured in the presence of 20-40 ng/mL OKT3 antibody and 2000-4000 IU/mL IL-2. In some embodiments, the PBMCs are cultured in the presence of 25-35 ng/ml OKT3 antibody and 2500-3500 IU/mL IL-2.

In some embodiments, the antigen-presenting feeder cells are PBMCs. In some embodiments, the antigen-presenting feeder cells are artificial antigen-presenting feeder cells. In an embodiment, the ratio of TILs to antigen-presenting feeder cells in the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to antigen-presenting feeder cells in the second expansion is between 1 to 50 and 1 to 300. In an embodiment, the ratio of TILs to antigen-presenting feeder cells in the second expansion is between 1 to 100 and 1 to 200.

In an embodiment, the second expansion procedures described herein require an excess of feeder cells during the second expansion. In many embodiments, the feeder cells are peripheral blood mononuclear cells (PBMCs) obtained from standard whole blood units from healthy blood donors. The PBMCs are obtained using standard methods such as Ficoll-Paque gradient separation. In an embodiment, artificial antigen-presenting (aAPC) cells are used in place of PBMCs.

In general, the allogenic PBMCs are inactivated, either via irradiation or heat treatment, and used in the TIL expansion procedures described herein, including the exemplary procedures described in, for example, Figure 7.

In an embodiment, artificial antigen presenting cells are used in the second expansion as a replacement for, or in combination with, PBMCs.

The expansion methods described herein generally use culture media with high doses of a cytokine, in particular IL-2, as is known in the art.

Alternatively, using combinations of cytokines for the rapid expansion and or second expansion of TILS is additionally possible, with combinations of two or more of IL-2, IL-15 and IL-21 as is generally outlined in International Publication No. WO 2015/189356 and W International Publication No. WO 2015/189357. Thus, possible combinations include IL-2 and IL-15, IL-2 and IL-21, IL-15 and IL-21 and IL-2, IL-15 and IL-21, with the latter finding particular use in many embodiments. The use of combinations of cytokines specifically favors the generation of lymphocytes, and in particular T-cells as described therein.

### E. STEP E: Harvest TILs

After the second expansion step, cells can be harvested. In some embodiments the TILs are harvested after one, two, three, four or more expansion steps, for example as provided in Figure 7. In some embodiments the TILs are harvested after two expansion steps, for example as provided in Figure 7.

TILs can be harvested in any appropriate and sterile manner, including for example by centrifugation. Methods for TIL harvesting are well known in the art and any such know methods can be employed with the present process. In some embodiments, TILS are harvest using an automated system.

In some embodiments, the harvest, for example, Step E according to Figure 7, is performed from a closed system bioreactor. In some embodiments, a closed system is employed for the TIL expansion, as described herein. In some embodiments, a single bioreactor is employed. In some embodiments, the single bioreactor employed is for example a GREX-10 or a GREX-100. In some embodiments, the closed system bioreactor is a single bioreactor.

### F. STEP F: Final Formulation/Transfer to Infusion Bag

After Steps A through E as provided in an exemplary order in Figure 7 and as outlined in detailed above and herein are complete, cells are transferred to a container for use in administration to a patient. In some embodiments, once a therapeutically sufficient number of TILs are obtained using the expansion methods described above, they are transferred to a container for use in administration to a patient.

Also disclosed herein but not claimed, TILs expanded using processes of the present disclosure are administered to a patient as a pharmaceutical composition. Also disclosed herein but not claimed, the pharmaceutical composition is a suspension of TILs in a sterile buffer. TILs expanded using processes of the present disclosure may be administered by any suitable route as known in the art. Also disclosed herein but not claimed, the T-cells are administered as a single intra-arterial or intravenous infusion, which preferably lasts approximately 30 to 60 minutes. Other suitable routes of administration include intraperitoneal, intrathecal, and intralymphatic.

In an embodiment, TILs expanded using processes of the foregoing disclosure may be for administration as compositions further comprising a cyropreservant. In an embodiment, TILs expanded using processes of the foregoing disclosure may be for administration as compositions further comprising a cyropreservant and an isotonic agent. In an embodiment, TILs expanded using processes of the foregoing disclosure may be for administration as compositions further comprising a cyropreservant comprising dimethylsulfoxide and an isotonic agent comprising sodium chloride, sodium gluconate, and sodium acetate. In an embodiment, TILs expanded using processes of the foregoing disclosure may be for administration as compositions further comprising a cyropreservant comprising dimethylsulfoxide and dextran 40 and an isotonic agent comprising sodium chloride, sodium gluconate, and sodium acetate. In an embodiment, TILs expanded using processes of the foregoing disclosure may be for administration as compositions delivered in a sterile infusion bag, such compositions further comprising a cyropreservant comprising dimethylsulfoxide and dextran 40 and an isotonic agent comprising sodium chloride, sodium gluconate, and sodium acetate.

1. Pharmaceutical Compositions, Dosages, and Dosing Regimens

In an embodiment, TILs expanded using the methods of the present disclosure are for administration to a patient as a pharmaceutical composition. The pharmaceutical composition may be a suspension of TILs in a sterile buffer. TILs expanded using processes of the present disclosure may be administered by any suitable route as known in the art. In some embodiments, the T-cells may be for administration as a single intra-arterial or intravenous infusion, which preferably lasts approximately 30 to 60 minutes. Other suitable routes of administration include intraperitoneal, intrathecal, and intralymphatic administration.

Any suitable dose of TILs can be administered. In some embodiments, a therapeutically sufficient number of TILs are needed for a suitable dosage. Also disclosed herein but not claimed, from about $2.3\times10^{10}$ to about $13.7\times10^{10}$ TILs are administered, with an average of around $7.8\times10^{10}$ TILs, particularly if the cancer is melanoma. In an embodiment, about $1.2\times10^{10}$ to about $4.3\times10^{10}$ of TILs are administered. Also disclosed herein but not claimed, about $3\times10^{10}$ to about $12\times10^{10}$ TILs are administered. Also disclosed herein but not claimed, about $4\times10^{10}$ to about $10\times10^{10}$ TILs are administered. Also disclosed herein but not claimed, about $5\times10^{10}$ to about $8\times10^{10}$ TILs are administered. Also disclosed herein but not claimed, about $6\times10^{10}$ to about $8\times10^{10}$ TILs are administered. Also disclosed herein but not claimed, about $7\times10^{10}$ to about $8\times10^{10}$ TILs are administered. In some embodiments, the therapeutically effective dosage is about $2.3\times10^{10}$ to about $13.7\times10^{10}$. In some embodiments, the therapeutically effective dosage is about $7.8\times10^{10}$ TILs, particularly of the cancer is melanoma. In some embodiments, the therapeutically effective dosage is about $1.2\times10^{10}$ to about $4.3\times10^{10}$ of TILs. In some embodiments, the therapeutically effective dosage is about $3\times10^{10}$ to about $12\times10^{10}$ TILs. In some embodiments, the therapeutically effective dosage is about $4\times10^{10}$ to about $10\times10^{10}$ TILs. In some embodiments, the therapeutically effective dosage is about $5\times10^{10}$ to about $8\times10^{10}$ TILs. In some embodiments, the therapeutically effective dosage is about $6\times10^{10}$ to about $8\times10^{10}$ TILs. In some embodiments, the therapeutically effective dosage is about $7\times10^{10}$ to about $8\times10^{10}$ TILs.

In some embodiments, the number of the TILs provided in the pharmaceutical compositions of the invention is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In an embodiment, the number of the TILs provided in the pharmaceutical compositions of the invention is in the range of $1\times10^6$ to $5\times10^6$, $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$, and $5\times10^{12}$ to $1\times10^{13}$. In some embodiments, the therapeutically effective dosage is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$,

$9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$.

In some embodiments, the concentration of the TILs provided in the pharmaceutical compositions of the invention is less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v of the pharmaceutical composition.

In some embodiments, the concentration of the TILs provided in the pharmaceutical compositions of the invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v of the pharmaceutical composition.

In some embodiments, the concentration of the TILs provided in the pharmaceutical compositions of the invention is in the range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12% or about 1% to about 10% w/w, w/v or v/v of the pharmaceutical composition.

In some embodiments, the concentration of the TILs provided in the pharmaceutical compositions of the invention is in the range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, about 0.1% to about 0.9% w/w, w/v or v/v of the pharmaceutical composition.

In some embodiments, the amount of the TILs provided in the pharmaceutical compositions of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of the TILs provided in the pharmaceutical compositions of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

The TILs provided in the pharmaceutical compositions of the invention are effective over a wide dosage range. The exact dosage will depend upon the route of administration, the form in which the compound is administered, the gender and age of the subject to be treated, the body weight of the subject to be treated, and the preference and experience of the attending physician. The clinically-established dosages of the TILs may also be used if appropriate. The amounts of the pharmaceutical compositions administered using the methods herein, such as the dosages of TILs, will be dependent on the human or mammal being treated, the severity of the disorder or condition, the rate of administration, the disposition of the active pharmaceutical ingredients and the discretion of the prescribing physician.

Also disclosed herein but not claimed, TILs may be administered in a single dose. Such administration may be by injection, e.g., intravenous injection. TILs may be administered in multiple doses. Dosing may be once, twice, three times, four times, five times, six times, or more than six times per year. Dosing may be once a month, once every two weeks, once a week, or once every other day. Administration of TILs may continue as long as necessary.

In some embodiments, an effective dosage of TILs is about $1\times10^6$, $2\times10^6$, $3\times10^6$, $4\times10^6$, $5\times10^6$ $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$,

$4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In some embodiments, an effective dosage of TILs is in the range of $1\times10^6$ to $5\times10^6$ $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$, and $5\times10^{12}$ to $1\times10^{13}$.

In some embodiments, an effective dosage of TILs is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg.

In some embodiments, an effective dosage of TILs is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg.

An effective amount of the TILs may be administered in either single or multiple doses by any of the accepted modes of administration of agents having similar utilities, including intranasal and transdermal routes, by intra-arterial injection, intravenously, intraperitoneally, parenterally, intramuscularly, subcutaneously, topically, by transplantation, or by inhalation.

### IV. TIL Manufacturing Processes - Gen 3 Processes

Also disclosed herein is a population of TILs made by the method of any of GEN 3 processes modified to use a small biopsy, core biopsy or fine needle aspirate as the. In some embodiments, the invention provides the method of any of GEN 3 processes modified to use a small biopsy, core biopsy or fine needle aspirate as the source of T cells for expansion in the priming first expansion of any such GEN 3 process, wherein (1) the duration of the priming first expansion is lengthened to achieve the desired TIL cell count in the population of TILs harvested after the rapid second expansion of such GEN 3 process or (2) the duration of the rapid second expansion of such GEN 3 process is lengthened to achieve the desired TIL cell count in the population of TILs harvested after the rapid second expansion or (3) the duration of the priming first expansion is lengthened and the duration of the rapid second expansion of such GEN 3 process is lengthened to achieve the desired TIL cell count in the population of TILs harvested after the rapid second expansion.

Also disclosed herein is a population of TILs made by the method of any of GEN 3 processes modified to use a small biopsy, core biopsy or fine needle aspirate as the source of T cells for expansion in the priming first expansion of any such GEN 3 process, wherein (1) the duration of the priming first expansion is lengthened to achieve the desired TIL cell count in the population of TILs harvested after the rapid second expansion of such GEN 3 process or (2) the duration of the rapid second expansion of such GEN 3 process is lengthened to achieve the desired TIL cell count in the population of TILs harvested after the rapid second expansion or (3) the duration of the priming first expansion is lengthened and the duration of the the rapid second expansion of such GEN 3 process is lengthened to achieve the desired TIL cell count in the population of TILs harvested after the rapid second expansion.

Without being limited to any particular theory, it is believed that the priming first expansion that primes an activation of T cells obtained from a tumor core or fragment obtained from a donor followed by the rapid second expansion that boosts the activation of T cells as described in some methods of the invention allows the preparation of expanded T cells that retain a "younger" phenotype, and as such the expanded T cells of the invention are expected to exhibit greater cytotoxicity against cancer cells than T cells expanded by other methods. In particular, it is believed that an activation of T cells that is primed by exposure to an anti-CD3 antibody (*e.g.* OKT-3), IL-2 and optionally antigen-presenting cells (APCs) and then boosted by subsequent exposure to additional anti-CD-3 antibody (*e.g.* OKT-3), IL-2 and APCs as taught by the methods of the invention limits or avoids the maturation of T cells in culture, yielding a population of T cells with a less mature phenotype, which T cells are less exhausted by expansion in culture and exhibit greater cytotoxicity against cancer cells. Thus, disclosed herein is a method of expanding TILs comprising: (a) performing a priming first expansion of a first population of T cells obtained from a tumor core or fragment obtained from a donor by culturing the first population of T cells to effect growth and to prime an activation of the first population of T cells; (b) after the activation of the first population of T cells primed in step (a) begins to decay, performing a rapid second expansion of the first population of T cells by culturing the first population of T cells to effect growth and to boost the activation of the first population of T cells to obtain a second population of T cells; and (c) harvesting the second population of T cells. The Gen 3 process, methods of treatment using TILs from the Gen 3 process, and compositions of TILs prepared by the Gen 3 can be used in conjunction with small biopsy/core biopsy with durations of expansion periods adjusted as needed to achieve necessary cell counts, as provided herein below and throughout the present application.

The step of rapid second expansion may be split into a plurality of steps to achieve a scaling up of the culture by: (a) performing the rapid second expansion by culturing T cells in a small scale culture in a first container, *e.g.,* a G-REX 100MCS container, for a period of about 3 to 4 days, and then (b) effecting the transfer of the T cells in the small scale culture to a second container larger than the first container, *e.g.,* a G-REX 500MCS container, and culturing the T cells from the small scale culture in a larger scale culture in the second container for a period of about 4 to 7 days. The step of rapid expansion may be split into a plurality of steps to achieve a scaling out of the culture by: (a) performing the rapid second expansion by culturing T cells in a first small scale culture in a first container, *e.g.,* a G-REX 100MCS container, for a period of about 3 to 4 days, and then (b) effecting the transfer and apportioning of the T cells from the first small scale culture into and amongst at least 2, 3, 4, 5, 6, 7, 8, 9, 10, 11, 12, 13, 14, 15, 16, 17, 18, 19, or 20 second containers that are equal in size to the first container, wherein in each second container the portion of the T cells from first small scale culture transferred to such second container is cultured in a second small scale culture for a period of about 4 to 7 days. The step of rapid expansion may be split into a plurality of steps to achieve a scaling out and scaling up of the culture by: (a) performing the rapid second expansion by culturing T cells in a small scale culture in a first container, *e.g.,* a G-REX 100MCS container, for a period of about 3 to 4 days, and then (b) effecting the transfer and apportioning of the T cells from the small scale culture into and amongst at least 2, 3, 4, 5, 6, 7, 8, 9, 10, 11, 12, 13, 14, 15, 16, 17, 18, 19, or 20 second containers that are larger in size than the first container, *e.g.,* G-REX 500MCS containers, wherein in each second container the portion of the T cells from the small scale culture transferred to such second container is cultured in a larger scale culture for a period of about 4 to 7 days. The step of rapid expansion may be split into a plurality of steps to achieve a scaling out and scaling up of the culture by: (a) performing the rapid second expansion by culturing T cells in a small scale culture in a first container, *e.g.,* a G-REX 100MCS container, for a period of about 4 days, and then (b) effecting the transfer and apportioning of the T cells from the small scale culture into and amongst 2, 3 or 4 second containers that are larger in size than the first container, *e.g.,* G-REX 500MCS containers, wherein in each second container the portion of the T cells from the small scale culture transferred to such second container is cultured in a larger scale culture for a period of about 5 days.

The rapid second expansion may be performed after the activation of T cells effected by the priming first expansion begins to decrease, abate, decay or subside.

The rapid second expansion may be performed after the activation of T cells effected by the priming first expansion has decreased by at or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%.

The rapid second expansion may be performed after the activation of T cells effected by the priming first expansion has decreased by a percentage in the range of at or about 1% to 100%.

The rapid second expansion may be performed after the activation of T cells effected by the priming first expansion has decreased by a percentage in the range of at or about 1% to 10%, 10% to 20%, 20% to 30%, 30% to 40%, 40% to 50%, 50% to 60%, 60% to 70%, 70% to 80%, 80% to 90%, or 90% to 100%.

The rapid second expansion may be performed after the activation of T cells effected by the priming first expansion has decreased by at least at or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99%.

The rapid second expansion may be performed after the activation of T cells effected by the priming first expansion has decreased by up to at or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%.

The decrease in the activation of T cells effected by the priming first expansion may be determined by a reduction in the amount of interferon gamma released by the T cells in response to stimulation with antigen.

The priming first expansion of T cells may be performed during a period of up to at or about 7 days.

The priming first expansion of T cells may be performed during a period of up to at or about 1 day, 2 days, 3 days, 4 days, 5 days, 6 days, or 7 days.

The priming first expansion of T cells may be performed during a period of 1 day, 2 days, 3 days, 4 days, 5 days, 6 days, or 7 days.

The rapid second expansion of T cells may be performed during a period of up to at or about 11 days.

The rapid second expansion of T cells may be performed during a period of up to at or about 1 day, 2 days, 3 days, 4 days, 5 days, 6 days, 7 days, 8 days, 9 days, 10 days or 11 days.

The rapid second expansion of T cells may be performed during a period of 1 day, 2 days, 3 days, 4 days, 5 days, 6 days, 7 days, 8 days, 9 days, 10 days or 11 days.

The priming first expansion of T cells may be performed during a period of from at or about 1 day to at or about 7 days and the rapid second expansion of T cells is performed during a period of from at or about 1 day to at or about 11 days.

The priming first expansion of T cells may be performed during a period of up to at or about 1 day, 2 days, 3 days, 4 days, 5 days, 6 days, or 7 days and the rapid second expansion of T cells is performed during a period of up to at or about 1 day, 2 days, 3 days, 4 days, 5 days, 6 days, 7 days, 8 days, 9 days, 10 days or 11 days.

The priming first expansion of T cells may be performed during a period of from at or about 1 day to at or about 7 days and the rapid second expansion of T cells is performed during a period of from at or about 1 day to at or about 9 days.

The priming first expansion of T cells may be performed during a period of 7 days and the rapid second expansion of T cells is performed during a period of 9 days.

The T cells may be tumor infiltrating lymphocytes (TILs).

The T cells may be marrow infiltrating lymphocytes (MILs).

The T cells may be obtained from a donor suffering from a cancer.

The T cells may be TILs obtained from a core biopsy or fine needle aspirate obtained from a tumor in a patient suffering from a cancer.

The T cells may be MILs obtained from bone marrow of a patient suffering from a hematologic malignancy.

In some embodiments, the donor is suffering from a cancer. In some embodiments, the cancer is the cancer is selected from the group consisting of melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), glioblastoma (including GBM), gastrointestinal cancer, renal cancer, and renal cell carcinoma. In some embodiments, the cancer is selected from the group consisting of melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), glioblastoma (including GBM), gastrointestinal cancer, renal cancer, and renal cell carcinoma. In some embodments, the donor is suffering from a tumor. In some embodiments, the tumor is a liquid tumor. In some embodiments, the tumor is a solid tumor. In some embodiments, the donor is suffering from a hematologic malignancy.

An exemplary TIL process known as process 3 (also referred to herein as GEN3) containing some of these features is depicted in Figure 85 (in particular, e.g., Figure 85B), and some of the advantages of this embodiment of the present invention over process 2A are described in Figures 1, 2, 30, and 31 (in particular, e.g., Figure 85B). Two embodiments of process 3 are shown in Figures 1 and 30 (in particular, e.g., Figure 85B). Process 2A or Gen 2 is also described in U.S. Patent Publication No. 2018/0280436

As discussed and generally outlined herein, TILs are taken from a patient sample and manipulated to expand their number prior to transplant into a patient using the TIL expansion process described herein and referred to as Gen 3. In some embodiments, the TILs may be optionally genetically manipulated as discussed below. In some embodiments, the TILs may be cryopreserved prior to or after expansion. Once thawed, they may also be restimulated to increase their metabolism prior to infusion into a patient.

Also disclosed herein but not claimed, the priming first expansion (including processes referred herein as the pre-Rapid Expansion (Pre-REP), as well as processes shown in Figure 85 (in particular, e.g., Figure 85B) as Step B) is shortened to 1 to 7 days and the rapid second expansion (including processes referred to herein as Rapid Expansion Protocol (REP) as well as processes shown in Figure 85 (in particular, e.g., Figure 85B) as Step D) is shortened to 1 to 9 days, as discussed in detail below as well as in the examples and figures. In some embodiments, the priming first expansion (for example, an expansion described as Step B in Figure 85 (in particular, *e.g.,* Figure 85B)) is shortened to 7 days and the rapid second expansion (for example, an expansion as described in Step D in Figure 85 (in particular, *e.g.,* Figure 85B)) is 7 to 9 days. In some embodiments, the combination of the priming first expansion and rapid second expansion (for example, expansions described as Step B and Step D in Figure 85 (in particular, *e.g.,* Figure 85B)) is 14-16 days, as discussed in detail below and in the examples and figures. Particularly, it is considered that certain embodiments of the present invention comprise a priming first expansion step in which TILs are activated by exposure to an anti-CD3 antibody, *e.g.,* OKT-3 in the presence of IL-2 or exposure to an antigen in the presence of at least IL-2 and an anti-CD3 antibody *e.g.* OKT-3. In certain embodiments, the TILs which are activated in the priming first expansion step as described above are a first population of TILs *i.e.* which are a primary cell population.

The "Step" Designations A, B, C, *etc.,* below are in reference to the non-limiting example in Figure 85 (in particular, *e.g.,* Figure 85B) and in reference to certain non-limiting embodiments described herein. The ordering of the Steps below and in Figure 85 (in particular, *e.g.,* Figure 85B) is exemplary and any combination or order of steps, as well as additional steps, repetition of steps, and/or omission of steps is contemplated by the present application and the methods disclosed herein.

In some embodiments, if the tumor is metastatic and the primary lesion has been efficiently treated/removed in the past, removal of one of the metastatic lesions may be needed. In some embodiments, the least invasive approach is to remove a skin lesion, or a lymph node on the neck or axillary area when available. A skin lesion is removed or small biopsy thereof may be removed. A lymph node or small biopsy thereof may be removed. In some embodiments, a lung or liver metastatic lesion, or an intra-abdominal or thoracic lymph node or small biopsy can thereof can be employed.

In some embodiments, the tumor is a melanoma. In some embodiments, the small biopsy for a melanoma comprises a mole or portion thereof.

In some embodiments, the small biopsy is a punch biopsy. In some embodiments, the punch biopsy is previously obtained with a circular blade pressed into the skin. In some embodiments, the punch biopsy is previously obtained with a circular blade pressed into the skin. around a suspicious mole. In some embodiments, the punch biopsy is previously obtained with a circular blade pressed into the skin, and a round piece of skin is removed. The small biopsy may be a punch biopsy and round portion of the tumor may be removed.

In some embodiments, the small biopsy is an excisional biopsy. Also disclosed herein but not claimed, the small biopsy is an excisional biopsy and the entire mole or growth is removed. Also disclosed herein but not claimed, the small biopsy is an excisional biopsy and the entire mole or growth is removed along with a small border of normal-appearing skin.

In some embodiments, the small biopsy is an incisional biopsy. Also disclosed herein but not claimed, the small biopsy is an incisional biopsy and only the most irregular part of a mole or growth is taken. Also disclosed herein but not claimed, the small biopsy is an incisional biopsy and the incisional biopsy is used when other techniques can't be completed, such as if a suspicious mole is very large.

In some embodiments, the small biopsy is a lung biopsy. In some embodiments, the small biopsy is previously obtained by bronchoscopy. Generally, bronchoscopy, the patient is put under anesthesia, and a small goes through the nose or mouth, down the throat, and into the bronchial passages, where small tools are used to remove some tissue. Also disclosed herein but not claimed, where the tumor or growth cannot be reached via bronchoscopy, a transthoracic needle biopsy can be employed. Generally, for a transthoracic needle biopsy, the patient is also under anesthesia and a needle is inserted through the skin directly into the suspicious spot to remove a small sample of tissue. Also disclosed herein but not claimed, a transthoracic needle biopsy may require interventional radiology (for example, the use of x-rays or CT scan to guide the needle). In some embodiments, the small biopsy is previously obtained by needle biopsy. In some embodiments, the small biopsy is previously obtained endoscopic ultrasound (for example, an endoscope with a light and is placed through the mouth into the esophagus). In some embodiments, the small biopsy is previously obtained surgery.

In some embodiments, the small biopsy is a head and neck biopsy. In some embodiments, the small biopsy is an incisional biopsy. Also disclosed herein but not claimed, the small biopsy is an incisional biopsy, wherein a small piece of tissue is cut from an abnormal-looking area. Also disclosed herein but not claimed, if the abnormal region is easily accessed, the sample may be taken without hospitalization. Also disclosed herein but not claimed, if the tumor is deeper inside the mouth or throat, the biopsy may need to be done in an operating room, with general anesthesia. In some embodiments, the small biopsy is an excisional biopsy. Also disclosed herein but not claimed, the small biopsy is an excisional biopsy, wherein the whole area is removed. In some embodiments, the small biopsy is a fine needle aspiration (FNA). Also disclosed herein but not claimed, the small biopsy is a fine needle aspiration (FNA), wherein a very thin needle attached to a syringe is used to extract (aspirate) cells from a tumor or lump. In some embodiments, the small biopsy is a punch biopsy. The small biopsy may be a punch biopsy, wherein punch forceps are used to remove a piece of the suspicious area.

In some embodiments, the small biopsy is a cervical biopsy. In some embodiments, the small biopsy is previously obtained via colposcopy. Generally, colposcopy methods employ the use of a lighted magnifying instrument attached to magnifying binoculars (a colposcope) which is then used to biopsy a small section of the surface of the cervix. In some embodiments, the small biopsy is a conization/cone biopsy. Also disclosed herein but not claimed, the small biopsy is a conization/cone biopsy, wherein an outpatient surgery may be needed to remove a larger piece of tissue from the cervix. In some embodiments, the cone biopsy, in addition to helping to confirm a diagnosis, a cone biopsy can serve as an initial treatment.

The term "solid tumor" refers to an abnormal mass of tissue that usually does not contain cysts or liquid areas. Solid tumors may be benign or malignant. The term "solid tumor cancer refers to malignant, neoplastic, or cancerous solid tumors. Solid tumor cancers include, but are not limited to, sarcomas, carcinomas, and lymphomas, such as cancers of the lung, breast, triple negative breast cancer, prostate, colon, rectum, and bladder. In some embodiments, the cancer is selected from cervical cancer, head and neck cancer, glioblastoma, ovarian cancer, sarcoma, pancreatic cancer, bladder cancer, breast cancer, triple negative breast cancer, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

In some embodiments, the sample from the tumor is obtained as a fine needle aspirate (FNA), a core biopsy, or a small biopsy (including, for example, a punch biopsy). In some embodiments, sample is placed first into a G-Rex 10. In some embodiments, sample is placed first into a G-Rex 10 when there are 1 or 2 core biopsy and/or small biopsy samples. In some embodiments, sample is placed first into a G-Rex 100 when there are 3, 4, 5, 6, 8, 9, or 10 or more core biopsy and/or small biopsy samples. In some embodiments, sample is placed first into a G-Rex 500 when there are 3, 4, 5, 6, 8, 9, or 10 or more core biopsy and/or small biopsy samples.

The FNA can be obtained from a tumor selected from the group consisting of lung, melanoma, head and neck, cervical, ovarian, pancreatic, glioblastoma, colorectal, and sarcoma. In some embodiments, the FNA is previously obtained from a lung tumor, such as a lung tumor from a patient with non-small cell lung cancer (NSCLC). In some cases, the patient with NSCLC has previously undergone a surgical treatment.

TILs described herein can be obtained from an FNA sample. In some cases, the FNA sample is previously obtained or isolated from the patient using a fine gauge needle ranging from a 18 gauge needle to a 25 gauge needle. The fine gauge needle can be 18 gauge, 19 gauge, 20 gauge, 21 gauge, 22 gauge, 23 gauge, 24 gauge, or 25 gauge. In some embodiments, the FNA sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In some cases, the TILs described herein are obtained from a core biopsy sample. In some cases, the small biopsy or core biopsy sample is previously obtained or isolated from the patient using a surgical or medical needle ranging from a 11 gauge needle to a 16 gauge needle. The needle can be 11 gauge, 12 gauge, 13 gauge, 14 gauge, 15 gauge, or 16 gauge. In some embodiments, the core biopsy sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In some embodiments, the TILs are obtained from tumor digests. In some embodiments, tumor digests were generated by incubation in enzyme media, for example but not limited to RPMI 1640, 2mM GlutaMAX, 10 μg/mL gentamicin, 30 U/mL DNase, and 1.0 mg/mL collagenase, followed by mechanical dissociation (GentleMACS, Miltenyi Biotec, Auburn, CA). After placing the tumor in enzyme media, the tumor can be mechanically dissociated for approximately 1 minute. The solution can then be incubated for 30 minutes at 37 °C in 5% $CO_2$ and it then mechanically disrupted again for approximately 1 minute. After being incubated again for 30 minutes at 37 °C in 5% $CO_2$, the tumor can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% $CO_2$. In some embodiments, at the end of the final incubation if the cell suspension contained a large number of red blood cells or dead cells, a density gradient separation using Ficoll can be performed to remove these cells.

In some embodiments, the harvested cell suspension prior to the first expansion step is called a "primary cell population" or a "freshly harvested" cell population.

In some embodiments, cells can be optionally frozen after sample harvest and stored frozen prior to entry into the expansion described in Step B, which is described in further detail below, as well as exemplified in Figure 85.

### A. STEP A: Obtain Patient tumor sample

In general, TILs are initially obtained from a patient tumor sample ("primary TILs") obtained by a core biopsy or similar procedure and are then expanded into a larger population for further manipulation as described herein, optionally cryopreserved, and optionally evaluated for phenotype and metabolic parameters.

A patient tumor sample may be obtained using methods known in the art, generally via surgical resection, core biopsy, needle biopsy or other means for obtaining a sample that contains a mixture of tumor and TIL cells. In general, the tumor sample may be from any solid tumor, including primary tumors, invasive tumors or metastatic tumors. In some embodiments, the sample can be from multiple small tumor samples or biopsies. In some embodiments, the sample can comprise multiple tumor samples (such as multiple cores) from a single tumor from the same patient. In some embodiments, the sample can comprise multiple tumor samples from one, two, three, or four tumors from the same patient (such as core biopsies obtained from multiple lesions in metastatic disease). In some embodiments, the sample can comprise multiple tumor samples from multiple tumors from the same patient. The tumor sample may also be a liquid tumor, such as a tumor obtained from a hematological malignancy. The solid tumor may be of any cancer type, including, but not limited to, breast, pancreatic, prostate, colorectal, lung, brain, renal, stomach, and skin (including but not limited to squamous cell carcinoma, basal cell carcinoma, and melanoma). In some embodiments, the cancer is selected from cervical cancer, head and neck cancer (including, for example, head and neck squamous cell carcinoma (HNSCC)), glioblastoma (GBM), gastrointestinal cancer, ovarian cancer, sarcoma, pancreatic cancer, bladder cancer, breast cancer, triple negative breast cancer, and non-small cell lung carcinoma. In some embodiments, useful TILs are obtained from malignant melanoma tumors, as these have been reported to have particularly high levels of TILs.

As indicated above, in some embodiments, the TILs are derived from solid tumor cores or fragments. In some embodiments, the solid tumor cores or fragments are subjected to enzymatic digestion. In some embodiments, the tumor cores or fragments are digested in in an enzyme mixture comprising collagenase, DNase, and hyaluronidase. In some embodiments, the tumor cores or fragments are digested in in an enzyme mixture comprising collagenase, DNase, and hyaluronidase for 1-2 hours. In some embodiments, the tumors are digested in in an enzyme mixture comprising collagenase, DNase, and hyaluronidase for 1-2 hours at 37°C, 5% $CO_2$. In some embodiments, the tumor cores or fragments are digested in in an enzyme mixture comprising collagenase, DNase, and hyaluronidase for 1-2 hours at 37°C, 5% $CO_2$ with rotation. In some embodiments, the tumor cores or fragments are digested overnight with constant rotation. In some embodiments, the tumor cores or fragments are digested overnight at 37°C, 5% $CO_2$ with constant rotation. In some embodiments, the tumor cores or fragments are combined with the enzymes to form a tumor digest reaction mixture.

In some embodiments, the TILs are not obtained from tumor digests. In some embodiments, the solid tumor cores are not fragmented.

In some embodiments, the tumor cores or fragments are reconstituted with the lyophilized enzymes in a sterile buffer. In some embodiments, the buffer is sterile HBSS.

In some embodiments, the enzyme mixture comprises collagenase. In some embodiments, the collagenase is collagenase IV. In some embodiments, the working stock for the collagenase is a 100 mg/mL 10X working stock.

In some embodiments, the enzyme mixture comprises DNAse. In some embodiments, the working stock for the DNAse is a 10,000IU/mL 10X working stock.

In some embodiments, the enzyme mixture comprises hyaluronidase. In some embodiments, the working stock for the hyaluronidase is a 10 mg/mL 10X working stock.

In some embodiments, the enzyme mixture comprises 10 mg/mL collagenase, 1000 IU/mL DNAse, and 1 mg/mL hyaluronidase.

In some embodiments, the enzyme mixture comprises 10 mg/mL collagenase, 500 IU/mL DNAse, and 1 mg/mL hyaluronidase.

In general, the cell suspension obtained from the tumor core or fragment is called a "primary cell population" or a "freshly obtained" or a "freshly isolated" cell population. In certain embodiments, the freshly obtained cell population of TILs is exposed to a cell culture medium comprising antigen presenting cells, IL-12 and OKT-3.

In some embodiments, TILs can be initially cultured from enzymatic tumor core or fragment digests and tumor cores or fragments previously obtained from patients. In an embodiment, TILs can be initially cultured from enzymatic tumor core or fragment digests and tumor cores or fragments previously obtained from patients.

In some embodiments, the TILs are obtained from tumor fragment or core digests. In some embodiments, tumor fragment or core digests are generated by incubation in enzyme media, for example but not limited to RPMI 1640, 2 mM GlutaMAX, 10 mg/mL gentamicin, 30 U/mL DNase, and 1.0 mg/mL collagenase, followed by mechanical dissociation (GentleMACS, Miltenyi Biotec, Auburn, CA). After placing the tumor fragment or core in enzyme media, the tumor fragment or core can be mechanically dissociated for approximately 1 minute. The solution can then be incubated for 30 minutes at 37 °C in 5% $CO_2$ and it then mechanically disrupted again for approximately 1 minute. After being incubated again for 30 minutes at 37 °C in 5% $CO_2$, the tumor fragment or core can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% $CO_2$. In some embodiments, at the end of the final incubation if the cell suspension contained a large number of red blood cells or dead cells, a density gradient separation using Ficoll can be performed to remove these cells.

Also disclosed herein but not claimed, if the tumor is metastatic and the primary lesion has been efficiently treated/removed in the past, removal of one of the metastatic lesions may be needed. Also disclosed herein but not claimed, the least invasive approach is to remove a skin lesion, or a lymph node on the neck or axillary area when available. Also disclosed herein but not claimed, a skin lesion is removed or small biopsy thereof is removed. Also disclosed herein but not claimed, a lymph node or small biopsy thereof is removed. Also disclosed herein but not claimed, a lung or liver metastatic lesion, or an intra-abdominal or thoracic lymph node or small biopsy can thereof can be employed.

In some embodiments, the tumor is a melanoma. In some embodiments, the small biopsy for a melanoma comprises a mole or portion thereof.

In some embodiments, the small biopsy is a punch biopsy. In some embodiments, the punch biopsy is previously obtained with a circular blade pressed into the skin. In some embodiments, the punch biopsy is previously obtained with a circular blade pressed into the skin. around a suspicious mole. In some embodiments, the punch biopsy is previously obtained with a circular blade pressed into the skin, and a round piece of skin is removed. Also disclosed herein but not claimed, the small biopsy is a punch biopsy and round portion of the tumor is removed.

In some embodiments, the small biopsy is an excisional biopsy. Also disclosed herein but not claimed, the small biopsy is an excisional biopsy and the entire mole or growth is removed. Also disclosed herein but not claimed, the small biopsy is an excisional biopsy and the entire mole or growth is removed along with a small border of normal-appearing skin.

In some embodiments, the small biopsy is an incisional biopsy. Also disclosed herein but not claimed, the small biopsy is an incisional biopsy and only the most irregular part of a mole or growth is taken. In some embodiments, the small biopsy is an incisional biopsy and the incisional biopsy is used when other techniques can't be completed, such as if a suspicious mole is very large.

In some embodiments, the small biopsy is a lung biopsy. In some embodiments, the small biopsy is previously obtained by bronchoscopy. Generally, bronchoscopy, the patient is put under anesthesia, and a small goes through the nose or mouth, down the throat, and into the bronchial passages, where small tools are used to remove some tissue. Also disclosed herein but not claimed, where the tumor or growth cannot be reached via bronchoscopy, a transthoracic needle biopsy can be employed. Generally, for a transthoracic needle biopsy, the patient is also under anesthesia and a needle is inserted through the skin directly into the suspicious spot to remove a small sample of tissue. Also disclosed herein but not claimed, a transthoracic needle biopsy may require interventional radiology (for example, the use of x-rays or CT scan to guide the needle). In some embodiments, the small biopsy is previously obtained by needle biopsy. In some embodiments, the small biopsy is previously obtained endoscopic ultrasound (for example, an endoscope with a light and is placed through the mouth into the esophagus). In some embodiments, the small biopsy is previously obtained surgery.

In some embodiments, the small biopsy is a head and neck biopsy. In some embodiments, the small biopsy is an incisional biopsy. Also disclosed herein but not claimed, the small biopsy is an incisional biopsy, wherein a small piece of tissue is cut from an abnormal-looking area. Also disclosed herein but not claimed, if the abnormal region is easily accessed, the sample may be taken without hospitalization. Also disclosed herein but not claimed, if the tumor is deeper inside the mouth or throat, the biopsy may need to be done in an operating room, with general anesthesia. In some embodiments, the small biopsy is an excisional biopsy. Also disclosed herein but not claimed, the small biopsy is an excisional biopsy, wherein the whole area is removed. In some embodiments, the small biopsy is a fine needle aspiration (FNA). In some embodiments, the small biopsy is a fine needle aspiration (FNA), wherein a very thin needle may be attached to a syringe is used to extract (aspirate) cells from a tumor or lump. In some embodiments, the small biopsy is a punch biopsy. In some embodiments, the small biopsy is a punch biopsy, wherein punch forceps may be used to remove a piece of the suspicious area.

In some embodiments, the small biopsy is a cervical biopsy. In some embodiments, the small biopsy is previously obtained via colposcopy. Generally, colposcopy methods employ the use of a lighted magnifying instrument attached to magnifying binoculars (a colposcope) which is then used to biopsy a small section of the surface of the cervix. In some embodiments, the small biopsy is a conization/cone biopsy. Also disclosed herein but not claimed, the small biopsy is a conization/cone biopsy, wherein an outpatient surgery may be needed to remove a larger piece of tissue from the cervix. Also disclosed herein but not claimed, the cone biopsy, in addition to helping to confirm a diagnosis, a cone biopsy can serve as an initial treatment.

The term "solid tumor" refers to an abnormal mass of tissue that usually does not contain cysts or liquid areas. Solid tumors may be benign or malignant. The term "solid tumor cancer refers to malignant, neoplastic, or cancerous solid tumors. Solid tumor cancers include, but are not limited to, sarcomas, carcinomas, and lymphomas, such as cancers of the lung, breast, triple negative breast cancer, prostate, colon, rectum, and bladder. In some embodiments, the cancer is selected from cervical cancer, head and neck cancer, glioblastoma, ovarian cancer, sarcoma, pancreatic cancer, bladder cancer, breast cancer, triple negative breast cancer, and non-small cell lung carcinoma. The tissue structure of solid tumors includes interdependent tissue compartments including the parenchyma (cancer cells) and the supporting stromal cells in which the cancer cells are dispersed and which may provide a supporting microenvironment.

In some embodiments, the sample from the tumor is previously obtained as a fine needle aspirate (FNA), a core biopsy, a small biopsy (including, for example, a punch biopsy). In some embodiments, sample is placed first into a G-Rex 10. In some embodiments, sample is placed first into a G-Rex 10 when there are 1 or 2 core biopsy and/or small biopsy samples. In some embodiments, sample is placed first into a G-Rex 100 when there are 3, 4, 5, 6, 8, 9, or 10 or more core biopsy and/or small biopsy samples. In some embodiments, sample is placed first into a G-Rex 500 when there are 3, 4, 5, 6, 8, 9, or 10 or more core biopsy and/or small biopsy samples.

The FNA can be obtained from a tumor selected from the group consisting of lung, melanoma, head and neck, cervical, ovarian, pancreatic, glioblastoma, colorectal, and sarcoma. In some embodiments, the FNA is previously obtained from a lung tumor, such as a lung tumor from a patient with non-small cell lung cancer (NSCLC). In some cases, the patient with NSCLC has previously undergone a surgical treatment.

TILs described herein can be obtained from an FNA sample. In some cases, the FNA sample is previously obtained or isolated from the patient using a fine gauge needle ranging from an 18 gauge needle to a 25 gauge needle. The fine gauge needle can be 18 gauge, 19 gauge, 20 gauge, 21 gauge, 22 gauge, 23 gauge, 24 gauge, or 25 gauge. In some embodiments, the FNA sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In some cases, the TILs described herein are obtained from a core biopsy sample. In some cases, the core biopsy or small biopsy sample is previously obtained or isolated from the patient using a surgical or medical needle ranging from an 11 gauge needle to a 16 gauge needle. The needle can be 11 gauge, 12 gauge, 13 gauge, 14 gauge, 15 gauge, or 16 gauge. In some embodiments, the core biopsy sample from the patient can contain at least 400,000 TILs, *e.g.,* 400,000 TILs, 450,000 TILs, 500,000 TILs, 550,000 TILs, 600,000 TILs, 650,000 TILs, 700,000 TILs, 750,000 TILs, 800,000 TILs, 850,000 TILs, 900,000 TILs, 950,000 TILs, or more.

In some embodiments, the TILs are obtained from tumor core or fragment digests. In some embodiments, tumor core or fragment digests were generated by incubation in enzyme media, for example but not limited to RPMI 1640, 2mM GlutaMAX, 10 μg/mL gentamicin, 30 U/mL DNase, and 1.0 mg/mL collagenase, followed by mechanical dissociation (GentleMACS, Miltenyi Biotec, Auburn, CA). After placing the tumor core or fragment in enzyme media, the tumor can be mechanically dissociated for approximately 1 minute. The solution can then be incubated for 30 minutes at 37 °C in 5% $CO_2$ and it then mechanically disrupted again for approximately 1 minute. After being incubated again for 30 minutes at 37 °C in 5% $CO_2$, the tumor core or fragment can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% $CO_2$. In some embodiments, at the end of the final incubation if the cell suspension contained a large number of red blood cells or dead cells, a density gradient separation using Ficoll can be performed to remove these cells.

In some embodiments, the cell suspension prior to the priming first expansion step is called a "primary cell population" or a "freshly obtained" or "freshly isolated" cell population.

In some embodiments, cells can be optionally frozen after sample isolation (e.g., after obtaining the tumor sample and/or after obtaining the cell suspension from the tumor sample) and stored frozen prior to entry into the expansion described in Step B, which is described in further detail below, as well as exemplified in Figure 85 (in particular, *e.g.,* Figure 85B).

### B. STEP B: Priming First Expansion

In some embodiments, the present methods provide for younger TILs, which may provide additional therapeutic benefits over older TILs (*i.e.,* TILs which have further undergone more rounds of replication prior to administration to a subject/patient). Features of young TILs have been described in the literature, for example Donia, et al., Scandinavian Journal of Immunology, 75:157-167 (2012); Dudley et al., Clin Cancer Res, 16:6122-6131 (2010); Huang et al., J Immunother, 28(3):258-267 (2005); Besser et al., Clin Cancer Res, 19(17):OF1-OF9 (2013); Besser et al., J Immunother 32:415-423 (2009); Robbins, et al., J Immunol 2004; 173:7125-7130; Shen et al., J Immunother, 30:123-129 (2007); Zhou, et al., J Immunother, 28:53-62 (2005); and Tran, et al., J Immunother, 31:742-751 (2008).

After biopsy or digestion of tumor fragments and/or tumor cores, for example such as described in Step A of Figure 85 (in particular, *e.g.*, Figure 85B), the resulting cells are cultured in serum containing IL-2, OKT-3, and feeder cells (*e.g.*, antigen-presenting feeder cells), under conditions that favor the growth of TILs over tumor and other cells. In some embodiments, the IL-2, OKT-3, and feeder cells are added at culture initiation along with the tumor fragment or core digest and/or tumor fragments or cores (*e.g.,* at Day 0). In some embodiments, the tumor fragments or core digests and/or tumor fragments or cores are incubated in a container with up to 60 fragments or cores per container and with 6000 IU/mL of IL-2. This primary cell population is cultured for a period of days, generally from 1 to 7 days, resulting in a bulk TIL population, generally about $1 \times 10^8$ bulk TIL cells. In some embodiments, this priming first expansion occurs for a period of 5 to 7 days, resulting in a bulk TIL population, generally about $1 \times 10^8$ bulk TIL cells. In some embodiments, this priming first expansion occurs for a period of about 6 to 7 days, resulting in a bulk TIL population, generally about $1 \times 10^8$ bulk TIL cells. In some embodiments, this priming first expansion occurs for a period of about 7 days, resulting in a bulk TIL population, generally about $1 \times 10^8$ bulk TIL cells.

In a preferred embodiment, expansion of TILs may be performed using a priming first expansion step (for example such as those described in Step B of Figure 85 (in particular, *e.g.*, Figure 85B), which can include processes referred to as pre-REP or priming REP and which contains feeder cells from Day 0 and/or from culture initiation) as described below and herein, followed by a rapid second expansion (Step D, including processes referred to as rapid expansion protocol (REP) steps) as described below under Step D and herein, followed by optional cryopreservation, and followed by a second Step D (including processes referred to as restimulation REP steps) as described below and herein. The TILs obtained from this process may be optionally characterized for phenotypic characteristics and metabolic parameters as described herein. In some embodiments, the tumor fragment is between about 1 $mm^3$ and 10 $mm^3$.

In some embodiments, the first expansion culture medium is referred to as "CM", an abbreviation for culture media. In some embodiments, CM for Step B consists of RPMI 1640 with GlutaMAX, supplemented with 10% human AB serum, 25 mM Hepes, and 10 μg/mL gentamicin.

In some embodiments, there are less than or equal to 240 tumor fragments or cores. In some embodiments, there are less than or equal to 240 tumor fragments or cores placed in less than or equal to 4 containers. In some embodiments, the containers are GREX100 MCS flasks. In some embodiments, less than or equal to 60 tumor fragments or cores are placed in 1 container. In some embodiments, each container comprises less than or equal to 500 mL of media per container. In some embodiments, the media comprises IL-2. In some embodiments, the media comprises 6000 IU/mL of IL-2. In some embodiments, the media comprises antigen-presenting feeder cells (also referred to herein as "antigen-presenting cells"). In some embodiments, the media comprises $2.5 \times 10^8$ antigen-presenting feeder cells per container. In some embodiments, the media comprises OKT-3. In some embodiments, the media comprises 30 ng/mL of OKT-3 per container. In some embodiments, the container is a GREX100 MCS flask. In some embodiments, the media comprises 6000 IU/mL of IL-2, 30 ng/mL of OKT-3, and $2.5 \times 10^8$ antigen-presenting feeder cells. In some embodiments, the media comprises 6000 IU/mL of IL-2, 30 ng/mL of OKT-3, and $2.5 \times 10^8$ antigen-presenting feeder cells per container.

After preparation of the tumor fragments or cores, the resulting cells *(i.e.,* which are from the fragments or cores, and which constitute a primary cell population) are cultured in media containing IL-2, antigen-presenting feeder cells and OKT-3 under conditions that favor the growth of TILs over tumor and other cells and which allow for TIL priming and accelerated growth from initiation of the culture on Day 0. In some embodiments, the tumor fragment or core digests and/or tumor fragments or cores are incubated in with 6000 IU/mL of IL-2, as well as antigen-presenting feeder cells and OKT-3. This primary cell population is cultured for a period of days, generally from 1 to 7 days, resulting in a bulk TIL population, generally about $1 \times 10^8$ bulk TIL cells. In some embodiments, the growth media during the priming first expansion comprises IL-2 or a variant thereof, as well as antigen-presenting feeder cells and OKT-3. In some embodiments, the IL-2 is recombinant human IL-2 (rhIL-2). In some embodiments the IL-2 stock solution has a specific activity of 20-30$\times 10^6$ IU/mg for a 1 mg vial. In some embodiments the IL-2 stock solution has a specific activity of $20 \times 10^6$ IU/mg for a 1 mg vial. In some embodiments the IL-2 stock solution has a specific activity of $25 \times 10^6$ IU/mg for a 1 mg vial. In some embodiments the IL-2 stock solution has a specific activity of $30 \times 10^6$ IU/mg for a 1 mg vial. In some embodiments, the IL- 2 stock solution has a final concentration of 4-8$\times 10^6$ IU/mg of IL-2. In some embodiments, the IL- 2 stock solution has a final concentration of 5-7$\times 10^6$ IU/mg of IL-2. In some embodiments, the IL- 2 stock solution has a final concentration of $6 \times 10^6$ IU/mg of IL-2. In some embodiments, the IL-2 stock solution is prepared as described in Example 5. In some embodiments, the priming first expansion culture media comprises about 10,000 IU/mL of IL-2, about 9,000 IU/mL of IL-2, about 8,000 IU/mL of IL-2, about 7,000 IU/mL of IL-2, about 6000 IU/mL of IL-2 or about 5,000 IU/mL of IL-2. In some embodiments, the priming first expansion culture media comprises about 9,000 IU/mL of IL-2 to about 5,000 IU/mL of IL-2. In some embodiments, the priming first expansion culture media comprises about 8,000 IU/mL of IL-2 to about 6,000 IU/mL of IL-2. In some embodiments, the priming first expansion culture media comprises about 7,000 IU/mL of IL-2 to about 6,000 IU/mL of IL-2. In some embodiments, the priming first expansion culture media comprises about 6,000 IU/mL of IL-2. In an embodiment, the cell culture medium further comprises IL-2. In some embodiments, the priming first expansion cell culture medium comprises about 3000 IU/mL of IL-2. In an embodiment, the priming first expansion cell culture medium further comprises IL-2. In a preferred embodiment, the priming first expansion cell culture medium comprises about 3000 IU/mL of IL-2. In an embodiment, the priming first expansion cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the priming first expansion cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or about 8000 IU/mL of IL-2.

In some embodiments, priming first expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15. In some embodiments, the priming first expansion culture media comprises about 500 IU/mL of IL-15 to about 100 IU/mL of IL-15. In some embodiments, the priming first expansion culture media comprises about 400 IU/mL of IL-15 to about 100 IU/mL of IL-15. In some embodiments, the priming first expansion culture media comprises about 300 IU/mL of IL-15 to about 100 IU/mL of IL-15. In some embodiments, the priming first expansion culture media comprises about 200 IU/mL of IL-15. In some embodiments, the priming first expansion cell culture medium comprises about 180 IU/mL of IL-15. In an embodiment, the priming first expansion cell culture medium further comprises IL-15. In a preferred embodiment, the priming first expansion cell culture medium comprises about 180 IU/mL of IL-15.

In some embodiments, priming first expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 IU/mL of IL-21, or about 0.5 IU/mL of IL-21. In some embodiments, the priming first expansion culture media comprises about 20 IU/mL of IL-21 to about 0.5 IU/mL of IL-21. In some embodiments, the priming first expansion culture media comprises about 15 IU/mL of IL-21 to about 0.5 IU/mL of IL-21.

In some embodiments, the priming first expansion culture media comprises about 12 IU/mL of IL-21 to about 0.5 IU/mL of IL-21. In some embodiments, the priming first expansion culture media comprises about 10 IU/mL of IL-21 to about 0.5 IU/mL of IL-21. In some embodiments, the priming first expansion culture media comprises about 5 IU/mL of IL-21 to about 1 IU/mL of IL-21. In some embodiments, the priming first expansion culture media comprises about 2 IU/mL of IL-21. In some embodiments, the priming first expansion cell culture medium comprises about 1 IU/mL of IL-21. In some embodiments, the priming first expansion cell culture medium comprises about 0.5 IU/mL of IL-21. In an embodiment, the cell culture medium further comprises IL-21. In a preferred embodiment, the priming first expansion cell culture medium comprises about 1 IU/mL of IL-21.

In an embodiment, the priming first expansion cell culture medium comprises OKT-3 antibody. In some embodiments, the priming first expansion cell culture medium comprises about 30 ng/mL of OKT-3 antibody. In an embodiment, the priming first expansion cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 μg/mL of OKT-3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT-3 antibody. In an embodiment, the cell culture medium comprises between 15 ng/ml and 30 ng/mL of OKT-3 antibody. In an embodiment, the cell culture medium comprises 30 ng/mL of OKT-3 antibody. In some embodiments, the OKT-3 antibody is muromonab.

TABLE 18: Amino acid sequences of muromonab (exemplary OKT-3 antibody)

| Identifier | Sequence (One-Letter Amino Acid Symbols) |
|---|---|
| SEQ ID NO:1 Muromonab heavy chain | QVQLQQSGAE LARPGASVKM SCKASGYTFT RYTMHWVKQR PGQGLEWIGY INPSRGYTNY 60<br>NQKFKDKATL TTDKSSSTAY MQLSSLTSED SAVYYCARYY DDHYCLDYWG QGTTLTVSSA 120<br>KTTAPSVYPL APVCGGTTGS SVTLGCLVKG YFPEPVTLTW NSGSLSSGVH TFPAVLQSDL 180<br>YTLSSSVTVT SSTWPSQSIT CNVAHPASST KVDKKIEPRP KSCDKTHTCP PCPAPELLGG 240<br>PSVFLFPPKP KDTLMISRTP EVTCVVVDVS HEDPEVKFNW YVDGVEVHNA KTKPREEQYN 300<br>STYRVVSVLT VLHQDWLNGK EYKCKVSNKA LPAPIEKTIS KAKGQPREPQ VYTLPPSRDE 360<br>LTKNQVSLTC LVKGFYPSDI AVEWESNGQP ENNYKTTPPV LDSDGSFFLY SKLTVDKSRW 420<br>QQGNVFSCSV MHEALHNHYT QKSLSLSPGK 450 |
| SEQ ID NO:2 Muromonab light chain | QIVLTQSPAI MSASPGEKVT MTCSASSSVS YMNWYQQKSG TSPKRWIYDT SKLASGVPAH 60<br>FRGSGSGTSY SLTISGMEAE DAATYYCQQW SSNPFTFGSG TKLEINRADT APTVSIFPPS 120<br>SEQLTSGGAS VVCFLNNFYP KDINVKWKID GSERQNGVLN SWTDQDSKDS TYSMSSTLTL 180<br>TKDEYERHNS YTCEATHKTS TSPIVKSFNR NEC 213 |

In some embodiments, the priming first expansion cell culture medium comprises one or more TNFRSF agonists in a cell culture medium. In some embodiments, the TNFRSF agonist comprises a 4-1BB agonist. In some embodiments, the TNFRSF agonist is a 4-1BB agonist, and the 4-1BB agonist is selected from the group consisting of urelumab, utomilumab, EU-101, a fusion protein, and fragments, derivatives, variants, biosimilars, and combinations thereof. In some embodiments, the TNFRSF agonist is added at a concentration sufficient to achieve a concentration in the cell culture medium of between 0.1 μg/mL and 100 μg/mL. In some embodiments, the TNFRSF agonist is added at a concentration sufficient to achieve a concentration in the cell culture medium of between 20 μg/mL and 40 μg/mL.

In some embodiments, in addition to one or more TNFRSF agonists, the priming first expansion cell culture medium further comprises IL-2 at an initial concentration of about 3000 IU/mL and OKT-3 antibody at an initial concentration of about 30 ng/mL, and wherein the one or more TNFRSF agonists comprises a 4-1BB agonist. In some embodiments, in addition to one or more TNFRSF agonists, the priming first expansion cell culture medium further comprises IL-2 at an initial concentration of about 6000 IU/mL and OKT-3 antibody at an initial concentration of about 30 ng/mL, and wherein the one or more TNFRSF agonists comprises a 4-1BB agonist.

In some embodiments, the priming first expansion culture medium is referred to as "CM", an abbreviation for culture media. In some embodiments, it is referred to as CM1 (culture medium 1). In some embodiments, CM consists of RPMI 1640 with GlutaMAX, supplemented with 10% human AB serum, 25 mM Hepes, and 10 μg/mL gentamicin. In some embodiments, the CM is the CM1 described in the Examples, *see,* Examples 1 and 14. In some embodiments, the priming first expansion occurs in an initial cell culture medium or a first cell culture medium. In some embodiments, the priming first expansion culture medium or the initial cell culture medium or the first cell culture medium comprises IL-2, OKT-3 and antigen-presenting feeder cells (also referred to herein as feeder cells).

Also disclosed herein but not claimed, the priming first expansion (including processes such as for example those described in Step B of Figure 85 (in particular, *e.g.,* Figure 85B), which can include those sometimes referred to as the pre-REP or priming REP) process is 1 to 7 days, as discussed in the examples and figures. Also disclosed herein but not claimed, the priming first expansion (including processes such as for example those described in Step B of Figure 85 (in particular, *e.g.*, Figure 85B), which can include those sometimes referred to as the pre-REP or priming REP) process is 2 to 7 days. In some embodiments, the priming first expansion (including processes such as for example those described in Step B of Figure 85 (in particular, *e.g.*, Figure 85B), which can include those sometimes referred to as the pre-REP or priming REP) process is 3 to 7 days. In some embodiments, the priming first expansion (including processes such as for example those described in Step B of Figure 85 (in particular, *e.g.*, Figure 85B), which can include those sometimes referred to as the pre-REP or priming REP) process is 4 to 7 days. In some embodiments, the priming first expansion (including processes such as for example those described in Step B of Figure 85 (in particular, *e.g.*, Figure 85B), which can include those sometimes referred to as the pre-REP or priming REP) process is 5 to 7 days. In some embodiments, the priming first expansion (including processes such as for example those described in Step B of Figure 85 (in particular, *e.g.*, Figure 85B), which can include those sometimes referred to as the pre-REP or priming REP) process is 6 to 7 days. In some embodiments, the priming first expansion (including processes such as for example those provided in Step B of Figure 85 (in particular, *e.g.*, Figure 85B), which can include those sometimes referred to as the pre-REP or priming REP) process is 7 days.

Also disclosed herein but not claimed, the priming first TIL expansion can proceed for 1 days to 7 days from when tumor cores or fragments are added to the cell culture medium and/or when the first priming expansion step is initiated. Also disclosed herein but not claimed, the priming first TIL expansion can proceed for 2 days to 7 days from when tumor cores or fragments are added to the cell culture medium and/or when the first priming expansion step is initiated. In some embodiments, the priming first TIL expansion can proceed for 3 days to 7 days from when tumor cores or fragments are added to the cell culture medium and/or when the first priming expansion step is initiated. In some embodiments, the priming first TIL expansion can proceed for 4 days to 7 days from when tumor cores or fragments are added to the cell culture medium and/or when the first priming expansion step is initiated. In some embodiments, the priming first TIL expansion can proceed for 5 days to 7 days from when tumor cores or fragments are added to the cell culture medium and/or when the first priming expansion step is initiated. In some embodiments, the priming first TIL expansion can proceed for 6 days to 7 days from when tumor cores or fragments are added to the cell culture medium and/or when the first priming expansion step is initiated. In some embodiments, the priming first TIL expansion can proceed for 7 days from when tumor cores or fragments are added to the cell culture medium and/or when the first priming expansion step is initiated. Also disclosed herein but not claimed, the priming first TIL expansion can proceed for 1 days to 10 days from when tumor cores or fragments are added to the cell culture medium and/or when the first priming expansion step is initiated. Also disclosed herein but not claimed, the priming first TIL expansion can proceed for 2 days to 10 days from when tumor cores or fragments are added to the cell culture medium and/or when the first priming expansion step is initiated. In some embodiments, the priming first TIL expansion can proceed for 3 days to 10 days from when tumor cores or fragments are added to the cell culture medium and/or when the first priming expansion step is initiated. In some embodiments, the priming first TIL expansion can proceed for 4 days to 7 days from when tumor cores or fragments are added to the cell culture medium and/or when the first priming expansion step is initiated. In some embodiments, the priming first TIL expansion can proceed for 5 days to 10 days from when tumor cores or fragments are added to the cell culture medium and/or when the first priming expansion step is initiated. In some embodiments, the priming first TIL expansion can proceed for 6 days to 10 days from when tumor cores or fragments are added to the cell culture medium and/or when the first priming expansion step is initiated. In some embodiments, the priming first TIL expansion can proceed for 8 days from when tumor cores or fragments are added to the cell culture medium and/or when the first priming expansion step is initiated. In some embodiments, the priming first TIL expansion can proceed for 9 days from when tumor cores or fragments are added to the cell culture medium and/or when the first priming expansion step is initiated. In some embodiments, the priming first TIL expansion can proceed for 10 days from when tumor cores or fragments are added to the cell culture medium and/or when the first priming expansion step is initiated. In some embodiments, the small biopsy (*e.g.,* the core biopsy) is removed from the culture at about day 1, 2, or 3. In some embodiments, the small biopsy (*e.g.,* the core biopsy) is removed from the culture at day 3.

Also disclosed herein but not claimed, the priming first TIL expansion can proceed for 8 days to 17 days from when tumor cores or fragments are added to the cell culture and/or when the first priming expansion step is initiated. Also disclosed herein but not claimed, the priming first TIL expansion can proceed for 9 days to 17 days from when tumor cores or fragments are added to the cell culture and/or when the first priming expansion step is initiated. Also disclosed herein but not claimed, the priming first TIL expansion can proceed for 10 days to 17 days from when tumor cores or fragments are added to the cell culture and/or when the first priming expansion step is initiated. Also disclosed herein but not claimed, the priming first TIL expansion can proceed for 11 days to 17 days from when tumor cores or fragments are added to the cell culture and/or when the first priming expansion step is initiated. Also disclosed herein but not claimed, the priming first TIL expansion can proceed for 12 days to 17 days from when tumor cores or fragments are added to the cell culture and/or when the first priming expansion step is initiated. Also disclosed herein but not claimed, the priming first TIL expansion can proceed for 13 days to 17 days from when tumor cores or fragments are added to the cell culture and/or when the first priming expansion step is initiated. Also disclosed herein but not claimed, the priming first TIL expansion can proceed for 14 days to 17 days from when tumor cores or fragments are added to the cell culture and/or when the first priming expansion step is initiated. Also disclosed herein but not claimed, the priming first TIL expansion can proceed for 15 days to 17 days from when tumor cores or fragments are added to the cell culture and/or when the first priming expansion step is initiated. Also disclosed herein but not claimed, the priming first TIL expansion can proceed for 16 days to 17 days from when tumor cores or fragments are added to the cell culture and/or when the first priming expansion step is initiated.

In some embodiments, the priming first expansion of the TILs can proceed for 3 days, 4 days, 5 days, 6 days, 7 days, 8 days, 9, days, or 10 days. Also disclosed herein but not claimed, the first TIL expansion can proceed for 1 day to 7 days. Also disclosed herein but not claimed, the first TIL expansion can proceed for 2 days to 7 days. In some embodiments, the first TIL expansion can proceed for 3 days to 7 days. In some embodiments, the first TIL expansion can proceed for 4 days to 7 days. In some embodiments, the first TIL expansion can proceed for 5 days to 7 days. In some embodiments, the first TIL expansion can proceed for 6 days to 7 days. Also disclosed herein but not claimed, the first TIL expansion can proceed for 1 day to 10 days. Also disclosed herein but not claimed, the first TIL expansion can proceed for 2 days to 10 days. In some embodiments, the first TIL expansion can proceed for 3 days to 10 days. In some embodiments, the first TIL expansion can proceed for 4 days to 10 days. In some embodiments, the first TIL expansion can proceed for 5 days to 10 days. In some embodiments, the first TIL expansion can proceed for 6 days to 10 days. In some embodiments, the first TIL expansion can proceed for 7 days to 10 days. In some embodiments, the first TIL expansion can proceed for 8 days to 10 days. In some embodiments, the first TIL expansion can proceed for 9 days to 10 days. In some embodiments, the first TIL expansion can proceed for 7 days. In some embodiments, the first TIL expansion can proceed for 8 days. In some embodiments, the first TIL expansion can proceed for 9 days. In some embodiments, the first TIL expansion can proceed for 10 days.

In some embodiments, the priming first expansion of the TILs can proceed for 8 days, 9 days, 10 days, 11 days, or 12 days. The first TIL expansion can proceed for 8 days to 17 days. The first TIL expansion can proceed for 9 days to 17 days. The first TIL expansion can proceed for 10 days to 17 days. The first TIL expansion can proceed for 11 days to 17 days. The first TIL expansion can proceed for 12 days to 17 days. The first TIL expansion can proceed for 13 days to 17 days. The first TIL expansion can proceed for 14 days to 17 days. The first TIL expansion can proceed for 15 days to 17 days. The first TIL expansion can proceed for 16 days to 17 days. The first TIL expansion can proceed for 17 days.

In some embodiments, a combination of IL-2, IL-7, IL-15, and/or IL-21 are employed as a combination during the priming first expansion. In some embodiments, IL-2, IL-7, IL-15, and/or IL-21 as well as any combinations thereof can be included during the priming first expansion, including, for example during Step B processes according to Figure 85 (in particular, e.g., Figure 85B), as well as described herein. In some embodiments, a combination of IL-2, IL-15, and IL-21 are employed as a combination during the priming first expansion. In some embodiments, IL-2, IL-15, and IL-21 as well as any combinations thereof can be included during Step B processes according to Figure 85 (in particular, e.g., Figure 85B) and as described herein.

In some embodiments, the priming first expansion, for example, Step B according to Figure 85 (in particular, e.g., Figure 85B), is performed in a closed system bioreactor. In some embodiments, a closed system is employed for the TIL expansion, as described herein. In some embodiments, a bioreactor is employed. In some embodiments, a bioreactor is employed as the container. In some embodiments, the bioreactor employed is for example a G-REX-10 or a G-REX-100. In some embodiments, the bioreactor employed is a G-REX-100. In some embodiments, the bioreactor employed is a G-REX-10.

1. Feeder Cells and Antigen Presenting Cells

In an embodiment, the priming first expansion procedures described herein (for example including expansion such as those described in Step B from Figure 85 (in particular, *e.g.,* Figure 85B), as well as those referred to as pre-REP or priming REP) require feeder cells (also referred to herein as "antigen-presenting cells") at the initiation of the TIL expansion and during the priming first expansion. In many embodiments, the feeder cells are peripheral blood mononuclear cells (PBMCs) obtained from standard whole blood units from allogeneic healthy blood donors. The PBMCs are obtained using standard methods such as Ficoll-Paque gradient separation. In some embodiments, $2.5 \times 10^8$ feeder cells are used during the priming first expansion. In some embodiments, $2.5 \times 10^8$ feeder cells per container are used during the priming first expansion. In some embodiments, $2.5 \times 10^8$ feeder cells per GREX-10 are used during the priming first expansion. In some embodiments, $2.5 \times 10^8$ feeder cells per GREX-100 are used during the priming first expansion.

In general, the allogenic PBMCs are inactivated, either via irradiation or heat treatment, and used in the REP procedures, as described in the examples, which provides an exemplary protocol for evaluating the replication incompetence of irradiate allogeneic PBMCs.

In some embodiments, PBMCs are considered replication incompetent and acceptable for use in the TIL expansion procedures described herein if the total number of viable cells on day 14 is less than the initial viable cell number put into culture on day 0 of the priming first expansion.

In some embodiments, PBMCs are considered replication incompetent and acceptable for use in the TIL expansion procedures described herein if the total number of viable cells, cultured in the presence of OKT3 and IL-2, on day 7 have not increased from the initial viable cell number put into culture on day 0 of the priming first expansion. In some embodiments, the PBMCs are cultured in the presence of 30 ng/mL OKT3 antibody and 3000 IU/mL IL-2. In some embodiments, the PBMCs are cultured in the presence of 30 ng/mL OKT3 antibody and 6000 IU/mL IL-2.

In some embodiments, PBMCs are considered replication incompetent and acceptable for use in the TIL expansion procedures described herein if the total number of viable cells, cultured in the presence of OKT3 and IL-2, on day 7 have not increased from the initial viable cell number put into culture on day 0 of the priming first expansion. In some embodiments, the PBMCs are cultured in the presence of 5-60 ng/mL OKT3 antibody and 1000-6000 IU/mL IL-2. In some embodiments, the PBMCs are cultured in the presence of 10-50 ng/mL OKT3 antibody and 2000-5000 IU/mL IL-2. In some embodiments, the PBMCs are cultured in the presence of 20-40 ng/mL OKT3 antibody and 2000-4000 IU/mL IL-2. In some embodiments, the PBMCs are cultured in the presence of 25-35 ng/mL OKT3 antibody and 2500-3500 IU/mL IL-2. In some embodiments, the PBMCs are cultured in the presence of 30 ng/mL OKT3 antibody and 6000 IU/mL IL-2. In some embodiments, the PBMCs are cultured in the presence of 15 ng/mL OKT3 antibody and 3000 IU/mL IL-2. In some embodiments, the PBMCs are cultured in the presence of 15 ng/mL OKT3 antibody and 6000 IU/mL IL-2.

In some embodiments, the antigen-presenting feeder cells are PBMCs. In some embodiments, the antigen-presenting feeder cells are artificial antigen-presenting feeder cells. In an embodiment, the ratio of TILs to antigen-presenting feeder cells in the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to antigen-presenting feeder cells in the second expansion is between 1 to 50 and 1 to 300. In an embodiment, the ratio of TILs to antigen-presenting feeder cells in the second expansion is between 1 to 100 and 1 to 200.

In an embodiment, the priming first expansion procedures described herein require a ratio of about $2.5 \times 10^8$ feeder cells to about $100 \times 10^6$ TILs. In another embodiment, the priming first expansion procedures described herein require a ratio of about $2.5 \times 10^8$ feeder cells to about $50 \times 10^6$ TILs. In yet another embodiment, the priming first expansion described herein require about $2.5 \times 10^8$ feeder cells to about $25 \times 10^6$ TILs. In yet another embodiment, the priming first expansion described herein require about $2.5 \times 10^8$ feeder cells. In yet another embodiment, the priming first expansion requires one-fourth, one-third, five-twelfths, or one-half of the number of feeder cells used in the rapid second expansion.

In some embodiments, the media in the priming first expansion comprises IL-2. In some embodiments, the media in the priming first expansion comprises 6000 IU/mL of IL-2. In some embodiments, the media in the priming first expansion comprises antigen-presenting feeder cells. In some embodiments, the media in the priming first expansion comprises $2.5 \times 10^8$ antigen-presenting feeder cells per container. In some embodiments, the media in the priming first expansion comprises OKT-3. In some embodiments, the media comprises 30 ng of OKT-3 per container. In some embodiments, the container is a GREX100 MCS flask. In some embodiments, the media comprises 6000 IU/mL of IL-2, 30 ng/mL of OKT-3, and $2.5 \times 10^8$ antigen-presenting feeder cells. In some embodiments, the media comprises 6000 IU/mL of IL-2, 30 ng/mL of OKT-3, and $2.5 \times 10^8$ antigen-presenting feeder cells per container. In some embodiments, the media comprises 500 mL of culture medium and 15 ug of OKT-3 per $2.5 \times 10^8$ antigen-presenting feeder cells per container. In some embodiments, the media comprises 500 mL of culture medium and 15 ug of OKT-3 per container. In some embodiments, the container is a GREX100 MCS flask. In some embodiments, the media comprises 500 mL of culture medium and 6000 IU/mL of IL-2, 30 ng/mL of OKT-3, and $2.5 \times 10^8$ antigen-presenting feeder cells. In some embodiments, the media comprises 500 mL of culture medium and 6000 IU/mL of IL-2, 15 ug of OKT-3, and $2.5 \times 10^8$ antigen-presenting feeder cells per container. In some embodiments, the media comprises 500 mL of culture medium and 15 ug of OKT-3 per $2.5 \times 10^8$ antigen-presenting feeder cells per container.

In an embodiment, the priming first expansion procedures described herein require an excess of feeder cells over TILs during the second expansion. In many embodiments, the feeder cells are peripheral blood mononuclear cells (PBMCs) obtained from standard whole blood units from allogeneic healthy blood donors. The PBMCs are obtained using standard methods such as Ficoll-Paque gradient separation. In an embodiment, artificial antigen-presenting (aAPC) cells are used in place of PBMCs.

In general, the allogenic PBMCs are inactivated, either via irradiation or heat treatment, and used in the TIL expansion procedures described herein, including the exemplary procedures described in the figures and examples.

In an embodiment, artificial antigen presenting cells are used in the priming first expansion as a replacement for, or in combination with, PBMCs.

2. Cytokines

The expansion methods described herein generally use culture media with high doses of a cytokine, in particular IL-2, as is known in the art.

Alternatively, using combinations of cytokines for the priming first expansion of TILs is additionally possible, with combinations of two or more of IL-2, IL-15 and IL-21 as is generally outlined in International Publication No. WO 2015/189356 and WO 2015/189357. Thus, possible combinations include IL-2 and IL-15, IL-2 and IL-21, IL-15 and IL-21, and IL-2, IL-15 and IL-21, with the latter finding particular use in many embodiments. The use of combinations of cytokines specifically favors the generation of lymphocytes, and in particular T-cells as described therein.

TABLE 19: Amino acid sequences of interleukins.

| Identifier | Sequence (One-Letter Amino Acid Symbols) |
|---|---|
| SEQ ID NO:3 recombinant human IL-2 (rhIL-2) | MAPTSSSTKK TQLQLEHLLL DLQMILNGIN NYKNPKLTRM LTFKFYMPKK ATELKHLQCL 60<br>EEELKPLEEV LNLAQSKNFH LRPRDLISNI NVIVLELKGS ETTFMCEYAD ETATIVEFLN 120<br>RWITFCQSII STLT 134 |
| SEQ ID NO:4 Aldesleukin | PTSSSTKKTQ LQLEHLLLDL QMILNGINNY KNPKLTRMLT FKFYMPKKAT ELKHLQCLEE 60<br>ELKPLEEVLN LAQSKNFHLR PRDLISNINV IVLELKGSET TFMCEYADET ATIVEFLNRW 120<br>ITFSQSIIST LT 132 |
| SEQ ID NO:5 recombinant human IL-4 (rhIL-4) | MHKCDITLQE IIKTLNSLTE QKTLCTELTV TDIFAASKNT TEKETFCRAA TVLRQFYSHH 60<br>EKDTRCLGAT AQQFHRHKQL IRFLKRLDRN LWGLAGLNSC PVKEANQSTL ENFLERLKTI 120<br>MREKYSKCSS 130 |
| SEQ ID NO:6 recombinant human IL-7 (rhIL-7) | MDCDIEGKDG KQYESVLMVS IDQLLDSMKE IGSNCLNNEF NFFKRHICDA NKEGMFLFRA 60<br>ARKLRQFLKM NSTGDFDLHL LKVSEGTTIL LNCTGQVKGR KPAALGEAQP TKSLEENKSL 120<br>KEQKKLNDLC FLKRLLQEIK TCWNKILMGT KEH 153 |
| SEQ ID NO:7 recombinant human IL-15 (rhIL-15) | MNWVNVISDL KKIEDLIQSM HIDATLYTES DVHPSCKVTA MKCFLLELQV ISLESGDASI 60<br>HDTVENLIIL ANNSLSSNGN VTESGCKECE ELEEKNIKEF LQSFVHIVQM FINTS 115 |
| SEQ ID NO:8 recombinant human IL-21 (rhIL-21) | MQDRHMIRMR QLIDIVDQLK NYVNDLVPEF LPAPEDVETN CEWSAFSCFQ KAQLKSANTG 60<br>NNERIINVSI KKLKRKPPST NAGRRQKHRL TCPSCDSYEK KPPKEFLERF KSLLQKMIHQ 120<br>HLSSRTHGSE DS 132 |

**C. STEP C: Priming First Expansion to Rapid Second Expansion Transition**

In some cases, the bulk TIL population obtained from the priming first expansion (which can include expansions sometimes referred to as pre-REP), including, for example the TIL population obtained from for example, Step B as indicated in Figure 85 (in particular, *e.g.,* Figure 85B), can be subjected to a rapid second expansion (which can include expansions sometimes referred to as Rapid Expansion Protocol (REP)) and then cryopreserved as discussed below. Similarly, in the case where genetically modified TILs will be used in therapy, the expanded TIL population from the priming first expansion or the expanded TIL population from the rapid second expansion can be subjected to genetic modifications for suitable treatments prior to the expansion step or after the priming first expansion and prior to the rapid second expansion.

In some embodiments, the TILs obtained from the priming first expansion (for example, from Step B as indicated in Figure 85 (in particular, e.g., Figure 85B)) are stored until phenotyped for selection. In some embodiments, the TILs obtained from the priming first expansion (for example, from Step B as indicated in Figure 85 (in particular, e.g., Figure 85B)) are not stored and proceed directly to the rapid second expansion. In some embodiments, the TILs obtained from the priming first expansion are not cryopreserved after the priming first expansion and prior to the rapid second expansion. In some embodiments, the transition from the priming first expansion to the second expansion occurs at about 2 days, 3 days, 4, days, 5 days, 6 days, or 7 days, from when tumor cores or fragments are added to the cell culture medium and/or when the first priming expansion step is initiated. In some embodiments, the transition from the priming first expansion to the rapid second expansion occurs at about 3 days to 7 days from when cores or fragments are added to the cell culture medium and/or when the first priming expansion step is initiated. In some embodiments, the transition from the priming first expansion to the second expansion occurs at about 4 days to 7 days from when cores or fragments are added to the cell culture medium and/or when the first priming expansion step is initiated. In some embodiments, the transition from the priming first expansion to the second expansion occurs at about 5 days to 7 days from when cores or fragments are added to the cell culture medium and/or when the first priming expansion step is initiated. In some embodiments, the transition from the priming first expansion to the second expansion occurs at about 6 days to 7 days from when cores or fragments are added to the cell culture medium and/or when the first priming expansion step is initiated. In some embodiments, the transition from the priming first expansion to the second expansion occurs at about 7 days from when cores or fragments are added to the cell culture medium and/or when the first priming expansion step is initiated.

In some embodiments, the transition from the priming first expansion to the rapid second expansion occurs at 1 day, 2 days, 3 days, 4 days, 5 days, 6 days, or 7 days, up to 10 days from when cores or fragments are added to the cell culture medium and/or when the first priming expansion step is initiated. In some embodiments, the transition from the priming first expansion to the rapid second expansion occurs 1 day to 7 days from when cores or fragments are added to the cell culture medium and/or when the first priming expansion step is initiated. In some embodiments, the transition from the priming first expansion to the second expansion occurs 2 days to 7 days from when cores or fragments are added to the cell culture medium and/or when the first priming expansion step is initiated. In some embodiments, the transition from the priming first expansion to the second expansion occurs 3 days to 7 days from when cores or fragments are added to the cell culture medium and/or when the first priming expansion step is initiated. In some embodiments, the transition from the priming first expansion to the rapid second expansion occurs 4 days to 7 days from when cores or fragments are added to the cell culture medium and/or when the first priming expansion step is initiated. In some embodiments, the transition from the priming first expansion to the rapid second expansion occurs 5 days to 7 days from when cores or fragments are added to the cell culture medium and/or when the first priming expansion step is initiated. In some embodiments, the transition from the priming first expansion to the rapid second expansion occurs 6 days to 7 days from when cores or fragments are added to the cell culture medium and/or when the first priming expansion step is initiated. In some embodiments, the transition from the priming first expansion to the rapid second expansion occurs 7 days from when cores or fragments are added to the cell culture medium and/or when the first priming expansion step is initiated.

In some embodiments, the TILs are not stored after the primary first expansion and prior to the rapid second expansion, and the TILs proceed directly to the rapid second expansion (for example, in some embodiments, there is no storage during the transition from Step B to Step D as shown in Figure 85 (in particular, e.g., Figure 85B)). In some embodiments, the transition occurs in closed system, as described herein. In some embodiments, the TILs from the priming first expansion, the second population of TILs, proceeds directly into the rapid second expansion with no transition period.

In some embodiments, the transition from the priming first expansion to the rapid second expansion, for example, Step C according to Figure 85 (in particular, e.g., Figure 85B), is performed in a closed system bioreactor. In some embodiments, a closed system is employed for the TIL expansion, as described herein. In some embodiments, a single bioreactor is employed. In some embodiments, the single bioreactor employed is for example a GREX-10 or a GREX-100. In some embodiments, the closed system bioreactor is a single bioreactor. In some embodiments, the transition from the priming first expansion to the rapid second expansion involves a scale-up in container size. In some embodiments, the priming first expansion is performed in a smaller container than the rapid second expansion. In some embodiments, the priming first expansion is performed in a GREX-100 and the rapid second expansion is performed in a GREX-500.

### D. STEP D: Rapid Second Expansion

In some embodiments, the TIL cell population is further expanded in number after harvest and the priming first expansion, after Step A and Step B, and the transition referred to as Step C, as indicated in Figure 85 (in particular, e.g., Figure 85B)). This further expansion is referred to herein as the rapid second expansion, which can include expansion processes generally referred to in the art as a rapid expansion process (Rapid Expansion Protocol or REP; as well as processes as indicated in Step D of Figure 85 (in particular, e.g., Figure 85B)). The rapid second expansion is generally accomplished using a culture media comprising a number of components, including feeder cells, a cytokine source, and an anti-CD3 antibody, in a gas-permeable container. In some embodiments, 1 day, 2 days, 3 days, or 4 days after initiation of the rapid second expansion *(i.e.,* at days 8, 9, 10, or 11 of the overall Gen 3 process), the TILs are transferred to a larger volume container.

In some embodiments, the rapid second expansion (which can include expansions sometimes referred to as REP; as well as processes as indicated in Step D of Figure 85 (in particular, e.g., Figure 85B)) of TIL can be performed using any TIL flasks or containers known by those of skill in the art. In some embodiments, the second TIL expansion can proceed for 3 days, 4, days, 5 days, 6 days, 7 days, 8 days, or 9 days after initiation of the rapid second expansion. Also disclosed herein but not claimed, the second TIL expansion can proceed for about 1 days to about 9 days after initiation of the rapid second expansion. Also disclosed herein but not claimed, the second TIL expansion can proceed for about 2 days to about 9 days after initiation of the rapid second expansion. In some embodiments, the second TIL expansion can proceed for about 3 days to about 9 days after initiation of the rapid second expansion. In some embodiments, the second TIL expansion can proceed for about 4 days to about 9 days after initiation of the rapid second expansion. In some embodiments, the second TIL expansion can proceed for about 5 days to about 9 days after initiation of the rapid second expansion. In some embodiments, the second TIL expansion can proceed for about 6 days to about 9 days after initiation of the rapid second expansion. In some embodiments, the second TIL expansion can proceed for about 7 days to about 9 days after initiation of the rapid second expansion. In some embodiments, the second TIL expansion can proceed for about 7 days to about 10 days after initiation of the rapid second expansion. In some embodiments, the second TIL expansion can proceed for about 1 day after initiation of the rapid second expansion. Also disclosed herein but not claimed, the second TIL expansion can proceed for about 2 days after initiation of the rapid second expansion. In some embodiments, the second TIL expansion can proceed for about 3 days after initiation of the rapid second expansion. In some embodiments, the second TIL expansion can proceed for about 4 days after initiation of the rapid second expansion. In some embodiments, the second TIL expansion can proceed for about 5 days after initiation of the rapid second expansion. In some embodiments, the second TIL expansion can proceed for about 6 days after initiation of the rapid second expansion. In some embodiments, the second TIL expansion can proceed for about 7 days after initiation of the rapid second expansion. In some embodiments, the second TIL expansion can proceed for about 8 days after initiation of the rapid second expansion. In some embodiments, the second TIL expansion can proceed for about 9 days after initiation of the rapid second expansion. In some embodiments, the second TIL expansion can proceed for about 10 days after initiation of the rapid second expansion.

In an embodiment, the rapid second expansion can be performed in a gas permeable container using the methods of the present disclosure (including, for example, expansions referred to as REP; as well as processes as indicated in Step D of Figure 85 (in particular, *e.g.,* Figure 85B)). In some embodiments, the TILs are expanded in the rapid second expansion in the presence of IL-2, OKT-3, and feeder cells (also referred herein as "antigen-presenting cells"). In some embodiments, the TILs are expanded in the rapid second expansion in the presence of IL-2, OKT-3, and feeder cells, wherein the feeder cells are added to a final concentration that is twice, 2.4 times, 2.5 times, 3 times, 3.5 times or 4 times the concentration of feeder cells present in the priming first expansion. For example, TILs can be rapidly expanded using non-specific T-cell receptor stimulation in the presence of interleukin-2 (IL-2) or interleukin-15 (IL-15). The non-specific T-cell receptor stimulus can include, for example, an anti-CD3 antibody, such as about 30 ng/mL of OKT3, a mouse monoclonal anti-CD3 antibody (commercially available from Ortho-McNeil, Raritan, NJ or Miltenyi Biotech, Auburn, CA) or UHCT-1 (commercially available from BioLegend, San Diego, CA, USA). TILs can be expanded to induce further stimulation of the TILs *in vitro* by including one or more antigens during the second expansion, including antigenic portions thereof, such as epitope(s), of the cancer, which can be optionally expressed from a vector, such as a human leukocyte antigen A2 (HLA-A2) binding peptide, *e.g.,* 0.3 μM MART-1 :26-35 (27 L) or gpl 00:209-217 (210M), optionally in the presence of a T-cell growth factor, such as 300 IU/mL IL-2 or IL-15. Other suitable antigens may include, e.g., NY-ESO-1, TRP-1, TRP-2, tyrosinase cancer antigen, MAGE-A3, SSX-2, and VEGFR2, or antigenic portions thereof. TIL may also be rapidly expanded by re-stimulation with the same antigen(s) of the cancer pulsed onto HLA-A2-expressing antigen-presenting cells. Alternatively, the TILs can be further re-stimulated with, e.g., example, irradiated, autologous lymphocytes or with irradiated HLA-A2+ allogeneic lymphocytes and IL-2. In some embodiments, the re-stimulation occurs as part of the second expansion. In some embodiments, the second expansion occurs in the presence of irradiated, autologous lymphocytes or with irradiated HLA-A2+ allogeneic lymphocytes and IL-2.

In an embodiment, the cell culture medium further comprises IL-2. In some embodiments, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or between 8000 IU/mL of IL-2.

In an embodiment, the cell culture medium comprises OKT-3 antibody. In some embodiments, the cell culture medium comprises about 30 ng/mL of OKT-3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 μg/mL of OKT-3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT-3 antibody. In an embodiment, the cell culture medium comprises between 30 ng/ml and 60 ng/mL of OKT-3 antibody. In an embodiment, the cell culture medium comprises about 30 ng/mL OKT-3. In an embodiment, the cell culture medium comprises about 60 ng/mL OKT-3. In some embodiments, the OKT-3 antibody is muromonab.

In some embodiments, the media in the rapid second expansion comprises IL-2. In some embodiments, the media comprises 6000 IU/mL of IL-2. In some embodiments, the media in the rapid second expansion comprises antigen-presenting feeder cells. In some embodiments, the media in the rapid second expansion comprises 7.5 × $10^8$ antigen-presenting feeder cells per container. In some embodiments, the media in the rapid second expansion comprises OKT-3. In some embodiments, the in the rapid second expansion media comprises 500 mL of culture medium and 30 ug of OKT-3 per container. In some embodiments, the container is a GREX100 MCS flask. In some embodiments, the in the rapid second expansion media comprises 6000 IU/mL of IL-2, 60 ng/mL of OKT-3, and 7.5 × $10^8$ antigen-presenting feeder cells. In some embodiments, the media comprises 500 mL of culture medium and 6000 IU/mL of IL-2, 30 ug of OKT-3, and 7.5 × $10^8$ antigen-presenting feeder cells per container.

In some embodiments, the media in the rapid second expansion comprises IL-2. In some embodiments, the media comprises 6000 IU/mL of IL-2. In some embodiments, the media in the rapid second expansion comprises antigen-presenting feeder cells. In some embodiments, the media comprises between 5 × $10^8$ and 7.5 × $10^8$antigen-presenting feeder cells per container. In some embodiments, the media in the rapid second expansion comprises OKT-3. In some embodiments, the media in the rapid second expansion comprises 500 mL of culture medium and 30 μg of OKT-3 per container. In some embodiments, the container is a GREX100 MCS flask. In some embodiments, the media in the rapid second expansion comprises 6000 IU/mL of IL-2, 60 ng/mL of OKT-3, and between 5 × $10^8$ and 7.5 × $10^8$ antigen-presenting feeder cells. In some embodiments, the media in the rapid second expansion comprises 500 mL of culture medium and 6000 IU/mL of IL-2, 30 μg of OKT-3, and between 5 × $10^8$ and 7.5 × $10^8$ antigen-presenting feeder cells per container.

In some embodiments, the cell culture medium comprises one or more TNFRSF agonists in a cell culture medium. In some embodiments, the TNFRSF agonist comprises a 4-1BB agonist. In some embodiments, the TNFRSF agonist is a 4-1BB agonist, and the 4-1BB agonist is selected from the group consisting of urelumab, utomilumab, EU-101, a fusion protein, and fragments, derivatives, variants, biosimilars, and combinations thereof. In some embodiments, the TNFRSF agonist is added at a concentration sufficient to achieve a concentration in the cell culture medium of between 0.1 μg/mL and 100 μg/mL. In some embodiments, the TNFRSF agonist is added at a concentration sufficient to achieve a concentration in the cell culture medium of between 20 μg/mL and 40 μg/mL.

In some embodiments, in addition to one or more TNFRSF agonists, the cell culture medium further comprises IL-2 at an initial concentration of about 3000 IU/mL and OKT-3 antibody at an initial concentration of about 30 ng/mL, and wherein the one or more TNFRSF agonists comprises a 4-1BB agonist.

In some embodiments, a combination of IL-2, IL-7, IL-15, and/or IL-21 are employed as a combination during the second expansion. In some embodiments, IL-2, IL-7, IL-15, and/or IL-21 as well as any combinations thereof can be included during the second expansion, including, for example during a Step D processes according to Figure 85 (in particular, e.g., Figure 85B), as well as described herein. In some embodiments, a combination of IL-2, IL-15, and IL-21 are employed as a combination during the second expansion. In some embodiments, IL-2, IL-15, and IL-21 as well as any combinations thereof can be included during Step D processes according to Figure 85 (in particular, e.g., Figure 85B) and as described herein.

In some embodiments, the second expansion can be conducted in a supplemented cell culture medium comprising IL-2, OKT-3, antigen-presenting feeder cells, and optionally a TNFRSF agonist. In some embodiments, the second expansion occurs in a supplemented cell culture medium. In some embodiments, the supplemented cell culture medium comprises IL-2, OKT-3, and antigen-presenting feeder cells. In some embodiments, the second cell culture medium comprises IL-2, OKT-3, and antigen-presenting cells (APCs; also referred to as antigen-presenting feeder cells). In some embodiments, the second expansion occurs in a cell culture medium comprising IL-2, OKT-3, and antigen-presenting feeder cells (*i.e.,* antigen presenting cells).

In some embodiments, the second expansion culture media comprises about 500 IU/mL of IL-15, about 400 IU/mL of IL-15, about 300 IU/mL of IL-15, about 200 IU/mL of IL-15, about 180 IU/mL of IL-15, about 160 IU/mL of IL-15, about 140 IU/mL of IL-15, about 120 IU/mL of IL-15, or about 100 IU/mL of IL-15. In some embodiments, the second expansion culture media comprises about 500 IU/mL of IL-15 to about 100 IU/mL of IL-15. In some embodiments, the second expansion culture media comprises about 400 IU/mL of IL-15 to about 100 IU/mL of IL-15. In some embodiments, the second expansion culture media comprises about 300 IU/mL of IL-15 to about 100 IU/mL of IL-15. In some embodiments, the second expansion culture media comprises about 200 IU/mL of IL-15. In some embodiments, the cell culture medium comprises about 180 IU/mL of IL-15. In an embodiment, the cell culture medium further comprises IL-15. In a preferred embodiment, the cell culture medium comprises about 180 IU/mL of IL-15.

In some embodiments, the second expansion culture media comprises about 20 IU/mL of IL-21, about 15 IU/mL of IL-21, about 12 IU/mL of IL-21, about 10 IU/mL of IL-21, about 5 IU/mL of IL-21, about 4 IU/mL of IL-21, about 3 IU/mL of IL-21, about 2 IU/mL of IL-21, about 1 IU/mL of IL-21, or about 0.5 IU/mL of IL-21. In some embodiments, the second expansion culture media comprises about 20 IU/mL of IL-21 to about 0.5 IU/mL of IL-21. In some embodiments, the second expansion culture media comprises about 15 IU/mL of IL-21 to about 0.5 IU/mL of IL-21. In some embodiments, the second expansion culture media comprises about 12 IU/mL of IL-21 to about 0.5 IU/mL of IL-21. In some embodiments, the second expansion culture media comprises about 10 IU/mL of IL-21 to about 0.5 IU/mL of IL-21. In some embodiments, the second expansion culture media comprises about 5 IU/mL of IL-21 to about 1 IU/mL of IL-21. In some embodiments, the second expansion culture media comprises about 2 IU/mL of IL-21. In some embodiments, the cell culture medium comprises about 1 IU/mL of IL-21. In some embodiments, the cell culture medium comprises about 0.5 IU/mL of IL-21. In an embodiment, the cell culture medium further comprises IL-21. In a preferred embodiment, the cell culture medium comprises about 1 IU/mL of IL-21.

In some embodiments the antigen-presenting feeder cells (APCs) are PBMCs. In an embodiment, the ratio of TILs to PBMCs and/or antigen-presenting cells in the rapid expansion and/or the second expansion is about 1 to 10, about 1 to 15, about 1 to 20, about 1 to 25, about 1 to 30, about 1 to 35, about 1 to 40, about 1 to 45, about 1 to 50, about 1 to 75, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to PBMCs in the rapid expansion and/or the second expansion is between 1 to 50 and 1 to 300. In an embodiment, the ratio of TILs to PBMCs in the rapid expansion and/or the second expansion is between 1 to 100 and 1 to 200.

In an embodiment, REP and/or the rapid second expansion is performed in flasks with the bulk TILs being mixed with a 100- or 200-fold excess of inactivated feeder cells, wherein the feeder cell concentration is at least 1.1 times (1.1X), 1.2X, 1.3X, 1.4X, 1.5X, 1.6X, 1.7X, 1.8X, 1.8X, 2X, 2.1X2.2X, 2.3X, 2.4X, 2.5X, 2.6X, 2.7X, 2.8X, 2.9X, 3.0X, 3.1X, 3.2X, 3.3X, 3.4X, 3.5X, 3.6X, 3.7X, 3.8X, 3.9X or 4.0X the feeder cell concentration in the priming first expansion, 30 ng/mL OKT3 anti-CD3 antibody and 6000 IU/mL IL-2 in 150 ml media. Media replacement is done (generally 2/3 media replacement via aspiration of 2/3 of spent media and replacement with an equal volume of fresh media) until the cells are transferred to an alternative growth chamber. Alternative growth chambers include G-REX flasks and gas permeable containers as more fully discussed below.

In some embodiments, the rapid second expansion (which can include processes referred to as the REP process) is 7 to 10 days, as discussed in the examples and figures. In some embodiments, the rapid second expansion (which can include processes referred to as the REP process) is 7 to 9 days, as discussed in the examples and figures. In some embodiments, the second expansion is 7 days. In some embodiments, the second expansion is 8 days. In some embodiments, the second expansion is 9 days. In some embodiments, the second expansion is 10 days.

In an embodiment, the second expansion (which can include expansions referred to as REP, as well as those referred to in Step D of Figure 85 (in particular, e.g., Figure 85B)) may be performed in 500 mL capacity gas permeable flasks with 100 cm gas-permeable silicon bottoms (G-Rex 100, commercially available from Wilson Wolf Manufacturing Corporation, New Brighton, MN, USA), $5 \times 10^6$ or $10 \times 10^6$ TIL may be cultured with PBMCs in 400 mL of 50/50 medium, supplemented with 5% human AB serum, 3000 IU per mL of IL-2 and 30 ng per ml of anti-CD3 (OKT3). The G-Rex 100 flasks may be incubated at 37°C in 5% $CO_2$. On day 5, 250 mL of supernatant may be removed and placed into centrifuge bottles and centrifuged at 1500 rpm ($491 \times g$) for 10 minutes. The TIL pellets may be re-suspended with 150 mL of fresh medium with 5% human AB serum, 6000 IU per mL of IL-2, and added back to the original GREX-100 flasks. When TIL are expanded serially in GREX-100 flasks, on day 10 or 11 the TILs can be moved to a larger flask, such as a GREX-500. The cells may be harvested on day 14 of culture. The cells may be harvested on day 15 of culture. The cells may be harvested on day 16 of culture. In some embodiments, media replacement is done until the cells are transferred to an alternative growth chamber. In some embodiments, 2/3 of the media is replaced by aspiration of spent media and replacement with an equal volume of fresh media. In some embodiments, alternative growth chambers include GREX flasks and gas permeable containers as more fully discussed below.

In an embodiment, the rapid second expansion (including expansions referred to as REP) is performed and further comprises a step wherein TILs are selected for superior tumor reactivity. Any selection method known in the art may be used. For example, the methods described in U.S. Patent Application Publication No. 2016/0010058 A1, may be used for selection of TILs for superior tumor reactivity.

Optionally, a cell viability assay can be performed after the rapid second expansion (including expansions referred to as the REP expansion), using standard assays known in the art. For example, a trypan blue exclusion assay can be done on a sample of the bulk TILs, which selectively labels dead cells and allows a viability assessment. In some embodiments, TIL samples can be counted and viability determined using a Cellometer K2 automated cell counter (Nexcelom Bioscience, Lawrence, MA). In some embodiments, viability is determined according to the standard Cellometer K2 Image Cytometer Automatic Cell Counter protocol.

The diverse antigen receptors of T and B lymphocytes are produced by somatic recombination of a limited, but large number of gene segments. These gene segments: V (variable), D (diversity), J (joining), and C (constant), determine the binding specificity and downstream applications of immunoglobulins and T-cell receptors (TCRs). The present invention provides a method for generating TILs which exhibit and increase the T-cell repertoire diversity. In some embodiments, the TILs obtained by the present method exhibit an increase in the T-cell repertoire diversity. In some embodiments, the TILs obtained in the second expansion exhibit an increase in the T-cell repertoire diversity. In some embodiments, the increase in diversity is an increase in the immunoglobulin diversity and/or the T-cell receptor diversity.

In some embodiments, the diversity is in the immunoglobulin is in the immunoglobulin heavy chain. In some embodiments, the diversity is in the immunoglobulin is in the immunoglobulin light chain. In some embodiments, the diversity is in the T-cell receptor. In some embodiments, the diversity is in one of the T-cell receptors selected from the group consisting of alpha, beta, gamma, and delta receptors. In some embodiments, there is an increase in the expression of T-cell receptor (TCR) alpha and/or beta. In some embodiments, there is an increase in the expression of T-cell receptor (TCR) alpha. In some embodiments, there is an increase in the expression of T-cell receptor (TCR) beta. In some embodiments, there is an increase in the expression of TCRab (*i.e.,* TCR$\alpha/\beta$).

In some embodiments, the rapid second expansion culture medium (e.g., sometimes referred to as CM2 or the second cell culture medium), comprises IL-2, OKT-3, as well as the antigen-presenting feeder cells (APCs), as discussed in more detail below. In some embodiments, the rapid second expansion culture medium (e.g., sometimes referred to as CM2 or the second cell culture medium), comprises 6000 IU/mL IL-2, 30 $\mu$g/flask OKT-3, as well as 7.5 $\times$ $10^8$ antigen-presenting feeder cells (APCs), as discussed in more detail below. In some embodiments, the rapid second expansion culture medium (e.g., sometimes referred to as CM2 or the second cell culture medium), comprises IL-2, OKT-3, as well as the antigen-presenting feeder cells (APCs), as discussed in more detail below. In some embodiments, the rapid second expansion culture medium (e.g., sometimes referred to as CM2 or the second cell culture medium), comprises 6000 IU/mL IL-2, 30 $\mu$g/flask OKT-3, as well as 5 $\times$ $10^8$ antigen-presenting feeder cells (APCs), as discussed in more detail below.

In some embodiments, the rapid second expansion, for example, Step D according to Figure 85 (in particular, e.g., Figure 85B), is performed in a closed system bioreactor. In some embodiments, a closed system is employed for the TIL expansion, as described herein. In some embodiments, a bioreactor is employed. In some embodiments, a bioreactor is employed as the container. In some embodiments, the bioreactor employed is for example a G-REX-100 or a G-REX-500. In some embodiments, the bioreactor employed is a G-REX-100. In some embodiments, the bioreactor employed is a G-REX-500.

1. Feeder Cells and Antigen Presenting Cells

In an embodiment, the rapid second expansion procedures described herein (for example including expansion such as those described in Step D from Figure 85 (in particular, *e.g.,* Figure 85B), as well as those referred to as REP) require an excess of feeder cells during REP TIL expansion and/or during the rapid second expansion. In many embodiments, the feeder cells are peripheral blood mononuclear cells (PBMCs) obtained from standard whole blood units from healthy blood donors. The PBMCs are obtained using standard methods such as Ficoll-Paque gradient separation.

In general, the allogenic PBMCs are inactivated, either via irradiation or heat treatment, and used in the REP procedures, as described in the examples, which provides an exemplary protocol for evaluating the replication incompetence of irradiate allogeneic PBMCs.

In some embodiments, PBMCs are considered replication incompetent and acceptable for use in the TIL expansion procedures described herein if the total number of viable cells on day 7 or 14 is less than the initial viable cell number put into culture on day 0 of the REP and/or day 0 of the second expansion *(i.e.,* the start day of the second expansion).

In some embodiments, PBMCs are considered replication incompetent and acceptable for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion *(i.e.,* the start day of the second expansion). In some embodiments, the PBMCs are cultured in the presence of 30 ng/mL OKT3 antibody and 3000 IU/mL IL-2. In some embodiments, the PBMCs are cultured in the presence of 30 ng/mL OKT3 antibody and 6000 IU/mL IL-2. In some embodiments, the PBMCs are cultured in the presence of 15 ng/mL OKT3 antibody and 3000 IU/mL IL-2. In some embodiments, the PBMCs are cultured in the presence of 15 ng/mL OKT3 antibody and 6000 IU/mL IL-2. In some embodiments, the PBMCs are cultured in the presence of 60 ng/mL OKT3 antibody and 3000 IU/mL IL-2. In some embodiments, the PBMCs are cultured in the presence of 60 ng/mL OKT3 antibody and 6000 IU/mL IL-2.

In some embodiments, PBMCs are considered replication incompetent and acceptable for use in the TIL expansion procedures described herein if the total number of viable cells, cultured in the presence of OKT3 and IL-2, on day 7 and day 14 has not increased from the initial viable cell number put into culture on day 0 of the REP and/or day 0 of the second expansion *(i.e.,* the start day of the second expansion). In some embodiments, the PBMCs are cultured in the presence of 30-60 ng/mL OKT3 antibody and 1000-6000 IU/mL IL-2. In some embodiments, the PBMCs are cultured in the presence of 30-60 ng/mL OKT3 antibody and 2000-5000 IU/mL IL-2. In some embodiments, the PBMCs are cultured in the presence of 30-60 ng/mL OKT3 antibody and 2000-4000 IU/mL IL-2. In some embodiments, the PBMCs are cultured in the presence of 30-60 ng/mL OKT3 antibody and 2500-3500 IU/mL IL-2. In some embodiments, the PBMCs are cultured in the presence of 30-60 ng/mL OKT3 antibody and 6000 IU/mL IL-2.

In some embodiments, the antigen-presenting feeder cells are PBMCs. In some embodiments, the antigen-presenting feeder cells are artificial antigen-presenting feeder cells. In an embodiment, the ratio of TILs to antigen-presenting feeder cells in the second expansion is about 1 to 10, about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to antigen-presenting feeder cells in the second expansion is between 1 to 50 and 1 to 300. In an embodiment, the ratio of TILs to antigen-presenting feeder cells in the second expansion is between 1 to 100 and 1 to 200.

In an embodiment, the second expansion procedures described herein require a ratio of about $5 \times 10^8$ feeder cells to about $100 \times 10^6$ TILs. In an embodiment, the second expansion procedures described herein require a ratio of about $7.5 \times 10^8$ feeder cells to about $100 \times 10^6$ TILs. In another embodiment, the second expansion procedures described herein require a ratio of about $5 \times 10^8$ feeder cells to about $50 \times 10^6$ TILs. In another embodiment, the second expansion procedures described herein require a ratio of about $7.5 \times 10^8$ feeder cells to about $50 \times 10^6$ TILs. In yet another embodiment, the second expansion procedures described herein require about $5 \times 10^8$ feeder cells to about $25 \times 10^6$ TILs. In yet another embodiment, the second expansion procedures described herein require about $7.5 \times 10^8$ feeder cells to about $25 \times 10^6$ TILs. In yet another embodiment, the rapid second expansion requires twice the number of feeder cells as the rapid second expansion. In yet another embodiment, when the priming first expansion described herein requires about $2.5 \times 10^8$ feeder cells, the rapid second expansion requires about $5 \times 10^8$ feeder cells. In yet another embodiment, when the priming first expansion described herein requires about $2.5 \times 10^8$ feeder cells, the rapid second expansion requires about $7.5 \times 10^8$ feeder cells. In yet another embodiment, the rapid second expansion requires two times (2.0X), 2.5X, 3.0X, 3.5X or 4.0X the number of feeder cells as the priming first expansion.

In an embodiment, the rapid second expansion procedures described herein require an excess of feeder cells during the rapid second expansion. In many embodiments, the feeder cells are peripheral blood mononuclear cells (PBMCs) obtained from standard whole blood units from allogeneic healthy blood donors. The PBMCs are obtained using standard methods such as Ficoll-Paque gradient separation. In an embodiment, artificial antigen-presenting (aAPC) cells are used in place of PBMCs. In some embodiments, the PBMCs are added to the rapid second expansion at twice the concentration of PBMCs that were added to the priming first expansion.

In general, the allogenic PBMCs are inactivated, either via irradiation or heat treatment, and used in the TIL expansion procedures described herein, including the exemplary procedures described in the figures and examples.

In an embodiment, artificial antigen presenting cells are used in the rapid second expansion as a replacement for, or in combination with, PBMCs.

2. Cytokines

The rapid second expansion methods described herein generally use culture media with high doses of a cytokine, in particular IL-2, as is known in the art.

Alternatively, using combinations of cytokines for the rapid second expansion of TILs is additionally possible, with combinations of two or more of IL-2, IL-15 and IL-21 as is generally outlined in WO 2015/189356 and WO 2015/189357. Thus, possible combinations include IL-2 and IL-15, IL-2 and IL-21, IL-15 and IL-21, and IL-2, IL-15 and IL-21, with the latter finding particular use in many embodiments. The use of combinations of cytokines specifically favors the generation of lymphocytes, and in particular T-cells as described therein.

### E. STEP E: Harvest TILS

After the rapid second expansion step, cells can be harvested. In some embodiments the TILs are harvested after one, two, three, four or more expansion steps, for example as provided in Figure 85 (in particular, *e.g.,* Figure 85B). In some embodiments the TILs are harvested after two expansion steps, for example as provided in Figure 85 (in particular, *e.g.,* Figure 85B). In some embodiments the TILs are harvested after two expansion steps, one priming first expansion and one rapid second expansion, for example as provided in Figure 85 (in particular, e.g., Figure 85B).

TILs can be harvested in any appropriate and sterile manner, including, for example by centrifugation. Methods for TIL harvesting are well known in the art and any such known methods can be employed with the present process. In some embodiments, TILS are harvested using an automated system.

Cell harvesters and/or cell processing systems are commercially available from a variety of sources, including, for example, Fresenius Kabi, Tomtec Life Science, Perkin Elmer, and Inotech Biosystems International, Inc. Any cell based harvester can be employed with the present methods. In some embodiments, the cell harvester and/or cell processing system is a membrane-based cell harvester. In some embodiments, cell harvesting is via a cell processing system, such as the LOVO system (manufactured by Fresenius Kabi). The term "LOVO cell processing system" also refers to any instrument or device manufactured by any vendor that can pump a solution comprising cells through a membrane or filter such as a spinning membrane or spinning filter in a sterile and/or closed system environment, allowing for continuous flow and cell processing to remove supernatant or cell culture media without pelletization. In some embodiments, the cell harvester and/or cell processing system can perform cell separation, washing, fluid-exchange, concentration, and/or other cell processing steps in a closed, sterile system.

In some embodiments, the rapid second expansion, for example, Step D according to Figure 85 (in particular, e.g., Figure 85B), is performed in a closed system bioreactor. In some embodiments, a closed system is employed for the TIL expansion, as described herein. In some embodiments, a bioreactor is employed. In some embodiments, a bioreactor is employed as the container. In some embodiments, the bioreactor employed is for example a G-REX-100 or a G-REX-500. In some embodiments, the bioreactor employed is a G-REX-100. In some embodiments, the bioreactor employed is a G-REX-500.

In some embodiments, Step E according to Figure 85 (in particular, e.g., Figure 85B), is performed according to the processes described herein. In some embodiments, the closed system is accessed via syringes under sterile conditions in order to maintain the sterility and closed nature of the system. In some embodiments, a closed system as described herein is employed.

In some embodiments, TILs are harvested according to the methods described in herein. In some embodiments, TILs between days 14 and 16 are harvested using the methods as described herein. In some embodiments, TILs are harvested at 14 days using the methods as described herein. In some embodiments, TILs are harvested at 15 days using the methods as described herein. In some embodiments, TILs are harvested at 16 days using the methods as described herein.

### F. STEP F: Final Formulation! Transfer to Infusion Bag

After Steps A through E as provided in an exemplary order in Figure 85 (in particular, e.g., Figure 85B) and as outlined in detailed above and herein are complete, cells are transferred to a container for use in administration to a patient. In some embodiments, once a therapeutically sufficient number of TILs are obtained using the expansion methods described above, they are transferred to a container for use in administration to a patient.

Also disclosed herein but not claimed, TILs expanded using the methods of the present disclosure are administered to a patient as a pharmaceutical composition. Also disclosed herein but not claimed, the pharmaceutical composition is a suspension of TILs in a sterile buffer. TILs expanded as disclosed herein may be administered by any suitable route as known in the art. Also disclosed herein but not claimed, the TILs are administered as a single intra-arterial or intravenous infusion, which preferably lasts approximately 30 to 60 minutes. Other suitable routes of administration include intraperitoneal, intrathecal, and intralymphatic.

In an embodiment, TILs expanded using processes of the foregoing disclosure may be for administration as compositions further comprising a cyropreservant. In an embodiment, TILs expanded using processes of the foregoing disclosure may be for administration as compositions further comprising a cyropreservant and an isotonic agent. In an embodiment, TILs expanded using processes of the foregoing disclosure may be for administration as compositions further comprising a cyropreservant comprising dimethylsulfoxide and an isotonic agent comprising sodium chloride, sodium gluconate, and sodium acetate. In an embodiment, TILs expanded using processes of the foregoing disclosure may be for administration as compositions further comprising a cyropreservant comprising dimethylsulfoxide and dextran 40 and an isotonic agent comprising sodium chloride, sodium gluconate, and sodium acetate. In an embodiment, TILs expanded using processes of the foregoing disclosure may be for administration as compositions delivered in a sterile infusion bag, such compositions further comprising a cyropreservant comprising dimethylsulfoxide and dextran 40 and an isotonic agent comprising sodium chloride, sodium gluconate, and sodium acetate.

### G. PBMC Feeder Cell Ratios

In some embodiments, the culture media used in expansion methods described herein (see for example, Figure 85 (in particular, e.g., Figure 85B)) include an anti-CD3 antibody e.g. OKT-3. An anti-CD3 antibody in combination with IL-2 induces T cell activation and cell division in the TIL population. This effect can be seen with full length antibodies as well as Fab and F(ab')2 fragments, with the former being generally preferred; see, *e.g.,* Tsoukas et al., J. Immunol. 1985, 135, 1719.

In an embodiment, the number of PBMC feeder layers is calculated as follows:

A. Volume of a T-cell (10 $\mu$m diameter): V= (4/3) $\pi r^3$ =523.6 $\mu m^3$

B. Columne of G-Rex 100 (M) with a 40 $\mu$m (4 cells) height: $V = (4/3)\ \pi r^3 = 4\times10^{12}\ \mu m^3$

C. Number cell required to fill column B: $4\times10^{12}\ \mu m^3$ / 523.6 $\mu m^3$ = $7.6\times10^{8}\ \mu m^3$ * 0.64 = $4.86\times10^{8}$

D. Number cells that can be optimally activated in 4D space: $4.86\times10^{8}$ / 24 = $20.25\times10^{6}$

E. Number of feeders and TIL extrapolated to G-Rex 500: TIL: $100\times10^6$ and Feeder: $2.5\times10^9$

In this calculation, an approximation of the number of mononuclear cells required to provide an icosahedral geometry for activation of TIL in a cylinder with a 100 $cm^2$ base is used. The calculation derives the experimental result of ~$5\times10^8$ for threshold activation of T-cells which closely mirrors NCI experimental data.[(1)] (C) The multiplier (0.64) is the random packing density for equivalent spheres as calculated by Jaeger and Nagel in 1992 [(2)]. (D) The divisor 24 is the number of equivalent spheres that could contact a similar object in 4 dimensional space "the Newton number"[(3)].

[(1)] Jin, Jianjian, et.al., Simplified Method of the Growth of Human Tumor Infiltrating Lymphocytes (TIL) in Gas-Permeable Flasks to Numbers Needed for Patient Treatment. J Immunother. 2012 Apr; 35(3): 283-292.

[(2)] Jaeger HM, Nagel SR. Physics of the granular state. Science. 1992 Mar 20;255(5051):1523-31.

[(3)] O. R. Musin (2003). "The problem of the twenty-five spheres". Russ. Math. Surv. 58 (4): 794-795.

In an embodiment, the number of antigen-presenting feeder cells exogenously supplied during the priming first expansion is approximately one-half the number of antigen-presenting feeder cells exogenously supplied during the rapid second expansion. In certain embodiments, the method comprises performing the priming first expansion in a cell culture medium which comprises approximately 50% fewer antigen presenting cells as compared to the cell culture medium of the rapid second expansion.

In another embodiment, the number of antigen-presenting feeder cells (APCs) exogenously supplied during the rapid second expansion is greater than the number of APCs exogenously supplied during the priming first expansion.

In another embodiment, the ratio of the number of APCs exogenously supplied during the rapid second expansion to the number of APCs exogenously supplied during the priming first expansion is selected from a range of from at or about 1.1:1 to at or about 20: 1.

In another embodiment, the ratio of the number of APCs exogenously supplied during the rapid second expansion to the number of APCs exogenously supplied during the priming first expansion is selected from a range of from at or about 1.1:1 to at or about 10: 1.

In another embodiment, the ratio of the number of APCs exogenously supplied during the rapid second expansion to the number of APCs exogenously supplied during the priming first expansion is selected from a range of from at or about 1.1:1 to at or about 9: 1.

In another embodiment, the ratio of the number of APCs exogenously supplied during the rapid second expansion to the number of APCs exogenously supplied during the priming first expansion is selected from a range of from at or about 1.1:1 to at or about 8:1.

In another embodiment, the ratio of the number of APCs exogenously supplied during the rapid second expansion to the number of APCs exogenously supplied during the priming first expansion is selected from a range of from at or about 1.1:1 to at or about 7: 1.

In another embodiment, the ratio of the number of APCs exogenously supplied during the rapid second expansion to the number of APCs exogenously supplied during the priming first expansion is selected from a range of from at or about 1.1:1 to at or about 6: 1.

In another embodiment, the ratio of the number of APCs exogenously supplied during the rapid second expansion to the number of APCs exogenously supplied during the priming first expansion is selected from a range of from at or about 1.1:1 to at or about 5:1.

In another embodiment, the ratio of the number of APCs exogenously supplied during the rapid second expansion to the number of APCs exogenously supplied during the priming first expansion is selected from a range of from at or about 1.1:1 to at or about 4: 1.

In another embodiment, the ratio of the number of APCs exogenously supplied during the rapid second expansion to the number of APCs exogenously supplied during the priming first expansion) is selected from a range of from at or about 1.1:1 to at or about 3: 1.

In another embodiment, the ratio of the number of APCs exogenously supplied during the rapid second expansion to the number of APCs exogenously supplied during the priming first expansion is selected from a range of from at or about 1.1:1 to at or about 2.9:1.

In another embodiment, the ratio of the number of APCs exogenously supplied during the rapid second expansion to the number of APCs exogenously supplied during the priming first expansion is selected from a range of from at or about 1.1:1 to at or about 2.8:1.

In another embodiment, the ratio of the number of APCs exogenously supplied during the rapid second expansion to the number of APCs exogenously supplied during the priming first expansion is selected from a range of from at or about 1.1:1 to at or about 2.7:1.

In another embodiment, the ratio of the number of APCs exogenously supplied during the rapid second expansion to the number of APCs exogenously supplied during the priming first expansion is selected from a range of from at or about 1.1:1 to at or about 2.6:1.

In another embodiment, the ratio of the number of APCs exogenously supplied during the rapid second expansion to the number of APCs exogenously supplied during the priming first expansion is selected from a range of from at or about 1.1:1 to at or about 2.5:1.

In another embodiment, the ratio of the number of APCs exogenously supplied during the rapid second expansion to the number of APCs exogenously supplied during the priming first expansion is selected from a range of from at or about 1.1:1 to at or about 2.4:1.

In another embodiment, the ratio of the number of APCs exogenously supplied during the rapid second expansion to the number of APCs exogenously supplied during the priming first expansion is selected from a range of from at or about 1.1:1 to at or about 2.3:1.

In another embodiment, the ratio of the number of APCs exogenously supplied during the rapid second expansion to the number of APCs exogenously supplied during the priming first expansion is selected from a range of from at or about 1.1:1 to at or about 2.2:1.

In another embodiment, the ratio of the number of APCs exogenously supplied during the rapid second expansion to the number of APCs exogenously supplied during the priming first expansion is selected from a range of from at or about 1.1:1 to at or about 2.1:1.

In another embodiment, the ratio of the number of APCs exogenously supplied during the rapid second expansion to the number of APCs exogenously supplied during the priming first expansion is selected from a range of from at or about 1.1:1 to at or about 2: 1.

In another embodiment, the ratio of the number of APCs exogenously supplied during the rapid second expansion to the number of APCs exogenously supplied during the priming first expansion is selected from a range of from at or about 2: 1 to at or about 10: 1.

In another embodiment, the ratio of the number of APCs exogenously supplied during the rapid second expansion to the number of APCs exogenously supplied during the priming first expansion is selected from a range of from at or about 2: 1 to at or about 5:1.

In another embodiment, the ratio of the number of APCs exogenously supplied during the rapid second expansion to the number of APCs exogenously supplied during the priming first expansion is selected from a range of from at or about 2:1 to at or about 4:1.

In another embodiment, the ratio of the number of APCs exogenously supplied during the rapid second expansion to the number of APCs exogenously supplied during the priming first expansion is selected from a range of from at or about 2: 1 to at or about 3: 1.

In another embodiment, the ratio of the number of APCs exogenously supplied during the rapid second expansion to the number of APCs exogenously supplied during the priming first expansion is selected from a range of from at or about 2:1 to at or about 2.9:1.

In another embodiment, the ratio of the number of APCs exogenously supplied during the rapid second expansion to the number of APCs exogenously supplied during the priming first expansion is selected from a range of from at or about 2:1 to at or about 2.8:1.

In another embodiment, the ratio of the number of APCs exogenously supplied during the rapid second expansion to the number of APCs exogenously supplied during the priming first expansion is selected from a range of from at or about 2:1 to at or about 2.7:1.

In another embodiment, the ratio of the number of APCs exogenously supplied during the rapid second expansion to the number of APCs exogenously supplied during the priming first expansion is selected from a range of from at or about 2:1 to at or about 2.6:1.

In another embodiment, the ratio of the number of APCs exogenously supplied during the rapid second expansion to the number of APCs exogenously supplied during the priming first expansion is selected from a range of from at or about 2:1 to at or about 2.5:1.

In another embodiment, the ratio of the number of APCs exogenously supplied during the rapid second expansion to the number of APCs exogenously supplied during the priming first expansion is selected from a range of from at or about 2:1 to at or about 2.4:1.

In another embodiment, the ratio of the number of APCs exogenously supplied during the rapid second expansion to the number of APCs exogenously supplied during the priming first expansion is selected from a range of from at or about 2:1 to at or about 2.3:1.

In another embodiment, the ratio of the number of APCs exogenously supplied during the rapid second expansion to the number of APCs exogenously supplied during the priming first expansion is selected from a range of from at or about about 2:1 to at or about 2.2:1.

In another embodiment, the ratio of the number of APCs exogenously supplied during the rapid second expansion to the number of APCs exogenously supplied during the priming first expansion is selected from a range of from at or about 2:1 to at or about 2.1:1.

In another embodiment, the ratio of the number of APCs exogenously supplied during the rapid second expansion to the number of APCs exogenously supplied during the priming first expansion is at or about 2:1.

In another embodiment, the ratio of the number of APCs exogenously supplied during the rapid second expansion to the number of APCs exogenously supplied during the priming first expansion is at or about 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, 4:1, 4.1:1, 4.2:1, 4.3:1, 4.4:1, 4.5:1, 4.6:1, 4.7:1, 4.8:1, 4.9:1, or 5:1.

In another embodiment, the number of APCs exogenously supplied during the priming first expansion is at or about $1 \times 10^8$, $1.1 \times 10^8$, $1.2 \times 10^8$, $1.3 \times 10^8$, $1.4 \times 10^8$, $1.5 \times 10^8$, $1.6 \times 10^8$, $1.7 \times 10^8$, $1.8 \times 10^8$, $1.9 \times 10^8$, $2 \times 10^8$, $2.1 \times 10^8$, $2.2 \times 10^8$, $2.3 \times 10^8$, $2.4 \times 10^8$, $2.5 \times 10^8$, $2.6 \times 10^8$, $2.7 \times 10^8$, $2.8 \times 10^8$, $2.9 \times 10^8$, $3 \times 10^8$, $3.1 \times 10^8$, $3.2 \times 10^8$, $3.3 \times 10^8$, $3.4 \times 10^8$ or $3.5 \times 10^8$ APCs, and the number of APCs exogenously supplied during the rapid second expansion is at or about $3.5 \times 10^8$, $3.6 \times 10^8$, $3.7 \times 10^8$, $3.8 \times 10^8$, $3.9 \times 10^8$, $4 \times 10^8$, $4.1 \times 10^8$, $4.2 \times 10^8$, $4.3 \times 10^8$, $4.4 \times 10^8$, $4.5 \times 10^8$, $4.6 \times 10^8$, $4.7 \times 10^8$, $4.8 \times 10^8$, $4.9 \times 10^8$,$5 \times 10^8$, $5.1 \times 10^8$, $5.2 \times 10^8$, $5.3 \times 10^8$, $5.4 \times 10^8$, $5.5 \times 10^8$, $5.6 \times 10^8$, $5.7 \times 10^8$, $5.8 \times 10^8$, $5.9 \times 10^8$, $6 \times 10^8$, $6.1 \times 10^8$, $6.2 \times 10^8$, $6.3 \times 10^8$, $6.4 \times 10^8$, $6.5 \times 10^8$, $6.6 \times 10^8$, $6.7 \times 10^8$, $6.8 \times 10^8$, $6.9 \times 10^8$, $7 \times 10^8$, $7.1 \times 10^8$, $7.2 \times 10^8$, $7.3 \times 10^8$, $7.4 \times 10^8$, $7.5 \times 10^8$, $7.6 \times 10^8$, $7.7 \times 10^8$, $7.8 \times 10^8$, $7.9 \times 10^8$, $8 \times 10^8$, $8.1 \times 10^8$, $8.2 \times 10^8$, $8.3 \times 10^8$, $8.4 \times 10^8$, $8.5 \times 10^8$, $8.6 \times 10^8$, $8.7 \times 10^8$, $8.8 \times 10^8$, $8.9 \times 10^8$, $9 \times 10^8$, $9.1 \times 10^8$, $9.2 \times 10^8$, $9.3 \times 10^8$, $9.4 \times 10^8$, $9.5 \times 10^8$, $9.6 \times 10^8$, $9.7 \times 10^8$, $9.8 \times 10^8$, $9.9 \times 10^8$ or $1 \times 10^9$ APCs.

In another embodiment, the number of APCs exogenously supplied during the priming first expansion is selected from the range of at or about $1.5 \times 10^8$ APCs to at or about $3 \times 10^8$ APCs, and the number of APCs exogenously supplied during the rapid second expansion is selected from the range of at or about $4 \times 10^8$ APCs to at or about $7.5 \times 10^8$ APCs.

In another embodiment, the number of APCs exogenously supplied during the priming first expansion is selected from the range of at or about $2 \times 10^8$ APCs to at or about $2.5 \times 10^8$ APCs, and the number of APCs exogenously supplied during the rapid second expansion is selected from the range of at or about $4.5 \times 10^8$ APCs to at or about $5.5 \times 10^8$ APCs.

In another embodiment, the number of APCs exogenously supplied during the priming first expansion is at or about $2.5 \times 10^8$ APCs, and the number of APCs exogenously supplied during the rapid second expansion is at or about $5 \times 10^8$ APCs.

In an embodiment, the number of APCs (including, for example, PBMCs) added at day 0 of the priming first expansion is approximately one-half of the number of PBMCs added at day 7 of the priming first expansion (e.g., day 7 of the method). In certain embodiments, the method comprises adding antigen presenting cells at day 0 of the priming first expansion to the first population of TILs and adding antigen presenting cells at day 7 to the second population of TILs, wherein the number of antigen presenting cells added at day 0 is approximately 50% of the number of antigen presenting cells added at day 7 of the priming first expansion (e.g., day 7 of the method).

In another embodiment, the number of APCs (including, for example, PBMCs) exogenously supplied at day 7 of the rapid second expansion is greater than the number of PBMCs exogenously supplied at day 0 of the priming first expansion.

In another embodiment, the APCs exogenously supplied in the priming first expansion are seeded in the culture flask at a density selected from a range of at or about $1.0 \times 10^6$ APCs/cm$^2$ to at or about $4.5 \times 10^6$ APCs/cm$^2$.

In another embodiment, the APCs exogenously supplied in the priming first expansion are seeded in the culture flask at a density selected from a range of at or about $1.5 \times 10^6$ APCs/cm$^2$ to at or about $3.5 \times 10^6$ APCs/cm$^2$.

In another embodiment, the APCs exogenously supplied in the priming first expansion are seeded in the culture flask at a density selected from a range of at or about $2 \times 10^6$ APCs/cm$^2$ to at or about $3 \times 10^6$ APCs/cm$^2$.

In another embodiment, the APCs exogenously supplied in the priming first expansion are seeded in the culture flask at a density of at or about $2 \times 10^6$ APCs/cm$^2$.

In another embodiment, the APCs exogenously supplied in the priming first expansion are seeded in the culture flask at a density of at or about $1.0 \times 10^6$, $1.1 \times 10^6$, $1.2 \times 10^6$, $1.3 \times 10^6$, $1.4 \times 10^6$, $1.5 \times 10^6$, $1.6 \times 10^6$, $1.7 \times 10^6$, $1.8 \times 10^6$, $1.9 \times 10^6$, $2 \times 10^6$, $2.1 \times 10^6$, $2.2 \times 10^6$, $2.3 \times 10^6$, $2.4 \times 10^6$, $2.5 \times 10^6$, $2.6 \times 10^6$, $2.7 \times 10^6$, $2.8 \times 10^6$, $2.9 \times 10^6$, $3 \times 10^6$, $3.1 \times 10^6$, $3.2 \times 10^6$, $3.3 \times 10^6$, $3.4 \times 10^6$, $3.5 \times 10^6$, $3.6 \times 10^6$, $3.7 \times 10^6$, $3.8 \times 10^6$, $3.9 \times 10^6$, $4 \times 10^6$, $4.1 \times 10^6$, $4.2 \times 10^6$, $4.3 \times 10^6$, $4.4 \times 10^6$ or $4.5 \times 10^6$ APCs/cm$^2$.

In another embodiment, the APCs exogenously supplied in the rapid second expansion are seeded in the culture flask at a density selected from a range of at or about $2.5 \times 10^6$ APCs/cm$^2$ to at or about $7.5 \times 10^6$ APCs/cm$^2$.

In another embodiment, the APCs exogenously supplied in the rapid second expansion are seeded in the culture flask at a density selected from a range of at or about $3.5 \times 10^6$ APCs/cm$^2$ to about $6.0 \times 10^6$ APCs/cm$^2$.

In another embodiment, the APCs exogenously supplied in the rapid second expansion are seeded in the culture flask at a density selected from a range of at or about $4.0 \times 10^6$ APCs/cm$^2$ to about $5.5 \times 10^6$ APCs/cm$^2$.

In another embodiment, the APCs exogenously supplied in the rapid second expansion are seeded in the culture flask at a density selected from a range of at or about $4.0 \times 10^6$ APCs/$cm^2$.

In another embodiment, the APCs exogenously supplied in the rapid second expansion are seeded in the culture flask at a density of at or about $2.5 \times 10^6$ APCs/$cm^2$, $2.6 \times 10^6$ APCs/$cm^2$, $2.7 \times 10^6$ APCs/$cm^2$, $2.8 \times 10^6$, $2.9 \times 10^6$, $3 \times 10^6$, $3.1 \times 10^6$, $3.2 \times 10^6$, $3.3 \times 10^6$, $3.4 \times 10^6$, $3.5 \times 10^6$, $3.6 \times 10^6$, $3.7 \times 10^6$, $3.8 \times 10^6$, $3.9 \times 10^6$, $4 \times 10^6$, $4.1 \times 10^6$, $4.2 \times 10^6$, $4.3 \times 10^6$, $4.4 \times 10^6$, $4.5 \times 10^6$, $4.6 \times 10^6$, $4.7 \times 10^6$, $4.8 \times 10^6$, $4.9 \times 10^6$, $5 \times 10^6$, $5.1 \times 10^6$, $5.2 \times 10^6$, $5.3 \times 10^6$, $5.4 \times 10^6$, $5.5 \times 10^6$, $5.6 \times 10^6$, $5.7 \times 10^6$, $5.8 \times 10^6$, $5.9 \times 10^6$, $6 \times 10^6$, $6.1 \times 10^6$, $6.2 \times 10^6$, $6.3 \times 10^6$, $6.4 \times 10^6$, $6.5 \times 10^6$, $6.6 \times 10^6$, $6.7 \times 10^6$, $6.8 \times 10^6$, $6.9 \times 10^6$, $7 \times 10^6$, $7.1 \times 10^6$, $7.2 \times 10^6$, $7.3 \times 10^6$, $7.4 \times 10^6$, or $7.5 \times 10^6$ APCs/$cm^2$.

In another embodiment, the APCs exogenously supplied in the priming first expansion are seeded in the culture flask at a density of at or about $1.0 \times 10^6$, $1.1 \times 10^6$, $1.2 \times 10^6$, $1.3 \times 10^6$, $1.4 \times 10^6$, $1.5 \times 10^6$, $1.6 \times 10^6$, $1.7 \times 10^6$, $1.8 \times 10^6$, $1.9 \times 10^6$, $2 \times 10^6$, $2.1 \times 10^6$, $2.2 \times 10^6$, $2.3 \times 10^6$, $2.4 \times 10^6$, $2.5 \times 10^6$, $2.6 \times 10^6$, $2.7 \times 10^6$, $2.8 \times 10^6$, $2.9 \times 10^6$, $3 \times 10^6$, $3.1 \times 10^6$, $3.2 \times 10^6$, $3.3 \times 10^6$, $3.4 \times 10^6$, $3.5 \times 10^6$, $3.6 \times 10^6$, $3.7 \times 10^6$, $3.8 \times 10^6$, $3.9 \times 10^6$, $4 \times 10^6$, $4.1 \times 10^6$, $4.2 \times 10^6$, $4.3 \times 10^6$, $4.4 \times 10^6$, or $4.5 \times 10^6$ APCs/$cm^2$ and the the APCs exogenously supplied in the rapid second expansion are seeded in the culture flask at a density of at or about $2.5 \times 10^6$ APCs/$cm^2$, $2.6 \times 10^6$ APCs/$cm^2$, $2.7 \times 10^6$ APCs/$cm^2$, $2.8 \times 10^6$, $2.9 \times 10^6$, $3 \times 10^6$, $3.1 \times 10^6$, $3.2 \times 10^6$, $3.3 \times 10^6$, $3.4 \times 10^6$, $3.5 \times 10^6$, $3.6 \times 10^6$, $3.7 \times 10^6$, $3.8 \times 10^6$, $3.9 \times 10^6$, $4 \times 10^6$, $4.1 \times 10^6$, $4.2 \times 10^6$, $4.3 \times 10^6$, $4.4 \times 10^6$, $4.5 \times 10^6$, $4.6 \times 10^6$, $4.7 \times 10^6$, $4.8 \times 10^6$, $4.9 \times 10^6$, $5 \times 10^6$, $5.1 \times 10^6$, $5.2 \times 10^6$, $5.3 \times 10^6$, $5.4 \times 10^6$, $5.5 \times 10^6$, $5.6 \times 10^6$, $5.7 \times 10^6$, $5.8 \times 10^6$, $5.9 \times 10^6$, $6 \times 10^6$, $6.1 \times 10^6$, $6.2 \times 10^6$, $6.3 \times 10^6$, $6.4 \times 10^6$, $6.5 \times 10^6$, $6.6 \times 10^6$, $6.7 \times 10^6$, $6.8 \times 10^6$, $6.9 \times 10^6$, $7 \times 10^6$, $7.1 \times 10^6$, $7.2 \times 10^6$, $7.3 \times 10^6$, $7.4 \times 10^6$, or $7.5 \times 10^6$ APCs/$cm^2$.

In another embodiment, the APCs exogenously supplied in the priming first expansion are seeded in the culture flask at a density selected from a range of at or about $1.0 \times 10^6$ APCs/$cm^2$ to at or about $4.5 \times 10^6$ APCs/$cm^2$, and the APCs exogenously supplied in the rapid second expansion are seeded in the culture flask at a density selected from a range of at or about $2.5 \times 10^6$ APCs/$cm^2$ to at or about $7.5 \times 10^6$ APCs/$cm^2$.

In another embodiment, the APCs exogenously supplied in the priming first expansion are seeded in the culture flask at a density selected from a range of at or about $1.5 \times 10^6$ APCs/$cm^2$ to at or about $3.5 \times 10^6$ APCs/$cm^2$, and the APCs exogenously supplied in the rapid second expansion are seeded in the culture flask at a density selected from a range of at or about $3.5 \times 10^6$ APCs/$cm^2$ to at or about $6 \times 10^6$ APCs/$cm^2$.

In another embodiment, the APCs exogenously supplied in the priming first expansion are seeded in the culture flask at a density selected from a range of at or about $2 \times 10^6$ APCs/$cm^2$ to at or about $3 \times 10^6$ APCs/$cm^2$, and the APCs exogenously supplied in the rapid second expansion are seeded in the culture flask at a density selected from a range of at or about $4 \times 10^6$ APCs/$cm^2$ to at or about $5.5 \times 10^6$ APCs/$cm^2$.

In another embodiment, the APCs exogenously supplied in the priming first expansion are seeded in the culture flask at a density at or about $2 \times 10^6$ APCs/$cm^2$ and the APCs exogenously supplied in the rapid second expansion are seeded in the culture flask at a density of at or about $4 \times 10^6$ APCs/$cm^2$.

In another embodiment, the ratio of the number of APCs (including, for example, PBMCs) exogenously supplied at day 7 of the rapid second expansion to the number of PBMCs exogenously supplied at day 0 of the priming first expansion is selected from a range of from at or about 1.1: 1 to at or about 20:1.

In another embodiment, the ratio of the number of APCs (including, for example, PBMCs) exogenously supplied at day 7 of the rapid second expansion to the number of PBMCs exogenously supplied at day 0 of the priming first expansion is selected from a range of from at or about 1.1: 1 to at or about 10:1.

In another embodiment, the ratio of the number of APCs (including, for example, PBMCs) exogenously supplied at day 7 of the rapid second expansion to the number of PBMCs exogenously supplied at day 0 of the priming first expansion is selected from a range of from at or about 1.1: 1 to at or about 9:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 8:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 7:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 6:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 5:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 4:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 3: 1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.9:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.8:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.7:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.6:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.5:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.4:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.3:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.2:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2.1:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 1.1:1 to at or about 2:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2:1 to at or about 10: 1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 5:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 4: 1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 3: 1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 2.9:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 2.8:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2:1 to at or about 2.7:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 2.6:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 2.5:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2:1 to at or about 2.4:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2: 1 to at or about 2.3:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2:1 to at or about 2.2:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is selected from a range of from at or about 2:1 to at or about 2.1:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is at or about 2:1.

In another embodiment, the ratio of the number of APCs (including, for example, PBMCs) exogenously supplied at day 7 of the rapid second expansion to the number of APCs (including, for example, PBMCs) exogenously supplied at day 0 of the priming first expansion is at or about 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, 4:1, 4.1:1, 4.2:1, 4.3:1, 4.4:1, 4.5:1, 4.6:1, 4.7:1, 4.8:1, 4.9:1, or 5:1.

In another embodiment, the number of APCs (including, for example, PBMCs) exogenously supplied at day 0 of the priming first expansion is at or about $1 \times 10^8$, $1.1 \times 10^8$, $1.2 \times 10^8$, $1.3 \times 10^8$, $1.4 \times 10^8$, $1.5 \times 10^8$, $1.6 \times 10^8$, $1.7 \times 10^8$, $1.8 \times 10^8$, $1.9 \times 10^8$, $2 \times 10^8$, $2.1 \times 10^8$, $2.2 \times 10^8$, $2.3 \times 10^8$, $2.4 \times 10^8$, $2.5 \times 10^8$, $2.6 \times 10^8$, $2.7 \times 10^8$, $2.8 \times 10^8$, $2.9 \times 10^8$, $3 \times 10^8$, $3.1 \times 10^8$, $3.2 \times 10^8$, $3.3 \times 10^8$, $3.4 \times 10^8$ or $3.5 \times 10^8$ APCs (including, for example, PBMCs), and the number of APCs (including, for example, PBMCs) exogenously supplied at day 7 of the rapid second expansion is at or about $3.5 \times 10^8$, $3.6 \times 10^8$, $3.7 \times 10^8$, $3.8 \times 10^8$, $3.9 \times 10^8$, $4 \times 10^8$, $4.1 \times 10^8$, $4.2 \times 10^8$, $4.3 \times 10^8$, $4.4 \times 10^8$, $4.5 \times 10^8$, $4.6 \times 10^8$, $4.7 \times 10^8$, $4.8 \times 10^8$, $4.9 \times 10^8$, $5 \times 10^8$, $5.1 \times 10^8$, $5.2 \times 10^8$, $5.3 \times 10^8$, $5.4 \times 10^8$, $5.5 \times 10^8$, $5.6 \times 10^8$, $5.7 \times 10^8$, $5.8 \times 10^8$, $5.9 \times 10^8$, $6 \times 10^8$, $6.1 \times 10^8$, $6.2 \times 10^8$, $6.3 \times 10^8$, $6.4 \times 10^8$, $6.5 \times 10^8$, $6.6 \times 10^8$, $6.7 \times 10^8$, $6.8 \times 10^8$, $6.9 \times 10^8$, $7 \times 10^8$, $7.1 \times 10^8$, $7.2 \times 10^8$, $7.3 \times 10^8$, $7.4 \times 10^8$, $7.5 \times 10^8$, $7.6 \times 10^8$, $7.7 \times 10^8$, $7.8 \times 10^8$, $7.9 \times 10^8$, $8 \times 10^8$, $8.1 \times 10^8$, $8.2 \times 10^8$, $8.3 \times 10^8$, $8.4 \times 10^8$, $8.5 \times 10^8$, $8.6 \times 10^8$, $8.7 \times 10^8$, $8.8 \times 10^8$, $8.9 \times 10^8$, $9 \times 10^8$, $9.1 \times 10^8$, $9.2 \times 10^8$, $9.3 \times 10^9$, $9.4 \times 10^8$,$9.5 \times 10^8$, $9.6 \times 10^8$, $9.7 \times 10^8$, $9.8 \times 10^8$, $9.9 \times 10^8$ or $1 \times 10^9$ APCs (including, for example, PBMCs).

In another embodiment, the number of APCs (including, for example, PBMCs) exogenously supplied at day 0 of the priming first expansion is selected from the range of at or about $1 \times 10^8$ APCs (including, for example, PBMCs) to at or about $3.5 \times 10^8$ APCs (including, for example, PBMCs), and the number of APCs (including, for example, PBMCs) exogenously supplied at day 7 of the rapid second expansion is selected from the range of at or about $3.5 \times 10^8$ APCs (including, for example, PBMCs) to at or about $1 \times 10^9$ APCs (including, for example, PBMCs).

In another embodiment, the number of APCs (including, for example, PBMCs) exogenously supplied at day 0 of the priming first expansion is selected from the range of at or about $1.5 \times 10^8$ APCs to at or about $3 \times 10^8$ APCs (including, for example, PBMCs), and the number of APCs (including, for example, PBMCs) exogenously supplied at day 7 of the rapid second expansion is selected from the range of at or about $4 \times 10^8$ APCs (including, for example, PBMCs) to at or about $7.5 \times 10^8$ APCs (including, for example, PBMCs).

In another embodiment, the number of APCs (including, for example, PBMCs) exogenously supplied at day 0 of the priming first expansion is selected from the range of at or about $2 \times 10^8$ APCs (including, for example, PBMCs) to at or about $2.5 \times 10^8$ APCs (including, for example, PBMCs), and the number of APCs (including, for example, PBMCs) exogenously supplied at day 7 of the rapid second expansion is selected from the range of at or about $4.5 \times 10^8$ APCs (including, for example, PBMCs) to at or about $5.5 \times 10^8$ APCs (including, for example, PBMCs).

In another embodiment, the number of APCs (including, for example, PBMCs) exogenously supplied at day 0 of the priming first expansion is at or about $2.5 \times 10^8$ APCs (including, for example, PBMCs) and the number of APCs (including, for example, PBMCs) exogenously supplied at day 7 of the rapid second expansion is at or about $5 \times 10^8$ APCs (including, for example, PBMCs).

In an embodiment, the number of layers of APCs (including, for example, PBMCs) added at day 0 of the priming first expansion is approximately one-half of the number of layers of APCs (including, for example, PBMCs) added at day 7 of the rapid second expansion. In certain embodiments, the method comprises adding antigen presenting cell layers at day 0 of the priming first expansion to the first population of TILs and adding antigen presenting cell layers at day 7 to the second population of TILs, wherein the number of antigen presenting cell layer added at day 0 is approximately 50% of the number of antigen presenting cell layers added at day 7.

In another embodiment, the number of layers of APCs (including, for example, PBMCs) exogenously supplied at day 7 of the rapid second expansion is greater than the number of layers of APCs (including, for example, PBMCs) exogenously supplied at day 0 of the priming first expansion.

In another embodiment, day 0 of the priming first expansion occurs in the presence of layered APCs (including, for example, PBMCs) with an average thickness of at or about 2 cell layers and day 7 of the rapid second expansion occurs in the presence of layered APCs (including, for example, PBMCs) with an average thickness of at or about 4 cell layers.

In another embodiment, day 0 of the priming first expansion occurs in the presence of layered APCs (including, for example, PBMCs) with an average thickness of at or about one cell layer and day 7 of the rapid second expansion occurs in the presence of layered APCs (including, for example, PBMCs) with an average thickness of at or about 3 cell layers.

In another embodiment, day 0 of the priming first expansion occurs in the presence of layered APCs (including, for example, PBMCs) with an average thickness of at or about 1.5 cell layers to at or about 2.5 cell layers and day 7 of the rapid second expansion occurs in the presence of layered APCs (including, for example, PBMCs) with an average thickness of at or about 3 cell layers.

In another embodiment, day 0 of the priming first expansion occurs in the presence of layered APCs (including, for example, PBMCs) with an average thickness of at or about one cell layer and day 7 of the rapid second expansion occurs in the presence of layered APCs (including, for example, PBMCs) with an average thickness of at or about 2 cell layers.

In another embodiment, day 0 of the priming first expansion occurs in the presence of layered APCs (including, for example, PBMCs) with an average thickness of of at or about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 cell layers and day 7 of the rapid second expansion occurs in the presence of layered APCs (including, for example, PBMCs) with an average thickness of at or about 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9 or 8 cell layers.

In another embodiment, day 0 of the priming first expansion occurs in the presence of layered APCs (including, for example, PBMCs) with an average thickness of at or about 1 cell layer to at or about 2 cell layers and day 7 of the rapid second expansion occurs in the presence of layered APCs (including, for example, PBMCs) with an average thickness of at or about 3 cell layers to at or about 10 cell layers.

In another embodiment, day 0 of the priming first expansion occurs in the presence of layered APCs (including, for example, PBMCs) with an average thickness of at or about 2 cell layers to at or about 3 cell layers and day 7 of the rapid second expansion occurs in the presence of layered APCs (including, for example, PBMCs) with an average thickness of at or about 4 cell layers to at or about 8 cell layers.

In another embodiment, day 0 of the priming first expansion occurs in the presence of layered APCs (including, for example, PBMCs) with an average thickness of at or about 2 cell layers and day 7 of the rapid second expansion occurs in the presence of layered APCs (including, for example, PBMCs) with an average thickness of at or about 4 cell layers to at or about 8 cell layers.

In another embodiment, day 0 of the priming first expansion occurs in the presence of layered APCs (including, for example, PBMCs) with an average thickness of at or about 1, 2 or 3 cell layers and day 7 of the rapid second expansion occurs in the presence of layered APCs (including, for example, PBMCs) with an average thickness of at or about 3, 4, 5, 6, 7, 8, 9 or 10 cell layers.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:10.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:8.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:7.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:6.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:5.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:4.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:3.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.1 to at or about 1:2.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.2 to at or about 1:8.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.3 to at or about 1:7.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.4 to at or about 1:6.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.5 to at or about 1:5.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.6 to at or about 1:4.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.7 to at or about 1:3.5.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.8 to at or about 1:3.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from the range of at or about 1:1.9 to at or about 1:2.5.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is at or about 1:2.

In another embodiment, day 0 of the priming first expansion occurs in the presence of layered APCs (including, for example, PBMCs) with a first average thickness equal to a first number of layers of APCs (including, for example, PBMCs) and day 7 of the rapid second expansion occurs in the presence of layered APCs (including, for example, PBMCs) with a second average thickness equal to a second number of layers of APCs (including, for example, PBMCs), wherein the ratio of the first number of layers of APCs (including, for example, PBMCs) to the second number of layers of APCs (including, for example, PBMCs) is selected from at or about 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, 1:3, 1:3.1, 1:3.2, 1:3.3, 1:3.4, 1:3.5, 1:3.6, 1:3.7, 1:3.8, 1:3.9, 1:4, 1:4.1, 1:4.2, 1:4.3, 1:4.4, 1:4.5, 1:4.6, 1:4.7, 1:4.8, 1:4.9, 1:5, 1:5.1, 1:5.2, 1:5.3, 1:5.4, 1:5.5, 1:5.6, 1:5.7, 1:5.8, 1:5.9, 1:6, 1:6.1, 1:6.2, 1:6.3, 1:6.4, 1:6.5, 1:6.6, 1:6.7, 1:6.8, 1:6.9, 1:7, 1:7.1, 1:7.2, 1:7.3, 1:7.4, 1:7.5, 1:7.6, 1:7.7, 1:7.8, 1:7.9, 1:8, 1:8.1, 1:8.2, 1:8.3, 1:8.4, 1:8.5, 1:8.6, 1:8.7, 1:8.8, 1:8.9, 1:9, 1:9.1, 1:9.2, 1:9.3, 1:9.4, 1:9.5, 1:9.6, 1:9.7, 1:9.8, 1:9.9 or 1:10.

### V. Optional Cell Medium Components

1. Anti-CD3 Antibodies

In some embodiments of Process 2A, the culture media used in expansion methods described herein (including those referred to as REP, see for example, Figure 1) also includes an anti-CD3 antibody. An anti-CD3 antibody in combination with IL-2 induces T cell activation and cell division in the TIL population. This effect can be seen with full length antibodies as well as Fab and F(ab')2 fragments, with the former being generally preferred; see, *e.g.,* Tsoukas et al., J. Immunol. 1985, 135, 1719.

As will be appreciated by those in the art, there are a number of suitable anti-human CD3 antibodies that find use in the invention, including anti-human CD3 polyclonal and monoclonal antibodies from various mammals, including, but not limited to, murine, human, primate, rat, and canine antibodies. In particular embodiments, the OKT3 anti-CD3 antibody is used (commercially available from Ortho-McNeil, Raritan, NJ or Miltenyi Biotech, Auburn, CA).

TABLE 4: Amino acid sequences of muromonab (exemplary OKT-3 antibody)

| Identifier | Sequence (One-Letter Amino Acid Symbols) |
|---|---|
| SEQ ID NO:1 Muromonab heavy chain | QVQLQQSGAE LARPGASVKM SCKASGYTFT RYTMHWVKQR PGQGLEWIGY INPSRGYTNY 60<br>NQKFKDKATL TTDKSSSTAY MQLSSLTSED SAVYYCARYY DDHYCLDYWG QGTTLTVSSA 120<br>KTTAPSVYPL APVCGGTTGS SVTLGCLVKG YFPEPVTLTW NSGSLSSGVH TFPAVLQSDL 180<br>YTLSSSVTVT SSTWPSQSIT CNVAHPASST KVDKKIEPRP KSCDKTHTCP PCPAPELLGG 240<br>PSVFLFPPKP KDTLMISRTP EVTCVVVDVS HEDPEVKFNW YVDGVEVHNA KTKPREEQYN 300<br>STYRVVSVLT VLHQDWLNGK EYKCKVSNKA LPAPIEKTIS KAKGQPREPQ VYTLPPSRDE 360<br>LTKNQVSLTC LVKGFYPSDI AVEWESNGQP ENNYKTTPPV LDSDGSFFLY SKLTVDKSRW 420<br>QQGNVFSCSV MHEALHNHYT QKSLSLSPGK 450 |
| SEQ ID NO:2 Muromonab light chain | QIVLTQSPAI MSASPGEKVT MTCSASSSVS YMNWYQQKSG TSPKRWIYDT SKLASGVPAH 60<br>FRGSGSGTSY SLTISGMEAE DAATYYCQQW SSNPFTFGSG TKLEINRADT APTVSIFPPS 120<br>SEQLTSGGAS VVCFLNNFYP KDINVKWKID GSERQNGVLN SWTDQDSKDS TYSMSSTLTL 180<br>TKDEYERHNS YTCEATHKTS TSPIVKSFNR NEC 213 |

2. 4-1BB (CD137) AGONISTS

In an embodiment, the TNFRSF agonist is a 4-1BB (CD137) agonist. The 4-1BB agonist may be any 4-1BB binding molecule known in the art. The 4-1BB binding molecule may be a monoclonal antibody or fusion protein capable of binding to human or mammalian 4-1BB. The 4-1BB agonists or 4-1BB binding molecules may comprise an immunoglobulin heavy chain of any isotype (*e.g.,* IgG, IgE, IgM, IgD, IgA, and IgY), class (*e.g.,* IgG1, IgG2, IgG3, IgG4, IgA1 and IgA2) or subclass of immunoglobulin molecule. The 4-1BB agonist or 4-1BB binding molecule may have both a heavy and a light chain. As used herein, the term binding molecule also includes antibodies (including full length antibodies), monoclonal antibodies (including full length monoclonal antibodies), polyclonal antibodies, multispecific antibodies (*e.g.,* bispecific antibodies), human, humanized or chimeric antibodies, and antibody fragments, *e.g.*, Fab fragments, F(ab') fragments, fragments produced by a Fab expression library, epitope-binding fragments of any of the above, and engineered forms of antibodies, *e.g.*, scFv molecules, that bind to 4-1BB. In an embodiment, the 4-1BB agonist is an antigen binding protein that is a fully human antibody. In an embodiment, the 4-1BB agonist is an antigen binding protein that is a humanized antibody. In some embodiments, 4-1BB agonists for use in the presently disclosed methods and compositions include anti-4-1BB antibodies, human anti-4-1BB antibodies, mouse anti-4-1BB antibodies, mammalian anti-4-1BB antibodies, monoclonal anti-4-1BB antibodies, polyclonal anti-4-1BB antibodies, chimeric anti-4-1BB antibodies, anti-4-1BB adnectins, anti-4-1BB domain antibodies, single chain anti-4-1BB fragments, heavy chain anti-4-1BB fragments, light chain anti-4-1BB fragments, anti-4-1BB fusion proteins, and fragments, derivatives, conjugates, variants, or biosimilars thereof. Agonistic anti-4-1BB antibodies are known to induce strong immune responses. Lee, et al., PLOS One 2013, 8, e69677. In a preferred embodiment, the 4-1BB agonist is an agonistic, anti-4-1BB humanized or fully human monoclonal antibody (*i.e.,* an antibody derived from a single cell line). In an embodiment, the 4-1BB agonist is EU-101 (Eutilex Co. Ltd.), utomilumab, or urelumab, or a fragment, derivative, conjugate, variant, or biosimilar thereof. In a preferred embodiment, the 4-1BB agonist is utomilumab or urelumab, or a fragment, derivative, conjugate, variant, or biosimilar thereof.

In a preferred embodiment, the 4-1BB agonist or 4-1BB binding molecule may also be a fusion protein. In a preferred embodiment, a multimeric 4-1BB agonist, such as a trimeric or hexameric 4-1BB agonist (with three or six ligand binding domains), may induce superior receptor (4-1BBL) clustering and internal cellular signaling complex formation compared to an agonistic monoclonal antibody, which typically possesses two ligand binding domains. Trimeric (trivalent) or hexameric (or hexavalent) or greater fusion proteins comprising three TNFRSF binding domains and IgG 1- Fc and optionally further linking two or more of these fusion proteins are described, *e.g.,* in Gieffers, et al., Mol. Cancer Therapeutics 2013, 12, 2735-47.

Agonistic 4-1BB antibodies and fusion proteins are known to induce strong immune responses. In a preferred embodiment, the 4-1BB agonist is a monoclonal antibody or fusion protein that binds specifically to 4-1BB antigen in a manner sufficient to reduce toxicity. In some embodiments, the 4-1BB agonist is an agonistic 4-1BB monoclonal antibody or fusion protein that abrogates antibody-dependent cellular toxicity (ADCC), for example NK cell cytotoxicity. In some embodiments, the 4-1BB agonist is an agonistic 4-1BB monoclonal antibody or fusion protein that abrogates antibody-dependent cell phagocytosis (ADCP). In some embodiments, the 4-1BB agonist is an agonistic 4-1BB monoclonal antibody or fusion protein that abrogates complement-dependent cytotoxicity (CDC). In some embodiments, the 4-1BB agonist is an agonistic 4-1BB monoclonal antibody or fusion protein which abrogates Fc region functionality.

In some embodiments, the 4-1BB agonists are characterized by binding to human 4-1BB (SEQ ID NO:9) with high affinity and agonistic activity. In an embodiment, the 4-1BB agonist is a binding molecule that binds to human 4-1BB (SEQ ID NO:9). In an embodiment, the 4-1BB agonist is a binding molecule that binds to murine 4-1BB (SEQ ID NO:10).

The amino acid sequences of 4-1BB antigen to which a 4-1BB agonist or binding molecule binds are summarized in Table DD.

TABLE 5. Amino acid sequences of 4-1BB antigens.

| Identifier | Sequence (One-Letter Amino Acid Symbols) |
|---|---|
| SEQ ID NO:9 human 4-1BB, Tumor necrosis factor receptor superfamily, member 9 (Homo sapiens) | MGNSCYNIVA TLLLVLNFER TRSLQDPCSN CPAGTFCDNN RNQICSPCPP NSFSSAGGQR 60<br>TCDICRQCKG VFRTRKECSS TSNAECDCTP GFHCLGAGCS MCEQDCKQGQ ELTKKGCKDC 120<br>CFGTFNDQKR GICRPWTNCS LDGKSVLVNG TKERDVVCGP SPADLSPGAS SVTPPAPARE 180<br>PGHSPQIISF FLALTSTALL FLLFFLTLRF SVVKRGRKKL LYIFKQPFMR PVQTTQEEDG 240<br>CSCRFPEEEE GGCEL 255 |
| SEQ ID NO:10 murine 4-1BB, Tumor necrosis factor receptor superfamily, member 9 (Mus musculus) | MGNNCYNVVV IVLLLVGCEK VGAVQNSCDN CQPGTFCRKY NPVCKSCPPS TFSSIGGQPN 60<br>CNICRVCAGY FRFKKFCSST HNAECECIEG FHCLGPQCTR CEKDCRPGQE LTKQGCKTCS 120<br>LGTFNDQNGT GVCRPWTNCS LDGRSVLKTG TTEKDVVCGP PVVSFSPSTT ISVTPEGGPG 180<br>GHSLQVLTLF LALTSALLLA LIFITLLFSV LKWIRKKFPH IFKQPFKKTT GAAQEEDACS 240<br>CRCPQEEEGG GGGYEL 256 |

In some embodiments, the compositions, processes and methods described include a 4-1BB agonist that binds human or murine 4-1BB with a $K_D$ of about 100 pM or lower, binds human or murine 4-1BB with a $K_D$ of about 90 pM or lower, binds human or murine 4-1BB with a $K_D$ of about 80 pM or lower, binds human or murine 4-1BB with a $K_D$ of about 70 pM or lower, binds human or murine 4-1BB with a $K_D$ of about 60 pM or lower, binds human or murine 4-1BB with a $K_D$ of about 50 pM or lower, binds human or murine 4-1BB with a $K_D$ of about 40 pM or lower, or binds human or murine 4-1BB with a $K_D$ of about 30 pM or lower.

In some embodiments, the compositions, processes and methods described include a 4-1BB agonist that binds to human or murine 4-1BB with a $k_{assoc}$ of about 7.5 $\times$ $10^5$ 1/M·s or faster, binds to human or murine 4-1BB with a $k_{assoc}$ of about 7.5 $\times$ $10^5$ 1/M·s or faster, binds to human or murine 4-1BB with a $k_{assoc}$ of about 8 $\times$ $10^5$ 1/M·s or faster, binds to human or murine 4-1BB with a $k_{assoc}$ of about 8.5 $\times$ $10^5$ 1/M·s or faster, binds to human or murine 4-1BB with a $k_{assoc}$ of about 9 $\times$ $10^5$ 1/M·s or faster, binds to human or murine 4-1BB with a $k_{assoc}$ of about 9.5 $\times$ $10^5$ 1/M·s or faster, or binds to human or murine 4-1BB with a $k_{assoc}$ of about 1 $\times$ $10^6$ 1/M·s or faster.

In some embodiments, the compositions, processes and methods described include a 4-1BB agonist that binds to human or murine 4-1BB with a $k_{dissoc}$ of about 2 $\times$ $10^{-5}$ 1/s or slower, binds to human or murine 4-1BB with a $k_{dissoc}$ of about 2.1 $\times$ $10^{-5}$ 1/s or slower , binds to human or murine 4-1BB with a $k_{dissoc}$ of about 2.2 $\times$ $10^{-5}$ 1/s or slower, binds to human or murine 4-1BB with a $k_{dissoc}$ of about 2.3 $\times$ $10^{-5}$ 1/s or slower, binds to human or murine 4-1BB with a $k_{dissoc}$ of about 2.4 $\times$ $10^{-5}$ 1/s or slower, binds to human or murine 4-1BB with a $k_{dissoc}$ of about 2.5 $\times$ $10^{-5}$ 1/s or slower, binds to human or murine 4-1BB with a $k_{dissoc}$ of about 2.6 $\times$ $10^{-5}$ 1/s or slower or binds to human or murine 4-1BB with a $k_{dissoc}$ of about 2.7 $\times$ $10^{-5}$ 1/s or slower, binds to human or murine 4-1BB with a $k_{dissoc}$ of about 2.8 $\times$ $10^{-5}$ 1/s or slower, binds to human or murine 4-1BB with a $k_{dissoc}$ of about 2.9 $\times$ $10^{-5}$ 1/s or slower, or binds to human or murine 4-1BB with a $k_{dissoc}$ of about 3 $\times$ $10^{-5}$ 1/s or slower.

In some embodiments, the compositions, processes and methods described include a 4-1BB agonist that binds to human or murine 4-1BB with an $IC_{50}$ of about 10 nM or lower, binds to human or murine 4-1BB with an $IC_{50}$ of about 9 nM or lower, binds to human or murine 4-1BB with an $IC_{50}$ of about 8 nM or lower, binds to human or murine 4-1BB with an $IC_{50}$ of about 7 nM or lower, binds to human or murine 4-1BB with an $IC_{50}$ of about 6 nM or lower, binds to human or murine 4-1BB with an $IC_{50}$ of about 5 nM or lower, binds to human or murine 4-1BB with an $IC_{50}$ of about 4 nM or lower, binds to human or murine 4-1BB with an $IC_{50}$ of about 3 nM or lower, binds to human or murine 4-1BB with an $IC_{50}$ of about 2 nM or lower, or binds to human or murine 4-1BB with an $IC_{50}$ of about 1 nM or lower.

In a preferred embodiment, the 4-1BB agonist is utomilumab, also known as PF-05082566 or MOR-7480, or a fragment, derivative, variant, or biosimilar thereof. Utomilumab is available from Pfizer, Inc. Utomilumab is an immunoglobulin G2-lambda, anti-[*Homo sapiens* TNFRSF9 (tumor necrosis factor receptor (TNFR) superfamily member 9, 4-1BB, T cell antigen ILA, CD137)], *Homo sapiens* (fully human) monoclonal antibody. The amino acid sequences of utomilumab are set forth in Table EE. Utomilumab comprises glycosylation sites at Asn59 and Asn292; heavy chain intrachain disulfide bridges at positions 22-96 ($V_H$-$V_L$), 143-199 ($C_H1$-$C_L$), 256-316 ($C_H2$) and 362-420 ($C_H3$); light chain intrachain disulfide bridges at positions 22'-87' ($V_H$-$V_L$) and 136'-195' ($C_H1$-$C_L$); interchain heavy chain-heavy chain disulfide bridges at IgG2A isoform positions 218-218, 219-219, 222-222, and 225-225, at IgG2A/B isoform positions 218-130, 219-219, 222-222, and 225-225, and at IgG2B isoform positions 219-130 (2), 222-222, and 225-225; and interchain heavy chain-light chain disulfide bridges at IgG2A isoform positions 130-213' (2), IgG2A/B isoform positions 218-213' and 130-213', and at IgG2B isoform positions 218-213' (2). The preparation and properties of utomilumab and its variants and fragments are described in U.S. Patent Nos. 8,821,867; 8,337,850; and 9,468,678, and International Patent Application Publication No. WO 2012/032433 A1. Preclinical characteristics of utomilumab are described in Fisher, et al., Cancer Immunolog. & Immunother. 2012, 61, 1721-33. Current clinical trials of utomilumab in a variety of hematological and solid tumor indications include U.S. National Institutes of Health clinicaltrials.gov identifiers NCT02444793, NCT01307267, NCT02315066, and NCT02554812.

In an embodiment, a 4-1BB agonist comprises a heavy chain given by SEQ ID NO:11 and a light chain given by SEQ ID NO:12. In an embodiment, a 4-1BB agonist comprises heavy and light chains having the sequences shown in SEQ ID NO:11 and SEQ ID NO:12, respectively, or antigen binding fragments, Fab fragments, single-chain variable fragments (scFv), variants, or conjugates thereof. In an embodiment, a 4-1BB agonist comprises heavy and light chains that are each at least 99% identical to the sequences shown in SEQ ID NO:11 and SEQ ID NO:12, respectively. In an embodiment, a 4-1BB agonist comprises heavy and light chains that are each at least 98% identical to the sequences shown in SEQ ID NO:11 and SEQ ID NO:12, respectively. In an embodiment, a 4-1BB agonist comprises heavy and light chains that are each at least 97% identical to the sequences shown in SEQ ID NO:11 and SEQ ID NO:12, respectively. In an embodiment, a 4-1BB agonist comprises heavy and light chains that are each at least 96% identical to the sequences shown in SEQ ID NO:11 and SEQ ID NO:12, respectively. In an embodiment, a 4-1BB agonist comprises heavy and light chains that are each at least 95% identical to the sequences shown in SEQ ID NO:11 and SEQ ID NO:12, respectively.

In an embodiment, the 4-1BB agonist comprises the heavy and light chain CDRs or variable regions (VRs) of utomilumab. In an embodiment, the 4-1BB agonist heavy chain variable region ($V_H$) comprises the sequence shown in SEQ ID NO: 13, and the 4-1BB agonist light chain variable region ($V_L$) comprises the sequence shown in SEQ ID NO:14, and conservative amino acid substitutions thereof. In an embodiment, a 4-1BB agonist comprises $V_H$ and $V_L$ regions that are each at least 99% identical to the sequences shown in SEQ ID NO:13 and SEQ ID NO:14, respectively. In an embodiment, a 4-1BB agonist comprises $V_H$ and $V_L$ regions that are each at least 98% identical to the sequences shown in SEQ ID NO:13 and SEQ ID NO:14, respectively. In an embodiment, a 4-1BB agonist comprises $V_H$ and $V_L$ regions that are each at least 97% identical to the sequences shown in SEQ ID NO:13 and SEQ ID NO:14, respectively. In an embodiment, a 4-1BB agonist comprises $V_H$ and $V_L$ regions that are each at least 96% identical to the sequences shown in SEQ ID NO:13 and SEQ ID NO:14, respectively. In an embodiment, a 4-1BB agonist comprises $V_H$ and $V_L$ regions that are each at least 95% identical to the sequences shown in SEQ ID NO:13 and SEQ ID NO:14, respectively. In an embodiment, a 4-1BB agonist comprises an scFv antibody comprising $V_H$ and $V_L$ regions that are each at least 99% identical to the sequences shown in SEQ ID NO:13 and SEQ ID NO:14.

In an embodiment, a 4-1BB agonist comprises heavy chain CDR1, CDR2 and CDR3 domains having the sequences set forth in SEQ ID NO:15, SEQ ID NO:16, and SEQ ID NO:17, respectively, and conservative amino acid substitutions thereof, and light chain CDR1, CDR2 and CDR3 domains having the sequences set forth in SEQ ID NO:18, SEQ ID NO:19, and SEQ ID NO:20, respectively, and conservative amino acid substitutions thereof.

In an embodiment, the 4-1BB agonist is a 4-1BB agonist biosimilar monoclonal antibody approved by drug regulatory authorities with reference to utomilumab. In an embodiment, the biosimilar monoclonal antibody comprises an 4-1BB antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is utomilumab. In some embodiments, the one or more post-translational modifications are selected from one or more of: glycosylation, oxidation, deamidation, and truncation. In some embodiments, the biosimilar is a 4-1BB agonist antibody authorized or submitted for authorization, wherein the 4-1BB agonist antibody is provided in a formulation which differs from the formulations of a reference medicinal product or reference biological product, wherein the reference medicinal product or reference biological product is utomilumab. The 4-1BB agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is utomilumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is utomilumab.

TABLE 6. Amino acid sequences for 4-1BB agonist antibodies related to utomilumab.

| Identifier | Sequence (One-Letter Amino Acid Symbols) |
|---|---|
| SEQ ID NO:11 heavy chain for utomilumab | EVQLVQSGAE VKKPGESLRI SCKGSGYSFS TYWISWVRQM PGKGLEWMGK IYPGDSYTNY 60<br>SPSFQGQVTI SADKSISTAY LQWSSLKASD TAMYYCARGY GIFDYWGQGT LVTVSSASTK 120<br>GPSVFPLAPC SRSTSESTAA LGCLVKDYFP EPVTVSWNSG ALTSGVHTFP AVLQSSGLYS 180<br>LSSVVTVPSS NFGTQTYTCN VDHKPSNTKV DKTVERKCCV ECPPCPAPPV AGPSVFLFPP 240<br>KPKDTLMISR TPEVTCVVVD VSHEDPEVQF NWYVDGVEVH NAKTKPREEQ FNSTFRVVSV 300<br>LTVVHQDWLN GKEYKCKVSN KGLPAPIEKT ISKTKGQPRE PQVYTLPPSR EEMTKNQVSL 360<br>TCLVKGFYPS DIAVEWESNG QPENNYKTTP PMLDSDGSFF LYSKLTVDKS RWQQGNVFSC 420<br>SVMHEALHNH YTQKSLSLSP G 441 |

(continued)

| Identifier | Sequence (One-Letter Amino Acid Symbols) |
|---|---|
| SEQ ID NO:12 light chain for utomilumab | SYELTQPPSV SVSPGQTASI TCSGDNIGDQ YAHWYQQKPG QSPVLVIYQD KNRPSGIPER 60<br>FSGSNSGNTA TLTISGTQAM DEADYYCATY TGFGSLAVFG GGTKLTVLGQ PKAAPSVTLF 120<br>PPSSEELQAN KATLVCLISD FYPGAVTVAW KADSSPVKAG VETTTPSKQS NNKYAASSYL 180<br>SLTPEQWKSH RSYSCQVTHE GSTVEKTVAP TECS 214 |
| SEQ ID NO:13 heavy chain variable region for utomilumab | EVQLVQSGAE VKKPGESLRI SCKGSGYSFS TYWISWVRQM PGKGLEWMG KIYPGDSYTN 60<br>YSPSFQGQVT ISADKSISTA YLQWSSLKAS DTAMYYCARG YGIFDYWGQ GTLVTVSS 118 |
| SEQ ID NO:14 light chain variable region for utomilumab | SYELTQPPSV SVSPGQTASI TCSGDNIGDQ YAHWYQQKPG QSPVLVIYQD KNRPSGIPER 60<br>FSGSNSGNTA TLTISGTQAM DEADYYCATY TGFGSLAVFG GGTKLTVL 108 |
| SEQ ID NO:15 heavy chain CDR1 for utomilumab | STYWIS 6 |
| SEQ ID NO:16 heavy chain CDR2 for utomilumab | KIYPGDSYTN YSPSFQG 17 |
| SEQ ID NO:17 heavy chain CDR3 for utomilumab | RGYGIFDY 8 |
| SEQ ID NO:18 light chain CDR1 for utomilumab | SGDNIGDQYA H 11 |
| SEQ ID NO:19 light chain CDR2 for utomilumab | QDKNRPS 7 |
| SEQ ID NO:20 light chain CDR3 for utomilumab | ATYTGFGSLA V 11 |

In a preferred embodiment, the 4-1BB agonist is the monoclonal antibody urelumab, also known as BMS-663513 and 20H4.9.h4a, or a fragment, derivative, variant, or biosimilar thereof. Urelumab is available from Bristol-Myers Squibb, Inc., and Creative Biolabs, Inc. Urelumab is an immunoglobulin G4-kappa, anti-[*Homo sapiens* TNFRSF9 (tumor necrosis factor receptor superfamily member 9, 4-1BB, T cell antigen ILA, CD137)], *Homo sapiens* (fully human) monoclonal antibody. The amino acid sequences of urelumab are set forth in Table EE. Urelumab comprises N-glycosylation sites at positions 298 (and 298"); heavy chain intrachain disulfide bridges at positions 22-95 ($V_H$-$V_L$), 148-204 ($C_H1$-$C_L$), 262-322 ($C_H2$) and 368-426 ($C_H3$) (and at positions 22"-95", 148"-204", 262"-322", and 368"-426"); light chain intrachain disulfide bridges at positions 23'-88' ($V_H$-$V_L$) and 136'-196' ($C_H1$-$C_L$) (and at positions 23'''-88''' and 136'''-196'''); interchain heavy chain-heavy chain disulfide bridges at positions 227-227" and 230-230"; and interchain heavy chain-light chain disulfide bridges at 135-216' and 135"-216'''. The preparation and properties of urelumab and its variants and fragments are described in U.S. Patent Nos. 7,288,638 and 8,962,804. The preclinical and clinical characteristics of urelumab are described in Segal, et al., Clin. Cancer Res. 2016, available at http:/dx.doi.org/ 10.1158/1078-0432.CCR-16-1272. Current clinical trials of urelumab in a variety of hematological and solid tumor indications include U.S. National Institutes of Health clinicaltrials.gov identifiers NCT01775631, NCT02110082, NCT02253992, and NCT01471210.

In an embodiment, a 4-1BB agonist comprises a heavy chain given by SEQ ID NO:21 and a light chain given by SEQ ID NO:22. In an embodiment, a 4-1BB agonist comprises heavy and light chains having the sequences shown in SEQ ID NO:21 and SEQ ID NO:22, respectively, or antigen binding fragments, Fab fragments, single-chain variable fragments (scFv), variants, or conjugates thereof. In an embodiment, a 4-1BB agonist comprises heavy and light chains that are each at least 99% identical to the sequences shown in SEQ ID NO:21 and SEQ ID NO:22, respectively. In an embodiment, a 4-1BB agonist comprises heavy and light chains that are each at least 98% identical to the sequences shown in SEQ ID NO:21 and SEQ ID NO:22, respectively. In an embodiment, a 4-1BB agonist comprises heavy and light chains that are each at least 97% identical to the sequences shown in SEQ ID NO:21 and SEQ ID NO:22, respectively. In an embodiment, a 4-1BB agonist comprises heavy and light chains that are each at least 96% identical to the sequences shown in SEQ ID NO:21 and SEQ ID NO:22, respectively. In an embodiment, a 4-1BB agonist comprises heavy and light chains that are each at least 95% identical to the sequences shown in SEQ ID NO:21 and SEQ ID NO:22, respectively.

In an embodiment, the 4-1BB agonist comprises the heavy and light chain CDRs or variable regions (VRs) of urelumab. In an embodiment, the 4-1BB agonist heavy chain variable region ($V_H$) comprises the sequence shown in SEQ ID NO:23, and the 4-1BB agonist light chain variable region ($V_L$) comprises the sequence shown in SEQ ID NO:24, and conservative amino acid substitutions thereof. In an embodiment, a 4-1BB agonist comprises $V_H$ and $V_L$ regions that are each at least 99% identical to the sequences shown in SEQ ID NO:23 and SEQ ID NO:24, respectively. In an embodiment, a 4-1BB agonist comprises $V_H$ and $V_L$ regions that are each at least 98% identical to the sequences shown in SEQ ID NO:23 and SEQ ID NO:24, respectively. In an embodiment, a 4-1BB agonist comprises $V_H$ and $V_L$ regions that are each at least 97% identical to the sequences shown in SEQ ID NO:23 and SEQ ID NO:24, respectively. In an embodiment, a 4-1BB agonist comprises $V_H$ and $V_L$ regions that are each at least 96% identical to the sequences shown in SEQ ID NO:23 and SEQ ID NO:24, respectively. In an embodiment, a 4-1BB agonist comprises $V_H$ and $V_L$ regions that are each at least 95% identical to the sequences shown in SEQ ID NO:23 and SEQ ID NO:24, respectively. In an embodiment, a 4-1BB agonist comprises an scFv antibody comprising $V_H$ and $V_L$ regions that are each at least 99% identical to the sequences shown in SEQ ID NO:23 and SEQ ID NO:24.

In an embodiment, a 4-1BB agonist comprises heavy chain CDR1, CDR2 and CDR3 domains having the sequences set forth in SEQ ID NO:25, SEQ ID NO:26, and SEQ ID NO:27, respectively, and conservative amino acid substitutions thereof, and light chain CDR1, CDR2 and CDR3 domains having the sequences set forth in SEQ ID NO:28, SEQ ID NO:29, and SEQ ID NO:30, respectively, and conservative amino acid substitutions thereof.

In an embodiment, the 4-1BB agonist is a 4-1BB agonist biosimilar monoclonal antibody approved by drug regulatory authorities with reference to urelumab. In an embodiment, the biosimilar monoclonal antibody comprises an 4-1BB antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is urelumab. In some embodiments, the one or more post-translational modifications are selected from one or more of: glycosylation, oxidation, deamidation, and truncation. In some embodiments, the biosimilar is a 4-1BB agonist antibody authorized or submitted for authorization, wherein the 4-1BB agonist antibody is provided in a formulation which differs from the formulations of a reference medicinal product or reference biological product, wherein the reference medicinal product or reference biological product is urelumab. The 4-1BB agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is urelumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is urelumab.

TABLE 7: Amino acid sequences for 4-1BB agonist antibodies related to urelumab.

| Identifier | Sequence (One-Letter Amino Acid Symbols) |
|---|---|
| SEQ ID NO:21 heavy chain for urelumab | QVQLQQWGAG LLKPSETLSL TCAVYGGSFS GYYWSWIRQS PEKGLEWIGE INHGGYVTYN 60<br>PSLESRVTIS VDTSKNQFSL KLSSVTAADT AVYYCARDYG PGNYDWYFDL WGRGTLVTVS 120<br>SASTKGPSVF PLAPCSRSTS ESTAALGCLV KDYFPEPVTV SWNSGALTSG VHTFPAVLQS 180<br>SGLYSLSSVV TVPSSSLGTK TYTCNVDHKP SNTKVDKRVE SKYGPPCPPC PAPEFLGGPS 240<br>VFLFPPKPKD TLMISRTPEV TCVVVDVSQE DPEVQFNWYV DGVEVHNAKT KPREEQFNST 300<br>YRVVSVLTVL HQDWLNGKEY KCKVSNKGLP SSIEKTISKA KGQPREPQVY TLPPSQEEMT 360<br>KNQVSLTCLV KGFYPSDIAV EWESNGQPEN NYKTTPPVLD SDGSFFLYSR LTVDKSRWQE 420<br>GNVFSCSVMH EALHNHYTQK SLSLSLGK 448 |
| SEQ ID NO:22 light chain for urelumab | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPPALTF CGGTKVEIKR TVAAPSVFIF 120<br>PPSDEQLKSG TASVVCLLNN FYPREAKVQW KVDNALQSGN SQESVTEQDS KDSTYSLSST 180<br>LTLSKADYEK HKVYACEVTH QGLSSPVTKS FNRGEC 216 |
| SEQ ID NO:23 variable heavy chain for urelumab | MKHLWFFLLL VAAPRWVLSQ VQLQQWGAGL LKPSETLSLT CAVYGGSFSG YYWSWIRQSP 60<br>EKGLEWIGEI NHGGYVTYNP SLESRVTISV DTSKNQFSLK LSSVTAADTA VYYCARDYGP 120 |

(continued)

| Identifier | Sequence (One-Letter Amino Acid Symbols) |
|---|---|
| SEQ ID NO:24 variable light chain for urelumab | MEAPAQLLFL LLLWLPDTTG EIVLTQSPAT LSLSPGERAT LSCRASQSVS SYLAWYQQKP 60<br>GQAPRLLIYD ASNRATGIPA RFSGSGSGTD FTLTISSLEP EDFAVYYCQQ 110 |
| SEQ ID NO:25 heavy chain CDR1 for urelumab | GYYWS 5 |
| SEQ ID NO:26 heavy chain CDR2 for urelumab | EINHGGYVTY NPSLES 16 |
| SEQ ID NO:27 heavy chain CDR3 for urelumab | DYGPGNYDWY FDL 13 |
| SEQ ID NO:28 light chain CDR1 for urelumab | RASQSVSSYL A 11 |
| SEQ ID NO:29 light chain CDR2 for urelumab | DASNRAT 7 |
| SEQ ID NO:30 light chain CDR3 for urelumab | QQRSDWPPAL T 11 |

In an embodiment, the 4-1BB agonist is selected from the group consisting of 1D8, 3Elor, 4B4 (BioLegend 309809), H4-1BB-M127 (BD Pharmingen 552532), BBK2 (Thermo Fisher MS621PABX), 145501 (Leinco Technologies B591), the antibody produced by cell line deposited as ATCC No. HB-11248 and disclosed in U.S. Patent No. 6,974,863, 5F4 (BioLegend 31 1503), C65-485 (BD Pharmingen 559446), antibodies disclosed in U.S. Patent Application Publication No. US 2005/0095244, antibodies disclosed in U.S. Patent No. 7,288,638 (such as 20H4.9-IgGl (BMS-663031)), antibodies disclosed in U.S. Patent No. 6,887,673 (such as 4E9 or BMS-554271), antibodies disclosed in U.S. Patent No. 7,214,493, antibodies disclosed in U.S. Patent No. 6,303,121, antibodies disclosed in U.S. Patent No. 6,569,997, antibodies disclosed in U.S. Patent No. 6,905,685 (such as 4E9 or BMS-554271), antibodies disclosed in U.S. Patent No. 6,362,325 (such as 1D8 or BMS-469492; 3H3 or BMS-469497; or 3El), antibodies disclosed in U.S. Patent No. 6,974,863 (such as 53A2); antibodies disclosed in U.S. Patent No. 6,210,669 (such as 1D8, 3B8, or 3El), antibodies described in U.S. Patent No. 5,928,893, antibodies disclosed in U.S. Patent No. 6,303,121, antibodies disclosed in U.S. Patent No. 6,569,997, antibodies disclosed in International Patent Application Publication Nos. WO 2012/177788, WO 2015/119923, and WO 2010/042433, and fragments, derivatives, conjugates, variants, or biosimilars thereof.

In an embodiment, the 4-1BB agonist is a 4-1BB agonistic fusion protein described in International Patent Application Publication Nos. WO 2008/025516 A1, WO 2009/007120 A1, WO 2010/003766 A1, WO 2010/010051 A1, and WO 2010/078966 A1; U.S. Patent Application Publication Nos. US 2011/0027218 A1, US 2015/0126709 A1, US 2011/0111494 A1, US 2015/0110734 A1, and US 2015/0126710 A1; and U.S. Patent Nos. 9,359,420, 9,340,599, 8,921,519, and 8,450,460.

In an embodiment, the 4-1BB agonist is a 4-1BB agonistic fusion protein as depicted in Structure I-A (C-terminal Fc-antibody fragment fusion protein) or Structure I-B (N-terminal Fc-antibody fragment fusion protein), or a fragment, derivative, conjugate, variant, or biosimilar thereof:

(I-A)

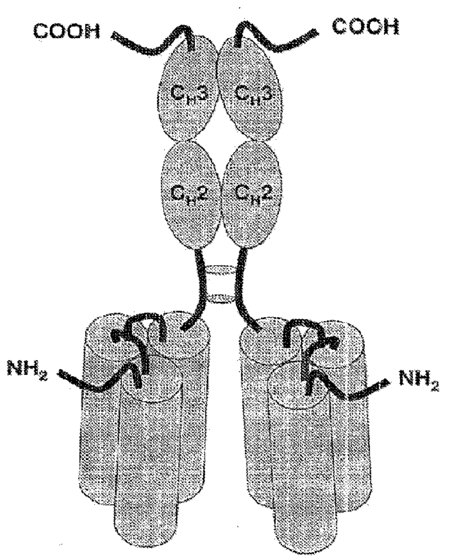


(I-B)

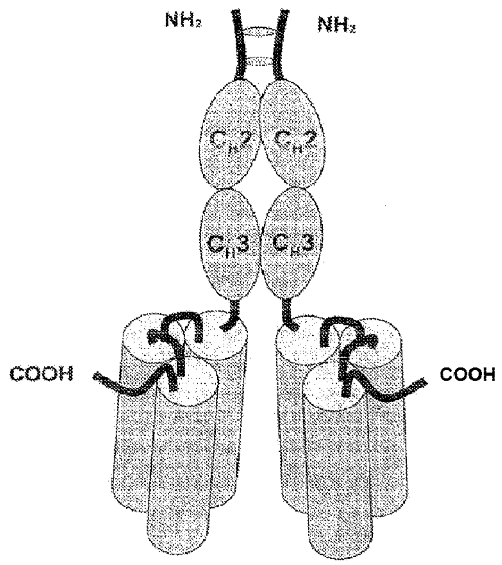


In structures I-A and I-B, the cylinders refer to individual polypeptide binding domains. Structures I-A and I-B comprise three linearly-linked TNFRSF binding domains derived from *e.g.,* 4-1BBL or an antibody that binds 4-1BB, which fold to form a trivalent protein, which is then linked to a second triavelent protein through IgG1-Fc (including $C_H3$ and $C_H2$ domains) is then used to link two of the trivalent proteins together through disulfide bonds (small elongated ovals), stabilizing the structure and providing an agonists capable of bringing together the intracellular signaling domains of the six receptors and signaling proteins to form a signaling complex. The TNFRSF binding domains denoted as cylinders may be scFv domains comprising, *e.g.,* a $V_H$ and a $V_L$ chain connected by a linker that may comprise hydrophilic residues and Gly and Ser sequences for flexibility, as well as Glu and Lys for solubility. Any scFv domain design may be used, such as those described in de Marco, Microbial Cell Factories, 2011, 10, 44; Ahmad, et al., Clin. & Dev. Immunol. 2012, 980250; or Monnier, et al., Antibodies, 2013, 2, 193-208. Fusion protein structures of this form are described in U.S. Patent Nos. 9,359,420, 9,340,599, 8,921,519, and 8,450,460.

Amino acid sequences for the other polypeptide domains of structure I-A are given in Table GG. The Fc domain preferably comprises a complete constant domain (amino acids 17-230 of SEQ ID NO:31) the complete hinge domain (amino acids 1-16 of SEQ ID NO:31) or a portion of the hinge domain (*e.g.,* amino acids 4-16 of SEQ ID NO:31). Preferred linkers for connecting a C-terminal Fc-antibody may be selected from the embodiments given in SEQ ID NO:32 to SEQ ID NO:41, including linkers suitable for fusion of additional polypeptides.

TABLE 8: Amino acid sequences for TNFRSF fusion proteins, including 4-1BB fusion proteins, with C-terminal Fc-antibody fragment fusion protein design (structure I-A).

| Identifier | Sequence (One-Letter Amino Acid Symbols) |
|---|---|
| SEQ ID NO: 31 Fc domain | KSCDKTHTCP PCPAPELLGG PSVFLFPPKP KDTLMISRTP EVTCVVVDVS HEDPEVKFNW 60<br>YVDGVEVHNA KTKPREEQYN STYRVVSVLT VLHQDWLNGK EYKCKVSNKA LPAPIEKTIS 120<br>KAKGQPREPQ VYTLPPSREE MTKNQVSLTC LVKGFYPSDI AVEWESNGQP ENNYKTTPPV 180<br>LDSDGSFFLY SKLTVDKSRW QQGNVFSCSV MHEALHNHYT QKSLSLSPGK 230 |
| SEQ ID NO: 32 linker | GGPGSSKSCD KTHTCPPCPA PE 22 |
| SEQ ID NO: 33 linker | GGSGSSKSCD KTHTCPPCPA PE 22 |
| SEQ ID NO: 34 linker | GGPGSSSSSS SKSCDKTHTC PPCPAPE 27 |

(continued)

| Identifier | Sequence (One-Letter Amino Acid Symbols) |
|---|---|
| SEQ ID NO: 35 linker | GGSGSSSSSS SKSCDKTHTC PPCPAPE 27 |
| SEQ ID NO: 36 linker | GGPGSSSSSS SSSKSCDKTH TCPPCPAPE 29 |
| SEQ ID NO: 37 linker | GGSGSSSSSS SSSKSCDKTH TCPPCPAPE 29 |
| SEQ ID NO: 38 linker | GGPGSSGSGS SDKTHTCPPC PAPE 24 |
| SEQ ID NO: 39 linker | GGPGSSGSGS DKTHTCPPCP APE 23 |
| SEQ ID NO: 40 linker | GGPSSSGSDK THTCPPCPAP E 21 |
| SEQ ID NO: 41 linker | GGSSSSSSSS GSDKTHTCPP CPAPE 25 |

Amino acid sequences for the other polypeptide domains of structure I-B are given in Table 9. If an Fc antibody fragment is fused to the N-terminus of an TNRFSF fusion protein as in structure I-B, the sequence of the Fc module is preferably that shown in SEQ ID NO:42, and the linker sequences are preferably selected from those embodiments set forth in SED ID NO:43 to SEQ ID NO:45.

TABLE 9: Amino acid sequences for TNFRSF fusion proteins, including 4-1BB fusion proteins, with N-terminal Fc-antibody fragment fusion protein design (structure I-B).

| Identifier | Sequence (One-Letter Amino Acid Symbols) |
|---|---|
| SEQ ID NO: 42 Fc domain | METDTLLLWV LLLWVPAGNG DKTHTCPPCP APELLGGPSV FLFPPKPKDT LMISRTPEVT 60<br>CVVVDVSHED PEVKFNWYVD GVEVHNAKTK PREEQYNSTY RVVSVLTVLH QDWLNGKEYK 120<br>CKVSNKALPA PIEKTISKAK GQPREPQVYT LPPSREEMTK NQVSLTCLVK GFYPSDIAVE 180<br>WESNGQPENN YKTTPPVLDS DGSFFLYSKL TVDKSRWQQG NVFSCSVMHE ALHNHYTQKS 240<br>LSLSPG 246 |
| SEQ ID NO: 43 linker | SGSGSGSGSG S 11 |
| SEQ ID NO: 44 linker | SSSSSSGSGS GS 12 |
| SEQ ID NO: 45 linker | SSSSSSGSGS GSGSGS 16 |

In an embodiment, a 4-1BB agonist fusion protein according to structures I-A or I-B comprises one or more 4-1BB binding domains selected from the group consisting of a variable heavy chain and variable light chain of utomilumab, a variable heavy chain and variable light chain of urelumab, a variable heavy chain and variable light chain of utomilumab, a variable heavy chain and variable light chain selected from the variable heavy chains and variable light chains described in Table GG, any combination of a variable heavy chain and variable light chain of the foregoing, and fragments, derivatives, conjugates, variants, and biosimilars thereof.

In an embodiment, a 4-1BB agonist fusion protein according to structures I-A or I-B comprises one or more 4-1BB binding domains comprising a 4-1BBL sequence. In an embodiment, a 4-1BB agonist fusion protein according to structures I-A or I-B comprises one or more 4-1BB binding domains comprising a sequence according to SEQ ID NO:46. In an embodiment, a 4-1BB agonist fusion protein according to structures I-A or I-B comprises one or more 4-1BB binding domains comprising a soluble 4-1BBL sequence. In an embodiment, a 4-1BB agonist fusion protein according to structures I-A or I-B comprises one or more 4-1BB binding domains comprising a sequence according to SEQ ID NO:47.

In an embodiment, a 4-1BB agonist fusion protein according to structures I-A or I-B comprises one or more 4-1BB binding domains that is a scFv domain comprising $V_H$ and $V_L$ regions that are each at least 95% identical to the sequences shown in SEQ ID NO:13 and SEQ ID NO:14, respectively, wherein the $V_H$ and $V_L$ domains are connected by a linker. In an embodiment, a 4-1BB agonist fusion protein according to structures I-A or I-B comprises one or more 4-1BB binding domains that is a scFv domain comprising $V_H$ and $V_L$ regions that are each at least 95% identical to the sequences shown in SEQ ID NO:23 and SEQ ID NO:24, respectively, wherein the $V_H$ and $V_L$ domains are connected by a linker. In an embodiment, a 4-1BB agonist fusion protein according to structures I-A or I-B comprises one or more 4-1BB binding domains that is a scFv domain comprising $V_H$ and $V_L$ regions that are each at least 95% identical to the $V_H$ and $V_L$ sequences given in Table II, wherein the $V_H$ and $V_L$ domains are connected by a linker.

TABLE 10: Additional polypeptide domains useful as 4-1BB binding domains in fusion proteins or as scFv 4-1BB agonist antibodies.

| Identifier | Sequence (One-Letter Amino Acid Symbols) |
|---|---|
| SEQ ID NO:46<br>4-1BBL | MEYASDASLD PEAPWPPAPR ARACRVLPWA LVAGLLLLLL LAAACAVFLA CPWAVSGARA 60<br>SPGSAASPRL REGPELSPDD PAGLLDLRQG MFAQLVAQNV LLIDGPLSWY SDPGLAGVSL 120<br>TGGLSYKEDT KELVVAKAGV YYVFFQLELR RVVAGEGSGS VSLALHLQPL RSAAGAAALA 180<br>LTVDLPPASS EARNSAFGFQ GRLLHLSAGQ RLGVHLHTEA RARHAWQLTQ GATVLGLFRV 240<br>TPEIPAGLPS PRSE 254 |
| SEQ ID NO:47<br>4-1BBL soluble domain | LRQGMFAQLV AQNVLLIDGP LSWYSDPGLA GVSLTGGLSY KEDTKELVVA KAGVYYVFFQ 60<br>LELRRVVAGE GSGSVSLALH LQPLRSAAGA AALALTVDLP PASSEARNSA FGFQGRLLHL 120<br>SAGQRLGVHL HTEARARHAW QLTQGATVLG LFRVTPEIPA GLPSPRSE 168 |
| SEQ ID NO:48<br>variable heavy chain for 4B4-1-1 version 1 | QVQLQQPGAE LVKPGASVKL SCKASGYTFS SYWMHWVKQR PGQVLEWIGE INPGNGHTNY 60<br>NEKFKSKATL TVDKSSSTAY MQLSSLTSED SAVYYCARSF TTARGFAYWG QGTLVTVS 118 |
| SEQ ID NO:49<br>variable light chain for 4B4-1-1 version 1 | DIVMTQSPAT QSVTPGDRVS LSCRASQTIS DYLHWYQQKS HESPRLLIKY ASQSISGIPS 60<br>RFSGSGSGSD FTLSINSVEP EDVGVYYCQD GHSFPPTFGG GTKLEIK 107 |
| SEQ ID NO:50<br>variable heavy chain for 4B4-1-1 version 2 | QVQLQQPGAE LVKPGASVKL SCKASGYTFS SYWMHWVKQR PGQVLEWIGE INPGNGHTNY 60<br>NEKFKSKATL TVDKSSSTAY MQLSSLTSED SAVYYCARSF TTARGFAYWG QGTLVTVSA 119 |
| SEQ ID NO:51<br>variable light chain for 4B4-1-1 version 2 | DIVMTQSPAT QSVTPGDRVS LSCRASQTIS DYLHWYQQKS HESPRLLIKY ASQSISGIPS 60<br>RFSGSGSGSD FTLSINSVEP EDVGVYYCQD GHSFPPTFGG GTKLEIKR 108 |
| SEQ ID NO:52<br>variable heavy chain for H39E3-2 | MDWTWRILFL VAAATGAHSE VQLVESGGGL VQPGGSLRLS CAASGFTFSD YWMSWVRQAP 60<br>GKGLEWVADI KNDGSYTNYA PSLTNRFTIS RDNAKNSLYL QMNSLRAEDT AVYYCARELT 120 |
| SEQ ID NO:53<br>variable light chain for H39E3-2 | MEAPAQLLFL LLLWLPDTTG DIVMTQSPDS LAVSLGERAT INCKSSQSLL SSGNQKNYL 60<br>WYQQKPGQPP KLLIYYASTR QSGVPDRFSG SGSGTDFTLT ISSLQAEDVA 110 |

In an embodiment, the 4-1BB agonist is a 4-1BB agonistic single-chain fusion polypeptide comprising (i) a first soluble 4-1BB binding domain, (ii) a first peptide linker, (iii) a second soluble 4-1BB binding domain, (iv) a second peptide linker, and (v) a third soluble 4-1BB binding domain, further comprising an additional domain at the N-terminal and/or C-terminal end, and wherein the additional domain is a Fab or Fc fragment domain. In an embodiment, the 4-1BB agonist is a 4-1BB agonistic single-chain fusion polypeptide comprising (i) a first soluble 4-1BB binding domain, (ii) a first peptide linker, (iii) a second soluble 4-1BB binding domain, (iv) a second peptide linker, and (v) a third soluble 4-1BB binding domain, further comprising an additional domain at the N-terminal and/or C-terminal end, wherein the additional domain is a Fab or Fc fragment domain, wherein each of the soluble 4-1BB domains lacks a stalk region (which contributes to trimerisation and provides a certain distance to the cell membrane, but is not part of the 4-1BB binding domain) and the first and the second peptide linkers independently have a length of 3-8 amino acids.

In an embodiment, the 4-1BB agonist is a 4-1BB agonistic single-chain fusion polypeptide comprising (i) a first soluble tumor necrosis factor (TNF) superfamily cytokine domain, (ii) a first peptide linker, (iii) a second soluble TNF superfamily cytokine domain, (iv) a second peptide linker, and (v) a third soluble TNF superfamily cytokine domain, wherein each of the soluble TNF superfamily cytokine domains lacks a stalk region and the first and the second peptide linkers independently have a length of 3-8 amino acids, and wherein each TNF superfamily cytokine domain is a 4-1BB binding domain.

In an embodiment, the 4-1BB agonist is a 4-1BB agonistic scFv antibody comprising any of the foregoing $V_H$ domains linked to any of the foregoing $V_L$ domains.

In an embodiment, the 4-1BB agonist is BPS Bioscience 4-1BB agonist antibody catalog no. 79097-2, commercially available from BPS Bioscience, San Diego, CA, USA. In an embodiment, the 4-1BB agonist is Creative Biolabs 4-1BB agonist antibody catalog no. MOM-18179, commercially available from Creative Biolabs, Shirley, NY, USA.

3. OX40 (CD134) AGONISTS

In an embodiment, the TNFRSF agonist is an OX40 (CD134) agonist. The OX40 agonist may be any OX40 binding molecule known in the art. The OX40 binding molecule may be a monoclonal antibody or fusion protein capable of binding to human or mammalian OX40. The OX40 agonists or OX40 binding molecules may comprise an immunoglobulin heavy chain of any isotype (*e.g.,* IgG, IgE, IgM, IgD, IgA, and IgY), class (*e.g.,* IgG1, IgG2, IgG3, IgG4, IgA1 and IgA2) or subclass of immunoglobulin molecule. The OX40 agonist or OX40 binding molecule may have both a heavy and a light chain. As used herein, the term binding molecule also includes antibodies (including full length antibodies), monoclonal antibodies (including full length monoclonal antibodies), polyclonal antibodies, multispecific antibodies (*e.g.,* bispecific antibodies), human, humanized or chimeric antibodies, and antibody fragments, *e.g.*, Fab fragments, F(ab') fragments, fragments produced by a Fab expression library, epitope-binding fragments of any of the above, and engineered forms of antibodies, *e.g.*, scFv molecules, that bind to OX40. In an embodiment, the OX40 agonist is an antigen binding protein that is a fully human antibody. In an embodiment, the OX40 agonist is an antigen binding protein that is a humanized antibody. In some embodiments, OX40 agonists for use in the presently disclosed methods and compositions include anti-OX40 antibodies, human anti-OX40 antibodies, mouse anti-OX40 antibodies, mammalian anti-OX40 antibodies, monoclonal anti-OX40 antibodies, polyclonal anti-OX40 antibodies, chimeric anti-OX40 antibodies, anti-OX40 adnectins, anti-OX40 domain antibodies, single chain anti-OX40 fragments, heavy chain anti-OX40 fragments, light chain anti-OX40 fragments, anti-OX40 fusion proteins, and fragments, derivatives, conjugates, variants, or biosimilars thereof. In a preferred embodiment, the OX40 agonist is an agonistic, anti-OX40 humanized or fully human monoclonal antibody (*i.e.*, an antibody derived from a single cell line).

In a preferred embodiment, the OX40 agonist or OX40 binding molecule may also be a fusion protein. OX40 fusion proteins comprising an Fc domain fused to OX40L are described, for example, in Sadun, et al., J. Immunother. 2009, 182, 1481-89. In a preferred embodiment, a multimeric OX40 agonist, such as a trimeric or hexameric OX40 agonist (with three or six ligand binding domains), may induce superior receptor (OX40L) clustering and internal cellular signaling complex formation compared to an agonistic monoclonal antibody, which typically possesses two ligand binding domains. Trimeric (trivalent) or hexameric (or hexavalent) or greater fusion proteins comprising three TNFRSF binding domains and IgG 1- Fc and optionally further linking two or more of these fusion proteins are described, *e.g.,* in Gieffers, et al., Mol. Cancer Therapeutics 2013, 12, 2735-47.

Agonistic OX40 antibodies and fusion proteins are known to induce strong immune responses. Curti, et al., Cancer Res. 2013, 73, 7189-98. In a preferred embodiment, the OX40 agonist is a monoclonal antibody or fusion protein that binds specifically to OX40 antigen in a manner sufficient to reduce toxicity. In some embodiments, the OX40 agonist is an agonistic OX40 monoclonal antibody or fusion protein that abrogates antibody-dependent cellular toxicity (ADCC), for example NK cell cytotoxicity. In some embodiments, the OX40 agonist is an agonistic OX40 monoclonal antibody or fusion protein that abrogates antibody-dependent cell phagocytosis (ADCP). In some embodiments, the OX40 agonist is an agonistic OX40 monoclonal antibody or fusion protein that abrogates complement-dependent cytotoxicity (CDC). In some embodiments, the OX40 agonist is an agonistic OX40 monoclonal antibody or fusion protein which abrogates Fc region functionality.

In some embodiments, the OX40 agonists are characterized by binding to human OX40 (SEQ ID NO:54) with high affinity and agonistic activity. In an embodiment, the OX40 agonist is a binding molecule that binds to human OX40 (SEQ ID NO:54). In an embodiment, the OX40 agonist is a binding molecule that binds to murine OX40 (SEQ ID NO:55). The amino acid sequences of OX40 antigen to which an OX40 agonist or binding molecule binds are summarized in Table 11.

TABLE 11: Amino acid sequences of OX40 antigens.

| Identifier | Sequence (One-Letter Amino Acid Symbols) |
|---|---|
| SEQ ID NO:54<br>human OX40<br>(Homo sapiens) | MCVGARRLGR GPCAALLLLG LGLSTVTGLH CVGDTYPSND RCCHECRPGN GMVSRCSRSQ 60<br>NTVCRPCGPG FYNDVVSSKP CKPCTWCNLR SGSERKQLCT ATQDTVCRCR AGTQPLDSYK 120<br>PGVDCAPCPP GHFSPGDNQA CKPWTNCTLA GKHTLQPASN SSDAICEDRD PPATQPQETQ 180<br>GPPARPITVQ PTEAWPRTSQ GPSTRPVEVP GGRAVAAILG LGLVLGLLGP LAILLALYLL 240<br>RRDQRLPPDA HKPPGGGSFR TPIQEEQADA HSTLAKI 277 |
| SEQ ID NO:55<br>murine OX40 (Mus musculus) | MYVWVQQPTA LLLLGLTLGV TARRLNCVKH TYPSGHKCCR ECQPGHGMVS RCDHTRDTLC 60<br>HPCETGFYNE AVNYDTCKQC TQCNHRSGSE LKQNCTPTQD TVCRCRPGTQ PRQDSGYKLG 120<br>VDCVPCPPGH FSPGNNQACK PWTNCTLSGK QTRHPASDSL DAVCEDRSLL ATLLWETQRP 180<br>TFRPTTVQST TVWPRTSELP SPPTLVTPEG PAFAVLLGLG LGLLAPLTVL LALYLLRKAW 240<br>RLPNTPKPCW GNSFRTPIQE EHTDAHFTLA KI 272 |

In some embodiments, the compositions, processes and methods described include a OX40 agonist that binds human or murine OX40 with a $K_D$ of about 100 pM or lower, binds human or murine OX40 with a $K_D$ of about 90 pM or lower, binds human or murine OX40 with a $K_D$ of about 80 pM or lower, binds human or murine OX40 with a $K_D$ of about 70 pM or lower, binds human or murine OX40 with a $K_D$ of about 60 pM or lower, binds human or murine OX40 with a $K_D$ of about 50 pM or lower, binds human or murine OX40 with a $K_D$ of about 40 pM or lower, or binds human or murine OX40 with a $K_D$ of about 30 pM or lower.

In some embodiments, the compositions, processes and methods described include a OX40 agonist that binds to human or murine OX40 with a $k_{assoc}$ of about 7.5 $\times$ $10^5$ 1/M·s or faster, binds to human or murine OX40 with a $k_{assoc}$ of about 7.5 $\times$ $10^5$ 1/M·s or faster, binds to human or murine OX40 with a $k_{assoc}$ of about 8 $\times$ $10^5$ 1/M·s or faster, binds to human or murine OX40 with a $k_{assoc}$ of about 8.5 $\times$ $10^5$ 1/M·s or faster, binds to human or murine OX40 with a $k_{assoc}$ of about 9 $\times$ $10^5$ 1/M·s or faster, binds to human or murine OX40 with a $k_{assoc}$ of about 9.5 $\times$ $10^5$ 1/M·s or faster, or binds to human or murine OX40 with a $k_{assoc}$ of about 1 $\times$ $10^6$ 1/M·s or faster.

In some embodiments, the compositions, processes and methods described include a OX40 agonist that binds to human or murine OX40 with a $k_{dissoc}$ of about 2 $\times$ $10^{-5}$ 1/s or slower, binds to human or murine OX40 with a $k_{dissoc}$ of about 2.1 $\times$ $10^{-5}$ 1/s or slower , binds to human or murine OX40 with a $k_{dissoc}$ of about 2.2 $\times$ $10^{-5}$ 1/s or slower, binds to human or murine OX40 with a $k_{dissoc}$ of about 2.3 $\times$ $10^{-5}$ 1/s or slower, binds to human or murine OX40 with a $k_{dissoc}$ of about 2.4 $\times$ $10^{-5}$ 1/s or slower, binds to human or murine OX40 with a $k_{dissoc}$ of about 2.5 $\times$ $10^{-5}$ 1/s or slower, binds to human or murine OX40 with a $k_{dissoc}$ of about 2.6 $\times$ $10^{-5}$ 1/s or slower or binds to human or murine OX40 with a $k_{dissoc}$ of about 2.7 $\times$ $10^{-5}$ 1/s or slower, binds to human or murine OX40 with a $k_{dissoc}$ of about 2.8 $\times$ $10^{-5}$ 1/s or slower, binds to human or murine OX40 with a $k_{dissoc}$ of about 2.9 $\times$ $10^{-5}$ 1/s or slower, or binds to human or murine OX40 with a $k_{dissoc}$ of about 3 $\times$ $10^{-5}$ 1/s or slower.

In some embodiments, the compositions, processes and methods described include OX40 agonist that binds to human or murine OX40 with an $IC_{50}$ of about 10 nM or lower, binds to human or murine OX40 with an $IC_{50}$ of about 9 nM or lower, binds to human or murine OX40 with an $IC_{50}$ of about 8 nM or lower, binds to human or murine OX40 with an $IC_{50}$ of about 7 nM or lower, binds to human or murine OX40 with an $IC_{50}$ of about 6 nM or lower, binds to human or murine OX40 with an $IC_{50}$ of about 5 nM or lower, binds to human or murine OX40 with an $IC_{50}$ of about 4 nM or lower, binds to human or murine OX40 with an $IC_{50}$ of about 3 nM or lower, binds to human or murine OX40 with an $IC_{50}$ of about 2 nM or lower, or binds to human or murine OX40 with an $IC_{50}$ of about 1 nM or lower.

In some embodiments, the OX40 agonist is tavolixizumab, also known as MEDI0562 or MEDI-0562. Tavolixizumab is available from the MedImmune subsidiary of AstraZeneca, Inc. Tavolixizumab is immunoglobulin G1-kappa, anti-[*Homo sapiens* TNFRSF4 (tumor necrosis factor receptor (TNFR) superfamily member 4, OX40, CD134)], humanized and chimeric monoclonal antibody. The amino acid sequences of tavolixizumab are set forth in Table KK. Tavolixizumab comprises N-glycosylation sites at positions 301 and 301", with fucosylated complex bi-antennary CHO-type glycans; heavy chain intrachain disulfide bridges at positions 22-95 ($V_H$-$V_L$), 148-204 ($C_H$1-$C_L$), 265-325 ($C_H$2) and 371-429 ($C_H$3) (and at positions 22"-95", 148"-204", 265"-325", and 371"-429"); light chain intrachain disulfide bridges at positions 23'-88' ($V_H$-$V_L$) and 134'-194' ($C_H$1-$C_L$) (and at positions 23‴-88‴ and 134‴-194‴); interchain heavy chain-heavy chain disulfide bridges at positions 230-230" and 233-233"; and interchain heavy chain-light chain disulfide bridges at 224-214' and 224"-214‴. Current clinical trials of tavolixizumab in a variety of solid tumor indications include U.S. National Institutes of Health clinicaltrials.gov identifiers NCT02318394 and NCT02705482.

In an embodiment, a OX40 agonist comprises a heavy chain given by SEQ ID NO:56 and a light chain given by SEQ ID NO:57. In an embodiment, a OX40 agonist comprises heavy and light chains having the sequences shown in SEQ ID NO:56 and SEQ ID NO:57, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:56 and SEQ ID NO:57, respectively. In an embodiment, a OX40 agonist comprises heavy and light chains that are each at least 98% identical to the sequences shown in SEQ ID NO:56 and SEQ ID NO:57, respectively. In an embodiment, a OX40 agonist comprises heavy and light chains that are each at least 97% identical to the sequences shown in SEQ ID NO:56 and SEQ ID NO:57, respectively. In an embodiment, a OX40 agonist comprises heavy and light chains that are each at least 96% identical to the sequences shown in SEQ ID NO:56 and SEQ ID NO:57, respectively. In an embodiment, a OX40 agonist comprises heavy and light chains that are each at least 95% identical to the sequences shown in SEQ ID NO:56 and SEQ ID NO:57, respectively.

In an embodiment, the OX40 agonist comprises the heavy and light chain CDRs or variable regions (VRs) of tavolixizumab. In an embodiment, the OX40 agonist heavy chain variable region ($V_H$) comprises the sequence shown in SEQ ID NO:58, and the OX40 agonist light chain variable region ($V_L$) comprises the sequence shown in SEQ ID NO:59, and conservative amino acid substitutions thereof. In an embodiment, a OX40 agonist comprises $V_H$ and $V_L$ regions that are each at least 99% identical to the sequences shown in SEQ ID NO:58 and SEQ ID NO:59, respectively. In an embodiment, a OX40 agonist comprises $V_H$ and $V_L$ regions that are each at least 98% identical to the sequences shown in SEQ ID NO:58 and SEQ ID NO:59, respectively. In an embodiment, a OX40 agonist comprises $V_H$ and $V_L$ regions that are each at least 97% identical to the sequences shown in SEQ ID NO:58 and SEQ ID NO:59, respectively. In an embodiment, a OX40 agonist comprises $V_H$ and $V_L$ regions that are each at least 96% identical to the sequences shown in SEQ ID NO:58 and SEQ ID NO:59, respectively. In an embodiment, a OX40 agonist comprises $V_H$ and $V_L$ regions that are each at least 95% identical to the sequences shown in SEQ ID NO:58 and SEQ ID NO:59, respectively. In an embodiment, an OX40 agonist comprises an scFv antibody comprising $V_H$ and $V_L$ regions that are each at least 99% identical to the sequences shown in SEQ ID NO:58 and SEQ ID NO:59.

In an embodiment, a OX40 agonist comprises heavy chain CDR1, CDR2 and CDR3 domains having the sequences set forth in SEQ ID NO:60, SEQ ID NO:61, and SEQ ID NO:62, respectively, and conservative amino acid substitutions thereof, and light chain CDR1, CDR2 and CDR3 domains having the sequences set forth in SEQ ID NO:63, SEQ ID NO:64, and SEQ ID NO:65, respectively, and conservative amino acid substitutions thereof.

In an embodiment, the OX40 agonist is a OX40 agonist biosimilar monoclonal antibody approved by drug regulatory authorities with reference to tavolixizumab. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is tavolixizumab. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is tavolixizumab. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is tavolixizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is tavolixizumab.

TABLE 12: Amino acid sequences for OX40 agonist antibodies related to tavolixizumab.

| Identifier | Sequence (One-Letter Amino Acid Symbols) |
|---|---|
| SEQ ID NO:56 heavy chain for tavolixizumab | QVQLQESGPG LVKPSQTLSL TCAVYGGSFS SGYWNWIRKH PGKGLEYIGY ISYNGITYHN 60<br>PSLKSRITIN RDTSKNQYSL QLNSVTPEDT AVYYCARYKY DYDGGHAMDY WGQGTLVTVS 120<br>SASTKGPSVF PLAPSSKSTS GGTAALGCLV KDYFPEPVTV SWNSGALTSG VHTFPAVLQS 180<br>SGLYSLSSVV TVPSSSLGTQ TYICNVNHKP SNTKVDKRVE PKSCDKTHTC PPCPAPELLG 240<br>GPSVFLFPPK PKDTLMISRT PEVTCVVVDV SHEDPEVKFN WYVDGVEVHN AKTKPREEQY 300<br>NSTYRVVSVL TVLHQDWLNG KEYKCKVSNK ALPAPIEKTI SKAKGQPREP QVYTLPPSRE 360<br>EMTKNQVSLT CLVKGFYPSD IAVEWESNGQ PENNYKTTPP VLDSDGSFFL YSKLTVDKSR 420<br>WQQGNVFSCS VMHEALHNHY TQKSLSLSPG K 451 |

(continued)

| Identifier | Sequence (One-Letter Amino Acid Symbols) |
|---|---|
| SEQ ID NO:57 light chain for tavolixizumab | DIQMTQSPSS LSASVGDRVT ITCRASQDIS NYLNWYQQKP GKAPKLLIYY TSKLHSGVPS 60<br>RFSGSGSGTD YTLTISSLQP EDFATYYCQQ GSALPWTFGQ GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:58 heavy chain variable region for tavolixizumab | QVQLQESGPG LVKPSQTLSL TCAVYGGSFS SGYWNWIRKH PGKGLEYIGY ISYNGITYHN 60<br>PSLKSRITIN RDTSKNQYSL QLNSVTPEDT AVYYCARYKY DYDGGHAMDY WGQGTLVT 118 |
| SEQ ID NO:59 light chain variable region for tavolixizumab | DIQMTQSPSS LSASVGDRVT ITCRASQDIS NYLNWYQQKP GKAPKLLIYY TSKLHSGVPS 60<br>RFSGSGSGTD YTLTISSLQP EDFATYYCQQ GSALPWTFGQ GTKVEIKR 108 |
| SEQ ID NO:60 heavy chain CDR1 for tavolixizumab | GSFSSGYWN 9 |
| SEQ ID NO:61 heavy chain CDR2 for tavolixizumab | YIGYISYNGI TYH 13 |
| SEQ ID NO:62 heavy chain CDR3 for tavolixizumab | RYKYDYDGGH AMDY 14 |
| SEQ ID NO:63 light chain CDR1 for tavolixizumab | QDISNYLN 8 |
| SEQ ID NO:64 light chain CDR2 for tavolixizumab | LLIYYTSKLH S 11 |
| SEQ ID NO:65 light chain CDR3 for tavolixizumab | QQGSALPW 8 |

In some embodiments, the OX40 agonist is 11D4, which is a fully human antibody available from Pfizer, Inc. The preparation and properties of 11D4 are described in U.S. Patent Nos. 7,960,515; 8,236,930; and 9,028,824. The amino acid sequences of 11D4 are set forth in Table 13.

In an embodiment, a OX40 agonist comprises a heavy chain given by SEQ ID NO:66 and a light chain given by SEQ ID NO:67. In an embodiment, a OX40 agonist comprises heavy and light chains having the sequences shown in SEQ ID NO:66 and SEQ ID NO:67, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:66 and SEQ ID NO:67, respectively. In an embodiment, a OX40 agonist comprises heavy and light chains that are each at least 98% identical to the sequences shown in SEQ ID NO:66 and SEQ ID NO:67, respectively. In an embodiment, a OX40 agonist comprises heavy and light chains that are each at least 97% identical to the sequences shown in SEQ ID NO:66 and SEQ ID NO:67, respectively. In an embodiment, a OX40 agonist comprises heavy and light chains that are each at least 96% identical to the sequences shown in SEQ ID NO:66 and SEQ ID NO:67, respectively. In an embodiment, a OX40 agonist comprises heavy and light chains that are each at least 95% identical to the sequences shown in SEQ ID NO:66 and SEQ ID NO:67, respectively.

In an embodiment, the OX40 agonist comprises the heavy and light chain CDRs or variable regions (VRs) of 11D4. In an embodiment, the OX40 agonist heavy chain variable region ($V_H$) comprises the sequence shown in SEQ ID NO:68, and the OX40 agonist light chain variable region ($V_L$) comprises the sequence shown in SEQ ID NO:69, and conservative amino acid substitutions thereof. In an embodiment, a OX40 agonist comprises $V_H$ and $V_L$ regions that are each at least 99% identical to the sequences shown in SEQ ID NO:68 and SEQ ID NO:69, respectively. In an embodiment, a OX40 agonist comprises $V_H$ and $V_L$ regions that are each at least 98% identical to the sequences shown in SEQ ID NO:68 and SEQ ID NO:69, respectively. In an embodiment, a OX40 agonist comprises $V_H$ and $V_L$ regions that are each at least 97% identical to the sequences shown in SEQ ID NO:68 and SEQ ID NO:69, respectively. In an embodiment, a OX40 agonist comprises $V_H$ and $V_L$ regions that are each at least 96% identical to the sequences shown in SEQ ID NO:68 and SEQ ID NO:69, respectively. In an embodiment, a OX40 agonist comprises $V_H$ and $V_L$ regions that are each at least 95% identical to the sequences shown in SEQ ID NO:68 and SEQ ID NO:69, respectively.

In an embodiment, a OX40 agonist comprises heavy chain CDR1, CDR2 and CDR3 domains having the sequences set forth in SEQ ID NO:70, SEQ ID NO:71, and SEQ ID NO:72, respectively, and conservative amino acid substitutions thereof, and light chain CDR1, CDR2 and CDR3 domains having the sequences set forth in SEQ ID NO:73, SEQ ID NO:74, and SEQ ID NO:75, respectively, and conservative amino acid substitutions thereof.

In an embodiment, the OX40 agonist is a OX40 agonist biosimilar monoclonal antibody approved by drug regulatory authorities with reference to 11D4. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is 11D4. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is 11D4. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 11D4. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 11D4.

TABLE 13: Amino acid sequences for OX40 agonist antibodies related to 11D4.

| Identifier | Sequence (One-Letter Amino Acid Symbols) |
|---|---|
| SEQ ID NO:66 heavy chain for 11D4 | EVQLVESGGG LVQPGGSLRL SCAASGFTFS SYSMNWVRQA PGKGLEWVSY ISSSSSTIDY 60<br>ADSVKGRFTI SRDNAKNSLY LQMNSLRDED TAVYYCARES GWYLFDYWGQ GTLVTVSSAS 120<br>TKGPSVFPLA PCSRSTSEST AALGCLVKDY FPEPVTVSWN SGALTSGVHT FPAVLQSSGL 180<br>YSLSSVVTVP SSNFGTQTYT CNVDHKPSNT KVDKTVERKC CVECPPCPAP PVAGPSVFLF 240<br>PPKPKDTLMI SRTPEVTCVV VDVSHEDPEV QFNWYVDGVE VHNAKTKPRE EQFNSTFRVV 300<br>SVLTVVHQDW LNGKEYKCKV SNKGLPAPIE KTISKTKGQP REPQVYTLPP SREEMTKNQV 360<br>SLTCLVKGFY PSDIAVEWES NGQPENNYKT TPPMLDSDGS FFLYSKLTVD KSRWQQGNVF 420<br>SCSVMHEALH NHYTQKSLSL SPGK 444 |
| SEQ ID NO:67 light chain for 11D4 | DIQMTQSPSS LSASVGDRVT ITCRASQGIS SWLAWYQQKP EKAPKSLIYA ASSLQSGVPS 60<br>RFSGSGSGTD FTLTISSLQP EDFATYYCQQ YNSYPPTFGG GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:68 heavy chain variable region for 11D4 | EVQLVESGGG LVQPGGSLRL SCAASGFTFS SYSMNWVRQA PGKGLEWVSY ISSSSSTIDY 60<br>ADSVKGRFTI SRDNAKNSLY LQMNSLRDED TAVYYCARES GWYLFDYWGQ GTLVTVSS 118 |
| SEQ ID NO:69 light chain variable region for 11D4 | DIQMTQSPSS LSASVGDRVT ITCRASQGIS SWLAWYQQKP EKAPKSLIYA ASSLQSGVPS 60<br>RFSGSGSGTD FTLTISSLQP EDFATYYCQQ YNSYPPTFGG GTKVEIK 107 |
| SEQ ID NO:70 heavy chain CDR1 for 11D4 | SYSMN 5 |
| SEQ ID NO:71 heavy chain CDR2 for 11D4 | YISSSSSTID YADSVKG 17 |

(continued)

| Identifier | Sequence (One-Letter Amino Acid Symbols) | |
|---|---|---|
| SEQ ID NO:72 heavy chain CDR3 for 11D4 | ESGWYLFDY | 9 |
| SEQ ID NO:73 light chain CDR1 for 11D4 | RASQGISSWL A | 11 |
| SEQ ID NO:74 light chain CDR2 for 11D4 | AASSLQS | 7 |
| SEQ ID NO:75 light chain CDR3 for 11D4 | QQYNSYPPT | 9 |

In some embodiments, the OX40 agonist is 18D8, which is a fully human antibody available from Pfizer, Inc. The preparation and properties of 18D8 are described in U.S. Patent Nos. 7,960,515; 8,236,930; and 9,028,824. The amino acid sequences of 18D8 are set forth in Table 14.

In an embodiment, a OX40 agonist comprises a heavy chain given by SEQ ID NO:76 and a light chain given by SEQ ID NO:77. In an embodiment, a OX40 agonist comprises heavy and light chains having the sequences shown in SEQ ID NO:76 and SEQ ID NO:77, respectively, or antigen binding fragments, Fab fragments, single-chain variable fragments (scFv), variants, or conjugates thereof. In an embodiment, a OX40 agonist comprises heavy and light chains that are each at least 99% identical to the sequences shown in SEQ ID NO:76 and SEQ ID NO:77, respectively. In an embodiment, a OX40 agonist comprises heavy and light chains that are each at least 98% identical to the sequences shown in SEQ ID NO:76 and SEQ ID NO:77, respectively. In an embodiment, a OX40 agonist comprises heavy and light chains that are each at least 97% identical to the sequences shown in SEQ ID NO:76 and SEQ ID NO:77, respectively. In an embodiment, a OX40 agonist comprises heavy and light chains that are each at least 96% identical to the sequences shown in SEQ ID NO:76 and SEQ ID NO:77, respectively. In an embodiment, a OX40 agonist comprises heavy and light chains that are each at least 95% identical to the sequences shown in SEQ ID NO:76 and SEQ ID NO:77, respectively.

In an embodiment, the OX40 agonist comprises the heavy and light chain CDRs or variable regions (VRs) of 18D8. In an embodiment, the OX40 agonist heavy chain variable region ($V_H$) comprises the sequence shown in SEQ ID NO:78, and the OX40 agonist light chain variable region ($V_L$) comprises the sequence shown in SEQ ID NO:79, and conservative amino acid substitutions thereof. In an embodiment, a OX40 agonist comprises $V_H$ and $V_L$ regions that are each at least 99% identical to the sequences shown in SEQ ID NO:78 and SEQ ID NO:79, respectively. In an embodiment, a OX40 agonist comprises $V_H$ and $V_L$ regions that are each at least 98% identical to the sequences shown in SEQ ID NO:78 and SEQ ID NO:79, respectively. In an embodiment, a OX40 agonist comprises $V_H$ and $V_L$ regions that are each at least 97% identical to the sequences shown in SEQ ID NO:78 and SEQ ID NO:79, respectively. In an embodiment, a OX40 agonist comprises $V_H$ and $V_L$ regions that are each at least 96% identical to the sequences shown in SEQ ID NO:78 and SEQ ID NO:79, respectively. In an embodiment, a OX40 agonist comprises $V_H$ and $V_L$ regions that are each at least 95% identical to the sequences shown in SEQ ID NO:78 and SEQ ID NO:79, respectively.

In an embodiment, a OX40 agonist comprises heavy chain CDR1, CDR2 and CDR3 domains having the sequences set forth in SEQ ID NO:80, SEQ ID NO:81, and SEQ ID NO:82, respectively, and conservative amino acid substitutions thereof, and light chain CDR1, CDR2 and CDR3 domains having the sequences set forth in SEQ ID NO:83, SEQ ID NO:84, and SEQ ID NO:85, respectively, and conservative amino acid substitutions thereof.

In an embodiment, the OX40 agonist is a OX40 agonist biosimilar monoclonal antibody approved by drug regulatory authorities with reference to 18D8. In an embodiment, the biosimilar monoclonal antibody comprises an OX40 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is 18D8. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is 18D8. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 18D8. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is 18D8.

TABLE 14: Amino acid sequences for OX40 agonist antibodies related to 18D8.

| Identifier | Sequence (One-Letter Amino Acid Symbols) |
|---|---|
| SEQ ID NO:76 heavy chain for 18D8 | EVQLVESGGG LVQPGRSLRL SCAASGFTFD DYAMHWVRQA PGKGLEWVSG ISWNSGSIGY 60<br>ADSVKGRFTI SRDNAKNSLY LQMNSLRAED TALYYCAKDQ STADYYFYYG MDVWGQGTTV 120<br>TVSSASTKGP SVFPLAPCSR STSESTAALG CLVKDYFPEP VTVSWNSGAL TSGVHTFPAV 180<br>LQSSGLYSLS SVVTVPSSNF GTQTYTCNVD HKPSNTKVDK TVERKCCVEC PPCPAPPVAG 240<br>PSVFLFPPKP KDTLMISRTP EVTCVVVDVS HEDPEVQFNW YVDGVEVHNA KTKPREEQFN 300<br>STFRVVSVLT VVHQDWLNGK EYKCKVSNKG LPAPIEKTIS KTKGQPREPQ VYTLPPSREE 360<br>MTKNQVSLTC LVKGFYPSDI AVEWESNGQP ENNYKTTPPM LDSDGSFFLY SKLTVDKSRW 420<br>QQGNVFSCSV MHEALHNHYT QKSLSLSPGK 450 |
| SEQ ID NO:77 light chain for 18D8 | EIVVTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPTFGQG TKVEIKRTVA APSVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFNR GEC 213 |
| SEQ ID NO:78 heavy chain variable region for 18D8 | EVQLVESGGG LVQPGRSLRL SCAASGFTFD DYAMHWVRQA PGKGLEWVSG ISWNSGSIGY 60<br>ADSVKGRFTI SRDNAKNSLY LQMNSLRAED TALYYCAKDQ STADYYFYYG MDVWGQGTTV 120<br>TVSS 124 |
| SEQ ID NO:79 light chain variable region for 18D8 | EIVVTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPTFGQG TKVEIK 106 |
| SEQ ID NO:80 heavy chain CDR1 for 18D8 | DYAMH 5 |
| SEQ ID NO:81 heavy chain CDR2 for 18D8 | GISWNSGSIG YADSVKG 17 |
| SEQ ID NO:82 heavy chain CDR3 for 18D8 | DQSTADYYFY YGMDV 15 |
| SEQ ID NO:83 light chain CDR1 for 18D8 | RASQSVSSYL A 11 |
| SEQ ID NO:84 light chain CDR2 for 18D8 | DASNRAT 7 |

(continued)

| Identifier | Sequence (One-Letter Amino Acid Symbols) |
|---|---|
| SEQ ID NO:85 light chain CDR3 for 18D8 | QQRSNWPT 8 |

In some embodiments, the OX40 agonist is Hu119-122, which is a humanized antibody available from GlaxoSmithKline plc. The preparation and properties of Hu119-122 are described in U.S. Patent Nos. 9,006,399 and 9,163,085, and in International Patent Publication No. WO 2012/027328. The amino acid sequences of Hu119-122 are set forth in Table 15.

In an embodiment, the OX40 agonist comprises the heavy and light chain CDRs or variable regions (VRs) of Hu119-122. In an embodiment, the OX40 agonist heavy chain variable region ($V_H$) comprises the sequence shown in SEQ ID NO:86, and the OX40 agonist light chain variable region ($V_L$) comprises the sequence shown in SEQ ID NO:87, and conservative amino acid substitutions thereof. In an embodiment, a OX40 agonist comprises $V_H$ and $V_L$ regions that are each at least 99% identical to the sequences shown in SEQ ID NO:86 and SEQ ID NO:87, respectively. In an embodiment, a OX40 agonist comprises $V_H$ and $V_L$ regions that are each at least 98% identical to the sequences shown in SEQ ID NO:86 and SEQ ID NO:87, respectively. In an embodiment, a OX40 agonist comprises $V_H$ and $V_L$ regions that are each at least 97% identical to the sequences shown in SEQ ID NO:86 and SEQ ID NO:87, respectively. In an embodiment, a OX40 agonist comprises $V_H$ and $V_L$ regions that are each at least 96% identical to the sequences shown in SEQ ID NO:86 and SEQ ID NO:87, respectively. In an embodiment, a OX40 agonist comprises $V_H$ and $V_L$ regions that are each at least 95% identical to the sequences shown in SEQ ID NO:86 and SEQ ID NO:87, respectively.

In an embodiment, a OX40 agonist comprises heavy chain CDR1, CDR2 and CDR3 domains having the sequences set forth in SEQ ID NO:88, SEQ ID NO:89, and SEQ ID NO:90, respectively, and conservative amino acid substitutions thereof, and light chain CDR1, CDR2 and CDR3 domains having the sequences set forth in SEQ ID NO:91, SEQ ID NO:92, and SEQ ID NO:93, respectively, and conservative amino acid substitutions thereof.

In an embodiment, the OX40 agonist is a OX40 agonist biosimilar monoclonal antibody approved by drug regulatory authorities with reference to Hu119-122. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is Hu1 19-122. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is Hu119-122. The OX40 agonist antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu119-122. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu119-122.

TABLE 15: Amino acid sequences for OX40 agonist antibodies related to Hu119-122.

| Identifier | Sequence (One-Letter Amino Acid Symbols) |
|---|---|
| SEQ ID NO:86 heavy chain variable region for Hull9-122 | EVQLVESGGG LVQPGGSLRL SCAASEYEFP SHDMSWVRQA PGKGLELVAA INSDGGSTYY 60<br>PDTMERRFTI SRDNAKNSLY LQMNSLRAED TAVYYCARHY DDYYAWFAYW GQGTMVTVSS 120 |
| SEQ ID NO:87 light chain variable region for Hull9-122 | EIVLTQSPAT LSLSPGERAT LSCRASKSVS TSGYSYMHWY QQKPGQAPRL LIYLASNLES 60<br>GVPARFSGSG SGTDFTLTIS SLEPEDFAVY YCQHSRELPL TFGGGTKVEI K 111 |

(continued)

| Identifier | Sequence (One-Letter Amino Acid Symbols) | |
|---|---|---|
| SEQ ID NO:88 heavy chain CDR1 for Hu119-122 | SHDMS | 5 |
| SEQ ID NO:89 heavy chain CDR2 for Hu119-122 | AINSDGGSTY YPDTMER | 17 |
| SEQ ID NO:90 heavy chain CDR3 for Hull9-122 | HYDDYYAWFA Y | 11 |
| SEQ ID NO:91 light chain CDR1 for Hull9-122 | RASKSVSTSG YSYMH | 15 |
| SEQ ID NO:92 light chain CDR2 for Hull9-122 | LASNLES | 7 |
| SEQ ID NO:93 light chain CDR3 for Hu119-122 | QHSRELPLT | 9 |

In some embodiments, the OX40 agonist is Hu106-222, which is a humanized antibody available from GlaxoSmithKline plc. The preparation and properties of Hu106-222 are described in U.S. Patent Nos. 9,006,399 and 9,163,085, and in International Patent Publication No. WO 2012/027328. The amino acid sequences of Hu106-222 are set forth in Table 16.

In an embodiment, the OX40 agonist comprises the heavy and light chain CDRs or variable regions (VRs) of Hu106-222. In an embodiment, the OX40 agonist heavy chain variable region ($V_H$) comprises the sequence shown in SEQ ID NO:94, and the OX40 agonist light chain variable region ($V_L$) comprises the sequence shown in SEQ ID NO:95, and conservative amino acid substitutions thereof. In an embodiment, a OX40 agonist comprises $V_H$ and $V_L$ regions that are each at least 99% identical to the sequences shown in SEQ ID NO:94 and SEQ ID NO:95, respectively. In an embodiment, a OX40 agonist comprises $V_H$ and $V_L$ regions that are each at least 98% identical to the sequences shown in SEQ ID NO:94 and SEQ ID NO:95, respectively. In an embodiment, a OX40 agonist comprises $V_H$ and $V_L$ regions that are each at least 97% identical to the sequences shown in SEQ ID NO:94 and SEQ ID NO:95, respectively. In an embodiment, a OX40 agonist comprises $V_H$ and $V_L$ regions that are each at least 96% identical to the sequences shown in SEQ ID NO:94 and SEQ ID NO:95, respectively. In an embodiment, a OX40 agonist comprises $V_H$ and $V_L$ regions that are each at least 95% identical to the sequences shown in SEQ ID NO:94 and SEQ ID NO:95, respectively.

In an embodiment, a OX40 agonist comprises heavy chain CDR1, CDR2 and CDR3 domains having the sequences set forth in SEQ ID NO:96, SEQ ID NO:97, and SEQ ID NO:98, respectively, and conservative amino acid substitutions thereof, and light chain CDR1, CDR2 and CDR3 domains having the sequences set forth in SEQ ID NO:99, SEQ ID NO:100, and SEQ ID NO:101, respectively, and conservative amino acid substitutions thereof.

In an embodiment, the OX40 agonist is a OX40 agonist biosimilar monoclonal antibody approved by drug regulatory authorities with reference to Hu106-222. In an embodiment, the biosimilar monoclonal antibody comprises an OX40 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is Hu106-222. In some embodiments, the one or more post-translational modifications are selected from one or more of: glycosylation, oxidation, deamidation, and truncation. In some embodiments, the biosimilar is a OX40 agonist antibody authorized or submitted for authorization, wherein the OX40 agonist antibody is provided in a formulation which differs from the formulations of a reference medicinal product or reference biological product, wherein the reference medicinal product or reference biological product is Hu106-222. The OX40 agonist antibody may be authorized by a drug regulatory authority such as the U.S. FDA and/or the European Union’s EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu106-222. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is Hu106-222.

TABLE 16: Amino acid sequences for OX40 agonist antibodies related to Hu106-222.

| Identifier | Sequence (One-Letter Amino Acid Symbols) |
|---|---|
| SEQ ID NO:94 heavy chain variable region for Hu106-222 | QVQLVQSGSE LKKPGASVKV SCKASGYTFT DYSMHWVRQA PGQGLKWMGW INTETGEPTY 60<br>ADDFKGRFVF SLDTSVSTAY LQISSLKAED TAVYYCANPY YDYVSYYAMD YWGQGTTVTV 120<br>SS 122 |
| SEQ ID NO:95 light chain variable region for Hu106-222 | DIQMTQSPSS LSASVGDRVT ITCKASQDVS TAVAWYQQKP GKAPKLLIYS ASYLYTGVPS 60<br>RFSGSGSGTD FTFTISSLQP EDIATYYCQQ HYSTPRTFGQ GTKLEIK 107 |
| SEQ ID NO:96 heavy chain CDR1 for Hu106-222 | DYSMH 5 |
| SEQ ID NO:97 heavy chain CDR2 for Hu106-222 | WINTETGEPT YADDFKG 17 |
| SEQ ID NO:98 heavy chain CDR3 for Hu106-222 | PYYDYVSYYA MDY 13 |
| SEQ ID NO:99 light chain CDR1 for Hu106-222 | KASQDVSTAV A 11 |
| SEQ ID NO: 100 light chain CDR2 for Hu106-222 | SASYLYT 7 |
| SEQ ID NO: 101 light chain CDR3 for Hu106-222 | QQHYSTPRT 9 |

In some embodiments, the OX40 agonist antibody is MEDI6469 (also referred to as 9B12). MEDI6469 is a murine monoclonal antibody. Weinberg, et al., J. Immunother. 2006, 29, 575-585. In some embodiments the OX40 agonist is an antibody produced by the 9B12 hybridoma, deposited with Biovest Inc. (Malvern, MA, USA), as described in Weinberg, et al., J. Immunother. 2006, 29, 575-585. In some embodiments, the antibody comprises the CDR sequences of MEDI6469. In some embodiments, the antibody comprises a heavy chain variable region sequence and/or a light chain variable region sequence of MEDI6469.

In an embodiment, the OX40 agonist is L106 BD (Pharmingen Product #340420). In some embodiments, the OX40 agonist comprises the CDRs of antibody L106 (BD Pharmingen Product #340420). In some embodiments, the OX40 agonist comprises a heavy chain variable region sequence and/or a light chain variable region sequence of antibody L106 (BD Pharmingen Product #340420). In an embodiment, the OX40 agonist is ACT35 (Santa Cruz Biotechnology, Catalog #20073). In some embodiments, the OX40 agonist comprises the CDRs of antibody ACT35 (Santa Cruz Biotechnology, Catalog #20073). In some embodiments, the OX40 agonist comprises a heavy chain variable region sequence and/or a light chain variable region sequence of antibody ACT35 (Santa Cruz Biotechnology, Catalog #20073). In an embodiment, the OX40 agonist is the murine monoclonal antibody anti-mCD134/mOX40 (clone OX86), commercially available from InVivoMAb, BioXcell Inc, West Lebanon, NH.

In an embodiment, the OX40 agonist is selected from the OX40 agonists described in International Patent Application Publication Nos. WO 95/12673, WO 95/21925, WO 2006/121810, WO 2012/027328, WO 2013/028231, WO 2013/038191, and WO 2014/148895; European Patent Application EP 0672141; U.S. Patent Application Publication Nos. US 2010/136030, US 2014/377284, US 2015/190506, and US 2015/132288 (including clones 20E5 and 12H3); and U.S. Patent Nos. 7,504,101, 7,550,140, 7,622,444, 7,696,175, 7,960,515, 7,961,515, 8,133,983, 9,006,399, and 9,163,085.

In an embodiment, the OX40 agonist is an OX40 agonistic fusion protein as depicted in Structure I-A (C-terminal Fc-antibody fragment fusion protein) or Structure I-B (N-terminal Fc-antibody fragment fusion protein), or a fragment, derivative, conjugate, variant, or biosimilar thereof. The properties of structures I-A and I-B are described above and in U.S. Patent Nos. 9,359,420, 9,340,599, 8,921,519, and 8,450,460. Amino acid sequences for the polypeptide domains of structure I-A are given in Table GG. The Fc domain preferably comprises a complete constant domain (amino acids 17-230 of SEQ ID NO:31) the complete hinge domain (amino acids 1-16 of SEQ ID NO:31) or a portion of the hinge domain (e.g., amino acids 4-16 of SEQ ID NO:31). Preferred linkers for connecting a C-terminal Fc-antibody may be selected from the embodiments given in SEQ ID NO:32 to SEQ ID NO:41, including linkers suitable for fusion of additional polypeptides. Likewise, amino acid sequences for the polypeptide domains of structure I-B are given in Table 9. If an Fc antibody fragment is fused to the N-terminus of an TNRFSF fusion protein as in structure I-B, the sequence of the Fc module is preferably that shown in SEQ ID NO:42, and the linker sequences are preferably selected from those embodiments set forth in SED ID NO:43 to SEQ ID NO:45.

In an embodiment, an OX40 agonist fusion protein according to structures I-A or I-B comprises one or more OX40 binding domains selected from the group consisting of a variable heavy chain and variable light chain of tavolixizumab, a variable heavy chain and variable light chain of 11D4, a variable heavy chain and variable light chain of 18D8, a variable heavy chain and variable light chain of Hu119-122, a variable heavy chain and variable light chain of Hu106-222, a variable heavy chain and variable light chain selected from the variable heavy chains and variable light chains described in Table OO, any combination of a variable heavy chain and variable light chain of the foregoing, and fragments, derivatives, conjugates, variants, and biosimilars thereof.

In an embodiment, an OX40 agonist fusion protein according to structures I-A or I-B comprises one or more OX40 binding domains comprising an OX40L sequence. In an embodiment, an OX40 agonist fusion protein according to structures I-A or I-B comprises one or more OX40 binding domains comprising a sequence according to SEQ ID NO:102. In an embodiment, an OX40 agonist fusion protein according to structures I-A or I-B comprises one or more OX40 binding domains comprising a soluble OX40L sequence. In an embodiment, a OX40 agonist fusion protein according to structures I-A or I-B comprises one or more OX40 binding domains comprising a sequence according to SEQ ID NO: 103. In an embodiment, a OX40 agonist fusion protein according to structures I-A or I-B comprises one or more OX40 binding domains comprising a sequence according to SEQ ID NO:104.

In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:58 and SEQ ID NO:59, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:68 and SEQ ID NO:69, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:78 and SEQ ID NO:79, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:86 and SEQ ID NO:87, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the sequences shown in SEQ ID NO:94 and SEQ ID NO:95, respectively, wherein the $V_H$ and $V_L$ domains are connected by a linker. In an embodiment, an OX40 agonist fusion protein according to structures I-A or I-B comprises one or more OX40 binding domains that is a scFv domain comprising $V_H$ and $V_L$ regions that are each at least 95% identical to the $V_H$ and $V_L$ sequences given in Table 17, wherein the $V_H$ and $V_L$ domains are connected by a linker.

TABLE 17: Additional polypeptide domains useful as OX40 binding domains in fusion proteins (e.g., structures I-A and I-B) or as scFv OX40 agonist antibodies.

| Identifier | Sequence (One-Letter Amino Acid Symbols) |
|---|---|
| SEQ ID NO:102 OX40L | MERVQPLEEN VGNAARPRFE RNKLLLVASV IQGLGLLLCF TYICLHFSAL QVSHRYPRIQ 60<br>SIKVQFTEYK KEKGFILTSQ KEDEIMKVQN NSVIINCDGF YLISLKGYFS QEVNISLHYQ 120<br>KDEEPLFQLK KVRSVNSLMV ASLTYKDKVY LNVTTDNTSL DDFHVNGGEL ILIHQNPGEF 180<br>CVL 183 |
| SEQ ID NO:103 OX40L soluble domain | SHRYPRIQSI KVQFTEYKKE KGFILTSQKE DEIMKVQNNS VIINCDGFYL ISLKGYFSQE 60<br>VNISLHYQKD EEPLFQLKKV RSVNSLMVAS LTYKDKVYLN VTTDNTSLDD FHVNGGELIL 120<br>IHQNPGEFCV L 131 |
| SEQ ID NO:104 OX40L soluble | YPRIQSIKVQ FTEYKKEKGF ILTSQKEDEI MKVQNNSVII NCDGFYLISL KGYFSQEVNI 60<br>SLHYQKDEEP LFQLKKVRSV NSLMVASLTY KDKVYLNVTT DNTSLDDFHV NGGELILIHQ 120 |
| domain (alternative) | NPGEFCVL 128 |
| SEQ ID NO:105 variable heavy chain for 008 | EVQLVESGGG LVQPGGSLRL SCAASGFTFS NYTMNWVRQA PGKGLEWVSA ISGSGGSTYY 60<br>ADSVKGRFTI SRDNSKNTLY LQMNSLRAED TAVYYCAKDR YSQVHYALDY WGQGTLVTVS 120 |
| SEQ ID NO:106 variable light chain for 008 | DIVMTQSPDS LPVTPGEPAS ISCRSSQSLL HSNGYNYLDW YLQKAGQSPQ LLIYLGSNRA 60<br>SGVPDRFSGS GSGTDFTLKI SRVEAEDVGV YYCQQYYNHP TTFGQGTK 108 |
| SEQ ID NO:107 variable heavy chain for 011 | EVQLVESGGG VVQPGRSLRL SCAASGFTFS DYTMNWVRQA PGKGLEWVSS ISGGSTYYAD 60<br>SRKGRFTISR DNSKNTLYLQ MNNLRAEDTA VYYCARDRYF RQQNAFDYWG QGTLVTVSSA 120 |
| SEQ ID NO:108 variable light chain for 011 | DIVMTQSPDS LPVTPGEPAS ISCRSSQSLL HSNGYNYLDW YLQKAGQSPQ LLIYLGSNRA 60<br>SGVPDRFSGS GSGTDFTLKI SRVEAEDVGV YYCQQYYNHP TTFGQGTK 108 |
| SEQ ID NO:109 variable heavy chain for 021 | EVQLVESGGG LVQPRGSLRL SCAASGFTFS SYAMNWVRQA PGKGLEWVAV ISYDGSNKYY 60<br>ADSVKGRFTI SRDNSKNTLY LQMNSLRAED TAVYYCAKDR YITLPNALDY WGQGTLVTVS 120 |
| SEQ ID NO:110 variable light chain for 021 | DIQMTQSPVS LPVTPGEPAS ISCRSSQSLL HSNGYNYLDW YLQKPGQSPQ LLIYLGSNRA 60<br>SGVPDRFSGS GSGTDFTLKI SRVEAEDVGV YYCQQYKSNP PTFGQGTK 108 |
| SEQ ID NO:111 variable heavy chain for 023 | EVQLVESGGG LVHPGGSLRL SCAGSGFTFS SYAMHWVRQA PGKGLEWVSA IGTGGGTYYA 60<br>DSVMGRFTIS RDNSKNTLYL QMNSLRAEDT AVYYCARYDN VMGLYWFDYW GQGTLVTVSS 120 |
| SEQ ID NO:112 variable light chain for 023 | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPPAFGG GTKVEIKR 108 |
| SEQ ID NO:113 heavy chain variable region | EVQLQQSGPE LVKPGASVKM SCKASGYTFT SYVMHWVKQK PGQGLEWIGY INPYNDGTKY 60<br>NEKFKGKATL TSDKSSSTAY MELSSLTSED SAVYYCANYY GSSLSMDYWG QGTSVTVSS 119 |
| SEQ ID NO:114 light chain variable region | DIQMTQTTSS LSASLGDRVT ISCRASQDIS NYLNWYQQKP DGTVKLLIYY TSRLHSGVPS 60<br>RFSGSGSGTD YSLTISNLEQ EDIATYFCQQ GNTLPWTFGG GTKLEIKR 108 |
| SEQ ID NO:115 heavy chain variable region | EVQLQQSGPE LVKPGASVKI SCKTSGYTFK DYTMHWVKQS HGKSLEWIGG IYPNNGGSTY 60<br>NQNFKDKATL TVDKSSSTAY MEFRSLTSED SAVYYCARMG YHGPHLDFDV WGAGTTVTVS 120<br>P 121 |
| SEQ ID NO:116 light chain variable region | DIVMTQSHKF MSTSLGDRVS ITCKASQDVG AAVAWYQQKP GQSPKLLIYW ASTRHTGVPD 60<br>RFTGGGSGTD FTLTISNVQS EDLTDYFCQQ YINYPLTFGG GTKLEIKR 108 |

(continued)

| Identifier | Sequence (One-Letter Amino Acid Symbols) |
|---|---|
| SEQ ID NO:117 heavy chain variable region of humanized antibody | QIQLVQSGPE LKKPGETVKI SCKASGYTFT DYSMHWVKQA PGKGLKWMGW INTETGEPTY 60<br>ADDFKGRFAF SLETSASTAY LQINNLKNED TATYFCANPY YDYVSYYAMD YWGHGTSVTV 120<br>SS 122 |
| SEQ ID NO:118 heavy chain variable region of humanized antibody | QVQLVQSGSE LKKPGASVKV SCKASGYTFT DYSMHWVRQA PGQGLKWMGW INTETGEPTY 60<br>ADDFKGRFVF SLDTSVSTAY LQISSLKAED TAVYYCANPY YDYVSYYAMD YWGQGTTVTV 120<br>SS 122 |
| SEQ ID NO:119 light chain variable region of humanized antibody | DIVMTQSHKF MSTSVRDRVS ITCKASQDVS TAVAWYQQKP GQSPKLLIYS ASYLYTGVPD 60<br>RFTGSGSGTD FTFTISSVQA EDLAVYYCQQ HYSTPRTFGG GTKLEIK 107 |
| SEQ ID NO:120 light chain variable region of humanized antibody | DIVMTQSHKF MSTSVRDRVS ITCKASQDVS TAVAWYQQKP GQSPKLLIYS ASYLYTGVPD 60<br>RFTGSGSGTD FTFTISSVQA EDLAVYYCQQ HYSTPRTFGG GTKLEIK 107 |
| SEQ ID NO:121 heavy chain variable region of humanized antibody | EVQLVESGGG LVQPGESLKL SCESNEYEFP SHDMSWVRKT PEKRLELVAA INSDGGSTYY 60<br>PDTMERRFII SRDNTKKTLY LQMSSLRSED TALYYCARHY DDYYAWFAYW GQGTLVTVSA 120 |
| SEQ ID NO:122 heavy chain variable region of humanized antibody | EVQLVESGGG LVQPGGSLRL SCAASEYEFP SHDMSWVRQA PGKGLELVAA INSDGGSTYY 60<br>PDTMERRFTI SRDNAKNSLY LQMNSLRAED TAVYYCARHY DDYYAWFAYW GQGTMVTVSS 120 |
| SEQ ID NO:123 light chain variable region of humanized antibody | DIVLTQSPAS LAVSLGQRAT ISCRASKSVS TSGYSYMHWY QQKPGQPPKL LIYLASNLES 60<br>GVPARFSGSG SGTDFTLNIH PVEEEDAATY YCQHSRELPL TFGAGTKLEL K 111 |
| SEQ ID NO:124 light chain | EIVLTQSPAT LSLSPGERAT LSCRASKSVS TSGYSYMHWY QQKPGQAPRL LIYLASNLES 60<br>GVPARFSGSG SGTDFTLTIS SLEPEDFAVY YCQHSRELPL TFGGGTKVEI K 111 |
| variable region of humanized antibody | |
| SEQ ID NO:125 heavy chain variable region | MYLGLNYVFI VFLLNGVQSE VKLEESGGGL VQPGGSMKLS CAASGFTFSD AWMDWVRQSP 60<br>EKGLEWVAEI RSKANNHATY YAESVNGRFT ISRDDSKSSV YLQMNSLRAE DTGIYYCTWG 120<br>EVFYFDYWGQ GTTLTVSS 138 |
| SEQ ID NO:126 light chain variable region | MRPSIQFLGL LLFWLHGAQC DIQMTQSPSS LSASLGGKVT ITCKSSQDIN KYIAWYQHKP 60<br>GKGPRLLIHY TSTLQPGIPS RFSGSGSGRD YSFSISNLEP EDIATYYCLQ YDNLLTFGAG 120<br>TKLELK 126 |

In an embodiment, the OX40 agonist is a OX40 agonistic single-chain fusion polypeptide comprising (i) a first soluble OX40 binding domain, (ii) a first peptide linker, (iii) a second soluble OX40 binding domain, (iv) a second peptide linker, and (v) a third soluble OX40 binding domain, further comprising an additional domain at the N-terminal and/or C-terminal end, and wherein the additional domain is a Fab or Fc fragment domain. In an embodiment, the OX40 agonist is a OX40 agonistic single-chain fusion polypeptide comprising (i) a first soluble OX40 binding domain, (ii) a first peptide linker, (iii) a second soluble OX40 binding domain, (iv) a second peptide linker, and (v) a third soluble OX40 binding domain, further comprising an additional domain at the N-terminal and/or C-terminal end, wherein the additional domain is a Fab or Fc fragment domain wherein each of the soluble OX40 binding domains lacks a stalk region (which contributes to trimerisation and provides a certain distance to the cell membrane, but is not part of the OX40 binding domain) and the first and the second peptide linkers independently have a length of 3-8 amino acids.

In an embodiment, the OX40 agonist is an OX40 agonistic single-chain fusion polypeptide comprising (i) a first soluble tumor necrosis factor (TNF) superfamily cytokine domain, (ii) a first peptide linker, (iii) a second soluble TNF superfamily cytokine domain, (iv) a second peptide linker, and (v) a third soluble TNF superfamily cytokine domain, wherein each of the soluble TNF superfamily cytokine domains lacks a stalk region and the first and the second peptide linkers independently have a length of 3-8 amino acids, and wherein the TNF superfamily cytokine domain is an OX40 binding domain.

In some embodiments, the OX40 agonist is MEDI6383. MEDI6383 is an OX40 agonistic fusion protein and can be prepared as described in U.S. Patent No. 6,312,700.

In an embodiment, the OX40 agonist is an OX40 agonistic scFv antibody comprising any of the foregoing $V_H$ domains linked to any of the foregoing $V_L$ domains.

In an embodiment, the OX40 agonist is Creative Biolabs OX40 agonist monoclonal antibody MOM-18455, commercially available from Creative Biolabs, Inc., Shirley, NY, USA.

In an embodiment, the OX40 agonist is OX40 agonistic antibody clone Ber-ACT35 commercially available from BioLegend, Inc., San Diego, CA, USA.

## VI. Optional Cell Viability Analyses

Optionally, a cell viability assay can be performed after the Step B first expansion of Process 2A (Gen 2) or Gen 3 processes, using standard assays known in the art. For example, a trypan blue exclusion assay can be done on a sample of the bulk TILs, which selectively labels dead cells and allows a viability assessment. Other assays for use in testing viability can include but are not limited to the Alamar blue assay; and the MTT assay.

1. Cell Counts, Viability, Flow Cytometry

In some embodiments of Process 2A (Gen 2) or Gen 3, cell counts and/or viability are measured. The expression of markers such as but not limited CD3, CD4, CD8, and CD56, as well as any other disclosed or described herein, can be measured by flow cytometry with antibodies, for example but not limited to those commercially available from BD Biosciences (BD Biosciences, San Jose, CA) using a FACSCanto™ flow cytometer (BD Biosciences). The cells can be counted manually using a disposable c-chip hemocytometer (VWR, Batavia, IL) and viability can be assessed using any method known in the art, including but not limited to trypan blue staining. In some embodiments of Process 2A (Gen 2) and/or Gen 3, the TILs exhibit an increase in CD8+ cells after either the first expansion, the second expansion, the priming first expansion, or the rapid second expansion. In some embodiments of Process 2A (Gen 2), the TILs exhibit an increase in CD8+ cells after either the first expansion and/or the second expansion. In some embodiments of Gen 3, the TILs exhibit an increase in CD8+ cells after the priming first expansion, or the rapid second expansion.

In some cases, the bulk TIL population can be cryopreserved immediately, using the protocols discussed below. Alternatively, the bulk TIL population can be subjected to REP and then cryopreserved as discussed below. Similarly, in the case where genetically modified TILs will be used in therapy, the bulk or REP TIL populations can be subjected to genetic modifications for suitable treatments.

2. Cell Cultures

In an embodiment, a method for expanding TILs may include using about 5,000 mL to about 25,000 mL of cell medium, about 5,000 mL to about 10,000 mL of cell medium, or about 5,800 mL to about 8,700 mL of cell medium. In an embodiment, expanding the number of TILs uses no more than one type of cell culture medium. Any suitable cell culture medium may be used, *e.g.,* AIM-V cell medium (L-glutamine, 50 μM streptomycin sulfate, and 10 μM gentamicin sulfate) cell culture medium (Invitrogen, Carlsbad CA). In this regard, the inventive methods advantageously reduce the amount of medium and the number of types of medium required to expand the number of TIL. In an embodiment, expanding the number of TIL may comprise adding fresh cell culture media to the cells (also referred to as feeding the cells) no more frequently than every third or fourth day. Expanding the number of cells in a gas permeable container simplifies the procedures necessary to expand the number of cells by reducing the feeding frequency necessary to expand the cells.

In an embodiment, the cell medium in the first and/or second gas permeable container is unfiltered. The use of unfiltered cell medium may simplify the procedures necessary to expand the number of cells. In an embodiment, the cell medium in the first and/or second gas permeable container lacks beta-mercaptoethanol (BME).

In an embodiment, TILs are expanded in gas-permeable containers. Gas-permeable containers have been used to expand TILs using PBMCs using methods, compositions, and devices known in the art, including those described in U.S. Patent Application Publication No. 2005/0106717 A1. In an embodiment, TILs are expanded in gas-permeable bags. In an embodiment, TILs are expanded using a cell expansion system that expands TILs in gas permeable bags, such as the Xuri Cell Expansion System W25 (GE Healthcare). In an embodiment, TILs are expanded using a cell expansion system that expands TILs in gas permeable bags, such as the WAVE Bioreactor System, also known as the Xuri Cell Expansion System W5 (GE Healthcare). In an embodiment, the cell expansion system includes a gas permeable cell bag with a volume selected from the group consisting of about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L. In an embodiment, TILs can be expanded in G-Rex flasks (commercially available from Wilson Wolf Manufacturing). Such embodiments allow for cell populations to expand from about $5 \times 10^5$ cells/cm$^2$ to between $10 \times 10^6$ and $30 \times 10^6$ cells/cm$^2$. In an embodiment this expansion is conducted without adding fresh cell culture media to the cells (also referred to as feeding the cells). In an embodiment, this is without feeding so long as medium resides at a height of about 10 cm in the GRex flask. In an embodiment this is without feeding but with the addition of one or more cytokines. In an embodiment, the cytokine can be added as a bolus without any need to mix the cytokine with the medium. Such containers, devices, and methods are known in the art and have been used to expand TILs, and include those described in U.S. Patent Application Publication No. US 2014/0377739A1, International Publication No. WO 2014/210036 A1, U.S. Patent Application Publication No. us 2013/0115617 A1, International Publication No. WO 2013/188427 A1, U.S. Patent Application Publication No. US 2011/0136228 A1, U.S. Patent No. US 8,809,050 B2, International publication No. WO 2011/072088 A2, U.S. Patent Application Publication No. US 2016/0208216 A1, U.S. Patent Application Publication No. US 2012/0244133 A1, International Publication No. WO 2012/129201 A1, U.S. Patent Application Publication No. US 2013/0102075 A1, U.S. Patent No. US 8,956,860 B2, International Publication No. WO 2013/173835 A1, U.S. Patent Application Publication No. US 2015/0175966 A1. Such processes are also described in Jin et al., J. Immunotherapy, 2012, 35:283-292.

## VII. Metabolic Health of Expanded TILs

Spare respiratory capacity (SRC) and glycolytic reserve can be evaluated for TILs expanded with the methods disclosed herein. The Seahorse XF Cell Mito Stress Test measures mitochondrial function by directly measuring the oxygen consumption rate (OCR) of cells, using modulators of respiration that target components of the electron transport chain in the mitochondria. The test compounds (oligomycin, FCCP, and a mix of rotenone and antimycin A, described below) are serially injected to measure ATP production, maximal respiration, and non-mitochondrial respiration, respectively. Proton leak and spare respiratory capacity are then calculated using these parameters and basal respiration. Each modulator targets a specific component of the electron transport chain. Oligomycin inhibits ATP synthase (complex V) and the decrease in OCR following injection of oligomycin correlates to the mitochondrial respiration associated with cellular ATP production. Carbonyl cyanide-4 (trifluoromethoxy) phenylhydrazone (FCCP) is an uncoupling agent that collapses the proton gradient and disrupts the mitochondrial membrane potential. As a result, electron flow through the electron transport chain is uninhibited and oxygen is maximally consumed by complex IV. The FCCP-stimulated OCR can then be used to calculate spare respiratory capacity, defined as the difference between maximal respiration and basal respiration. Spare respiratory capacity (SRC) is a measure of the ability of the cell to respond to increased energy demand. The third injection is a mix of rotenone, a complex I inhibitor, and antimycin A, a complex III inhibitor. This combination shuts down mitochondrial respiration and enables the calculation of nonmitochondrial respiration driven by processes outside the mitochondria.

The metabolic assay may be basal respiration.

In general, TILs have a basal respiration rate that is at least 50%, at least 55%, at least 60%, at least 65%, at least 70%, at least 75%, at least 80%, at least 85%, at least 90%, at least 95%, at least 97%, at least 98%, or at least 99% of the basal respiration rate of freshly harvested TILs. In some embodiments, the basal respiration rate is from about 50% to about 99% of the basal respiration rate of freshly harvested TILs. In some embodiments, the basal respiration rate is from about 60% to about 99% of the basal respiration rate of freshly harvested TILs. In some embodiments, the basal respiration rate is from about 70% to about 99% of the basal respiration rate of freshly harvested TILs. In some embodiments, the basal respiration rate is from about 80% to about 99% of the basal respiration rate of freshly harvested TILs. In some embodiments, the basal respiration rate is from about 90% to about 99% of the basal respiration rate of freshly harvested TILs. In some embodiments, the basal respiration rate is from about 95% to about 99% of the basal respiration rate of freshly harvested TILs. In some embodiments, the TILs have a basal respiration rate that is not statistically significantly different than the basal respiration rate of freshly harvested TILs.

The metabolic assay may be spare respiratory capacity.

The metabolic assay may be glycolytic reserve. The metabolic assay may be spare respiratory capacity. To measure cellular (respiratory) metabolism cells were treated with inhibitors of mitochondrial respiration and glycolysis to determine a metabolic profile for the TIL consisting of the following measures: baseline oxidative phosphorylation (as measured by OCR), spare respiratory capacity, baseline glycolytic activity (as measured by ECAR), and glycolytic reserve. Metabolic profiles were performed using the Seahorse Combination Mitochondrial/Glycolysis Stress Test Assay (including the kit commercially available from Agilent®), which allows for determining a cells' capacity to perform glycolysis upon blockage of mitochondrial ATP production. Cells may be starved of glucose, then glucose is injected, followed by a stress agent. The stress agent may be selected from the group consisting of oligomycin, FCCP, rotenone, antimycin A and/or 2-deoxyglucose (2-DG), as well as combinations thereof. Oligomycin may be added at 10 mM. FCCP may be added at 10 mM. Rotenone may be added at 2.5 mM. Antimycin A may be added at 2.5 mM. 2-deoxyglucose (2-DG) may be added at 500 mM. Glycolytic capacity, glycolytic reserve, and/or non-glycolytic acidification may be measured. In general, TILs have a glycolytic reserve that is at least 50%, at least 55%, at least 60%, at least 65%, at least 70%, at least 75%, at least 80%, at least 85%, at least 90%, at least 95%, at least 97%, at least 98%, or at least 99% of the basal respiration rate of freshly harvested TILs. In some embodiments, the glycolytic reserve is from about 50% to about 99% of the *basal respiration rate* of freshly harvested TILs. In some embodiments, the glycolytic reserve is from about 60% to about 99% of the *basal respiration rate* of freshly harvested TILs. In some embodiments, the glycolytic reserve is from about 70% to about 99% of the *basal respiration rate* of freshly harvested TILs. In some embodiments, the glycolytic reserve is from about 80% to about 99% of the *basal respiration rate* of freshly harvested TILs. In some embodiments, the glycolytic reserve is from about 90% to about 99% of the *basal respiration rate* of freshly harvested TILs. In some embodiments, the glycolytic reserve is from about 95% to about 99% of the *basal respiration rate* of freshly harvested TILs.

The metabolic assay may be basal glycolysis. In some the second expansion TILs or second additional expansion TILs (such as, for example, those described in Step D of Figure 7, including TILs referred to as reREP TILs) have an increase in basal glycolysis of at least two-fold, at least three-fold, at least four-fold, at least five-fold, at least six-fold, at least 7-fold, at least eight-fold, at least nine-fold, or at least ten-fold. In some embodiments, the second expansion TILs or second additional expansion TILs (such as, for example, those described in Step D of Figure 7, including TILs referred to as reREP TILs) have an increase in basal glycolysis of about two-fold to about ten-fold. In some embodiments, the second expansion TILs or second additional expansion TILs (such as, for example, those described in Step D of Figure 7, including TILs referred to as reREP TILs) have an increase in basal glycolysis of about two-fold to about eight-fold. In some embodiments, the second expansion TILs or second additional expansion TILs (such as, for example, those described in Step D of Figure 7, including TILs referred to as reREP TILs) have an increase in basal glycolysis of about three-fold to about seven-fold. In some embodiments, the second expansion TILs or second additional expansion TILs (such as, for example, those described in Step D of Figure 7, including TILs referred to as reREP TILs) have an increase in basal glycolysis of about two-fold to about four-fold. In some embodiments, the second expansion TILs or second additional expansion TILs (such as, for example, those described in Step D of Figure 7, including TILs referred to as reREP TILs) have an increase in basal glycolysis of about two-fold to about three-fold.

In general, the second expansion TILs or second additional expansion TILs (such as, for example, those described in Step D of Figure 7, including TILs referred to as reREP TILs) have a spare respiratory capacity that is at least is at least 50%, at least 55%, at least 60%, at least 65%, at least 70%, at least 75%, at least 80%, at least 85%, at least 90%, at least 95%, at least 97%, at least 98%, or at least 99% of the basal respiration rate of freshly harvested TILs. In some embodiments, the spare respiratory capacity is from about 50% to about 99% of the *basal respiration rate* of freshly harvested TILs. In some embodiments, the spare respiratory capacity is from about 50% to about 99% of the *basal respiration rate* of freshly harvested TILs. In some embodiments, the spare respiratory capacity is from about 60% to about 99% of the *basal respiration rate* of freshly harvested TILs. In some embodiments, the spare respiratory capacity is from about 70% to about 99% of the *basal respiration rate* of freshly harvested TILs. In some embodiments, the spare respiratory capacity is from about 80% to about 99% of the *basal respiration rate* of freshly harvested TILs. In some embodiments, the spare respiratory capacity is from about 90% to about 99% of the *basal respiration rate* of freshly harvested TILs. In some embodiments, the spare respiratory capacity is from about 95% to about 99% of the *basal respiration rate* of freshly harvested TILs. In some embodiments, the second expansion TILs or second additional expansion TILs (such as, for example, those described in Step D of Figure 7, including TILs referred to as reREP TILs) have a spare respiratory capacity that is not statistically significantly different than the *basal respiration rate* of freshly harvested TILs.

## VIII. FUNCTIONAL CHARACTERIZATION OF TILs

Granzyme B Production: Granzyme B is another measure of the ability of TIL to kill target cells. Media supernatants restimulated as described above using antibodies to CD3, CD28, and CD137/4-1BB were also evaluated for their levels of Granzyme B using the Human Granzyme B DuoSet ELISA Kit (R & D Systems, Minneapolis, MN) according to the manufacturer's instructions. In some embodiments, the second expansion TILs or second additional expansion TILs (such as, for example, those described in Step D of Figure 7, including TILs referred to as reREP TILs) have increased Granzyme B production. In some embodiments, the second expansion TILs or second additional expansion TILs (such as, for example, those described in Step D of Figure 7, including TILs referred to as reREP TILs) have increased cytotoxic activity.

In some embodiments, telomere length can be used as a measure of cell viability and/or cellular function. Telomere length measurement: Diverse methods have been used to measure the length of telomeres in genomic DNA and cytological preparations. The telomere restriction fragment (TRF) analysis is the gold standard to measure telomere length (de Lange *et al.,* 1990). However, the major limitation of TRF is the requirement of a large amount of DNA (1.5 μg). Two widely used techniques for the measurement of telomere lengths namely, fluorescence in situ hybridization (FISH; Agilent Technologies, Santa Clara, CA) and quantitative PCR can be employed with the present invention. In some embodiments, there is no change in telomere length between the initially harvest TILs in Step A and the expanded TILs from for example Step D as provided in Figure 7.

In some embodiments, TIL health is measured by IFN-gamma secretion. In some embodiments, a potency assay for IFN-γ production is employed. IFN-γ production is another measure of cytotoxic potential. IFN-γ production can be measured by determining the levels of the cytokine IFN-γ in the media of TIL stimulated with antibodies to CD3, CD28, and CD137/4-1BB. IFN-γ levels in media from these stimulated TIL can be determined using by measuring IFN-γ release. In some embodiments, an increase in IFN-γ production in for example Step D as provided in Figure 7 TILs as compared to initially harvested TILs in for example Step A as provided in Figure 7 is indicative of an increase in cytotoxic potential of the Step D TILs.

The Bioluminescent Redirected Lysis Assay: The cytotoxic potential of TIL to lyse target cells was assessed using a co-culture assay of TIL with the bioluminescent cell line, P815 (Clone G6), according to a Bioluminescent redirected lysis assay (potency assay) for TIL assay which measures TIL cytotoxicity in a highly sensitive dose dependent manner.

Also disclosed herein but not claimed is an assay for assessing TIL viability, using the methods as described above and herein, including, for example, as described in the Examples and Figures. In some embodiments, the TILs are expanded as discussed above, including for example as provided in Figure 7. The TILs may be cryopreserved prior to being assessed for viability. The viability assessment may include thawing the TILs prior to performing a first expansion, a second expansion, and an additional second expansion. Certain methods disclosed herein may provide an assay for assessing cell proliferation, cell toxicity, cell death, and/or other terms related to viability of the TIL population. Viability can be measured by any of the TIL metabolic assays described above as well as any methods know for assessing cell viability that are known in the art. Certain methods disclosed herein provide an assay for assessment of cell proliferation, cell toxicity, cell death, and/or other terms related to viability of the TILs expanded using the methods described herein, including those exemplified in Figure 7.

In some embodiments, the metabolic assay is basal respiration. In general, second expansion TILs have a basal respiration rate that is at least 50%, at least 55%, at least 60%, at least 65%, at least 70%, at least 75%, at least 80%, at least 85%, at least 90%, at least 95%, at least 97%, at least 98%, or at least 99% of the basal respiration rate of 2A process TILs (see, for example Figure 7). In some embodiments, the basal respiration rate is from about 50% to about 99% of the basal respiration rate of 2A process TILs (see, for example Figure 7). In some embodiments, the basal respiration rate is from about 60% to about 99% of the basal respiration rate of 2A process TILs (see, for example Figure 7). In some embodiments, the basal respiration rate is from about 70% to about 99% of the basal respiration rate of 2A process TILs (see, for example Figure 7). In some embodiments, the basal respiration rate is from about 80% to about 99% of the basal respiration rate of 2A process TILs (see, for example Figure 7). In some embodiments, the basal respiration rate is from about 90% to about 99% of the basal respiration rate of 2A process TILs (see, for example Figure 7). In some embodiments, the basal respiration rate is from about 95% to about 99% of the basal respiration rate of 2A process TILs (see, for example Figure 7). In some embodiments, the second expansion TILs or second additional expansion TILs (such as, for example, those described in Step D of Figure 7, including TILs referred to as reREP TILs) have a basal respiration rate that is not statistically significantly different than the basal respiration rate of 2A process TILs (see, for example Figure 7).

In general, the second expansion TILs or second additional expansion TILs (such as, for example, those described in Step D of Figure 7, including TILs referred to as reREP TILs) have a spare respiratory capacity that is at least is at least 50%, at least 55%, at least 60%, at least 65%, at least 70%, at least 75%, at least 80%, at least 85%, at least 90%, at least 95%, at least 97%, at least 98%, or at least 99% of the basal respiration rate of 2A process TILs (see, for example Figure 7). In some embodiments, the spare respiratory capacity is from about 50% to about 99% of the *basal respiration rate* of freshly harvested TILs. In some embodiments, the spare respiratory capacity is from about 50% to about 99% of the *basal respiration rate* of 2A process TILs (see, for example Figure 7). In some embodiments, the spare respiratory capacity is from about 60% to about 99% of the *basal respiration rate* of 2A process TILs (see, for example Figure 7). In some embodiments, the spare respiratory capacity is from about 70% to about 99% of the *basal respiration rate* of 2A process TILs (see, for example Figure 7). In some embodiments, the spare respiratory capacity is from about 80% to about 99% of the *basal respiration rate* of 2A process TILs (see, for example Figure 7). In some embodiments, the spare respiratory capacity is from about 90% to about 99% of the *basal respiration rate* of 2A process TILs (see, for example Figure 7). In some embodiments, the spare respiratory capacity is from about 95% to about 99% of the *basal respiration rate* of 2A process TILs (see, for example Figure 7). In some embodiments, the second expansion TILs or second additional expansion TILs (such as, for example, those described in Step D of Figure 7, including TILs referred to as reREP TILs) have a spare respiratory capacity that is not statistically significantly different than the *basal*

*respiration rate* of 2A process TILs (see, for example Figure 7).

In general, second expansion TILs or second additional expansion TILs (such as, for example, those described in Step D of Figure 7, including TILs referred to as reREP TILs) have a spare respiratory capacity that is at least is at least 50%, at least 55%, at least 60%, at least 65%, at least 70%, at least 75%, at least 80%, at least 85%, at least 90%, at least 95%, at least 97%, at least 98%, or at least 99% of the basal respiration rate of 2A process TILs (see, for example Figure 7). The metabolic assay measured may be glycolytic reserve. The metabolic assay may be spare respiratory capacity. To measure cellular (respiratory) metabolism cells were treated with inhibitors of mitochondrial respiration and glycolysis to determine a metabolic profile for the TIL consisting of the following measures: baseline oxidative phosphorylation (as measured by OCR), spare respiratory capacity, baseline glycolytic activity (as measured by ECAR), and glycolytic reserve. Metabolic profiles were performed using the Seahorse Combination Mitochondrial/Glycolysis Stress Test Assay (including the kit commercially available from Agilent®), which allows for determining a cells' capacity to perform glycolysis upon blockage of mitochondrial ATP production. Cells may be starved of glucose, then glucose is injected, followed by a stress agent. The stress agent may be selected from the group consisting of oligomycin, FCCP, rotenone, antimycin A and/or 2-deoxyglucose (2-DG), as well as combinations thereof. Oligomycin may be added at 10 mM. FCCP may be added at 10 mM. Rotenone may be added at 2.5 mM. Antimycin A may be added at 2.5 mM. 2-deoxyglucose (2-DG) may be added at 500 mM. Glycolytic capacity, glycolytic reserve, and/or non-glycolytic acidification are measured. In general, TILs have a glycolytic reserve that is at least 50%, at least 55%, at least 60%, at least 65%, at least 70%, at least 75%, at least 80%, at least 85%, at least 90%, at least 95%, at least 97%, at least 98%, or at least 99% of the basal respiration rate of 2A process TILs (see, for example Figure 7). In some embodiments, the glycolytic reserve is from about 50% to about 99% of the *basal respiration rate* of 2A process TILs (see, for example Figure 7). In some embodiments, the glycolytic reserve is from about 60% to about 99% of the *basal respiration rate* of freshly harvested TILs. In some embodiments, the glycolytic reserve is from about 70% to about 99% of the *basal respiration rate* of 2A process TILs (see, for example Figure 7). In some embodiments, the glycolytic reserve is from about 80% to about 99% of the *basal respiration rate* of 2A process TILs (see, for example Figure 7). In some embodiments, the glycolytic reserve is from about 90% to about 99% of the *basal respiration rate* of 2A process TILs (see, for example Figure 7). In some embodiments, the glycolytic reserve is from about 95% to about 99% of the *basal respiration rate* of 2A process TILs (see, for example Figure 7).

The metabolic assay may be basal glycolysis. In some embodiments, second expansion TILs or second additional expansion TILs (such as, for example, those described in Step D of Figure 7, including TILs referred to as reREP TILs) have an increase in basal glycolysis of at least two-fold, at least three-fold, at least four-fold, at least five-fold, at least six-fold, at least 7-fold, at least eight-fold, at least nine-fold, or at least ten-fold. In some embodiments, the second expansion TILs or second additional expansion TILs (such as, for example, those described in Step D of Figure 7, including TILs referred to as reREP TILs) have an increase in basal glycolysis of about two-fold to about ten-fold. In some embodiments, the second expansion TILs or second additional expansion TILs (such as, for example, those described in Step D of Figure 7, including TILs referred to as reREP TILs) have an increase in basal glycolysis of about two-fold to about eight-fold. In some embodiments, second expansion TILs or second additional expansion TILs (such as, for example, those described in Step D of Figure 7, including TILs referred to as reREP TILs) have an increase in basal glycolysis of about three-fold to about seven-fold. In some embodiments, the second expansion TILs or second additional expansion TILs (such as, for example, those described in Step D of Figure 7, including TILs referred to as reREP TILs) have an increase in basal glycolysis of about two-fold to about four-fold. In some embodiments, the second expansion TILs or second additional expansion TILs (such as, for example, those described in Step D of Figure 7, including TILs referred to as reREP TILs) have an increase in basal glycolysis of about two-fold to about three-fold.

In general, the second expansion TILs or second additional expansion TILs (such as, for example, those described in Step D of Figure 7, including TILs referred to as reREP TILs) have a glycolytic reserve that is at least 50%, at least 55%, at least 60%, at least 65%, at least 70%, at least 75%, at least 80%, at least 85%, at least 90%, at least 95%, at least 97%, at least 98%, or at least 99% of the basal respiration rate of 2A process TILs (see, for example Figure 7). In some embodiments, the glycolytic reserve is from about 50% to about 99% of the *basal respiration rate* of 2A process TILs (see, for example Figure 7). In some embodiments, the glycolytic reserve is from about 60% to about 99% of the *basal respiration rate* of 2A process TILs (see, for example Figure 7). In some embodiments, the glycolytic reserve is from about 70% to about 99% of the *basal respiration rate* of 2A process TILs (see, for example Figure 7). In some embodiments, the glycolytic reserve is from about 80% to about 99% of the *basal respiration rate* of 2A process TILs (see, for example Figure 7). In some embodiments, the glycolytic reserve is from about 90% to about 99% of the *basal respiration rate* of 2A process TILs (see, for example Figure 7). In some embodiments, the glycolytic reserve is from about 95% to about 99% of the *basal respiration rate* of 2A process TILs (see, for example Figure 7).

Granzyme B Production: Granzyme B is another measure of the ability of TIL to kill target cells. Media supernatants restimulated as described above using antibodies to CD3, CD28, and CD137/4-1BB were also evaluated for their levels of Granzyme B using the Human Granzyme B DuoSet ELISA Kit (R & D Systems, Minneapolis, MN) according to the manufacturer's instructions.

Also disclosed herein but not claimed is an assay for assessing TIL viability, using the methods as described above. The TILs may be expanded as discussed above, including for example as provided in Figure 7. The TILs may be cryopreserved prior to being assessed for viability. The viability assessment includes thawing the TILs prior to performing a first expansion, a second expansion, and an additional second expansion. The methods disclosed herein may provide an assay for assessing cell proliferation, cell toxicity, cell death, and/or other terms related to viability of the TIL population. Viability can be measured by any of the TIL metabolic assays described above as well as any methods know for assessing cell viability that are known in the art. Certain methods disclosed herein may provide as assay for assessment of cell proliferation, cell toxicity, cell death, and/or other terms related to viability of the TILs expanded using the methods described herein, including those exemplified in Figure 7.

Also disclosed herein but not claimed are assay methods for determining TIL viability. The present disclosure provides methods for assaying TILs for viability by expanding tumor infiltrating lymphocytes (TILs) into a larger population of TILs comprising:

(i) obtaining a first population of TILs which has been previously expanded;

(ii) performing a first expansion by culturing the first population of TILs in a cell culture medium comprising IL-2 to produce a second population of TILs, wherein the cell culture medium is supplemented with OKT-3 at day 3, wherein the first expansion is performed for about 3 days to about 19 days in order to obtain the second population of TILs, wherein the second population of TILs comprises at least $5 \times 10^7$ TILs by about 10 days to about 19 days when the first population of TILs is from a core biopsy; and

(iii) performing a second expansion by supplementing the cell culture medium of the second population of TILs with additional IL-2, OKT-3, and antigen presenting cells (APCs), to produce a third population of TILs, wherein the third population of TILs is at least 100-fold greater in number than the second population of TILs, and wherein the second expansion is performed for about 11 days to about 14 days in order to obtain the third population of TILs, wherein the third population of TILs comprises an increased subpopulation of effector T cells and/or central memory T cells relative to the second population of TILs, and wherein the third population is further assayed for viability. In some embodiments, the method further comprises:

(iv) performing an additional second expansion by supplementing the cell culture medium of the third population of TILs with additional IL-2, additional OKT-3, and additional APCs, wherein the additional second expansion is performed for at least 14 days to obtain a larger population of TILs than obtained in step (iii), wherein the larger population of TILs comprises an increased subpopulation of effector T cells and/or central memory T cells relative to the third population of TILs, and wherein the third population is further assayed for viability.

In some methods disclosed herein, prior to step (i), the cells are cryopreserved.

In some methods disclosed herein, the cells are thawed prior to performing step (i).

In some methods disclosed herein, step (iv) is repeated one to four times in order to obtain sufficient TILs for analysis.

In some methods disclosed herein, steps (i) through (iii) or (iv) are performed within a period of about 21 days to about 33 days.

In some methods disclosed herein, steps (i) through (iii) or (iv) are performed within a period of about 21 days to about 30 days.

In some methods disclosed herein, steps (i) through (iii) or (iv) are performed within a period of about 21 days to about 28 days.

In some methods disclosed herein, steps (i) through (iii) or (iv) are performed within about 24 days.

In some methods disclosed herein, the cells from steps (iii) or (iv) express CD4, CD8, and TCR $\alpha$ $\beta$ at levels similar to freshly harvested cells.

In some methods disclosed herein, the antigen presenting cells are peripheral blood mononuclear cells (PBMCs).

In some methods disclosed herein, the PBMCs are added to the cell culture on any of days 9 through 17 in step (iii).

In some methods disclosed herein, the effector T cells and/or central memory T cells in the larger population of TILs in step (iv) exhibit one or more characteristics selected from the group consisting of expression of CD27, expression of CD28, longer telomeres, increased CD57 expression, and decreased CD56 expression, relative to effector T cells, and/or central memory T cells in the third population of cells.

In some methods disclosed herein, the effector T cells and/or central memory T cells exhibit increased CD57 expression and decreased CD56 expression.

In some methods disclosed herein, the APCs are artificial APCs (aAPCs).

In some methods disclosed herein, the method further comprises the step of transducing the first population of TILs with an expression vector comprising a nucleic acid encoding a high-affinity T cell receptor.

In some methods disclosed herein, the step of transducing occurs before step (i).

In some methods disclosed herein, the method further comprises the step of transducing the first population of TILs with an expression vector comprising a nucleic acid encoding a chimeric antigen receptor (CAR) comprising a single chain variable fragment antibody fused with at least one endodomain of a T-cell signaling molecule.

In some methods disclosed herein, the step of transducing occurs before step (i).

In some methods disclosed herein, the TILs are assayed for viability.

In some methods disclosed herein, the TILs are assayed for viability after cryopreservation.

In some methods disclosed herein, the TILs are assayed for viability after cryopreservation and after step (iv).

Also disclosed herein but not claimed is a method of treating a subject with cancer comprising administering expanded tumor infiltrating lymphocytes (TILs) comprising: (i) obtaining a first population of TILs from a fine needle aspirate (FNA) or a core biopsy obtained from a tumor in a patient; (ii) performing a first expansion by culturing the first population of TILs in a cell culture medium comprising IL-2 to produce a second population of TILs, wherein the cell culture medium is supplemented with OKT-3 at day 3, wherein the first expansion is performed for about 3 days to about 19 days in order to obtain the second population of TILs, wherein the second population of TILs comprises at least $5 \times 10^7$ TILs by about 10 days to about 19 days when the first population of TILs is from a core biopsy; (iii) performing a second expansion by supplementing the cell culture medium of the second population of TILs with additional IL-2, OKT-3, and antigen presenting cells (APCs), to produce a third population of TILs, wherein the third population of TILs is at least 25-fold greater in number than the second population of TILs, and wherein the second expansion is performed for about 11 days to about 14 days in order to obtain the third population of TILs, wherein the third population of TILs is a therapeutic population of TILs; and (iv) administering a therapeutically effective dosage of the third population of TILs to the patient.

According to the present disclosure, a method for assaying TILs for viability and/or further use in administration to a subject. The method for assay tumor infiltrating lymphocytes (TILs) may comprise:

(i) obtaining a first population of TILs;

(ii) performing a first expansion by culturing the first population of TILs in a cell culture medium comprising IL-2 to produce a second population of TILs, wherein the cell culture medium is supplemented with OKT-3 at day 3, wherein the first expansion is performed for about 3 days to about 19 days in order to obtain the second population of TILs, wherein the second population of TILs comprises at least $5 \times 10^7$ TILs by about 10 days to about 19 days when the first population of TILs is from a core biopsy; and

(iii) performing a second expansion by supplementing the cell culture medium of the second population of TILs with additional IL-2, OKT-3, and antigen presenting cells (APCs), to produce a third population of TILs, wherein the third population of TILs is at least 50-fold greater in number than the second population of TILs, and wherein the second expansion is performed for about 11 days to about 14 days in order to obtain the third population of TILs;

(iv) harvesting, washing, and cryopreserving the third population of TILs;

(v) storing the cryopreserved TILs at a cryogenic temperature;

(vi) thawing the third population of TILs to provide a thawed third population of TILs; and

(vii) performing an additional second expansion of a portion of the thawed third population of TILs by supplementing the cell culture medium of the third population with IL-2, OKT-3, and APCs for a reREP period of at least 3 days, wherein the third expansion is performed to obtain a fourth population of TILs, wherein the number of TILs in the fourth population of TILs is compared to the number of TILs in the third population of TILs to obtain a ratio;

(viii) determining based on the ratio in step (vii) whether the thawed population of TILs is suitable for administration to a patient;

(ix) administering a therapeutically effective dosage of the thawed population of TILs to the patient when the ratio of the number of TILs in the fourth population of TILs to the number of TILs in the third population of TILs is determined to be greater than 5:1 in step (viii).

In some methods disclosed herein, the reREP period is performed until the ratio of the number of TILs in the fourth population of TILs to the number of TILs in the third population of TILs is greater than 50:1.

In some methods disclosed herein, the number of TILs sufficient for a therapeutically effective dosage is from about $2.3\times10^{10}$ to about $13.7\times10^{10}$.

In some methods disclosed herein, steps (i) through (vii) are performed within a period of about 21 days to about 33 days. In some methods disclosed herein, steps (i) through (vii) are performed within a period of about 21 days to about 30 days. In some methods disclosed herein, steps (i) through (vii) are performed within a period of about 21 days to about 28 days. In some methods disclosed herein, steps (i) through (vii) are performed within about 24 days. In some methods disclosed herein, the cells from steps (iii) or (vii) express CD4, CD8, and TCR $\alpha$ $\beta$ at levels similar to freshly harvested cells. In some embodiments the cells are TILs.

In some methods disclosed herein, the antigen presenting cells are peripheral blood mononuclear cells (PBMCs). In some embodiments, the PBMCs are added to the cell culture on any of days 3 through 12 in step (ii) and/or any of days 11 through 14 in step (iii).

**In** some methods disclosed herein, the effector T cells and/or central memory T cells in the larger population of TILs in steps (iii) or (vii) exhibit one or more characteristics selected from the group consisting of expression of CD27, expression of CD28, longer telomeres, increased CD57 expression, and decreased CD56 expression, relative to effector T cells, and/or central memory T cells in the third population of cells.

In some methods disclosed herein, the effector T cells and/or central memory T cells exhibit increased CD57 expression and decreased CD56 expression.

In some methods disclosed herein, the APCs are artificial APCs (aAPCs).

In some methods disclosed herein, the step of transducing the first population of TILs with an expression vector comprising a nucleic acid encoding a high-affinity T cell receptor.

In some methods disclosed herein, the step of transducing occurs before step (i).

In some methods disclosed herein, the step of transducing the first population of TILs with an expression vector comprising a nucleic acid encoding a chimeric antigen receptor (CAR) comprising a single chain variable fragment antibody fused with at least one endodomain of a T-cell signaling molecule.

In some methods disclosed herein, the step of transducing occurs before step (i).

**In** some methods disclosed herein, the TILs are assayed for viability after step (vii).

The present disclosure also provides further methods for assaying TILs. The disclosure provides a method for assaying TILs comprising:

(i) obtaining a portion of a first population of cryopreserved TILs obtained from a core biopsy sample;

(ii) thawing the portion of the first population of cryopreserved TILs;

(iii) performing a first expansion by culturing the portion of the first population of TILs in a cell culture medium comprising IL-2, OKT-3, and antigen presenting cells (APCs) for a reREP period of at least 3 days, to produce a second population of TILs, wherein the portion from the first population of TILs is compared to the second population of TILs to obtain a ratio of the number of TILs, wherein the ratio of the number of TILs in the second population of TILs to the number of TILs in the portion of the first population of TILs is greater than 5:1, wherein the second population of TILs comprises at least $5 \times 10^7$ TILs by about 10 days to about 19 days;

(iv) determining based on the ratio in step (iii) and the number of TILs in step (iii) whether the first population of TILs is suitable for use in therapeutic administration to a patient;

(v) determining the first population of TILs is suitable for use in therapeutic administration when the ratio of the number of TILs in the second population of TILs to the number of TILs in the first population of TILs is determined to be greater than 5:1 in step (iv).

In some methods disclosed herein, the ratio of the number of TILs in the second population of TILs to the number of TILs in the portion of the first population of TILs is greater than 50:1.

In some methods disclosed herein, the method further comprises performing expansion of the entire first population of cryopreserved TILs from step (i) according to the methods as described in any of the embodiments provided herein.

In some methods disclosed herein, the method further comprises administering the entire first population of cryopreserved TILs from step (i) to the patient.

## IX. Closed Systems for TIL Manufacturing

The present invention provides for the use of closed systems during the TIL culturing process using Gen 2 processes or Gen 3 processes. Such closed systems allow for preventing and/or reducing microbial contamination, allow for the use of fewer flasks, and allow for cost reductions. In some embodiments, the closed system uses two containers.
Such closed systems are well-known in the art and can be found, for example, at http://www.fda.gov/cber/guidelines.htm and https://www.fda.gov/BiologicsBloodVaccines/GuidanceComplianceRegulatoryInformation/G uidances/Blood/ucm076779.htm.
Sterile connecting devices (STCDs) produce sterile welds between two pieces of compatible tubing. This procedure permits sterile connection of a variety of containers and tube diameters. In some embodiments, the closed systems include luer lock and heat sealed systems. In some embodiments, the closed system is accessed via syringes under sterile conditions in order to maintain the sterility and closed nature of the system. In some embodiments, a closed system is employed. In some embodiments, the TILs are formulated into a final product formulation container.
In some embodiments, the closed system uses one container from the time the tumor fragments are obtained until the TILs are ready for administration to the patient or cryopreserving. In some embodiments when two containers are used, the first container is a closed G-container and the population of TILs is centrifuged and transferred to an infusion bag without opening the first closed G-container. In some embodiments, when two containers are used, the infusion bag is a HypoThermosol-containing infusion bag. A closed system or closed TIL cell culture system is characterized in that once the tumor sample and/or tumor fragments have been added, the system is tightly sealed from the outside to form a closed environment free from the invasion of bacteria, fungi, and/or any other microbial contamination.
In some embodiments, the reduction in microbial contamination is between about 5% and about 100%. In some embodiments, the reduction in microbial contamination is between about 5% and about 95%. In some embodiments, the reduction in microbial contamination is between about 5% and about 90%. In some embodiments, the reduction in microbial contamination is between about 10% and about 90%. In some embodiments, the reduction in microbial contamination is between about 15% and about 85%. In some embodiments, the reduction in microbial contamination is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 97%, about 98%, about 99%, or about 100%.
The closed system allows for TIL growth in the absence and/or with a significant reduction in microbial contamination.
Moreover, pH, carbon dioxide partial pressure and oxygen partial pressure of the TIL cell culture environment each vary as the cells are cultured. Consequently, even though a medium appropriate for cell culture is circulated, the closed environment still needs to be constantly maintained as an optimal environment for TIL proliferation. To this end, it is desirable that the physical factors of pH, carbon dioxide partial pressure and oxygen partial pressure within the culture liquid of the closed environment be monitored by means of a sensor, the signal whereof is used to control a gas exchanger installed at the inlet of the culture environment, and the that gas partial pressure of the closed environment be adjusted in real time according to changes in the culture liquid so as to optimize the cell culture environment. In some embodiments, the present invention provides a closed cell culture system which incorporates at the inlet to the closed environment a gas exchanger equipped with a monitoring device which measures the pH, carbon dioxide partial pressure and oxygen partial pressure of the closed environment, and optimizes the cell culture environment by automatically adjusting gas concentrations based on signals from the monitoring device.
In some embodiments, the pressure within the closed environment is continuously or intermittently controlled. That is, the pressure in the closed environment can be varied by means of a pressure maintenance device for example, thus ensuring that the space is suitable for growth of TILs in a positive pressure state, or promoting exudation of fluid in a negative pressure state and thus promoting cell proliferation. By applying negative pressure intermittently, moreover, it is possible to uniformly and efficiently replace the circulating liquid in the closed environment by means of a temporary shrinkage in the volume of the closed environment.
In some embodiments, optimal culture components for proliferation of the TILs can be substituted or added, and including factors such as IL-2 and/or OKT3, as well as combination, can be added.

## X. Optional Genetic Engineering of TILs

In some embodiments of Gen 2 or Gen 3 processes, the expanded TILs of the present invention are further manipulated before, during, or after an expansion step, including during closed, sterile manufacturing processes, each as provided herein, in order to alter protein expression in a transient manner. In some embodiments, the transiently altered protein expression is due to transient gene editing. In some embodiments, the expanded TILs of the present invention are treated with transcription factors (TFs) and/or other molecules capable of transiently altering protein expression in the TILs. In some embodiments, the TFs and/or other molecules that are capable of transiently altering protein expression provide for altered expression of tumor antigens and/or an alteration in the number of tumor antigen-specific T cells in a population of TILs.

In certain embodiments, the method comprises genetically editing a population of TILs. In certain embodiments, the method comprises genetically editing the first population of TILs, the second population of TILs and/or the third population of TILs.

In some embodiments, the present invention includes genetic editing through nucleotide insertion, such as through ribonucleic acid (RNA) insertion, including insertion of messenger RNA (mRNA) or small (or short) interfering RNA (siRNA), into a population of TILs for promotion of the expression of one or more proteins or inhibition of the expression of one or more proteins, as well as simultaneous combinations of both promotion of one set of proteins with inhibition of another set of proteins.

In some embodiments, the expanded TILs of the present invention undergo transient alteration of protein expression. In some embodiments, the transient alteration of protein expression occurs in the bulk TIL population prior to first expansion, including, for example in the TIL population obtained from for example, Step A as indicated in Figure 85 (particularly Figure 85B). In some embodiments, the transient alteration of protein expression occurs during the first expansion, including, for example in the TIL population expanded in for example, Step B as indicated in Figure 85 (for example Figure 85B). In some embodiments, the transient alteration of protein expression occurs after the first expansion, including, for example in the TIL population in transition between the first and second expansion (*e.g.* the second population of TILs as described herein), the TIL population obtained from for example, Step B and included in Step C as indicated in Figure 85. In some embodiments, the transient alteration of protein expression occurs in the bulk TIL population prior to second expansion, including, for example in the TIL population obtained from for example, Step C and prior to its expansion in Step D as indicated in Figure 85. In some embodiments, the transient alteration of protein expression occurs during the second expansion, including, for example in the TIL population expanded in for example, Step D as indicated in Figure 85 (*e.g.* the third population of TILs). In some embodiments, the transient alteration of protein expression occurs after the second expansion, including, for example in the TIL population obtained from the expansion in for example, Step D as indicated in Figure 85.

**In** an embodiment, a method of transiently altering protein expression in a population of TILs includes the step of electroporation. Electroporation methods are known in the art and are described, *e.g.,* in Tseng, Biophys. J. 1991, 60, 297-306, and U.S. Patent Application Publication No. 2014/0227237 A1. In an embodiment, a method of transiently altering protein expression in population of TILs includes the step of calcium phosphate transfection. Calcium phosphate transfection methods (calcium phosphate DNA precipitation, cell surface coating, and endocytosis) are known in the art and are described in Graham and van der Eb, Virology 1973, 52, 456-467; Wigler, et al., Proc. Natl. Acad. Sci. 1979, 76, 1373-1376; and Chen and Okayarea, Mol. Cell. Biol. 1987, 7, 2745-2752; and in U.S. Patent No. 5,593,875. In an embodiment, a method of transiently altering protein expression in a population of TILs includes the step of liposomal transfection. Liposomal transfection methods, such as methods that employ a 1:1 (w/w) liposome formulation of the cationic lipid *N*-[1-(2,3-dioleyloxy)propyl]-*n*,*n*,*n*-trimethylammonium chloride (DOTMA) and dioleoyl phophotidylethanolamine (DOPE) in filtered water, are known in the art and are described in Rose, et al., Biotechniques 1991, 10, 520-525 and Felgner, et al., Proc. Natl. Acad. Sci. USA, 1987, 84, 7413-7417 and in U.S. Patent Nos. 5,279,833; 5,908,635; 6,056,938; 6,110,490; 6,534,484; and 7,687,070. In an embodiment, a method of transiently altering protein expression in a population of TILs includes the step of transfection using methods described in U.S. Patent Nos. 5,766,902; 6,025,337; 6,410,517; 6,475,994; and 7,189,705.

In some embodiments, transient alteration of protein expression results in an increase in Stem Memory T cells (TSCMs). TSCMs are early progenitors of antigen-experienced central memory T cells. TSCMs generally display the long-term survival, self-renewal, and multipotency abilities that define stem cells, and are generally desirable for the generation of effective TIL products. TSCM have shown enhanced anti-tumor activity compared with other T cell subsets in mouse models of adoptive cell transfer (Gattinoni et al. Nat Med 2009, 2011; Gattinoni, Nature Rev. Cancer, 2012; Cieri et al. Blood 2013). In some embodiments, transient alteration of protein expression results in a TIL population with a composition comprising a high proportion of TSCM. In some embodiments, transient alteration of protein expression results in an at least 5%, at least 10%, at least 10%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% increase in TSCM percentage. In some embodiments, transient alteration of protein expression results in an at least a 1-fold, 2-fold, 3-fold, 4-fold, 5-fold, or 10-fold increase in TSCMs in the TIL population. In some embodiments, transient alteration of protein expression results in a TIL population with at least at least 5%, at least 10%, at least 10%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% TSCMs. In some embodiments, transient alteration of protein expression results in a therapeutic TIL population with at least at least 5%, at least 10%, at least 10%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% TSCMs.

In some embodiments, transient alteration of protein expression results in rejuvenation of antigen-experienced T-cells. In some embodiments, rejuvenation includes, for example, increased proliferation, increased T-cell activation, and/or increased antigen recognition.

In some embodiments, transient alteration of protein expression alters the expression in a large fraction of the T-cells in order to preserve the tumor-derived TCR repertoire. In some embodiments, transient alteration of protein expression does not alter the tumor-derived TCR repertoire. In some embodiments, transient alteration of protein expression maintains the tumor-derived TCR repertoire.

**In** some embodiments, transient alteration of protein results in altered expression of a particular gene. In some embodiments, the transient alteration of protein expression targets a gene including but not limited to PD-1 (also referred to as PDCD1 or CC279), TGFBR2, CCR4/5, CBLB (CBL-B), CISH, CCRs (chimeric co-stimulatory receptors), IL-2, IL-12, IL-15, IL-21, NOTCH 1/2 ICD, TIM3, LAG3, TIGIT, TGFβ, CCR2, CCR4, CCR5, CXCR1, CXCR2, CSCR3, CCL2 (MCP-1), CCL3 (MIP-1α), CCL4 (MIP1-β), CCL5 (RANTES), CXCL1/CXCL8, CCL22, CCL17, CXCL1/CXCL8, VHL, CD44, PIK3CD, SOCS1, and/or cAMP protein kinase A (PKA). In some embodiments, the transient alteration of protein expression targets a gene selected from the group consisting of PD-1, TGFBR2, CCR4/5, CBLB (CBL-B), CISH, CCRs (chimeric co-stimulatory receptors), IL-2, IL-12, IL-15, IL-21, NOTCH 1/2 ICD, TIM3, LAG3, TIGIT, TGFβ, CCR2, CCR4, CCR5, CXCR1, CXCR2, CSCR3, CCL2 (MCP-1), CCL3 (MIP-1α), CCL4 (MIP1-β), CCL5 (RANTES), CXCL1/CXCL8, CCL22, CCL17, CXCL1/CXCL8, VHL, CD44, PIK3CD, SOCS1, and/or cAMP protein kinase A (PKA). In some embodiments, the transient alteration of protein expression targets PD-1. In some embodiments, the transient alteration of protein expression targets TGFBR2. In some embodiments, the transient alteration of protein expression targets CCR4/5. In some embodiments, the transient alteration of protein expression targets CBLB. In some embodiments, the transient alteration of protein expression targets CISH. In some embodiments, the transient alteration of protein expression targets CCRs (chimeric co-stimulatory receptors). In some embodiments, the transient alteration of protein expression targets IL-2. In some embodiments, the transient alteration of protein expression targets IL-12. In some embodiments, the transient alteration of protein expression targets IL-15. In some embodiments, the transient alteration of protein expression targets IL-21. In some embodiments, the transient alteration of protein expression targets NOTCH 1/2 ICD. In some embodiments, the transient alteration of protein expression targets TIM3. In some embodiments, the transient alteration of protein expression targets LAG3. In some embodiments, the transient alteration of protein expression targets TIGIT. In some embodiments, the transient alteration of protein expression targets TGFβ. In some embodiments, the transient alteration of protein expression targets CCR1. In some embodiments, the transient alteration of protein expression targets CCR2. In some embodiments, the transient alteration of protein expression targets CCR4. In some embodiments, the transient alteration of protein expression targets CCR5. In some embodiments, the transient alteration of protein expression targets CXCR1. In some embodiments, the transient alteration of protein expression targets CXCR2. In some embodiments, the transient alteration of protein expression targets CSCR3. In some embodiments, the transient alteration of protein expression targets CCL2 (MCP-1). In some embodiments, the transient alteration of protein expression targets CCL3 (MIP-1α). In some embodiments, the transient alteration of protein expression targets CCL4 (MIP1-β). In some embodiments, the transient alteration of protein expression targets CCL5 (RANTES). In some embodiments, the transient alteration of protein expression targets CXCL1. In some embodiments, the transient alteration of protein expression targets CXCL8. In some embodiments, the transient alteration of protein expression targets CCL22. In some embodiments, the transient alteration of protein expression targets CCL17. In some embodiments, the transient alteration of protein expression targets VHL. In some embodiments, the transient alteration of protein expression targets CD44. In some embodiments, the transient alteration of protein expression targets PIK3CD. In some embodiments, the transient alteration of protein expression targets SOCS1. In some embodiments, the transient alteration of protein expression targets cAMP protein kinase A (PKA).

In some embodiments, the transient alteration of protein expression results in increased and/or overexpression of a chemokine receptor. In some embodiments, the chemokine receptor that is overexpressed by transient protein expression includes a receptor with a ligand that includes but is not limited to CCL2 (MCP-1), CCL3 (MIP-1α), CCL4 (MIP1-β), CCL5 (RANTES), CXCL1, CXCL8, CCL22, and/or CCL17.

In some embodiments, the transient alteration of protein expression results in a decrease and/or reduced expression of PD-1, CTLA-4, TIM-3, LAG-3, TIGIT, TGFβR2, and/or TGFβ (including resulting in, for example, TGFβ pathway blockade). In some embodiments, the transient alteration of protein expression results in a decrease and/or reduced expression of CBLB (CBL-B). In some embodiments, the transient alteration of protein expression results in a decrease and/or reduced expression of CISH.

In some embodiments, the transient alteration of protein expression results in increased and/or overexpression of chemokine receptors in order to, for example, improve TIL trafficking or movement to the tumor site. In some embodiments, the transient alteration of protein expression results in increased and/or overexpression of a CCR (chimeric co-stimulatory receptor). In some embodiments, the transient alteration of protein expression results in increased and/or overexpression of a chemokine receptor selected from the group consisting of CCR1, CCR2, CCR4, CCR5, CXCR1, CXCR2, and/or CSCR3.

In some embodiments, the transient alteration of protein expression results in increased and/or overexpression of an interleukin. In some embodiments, the transient alteration of protein expression results in increased and/or overexpression of an interleukin selected from the group consisting of IL-2, IL-12, IL-15, and/or IL-21.

In some embodiments, the transient alteration of protein expression results in increased and/or overexpression of NOTCH 1/2 ICD. In some embodiments, the transient alteration of protein expression results in increased and/or overexpression of VHL. In some embodiments, the transient alteration of protein expression results in increased and/or overexpression of CD44. In some embodiments, the transient alteration of protein expression results in increased and/or overexpression of PIK3CD. In some embodiments, the transient alteration of protein expression results in increased and/or overexpression of SOCS1,

In some embodiments, the transient alteration of protein expression results in decreased and/or reduced expression of cAMP protein kinase A (PKA).

In some embodiments, the transient alteration of protein expression results in decreased and/or reduced expression of a molecule selected from the group consisting of PD-1, LAG3, TIM3, CTLA-4, TIGIT, CISH, TGFβR2, PKA, CBLB, BAFF (BR3), and combinations thereof. In some embodiments, the transient alteration of protein expression results in decreased and/or reduced expression of two molecules selected from the group consisting of PD-1, LAG3, TIM3, CTLA-4, TIGIT, CISH, TGFβR2, PKA, CBLB, BAFF (BR3), and combinations thereof. In some embodiments, the transient alteration of protein expression results in decreased and/or reduced expression of PD-1 and one molecule selected from the group consisting of LAG3, TIM3, CTLA-4, TIGIT, CISH, TGFβR2, PKA, CBLB, BAFF (BR3), and combinations thereof. In some embodiments, the transient alteration of protein expression results in decreased and/or reduced expression of PD-1, LAG-3, CISH, CBLB, TIM3, and combinations thereof. In some embodiments, the transient alteration of protein expression results in decreased and/or reduced expression of PD-1 and one of LAG3, CISH, CBLB, TIM3, and combinations thereof. In some embodiments, the transient alteration of protein expression results in decreased and/or reduced expression of PD-1 and LAG3. In some embodiments, the transient alteration of protein expression results in decreased and/or reduced expression of PD-1 and CISH. In some embodiments, the transient alteration of protein expression results in decreased and/or reduced expression of PD-1 and CBLB. In some embodiments, the transient alteration of protein expression results in decreased and/or reduced expression of LAG3 and CISH. In some embodiments, the transient alteration of protein expression results in decreased and/or reduced expression of LAG3 and CBLB. In some embodiments, the transient alteration of protein expression results in decreased and/or reduced expression of CISH and CBLB. In some embodiments, the transient alteration of protein expression results in decreased and/or reduced expression of TIM3 and PD-1. In some embodiments, the transient alteration of protein expression results in decreased and/or reduced expression of TIM3 and LAG3. In some embodiments, the transient alteration of protein expression results in decreased and/or reduced expression of TIM3 and CISH. In some embodiments, the transient alteration of protein expression results in decreased and/or reduced expression of TIM3 and CBLB.

In some embodiments, an adhesion molecule selected from the group consisting of CCR2, CCR4, CCR5, CXCR2, CXCR3, CX3CR1, and combinations thereof, is inserted by a gammaretroviral or lentiviral method into the first population of TILs, second population of TILs, or harvested population of TILs (*e.g.,* the expression of the adhesion molecule is increased).

In some embodiments, the transient alteration of protein expression results in decreased and/or reduced expression of a molecule selected from the group consisting of PD-1, LAG3, TIM3, CTLA-4, TIGIT, CISH, TGFβR2, PKA, CBLB, BAFF (BR3), and combinations thereof, and increased and/or enhanced expression of CCR2, CCR4, CCR5, CXCR2, CXCR3, CX3CR1, and combinations thereof. In some embodiments, the transient alteration of protein expression results in decreased and/or reduced expression of a molecule selected from the group consisting of PD-1, LAG3, TIM3, CISH, CBLB, and combinations thereof, and increased and/or enhanced expression of CCR2, CCR4, CCR5, CXCR2, CXCR3, CX3CR1, and combinations thereof.

**In** some embodiments, there is a reduction in expression of about 5%, about 10%, about 10%, about 20%, about 25%, about 30%, about 35%, about 40%, about 45%, about 50%, about 55%, about 60%, about 65%, about 70%, about 75%, about 80%, about 85%, about 90%, or about 95%. In some embodiments, there is a reduction in expression of at least about 65%, about 70%, about 75%, about 80%, about 85%, about 90%, or about 95%. In some embodiments, there is a reduction in expression of at least about 75%, about 80%, about 85%, about 90%, or about 95%. In some embodiments, there is a reduction in expression of at least about 80%, about 85%, about 90%, or about 95%. In some embodiments, there is a reduction in expression of at least about 85%, about 90%, or about 95%. In some embodiments, there is a reduction in expression of at least about 80%. In some embodiments, there is a reduction in expression of at least about 85%, In some embodiments, there is a reduction in expression of at least about 90%. In some embodiments, there is a reduction in expression of at least about 95%. In some embodiments, there is a reduction in expression of at least about 99%.

In some embodiments, there is an increase in expression of about 5%, about 10%, about 10%, about 20%, about 25%, about 30%, about 35%, about 40%, about 45%, about 50%, about 55%, about 60%, about 65%, about 70%, about 75%, about 80%, about 85%, about 90%, or about 95%. In some embodiments, there is an increase in expression of at least about 65%, about 70%, about 75%, about 80%, about 85%, about 90%, or about 95%. In some embodiments, there is an increase in expression of at least about 75%, about 80%, about 85%, about 90%, or about 95%. In some embodiments, there is an increase in expression of at least about 80%, about 85%, about 90%, or about 95%. In some embodiments, there is an increase in expression of at least about 85%, about 90%, or about 95%. In some embodiments, there is an increase in expression of at least about 80%. In some embodiments, there is an increase in expression of at least about 85%, In some embodiments, there is an increase in expression of at least about 90%. In some embodiments, there is an increase in expression of at least about 95%. In some embodiments, there is an increase in expression of at least about 99%.

In some embodiments, transient alteration of protein expression is induced by treatment of the TILs with transcription factors (TFs) and/or other molecules capable of transiently altering protein expression in the TILs. In some embodiments, the SQZ vector-free microfluidic platform is employed for intracellular delivery of the transcription factors (TFs) and/or other molecules capable of transiently altering protein expression. Such methods demonstrating the ability to deliver proteins, including transcription factors, to a variety of primary human cells, including T cells (Sharei et al. PNAS 2013, as well as Sharei et al., PLOS ONE 2015 and Greisbeck et al., J. Immunology vol. 195, 2015) have been described; see, for example, International Patent Publications WO 2013/059343A1, WO 2017/008063A1, and WO 2017/123663A1. Such methods as described in International Patent Publications WO 2013/059343A1, WO 2017/008063A1, and WO 2017/123663A1 can be employed with the present invention in order to expose a population of TILs to transcription factors (TFs) and/or other molecules capable of inducing transient protein expression, wherein said TFs and/or other molecules capable of inducing transient protein expression provide for increased expression of tumor antigens and/or an increase in the number of tumor antigen-specific T cells in the population of TILs, thus resulting in reprogramming of the TIL population and an increase in therapeutic efficacy of the reprogrammed TIL population as compared to a non-reprogrammed TIL population. In some embodiments, the reprogramming results in an increased subpopulation of effector T cells and/or central memory T cells relative to the starting or prior population (i.e., prior to reprogramming) population of TILs, as described herein.

In some embodiments, the transcription factor (TF) includes but is not limited to TCF-1, NOTCH 1/2 ICD, and/or MYB. In some embodiments, the transcription factor (TF) is TCF-1. In some embodiments, the transcription factor (TF) is NOTCH 1/2 ICD. In some embodiments, the transcription factor (TF) is MYB. In some embodiments, the transcription factor (TF) is administered with induced pluripotent stem cell culture (iPSC), such as the commercially available KNOCKOUT Serum Replacement (Gibco/ThermoFisher), to induce additional TIL reprogramming. In some embodiments, the transcription factor (TF) is administered with an iPSC cocktail to induce additional TIL reprogramming. In some embodiments, the transcription factor (TF) is administered without an iPSC cocktail. In some embodiments, reprogramming results in an increase in the percentage of TSCMs. In some embodiments, reprogramming results in an increase in the percentage of TSCMs by about 5%, about 10%, about 10%, about 20%, about 25%, about 30%, about 35%, about 40%, about 45%, about 50%, about 55%, about 60%, about 65%, about 70%, about 75%, about 80%, about 85%, about 90%, or about 95% TSCMs.

In some embodiments, a method of transient altering protein expression, as described above, may be combined with a method of genetically modifying a population of TILs includes the step of stable incorporation of genes for production of one or more proteins. In certain embodiments, the method comprises a step of genetically modifying a population of TILs. In certain embodiments, the method comprises genetically modifying the first population of TILs, the second population of TILs and/or the third population of TILs. In an embodiment, a method of genetically modifying a population of TILs includes the step of retroviral transduction. In an embodiment, a method of genetically modifying a population of TILs includes the step of lentiviral transduction. Lentiviral transduction systems are known in the art and are described, *e.g.,* in Levine, et al., Proc. Nat'l Acad. Sci. 2006,103, 17372-77; Zufferey, et al., Nat. Biotechnol. 1997, 15, 871-75; Dull, et al., J. Virology 1998, 72, 8463-71, and U.S. Patent No. 6,627,442. In an embodiment, a method of genetically modifying a population of TILs includes the step of gamma-retroviral transduction. Gamma-retroviral transduction systems are known in the art and are described, *e.g.,* Cepko and Pear, Cur. Prot. Mol. Biol. 1996, 9.9.1-9.9.16. In an embodiment, a method of genetically modifying a population of TILs includes the step of transposon-mediated gene transfer. Transposon-mediated gene transfer systems are known in the art and include systems wherein the transposase is provided as DNA expression vector or as an expressible RNA or a protein such that long-term expression of the transposase does not occur in the transgenic cells, for example, a transposase provided as an mRNA *(e.g.,* an mRNA comprising a cap and poly-A tail). Suitable transposon-mediated gene transfer systems, including the salmonid-type Tel-like transposase (SB or Sleeping Beauty transposase), such as SB10, SB11, and SB100x, and engineered enzymes with increased enzymatic activity, are described in, *e.g.,* Hackett, et al., Mol. Therapy 2010, 18, 674-83 and U.S. Patent No. 6,489,458.

In some embodiments, transient alteration of protein expression is a reduction in expression induced by self-delivering RNA interference (sdRNA), which is a chemically-synthesized asymmetric siRNA duplex with a high percentage of 2'-OH substitutions (typically fluorine or $-OCH_3$) which comprises a 20-nucleotide antisense (guide) strand and a 13 to 15 base sense (passenger) strand conjugated to cholesterol at its 3' end using a tetraethylenglycol (TEG) linker. In some embodiments, the method comprises transient alteration of protein expression in a population of TILs, comprising the use of self-delivering RNA interference (sdRNA), which is a chemically-synthesized asymmetric siRNA duplex with a high percentage of 2'-OH substitutions (typically fluorine or $-OCH_3$) which comprises a 20-nucleotide antisense (guide) strand and a 13 to 15 base sense (passenger) strand conjugated to cholesterol at its 3' end using a tetraethylenglycol (TEG) linker. Methods of using sdRNA have been described in Khvorova and Watts, Nat. Biotechnol. 2017, 35, 238-248; Byrne, et al., J. Ocul. Pharmacol. Ther. 2013, 29, 855-864; and Ligtenberg, et al., Mol. Therapy, 2018, in press. In an embodiment, delivery of sdRNA to a TIL population is accomplished without use of electroporation, SQZ, or other methods, instead using a 1 to 3 day period in which a TIL population is exposed to sdRNA at a concentration of 1 μM/10,000 TILs in medium. In certain embodiments, the method comprises delivery sdRNA to a TILs population comprising exposing the TILs population to sdRNA at a concentration of 1 μM/10,000 TILs in medium for a period of between 1 to 3 days. In an embodiment, delivery of sdRNA to a TIL population is accomplished using a 1 to 3 day period in which a TIL population is exposed to sdRNA at a concentration of 10 μM/10,000 TILs in medium. In an embodiment, delivery of sdRNA to a TIL population is accomplished using a 1 to 3 day period in which a TIL population is exposed to sdRNA at a concentration of 50 μM/10,000 TILs in medium. In an embodiment, delivery of sdRNA to a TIL population is accomplished using a 1 to 3 day period in which a TIL population is exposed to sdRNA at a concentration of between 0.1 μM/10,000 TILs and 50 μM/10,000 TILs in medium. In an embodiment, delivery of sdRNA to a TIL population is accomplished using a 1 to 3 day period in which a TIL population is exposed to sdRNA at a concentration of between 0.1 μM/10,000 TILs and 50 μM/10,000 TILs in medium, wherein the exposure to sdRNA is performed two, three, four, or five times by addition of fresh sdRNA to the media. Other suitable processes are described, for example, in U.S. Patent Application Publication No. US 2011/0039914 A1, US 2013/0131141 A1, and US 2013/0131142 A1, and U.S. Patent No. 9,080,171.

In some embodiments, sdRNA is inserted into a population of TILs during manufacturing. In some embodiments, the sdRNA encodes RNA that interferes with NOTCH 1/2 ICD, PD-1, CTLA-4 TIM-3, LAG-3, TIGIT, TGFβ, TGFBR2, cAMP protein kinase A (PKA), BAFF BR3, CISH, and/or CBLB. In some embodiments, the reduction in expression is determined based on a percentage of gene silencing, for example, as assessed by flow cytometry and/or qPCR. In some embodiments, there is a reduction in expression of about 5%, about 10%, about 10%, about 20%, about 25%, about 30%, about 35%, about 40%, about 45%, about 50%, about 55%, about 60%, about 65%, about 70%, about 75%, about 80%, about 85%, about 90%, or about 95%. In some embodiments, there is a reduction in expression of at least about 65%, about 70%, about 75%, about 80%, about 85%, about 90%, or about 95%. In some embodiments, there is a reduction in expression of at least about 75%, about 80%, about 85%, about 90%, or about 95%. In some embodiments, there is a reduction in expression of at least about 80%, about 85%, about 90%, or about 95%. In some embodiments, there is a reduction in expression of at least about 85%, about 90%, or about 95%. In some embodiments, there is a reduction in expression of at least about 80%. In some embodiments, there is a reduction in expression of at least about 85%, In some embodiments, there is a reduction in expression of at least about 90%. In some embodiments, there is a reduction in expression of at least about 95%. In some embodiments, there is a reduction in expression of at least about 99%.

The self-deliverable RNAi technology based on the chemical modification of siRNAs can be employed with the methods of the present invention to successfully deliver the sdRNAs to the TILs as described herein. The combination of backbone modifications with asymmetric siRNA structure and a hydrophobic ligand *(see,* for eample, Ligtenberg, et al., Mol. Therapy, 2018 and US20160304873) allow sdRNAs to penetrate cultured mammalian cells without additional formulations and methods by simple addition to the culture media, capitalizing on the nuclease stability of sdRNAs. This stability allows the support of constant levels of RNAi-mediated reduction of target gene activity simply by maintaining the active concentration of sdRNA in the media. While not being bound by theory, the backbone stabilization of sdRNA provides for extended reduction in gene expression effects which can last for months in non-dividing cells.

In some embodiments, over 95% transfection efficiency of TILs and a reduction in expression of the target by various specific sdRNA occurs. In some embodiments, sdRNAs containing several unmodified ribose residues were replaced with fully modified sequences to increase potency and/or the longevity of RNAi effect. In some embodiments, a reduction in expression effect is maintained for 12 hours, 24 hours, 36 hours, 48 hours, 5 days, 6 days, 7 days, or 8 days or more. In some embodiments, the reduction in expression effect decreases at 10 days or more post sdRNA treatment of the TILs. In some embodiments, more than 70% reduction in expression of the target expression is maintained. In some embodiments, more than 70% reduction in expression of the target expression is maintained TILs. In some embodiments, a reduction in expression in the PD-1/PD-L1 pathway allows for the TILs to exhibit a more potent *in vivo* effect, which is in some embodiments, due to the avoidance of the suppressive effects of the PD-1/PD-L1 pathway. In some embodiments, a reduction in expression of PD-1 by sdRNA results in an increase TIL proliferation.

Small interfering RNA (siRNA), sometimes known as short interfering RNA or silencing RNA, is a double stranded RNA molecule, generally 19-25 base pairs in length. siRNA is used in RNA interference (RNAi), where it interferes with expression of specific genes with complementary nucleotide sequences.

Double stranded DNA (dsRNA) can be generally used to define any molecule comprising a pair of complementary strands of RNA, generally a sense (passenger) and antisense (guide) strands, and may include single-stranded overhang regions. The term dsRNA, contrasted with siRNA, generally refers to a precursor molecule that includes the sequence of an siRNA molecule which is released from the larger dsRNA molecule by the action of cleavage enzyme systems, including Dicer.

sdRNA (self-deliverable RNA) are a new class of covalently modified RNAi compounds that do not require a delivery vehicle to enter cells and have improved pharmacology compared to traditional siRNAs. "Self-deliverable RNA" or "sdRNA" is a hydrophobically modified RNA interfering-antisense hybrid, demonstrated to be highly efficacious *in vitro* in primary cells and *in vivo* upon local administration. Robust uptake and/or silencing without toxicity has been demonstrated. sdRNAs are generally asymmetric chemically modified nucleic acid molecules with minimal double stranded regions. sdRNA molecules typically contain single stranded regions and double stranded regions, and can contain a variety of chemical modifications within both the single stranded and double stranded regions of the molecule. Additionally, the sdRNA molecules can be attached to a hydrophobic conjugate such as a conventional and advanced sterol-type molecule, as described herein. sdRNAs and associated methods for making such sdRNAs have also been described extensively in, for example, US20160304873, WO2010033246, WO2017070151, WO2009102427, WO2011119887, WO2010033247A2, WO2009045457, WO2011119852. To optimize sdRNA structure, chemistry, targeting position, sequence preferences, and the like, a proprietary algorithm has been developed and utilized for sdRNA potency prediction (*see,* for example, US 20160304873). Based on these analyses, functional sdRNA sequences have been generally defined as having over 70% reduction in expression at 1 μM concentration, with a probability over 40%.

In some embodiments, the sdRNA sequences used in the invention exhibit a 70% reduction in expression of the target gene. In some embodiments, the sdRNA sequences used in the invention exhibit a 75% reduction in expression of the target gene.

In some embodiments, the sdRNA sequences used in the invention exhibit an 80% reduction in expression of the target gene. In some embodiments, the sdRNA sequences used in the invention exhibit an 85% reduction in expression of the target gene. In some embodiments, the sdRNA sequences used in the invention exhibit a 90% reduction in expression of the target gene. In some embodiments, the sdRNA sequences used in the invention exhibit a 95% reduction in expression of the target gene. In some embodiments, the sdRNA sequences used in the invention exhibit a 99% reduction in expression of the target gene. In some embodiments, the sdRNA sequences used in the invention exhibit a reduction in expression of the target gene when delivered at a concentration of about 0.25 μM to about 4 μM. In some embodiments, the sdRNA sequences used in the invention exhibit a reduction in expression of the target gene when delivered at a concentration of about 0.25 μM. In some embodiments, the sdRNA sequences used in the invention exhibit a reduction in expression of the target gene when delivered at a concentration of about 0.5 μM. In some embodiments, the sdRNA sequences used in the invention exhibit a reduction in expression of the target gene when delivered at a concentration of about 0.75 μM. In some embodiments, the sdRNA sequences used in the invention exhibit a reduction in expression of the target gene when delivered at a concentration of about 1.0 μM. In some embodiments, the sdRNA sequences used in the invention exhibit a reduction in expression of the target gene when delivered at a concentration of about 1.25 μM. In some embodiments, the sdRNA sequences used in the invention exhibit a reduction in expression of the target gene when delivered at a concentration of about 1.5 μM. In some embodiments, the sdRNA sequences used in the invention exhibit a reduction in expression of the target gene when delivered at a concentration of about 1.75 μM. In some embodiments, the sdRNA sequences used in the invention exhibit a reduction in expression of the target gene when delivered at a concentration of about 2.0 μM. In some embodiments, the sdRNA sequences used in the invention exhibit a reduction in expression of the target gene when delivered at a concentration of about 2.25 μM. In some embodiments, the sdRNA sequences used in the invention exhibit a reduction in expression of the target gene when delivered at a concentration of about 2.5 μM. In some embodiments, the sdRNA sequences used in the invention exhibit a reduction in expression of the target gene when delivered at a concentration of about 2.75 μM. In some embodiments, the sdRNA sequences used in the invention exhibit a reduction in expression of the target gene when delivered at a concentration of about 3.0 μM. In some embodiments, the sdRNA sequences used in the invention exhibit a reduction in expression of the target gene when delivered at a concentration of about 3.25 μM. In some embodiments, the sdRNA sequences used in the invention exhibit a reduction in expression of the target gene when delivered at a concentration of about 3.5 μM. In some embodiments, the sdRNA sequences used in the invention exhibit a reduction in expression of the target gene when delivered at a concentration of about 3.75 μM. In some embodiments, the sdRNA sequences used in the invention exhibit a reduction in expression of the target gene when delivered at a concentration of about 4.0 μM.

In some emodiments, the oligonucleotide agents comprise one or more modification to increase stability and/or effectiveness of the therapeutic agent, and to effect efficient delivery of the oligonucleotide to the cells or tissue to be treated. Such modifications can include a 2'-O-methyl modification, a 2'-O-Fluro modification, a diphosphorothioate modification, 2' F modified nucleotide, a2'-O-methyl modified and/or a 2'deoxy nucleotide. In some embodiments, the oligonucleotide is modified to include one or more hydrophobic modifications including, for example, sterol, cholesterol, vitamin D, naphtyl, isobutyl, benzyl, indol, tryptophane, and/or phenyl. In an additional particular embodiment, chemically modified nucleotides are combination of phosphorothioates, 2'-O-methyl, 2'deoxy, hydrophobic modifications and phosphorothioates. In some embodiments, the sugars can be modified and modified sugars can include but are not limited to D-ribose, 2'-O-alkyl (including 2'-O-methyl and 2'-0-ethyl), i.e., 2'-alkoxy, 2'-amino, 2'-S-alkyl, 2'-halo (including 2'-fluoro), T- methoxyethoxy, 2'-allyloxy (-$OCH_2CH=CH_2$), 2'-propargyl, 2'-propyl, ethynyl, ethenyl, propenyl, and cyano and the like. In one embodiment, the sugar moiety can be a hexose and incorporated into an oligonucleotide as described (Augustyns, K., et al., Nucl. Acids. Res. 18:4711 (1992)).

In some embodiments, the double-stranded oligonucleotide of the invention is double-stranded over its entire length, *i.e.,* with no overhanging single-stranded sequence at either end of the molecule, *i.e.,* is blunt-ended. In some embodiments, the individual nucleic acid molecules can be of different lengths. In other words, a double-stranded oligonucleotide of the invention is not double-stranded over its entire length. For instance, when two separate nucleic acid molecules are used, one of the molecules, *e.g.,* the first molecule comprising an antisense sequence, can be longer than the second molecule hybridizing thereto (leaving a portion of the molecule single-stranded). In some embodiments, when a single nucleic acid molecule is used a portion of the molecule at either end can remain single-stranded.

In some embodiments, a double-stranded oligonucleotide of the invention contains mismatches and/or loops or bulges, but is double-stranded over at least about 70% of the length of the oligonucleotide. In some embodiments, a double-stranded oligonucleotide of the invention is double-stranded over at least about 80% of the length of the oligonucleotide. In another embodiment, a double-stranded oligonucleotide of the invention is double-stranded over at least about 90%-95% of the length of the oligonucleotide. In some embodiments, a double-stranded oligonucleotide of the invention is double-stranded over at least about 96%-98% of the length of the oligonucleotide. In some embodiments, the double-stranded oligonucleotide of the invention contains at least or up to 1, 2, 3, 4, 5, 6, 7, 8, 9, 10, 11, 12, 13, 14, or 15 mismatches.

In some embodiments, the oligonucleotide can be substantially protected from nucleases *e.g.,* by modifying the 3' or 5' linkages (*e.g.,* U.S. Pat. No. 5,849,902 and WO 98/13526). For example, oligonucleotides can be made resistant by the inclusion of a "blocking group." The term "blocking group" as used herein refers to substituents (*e.g.*, other than OH groups) that can be attached to oligonucleotides or nucleomonomers, either as protecting groups or coupling groups for synthesis (*e.g.,* FITC, propyl ($CH_2$-$CH_2$-$CH_3$), glycol (-O-$CH_2$-$CH_2$-O-) phosphate ($PO_3^{2+}$), hydrogen phosphonate, or phosphoramidite). "Blocking groups" can also include "end blocking groups" or "exonuclease blocking groups" which protect the 5' and 3' termini of the oligonucleotide, including modified nucleotides and non-nucleotide exonuclease resistant structures.

In some embodiments, at least a portion of the contiguous polynucleotides within the sdRNA are linked by a substitute linkage, *e.g.,* a phosphorothioate linkage.

In some embodiments, chemical modification can lead to at least a 1.5, 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 225, 250, 275, 300, 325, 350, 375, 400, 425, 450, 475, 500 enhancements in cellular uptake. In some embodiments, at least one of the C or U residues includes a hydrophobic modification. In some embodiments, a plurality of Cs and Us contain a hydrophobic modification. In some embodiments, at least 10%, 15%, 20%, 30%, 40%, 50%, 55%, 60% 65%, 70%, 75%, 80%, 85%, 90% or at least 95% of the Cs and Us can contain a hydrophobic modification. In some embodiments, all of the Cs and Us contain a hydrophobic modification.

In some embodiments, the sdRNA or sd-rxRNAs exhibit enhanced endosomal release of sd-rxRNA molecules through the incorporation of protonatable amines. In some embodiments, protonatable amines are incorporated in the sense strand (in the part of the molecule which is discarded after RISC loading). In some embodiments, the sdRNA compounds of the invention comprise an asymmetric compound comprising a duplex region (required for efficient RISC entry of 10-15 bases long) and single stranded region of 4-12 nucleotides long; with a 13 nucleotide duplex. In some embodiments, a 6 nucleotide single stranded region is employed. In some embodiments, the single stranded region of the sdRNA comprises 2-12 phosphorothioate intemucleotide linkages (referred to as phosphorothioate modifications). In some embodiments, 6-8 phosphorothioate intemucleotide linkages are employed. In some embodiments, the sdRNA compounds of the invention also include a unique chemical modification pattern, which provides stability and is compatible with RISC entry.

The guide strand, for example, may also be modified by any chemical modification which confirms stability without interfering with RISC entry. In some embodiments, the chemical modification pattern in the guide strand includes the majority of C and U nucleotides being 2' F modified and the 5' end being phosphorylated.

In some embodiments, at least 30% of the nucleotides in the sdRNA or sd-rxRNA are modified. In some embodiments, at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% of the nucleotides in the sdRNA or sd-rxRNA are modified. In some embodiments, 100% of the nucleotides in the sdRNA or sd-rxRNA are modified.

**In** some embodiments, the sdRNA molecules have minimal double stranded regions. In some embodiments the region of the molecule that is double stranded ranges from 8-15 nucleotides long. In some embodiments, the region of the molecule that is double stranded is 8, 9, 10, 11, 12, 13, 14 or 15 nucleotides long. In some embodiments the double stranded region is 13 nucleotides long. There can be 100% complementarity between the guide and passenger strands, or there may be one or more mismatches between the guide and passenger strands. In some embodiments, on one end of the double stranded molecule, the molecule is either blunt-ended or has a one-nucleotide overhang. The single stranded region of the molecule is in some embodiments between 4-12 nucleotides long. In some embodiments, the single stranded region can be 4, 5, 6, 7, 8, 9, 10, 11 or 12 nucleotides long. In some embodiments, the single stranded region can also be less than 4 or greater than 12 nucleotides long. In certain embodiments, the single stranded region is 6 or 7 nucleotides long.

In some embodiments, the sdRNA molecules have increased stability. In some instances, a chemically modified sdRNA or sd-rxRNA molecule has a half-life in media that is longer than 1, 2, 3, 4, 5, 6, 7, 8, 9, 10, 11, 12, 13, 14, 15, 16, 17, 18, 19, 20, 21, 22, 23, 24 or more than 24 hours, including any intermediate values. In some embodiments, the sd-rxRNA has a half-life in media that is longer than 12 hours.

In some embodiments, the sdRNA is optimized for increased potency and/or reduced toxicity. In some embodiments, nucleotide length of the guide and/or passenger strand, and/or the number of phosphorothioate modifications in the guide and/or passenger strand, can in some aspects influence potency of the RNA molecule, while replacing 2'-fluoro (2'F) modifications with 2'-0-methyl (2'OMe) modifications can in some aspects influence toxicity of the molecule. In some embodiments, reduction in 2'F content of a molecule is predicted to reduce toxicity of the molecule. In some embodiments, the number of phosphorothioate modifications in an RNA molecule can influence the uptake of the molecule into a cell, for example the efficiency of passive uptake of the molecule into a cell. In some embodiments, the sdRNA has no 2'F modification and yet are characterized by equal efficacy in cellular uptake and tissue penetration.

In some embodiments, a guide strand is approximately 18-19 nucleotides in length and has approximately 2-14 phosphate modifications. For example, a guide strand can contain 2, 3, 4, 5, 6, 7, 8, 9, 10, 11, 12, 13, 14 or more than 14 nucleotides that are phosphate-modified. The guide strand may contain one or more modifications that confer increased stability without interfering with RISC entry. The phosphate modified nucleotides, such as phosphorothioate modified nucleotides, can be at the 3' end, 5' end or spread throughout the guide strand. In some embodiments, the 3' terminal 10 nucleotides of the guide strand contain 1, 2, 3, 4, 5, 6, 7, 8, 9 or 10 phosphorothioate modified nucleotides. The guide strand can also contain 2'F and/or 2'OMe modifications, which can be located throughout the molecule. In some embodiments, the nucleotide in position one of the guide strand (the nucleotide in the most 5' position of the guide strand) is 2'OMe modified and/or phosphorylated. C and U nucleotides within the guide strand can be 2'F modified. For example, C and U nucleotides in positions 2-10 of a 19 nt guide strand (or corresponding positions in a guide strand of a different length) can be 2'F modified. C and U nucleotides within the guide strand can also be 2'OMe modified. For example, C and U nucleotides in positions 11-18 of a 19 nt guide strand (or corresponding positions in a guide strand of a different length) can be 2'OMe modified. In some embodiments, the nucleotide at the most 3' end of the guide strand is unmodified. In certain embodiments, the majority of Cs and Us within the guide strand are 2'F modified and the 5' end of the guide strand is phosphorylated. In other embodiments, position 1 and the Cs or Us in positions 11-18 are 2'OMe modified and the 5' end of the guide strand is phosphorylated. In other embodiments, position 1 and the Cs or Us in positions 11-18 are 2'OMe modified, the 5' end of the guide strand is phosphorylated, and the Cs or Us in position 2-10 are 2'F modified.

The self-deliverable RNAi technology provides a method of directly transfecting cells with the RNAi agent, without the need for additional formulations or techniques. The ability to transfect hard-to-transfect cell lines, high *in vivo* activity, and simplicity of use, are characteristics of the compositions and methods that present significant functional advantages over traditional siRNA-based techniques, and as such, the sdRNA methods are employed in several embodiments related to the methods of reduction in expression of the target gene in the TILs of the present invention. The sdRNAi methods allows direct delivery of chemically synthesized compounds to a wide range of primary cells and tissues, both *ex-vivo* and *in vivo.* The sdRNAs described in some embodiments of the invention herein are commercially available from Advirna LLC, Worcester, MA, USA.

The sdRNA are formed as hydrophobically-modified siRNA-antisense oligonucleotide hybrid structures, and are disclosed, for example in Byrne et al., December 2013, J. Ocular Pharmacology and Therapeutics, 29(10): 855-864.

In some embodiments, the sdRNA oligonucleotides can be delivered to the TILs described herein using sterile electroporation. In certain embodiments, the method comprises sterile electroporation of a population of TILs to deliver sdRNA oligonucleotides.

In some embodiments, the oligonucleotides can be delivered to the cells in combination with a transmembrane delivery system. In some embodimets, this transmembrane delivery system comprises lipids, viral vectors, and the like. In some embodiments, the oligonucleotide agent is a self-delivery RNAi agent, that does not require any delivery agents. In certain embodiments, the method comprises use of a transmembrane delivery system to deliver sdRNA oligonucleotides to a population of TILs.

Oligonucleotides and oligonucleotide compositions are contacted with (*e.g.,* brought into contact with, also referred to herein as administered or delivered to) and taken up by TILs described herein, including through passive uptake by TILs. The sdRNA can be added to the TILs as described herein during the first expansion, for example Figure 7 and/or Figure 85, Step B, after the first expansion, for example, during Step C, before or during the second expansion, for example before or during Step D, after Step D and before harvest in Step E, during or after harvest in Step F, before or during final formulation and/or transfer to infusion Bag in Step F, as well as before any optional cryopreservation step in Step F. Mroeover, sdRNA can be added after thawing from any cryopreservation step in Step F. In an embodiment, one or more sdRNAs targeting genes as described herein, including PD-1, LAG-3, TIM-3, CISH, and CBLB, may be added to cell culture media comprising TILs and other agents at concentrations selected from the group consisting of 100 nM to 20 mM, 200 nM to 10 mM, 500 nm to 1 mM, 1 μM to 100 μM, and 1 μM to 100 μM. In an embodiment, one or more sdRNAs targeting genes as described herein, including PD-1, LAG-3, TIM-3, CISH, and CBLB, may be added to cell culture media comprising TILs and other agents at amounts selected from the group consisting of 0.1 μM sdRNA/10,000 TILs/100 μL media, 0.5 μM sdRNA/10,000 TILs /100 μL media, 0.75 μM sdRNA/10,000 TILs /100 μL media, 1 μM sdRNA/10,000 TILs /100 μL media, 1.25 μM sdRNA/10,000 TILs /100 μL media, 1.5 μM sdRNA/10,000 TILs /100 μL media, 2 μM sdRNA/10,000 TILs /100 μL media, 5 μM sdRNA/10,000 TILs /100 μL media, or 10 μM sdRNA/10,000 TILs /100 μL media. In an embodiment, one or more sdRNAs targeting genes as described herein, including PD-1, LAG-3, TIM-3, CISH, and CBLB, may be added to TIL cultures during the pre-REP or REP stages twice a day, once a day, every two days, every three days, every four days, every five days, every six days, or every seven days.

Oligonucleotide compositions of the invention, including sdRNA, can be contacted with TILs as described herein during the expansion process, for example by dissolving sdRNA at high concentrations in cell culture media and allowing sufficient time for passive uptake to occur. In certain embodiments, the method of the present invention comprises contacting a population of TILs with an oligonucleotide composition as described herein. In certain embodiments, the method comprises dissolving an oligonucleotide *e.g.* sdRNA in a cell culture media and contacting the cell culture media with a population of TILs. The TILs may be a first population, a second population and/or a third population as described herein.

In some embodiments, delivery of oligonucleotides into cells can be enhanced by suitable art recognized methods including calcium phosphate, DMSO, glycerol or dextran, electroporation, or by transfection, *e.g.*, using cationic, anionic, or neutral lipid compositions or liposomes using methods known in the art (*see, e.g.,* WO 90/14074; WO 91/16024; WO 91/17424; U.S. Pat. No. 4,897,355; Bergan et a 1993. Nucleic Acids Research. 21:3567).

In some embodiments, more than one sdRNA is used to reduce expression of a target gene. In some embodiments, one or more of PD-1, TIM-3, CBLB, LAG3 and/or CISH targeting sdRNAs are used together. In some embodiments, a PD-1 sdRNA is used with one or more of TIM-3, CBLB, LAG3 and/or CISH in order to reduce expression of more than one gene target. In some embodiments, a LAG3 sdRNA is used in combination with a CISH targeting sdRNA to reduce gene expression of both targets. In some embodiments, the sdRNAs targeting one or more of PD-1, TIM-3, CBLB, LAG3 and/or CISH herein are commercially available from Advirna LLC, Worcester, MA, USA.

In some embodiments, the sdRNA targets a gene selected from the group consisting of PD-1, LAG3, TIM3, CTLA-4, TIGIT, CISH, TGFβR2, PKA, CBLB, BAFF (BR3), and combinations thereof. In some embodiments, the sdRNA targets a gene selected from the group consisting of PD-1, LAG3, TIM3, CTLA-4, TIGIT, CISH, TGFβR2, PKA, CBLB, BAFF (BR3), and combinations thereof. In some embodiments, one sdRNA targets PD-1 and another sdRNA targets a gene selected from the group consisting of LAG3, TIM3, CTLA-4, TIGIT, CISH, TGFβR2, PKA, CBLB, BAFF (BR3), and combinations thereof. In some embodiments, the sdRNA targets a gene selected from PD-1, LAG-3, CISH, CBLB, TIM3, and combinations thereof. In some embodiments, the sdRNA targets a gene selected from PD-1 and one of LAG3, CISH, CBLB, TIM3, and combinations thereof. In some embodiments, one sdRNA targets PD-1 and one sdRNA targets LAG3. In some embodiments, one sdRNA targets PD-1 and one sdRNA targets CISH. In some embodiments, one sdRNA targets PD-1 and one sdRNA targets CBLB. In some embodiments, one sdRNA targets LAG3 and one sdRNA targets CISH. In some embodiments, one sdRNA targets LAG3 and one sdRNA targets CBLB. In some embodiments, one sdRNA targets CISH and one sdRNA targets CBLB. In some embodiments, one sdRNA targets TIM3 and one sdRNA targets PD-1. In some embodiments, one sdRNA targets TIM3 and one sdRNA targets LAG3. In some embodiments, one sdRNA targets TIM3 and one sdRNA targets CISH. In some embodiments, one sdRNA targets TIM3 and one sdRNA targets CBLB.

As discussed above, the present disclosure provides tumor infiltrating lymphocytes (TILs) that have been genetically modified via gene-editing to enhance their therapeutic effect. Embodiments of the present invention embrace genetic editing through nucleotide insertion (RNA or DNA) into a population of TILs for both promotion of the expression of one or more proteins and inhibition of the expression of one or more proteins, as well as combinations thereof. Embodiments of the present invention also provide methods for expanding TILs into a therapeutic population, wherein the methods comprise gene-editing the TILs. There are several gene-editing technologies that may be used to genetically modify a population of TILs, which are suitable for use in accordance with the present invention.

**In** some embodiments, the method comprises a method of genetically modifying a population of TILs which include the step of stable incorporation of genes for production of one or more proteins. In an embodiment, a method of genetically modifying a population of TILs includes the step of retroviral transduction. In an embodiment, a method of genetically modifying a population of TILs includes the step of lentiviral transduction. Lentiviral transduction systems are known in the art and are described, *e.g.,* in Levine, et al., Proc. Nat'l Acad. Sci. 2006, 103, 17372-77; Zufferey, et al., Nat. Biotechnol. 1997, 15, 871-75; Dull, et al., J. Virology 1998, 72, 8463-71, and U.S. Patent No. 6,627,442. In an embodiment, a method of genetically modifying a population of TILs includes the step of gamma-retroviral transduction. Gamma-retroviral transduction systems are known in the art and are described, *e.g.,* Cepko and Pear, Cur. Prot. Mol. Biol. 1996, 9.9.1-9.9.16. In an embodiment, a method of genetically modifying a population of TILs includes the step of transposon-mediated gene transfer. Transposon-mediated gene transfer systems are known in the art and include systems wherein the transposase is provided as DNA expression vector or as an expressible RNA or a protein such that long-term expression of the transposase does not occur in the transgenic cells, for example, a transposase provided as an mRNA *(e.g.,* an mRNA comprising a cap and poly-A tail). Suitable transposon-mediated gene transfer systems, including the salmonid-type Tel-like transposase (SB or Sleeping Beauty transposase), such as SB10, SB11, and SB100x, and engineered enzymes with increased enzymatic activity, are described in, *e.g.,* Hackett, et al., Mol. Therapy 2010, 18, 674-83 and U.S. Patent No. 6,489,458.

In an embodiment, the method comprises a method of genetically modifying a population of TILs *e.g.* a first population, a second population and/or a third population as described herein. In an embodiment, a method of genetically modifying a population of TILs includes the step of stable incorporation of genes for production or inhibition (*e.g.,* silencing) of one ore more proteins. In an embodiment, a method of genetically modifying a population of TILs includes the step of electroporation. Electroporation methods are known in the art and are described, *e.g.,* in Tseng, Biophys. J. 1991, 60, 297-306, and U.S. Patent Application Publication No. 2014/0227237 A1. Other electroporation methods known in the art, such as those described in U.S. Patent Nos. 5,019,034; 5,128,257; 5,137,817; 5,173,158; 5,232,856; 5,273,525; 5,304,120; 5,318,514; 6,010,613 and 6,078,490. In an embodiment, the electroporation method is a sterile electroporation method. In an embodiment, the electroporation method is a pulsed electroporation method. In an embodiment, the electroporation method is a pulsed electroporation method comprising the steps of treating TILs with pulsed electrical fields to alter, manipulate, or cause defined and controlled, permanent or temporary changes in the TILs, comprising the step of applying a sequence of at least three single, operator-controlled, independently programmed, DC electrical pulses, having field strengths equal to or greater than 100 V/cm, to the TILs, wherein the sequence of at least three DC electrical pulses has one, two, or three of the following characteristics: (1) at least two of the at least three pulses differ from each other in pulse amplitude; (2) at least two of the at least three pulses differ from each other in pulse width; and (3) a first pulse interval for a first set of two of the at least three pulses is different from a second pulse interval for a second set of two of the at least three pulses. In an embodiment, the electroporation method is a pulsed electroporation method comprising the steps of treating TILs with pulsed electrical fields to alter, manipulate, or cause defined and controlled, permanent or temporary changes in the TILs, comprising the step of applying a sequence of at least three single, operator-controlled, independently programmed, DC electrical pulses, having field strengths equal to or greater than 100 V/cm, to the TILs, wherein at least two of the at least three pulses differ from each other in pulse amplitude. In an embodiment, the electroporation method is a pulsed electroporation method comprising the steps of treating TILs with pulsed electrical fields to alter, manipulate, or cause defined and controlled, permanent or temporary changes in the TILs, comprising the step of applying a sequence of at least three single, operator-controlled, independently programmed, DC electrical pulses, having field strengths equal to or greater than 100 V/cm, to the TILs, wherein at least two of the at least three pulses differ from each other in pulse width. In an embodiment, the electroporation method is a pulsed electroporation method comprising the steps of treating TILs with pulsed electrical fields to alter, manipulate, or cause defined and controlled, permanent or temporary changes in the TILs, comprising the step of applying a sequence of at least three single, operator-controlled, independently programmed, DC electrical pulses, having field strengths equal to or greater than 100 V/cm, to the TILs, wherein a first pulse interval for a first set of two of the at least three pulses is different from a second pulse interval for a second set of two of the at least three pulses. In an embodiment, the electroporation method is a pulsed electroporation method comprising the steps of treating TILs with pulsed electrical fields to induce pore formation in the TILs, comprising the step of applying a sequence of at least three DC electrical pulses, having field strengths equal to or greater than 100 V/cm, to TILs, wherein the sequence of at least three DC electrical pulses has one, two, or three of the following characteristics: (1) at least two of the at least three pulses differ from each other in pulse amplitude; (2) at least two of the at least three pulses differ from each other in pulse width; and (3) a first pulse interval for a first set of two of the at least three pulses is different from a second pulse interval for a second set of two of the at least three pulses, such that induced pores are sustained for a relatively long period of time, and such that viability of the TILs is maintained. In an embodiment, a method of genetically modifying a population of TILs includes the step of calcium phosphate transfection. Calcium phosphate transfection methods (calcium phosphate DNA precipitation, cell surface coating, and endocytosis) are known in the art and are described in Graham and van der Eb, Virology 1973, 52, 456-467; Wigler, et al., Proc. Natl. Acad. Sci. 1979, 76, 1373-1376; and Chen and Okayarea, Mol. Cell. Biol. 1987, 7, 2745-2752; and in U.S. Patent No. 5,593,875. In an embodiment, a method of genetically modifying a population of TILs includes the step of liposomal transfection. Liposomal transfection methods, such as methods that employ a 1:1 (w/w) liposome formulation of the cationic lipid *N*-[1-(2,3-dioleyloxy)propyl]-*n*,*n*,*n*-trimethylammonium chloride (DOTMA) and dioleoyl phophotidylethanolamine (DOPE) in filtered water, are known in the art and are described in Rose, et al., Biotechniques 1991, 10, 520-525 and Felgner, et al., Proc. Natl. Acad. Sci. USA, 1987, 84, 7413-7417 and in U.S. Patent Nos. 5,279,833; 5,908,635; 6,056,938; 6,110,490; 6,534,484; and 7,687,070. In an embodiment, a method of genetically modifying a population of TILs includes the step of transfection using methods described in U.S. Patent Nos. 5,766,902; 6,025,337; 6,410,517; 6,475,994; and 7,189,705. The TILs may be a first population, a second population and/or a third population of TILs as described herein.

According to an embodiment, the gene-editing process may comprise the use of a programmable nuclease that mediates the generation of a double-strand or single-strand break at one or more immune checkpoint genes. Such programmable nucleases enable precise genome editing by introducing breaks at specific genomic loci, *i.e.,* they rely on the recognition of a specific DNA sequence within the genome to target a nuclease domain to this location and mediate the generation of a double-strand break at the target sequence. A double-strand break in the DNA subsequently recruits endogenous repair machinery to the break site to mediate genome editing by either non-homologous end-joining (NHEJ) or homology-directed repair (HDR). Thus, the repair of the break can result in the introduction of insertion/deletion mutations that disrupt (*e.g.,* silence, repress, or enhance) the target gene product.

Major classes of nucleases that have been developed to enable site-specific genomic editing include zinc finger nucleases (ZFNs), transcription activator-like nucleases (TALENs), and CRISPR-associated nucleases (*e.g.*, CRISPR/Cas9). These nuclease systems can be broadly classified into two categories based on their mode of DNA recognition: ZFNs and TALENs achieve specific DNA binding via protein-DNA interactions, whereas CRISPR systems, such as Cas9, are targeted to specific DNA sequences by a short RNA guide molecule that base-pairs directly with the target DNA and by protein-DNA interactions. *See, e.g.,* Cox et al., Nature Medicine, 2015, Vol. 21, No. 2.

Non-limiting examples of gene-editing methods that may be used in accordance with TIL expansion methods of the present invention include CRISPR methods, TALE methods, and ZFN methods, which are described in more detail below. According to an embodiment, a method for expanding TILs into a therapeutic population may be carried out in accordance with any embodiment of the methods described herein (*e.g.,* GEN 3 process) or as described in PCT/US2017/058610, PCT/US2018/012605, or PCT/US2018/012633, wherein the method further comprises gene-editing at least a portion of the TILs by one or more of a CRISPR method, a TALE method or a ZFN method, in order to generate TILs that can provide an enhanced therapeutic effect. According to an embodiment, gene-edited TILs can be evaluated for an improved therapeutic effect by comparing them to non-modified TILs *in vitro, e.g.,* by evaluating *in vitro* effector function, cytokine profiles, etc. compared to unmodified TILs. In certain embodiments, the method comprises gene editing a population of TILs using CRISPR, TALE and/ or ZFN methods.

In some embodiments of the present invention, electroporation is used for delivery of a gene editing system, such as CRISPR, TALEN, and ZFN systems. In some embodiments of the present invention, the electroporation system is a flow electroporation system. An example of a suitable flow electroporation system suitable for use with some embodiments of the present invention is the commercially-available MaxCyte STX system. There are several alternative commercially-available electroporation instruments which may be suitable for use with the present invention, such as the AgilePulse system or ECM 830 available from BTX-Harvard Apparatus, Cellaxess Elektra (Cellectricon), Nucleofector (Lonza/Amaxa), GenePulser MXcell (BIORAD), iPorator-96 (Primax) or siPORTer96 (Ambion). In some embodiments of the present invention, the electroporation system forms a closed, sterile system with the remainder of the TIL expansion method. In some embodiments of the present invention, the electroporation system is a pulsed electroporation system as described herein, and forms a closed, sterile system with the remainder of the TIL expansion method.

A method for expanding TILs into a therapeutic population may be carried out in accordance with any embodiment of the methods described herein (*e.g.,* process GEN 3) or as described in PCT/US2017/058610, PCT/US2018/012605, or PCT/US2018/012633, wherein the method further comprises gene-editing at least a portion of the TILs by a CRISPR method (*e.g.*, CRISPR/Cas9 or CRISPR/Cpf1). According to particular embodiments, the use of a CRISPR method during the TIL expansion process causes expression of one or more immune checkpoint genes to be silenced or reduced in at least a portion of the therapeutic population of TILs. Alternatively, the use of a CRISPR method during the TIL expansion process causes expression of one or more immune checkpoint genes to be enhanced in at least a portion of the therapeutic population of TILs.

CRISPR stands for "Clustered Regularly Interspaced Short Palindromic Repeats." A method of using a CRISPR system for gene editing is also referred to herein as a CRISPR method. There are three types of CRISPR systems which incorporate RNAs and Cas proteins, and which may be used in accordance with the present invention: Types I, II, and III. The Type II CRISPR (exemplified by Cas9) is one of the most well-characterized systems.

CRISPR technology was adapted from the natural defense mechanisms of bacteria and archaea (the domain of single-celled microorganisms). These organisms use CRISPR-derived RNA and various Cas proteins, including Cas9, to foil attacks by viruses and other foreign bodies by chopping up and destroying the DNA of a foreign invader. A CRISPR is a specialized region of DNA with two distinct characteristics: the presence of nucleotide repeats and spacers. Repeated sequences of nucleotides are distributed throughout a CRISPR region with short segments of foreign DNA (spacers) interspersed among the repeated sequences. In the type II CRISPR/Cas system, spacers are integrated within the CRISPR genomic loci and transcribed and processed into short CRISPR RNA (crRNA). These crRNAs anneal to trans-activating crRNAs (tracrRNAs) and direct sequence-specific cleavage and silencing of pathogenic DNA by Cas proteins. Target recognition by the Cas9 protein requires a "seed" sequence within the crRNA and a conserved dinucleotide-containing protospacer adjacent motif (PAM) sequence upstream of the crRNA-binding region. The CRISPR/Cas system can thereby be retargeted to cleave virtually any DNA sequence by redesigning the crRNA. The crRNA and tracrRNA in the native system can be simplified into a single guide RNA (sgRNA) of approximately 100 nucleotides for use in genetic engineering. The CRISPR/Cas system is directly portable to human cells by co-delivery of plasmids expressing the Cas9 endo-nuclease and the necessary crRNA components. Different variants of Cas proteins may be used to reduce targeting limitations (*e.g.,* orthologs of Cas9, such as Cpf1).

Non-limiting examples of genes that may be silenced or inhibited by permanently gene-editing TILs via a CRISPR method include PD-1, CTLA-4, LAG-3, HAVCR2 (TIM-3), Cish, TGFβ, PKA, CBL-B, PPP2CA, PPP2CB, PTPN6, PTPN22, PDCD1, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, and GUCY1B3.

Non-limiting examples of genes that may be enhanced by permanently gene-editing TILs via a CRISPR method include CCR2, CCR4, CCR5, CXCR2, CXCR3, CX3CR1, IL-2, IL12, IL-15, and IL-21.

Examples of systems, methods, and compositions for altering the expression of a target gene sequence by a CRISPR method, and which may be used in accordance with embodiments of the present invention, are described in U.S. Patent Nos. 8,697,359; 8,993,233; 8,795,965; 8,771,945; 8,889,356; 8,865,406; 8,999,641; 8,945,839; 8,932,814; 8,871,445; 8,906,616; and 8,895,308. Resources for carrying out CRISPR methods, such as plasmids for expressing CRISPR/Cas9 and CRISPR/Cpf1, are commercially available from companies such as GenScript.

In an embodiment, genetic modifications of populations of TILs, as described herein, may be performed using the CRISPR/Cpf1 system as described in U.S. Patent No. US 9790490.

A method for expanding TILs into a therapeutic population may be carried out in accordance with any embodiment of the methods described herein (*e.g.,* process 2A) or as described in PCT/US2017/058610, PCT/US2018/012605, or PCT/US2018/012633, wherein the method further comprises gene-editing at least a portion of the TILs by a TALE method. According to particular embodiments, the use of a TALE method during the TIL expansion process causes expression of one or more immune checkpoint genes to be silenced or reduced in at least a portion of the therapeutic population of TILs. Alternatively, the use of a TALE method during the TIL expansion process causes expression of one or more immune checkpoint genes to be enhanced in at least a portion of the therapeutic population of TILs.

TALE stands for "Transcription Activator-Like Effector" proteins, which include TALENs ("Transcription Activator-Like Effector Nucleases"). A method of using a TALE system for gene editing may also be referred to herein as a TALE method. TALEs are naturally occurring proteins from the plant pathogenic bacteria genus *Xanthomonas,* and contain DNA-binding domains composed of a series of 33-35-amino-acid repeat domains that each recognizes a single base pair. TALE specificity is determined by two hypervariable amino acids that are known as the repeat-variable di-residues (RVDs). Modular TALE repeats are linked together to recognize contiguous DNA sequences. A specific RVD in the DNA-binding domain recognizes a base in the target locus, providing a structural feature to assemble predictable DNA-binding domains. The DNA binding domains of a TALE are fused to the catalytic domain of a type IIS FokI endonuclease to make a targetable TALE nuclease. To induce site-specific mutation, two individual TALEN arms, separated by a 14-20 base pair spacer region, bring FokI monomers in close proximity to dimerize and produce a targeted double-strand break.

Several large, systematic studies utilizing various assembly methods have indicated that TALE repeats can be combined to recognize virtually any user-defined sequence. Custom-designed TALE arrays are also commercially available through Cellectis Bioresearch (Paris, France), Transposagen Biopharmaceuticals (Lexington, KY, USA), and Life Technologies (Grand Island, NY, USA). TALE and TALEN methods suitable for use in the present invention are described in U.S. Patent Application Publication Nos. US 2011/0201118 A1; US 2013/0117869 A1; US 2013/0315884 A1; US 2015/0203871 A1 and US 2016/0120906 A1.

Non-limiting examples of genes that may be silenced or inhibited by permanently gene-editing TILs via a TALE method include PD-1, CTLA-4, LAG-3, HAVCR2 (TIM-3), Cish, TGFβ, PKA, CBL-B, PPP2CA, PPP2CB, PTPN6, PTPN22, PDCD1, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, and GUCY1B3.

Non-limiting examples of genes that may be enhanced by permanently gene-editing TILs via a TALE method include CCR2, CCR4, CCR5, CXCR2, CXCR3, CX3CR1, IL-2, IL12, IL-15, and IL-21.

Examples of systems, methods, and compositions for altering the expression of a target gene sequence by a TALE method, and which may be used in accordance with embodiments of the present invention, are described in U.S. Patent No. 8,586,526.

A method for expanding TILs into a therapeutic population may be carried out in accordance with any embodiment of the methods described herein (*e.g.,* process GEN 3) or as described in PCT/US2017/058610, PCT/US2018/012605, or PCT/US2018/012633, wherein the method further comprises gene-editing at least a portion of the TILs by a zinc finger or zinc finger nuclease method. According to particular embodiments, the use of a zinc finger method during the TIL expansion process causes expression of one or more immune checkpoint genes to be silenced or reduced in at least a portion of the therapeutic population of TILs. Alternatively, the use of a zinc finger method during the TIL expansion process causes expression of one or more immune checkpoint genes to be enhanced in at least a portion of the therapeutic population of TILs.

An individual zinc finger contains approximately 30 amino acids in a conserved ββα configuration. Several amino acids on the surface of the α-helix typically contact 3 bp in the major groove of DNA, with varying levels of selectivity. Zinc fingers have two protein domains. The first domain is the DNA binding domain, which includes eukaryotic transcription factors and contain the zinc finger. The second domain is the nuclease domain, which includes the FokI restriction enzyme and is responsible for the catalytic cleavage of DNA.

The DNA-binding domains of individual ZFNs typically contain between three and six individual zinc finger repeats and can each recognize between 9 and 18 base pairs. If the zinc finger domains are specific for their intended target site then even a pair of 3-finger ZFNs that recognize a total of 18 base pairs can, in theory, target a single locus in a mammalian genome. One method to generate new zinc-finger arrays is to combine smaller zinc-finger "modules" of known specificity. The most common modular assembly process involves combining three separate zinc fingers that can each recognize a 3 base pair DNA sequence to generate a 3-finger array that can recognize a 9 base pair target site. Alternatively, selection-based approaches, such as oligomerized pool engineering (OPEN) can be used to select for new zinc-finger arrays from randomized libraries that take into consideration context-dependent interactions between neighboring fingers. Engineered zinc fingers are available commercially; Sangamo Biosciences (Richmond, CA, USA) has developed a propriety platform (CompoZr®) for zinc-finger construction in partnership with Sigma-Aldrich (St. Louis, MO, USA).

Non-limiting examples of genes that may be silenced or inhibited by permanently gene-editing TILs via a zinc finger method include PD-1, CTLA-4, LAG-3, HAVCR2 (TIM-3), Cish, TGFβ, PKA, CBL-B, PPP2CA, PPP2CB, PTPN6, PTPN22, PDCD1, BTLA, CD160, TIGIT, CD96, CRTAM, LAIR1, SIGLEC7, SIGLEC9, CD244, TNFRSF10B, TNFRSF10A, CASP8, CASP10, CASP3, CASP6, CASP7, FADD, FAS, SMAD2, SMAD3, SMAD4, SMAD10, SKI, SKIL, TGIF1, IL10RA, IL10RB, HMOX2, IL6R, IL6ST, EIF2AK4, CSK, PAG1, SIT1, FOXP3, PRDM1, BATF, GUCY1A2, GUCY1A3, GUCY1B2, and GUCY1B3.

Non-limiting examples of genes that may be enhanced by permanently gene-editing TILs via a zinc finger method include CCR2, CCR4, CCR5, CXCR2, CXCR3, CX3CR1, IL-2, IL12, IL-15, and IL-21.

Examples of systems, methods, and compositions for altering the expression of a target gene sequence by a zinc finger method, which may be used in accordance with embodiments of the present invention, are described in U.S. Patent Nos. 6,534,261, 6,607,882, 6,746,838, 6,794,136, 6,824,978, 6,866,997, 6,933,113, 6,979,539, 7,013,219, 7,030,215, 7,220,719, 7,241,573, 7,241,574, 7,585,849, 7,595,376, 6,903,185, and 6,479,626.

Other examples of systems, methods, and compositions for altering the expression of a target gene sequence by a zinc finger method, which may be used in accordance with embodiments of the present invention, are described in Beane, et al., Mol. Therapy, 2015, 23 1380-1390.

In some embodiments, the TILs are optionally genetically engineered to include additional functionalities, including, but not limited to, a high-affinity T cell receptor (TCR), *e.g.,* a TCR targeted at a tumor-associated antigen such as MAGE-1, HER2, or NY-ESO-1, or a chimeric antigen receptor (CAR) which binds to a tumor-associated cell surface molecule (*e.g.,* mesothelin) or lineage-restricted cell surface molecule (*e.g.,* CD19). In certain embodiments, the method comprises genetically engineering a population of TILs to include a high-affinity T cell receptor (TCR), *e.g.,* a TCR targeted at a tumor-associated antigen such as MAGE-1, HER2, or NY-ESO-1, or a chimeric antigen receptor (CAR) which binds to a tumor-associated cell surface molecule (*e.g.*, mesothelin) or lineage-restricted cell surface molecule (*e.g.,* CD19). Aptly, the population of TILs may be a first population, a second population and/or a third population as described herein.

## XI. Closed Systems for TIL Manufacturing

The present invention provides for the use of closed systems during the TIL culturing process, for example, in conjunction with Gen 2 or Gen 3 processes. Such closed systems allow for preventing and/or reducing microbial contamination, allow for the use of fewer flasks, and allow for cost reductions. In some embodiments, the closed system uses two containers.

Such closed systems are well-known in the art and can be found, for example, at http://www.fda.gov/cber/guidelines.htm and https://www.fda.gov/BiologicsBloodVaccines/GuidanceComplianceRegulatoryInformation/G uidances/Blood/ucm076779.htm.

Sterile connecting devices (STCDs) produce sterile welds between two pieces of compatible tubing. This procedure permits sterile connection of a variety of containers and tube diameters. In some embodiments, the closed systems include luer lock and heat sealed systems as described. In some embodiments, the closed system is accessed via syringes under sterile conditions in order to maintain the sterility and closed nature of the system. In some embodiments, a closed system as described hereinis employed. In some embodiments, the TILs are formulated into a final product formulation container according to the method described herein.

In some embodiments, the closed system uses one container from the time the tumor fragments are obtained until the TILs are ready for administration to the patient or cryopreserving. In some embodiments when two containers are used, the first container is a closed G-container and the population of TILs is centrifuged and transferred to an infusion bag without opening the first closed G-container. In some embodiments, when two containers are used, the infusion bag is a HypoThermosol-containing infusion bag. A closed system or closed TIL cell culture system is characterized in that once the tumor sample and/or tumor fragments have been added, the system is tightly sealed from the outside to form a closed environment free from the invasion of bacteria, fungi, and/or any other microbial contamination.

In some embodiments, the reduction in microbial contamination is between about 5% and about 100%. In some embodiments, the reduction in microbial contamination is between about 5% and about 95%. In some embodiments, the reduction in microbial contamination is between about 5% and about 90%. In some embodiments, the reduction in microbial contamination is between about 10% and about 90%. In some embodiments, the reduction in microbial contamination is between about 15% and about 85%. In some embodiments, the reduction in microbial contamination is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 97%, about 98%, about 99%, or about 100%.

The closed system allows for TIL growth in the absence and/or with a significant reduction in microbial contamination.

Moreover, pH, carbon dioxide partial pressure and oxygen partial pressure of the TIL cell culture environment each vary as the cells are cultured. Consequently, even though a medium appropriate for cell culture is circulated, the closed environment still needs to be constantly maintained as an optimal environment for TIL proliferation. To this end, it is desirable that the physical factors of pH, carbon dioxide partial pressure and oxygen partial pressure within the culture liquid of the closed environment be monitored by means of a sensor, the signal whereof is used to control a gas exchanger installed at the inlet of the culture environment, and the that gas partial pressure of the closed environment be adjusted in real time according to changes in the culture liquid so as to optimize the cell culture environment. In some embodiments, the present invention provides a closed cell culture system which incorporates at the inlet to the closed environment a gas exchanger equipped with a monitoring device which measures the pH, carbon dioxide partial pressure and oxygen partial pressure of the closed environment, and optimizes the cell culture environment by automatically adjusting gas concentrations based on signals from the monitoring device.

In some embodiments, the pressure within the closed environment is continuously or intermittently controlled. That is, the pressure in the closed environment can be varied by means of a pressure maintenance device for example, thus ensuring that the space is suitable for growth of TILs in a positive pressure state, or promoting exudation of fluid in a negative pressure state and thus promoting cell proliferation. By applying negative pressure intermittently, moreover, it is possible to uniformly and efficiently replace the circulating liquid in the closed environment by means of a temporary shrinkage in the volume of the closed environment.

In some embodiments, optimal culture components for proliferation of the TILs can be substituted or added, and including factors such as IL-2 and/or OKT3, as well as combination, can be added.

## XII. Optional Cryopreservation of TILs

Either the bulk TIL population (for example the second population of TILs) or the expanded population of TILs (for example the third population of TILs) prepared by Gen 2 or Gen 3 processes can be optionally cryopreserved. In some embodiments, cryopreservation occurs on the therapeutic TIL population. In some embodiments, cryopreservation occurs on the TILs harvested after the second expansion. In some embodiments, cryopreservation occurs on the TILs in exemplary Step F of Figure 85 (in particular, *e.g.*, Figure 85B). In some embodiments, the TILs are cryopreserved in the infusion bag. In some embodiments, the TILs are cryopreserved prior to placement in an infusion bag. In some embodiments, the TILs are cryopreserved and not placed in an infusion bag. In some embodiments, cryopreservation is performed using a cryopreservation medium. In some embodiments, the cryopreservation media contains dimethylsulfoxide (DMSO). This is generally accomplished by putting the TIL population into a freezing solution, *e.g.* 85% complement inactivated AB serum and 15% dimethyl sulfoxide (DMSO). The cells in solution are placed into cryogenic vials and stored for 24 hours at -80 °C, with optional transfer to gaseous nitrogen freezers for cryopreservation. See, Sadeghi, et al., Acta Oncologica 2013, 52, 978-986.

When appropriate, the cells are removed from the freezer and thawed in a 37 °C water bath until approximately 4/5 of the solution is thawed. The cells are generally resuspended in complete media and optionally washed one or more times. In some embodiments, the thawed TILs can be counted and assessed for viability as is known in the art.

In a preferred embodiment, a population of TILs is cryopreserved using CS10 cryopreservation media (CryoStor 10, BioLife Solutions). In a preferred embodiment, a population of TILs is cryopreserved using a cryopreservation media containing dimethylsulfoxide (DMSO). In a preferred embodiment, a population of TILs is cryopreserved using a 1:1 (vol:vol) ratio of CS10 and cell culture media. In a preferred embodiment, a population of TILs is cryopreserved using about a 1:1 (vol:vol) ratio of CS10 and cell culture media, further comprising additional IL-2.

**As** discussed above, and exemplified in Steps A through E as provided in Figure 85 (in particular, *e.g.*, Figure 85B), cryopreservation can occur at numerous points throughout the TIL expansion process. In some embodiments, the expanded population of TILs after the second expansion (as provided for example, according to Step D of Figure 85 (in particular, *e.g.,* Figure 85B)) can be cryopreserved. Cryopreservation can be generally accomplished by placing the TIL population into a freezing solution, *e.g.,* 85% complement inactivated AB serum and 15% dimethyl sulfoxide (DMSO). The cells in solution are placed into cryogenic vials and stored for 24 hours at -80 °C, with optional transfer to gaseous nitrogen freezers for cryopreservation. See Sadeghi, et al., Acta Oncologica 2013, 52, 978-986. In some embodiments, the TILs are cryopreserved in 5% DMSO. In some embodiments, the TILs are cryopreserved in cell culture media plus 5% DMSO. In some embodiments, the TILs are cryopreserved according to the methods provided herein.

When appropriate, the cells are removed from the freezer and thawed in a 37 °C water bath until approximately 4/5 of the solution is thawed. The cells are generally resuspended in complete media and optionally washed one or more times. In some embodiments, the thawed TILs can be counted and assessed for viability as is known in the art.

In some cases, the Step B TIL population can be cryopreserved immediately, using the protocols discussed below. Alternatively, the bulk TIL population can be subjected to Step C and Step D and then cryopreserved after Step D. Similarly, in the case where genetically modified TILs will be used in therapy, the Step B or Step D TIL populations can be subjected to genetic modifications for suitable treatments.

## XIII. Phenotypic Characteristics of Expanded TILs

Also disclosed herein but not claimed, the TILs are analyzed for expression of numerous phenotype markers after expansion, including those described herein and in the Examples. Expression of one or more phenotypic markers may be examined. The phenotypic characteristics of the TILs may be analyzed after the first expansion in Step B. The phenotypic characteristics of the TILs may be analyzed during the transition in Step C. The phenotypic characteristics of the TILs may be analyzed during the transition according to Step C and after cryopreservation. The phenotypic characteristics of the TILs may be analyzed after the second expansion according to Step D. The phenotypic characteristics of the TILs may be analyzed after two or more expansions according to Step D.

The marker may be selected from the group consisting of CD8 and CD28. Expression of CD8 may be examined. Expression of CD28 may be examined. The expression of CD8 and/or CD28 may be higher on TILs produced according the current invention process, as compared to other processes (*e.g.,* the Gen 3 process as provided for example in Figure 85 (in particular, *e.g.*, Figure 85B), as compared to the 2A process as provided for example in Figure 85 (in particular, *e.g.*, Figure 85B)). The expression of CD8 may be higher on TILs produced according the current invention process, as compared to other processes *(e.g.,* the Gen 3 process as provided for example in Figure 85 (in particular, *e.g.,* Figure 85B), as compared to the 2A process as provided for example in Figure 85 (in particular, *e.g.,* Figure 85B)). The expression of CD28 may be higher on TILs produced according the current invention process, as compared to other processes *(e.g.,* the Gen 3 process as provided for example in Figure 85 (in particular, *e.g.*, Figure 85B), as compared to the 2A process as provided for example in Figure 85 (in particular, *e.g.*, Figure 85A)). Expression of one or more regulatory markers may be measured.

No selection of the first population of TILs, second population of TILs, third population of TILs, or harvested TIL population based on CD8 and/or CD28 expression may be performed during any of the steps for the method for expanding tumor infiltrating lymphocytes (TILs) described herein.

The percentage of central memory cells may be higher on TILs produced according the current invention process, as compared to other processes *(e.g.,* the Gen 3 process as provided for example in Figure 85 (in particular, *e.g.*, Figure 85B), as compared to the 2A process as provided for example in Figure 85 (in particular, *e.g.*, Figure 85A)). The memory marker for central memory cells may be selected from the group consisting of CCR7 and CD62L.

Also disclosed herein but not claimed, restimulated TILs can also be evaluated for cytokine release, using cytokine release assays. TILs can be evaluated for interferon-γ (IFN-γ) secretion. The IFN-γ secretion may be measured by an ELISA assay. The IFN-γ secretion may be measured by an ELISA assay after the rapid second expansion step, after Step D as provided in for example, Figure 85 (in particular, *e.g.*, Figure 85B). TIL health may be measured by IFN-gamma (IFN-γ) secretion. In some embodiments, IFN-γ secretion is indicative of active TILs. A potency assay for IFN-γ production may be employed. IFN-γ production is another measure of cytotoxic potential. IFN-γ production can be measured by determining the levels of the cytokine IFN-γ in the media of TIL stimulated with antibodies to CD3, CD28, and CD137/4-1BB. IFN-γ levels in media from these stimulated TIL can be determined using by measuring IFN-γ release. In some embodiments, an increase in IFN-γ production in for example Step D in the Gen 3 process as provided in Figure 85 (in particular, *e.g.,* Figure 85B) TILs as compared to for example Step D in the 2A process as provided in Figure 85 (in particular, *e.g.*, Figure 85A) is indicative of an increase in cytotoxic potential of the Step D TILs. In some embodiments, IFN-γ secretion is increased one-fold, two-fold, three-fold, four-fold, or five-fold or more. In some embodiments, IFN-γ secretion is increased one-fold. In some embodiments, IFN-γ secretion is increased two-fold. In some embodiments, IFN-γ secretion is increased three-fold. In some embodiments, IFN-γ secretion is increased four-fold. In some embodiments, IFN-γ secretion is increased five-fold. IFN-γ may be measured using a Quantikine ELISA kit. IFN-γ may be measured in TILs *ex vivo.* IFN-γ may be measured in TILs *ex vivo,* including TILs produced by the methods of the present invention, including, for example Figure 85B methods.

The diverse antigen receptors of T and B lymphocytes are produced by somatic recombination of a limited, but large number of gene segments. These gene segments: V (variable), D (diversity), J (joining), and C (constant), determine the binding specificity and downstream applications of immunoglobulins and T-cell receptors (TCRs). The present invention provides a method for generating TILs which exhibit and increase the T-cell repertoire diversity. In some embodiments, the TILs obtained by the present method exhibit an increase in the T-cell repertoire diversity. In some embodiments, the TILs obtained by the present method exhibit an increase in the T-cell repertoire diversity as compared to freshly harvested TILs and/or TILs prepared using other methods than those provide herein including, for example, methods other than those embodied in Figure 85 (in particular, *e.g.*, Figure 85B). In some embodiments, the TILs obtained by the present method exhibit an increase in the T-cell repertoire diversity as compared to freshly harvested TILs and/or TILs prepared using methods referred to as process 2A, as exemplified in Figure 85 (in particular, *e.g.,* Figure 85A). In some embodiments, the TILs obtained in the first expansion exhibit an increase in the T-cell repertoire diversity. In some embodiments, the increase in diversity is an increase in the immunoglobulin diversity and/or the T-cell receptor diversity. In some embodiments, the diversity is in the immunoglobulin is in the immunoglobulin heavy chain. In some embodiments, the diversity is in the immunoglobulin is in the immunoglobulin light chain. In some embodiments, the diversity is in the T-cell receptor. In some embodiments, the diversity is in one of the T-cell receptors selected from the group consisting of alpha, beta, gamma, and delta receptors. In some embodiments, there is an increase in the expression of T-cell receptor (TCR) alpha and/or beta. In some embodiments, there is an increase in the expression of T-cell receptor (TCR) alpha. In some embodiments, there is an increase in the expression of T-cell receptor (TCR) beta. In some embodiments, there is an increase in the expression of TCRab *(i.e.,* TCRα/β). In some embodiments, the process as described herein *(e.g.,* the Gen 3 process) shows higher clonal diversity as compared to other processes, for example the process referred to as the Gen 2 based on the number of unique peptide CDRs within the sample (*see,* for example Figures 12-14).

The phenotypic characterization may be examined after cryopreservation.

## XIV. Additional Process Embodiments

Also disclosed herein but not claimed is a method for expanding tumor infiltrating lymphocytes (TILs) into a therapeutic population of TILs comprising: (a) obtaining a first population of TILs from one or more tumor fragments or cores from a subject; (b) performing a priming first expansion by culturing the first population of TILs in a cell culture medium comprising IL-2 and OKT-3, wherein the priming first expansion is performed for about 1 to 17 days to obtain the second population of TILs, wherein the second population of TILs is greater in number than the first population of TILs; (c) performing a rapid second expansion by contacting the second population of TILs with a cell culture medium comprising IL-2, OKT-3 and exogenous antigen presenting cells (APCs) to produce a third population of TILs, wherein the rapid second expansion is performed for about 1 to 11 days to obtain the third population of TILs, wherein the third population of TILs is a therapeutic population of TILs; and (d) harvesting the therapeutic population of TILs obtained from step (c). In some embodiments, the step of rapid second expansion is split into a plurality of steps to achieve a scaling up of the culture by: (1) performing the rapid second expansion by culturing the second population of TILs in a small scale culture in a first container, *e.g.,* a G-REX 100MCS container, for a period of about 3 to 4 days, and then (2) effecting the transfer of the second population of TILs from the small scale culture to a second container larger than the first container, *e.g.,* a G-REX 500MCS container, wherein in the second container the second population of TILs from the small scale culture is cultured in a larger scale culture for a period of about 4 to 7 days. In some embodiments, the step of rapid expansion is split into a plurality of steps to achieve a scaling out of the culture by: (1) performing the rapid second expansion by culturing the second population of TILs in a first small scale culture in a first container, *e.g.,* a G-REX 100MCS container, for a period of about 3 to 4 days, and then (2) effecting the transfer and apportioning of the second population of TILs from the first small scale culture into and amongst at least 2, 3, 4, 5, 6, 7, 8, 9, 10, 11, 12, 13, 14, 15, 16, 17, 18, 19, or 20 second containers that are equal in size to the first container, wherein in each second container the portion of the second population of TILs from the first small scale culture transferred to such second container is cultured in a second small scale culture for a period of about 4 to 7 days. In some embodiments, the step of rapid expansion is split into a plurality of steps to achieve a scaling out and scaling up of the culture by: (1) performing the rapid second expansion by culturing the second population of TILs in a small scale culture in a first container, *e.g.,* a G-REX 100MCS container, for a period of about 3 to 4 days, and then (2) effecting the transfer and apportioning of the second population of TILs from the first small scale culture into and amongst at least 2, 3, 4, 5, 6, 7, 8, 9, 10, 11, 12, 13, 14, 15, 16, 17, 18, 19, or 20 second containers that are larger in size than the first container, *e.g.*, G-REX 500MCS containers, wherein in each second container the portion of the second population of TILs transferred from the small scale culture to such second container is cultured in a larger scale culture for a period of about 4 to 7 days. In some embodiments, the step of rapid expansion is split into a plurality of steps to achieve a scaling out and scaling up of the culture by: (1) performing the rapid second expansion by culturing the second population of TILs in a small scale culture in a first container, *e.g.,* a G-REX 100MCS container, for a period of about 4 days, and then (2) effecting the transfer and apportioning of the second population of TILs from the first small scale culture into and amongst 2, 3 or 4 second containers that are larger in size than the first container, *e.g.*, G-REX 500MCS containers, wherein in each second container the portion of the second population of TILs transferred from the small scale culture to such second container is cultured in a larger scale culture for a period of about 5 days.

**In** another embodiment, the invention provides the method described in any of the preceding paragraphs as applicable above modified such that in step (b) the primary first expansion is performed by contacting the first population of TILs with a culture medium which further comprises exogneous antigen-presenting cells (APCs), wherein the number of APCs in the culture medium in step (c) is greater than the number of APCs in the culture medium in step (b).

In another embodiment, the invention provides the method described in any of the preceding paragraphs as applicable above modified such that in step (c) the culture medium is supplemented with additional exogenous APCs.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 20: 1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 10: 1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 9: 1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 8:1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 7:1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 6: 1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 5:1.

In another embodiment, the invention provides the method described in any one of the preceding paragraphs as applicable above modified such that the ratio of number of APCs added in the rapid second expansion to the number of APCs added in step (b) is selected from a range of from at or about 1.1: 1 to at or about 4: 1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 3: 1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.9:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.8:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.7:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.6:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.5:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.4:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.3:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.2:1.

In another embodiment, the invention provides the method described in any of the preceding paragraphs as applicable above modified such that the ratio of number of APCs added in the rapid second expansion to the number of APCs added in step (b) is selected from a range of from at or about 1.1:1 to at or about 2.1:1.

In another embodiment, the invention provides the method described in any of the preceding paragraphs as applicable above modified such that the ratio of number of APCs added in the rapid second expansion to the number of APCs added in step (b) is selected from a range of from at or about 1.1: 1 to at or about 2:1.

In another embodiment, the invention provides the method described in any of the preceding paragraphs as applicable above modified such that the ratio of number of APCs added in the rapid second expansion to the number of APCs added in step (b) is selected from a range of from at or about 2: 1 to at or about 10:1.

In another embodiment, the invention provides the method described in any of the preceding paragraphs as applicable above modified such that the ratio of number of APCs added in the rapid second expansion to the number of APCs added in step (b) is selected from a range of from at or about 2: 1 to at or about 5:1.

In another embodiment, the invention provides the method described in any of the preceding paragraphs as applicable above modified such that the ratio of number of APCs added in the rapid second expansion to the number of APCs added in step (b) is selected from a range of from at or about 2: 1 to at or about 4: 1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 3:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.9:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.8:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.7:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.6:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.5:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.4:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.3:1.

In another embodiment, the invention provides the method described in any of the preceding paragraphs as applicable above modified such that the ratio of number of APCs added in the rapid second expansion to the number of APCs added in step (b) is selected from a range of from at or about about 2:1 to at or about 2.2:1.

In another embodiment, the invention provides the method described in any of the preceding paragraphs as applicable above modified such that the ratio of number of APCs added in the rapid second expansion to the number of APCs added in step (b) is selected from a range of from at or about 2:1 to at or about 2.1:1.

In another embodiment, the invention provides the method described in any of the preceding paragraphs as applicable above modified such that the ratio of number of APCs added in the rapid second expansion to the number of APCs added in step (b) is at or about 2:1.

In another embodiment, the invention provides the method described in any of the preceding paragraphs as applicable above modified such that the ratio of number of APCs added in the rapid second expansion to the number of APCs added in step (b) is at or about 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, 4:1, 4.1:1, 4.2:1, 4.3:1, 4.4:1, 4.5:1, 4.6:1, 4.7:1, 4.8:1, 4.9:1, or 5:1.

In another embodiment, the invention provides the method described in any of the preceding paragraphs as applicable above modified such that the number of APCs added in the primary first expansion is at or about $1 \times 10^8$, $1.1 \times 10^8$, $1.2 \times 10^8$, $1.3 \times 10^8$, $1.4 \times 10^8$, $1.5 \times 10^8$, $1.6 \times 10^8$, $1.7 \times 10^8$, $1.8 \times 10^8$, $1.9 \times 10^8$, $2 \times 10^8$, $2.1 \times 10^8$, $2.2 \times 10^8$, $2.3 \times 10^8$, $2.4 \times 10^8$, $2.5 \times 10^8$, $2.6 \times 10^8$, $2.7 \times 10^8$, $2.8 \times 10^8$, $2.9 \times 10^8$, $3 \times 10^8$, $3.1 \times 10^8$, $3.2 \times 10^8$, $3.3 \times 10^8$, $3.4 \times 10^8$ or $3.5 \times 10^8$ APCs, and such that the number of APCs added in the rapid second expansion is at or about $3.5 \times 10^8$, $3.6 \times 10^8$, $3.7 \times 10^8$, $3.8 \times 10^8$, $3.9 \times 10^8$, $4 \times 10^8$, $4.1 \times 10^8$, $4.2 \times 10^8$, $4.3 \times 10^8$, $4.4 \times 10^8$, $4.5 \times 10^8$, $4.6 \times 10^8$, $4.7 \times 10^8$, $4.8 \times 10^8$, $4.9 \times 10^8$, $5 \times 10^8$, $5.1 \times 10^8$, $5.2 \times 10^8$, $5.3 \times 10^8$, $5.4 \times 10^8$, $5.5 \times 10^8$, $5.6 \times 10^8$, $5.7 \times 10^8$, $5.8 \times 10^8$, $5.9 \times 10^8$, $6 \times 10^8$, $6.1 \times 10^8$, $6.2 \times 10^8$, $6.3 \times 10^8$, $6.4 \times 10^8$, $6.5 \times 10^8$, $6.6 \times 10^8$, $6.7 \times 10^8$, $6.8 \times 10^8$, $6.9 \times 10^8$, $7 \times 10^8$, $7.1 \times 10^8$, $7.2 \times 10^8$, $7.3 \times 10^8$, $7.4 \times 10^8$, $7.5 \times 10^8$, $7.6 \times 10^8$, $7.7 \times 10^8$, $7.8 \times 10^8$, $7.9 \times 10^8$, $8 \times 10^8$, $8.1 \times 10^8$, $8.2 \times 10^8$, $8.3 \times 10^8$, $8.4 \times 10^8$, $8.5 \times 10^8$, $8.6 \times 10^8$, $8.7 \times 10^8$, $8.8 \times 10^8$, $8.9 \times 10^8$, $9 \times 10^8$, $9.1 \times 10^8$, $9.2 \times 10^8$, $9.3 \times 10^8$, $9.4 \times 10^8$, $9.5 \times 10^8$, $9.6 \times 10^8$, $9.7 \times 10^8$, $9.8 \times 10^8$, $9.9 \times 10^8$ or $1 \times 10^9$ APCs.

In another embodiment, the invention provides the method described in any of the preceding paragraphs as applicable above modified such that the number of APCs added in the primary first expansion is selected from the range of at or about $1 \times 10^8$ APCs to at or about $3.5 \times 10^8$ APCs, and wherein the number of APCs added in the rapid second expansion is selected from the range of at or about $3.5 \times 10^8$ APCs to at or about $1 \times 10^9$ APCs.

In another embodiment, the invention provides the method described in any of the preceding paragraphs as applicable above modified such that the number of APCs added in the primary first expansion is selected from the range of at or about $1.5 \times 10^8$ APCs to at or about $3 \times 10^8$ APCs, and wherein the number of APCs added in the rapid second expansion is selected from the range of at or about $4 \times 10^8$ APCs to at or about $7.5 \times 10^8$ APCs.

In another embodiment, the invention provides the method described in any of the preceding paragraphs as applicable above modified such that the number of APCs added in the primary first expansion is selected from the range of at or about $2 \times 10^8$ APCs to at or about $2.5 \times 10^8$ APCs, and wherein the number of APCs added in the rapid second expansion is selected from the range of at or about $4.5 \times 10^8$ APCs to at or about $5.5 \times 10^8$ APCs.

In another embodiment, the invention provides the method described in any of the preceding paragraphs as applicable above modified such that at or about $2.5 \times 10^8$ APCs are added to the primary first expansion and at or about $5 \times 10^8$ APCs are added to the rapid second expansion.

In another embodiment, the invention provides the method described in any of the preceding paragraphs as applicable above modified such that the antigen-presenting cells are peripheral blood mononuclear cells (PBMCs).

In another embodiment, the invention provides the method described in any of the preceding paragraphs as applicable above modified such that the multiple tumor fragments are distributed into a plurality of separate containers, in each of which separate containers the first population of TILs is obtained in step (a), the second population of TILs is obtained in step (b), and the third population of TILs is obtained in step (c), and the therapeutic populations of TILs from the plurality of containers in step (c) are combined to yield the harvested TIL population from step (d).

In another embodiment, the invention provides the method described in any of the preceding paragraphs as applicable above modified such that the first population of TILs is obtained from multiple tumor fragments or cores from the subject in step (a).

In another embodiment, the invention provides the method described in any of the preceding paragraphs as applicable above modified such that the multiple tumors are evenly distributed into the plurality of separate containers.

In another embodiment, the invention provides the method described in any of the preceding paragraphs as applicable above modified such that the plurality of separate containers comprises at least two separate containers.

In another embodiment, the invention provides the method described in any of the preceding paragraphs as applicable above modified such that the plurality of separate containers comprises from two to twenty separate containers.

In another embodiment, the invention provides the method described in any of the preceding paragraphs as applicable above modified such that the plurality of separate containers comprises from two to fifteen separate containers.

In another embodiment, the invention provides the method described in any of the preceding paragraphs as applicable above modified such that the plurality of separate containers comprises from two to ten separate containers.

In another embodiment, the invention provides the method described in any of the preceding paragraphs as applicable above modified such that the plurality of separate containers comprises from two to five separate containers.

In another embodiment, the invention provides the method described in any of the preceding paragraphs as applicable above modified such that the plurality of separate containers comprises 2, 3, 4, 5, 6, 7, 8, 9, 10, 11, 12, 13,

14, 15, 16, 17, 18, 19, or 20 separate containers.

In another embodiment, the invention provides the method described in any of the preceding paragraphs as applicable above modified such that for each container in which the priming first expansion is performed on a first population of TILs in step (b) the rapid second expansion in step (c) is performed in the same container on the second population of TILs produced from such first population of TILs.

In another embodiment, the invention provides the method described in any of the preceding paragraphs as applicable above modified such that each of the separate containers comprises a first gas-permeable surface area.

In another embodiment, the invention provides the method described in any of the preceding paragraphs as applicable above modified such that the multiple tumor fragments are distributed in a single container.

In another embodiment, the invention provides the method described in any of the preceding paragraphs as applicable above modified such that the single container comprises a first gas-permeable surface area.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein in step (b) the APCs are layered onto the first gas-permeable surface area at an average thickness of at or about one cell layer to at or about three cell layers.

In another embodiment, the invention provides the method described in any of the preceding paragraphs as applicable above modified such that in step (b) the APCs are layered onto the first gas-permeable surface area at an average thickness of at or about 1.5 cell layers to at or about 2.5 cell layers.

In another embodiment, the invention provides the method described in any of the preceding paragraphs as applicable above modified such that in step (b) the APCs are layered onto the first gas-permeable surface area at an average thickness of at or about 2 cell layers.

In another embodiment, the invention provides the method described in any of the preceding paragraphs as applicable above modified such that in step (b) the APCs are layered onto the first gas-permeable surface area at an average thickness of at or about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 cell layers.

In another embodiment, the invention provides the method described in any of the preceding paragraphs as applicable above modified such that in step (c) the APCs are layered onto the first gas-permeable surface area at an average thickness of at or about 3 cell layers to at or about 10 cell layers.

In another embodiment, the invention provides the method described in any of the preceding paragraphs as applicable above modified such that in step (c) the APCs are layered onto the first gas-permeable surface area at an average thickness of at or about 4 cell layers to at or about 8 cell layers.

In another embodiment, the invention provides the method described in any of the preceding paragraphs as applicable above modified such that in step (c) the APCs are layered onto the first gas-permeable surface area at an average thickness of at or about 3, 4, 5, 6, 7, 8, 9 or 10 cell layers.

In another embodiment, the invention provides the method described in any of the preceding paragraphs as applicable above modified such that in step (c) the APCs are layered onto the first gas-permeable surface area at an average thickness of at or about 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9 or 8 cell layers.

In another embodiment, the invention provides the method described in any of the preceding paragraphs as applicable above modified such that in step (b) the priming first expansion is performed in a first container comprising a first gas-permeable surface area and in step (c) the rapid second expansion is performed in a second container comprising a second gas-permeable surface area.

In another embodiment, the invention provides the method described in any of the preceding paragraphs as applicable above modified such that the second container is larger than the first container.

In another embodiment, the invention provides the method described in any of the preceding paragraphs as applicable above modified such that in step (b) the priming first expansion is performed by supplementing the cell culture medium of the first population of TILs with additional antigen-presenting cells (APCs), wherein the number of APCs added in step (c) is greater than the number of APCs added in step (b), and wherein in step (b) the APCs are layered onto the first gas-permeable surface area at an average thickness of at or about one cell layer to at or about three cell layers.

In another embodiment, the invention provides the method described in any of the preceding paragraphs as applicable above modified such that in step (b) the APCs are layered onto the first gas-permeable surface area at an average thickness of at or about 1.5 cell layers to at or about 2.5 cell layers.

In another embodiment, the invention provides the method described in any of the preceding paragraphs as applicable above modified such that in step (b) the APCs are layered onto the first gas-permeable surface area at an average thickness of at or about 2 cell layers.

In another embodiment, the invention provides the method described in any of the preceding paragraphs as applicable modified such that in step (b) the APCs are layered onto the first gas-permeable surface area at an average thickness of at or about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 cell layers.

In another embodiment, the invention provides the method described in any of the preceding paragraphs as applicable above modified such that in step (c) the APCs are layered onto the second gas-permeable surface area at an average thickness of at or about 3 cell layers to at or about 10 cell layers.

In another embodiment, the invention provides the method described in any of the preceding paragraphs as applicable above modified such that in step (c) the APCs are layered onto the second gas-permeable surface area at an average thickness of at or about 4 cell layers to at or about 8 cell layers.

In another embodiment, the invention provides the method described in any of the preceding paragraphs as applicable above modified such that in step (c) the APCs are layered onto the second gas-permeable surface area at an average thickness of at or about 3, 4, 5, 6, 7, 8, 9 or 10 cell layers.

In another embodiment, the invention provides the method described in any of the preceding paragraphs as applicable modified such that in step (c) the APCs are layered onto the second gas-permeable surface area at an average thickness of at or about 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9 or 8 cell layers.

In another embodiment, the invention provides the method described in any of the preceding paragraphs as applicable above modified such that in step (b) the priming first expansion is performed in a first container comprising a first gas-permeable surface area and in step (c) the rapid second expansion is performed in the first container.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein in step (b) the APCs are layered onto the first gas-permeable surface area at an average thickness of at or about one cell layer to at or about three cell layers.

In another embodiment, the invention provides the method described in any of the preceding paragraphs as applicable above modified such that in step (b) the APCs are layered onto the first gas-permeable surface area at an average thickness of at or about 1.5 cell layers to at or about 2.5 cell layers.

In another embodiment, the invention provides the method described in any of the preceding paragraphs as applicable above modified such that in step (b) the APCs are layered onto the first gas-permeable surface area at an average thickness of at or about 2 cell layers.

In another embodiment, the invention provides the method described any of the preceding paragraphs as applicable above modified such that in step (b) the APCs are layered onto the first gas-permeable surface area at an average thickness of at or about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3 cell layers.

In another embodiment, the invention provides the method described in any of the preceding paragraphs as applicable above modified such that in step (c) the APCs are layered onto the first gas-permeable surface area at an average thickness of at or about 3 cell layers to at or about 10 cell layers.

In another embodiment, the invention provides the method described in any of the preceding paragraphs as applicable above modified such that in step (c) the APCs are layered onto the first gas-permeable surface area at an average thickness of at or about 4 cell layers to at or about 8 cell layers.

In another embodiment, the invention provides the method described in any of the preceding paragraphs as applicable above modified such that in step (c) the APCs are layered onto the first gas-permeable surface area at an average thickness of at or about 3, 4, 5, 6, 7, 8, 9 or 10 cell layers.

In another embodiment, the invention provides the method described in any of the preceding paragraphs as applicable above modified such that in step (c) the APCs are layered onto the first gas-permeable surface area at an average thickness of at or about 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9 or 8 cell layers.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:10.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:9.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:8.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:7.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:6.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:5.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:4.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:3.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.1 to at or about 1:2.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.2 to at or about 1:8.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.3 to at or about 1:7.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.4 to at or about 1:6.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.5 to at or about 1:5.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.6 to at or about 1:4.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.7 to at or about 1:3.5.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.8 to at or about 1:3.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:1.9 to at or about 1:2.5.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from the range of at or about 1:2.

In another embodiment, the invention provides the method described in any of the preceding paragraphs as applicable above modified such that in step (b) the primary first expansion is performed by supplementing the cell culture medium of the first population of TILs with additional antigen-presenting cells (APCs), wherein the number of APCs added in step (c) is greater than the number of APCs added in step (b), and wherein the ratio of the average number of layers of APCs layered in step (b) to the average number of layers of APCs layered in step (c) is selected from at or about 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, 1:3, 1:3.1, 1:3.2, 1:3.3, 1:3.4, 1:3.5, 1:3.6, 1:3.7, 1:3.8, 1:3.9, 1:4, 1:4.1, 1:4.2, 1:4.3, 1:4.4, 1:4.5, 1:4.6, 1:4.7, 1:4.8, 1:4.9, 1:5, 1:5.1, 1:5.2, 1:5.3, 1:5.4, 1:5.5, 1:5.6, 1:5.7, 1:5.8, 1:5.9, 1:6, 1:6.1, 1:6.2, 1:6.3, 1:6.4, 1:6.5, 1:6.6, 1:6.7, 1:6.8, 1:6.9, 1:7, 1:7.1, 1:7.2, 1:7.3, 1:7.4, 1:7.5, 1:7.6, 1:7.7, 1:7.8, 1:7.9, 1:8, 1:8.1, 1:8.2, 1:8.3, 1:8.4, 1:8.5, 1:8.6, 1:8.7, 1:8.8, 1:8.9, 1:9, 1:9.1, 1:9.2, 1:9.3, 1:9.4, 1:9.5, 1:9.6, 1:9.7, 1:9.8, 1:9.9 or 1:10.

In another embodiment, the invention provides the method described in any of the preceding paragraphs as applicable above modified such that the ratio of the number of TILs in the second population of TILs to the number of TILs in the first population of TILs is at or about 1.5:1 to at or about 100:1.

In another embodiment, the invention provides the method described in any of the preceding paragraphs as applicable above modified such that the ratio of the number of TILs in the second population of TILs to the number of TILs in the first population of TILs is at or about 50:1.

In another embodiment, the invention provides the method described in any of the preceding paragraphs as applicable above modified such that the ratio of the number of TILs in the second population of TILs to the number of TILs in the first population of TILs is at or about 25:1.

In another embodiment, the invention provides the method described in any of the preceding paragraphs as applicable above modified such that the ratio of the number of TILs in the second population of TILs to the number of TILs in the first population of TILs is at or about 20:1.

In another embodiment, the invention provides the method described in any of the preceding paragraphs as applicable above modified such that the ratio of the number of TILs in the second population of TILs to the number of TILs in the first population of TILs is at or about 10:1.

In another embodiment, the invention provides the method described in any of the preceding paragraphs as applicable above modified such that the second population of TILs is at least at or about 50-fold greater in number than the first population of TILs.

In another embodiment, the invention provides the method described in any of the preceding paragraphs as applicable above modified such that the second population of TILs is at least at or about 1-, 2-, 3-, 4-, 5-, 6-, 7-, 8-, 9-, 10-, 11-, 12-, 13-, 14-, 15-, 16-, 17-, 18-, 19-, 20-, 21-, 22-, 23-, 24-, 25-, 26-, 27-, 28-, 29-, 30-, 31-, 32-, 33-, 34-, 35-, 36-, 37-, 38-, 39-, 40-, 41-, 42-, 43-, 44-, 45-, 46-, 47-, 48-, 49- or 50-fold greater in number than the first population of TILs.

In another embodiment, the invention provides the method described in any of the preceding paragraphs as applicable above modified such that at or about 2 days or at or about 3 days after the commencement of the second period in step (c), the cell culture medium is supplemented with additional IL-2.

In another embodiment, the invention provides the method described in any of the preceding paragraphs as applicable above modified to further comprise the step of cryopreserving the harvested TIL population in step (d) using a cryopreservation process.

In another embodiment, the invention provides the method described in any of the preceding paragraphs as applicable above modified to comprise performing after step (d) the additional step of (e) transferring the harvested TIL population from step (d) to an infusion bag that optionally contains HypoThermosol.

In another embodiment, the invention provides the method described in any of the preceding paragraphs as applicable above modified to comprise the step of cryopreserving the infusion bag comprising the harvested TIL population in step (e) using a cryopreservation process.

In another embodiment, the invention provides the method described in any of the preceding paragraphs as applicable above modified such that the cryopreservation process is performed using a 1:1 ratio of harvested TIL population to cryopreservation media.

In another embodiment, the invention provides the method described in any of the preceding paragraphs as applicable above modified such that the antigen-presenting cells are peripheral blood mononuclear cells (PBMCs).

In another embodiment, the invention provides the method described in any of the preceding paragraphs as applicable above modified such that the PBMCs are irradiated and allogeneic.

In another embodiment, the invention provides the method described in any of the preceding paragraphs as applicable above modified such that the total number of APCs added to the cell culture in step (b) is $2.5 \times 10^8$.

In another embodiment, the invention provides the method described in any of the preceding paragraphs as applicable above modified such that the total number of APCs added to the cell culture in step (c) is $5 \times 10^8$.

In another embodiment, the invention provides the method described in any of the preceding paragraphs as applicable above modified such that the APCs are PBMCs.

In another embodiment, the invention provides the method described in any of the preceding paragraphs as applicable above modified such that the PBMCs are irradiated and allogeneic.

In another embodiment, the invention provides the method described in any of the preceding paragraphs as applicable above modified such that the antigen-presenting cells are artificial antigen-presenting cells.

In another embodiment, the invention provides the method described in any of the preceding paragraphs as applicable above modified such that the harvesting in step (d) is performed using a membrane-based cell processing system.

In another embodiment, the invention provides the method described in any of the preceding paragraphs as applicable above modified such that the harvesting in step (d) is performed using a LOVO cell processing system.

In another embodiment, the invention provides the method described in any of the preceding paragraphs as applicable above modified such that the multiple fragments comprise at or about 5 to at or about 60 cores or fragments per container in step (b).

In another embodiment, the invention provides the method described in any of the preceding paragraphs as applicable above modified such that the multiple fragments comprise at or about 10 to at or about 60 cores or fragments per container in step (b).

In another embodiment, the invention provides the method described in any of the preceding paragraphs as applicable above modified such that the multiple fragments comprise at or about 15 to at or about 60 cores or fragments per container in step (b).

In another embodiment, the invention provides the method described in any of the preceding paragraphs as applicable above modified such that the multiple fragments comprise at or about 20 to at or about 60 cores or fragments per container in step (b).

In another embodiment, the invention provides the method described in any of the preceding paragraphs as applicable above modified such that the multiple fragments comprise at or about 25 to at or about 60 cores or fragments per container in step (b).

In another embodiment, the invention provides the method described in any of the preceding paragraphs as applicable above modified such that the multiple fragments comprise at or about 30 to at or about 60 cores or fragments per container in step (b).

In another embodiment, the invention provides the method described in any of the preceding paragraphs as applicable above modified such that the multiple fragments comprise at or about 35 to at or about 60 cores or fragments per container in step (b).

In another embodiment, the invention provides the method described in any of the preceding paragraphs as applicable above modified such that the multiple fragments comprise at or about 40 to at or about 60 cores or fragments per container in step (b).

In another embodiment, the invention provides the method described in any of the preceding paragraphs as applicable above modified such that the multiple fragments comprise at or about 45 to at or about 60 cores or fragments per container in step (b).

In another embodiment, the invention provides the method described in any of the preceding paragraphs as applicable above modified such that the multiple fragments comprise at or about 50 to at or about 60 cores or fragments per container in step (b).

In another embodiment, the invention provides the method described in any of the preceding paragraphs as applicable above modified such that the multiple cores or fragments comprise at or about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 cores or fragment(s) per container in step (b).

In another embodiment, the invention provides the method described in any of the preceding paragraphs as applicable above modified such that each cores or fragment has a volume of at or about 1 $mm^3$.

In another embodiment, the invention provides the method described in any of the preceding paragraphs as applicable above modified such that each core or fragment has a volume of at or about 1 $mm^3$ to at or about 10 $mm^3$.

In another embodiment, the invention provides the method described in any of the preceding paragraphs as applicable above modified such that each core fragment has a volume of at or about 1 $mm^3$ to at or about 9 $mm^3$.

In another embodiment, the invention provides the method described in any of the preceding paragraphs as applicable above modified such that each fragment has a volume of at or about 1 $mm^3$ to at or about 8 $mm^3$.

In another embodiment, the invention provides the method described in any of the preceding paragraphs as applicable above modified such that each fragment has a volume of at or about 1 $mm^3$ to at or about 7 $mm^3$.

In another embodiment, the invention provides the method described in any of the preceding paragraphs as applicable above modified such that each fragment has a volume of at or about 1 $mm^3$ to at or about 6 $mm^3$.

In another embodiment, the invention provides the method described in any of the preceding paragraphs as applicable above modified such that each fragment has a volume of at or about 1 $mm^3$ to at or about 5 $mm^3$.

In another embodiment, the invention provides the method described in any of the preceding paragraphs as applicable above modified such that each fragment has a volume of at or about 1 $mm^3$ to at or about 4 $mm^3$.

In another embodiment, the invention provides the method described in any of the preceding paragraphs as applicable above modified such that each fragment has a volume of at or about 1 $mm^3$ to at or about 3 $mm^3$.

In another embodiment, the invention provides the method described in any of the preceding paragraphs as applicable above modified such that each fragment has a volume of at or about 1 $mm^3$ to at or about 2 $mm^3$.

In another embodiment, the invention provides the method described in any of the preceding paragraphs as applicable above modified such that each fragment has a volume of at or about 1, 2, 3, 4, 5, 6, 7, 8, 9 or 10 $mm^3$.

In another embodiment, the invention provides the method described in any of the preceding paragraphs as applicable above modified such that the cell culture medium is provided in a container that is a G-container or a Xuri cellbag.

In another embodiment, the invention provides the method described in any of the preceding paragraphs as applicable above modified such that the IL-2 concentration in the cell culture medium is about 10,000 IU/mL to about 5,000 IU/mL.

In another embodiment, the invention provides the method described in any of the preceding paragraphs as applicable above modified such that the IL-2 concentration in the cell culture medium is about 6,000 IU/mL.

In another embodiment, the invention provides the method described in any of the preceding paragraphs as applicable above modified such that the cryopreservation media comprises dimethlysulfoxide (DMSO).

In another embodiment, the invention provides the method described in any of the preceding paragraphs as applicable above modified such that the cryopreservation media comprises 7% to 10% DMSO.

In another embodiment, the invention provides the method described in any of the preceding paragraphs as applicable above modified such that the first period in step (b) is performed within a period of at or about 3 days, 4 days, 5 days, 6 days, 7 days, 8 days, 9 days, 10 days, 11 days, 12 days.

In another embodiment, the invention provides the method described in any of the preceding paragraphs as applicable above modified such that the second period in step (c) is performed within a period of at or about 3 days, 4 days, 5 days, 6 days, 7 days, 8 days, 9 days, 10 days or 11 days.

In another embodiment, the invention provides the method described in any of the preceding paragraphs as applicable above modified such that the first period in step (b) and the second period in step (c) are each individually performed within a period of at or about 3 days, 4 days, 5 days, 6 days, 7 days, 8 days, 9 days, 10 days, 11 days or 12 days.

In another embodiment, the invention provides the method described in any of the preceding paragraphs as applicable above modified such that the first period in step (b) and the second period in step (c) are each individually performed within a period of at or about 5 days, 6 days, 7 days, 8 days, 9 days, 10 days, 11 days, or 12 days.

In another embodiment, the invention provides the method described in any of the preceding paragraphs as applicable above modified such that the first period in step (b) and the second period in step (c) are each individually performed within a period of at or about 7 days.

In another embodiment, the invention provides the method described in any of the preceding paragraphs as applicable above modified such that steps (a) through (d) are performed in a total of at or about 14 days to at or about 28 days.

In another embodiment, the invention provides the method described in any of the preceding paragraphs as applicable above modified such that steps (a) through (d) are performed in a total of at or about 15 days to at or about 28 days.

In another embodiment, the invention provides the method described in any of the preceding paragraphs as applicable above modified such that steps (a) through (d) are performed in a total of at or about 16 days to at or about 28 days.

In another embodiment, the invention provides the method described in any of the preceding paragraphs as applicable above modified such that steps (a) through (d) are performed in a total of at or about 17 days to at or about 28 days.

In another embodiment, the invention provides the method described in any of the preceding paragraphs as applicable above modified such that steps (a) through (d) are performed in a total of at or about 18 days to at or about 28 days.

In another embodiment, the invention provides the method described in any of the preceding paragraphs as applicable above modified such that steps (a) through (d) are performed in a total of at or about 19 days to at or about 28 days.

In another embodiment, the invention provides the method described in any of the preceding paragraphs as applicable above modified such that steps (a) through (d) are performed in a total of at or about 20 days to at or about 28 days.

In another embodiment, the invention provides the method described in any of the preceding paragraphs as applicable above modified such that steps (a) through (d) are performed in a total of at or about 21 days to at or about 28 days.

In another embodiment, the invention provides the method described in any of the preceding paragraphs as applicable above modified such that steps (a) through (d) are performed in a total of at or about 22 days to at or about 28 days.

In another embodiment, the invention provides the method described in any of the preceding paragraphs as applicable above modified such that steps (a) through (d) are performed in a total of at or about 23 days to at or about 28 days.

In another embodiment, the invention provides the method described in any of the preceding paragraphs as applicable above modified such that steps (a) through (d) are performed in a total of at or about 24 days to at or about 28 days.

In another embodiment, the invention provides the method described in any of the preceding paragraphs as applicable above modified such that steps (a) through (d) are performed in a total of at or about 25 days to at or about 28 days.

In another embodiment, the invention provides the method described in any of the preceding paragraphs as applicable above modified such that steps (a) through (d) are performed in a total of at or about 26 days to at or about 28 days.

In another embodiment, the invention provides the method described in any of the preceding paragraphs as applicable above modified such that steps (a) through (d) are performed in a total of at or about 27 days to at or about 28 days.

In another embodiment, the invention provides the method described in any of the preceding paragraphs as applicable above modified such that steps (a) through (d) are performed in a total of at or about 14 days.

In another embodiment, the invention provides the method described in any of the preceding paragraphs as applicable above modified such that steps (a) through (d) are performed in a total of at or about 15 days.

In another embodiment, the invention provides the method described in any of the preceding paragraphs as applicable above modified such that steps (a) through (d) are performed in a total of at or about 16 days.

In another embodiment, the invention provides the method described in any of the preceding paragraphs as applicable above modified such that steps (a) through (d) are performed in a total of at or about 17 days.

In another embodiment, the invention provides the method described in any of the preceding paragraphs as applicable above modified such that steps (a) through (d) are performed in a total of at or about 18 days.

In another embodiment, the invention provides the method described in any of the preceding paragraphs as applicable above modified such that steps (a) through (d) are performed in a total of at or about 19 days.

In another embodiment, the invention provides the method described in any of the preceding paragraphs as applicable above modified such that steps (a) through (d) are performed in a total of at or about 20 days.

In another embodiment, the invention provides the method described in any of the preceding paragraphs as applicable above modified such that steps (a) through (d) are performed in a total of at or about 21 days.

In another embodiment, the invention provides the method described in any of the preceding paragraphs as applicable above modified such that steps (a) through (d) are performed in a total of at or about 22 days.

In another embodiment, the invention provides the method described in any of the preceding paragraphs as applicable above modified such that steps (a) through (d) are performed in a total of at or about 23 days.

In another embodiment, the invention provides the method described in any of the preceding paragraphs as applicable above modified such that steps (a) through (d) are performed in a total of at or about 24 days.

In another embodiment, the invention provides the method described in any of the preceding paragraphs as applicable above modified such that steps (a) through (d) are performed in a total of at or about 25 days.

In another embodiment, the invention provides the method described in any of the preceding paragraphs as applicable above modified such that steps (a) through (d) are performed in a total of at or about 26 days.

In another embodiment, the invention provides the method described in any of the preceding paragraphs as applicable above modified such that steps (a) through (d) are performed in a total of at or about 27 days.

In another embodiment, the invention provides the method described in any of the preceding paragraphs as applicable above modified such that steps (a) through (d) are performed in a total of at or about 28 days.

In another embodiment, the invention provides the method described in any of the preceding paragraphs as applicable above modified such that steps (a) through (d) are performed in a total of at or about 16 days or less.

In another embodiment, the invention provides the method described in any of the preceding paragraphs as applicable above modified such that steps (a) through (d) are performed in a total of at or about 20 days or less.

In another embodiment, the invention provides the method described in any of the preceding paragraphs as applicable above modified such that steps (a) through (d) are performed in a total of at or about 24 days or less.

In another embodiment, the invention provides the method described in any of the preceding paragraphs as applicable above modified such that steps (a) through (d) are performed in a total of at or about 28 days or less.

In another embodiment, the invention provides the method described in any of the preceding paragraphs as applicable above modified such that the therapeutic population of TILs harvested in step (d) comprises sufficient TILs for a therapeutically effective dosage of the TILs.

In another embodiment, the invention provides the method described in any of the preceding paragraphs as applicable above modified such that the number of TILs sufficient for a therapeutically effective dosage is from at or about $2.3\times10^{10}$ to at or about $13.7\times10^{10}$.

In another embodiment, the invention provides the method described in any of the preceding paragraphs as applicable above modified such that the third population of TILs in step (c) provides for increased efficacy, increased interferon-gamma production, and/or increased polyclonality.

In another embodiment, the invention provides the method described in any of the preceding paragraphs as applicable above modified such that the third population of TILs in step (c) provides for at least a one-fold to five-fold or more interferon-gamma production as compared to TILs prepared by a process longer than 16 days.

In another embodiment, the invention provides the method described in any of the preceding paragraphs as applicable above modified such that the effector T cells and/or central memory T cells obtained from the third population of TILs step (c) exhibit increased CD8 and CD28 expression relative to effector T cells and/or central memory T cells obtained from the second population of cells step (b).

In another embodiment, the invention provides the method described in any of the preceding paragraphs as applicable above modified such that each container recited in the method is a closed container.

In another embodiment, the invention provides the method described in any of the preceding paragraphs as applicable above modified such that each container recited in the method is a G-container.

In another embodiment, the invention provides the method described in any of the preceding paragraphs as applicable above modified such that each container recited in the method is a GREX-10.

In another embodiment, the invention provides the method described in any of the preceding paragraphs as applicable above modified such that each container recited in the method is a GREX-100.

In another embodiment, the invention provides the method described in any of the preceding paragraphs as applicable above modified such that each container recited in the method is a GREX-500.

Also disclosed herein but not claimed is the therapeutic population of tumor infiltrating lymphocytes (TILs) made by the method described in any of the preceding paragraphs as applicable above.

The disclosure herein also provides a therapeutic population of tumor infiltrating lymphocytes (TILs) prepared from tumor tissue of a patient, wherein the therapeutic population of TILs provides for increased efficacy, increased interferon-gamma production, and/or increased polyclonality compared to TILs prepared by a process in which the first expansion of TILs is performed without any added antigen-presenting cells (APCs) or OKT3.

The disclosure herein also provides a therapeutic population of tumor infiltrating lymphocytes (TILs) prepared from tumor tissue of a patient, wherein the therapeutic population of TILs provides for increased efficacy, increased interferon-gamma production, and/or increased polyclonality compared to TILs prepared by a process in which the first expansion of TILs is performed without any added antigen-presenting cells (APCs).

The disclosure herein also provides a therapeutic population of tumor infiltrating lymphocytes (TILs) prepared from tumor tissue of a patient, wherein the therapeutic population of TILs provides for increased efficacy, increased interferon-gamma production, and/or increased polyclonality compared to TILs prepared by a process in which the first expansion of TILs is performed without any added OKT3.

The disclosure herein also provides a therapeutic population of tumor infiltrating lymphocytes (TILs) prepared from tumor tissue of a patient, wherein the therapeutic population of TILs provides for increased efficacy, increased interferon-gamma production, and/or increased polyclonality compared to TILs prepared by a process in which the first expansion of TILs is performed with no added antigen-presenting cells (APCs) and no added OKT3.

The disclosure herein also provides a therapeutic population of tumor infiltrating lymphocytes (TILs) prepared from tumor tissue of a patient, wherein the therapeutic population of TILs provides for increased efficacy, increased interferon-gamma production, and/or increased polyclonality compared to TILs prepared by a process by a process longer than 16 days.

The disclosure herein also provides for the therapeutic population of TILs described in any of the preceding paragraphs as applicable above that provides for increased interferon-gamma production.

The disclosure herein also provides for the therapeutic population of TILs described in any of the preceding paragraphs as applicable above that provides for increased polyclonality.

The disclosure herein also provides for the therapeutic population of TILs described in any of the preceding paragraphs as applicable above that provides for increased efficacy.

The disclosure herein also provides for the therapeutic population of TILs described in any of the preceding paragraphs as applicable above modified such that the therapeutic population of TILs is capable of at least one-fold more interferon-gamma production as compared to TILs prepared by a process longer than 16 days.

The disclosure herein also provides for the therapeutic population of TILs described in any of the preceding paragraphs as applicable above modified such that the therapeutic population of TILs is capable of at least two-fold more interferon-gamma production as compared to TILs prepared by a process longer than 16 days.

The disclosure herein also provides for the therapeutic population of TILs described in any of the preceding paragraphs as applicable above modified such that the therapeutic population of TILs is capable of at least three-fold more interferon-gamma production as compared to TILs prepared by a process longer than 16 days.

The disclosure herein also provides for a therapeutic population of tumor infiltrating lymphocytes (TILs) that is capable of at least one-fold more interferon-gamma production as compared to TILs prepared by a process in which the first expansion of TILs is performed without any added antigen-presenting cells (APCs).

The disclosure herein also provides for a therapeutic population of tumor infiltrating lymphocytes (TILs) that is capable of at least one-fold more interferon-gamma production as compared to TILs prepared by a process in which the first expansion of TILs is performed without any added OKT3.

The disclosure herein also provides for a therapeutic population of TILs that is capable of at least two-fold more interferon-gamma production as compared to TILs prepared by a process in which the first expansion of TILs is performed without any added APCs.

The disclosure herein also provides for a therapeutic population of TILs that is capable of at least two-fold more interferon-gamma production as compared to TILs prepared by a process in which the first expansion of TILs is performed without any added OKT3.

The disclosure herein also provides for a therapeutic population of TILs that is capable of at least three-fold more interferon-gamma production as compared to TILs prepared by a process in which the first expansion of TILs is performed without any added APCs.

The disclosure herein also provides for a therapeutic population of TILs that is capable of at least three-fold more interferon-gamma production as compared to TILs prepared by a process in which the first expansion of TILs is performed without any added OKT3.

In another embodiment, the invention provides a method of expanding T cells comprising: (a) performing a priming first expansion of a first population of T cells obtained from a tumor fragment or core obtained from a donor by culturing the first population of T cells to effect growth and to prime an activation of the first population of T cells; (b) after the activation of the first population of T cells primed in step (a) begins to decay, performing a rapid second expansion of the first population of T cells by culturing the first population of T cells to effect growth and to boost the activation of the first population of T cells to obtain a second population of T cells; and (c) harvesting the second population of T cells. In another embodiment, the step of rapid second expansion is split into a plurality of steps to achieve a scaling up of the culture by: (a) performing the rapid second expansion by culturing the first population of T cells in a small scale culture in a first container, *e.g.,* a G-REX 100MCS container, for a period of about 3 to 4 days, and then (b) effecting the transfer of the first population of T cells from the small scale culture to a second container larger than the first container, *e.g.,* a G-REX 500MCS container, and culturing the first population of T cells from the small scale culture in a larger scale culture in the second container for a period of about 4 to 7 days. In another embodiment, the step of rapid expansion is split into a plurality of steps to achieve a scaling out of the culture by: (a) performing the rapid second expansion by culturing the first population of T cells in a first small scale culture in a first container, *e.g.,* a G-REX 100MCS container, for a period of about 3 to 4 days, and then (b) effecting the transfer and apportioning of the first population of T cells from the first small scale culture into and amongst at least 2, 3, 4, 5, 6, 7, 8, 9, 10, 11, 12, 13, 14, 15, 16, 17, 18, 19, or 20 second containers that are equal in size to the first container, wherein in each second container the portion of the first population of T cells from first small scale culture transferred to such second container is cultured in a second small scale culture for a period of about 4 to 7 days. In another embodiment, the step of rapid expansion is split into a plurality of steps to achieve a scaling out and scaling up of the culture by: (a) performing the rapid second expansion by culturing the first population of T cells in a small scale culture in a first container, *e.g.,* a G-REX 100MCS container, for a period of about 3 to 4 days, and then (b) effecting the transfer and apportioning of the first population of T cells from the small scale culture into and amongst at least 2, 3, 4, 5, 6, 7, 8, 9, 10, 11, 12, 13, 14, 15, 16, 17, 18, 19, or 20 second containers that are larger in size than the first container, e.g., G-REX 500MCS containers, wherein in each second container the portion of the first population of T cells from the small scale culture transferred to such second container is cultured in a larger scale culture for a period of about 4 to 7 days. In another embodiment, the step of rapid expansion is split into a plurality of steps to achieve a scaling out and scaling up of the culture by: (a) performing the rapid second expansion by culturing the first population of T cells in a small scale culture in a first container, *e.g.,* a G-REX 100MCS container, for a period of about 4 days, and then (b) effecting the transfer and apportioning of the first population of T cells from the small scale culture into and amongst 2, 3 or 4 second containers that are larger in size than the first container, *e.g.,* G-REX 500MCS containers, wherein in each second container the portion of the first population of T cells from the small scale culture transferred to such second container is cultured in a larger scale culture for a period of about 5 days.

Also disclosed herein but not claimed, the method described in any of the preceding paragraphs as applicable above modified such that the priming first expansion of step (a) is performed during a period of up to 17 days.

In another embodiment, the invention provides the method described in any of the preceding paragraphs as applicable above modified such that the rapid second expansion of step (b) is performed during a period of up to 11 days.

Also disclosed herein but not claimed, the method described in any of the preceding paragraphs as applicable above modified such that the priming first expansion in step (a) is performed during a period of 17 days and the rapid second expansion of step (b) is performed during a period of up to 9 days.

In another embodiment, the invention provides the method described in any of the preceding paragraphs as applicable above modified such that in step (a) the first population of T cells is cultured in a first culture medium comprising OKT-3 and IL-2.

In another embodiment, the invention provides the method described in any of the preceding paragraphs as applicable above modified such that the first culture medium comprises OKT-3, IL-2 and antigen-presenting cells (APCs).

In another embodiment, the invention provides the method described in any of the preceding paragraphs as applicable above modified such that in step (b) the first population of T cells is cultured in a second culture medium comprising OKT-3, IL-2 and antigen-presenting cells (APCs).

In another embodiment, the invention provides the method described in any of the preceding paragraphs as applicable above modified such that in step (a) the first population of T cells is cultured in a first culture medium in a container comprising a first gas-permeable surface, wherein the first culture medium comprises OKT-3, IL-2 and a first population of antigen-presenting cells (APCs), wherein the first population of APCs is exogenous to the donor of the first population of T cells and the first population of APCs is layered onto the first gas-permeable surface, wherein in step (b) the first population of T cells is cultured in a second culture medium in the container, wherein the second culture medium comprises OKT-3, IL-2 and a second population of APCs, wherein the second population of APCs is exogenous to the donor of the first population of T cells and the second population of APCs is layered onto the first gas-permeable surface, and wherein the second population of APCs is greater than the first population of APCs.

In another embodiment, the invention provides the method described in any of the preceding paragraphs as applicable above modified such that the ratio of the number of APCs in the second population of APCs to the number of APCs in the first population of APCs is about 2: 1.

In another embodiment, the invention provides the method described in any of the preceding paragraphs as applicable above modified such that the number of APCs in the first population of APCs is about $2.5 \times 10^8$ and the number of APCs in the second population of APCs is about $5 \times 10^8$.

In another embodiment, the invention provides the method described in any of the preceding paragraphs as applicable above modified such that in step (a) the first population of APCs is layered onto the first gas-permeable surface at an average thickness of 2 layers of APCs.

In another embodiment, the invention provides the method described in any of the preceding paragraphs as applicable above modified such that in step (b) the second population of APCs is layered onto the first gas-permeable surface at an average thickness selected from the range of 4 to 8 layers of APCs.

In another embodiment, the invention provides the method described in any of the preceding paragraphs as applicable above modified such that the ratio of the average number of layers of APCs layered onto the first gas-permeable surface in step (b) to the average number of layers of APCs layered onto the first gas-permeable surface in step (a) is 2:1.

In another embodiment, the invention provides the method described in any of the preceding paragraphs as applicable above modified such that in step (a) the first population of APCs is seeded on the first gas permeable surface at a density selected from the range of at or about $1.0 \times 10^6$ APCs/$cm^2$ to at or about $4.5 \times 10^6$ APCs/$cm^2$.

In another embodiment, the invention provides the method described in any of the preceding paragraphs as applicable above modified such that in step (a) the first population of APCs is seeded on the first gas permeable surface at a density selected from the range of at or about $1.5 \times 10^6$ APCs/$cm^2$ to at or about $3.5 \times 10^6$ APCs/$cm^2$.

In another embodiment, the invention provides the method described in any of the preceding paragraphs as applicable above modified such that in step (a) the first population of APCs is seeded on the first gas permeable surface at a density selected from the range of at or about $2.0 \times 10^6$ APCs/$cm^2$ to at or about $3.0 \times 10^6$ APCs/$cm^2$.

In another embodiment, the invention provides the method described in any of the preceding paragraphs as applicable above modified such that in step (a) the first population of APCs is seeded on the first gas permeable surface at a density of at or about $2.0 \times 10^6$ APCs/$cm^2$.

In another embodiment, the invention provides the method described any of the preceding paragraphs as applicable above modified such that in step (b) the second population of APCs is seeded on the first gas permeable surface at a density selected from the range of at or about $2.5 \times 10^6$ APCs/$cm^2$ to at or about $7.5 \times 10^6$ APCs/$cm^2$.

In another embodiment, the invention provides the method described in any of the preceding paragraphs as applicable above modified such that in step (b) the second population of APCs is seeded on the first gas permeable surface at a density selected from the range of at or about $3.5 \times 10^6$ APCs/$cm^2$ to at or about $6.0 \times 10^6$ APCs/$cm^2$.

In another embodiment, the invention provides the method described in any of the preceding paragraphs as applicable above modified such that in step (b) the second population of APCs is seeded on the first gas permeable surface at a density selected from the range of at or about $4.0 \times 10^6$ APCs/$cm^2$ to at or about $5.5 \times 10^6$ APCs/$cm^2$.

In another embodiment, the invention provides the method described any of the preceding paragraphs as applicable above modified such that in step (b) the second population of APCs is seeded on the first gas permeable surface at a density of at or about $4.0 \times 10^6$ APCs/$cm^2$.

In another embodiment, the invention provides the method described in any of the preceding paragraphs as applicable above modified such that in step (a) the first population of APCs is seeded on the first gas permeable surface at a density selected from the range of at or about $1.0 \times 10^6$ APCs/$cm^2$ to at or about $4.5 \times 10^6$ APCs/$cm^2$ and in step (b) the second population of APCs is seeded on the first gas permeable surface at a density selected from the range of at or about $2.5 \times 10^6$ APCs/$cm^2$ to at or about $7.5 \times 10^6$ APCs/$cm^2$.

In another embodiment, the invention provides the method described in any of the preceding paragraphs as applicable above modified such that in step (a) the first population of APCs is seeded on the first gas permeable surface at a density selected from the range of at or about $1.5 \times 10^6$ APCs/$cm^2$ to at or about $3.5 \times 10^6$ APCs/$cm^2$ and in step (b) the second population of APCs is seeded on the first gas permeable surface at a density selected from the range of at or about $3.5 \times 10^6$ APCs/$cm^2$ to at or about $6.0 \times 10^6$ APCs/$cm^2$.

In another embodiment, the invention provides the method described in any of the preceding paragraphs as applicable above modified such that in step (a) the first population of APCs is seeded on the first gas permeable surface at a density selected from the range of at or about $2.0 \times 10^6$ APCs/$cm^2$ to at or about $3.0 \times 10^6$ APCs/$cm^2$ and in step (b) the second population of APCs is seeded on the first gas permeable surface at a density selected from the range of at or about $4.0 \times 10^6$ APCs/$cm^2$ to at or about $5.5 \times 10^6$ APCs/$cm^2$.

In another embodiment, the invention provides the method described in any of the preceding paragraphs as applicable above modified such that in step (a) the first population of APCs is seeded on the first gas permeable surface at a density of at or about $2.0 \times 10^6$ APCs/$cm^2$ and in step (b) the second population of APCs is seeded on the first gas permeable surface at a density of at or about $4.0 \times 10^6$ APCs/$cm^2$.

In another embodiment, the invention provides the method described in any of the preceding paragraphs as applicable above modified such that the APCs are peripheral blood mononuclear cells (PBMCs).

In another embodiment, the invention provides the method described in any of the preceding paragraphs as applicable above modified such that the PBMCs are irradiated and exogenous to the donor of the first population of T cells.

In another embodiment, the invention provides the method described in any of the preceding paragraphs as applicable above modified such that the T cells are tumor infiltrating lymphocytes (TILs), the first population of T cells is a first population of TILs in step (a), the second population of T cells is a second population of TILs in step (b), the second population of TILs is a therapeutic population of TILs, and the therapeutic population of TILs is harvested in step (c).

Also disclosed herein the method described in any of the preceding paragraphs as applicable above modified such that the T cells are marrow infiltrating lymphocytes (MILs).

In another embodiment, the invention provides the method described in any of the preceding paragraphs as applicable above modified such that the T cell phenotype is CD3+ and CD45+.

## XV. Pharmaceutical Compositions, Dosages, and Dosing Regimens

In an embodiment, TILs expanded using the methods of the present disclosure are for administration to a patient as a pharmaceutical composition. The pharmaceutical composition may be a suspension of TILs in a sterile buffer. TILs expanded using PBMCs of the present disclosure may be administered by any suitable route as known in the art. In some embodiments, the T-cells are for administration as a single intra-arterial or intravenous infusion, which preferably lasts approximately 30 to 60 minutes. Other suitable routes of administration include intraperitoneal, intrathecal, and intralymphatic administration.

Any suitable dose of TILs can be administered. In some embodiments, from about $2.3\times10^{10}$ to about $13.7\times10^{10}$ TILs are for administration, with an average of around $7.8\times10^{10}$ TILs, particularly if the cancer is melanoma. In an embodiment, about $1.2\times10^{10}$ to about $4.3\times10^{10}$ of TILs are for administration. In some embodiments, about $3\times10^{10}$ to about $12\times10^{10}$ TILs are for administration. In some embodiments, about $4\times10^{10}$ to about $10\times10^{10}$ TILs are for administration. In some embodiments, about $5\times10^{10}$ to about $8\times10^{10}$ TILs are for administration. In some embodiments, about $6\times10^{10}$ to about $8\times10^{10}$ TILs are for administration. In some embodiments, about $7\times10^{10}$ to about $8\times10^{10}$ TILs are for administration. In some embodiments, the therapeutically effective dosage is about $2.3\times10^{10}$ to about $13.7\times10^{10}$. In some embodiments, the therapeutically effective dosage is about $7.8\times10^{10}$ TILs, particularly of the cancer is melanoma. In some embodiments, the therapeutically effective dosage is about $1.2\times10^{10}$ to about $4.3\times10^{10}$ of TILs. In some embodiments, the therapeutically effective dosage is about $3\times10^{10}$ to about $12\times10^{10}$ TILs. In some embodiments, the therapeutically effective dosage is about $4\times10^{10}$ to about $10\times10^{10}$ TILs. In some embodiments, the therapeutically effective dosage is about $5\times10^{10}$ to about $8\times10^{10}$ TILs. In some embodiments, the therapeutically effective dosage is about $6\times10^{10}$ to about $8\times10^{10}$ TILs. In some embodiments, the therapeutically effective dosage is about $7\times10^{10}$ to about $8\times10^{10}$ TILs.

In some embodiments, the number of the TILs provided in the pharmaceutical compositions of the invention is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In an embodiment, the number of the TILs provided in the pharmaceutical compositions of the invention is in the range of $1\times10^6$ to $5\times10^6$, $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$, and $5\times10^{12}$ to $1\times10^{13}$.

In some embodiments, the concentration of the TILs provided in the pharmaceutical compositions of the invention is less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v of the pharmaceutical composition.

In some embodiments, the concentration of the TILs provided in the pharmaceutical compositions of the invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v of the pharmaceutical composition.

In some embodiments, the concentration of the TILs provided in the pharmaceutical compositions of the invention is in the range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12% or about 1% to about 10% w/w, w/v or v/v of the pharmaceutical composition.

In some embodiments, the concentration of the TILs provided in the pharmaceutical compositions of the invention is in the range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, about 0.1% to about 0.9% w/w, w/v or v/v of the pharmaceutical composition.

In some embodiments, the amount of the TILs provided in the pharmaceutical compositions of the invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of the TILs provided in the pharmaceutical compositions of the invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

The TILs provided in the pharmaceutical compositions of the invention are effective over a wide dosage range. The exact dosage will depend upon the route of administration, the form in which the compound is administered, the gender and age of the subject to be treated, the body weight of the subject to be treated, and the preference and experience of the attending physician. The clinically-established dosages of the TILs may also be used if appropriate. The amounts of the pharmaceutical compositions administered using the methods herein, such as the dosages of TILs, will be dependent on the human or mammal being treated, the severity of the disorder or condition, the rate of administration, the disposition of the active pharmaceutical ingredients and the discretion of the prescribing physician.

In some embodiments, TILs may be for administration in a single dose. Such administration may be by injection, e.g., intravenous injection. In some embodiments, TILs may be for administration in multiple doses. Dosing may be once, twice, three times, four times, five times, six times, or more than six times per year. Dosing may be once a month, once every two weeks, once a week, or once every other day. Administration of TILs may continue as long as necessary.

In some embodiments, an effective dosage of TILs is about $1\times10^6$, $2\times10^6$, $3\times10^6$, $4\times10^6$, $5\times10^6$, $6\times10^6$, $7\times10^6$, $8\times10^6$, $9\times10^6$, $1\times10^7$, $2\times10^7$, $3\times10^7$, $4\times10^7$, $5\times10^7$, $6\times10^7$, $7\times10^7$, $8\times10^7$, $9\times10^7$, $1\times10^8$, $2\times10^8$, $3\times10^8$, $4\times10^8$, $5\times10^8$, $6\times10^8$, $7\times10^8$, $8\times10^8$, $9\times10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$, $2\times10^{10}$, $3\times10^{10}$, $4\times10^{10}$, $5\times10^{10}$, $6\times10^{10}$, $7\times10^{10}$, $8\times10^{10}$, $9\times10^{10}$, $1\times10^{11}$, $2\times10^{11}$, $3\times10^{11}$, $4\times10^{11}$, $5\times10^{11}$, $6\times10^{11}$, $7\times10^{11}$, $8\times10^{11}$, $9\times10^{11}$, $1\times10^{12}$, $2\times10^{12}$, $3\times10^{12}$, $4\times10^{12}$, $5\times10^{12}$, $6\times10^{12}$, $7\times10^{12}$, $8\times10^{12}$, $9\times10^{12}$, $1\times10^{13}$, $2\times10^{13}$, $3\times10^{13}$, $4\times10^{13}$, $5\times10^{13}$, $6\times10^{13}$, $7\times10^{13}$, $8\times10^{13}$, and $9\times10^{13}$. In some embodiments, an effective dosage of TILs is in the range of $1\times10^6$ to $5\times10^6$, $5\times10^6$ to $1\times10^7$, $1\times10^7$ to $5\times10^7$, $5\times10^7$ to $1\times10^8$, $1\times10^8$ to $5\times10^8$, $5\times10^8$ to $1\times10^9$, $1\times10^9$ to $5\times10^9$, $5\times10^9$ to $1\times10^{10}$, $1\times10^{10}$ to $5\times10^{10}$, $5\times10^{10}$ to $1\times10^{11}$, $5\times10^{11}$ to $1\times10^{12}$, $1\times10^{12}$ to $5\times10^{12}$, and $5\times10^{12}$ to $1\times10^{13}$.

In some embodiments, an effective dosage of TILs is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg.

In some embodiments, an effective dosage of TILs is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg.

An effective amount of the TILs may be for administration in either single or multiple doses by any of the accepted modes of administration of agents having similar utilities, including intranasal and transdermal routes, by intra-arterial injection, intravenously, intraperitoneally, parenterally, intramuscularly, subcutaneously, topically, by transplantation, or by inhalation.

Also disclosed herein but not claimed is an infusion bag comprising the therapeutic population of TILs described in any of the preceding paragraphs as applicable above.

Also disclosed herein but not claimed is a tumor infiltrating lymphocyte (TIL) composition comprising the therapeutic population of TILs described in any of the preceding paragraphs as applicable above and a pharmaceutically acceptable carrier.

Also disclosed herein but not claimed is an infusion bag comprising the TIL composition described in any of the preceding paragraphs as applicable above.

Also disclosed herein but not claimed is a cryopreserved preparation of the therapeutic population of TILs described in in any of the preceding paragraphs as applicable above.

Also disclosed herein but not claimed is a tumor infiltrating lymphocyte (TIL) composition comprising the therapeutic population of TILs described in any of the preceding paragraphs as applicable above and a cryopreservation media.

Also disclosed herein but not claimed is the TIL composition described in any of the preceding paragraphs as applicable above modified such that the cryopreservation media contains DMSO.

Also disclosed herein but not claimed is the TIL composition described in any of the preceding paragraphs as applicable above modified such that the cryopreservation media contains 7-10% DMSO.

Also disclosed herein but not claimed is a cryopreserved preparation of the TIL composition described in any of the preceding paragraphs as applicable above.

## XVI. Methods of Treating Patients

Methods of treatment begin with the initial TIL collection and culture of TILs. Such methods have been both described in the art by, for example, Jin et al., J. Immunotherapy, 2012, 35(3):283-292. Methods of treatment which are not claimed are described throughout the sections below, including the Examples.

The expanded TILs produced according the methods described herein, including, for example as described in Steps A through F above or according to Steps A through F above (also as shown, for example, in Figure 85 (in particular, e.g., Figure 85B)) find particular use in the treatment of patients with cancer (for example, as described in Goff, et al., J. Clinical Oncology, 2016, 34(20):2389-239, as well as the supplemental content. In some embodiments, TIL were grown from resected deposits of metastatic melanoma as previously described (see, Dudley, et al., J Immunother., 2003, 26:332-342). Fresh tumor can be dissected under sterile conditions. A representative sample can be collected for formal pathologic analysis. Single fragments of 2 $mm^3$ to 3 $mm^3$ may be used. In some embodiments, 5, 10, 15, 20, 25 or 30 samples per patient are obtained. In some embodiments, 20, 25, or 30 samples per patient are obtained. In some embodiments, 20, 22, 24, 26, or 28 samples per patient are obtained. In some embodiments, 24 samples per patient are obtained. Samples can be placed in individual wells of a 24-well plate, maintained in growth media with high-dose IL-2 (6,000 IU/mL), and monitored for destruction of tumor and/or proliferation of TIL. Any tumor with viable cells remaining after processing can be enzymatically digested into a single cell suspension and cryopreserved, as described herein.

Successfully grown TIL can be sampled for phenotype analysis (CD3, CD4, CD8, and CD56) and tested against autologous tumor when available. TIL can be considered reactive if overnight coculture yielded interferon-gamma (IFN-$\gamma$) levels > 200 pg/mL and twice background. (Goff, et al., J Immunother., 2010, 33:840-847). Cultures with evidence of autologous reactivity or sufficient growth patterns can be selected for a second expansion (for example, a second expansion as provided in according to Step D of Figure 85 (in particular, *e.g.,* Figure 85B)), including second expansions that are sometimes referred to as rapid expansion (REP). Expanded TILs with high autologous reactivity (for example, high proliferation during a second expansion), may be selected for an additional second expansion. TILs with high autologous reactivity (for example, high proliferation during second expansion as provided in Step D of Figure 85 (in particular, *e.g.,* Figure 85B)), may be selected for an additional second expansion according to Step D of Figure 85 (in particular, *e.g.,* Figure 85B).

Also disclosed herein but not claimed, the patient is not moved directly to ACT (adoptive cell transfer), for example, after tumor harvesting and/or a first expansion, the cells are not utilized immediately. TILs can be cryopreserved and thawed 2 days before administration to a patient. TILs can be cryopreserved and thawed 1 day before administration to a patient. The TILs can be cryopreserved and thawed immediately before the administration to a patient.

Cell phenotypes of cryopreserved samples of infusion bag TIL can be analyzed by flow cytometry (*e.g.,* FlowJo™)

for surface markers CD3, CD4, CD8, CCR7, and CD45RA (BD BioSciences), as well as by any of the methods described herein. Serum cytokines were measured by using standard enzyme-linked immunosorbent assay techniques. A rise in serum IFN-g was defined as >100 pg/mL and greater than 4 3 baseline levels.

In some embodiments, the TILs produced by the methods provided herein, for example those exemplified in Figure 85 (in particular, *e.g.,* Figure 85B), provide for a surprising improvement in clinical efficacy of the TILs. In some embodiments, the TILs produced by the methods provided herein, for example those exemplified in Figure 85 (in particular, *e.g.,* Figure 85B), exhibit increased clinical efficacy as compared to TILs produced by methods other than those described herein, including, for example, methods other than those exemplified in Figure 85 (in particular, *e.g.,* Figure 85B). In some embodiments, the methods other than those described herein include methods referred to as process 1C and/or Generation 1 (Gen 1). In some embodiments, the increased efficacy is measured by DCR, ORR, and/or other clinical responses. In some embodiments, the TILS produced by the methods provided herein, for example those exemplified in Figure 85 (in particular, *e.g.,* Figure 85B), exhibit a similar time to response and safety profile compared to TILs produced by methods other than those described herein, including, for example, methods other than those exemplified in Figure 85 (in particular, *e.g.,* Figure 85B), for example the Gen 1 process.

In some embodiments, IFN-gamma (IFN-$\gamma$) is indicative of treatment efficacy and/or increased clinical efficacy. In some embodiments, IFN-$\gamma$ in the blood of subjects treated with TILs is indicative of active TILs. A potency assay for IFN-$\gamma$ production may be employed. IFN-$\gamma$ production is another measure of cytotoxic potential. IFN-$\gamma$ production can be measured by determining the levels of the cytokine IFN-$\gamma$ in the blood, serum, or TILs *ex vivo* of a subject treated with TILs prepared by the methods of the present invention, including those as described for example in Figure 85 (in particular, *e.g.,* Figure 85B). In some embodiments, an increase in IFN-$\gamma$ is indicative of treatment efficacy in a patient treated with the TILs produced by the methods of the present invention. In some embodiments, IFN-$\gamma$ is increased one-fold, two-fold, three-fold, four-fold, or five-fold or more as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ secretion is increased one-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ secretion is increased two-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ secretion is increased three-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ secretion is increased four-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ secretion is increased five-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-$\gamma$ is measured using a Quantikine ELISA kit. In some embodiments, IFN-$\gamma$ is measured in TILs *ex vivo* of a subject treated with TILs prepared by the methods of the present invention, including those as described for example in Figure 85 (in particular, *e.g.,* Figure 85B). IFN-$\gamma$ may be measured in blood of a subject treated with TILs prepared by the methods of the present invention, including those as described for example in Figure 85 (in particular, *e.g.,* Figure 85B). IFN-$\gamma$ may be measured in TILs serum of a subject treated with TILs prepared by the methods of the present invention, including those as described for example in Figure 85 (in particular, *e.g.,* Figure 85B).

In some embodiments, the TILs prepared by the methods of the present invention, including those as described for example in Figure 85 (in particular, *e.g.,* Figure 85B), exhibit increased polyclonality as compared to TILs produced by other methods, including those not exemplified in Figure 85 (in particular, *e.g.,* Figure 85B), such as for example, methods referred to as process 1C methods. In some embodiments, significantly improved polyclonality and/or increased polyclonality is indicative of treatment efficacy and/or increased clinical efficacy. In some embodiments, polyclonality refers to the T-cell repertoire diversity. In some embodiments, an increase in polyclonality can be indicative of treatment efficacy with regard to administration of the TILs produced by the methods of the present invention. In some embodiments, polyclonality is increased one-fold, two-fold, ten-fold, 100-fold, 500-fold, or 1000-fold as compared to TILs prepared using methods than those provide herein including, for example, methods other than those embodied in Figure 85 (in particular, *e.g.,* Figure 85B). In some embodiments, polyclonality is increased one-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased two-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased ten-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 100-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 500-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 1000-fold as compared to an untreated patient and/or as compared to a patient treated with TILs prepared using other methods than those provide herein including, for example, methods other than those embodied in Figure 85 (in particular, *e.g.,* Figure 85B).

Measures of efficacy can include the disease control rate (DCR) as well as overall response rate (ORR), as known in the art as well as described herein.

1. **Methods of Treating Cancers and Other Diseases**

The compositions and methods described herein can be for use in a method for treating diseases. They may be for use in treating hyperproliferative disorders. They may also be for use in treating other disorders as described herein and in the following paragraphs.

The hyperproliferative disorder may be cancer. The hyperproliferative disorder may be a solid tumor cancer. The solid tumor cancer may be selected from the group consisting of glioblastoma (GBM), gastrointestinal cancer, melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), renal cancer, and renal cell carcinoma. The hyperproliferative disorder may be a hematological malignancy. The solid tumor cancer may be selected from the group consisting of chronic lymphocytic leukemia, acute lymphoblastic leukemia, diffuse large B cell lymphoma, non-Hodgkin’s lymphoma, Hodgkin’s lymphoma, follicular lymphoma, and mantle cell lymphoma.

The cancer may be a hypermutated cancer phenotype. Hypermutated cancers are extensively described in Campbell, et al. (Cell, 171:1042-1056 (2017)). A hypermutated tumors may comprise between 9 and 10 mutations per megabase (Mb). Pediatric hypermutated tumors may comprise 9.91 mutations per megabase (Mb). Adult hypermutated tumors may comprise 9 mutations per megabase (Mb). Enhanced hypermutated tumors may comprise between 10 and 100 mutations per megabase (Mb). Enhanced pediatric hypermutated tumors may comprise between 10 and 100 mutations per megabase (Mb). Enhanced adult hypermutated tumors may comprise between 10 and 100 mutations per megabase (Mb). An ultra-hypermutated tumors may comprise greater than 100 mutations per megabase (Mb). Pediatric ultra-hypermutated tumors may comprise greater than 100 mutations per megabase (Mb). Adult ultra-hypermutated tumors may comprise greater than 100 mutations per megabase (Mb).

The hypermutated tumors may have mutations in replication repair pathways. The hypermutated tumors may have mutations in replication repair associated DNA polymerases. The hypermutated tumors may have microsatellite instability. The ultra-hypermutated tumors may have mutations in replication repair associated DNA polymerases and have microsatellite instability. Hypermutation in the tumor may be correlated with response to immune checkpoint inhibitors. Hypermutated tumors may be resistant to treatment with immune checkpoint inhibitors. Hypermutated tumors can be treated using the TILs of the present invention. Hypermutation in the tumor is caused by environmental factors (extrinsic exposures). For example, UV light can be the primary cause of the high numbers of mutations in malignant melanoma (*see,* for example, Pfeifer, G.P., You, Y.H., and Besaratinia, A. (2005). Mutat. Res. 571, 19-31.; Sage, E. (1993). Photochem. Photobiol. 57, 163-174.). Hypermutation in the tumor can be caused by the greater than 60 carcinogens in tobacco smoke for tumors of the lung and larynx, as well as other tumors, due to direct mutagen exposure (*see,* for example, Pleasance, E.D., Stephens, P.J., O’Meara, S., McBride, D.J., Meynert, A., Jones, D., Lin, M.L., Beare, D., Lau, K.W., Greenman, C., et al. (2010). Nature 463, 184-190). Hypermutation in the tumor may be caused by dysregulation of apolipoprotein B mRNA editing enzyme, catalytic polypeptide-like (APOBEC) family members, which has been shown to result in increased levels of C to T transitions in a wide range of cancers (see, for example, Roberts, S.A., Lawrence, M.S., Klimczak, L.J., Grimm, S.A., Fargo, D., Stojanov, P., Kiezun, A., Kryukov, G.V., Carter, S.L., Saksena, G., et al. (2013). Nat. Genet. 45, 970-976). Hypermutation in the tumor may be caused by defective DNA replication repair by mutations that compromise proofreading, which is performed by the major replicative enzymes Pol3 and Pold1. Hypermutation in the tumor may be caused by defects in DNA mismatch repair, which are associated with hypermutation in colorectal, endometrial, and other cancers (see, for example, Kandoth, C., Schultz, N., Cherniack, A.D., Akbani, R., Liu, Y., Shen, H., Robertson, A.G., Pashtan, I., Shen, R., Benz, C.C., et al.; (2013). Nature 497, 67-73.; Muzny, D.M., Bainbridge, M.N., Chang, K., Dinh, H.H., Drummond, J.A., Fowler, G., Kovar, C.L., Lewis, L.R., Morgan, M.B., Newsham, I.F., et al.; (2012). Nature 487, 330-337). DNA replication repair mutations may also be found in cancer predisposition syndromes, such as constitutional or biallelic mismatch repair deficiency (CMMRD), Lynch syndrome, and polymerase proofreading-associated polyposis (PPAP).

Also disclosed herein but not claimed is a method of treating a cancer with a population of TILs, wherein the cancer is a hypermutated cancer. Also disclosed herein but not claimed is a method of treating a cancer with a population of TILs, wherein the cancer is an enhanced hypermutated cancer. Also disclosed herein but not claimed is a method of treating a cancer with a population of TILs, wherein the cancer is an ultra-hypermutated cancer.

Also disclosed herein but not claimed is a method of treating a cancer with a population of TILs, wherein a patient is pre-treated with non-myeloablative chemotherapy prior to an infusion of TILs according to the present disclosure. The non-myeloablative chemotherapy may be cyclophosphamide 60 mg/kg/d for 2 days (days 27 and 26 prior to TIL infusion) and fludarabine 25 mg/$m^2$/d for 5 days (days 27 to 23 prior to TIL infusion). Also disclosed herein but not claimed, after non-myeloablative chemotherapy and TIL infusion (at day 0) according to the present disclosure, the patient receives an intravenous infusion of IL-2 intravenously at 720,000 IU/kg every 8 hours to physiologic tolerance.

Efficacy of the compounds and combinations of compounds described herein in treating, preventing and/or managing the indicated diseases or disorders can be tested using various models known in the art, which provide guidance for treatment of human disease. For example, models for determining efficacy of treatments for ovarian cancer are described, *e.g.,* in Mullany, et al., Endocrinology 2012, 153, 1585-92; and Fong, et al., J. Ovarian Res. 2009, 2, 12. Models for determining efficacy of treatments for pancreatic cancer are described in Herreros-Villanueva, et al., World J. Gastroenterol. 2012, 18, 1286-1294. Models for determining efficacy of treatments for breast cancer are described, *e.g.,* in Fantozzi, Breast Cancer Res. 2006, 8, 212. Models for determining efficacy of treatments for melanoma are described, *e.g.,* in Damsky, et al., Pigment Cell & Melanoma Res. 2010, 23, 853-859. Models for determining efficacy of treatments for lung cancer are described, *e.g.,* in Meuwissen, et al., Genes & Development, 2005, 19, 643-664. Models for determining efficacy of treatments for lung cancer are described, *e.g.,* in Kim, Clin. Exp. Otorhinolaryngol. 2009, 2, 55-60; and Sano, Head Neck Oncol. 2009, 1, 32.

In some embodiments, IFN-gamma (IFN-γ) is indicative of treatment efficacy for hyperproliferative disorder treatment. In some embodiments, IFN-γ in the blood of subjects treated with TILs is indicative of active TILs. A potency assay for IFN-γ production may be employed. IFN-γ production is another measure of cytotoxic potential. IFN-γ production can be measured by determining the levels of the cytokine IFN-γ in the blood of a subject treated with TILs prepared by the methods of the present invention, including those as described for example in Figure 85 (in particular, *e.g.,* Figure 85B). In some embodiments, the TILs obtained by the present method provide for increased IFN-γ in the blood of subjects treated with the TILs of the present method as compared subjects treated with TILs prepared using methods referred to as the Gen 3 process, as exemplified Figure 85 (in particular, *e.g.,* Figure 85B) and throughout this application. In some embodiments, an increase in IFN-γ is indicative of treatment efficacy in a patient treated with the TILs produced by the methods of the present invention. In some embodiments, IFN-γ is increased one-fold, two-fold, three-fold, four-fold, or five-fold or more as compared to an untreated patient and/or as compared to a patient treated with TILs prepared using other methods than those provide herein including, for example, methods other than those embodied in Figure 85 (in particular, *e.g.,* Figure 85B). In some embodiments, IFN-γ secretion is increased one-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-γ secretion is increased two-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-γ secretion is increased three-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-γ secretion is increased four-fold as compared to an untreated patient and/or as compared to a patient treated with TILs prepared using other methods than those provide herein including, for example, methods other than those embodied in Figure 85 (in particular, *e.g.,* Figure 85B). In some embodiments, IFN-γ secretion is increased five-fold as compared to an untreated patient and/or as compared to a patient treated with TILs prepared using other methods than those provide herein including, for example, methods other than those embodied in Figure 85 (in particular, *e.g.,* Figure 85B). IFN-γ may be measured using a Quantikine ELISA kit. IFN-γ may be measured using a Quantikine ELISA kit. IFN-γ may be measured in TILs *ex vivo* from a patient treated with the TILs produced by the methods of the present invention. IFN-γ may be measured in blood in a patient treated with the TILs produced by the methods of the present invention. IFN-γ may be measured in serum in a patient treated with the TILs produced by the methods of the present invention.

In some embodiments, the TILs prepared by the methods of the present invention, including those as described for example in Figure 85 (in particular, *e.g.,* Figure 85B), exhibit increased polyclonality as compared to TILs produced by other methods, including those not exemplified in Figure 85 (in particular, *e.g.,* Figure 85B), such as for example, methods referred to as process 1C methods. In some embodiments, significantly improved polyclonality and/or increased polyclonality is indicative of treatment efficacy and/or increased clinical efficacy for cancer treatment. In some embodiments, polyclonality refers to the T-cell repertoire diversity. In some embodiments, an increase in polyclonality can be indicative of treatment efficacy with regard to administration of the TILs produced by the methods of the present invention. In some embodiments, polyclonality is increased one-fold, two-fold, ten-fold, 100-fold, 500-fold, or 1000-fold as compared to TILs prepared using methods than those provide herein including, for example, methods other than those embodied in Figure 85 (in particular, *e.g.,* Figure 85B). In some embodiments, polyclonality is increased one-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased two-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased ten-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 100-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 500-fold as compared to an untreated patient and/or as compared to a patient treated with TILs prepared using other methods than those provide herein including, for example, methods other than those embodied in Figure 85 (in particular, *e.g.,* Figure 85B). In some embodiments, polyclonality is increased 1000-fold as compared to an untreated patient and/or as compared to a patient treated with TILs prepared using other methods than those provide herein including, for example, methods other than those embodied in Figure 85 (in particular, *e.g.,* Figure 85B).

2. **Methods of co-administration**

In some embodiments, the TILs produced as described herein, including, for example TILs derived from a method described in Steps A through F of Figure 85 (in particular, e.g., Figure 85B), can be for administration in combination with one or more immune checkpoint regulators, such as the antibodies described below. For example, antibodies that target PD-1 and which can be co-administered with the TILs of the present invention include, *e.g.,* but are not limited to nivolumab (BMS-936558, Bristol-Myers Squibb; Opdivo®), pembrolizumab (lambrolizumab, MK03475 or MK-3475, Merck; Keytruda®), humanized anti-PD-1 antibody JS001 (ShangHai JunShi), monoclonal anti-PD-1 antibody TSR-042 (Tesaro, Inc.), Pidilizumab (anti-PD-1 mAb CT-011, Medivation), anti-PD-1 monoclonal Antibody BGB-A317 (BeiGene), and/or anti-PD-1 antibody SHR-1210 (ShangHai HengRui), human monoclonal antibody REGN2810 (Regeneron), human monoclonal antibody MDX-1106 (Bristol-Myers Squibb), and/or humanized anti-PD-1 IgG4 antibody PDR001 (Novartis). In some embodiments, the PD-1 antibody is from clone: RMP1-14 (rat IgG) - BioXcell cat# BP0146. Other suitable antibodies suitable for use in co-administration methods with TILs produced according to Steps A through F as described herein are anti-PD-1 antibodies disclosed in U.S. Patent No. 8,008,449. The antibody or antigen-binding portion thereof binds specifically to PD-L1 and inhibits its interaction with PD-1, thereby increasing immune activity. Any antibodies known in the art which bind to PD-L1 and disrupt the interaction between the PD-1 and PD-L1, and stimulates an anti-tumor immune response, are suitable for use in co-administration methods with TILs produced according to Steps A through F as described herein. For example, antibodies that target PD-L1 and are in clinical trials, include BMS-936559 (Bristol-Myers Squibb) and MPDL3280A (Genentech). Other suitable antibodies that target PD-L1 are disclosed in U.S. Patent No. 7,943,743. It will be understood by one of ordinary skill that any antibody which binds to PD-1 or PD-L1, disrupts the PD-1/PD-L1 interaction, and stimulates an anti-tumor immune response, are suitable for use in co-administration methods with TILs produced according to Steps A through F as described herein. Also disclosed herein but not claimed, the subject administered the combination of TILs produced according to Steps A through F is co administered with a and anti-PD-1 antibody when the patient has a cancer type that is refractory to administration of the anti-PD-1 antibody alone. Also disclosed herein but not claimed, the patient is administered TILs in combination with and anti-PD-1 when the patient has refractory melanoma. Also disclosed herein but not claimed, the patient is administered TILs in combination with and anti-PD-1 when the patient has non-small-cell lung carcinoma (NSCLC).

3. **Optional Lymphodepletion Preconditioning of Patients**

Also disclosed herein but not claimed is a method of treating a cancer with a population of TILs, wherein a patient is pre-treated with non-myeloablative chemotherapy prior to an infusion of TILs according to the present disclosure. Also disclosed herein but not claimed is a population of TILs for use in the treatment of cancer in a patient which has been pre-treated with non-myeloablative chemotherapy. Also disclosed herein but not claimed, the population of TILs is for administration by infusion. The non-myeloablative chemotherapy may be cyclophosphamide 60 mg/kg/d for 2 days (days 27 and 26 prior to TIL infusion) and fludarabine 25 mg/$m^2$/d for 5 days (days 27 to 23 prior to TIL infusion). Also disclosed herein but not claimed, after non-myeloablative chemotherapy and TIL infusion (at day 0) according to the present disclosure, the patient receives an intravenous infusion of IL-2 (aldesleukin, commercially available as PROLEUKIN) intravenously at 720,000 IU/kg every 8 hours to physiologic tolerance. Also disclosed herein but not claimed, the population of TILs is for use in treating cancer in combination with IL-2, wherein the IL-2 is administered after the population of TILs.

Experimental findings indicate that lymphodepletion prior to adoptive transfer of tumor-specific T lymphocytes plays a key role in enhancing treatment efficacy by eliminating regulatory T cells and competing elements of the immune system ('cytokine sinks'). Accordingly, a lymphodepletion step (sometimes also referred to as "immunosuppressive conditioning") may be utilized on the patient prior to the introduction of the TILs of the invention.

**In** general, lymphodepletion is achieved using administration of fludarabine or cyclophosphamide (the active form being referred to as mafosfamide) and combinations thereof. Such methods are described in Gassner, et al., Cancer Immunol. Immunother. 2011, 60, 75-85, Muranski, et al., Nat. Clin. Pract. Oncol., 2006, 3, 668-681, Dudley, et al., J. Clin. Oncol. 2008, 26, 5233-5239, and Dudley, et al., J. Clin. Oncol. 2005, 23, 2346-2357.

Also disclosed herein but not claimed, the fludarabine is administered at a concentration of 0.5 μg/mL -10 μg/mL fludarabine. The fludarabine may be administered at a concentration of 1 μg/mL fludarabine. The fludarabine treatment may be administered for 1 day, 2 days, 3 days, 4 days, 5 days, 6 days, or 7 days or more. Also disclosed herein but not claimed, the fludarabine is administered at a dosage of 10 mg/kg/day, 15 mg/kg/day, 20 mg/kg/day, 25 mg/kg/day, 30 mg/kg/day, 35 mg/kg/day, 40 mg/kg/day, or 45 mg/kg/day. The fludarabine treatment may be administered for 2-7 days at 35 mg/kg/day. The fludarabine treatment may be administered for 4-5 days at 35 mg/kg/day. The fludarabine treatment may be administered for 4-5 days at 25 mg/kg/day.

Also disclosed herein but not claimed, the mafosfamide, the active form of cyclophosphamide, is obtained at a concentration of 0.5 μg/mL -10 μg/mL by administration of cyclophosphamide. Mafosfamide, the active form of cyclophosphamide, may be obtained at a concentration of 1 μg/mL by administration of cyclophosphamide. Also disclosed herein but not claimed, the cyclophosphamide treatment is administered for 1 day, 2 days, 3 days, 4 days, 5 days, 6 days, or 7 days or more. Also disclosed herein but not claimed, the cyclophosphamide is administered at a dosage of 100 mg/m$^2$/day, 150 mg/m$^2$/day, 175 mg/m$^2$/day, 200 mg/m$^2$/day, 225 mg/m$^2$/day, 250 mg/m$^2$/day, 275 mg/m$^2$/day, or 300 mg/m$^2$/day. Also disclosed herein but not claimed, the cyclophosphamide is administered intravenously (*i.e.,* i.v.) Also disclosed herein but not claimed, the cyclophosphamide treatment is administered for 2-7 days at 35 mg/kg/day Also disclosed herein but not claimed, the cyclophosphamide treatment is administered for 4-5 days at 250 mg/m$^2$/day i.v. Also disclosed herein but not claimed, the cyclophosphamide treatment is administered for 4 days at 250 mg/m$^2$/day i.v.

Also disclosed herein but not claimed, lymphodepletion is performed by administering the fludarabine and the cyclophosphamide together to a patient. Fludarabine may be administered at 25 mg/m$^2$/day i.v. and cyclophosphamide may be administered at 250 mg/m$^2$/day i.v. over 4 days.

Also disclosed herein but not claimed, the lymphodepletion may be performed by administration of cyclophosphamide at a dose of 60 mg/m$^2$/day for two days followed by administration of fludarabine at a dose of 25 mg/m$^2$/day for five days.

4. **IL-2 Regimens**

Also disclosed herein but not claimed, the IL-2 regimen comprises a high-dose IL-2 regimen, wherein the high-dose IL-2 regimen comprises aldesleukin, or a biosimilar or variant thereof, administered intravenously starting on the day after administering a therapeutically effective portion of the therapeutic population of TILs, wherein the aldesleukin or a biosimilar or variant thereof is administered at a dose of 0.037 mg/kg or 0.044 mg/kg IU/kg (patient body mass) using 15-minute bolus intravenous infusions every eight hours until tolerance, for a maximum of 14 doses. Following 9 days of rest, this schedule may be repeated for another 14 doses, for a maximum of 28 doses in total.

Also disclosed herein but not claimed, the IL-2 regimen comprises a decrescendo IL-2 regimen. Decrescendo IL-2 regimens have been described in O'Day, et al., J. Clin. Oncol. 1999, 17, 2752-61 and Eton, et al., Cancer 2000, 88, 1703-9. Also disclosed herein but not claimed, a decrescendo IL-2 regimen comprises 18 $\times$ 10$^6$ IU/m$^2$ administered intravenously over 6 hours, followed by 18 $\times$ 10$^6$ IU/m$^2$ administered intravenously over 12 hours, followed by 18 $\times$ 10$^6$ IU/m$^2$ administered intravenously over 24 hrs, followed by 4.5 $\times$ 10$^6$ IU/m$^2$ administered intravenously over 72 hours. This treatment cycle may be repeated every 28 days for a maximum of four cycles. In an embodiment, a decrescendo IL-2 regimen comprises 18,000,000 IU/m$^2$ on day 1, 9,000,000 IU/m$^2$ on day 2, and 4,500,000 IU/m$^2$ on days 3 and 4.

Also disclosed herein but not claimed, the IL-2 regimen comprises administration of pegylated IL-2 every 1, 2, 4, 6, 7, 14 or 21 days at a dose of 0.10 mg/day to 50 mg/day.

5. **Adoptive Cell Transfer**

Adoptive cell transfer (ACT) is an effective form of immunotherapy and involves the transfer of immune cells with antitumor activity into cancer patients. ACT is a treatment approach that involves the identification, *in vitro,* of lymphocytes with antitumor activity, the *in vitro* expansion of these cells to large numbers and their infusion into the cancer-bearing host. Lymphocytes used for adoptive transfer can be derived from the stroma of resected tumors (tumor infiltrating lymphocytes or TILs). TILs for ACT can be prepared as described herein. In some embodiments, the TILs are prepared, for example, according to a method as described in Figure 85 (in particular, *e.g.,* Figure 85B). They can also be derived or from blood if they are genetically engineered to express antitumor T-cell receptors (TCRs) or chimeric antigen receptors (CARs), enriched with mixed lymphocyte tumor cell cultures (MLTCs), or cloned using autologous antigen presenting cells and tumor derived peptides. ACT in which the lymphocytes originate from the cancer-bearing host to be infused is termed autologous ACT. U.S. Publication No. 2011/0052530 relates to a method for performing adoptive cell therapy to promote cancer regression, primarily for treatment of patients suffering from metastatic melanoma. Also disclosed herein but not claimed, TILs can be administered as described herein. TILs can be for administration in a single dose. Such administration may be by injection, *e.g.,* intravenous injection. TILs and/or cytotoxic lymphocytes may be for administration in multiple doses. Dosing may be once, twice, three times, four times, five times, six times, or more than six times per year. Dosing may be once a month, once every two weeks, once a week, or once every other day. Administration of TILs and/or cytotoxic lymphocytes may continue as long as necessary.

6. **Additional Methods of Treatment**

Also disclosed herein but not claimed is a method for treating a subject with cancer comprising administering to the subject a therapeutically effective dosage of the therapeutic TIL population described in any of the preceding paragraphs as applicable above.

Also disclosed herein but not claimed is a method for treating a subject with cancer comprising administering to the subject a therapeutically effective dosage of the TIL composition described in any of the preceding paragraphs as applicable above

Also disclosed herein but not claimed is the method for treating a subject with cancer described in any of the preceding paragraphs as applicable above modified such that prior to administering the therapeutically effective dosage of the therapeutic TIL population and the TIL composition described in any of the preceding paragraphs as applicable above, respectively, a non-myeloablative lymphodepletion regimen has been administered to the subject.

Also disclosed herein but not claimed is the method for treating a subject with cancer described in any of the preceding paragraphs as applicable above modified such that the non-myeloablative lymphodepletion regimen comprises the steps of administration of cyclophosphamide at a dose of 60 mg/m$^2$/day for two days followed by administration of fludarabine at a dose of 25 mg/m$^2$/day for five days.

Also disclosed herein but not claimed is the method for treating a subject with cancer described in any of the preceding paragraphs as applicable above modified to further comprise the step of treating the subject with a high-dose IL-2 regimen starting on the day after administration of the TIL cells to the subject.

Also disclosed herein but not claimed is the method for treating a subject with cancer described in any of the preceding paragraphs as applicable above modified such that the the high-dose IL-2 regimen comprises 600,000 or 720,000 IU/kg administered as a 15-minute bolus intravenous infusion every eight hours until tolerance.

Also disclosed herein but not claimed is the method for treating a subject with cancer described in any of the preceding paragraphs as applicable above modified such that the cancer is a solid tumor.

Also disclosed herein but not claimed is the method for treating a subject with cancer described in any of the preceding paragraphs as applicable above modified such that the cancer is melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), glioblastoma (including GBM), gastrointestinal cancer, renal cancer, or renal cell carcinoma.

Also disclosed herein but not claimed is the method for treating a subject with cancer described in any of the preceding paragraphs as applicable above modified such that the cancer is melanoma, HNSCC, cervical cancers, NSCLC, glioblastoma (including GBM), and gastrointestinal cancer.

Also disclosed herein but not claimed is the method for treating a subject with cancer described in any of the preceding paragraphs as applicable above modified such that the cancer is melanoma.

Also disclosed herein but not claimed is the method for treating a subject with cancer described in any of the preceding paragraphs as applicable above modified such that the cancer is HNSCC.

Also disclosed herein but not claimed is the method for treating a subject with cancer described in any of the preceding paragraphs as applicable above modified such that the cancer is a cervical cancer.

Also disclosed herein but not claimed is the method for treating a subject with cancer described in any of the preceding paragraphs as applicable above modified such that the cancer is NSCLC.

Also disclosed herein but not claimed is the method for treating a subject with cancer described in any of the preceding paragraphs as applicable above modified such that the cancer is glioblastoma (including GBM).

Also disclosed herein but not claimed is the method for treating a subject with cancer described in any of the preceding paragraphs as applicable above modified such that the cancer is gastrointestinal cancer.

Also disclosed herein but not claimed is the method for treating a subject with cancer described in any of the preceding paragraphs as applicable above modified such that the cancer is a hypermutated cancer.

Also disclosed herein but not claimed is the method for treating a subject with cancer described in any of the preceding paragraphs as applicable above modified such that the cancer is a pediatric hypermutated cancer.

Also disclosed herein is the therapeutic TIL population described in any of the preceding paragraphs as applicable above for use in a method for treating a subject with cancer comprising administering to the subject a therapeutically effective dosage of the therapeutic TIL population.

Also disclosed herein is the TIL composition described in any of the preceding paragraphs as applicable above for use in a method for treating a subject with cancer comprising administering to the subject a therapeutically effective dosage of the TIL composition.

Also disclosed herein is the therapeutic TIL population described in any of the preceding paragraphs as applicable above or the TIL composition described in any of the preceding paragraphs as applicable above modified such that prior to administering to the subject the therapeutically effective dosage of the therapeutic TIL population described in any of the preceding paragraphs as applicable above or the TIL composition described in any of the preceding paragraphs as applicable above, a non-myeloablative lymphodepletion regimen has been administered to the subject.

Also disclosed herein the therapeutic TIL population or the TIL composition described in any of the preceding paragraphs as applicable above modified such that the non-myeloablative lymphodepletion regimen comprises the steps of administration of cyclophosphamide at a dose of 60 $mg/m^2$/day for two days followed by administration of fludarabine at a dose of 25 $mg/m^2$/day for five days.

Also disclosed herein is the therapeutic TIL population or the TIL composition described in any of the preceding paragraphs as applicable above modified to further comprise the step of treating patient with a high-dose IL-2 regimen starting on the day after administration of the TIL cells to the patient.

Also disclosed herein is the therapeutic TIL population or the TIL composition described in any of the preceding paragraphs as applicable above modified such that the high-dose IL-2 regimen comprises 600,000 or 720,000 IU/kg administered as a 15-minute bolus intravenous infusion every eight hours until tolerance.

**Also** disclosed herein is the therapeutic TIL population or the TIL composition described in any of the preceding paragraphs as applicable above modified such that the cancer is a solid tumor.

Also disclosed herein is the therapeutic TIL population or the TIL composition described in any of the preceding paragraphs as applicable above modified such that the cancer is melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), glioblastoma (including GBM), gastrointestinal cancer, renal cancer, or renal cell carcinoma.

**Also** disclosed herein is the therapeutic TIL population or the TIL composition described in any of the preceding paragraphs as applicable above modified such that the cancer is melanoma, HNSCC, cervical cancers, NSCLC, glioblastoma (including GBM), and gastrointestinal cancer.

**Also** disclosed herein is the therapeutic TIL population or the TIL composition described in any of the preceding paragraphs as applicable above modified such that the cancer is melanoma.

**Also** disclosed herein is the therapeutic TIL population or the TIL composition described in any of the preceding paragraphs as applicable above modified such that the cancer is HNSCC.

**Also** disclosed herein is the therapeutic TIL population or the TIL composition described in any of the preceding paragraphs as applicable above modified such that the cancer is a cervical cancer.

Also disclosed herein is the therapeutic TIL population or the TIL composition described in any of the preceding paragraphs as applicable above modified such that the cancer is NSCLC.

Also disclosed herein is the therapeutic TIL population or the TIL composition described in any of the preceding paragraphs as applicable above modified such that the cancer is glioblastoma (including GBM).

**Also** disclosed herein is the therapeutic TIL population or the TIL composition described in any of the preceding paragraphs as applicable above modified such that the cancer is gastrointestinal cancer.

**Also** disclosed herein is the therapeutic TIL population or the TIL composition described in any of the preceding paragraphs as applicable above modified such that the cancer is a hypermutated cancer.

**Also** disclosed herein is the therapeutic TIL population or the TIL composition described in any of the preceding paragraphs as applicable above modified such that the cancer is a pediatric hypermutated cancer.

Also disclosed herein but not claimed is the use of the therapeutic TIL population described in any of the preceding paragraphs as applicable above in a method of treating cancer in a subject comprising administering to the subject a therapeutically effective dosage of the therapeutic TIL population.

Also disclosed herein but not claimed is the use of the TIL composition described in any of the preceding paragraphs as applicable above in a method of treating cancer in a subject comprising administering to the subject a therapeutically effective dosage of the TIL composition.

Also disclosed herein but not claimed is the use of the therapeutic TIL population described in any of the preceding paragraphs as applicable above or the TIL composition described in any of the preceding paragraphs as applicable above in a method of treating cancer in a subject comprising administering to the subject a non-myeloablative lymphodepletion regimen and then administering to the subject a therapeutically effective dosage of the therapeutic TIL population described in any of the preceding paragraphs as applicable above or the therapeutically effective dosage of the TIL composition described in any of the preceding paragraphs as applicable above.

Also disclosed herein but not claimed is the use of the therapeutic TIL population or the TIL composition described in any of the preceding paragraphs as applicable above modified such that the non-myeloablative lymphodepletion regimen comprises the steps of administration of cyclophosphamide at a dose of 60 $mg/m^2$/day for two days followed by administration of fludarabine at a dose of 25 $mg/m^2$/day for five days.

Also disclosed herein but not claimed is the use of the therapeutic TIL population described in any of the preceding paragraphs as applicable above or the use of the TIL composition described in any of the preceding paragraphs as applicable above modified to further comprise the step of treating patient with a high-dose IL-2 regimen starting on the day after administration of the TIL cells to the patient.

Also disclosed herein but not claimed is the use of the therapeutic TIL population or the TIL composition described in any of the preceding paragraphs as applicable above modified such that the high-dose IL-2 regimen comprises 600,000 or 720,000 IU/kg administered as a 15-minute bolus intravenous infusion every eight hours until tolerance.

Also disclosed herein but not claimed is the use of the therapeutic TIL population or the TIL composition described in any of the preceding paragraphs as applicable above modified such that the cancer is a solid tumor.

Also disclosed herein but not claimed is the use of the therapeutic TIL population or the TIL composition described in any of the preceding paragraphs as applicable above modified such that the cancer is melanoma, ovarian cancer, cervical cancer, non-small-cell lung cancer (NSCLC), lung cancer, bladder cancer, breast cancer, cancer caused by human papilloma virus, head and neck cancer (including head and neck squamous cell carcinoma (HNSCC)), glioblastoma (including GBM), gastrointestinal cancer, renal cancer, or renal cell carcinoma.

Also disclosed herein but not claimed is the use of the therapeutic TIL population or the TIL composition described in any of the preceding paragraphs as applicable above modified such that the cancer is melanoma, HNSCC, cervical cancers, NSCLC, glioblastoma (including GBM), and gastrointestinal cancer.

Also disclosed herein but not claimed is the use of the therapeutic TIL population or the TIL composition described in any of the preceding paragraphs as applicable above modified such that the cancer is melanoma.

Also disclosed herein but not claimed is the use of the therapeutic TIL population or the TIL composition described in any of the preceding paragraphs as applicable above modified such that the cancer is HNSCC.

Also disclosed herein but not claimed is the use of the therapeutic TIL population or the TIL composition described in any of the preceding paragraphs as applicable above modified such that the cancer is a cervical cancer.

Also disclosed herein but not claimed is the use of the therapeutic TIL population or the TIL composition described in any of the preceding paragraphs as applicable above modified such that the cancer is NSCLC.

Also disclosed herein but not claimed is the use of the therapeutic TIL population or the TIL composition described in any of the preceding paragraphs as applicable above modified such that the cancer is glioblastoma (including GBM).

Also disclosed herein but not claimed is use of the therapeutic TIL population or the TIL composition described in any of the preceding paragraphs as applicable above modified such that the cancer is gastrointestinal cancer.

Also disclosed herein but not claimed is the use of the therapeutic TIL population or the TIL composition described in any of the preceding paragraphs as applicable above modified such that the cancer is a hypermutated cancer.

Also disclosed herein but not claimed is the use of the therapeutic TIL population or the TIL composition described in any of the preceding paragraphs as applicable above modified such that the cancer is a pediatric hypermutated cancer.

7. Exemplary Treatment Embodiments

Also disclosed herein but not claimed is a method of treating a cancer with a population of tumor infiltrating lymphocytes (TILs) comprising the steps of (a) obtaining a first population of TILs from a tumor fragment or core obtained from a patient; (b) performing an initial expansion of the first population of TILs in a first cell culture medium to obtain a second population of TILs, wherein the second population of TILs is at least 5-fold greater in number than the first population of TILs, and wherein the first cell culture medium comprises IL-2; (c) performing a rapid expansion of the second population of TILs using a population of myeloid artificial antigen presenting cells (myeloid aAPCs) in a second cell culture medium to obtain a third population of TILs, wherein the third population of TILs is at least 50-fold greater in number than the second population of TILs after 7 days from the start of the rapid expansion; and wherein the second cell culture medium comprises IL-2 and OKT-3; (d) administering a therapeutically effective portion of the third population of TILs to a patient with the cancer. The IL-2 may be present at an initial concentration of about 3000 IU/mL and OKT-3 antibody is present at an initial concentration of about 30 ng/mL in the second cell culture medium. First expansion may be performed over a period not greater than 14 days. The first expansion may be performed using a gas permeable container. The second expansion may be performed using a gas permeable container. The ratio of the second population of TILs to the population of aAPCs in the rapid expansion may be between 1 to 80 and 1 to 400. The ratio of the second population of TILs to the population of aAPCs in the rapid expansion may be about 1 to 300. The cancer for treatment may be selected from the group consisting of melanoma, ovarian cancer, cervical cancer, non-small-cell lung cancer (NSCLC), lung cancer, bladder cancer, breast cancer, cancer caused by human papilloma virus, head and neck cancer, renal cancer, and renal cell carcinoma. The cancer for treatment may be selected from the group consisting of melanoma, ovarian cancer, and cervical cancer. The cancer for treatment may be melanoma. The cancer for treatment may be ovarian cancer. The cancer for treatment may be cervical cancer. Also disclosed herein but not claimed, the method of treating cancer further comprises the step of treating the patient with a non-myeloablative lymphodepletion regimen prior to administering the third population of TILs to the patient. The non-myeloablative lymphodepletion regimen may comprise the steps of administration of cyclophosphamide at a dose of 60 mg/m$^2$/day for two days followed by administration of fludarabine at a dose of 25 mg/m$^2$/day for five days. The high dose IL-2 regimen may comprise 600,000 or 720,000 IU/kg of aldesleukin, or a biosimilar or variant thereof, administered as a 15-minute bolus intravenous infusion every eight hours until tolerance.

Also disclosed herein but not claimed is a method of treating a subject with cancer comprising administering expanded tumor infiltrating lymphocytes (TILs) comprising: (i) obtaining a first population of TILs from a fine needle aspirate (FNA) or a small biopsy obtained from a tumor in a patient; (ii) performing a first expansion by culturing the first population of TILs in a cell culture medium comprising IL-2 to produce a second population of TILs, wherein the cell culture medium is supplemented with OKT-3 at day 3, wherein the first expansion is performed for about 3 days to about 19 days in order to obtain the second population of TILs, wherein the second population of TILs comprises at least 5 $\times$ 10$^7$ TILs by about 10 days to about 19 days when the first population of TILs is from a small biopsy; (iii) performing a second expansion by supplementing the cell culture medium of the second population of TILs with additional IL-2, OKT-3, and antigen presenting cells (APCs), to produce a third population of TILs, wherein the third population of TILs is at least 25-fold greater in number than the second population of TILs, and wherein the second expansion is performed for about 11 days to about 14 days in order to obtain the third population of TILs, wherein the third population of TILs is a therapeutic population of TILs; and (iv) administering a therapeutically effective dosage of the third population of TILs to the patient. The IL-2 may be present at an initial concentration of about 3000 IU/mL and OKT-3 antibody is present at an initial concentration of about 30 ng/mL in the second cell culture medium. The first expansion may be performed using a gas permeable container. The second expansion may be performed using a gas permeable container. The ratio of the second population of TILs to the population of APCs in the rapid expansion may be between 1 to 80 and 1 to 400. The ratio of the second population of TILs to the population of APCs in the rapid expansion may be about 1 to 300. The APCs may be PMBCs. The small biopsy may be obtained from a tumor selected from the group consisting of pancreatic, melanoma, breast, and ovarian. The cancer to be treated may be selected from the group consisting of pancreatic, melanoma, breast, and ovarian. The FNA may be obtained from a tumor selected from the group consisting of lung, melanoma, head and neck, cervical, ovarian, pancreatic, glioblastoma, colorectal, and sarcoma. The cancer to be treated may be selected from the group consisting of lung, melanoma, head and neck, cervical, ovarian, pancreatic, glioblastoma, colorectal, and sarcoma. Also disclosed herein but not claimed, the method of treating cancer further comprises the step of treating the patient with a non-myeloablative lymphodepletion regimen prior to administering the third population of TILs to the patient. The non-myeloablative lymphodepletion regimen may comprise the steps of administration of cyclophosphamide at a dose of 60 mg/m$^2$/day for two days followed by administration of fludarabine at a dose of 25 mg/m$^2$/day for five days. The high dose IL-2 regimen may comprise 600,000 or 720,000 IU/kg of aldesleukin, or a biosimilar or variant thereof, administered as a 15-minute bolus intravenous infusion every eight hours until tolerance.

## EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### EXAMPLE 1: PREPARATION OF MEDIA FOR PRE-REP AND REP PROCESSES

This Example describes the procedure for the preparation of tissue culture media for use in protocols involving the culture of tumor infiltrating lymphocytes (TIL) derived from various tumor types including, but not limited to, metastatic melanoma, head and neck squamous cell carcinoma (HNSCC), ovarian carcinoma, triple-negative breast carcinoma, and lung adenocarcinoma. This media can be used for preparation of any of the TILs described in the present application and Examples.

## Preparation of CM1

Removed the following reagents from cold storage and warmed them in a 37°C water bath: (RPMI1640, Human AB serum, 200mM L-glutamine). Prepared CM1 medium according to Table 32 below by adding each of the ingredients into the top section of a 0.2 μm filter unit appropriate to the volume to be filtered. Store at 4°C.

**TABLE 32: Preparation of CM1**

| Ingredient | Final concentration | Final Volume 500 ml | Final Volume IL |
|---|---|---|---|
| RPMI1640 | NA | 450 mL | 900 mL |
| Human AB serum, heat-inactivated 10% | 50 mL | 100 mL | |
| 200 mM L-glutamine | 2mM | 5 mL | 10 mL |
| 55 mM BME | 55 μM | 0.5 mL | 1 mL |
| 50 mg/mL gentamicin sulfate | 50 μg/mL | 0.5 mL | 1 mL |

On the day of use, prewarmed required amount of CM1 in 37°C water bath and add 6000 IU/mL IL-2.
Additional supplementation - as needed according to Table 33.

**TABLE 33: Additional supplementation of CM1, as needed.**

| **Supplement** | **Stock concentration** | **Dilution** | **Final concentration** |
|---|---|---|---|
| GlutaMAX™ | 200mM | 1:100 | 2mM |
| Penicillin/streptomycin | 10,000 U/mL penicillin<br>10,000 μg/mL streptomycin | 1:100 | 100 U/mL penicillin<br>100 μg/mL streptomycin |
| Amphotericin B | 250 μg/mL | 1:100 | 2.5 μg/mL |

## Preparation of CM2

Removed prepared CM1 from refrigerator or prepare fresh CM1 as per Section 7.3 above. Removed AIM-V® from refrigerator and prepared the amount of CM2 needed by mixing prepared CM1 with an equal volume of AIM-V® in a sterile media bottle. Added 3000 IU/mL IL-2 to CM2 medium on the day of usage. Made sufficient amount of CM2 with 3000 IU/mL IL-2 on the day of usage. Labeled the CM2 media bottle with its name, the initials of the preparer, the date it was filtered/prepared, the two-week expiration date and store at 4°C until needed for tissue culture.

## Preparation of CM3

Prepared CM3 on the day it was required for use. CM3 was the same as AIM-V® medium, supplemented with 3000 IU/ml IL-2 on the day of use. Prepared an amount of CM3 sufficient to experimental needs by adding IL-2 stock solution directly to the bottle or bag of AIM-V. Mixed well by gentle shaking. Label bottle with "3000 IU/mL IL-2" immediately after adding to the AIM-V. If there was excess CM3, stored it in bottles at 4°C labeled with the media name, the initials of the preparer, the date the media was prepared, and its expiration date (7 days after preparation). Discarded media supplemented with IL-2 after 7 days storage at 4°C.

## Preparation of CM4

CM4 was the same as CM3, with the additional supplement of 2mM GlutaMAX™ (final concentration). For every 1 L of CM3, added 10 m1 of 200 mM GlutaMAX™. Prepared an amount of CM4 sufficient to experimental needs by adding IL-2 stock solution and GlutaMAX™ stock solution directly to the bottle or bag of AIM-V. Mixed well by gentle shaking. Labeled bottle with "3000 IL/ml IL-2 and GlutaMAX" immediately after adding to the AIM-V. If there was excess CM4, stored it in bottles at 4°C labeled with the media name, "GlutaMAX", and its expiration date (7 days after preparation). Discarded media supplemented with IL-2 after 7-days storage at 4°C.

**EXAMPLE 2: USE OF IL-2, IL-15, AND IL-21 CYTOKINE COCKTAIL**

This example describes the use of IL-2, IL-15, and IL-21 cytokines, which serve as additional T cell growth factors, in combination with the TIL process of the Examples.

Using the processes described herein, TILs were grown from colorectal, melanoma, cervical, triple negative breast, lung and renal tumors in presence of IL-2 in one arm of the experiment and, in place of IL-2, a combination of IL-2, IL-15, and IL-21 in another arm at the initiation of culture. At the completion of the pre-REP, cultures were assessed for expansion, phenotype, function (CD107a+ and IFNγ) and TCR Vβ repertoire. IL-15 and IL-21 are described elsewhere herein and in Gruijl, et al., IL-21 promotes the expansion of CD27+CD28+ tumor infiltrating lymphocytes with high cytotoxic potential and low collateral expansion of regulatory T cells, Santegoets, S. J., J Transl Med., 2013, 11:37 (https://www.ncbi.nlm.nih.gov/pmc/articles/PMC3626797/).

The results showed that enhanced TIL expansion (>20%), in both $CD4^+$ and $CD8^+$ cells in the IL-2, IL-15, and IL-21 treated conditions were observed in multiple histologies relative to the IL-2 only conditions. There was a skewing towards a predominantly $CD8^+$ population with a skewed TCR Vβ repertoire in the TILs obtained from the IL-2, IL-15, and IL-21 treated cultures relative to the IL-2 only cultures. IFNγ and CD107a were elevated in the IL-2, IL-15, and IL-21 treated TILs, in comparison to TILs treated only IL-2.

**EXAMPLE 3: QUALIFYING INDIVIDUAL LOTS OF GAMMA-IRRADIATED PERIPHERAL MONONUCLEAR CELLS**

This Example describes a novel abbreviated procedure for qualifying individual lots of gamma-irradiated peripheral mononuclear cells (PBMCs, also known as MNC) for use as allogeneic feeder cells in the exemplary methods described herein.

Each irradiated MNC feeder lot was prepared from an individual donor. Each lot or donor was screened individually for its ability to expand TIL in the REP in the presence of purified anti-CD3 (clone OKT3) antibody and interleukin-2 (IL-2). In addition, each lot of feeder cells was tested without the addition of TIL.to verify that the received dose of gamma radiation was sufficient to render them replication incompetent.

**Background**

Gamma-irradiated, growth-arrested MNC feeder cells were required for REP of TIL. Membrane receptors on the feeder MNCs bind to anti-CD3 (clone OKT3) antibody and crosslink to TIL in the REP flask, stimulating the TIL to expand. Feeder lots were prepared from the leukapheresis of whole blood taken from individual donors. The leukapheresis product was subjected to centrifugation over Ficoll-Hypaque, washed, irradiated, and cryopreserved under GMP conditions.

It is important that patients who received TIL therapy not be infused with viable feeder cells as this can result in Graft-Versus-Host Disease (GVHD). Feeder cells are therefore growth-arrested by dosing the cells with gamma-irradiation, resulting in double strand DNA breaks and the loss of cell viability of the MNC cells upon reculture.

**Evaluation Criteria and Experimental Set-Up**

Feeder lots were evaluated on two criteria: 1) their ability to expand TIL in co-culture >100-fold and 2) their replication incompetency.

Feeder lots were tested in mini-REP format utilizing two primary pre-REP TIL lines grown in upright T25 tissue culture flasks. Feeder lots were tested against two distinct TIL lines, as each TIL line is unique in its ability to proliferate in response to activation in a REP. As a control, a lot of irradiated MNC feeder cells which has historically been shown to meet the criteria above was run alongside the test lots.

To ensure that all lots tested in a single experiment receive equivalent testing, sufficient stocks of the same pre-REP TIL lines were available to test all conditions and all feeder lots.

For each lot of feeder cells tested, there was a total of six T25 flasks: Pre-REP TIL line #1 (2 flasks); Pre-REP TIL line #2 (2 flasks); and Feeder control (2 flasks). Flasks containing TIL lines #1 and #2 evaluated the ability of the feeder lot to expand TIL. The feeder control flasks evaluated the replication incompetence of the feeder lot.

**EXPERIMENTAL PROTOCOL**

**Day -2/3, Thaw of TIL lines**

Prepared CM2 medium. Warmed CM2 in 37°C water bath. Prepared 40 mL of CM2 supplemented with 3000 IU/ml IL-2. Keep warm until use. Placed 20 mL of pre-warmed CM2 without IL-2 into each of two 50 mL conical tubes labeled with names of the TIL lines used. Removed the two designated pre-REP TIL lines from $LN_2$ storage and transferred the vials to the tissue culture room. Thawed vials by placing them inside a sealed zipper storage bag in a 37°C water bath until a small amount of ice remains.

Using a sterile transfer pipet, immediately transferred the contents of vial into the 20 mL of CM2 in the prepared, labeled 50 mL conical tube. QS to 40 mL using CM2 without IL-2 to wash cells. Centrifuged at 400 x CF for 5 minutes. Aspirated the supernatant and resuspend in 5 mL warm CM2 supplemented with 3000 IU/mL IL-2.

Removed small aliquot (20 μL) in duplicate for cell counting using an automated cell counter. Record the counts. While counting, placed the 50 mL conical tube with TIL cells into a humidified 37°C, 5% $CO_2$ incubator, with the cap loosened to allow for gas exchange. Determined cell concentration and diluted TIL to $1 \times 10^6$ cells/mL in CM2 supplemented with IL-2 at 3000 IU/mL.

Cultured in 2 mL/well of a 24-well tissue culture plate in as many wells as needed in a humidified 37°C incubator until Day 0 of the mini-REP. Cultured the different TIL lines in separate 24-well tissue culture plates to avoid confusion and potential cross-contamination.

**Day 0, initiate Mini-REP**

Prepared enough CM2 medium for the number of feeder lots to be tested. *(e.g.,* for testing 4 feeder lots at one time, prepared 800 mL of CM2 medium). Aliquoted a portion of the CM2 prepared above and supplemented it with 3000 IU/mL IL-2 for the culturing of the cells. *(e.g.,* for testing 4 feeder lots at one time, prepare 500 mL of CM2 medium with 3000 IU/mL IL-2).

Working with each TIL line separately to prevent cross-contamination, removed the 24-well plate with TIL culture from the incubator and transferred to the BSC.

Using a sterile transfer pipet or 100-1000 μL Pipettor and tip, removed about 1 mL of medium from each well of TIL to be used and place in an unused well of the 24-well tissue culture plate.

Using a fresh sterile transfer pipet or 100-1000 μL Pipettor and tip, mixed remaining medium with TIL in wells to resuspend the cells and then transferred the cell suspension to a 50 mL conical tube labeled with the TIL name and recorded the volume.

Washed the wells with the reserved media and transferred that volume to the same 50 mL conical tube. Spun the cells at 400 x CF to collect the cell pellet. Aspirated off the media supernatant and resuspend the cell pellet in 2-5 mL of CM2 medium containing 3000 IU/mL IL-2, volume to be used based on the number of wells harvested and the size of the pellet - volume should be sufficient to ensure a concentration of $>1.3 \times 10^6$ cells/mL.

Using a serological pipet, mixed the cell suspension thoroughly and recorded the volume. Removed 200 μL for a cell count using an automated cell counter. While counting, placed the 50 mL conical tube with TIL cells into a humidified, 5% $CO_2$, 37°C incubator, with the cap loosened to allow gas exchange. Recorded the counts.

Removed the 50 ml conical tube containing the TIL cells from the incubator and resuspend them cells at a concentration of $1.3 \times 10^6$ cells/mL in warm CM2 supplemented with 3000 IU/mL IL-2. Returned the 50 mL conical tube to the incubator with a loosened cap.

Repeated steps above for the second TIL line.

Just prior to plating the TIL into the T25 flasks for the experiment, TIL were diluted 1: 10 for a final concentration of $1.3 \times 10^5$ cells/mL as per below.

**Prepare MACS GMP CD3 pure (OKT3) working solution**

Took out stock solution of OKT3 (1 mg/mL) from 4°C refrigerator and placed in BSC. A final concentration of 30 ng/mL OKT3 was used in the media of the mini-REP.

600 ng of OKT3 were needed for 20 ml in each T25 flask of the experiment; this was the equivalent of 60 μL of a 10 μg/mL solution for each 20 mL, or 360 μL for all 6 flasks tested for each feeder lot.

For each feeder lot tested, made 400 μL of a 1:100 dilution of 1 mg/mL OKT3 for a working concentration of 10 μg/mL *(e.g.,* for testing 4 feeder lots at one time, make 1600 μL of a 1:100 dilution of 1 mg/mL OKT3: 16 μL of 1 mg/mL OKT3 + 1.584 mL of CM2 medium with 3000 IU/mL IL-2.)

**Prepare T25 flasks**

Labeled each flask and filled flask with the CM2 medium prior to preparing the feeder cells. Placed flasks into 37°C humidified 5% $CO_2$ incubator to keep media warm while waiting to add the remaining components. Once feeder cells were prepared, the components will be added to the CM2 in each flask.

TABLE 34: Solutions

| Component | Volume in co-culture flasks | Volume in control (feeder only) flasks |
|---|---|---|
| CM2 + 3000 IU/ml IL-2 | 18ml | 19ml |
| MNC: 1.3 × $10^7$/ml in CM2 + 3000IU IL-2 (final concentration 1.3 × $10^7$/flask) | 1ml | 1ml |
| OKT3: 10μg/ml in CM2 + 3000IU IL-2 | 60μl | 60μl |
| TIL: 1.3 × $10^5$/ml in CM2 with 3000IU of IL-2 (final concentration 1.3 × $10^5$/flask) | 1ml | 0 |

**Prepare Feeder Cells**

**A** minimum of 78 × $10^6$ feeder cells were needed per lot tested for this protocol. Each 1 ml vial frozen by SDBB had 100 × $10^6$ viable cells upon freezing. Assuming a 50% recovery upon thaw from $LN_2$ storage, it was recommended to thaw at least two 1 mL vials of feeder cells per lot giving an estimated 100 × $10^6$ viable cells for each REP. Alternately, if supplied in 1.8 ml vials, only one vial provided enough feeder cells.

Before thawing feeder cells, pre-warmed approximately 50 mL of CM2 without IL-2 for each feeder lot to be tested. Removed the designated feeder lot vials from $LN_2$ storage, placed in zipper storage bag, and place on ice. Thawed vials inside closed zipper storage bag by immersing in a 37°C water bath. Removed vials from zipper bag, spray or wipe with 70% EtOH and transferred vials to BSC.

Using a transfer pipet immediately transferred the contents of feeder vials into 30 mL of warm CM2 in a 50 mL conical tube. Washed vial with a small volume of CM2 to remove any residual cells in the vial. Centrifuged at 400 x CF for 5 minutes. Aspirated the supernatant and resuspended in 4 mL warm CM2 plus 3000 IU/mL IL-2. Removed 200 μL for cell counting using the Automated Cell Counter. Recorded the counts.

Resuspended cells at 1.3 × $10^7$ cells/mL in warm CM2 plus 3000 IU/mL IL-2. Diluted TIL cells from 1.3 × $10^6$ cells/mL to 1.3 × $10^5$ cells/mL.

**Setup Co-Culture**

Diluted TIL cells from 1.3 × $10^6$ cells/mL to 1.3 × $10^5$ cells/mL. Added 4.5 ml of CM2 medium to a 15 mL conical tube. Removed TIL cells from incubator and resuspended well using a 10 mL serological pipet. Removed 0.5 mL of cells from the 1.3 × $10^6$ cells/mL TIL suspension and added to the 4.5 mL of medium in the 15 mL conical tube. Returned TIL stock vial to incubator. Mixed well. Repeated for the second TIL line.

Transferred flasks with pre-warmed media for a single feeder lot from the incubator to the BSC. Mixed feeder cells by pipetting up and down several times with a 1 mL pipet tip and transferred 1 mL (1.3 × $10^7$ cells) to each flask for that feeder lot. Added 60 μL of OKT3 working stock (10 μg/mL) to each flask. Returned the two control flasks to the incubator.

Transferred 1 mL (1.3 × $10^5$) of each TIL lot to the correspondingly labeled T25 flask. Returned flasks to the incubator and incubate upright. Did not disturb until Day 5.

Repeated for all feeder lots tested.

**Day 5, Media change**

Prepared CM2 with 3000 IU/mL IL-2. 10 ml is needed for each flask. With a 10 mL pipette, transferred 10 mL warm CM2 with 3000 IU/mL IL-2 to each flask. Returned flasks to the incubator and incubated upright until Day 7. Repeated for all feeder lots tested.

**Day 7, Harvest**

Removed flasks from the incubator and transfer to the BSC, care as taken not to disturb the cell layer on the bottom of the flask. Without disturbing the cells growing on the bottom of the flasks, removed 10 ml of medium from each test flask and 15 ml of medium from each of the control flasks.

Using a 10 mL serological pipet, resuspended the cells in the remaining medium and mix well to break up any clumps of cells. After thoroughly mixing cell suspension by pipetting, removed 200 μL for cell counting. Counted the TIL using the appropriate standard operating procedure in conjunction with the automatic cell counter equipment. Recorded counts in Day 7.

Repeated for all feeder lots tested.

Feeder control flasks were evaluated for replication incompetence and flasks containing TIL were evaluated for fold expansion from Day 0 according to Table TT below.

**Day 7, Continuation of Feeder Control Flasks to Day 14**

After completing the Day 7 counts of the feeder control flasks, added 15 mL of fresh CM2 medium containing 3000 IU/mL IL-2 to each of the control flasks. Returned the control flasks to the incubator and incubated in an upright position until Day 14.

**Day 14, Extended Non-proliferation of Feeder Control Flasks**

Removed flasks from the incubator and transfer to the BSC, care was taken not to disturb the cell layer on the bottom of the flask. Without disturbing the cells growing on the bottom of the flasks, removed approximately 17 ml of medium from each control flasks. Using a 5 mL serological pipet, resuspended the cells in the remaining medium and mixed well to break up any clumps of cells. Recorded the volumes for each flask.

After thoroughly mixing cell suspension by pipetting, removed 200 μl for cell counting. Counted the TIL using the appropriate standard operating procedure in conjunction with the automatic cell counter equipment. Recorded counts.

Repeated for all feeder lots tested.

**RESULTS AND ACCEPTANCE CRITERIA**

**Results**

The dose of gamma irradiation was sufficient to render the feeder cells replication incompetent. All lots were expected to meet the evaluation criteria and also demonstrated a reduction in the total viable number of feeder cells remaining on Day 7 of the REP culture compared to Day 0.

All feeder lots were expected to meet the evaluation criteria of 100-fold expansion of TIL growth by Day 7 of the REP culture.

Day 14 counts of Feeder Control flasks were expected to continue the non-proliferative trend seen on Day 7.

**Acceptance Criteria**

The following acceptance criteria were met for each replicate TIL line tested for each lot of feeder cells

Acceptance was two-fold, as follows (outlined in Table 35 below).

TABLE 35: Acceptance Criteria

| **Test** | **Acceptance criteria** |
|---|---|
| Irradiation of MNC / Replication Incompetence | No growth observed at 7 and 14 days |
| TIL expansion | At least a 100-fold expansion of each TIL (minimum of $1.3 \times 10^7$ viable cells) |

Evaluated whether the dose of radiation was sufficient to render the MNC feeder cells replication incompetent when cultured in the presence of 30 ng/mL OKT3 antibody and 3000 IU/mL IL-2. Replication incompetence was evaluated by total viable cell count (TVC) as determined by automated cell counting on Day 7 and Day 14 of the REP.

Acceptance criteria was "No Growth," meaning the total viable cell number has not increased on Day 7 and Day 14 from the initial viable cell number put into culture on Day 0 of the REP.

Evaluated the ability of the feeder cells to support TIL expansion. TIL growth was measured in terms of fold expansion of viable cells from the onset of culture on Day 0 of the REP to Day 7 of the REP. On Day 7, TIL cultures achieved a minimum of 100-fold expansion, (*i.e.,* greater than 100 times the number of total viable TIL cells put into culture on REP Day 0), as evaluated by automated cell counting.

**Contingency Testing of MNC Feeder Lots that do not meet acceptance criteria**

In the event that an MNC feeder lot did not meet the either of the acceptance criteria outlined above, the following steps will be taken to retest the lot to rule out simple experimenter error as its cause.

If there are two or more remaining satellite testing vials of the lot, then the lot was retested. If there were one or no remaining satellite testing vials of the lot, then the lot was failed according to the acceptance criteria listed above.

In order to be qualified, the lot in question and the control lot had to achieve the acceptance criteria above. Upon meeting these criteria, the lot was then released for use.

**EXAMPLE 4: QUALIFYING INDIVIDUAL LOTS OF GAMMA-IRRADIATED PERIPHERAL BLOOD MONONUCLEAR CELLS**

This Example describes a novel abbreviated procedure for qualifying individual lots of gamma-irradiated peripheral blood mononuclear cells (PBMC) for use as allogeneic feeder cells in the exemplary methods described herein. This example provides a protocol for the evaluation of irradiated PBMC cell lots for use in the production of clinical lots of TIL. Each irradiated PBMC lot was prepared from an individual donor. Over the course of more than 100 qualification protocols, it has been shown that, in all cases, irradiated PBMC lots from SDBB (San Diego Blood Bank) expand TIL >100-fold on Day 7 of a REP. This modified qualification protocol was intended to apply to irradiated donor PBMC lots from SDBB which were then further tested to verify that the received dose of gamma radiation was sufficient to render them replication incompetent. Once demonstrated that they maintained replication incompetence over the course of 14 days, donor PBMC lots were considered "qualified" for usage to produce clinical lots of TIL.

**Background**

Gamma-irradiated, growth-arrested PBMC were required for current standard REP of TIL. Membrane receptors on the PBMCs bind to anti-CD3 (clone OKT3) antibody and crosslink to TIL in culture, stimulating the TIL to expand. PBMC lots were prepared from the leukapheresis of whole blood taken from individual donors. The leukapheresis product was subjected to centrifugation over Ficoll-Hypaque, washed, irradiated, and cryopreserved under GMP conditions.

It is important that patients who received TIL therapy not be infused with viable PBMCs as this could result in Graft-Versus-Host Disease (GVHD). Donor PBMCs are therefore growth-arrested by dosing the cells with gamma-irradiation, resulting in double strand DNA breaks and the loss of cell viability of the PBMCs upon reculture.

**Evaluation Criteria**

7.2.1 Evaluation criterion for irradiated PBMC lots was their replication incompetency.

**Experimental Set-up**

Feeder lots were tested in mini-REP format as if they were to be co-cultured with TIL, using upright T25 tissue culture flasks. Control lot: One lot of irradiated PBMCs, which had historically been shown to meet the criterion of 7.2.1, was run alongside the experimental lots as a control. For each lot of irradiated donor PBMC tested, duplicate flasks were run.

**Experimental Protocol**

**Day 0**

Prepared ~90 ml of CM2 medium for each lot of donor PBMC to be tested. Kept CM2 warm in 37°C water bath. Thawed an aliquot of $6 \times 10^6$ IU/mL IL-2. Returned the CM2 medium to the BSC, wiping with 70% EtOH prior to placing in hood. For each lot of PBMC tested, removed about 60 mL of CM2 to a separate sterile bottle. Added IL-2 from the thawed $6 \times 10^6$ IU/mL stock solution to this medium for a final concentration of 3000 IU/mL. Labeled this bottle as "CM2/IL2" (or similar) to distinguish it from the unsupplemented CM2.

**Prepare OKT3**

Took out the stock solution of anti-CD3 (OKT3) from the 4°C refrigerator and placed in the BSC. A final concentration of 30 ng/mL OKT3 was used in the media of the mini-REP. Prepared a 10 μg/mL working solution of anti-CD3 (OKT3) from the 1 mg/mL stock solution. Placed in refrigerator until needed.

For each PBMC lot tested, prepare 150 μL of a 1:100 dilution of the anti-CD3 (OKT3) stock. For example, for testing 4 PBMC lots at one time, prepare 600 μL of 10 μg/mL anti-CD3 (OKT3) by adding 6 μL of the 1 mg/mL stock solution to 594 μL of CM2 supplemented with 3000 IU/mL IL-2.

**Prepare Flasks**

Added 19 mL per flask of CM2/IL-2 to the labeled T25 flasks and placed flasks into 37°C, humidified, 5% $CO_2$ incubator while preparing cells.

**Prepare Irradiate PBMC**

Retrieved vials of PBMC lots to be tested from $LN_2$ storage. These were placed at - 80°C or kept on dry ice prior to thawing. Placed 30 mL of CM2 (without IL-2 supplement) into 50 mL conical tubes for each lot to be thawed. Labeled each tube with the different lot numbers of the PBMC to be thawed. Capped tubes tightly and place in 37°C water bath prior to use. As needed, returned 50 mL conical tubes to the BSC, wiping with 70% EtOH prior to placing in the hood.

Removed a vial PBMC from cold storage and place in a floating tube rack in a 37°C water bath to thaw. Allowed thaw to proceed until a small amount of ice remains in the vial. Using a sterile transfer pipet, immediately transferred the contents of the vial into the 30 mL of CM2 in the 50 ml conical tube. Removed about 1 mL of medium from the tube to rinse the vial; returned rinse to the 50 mL conical tube. Capped tightly and swirl gently to wash cells.

Centrifuged at 400 x g for 5 min at room temperature. Aspirated the supernatant and resuspend the cell pellet in 1 mL of warm CM2/IL-2 using a 1000 μL pipet tip. Alternately, prior to adding medium, resuspended cell pellet by dragging capped tube along an empty tube rack. After resuspending the cell pellet, brought volume to 4 mL using CM2/IL-2 medium. Recorded volume.

Removed a small aliquot *(e.g.,* 100 μL) for cell counting using an automated cell counter. Performed counts in duplicate according to the particular automated cell counter SOP. It most likely was necessary to perform a dilution of the PBMC prior to performing the cell counts. A recommended starting dilution was 1: 10, but this varied depending on the type of cell counter used. Recorded the counts.

Adjusted concentration of PBMC to $1.3 \times 10^7$ cells/mL using CM2/IL-2 medium. Mixed well by gentle swirling or by gently aspirating up-and-down using a serological pipet.

**Set Up Culture Flasks**

Returned two labeled T25 flasks to the BSC from the tissue culture incubator. Returned the 10 μg/mL vial of anti-CD3/OKT3 to the BSC. Added 1 mL of the $1.3 \times 10^7$ PBMC cell suspension to each flask. Added 60 μL of the 10 μg/mL anti-CD3/OKT3 to each flask. Returned capped flasks to the tissue culture incubators for 14 days of growth without disturbance. Placed anti-CD3/OKT3 vial back into the refrigerator until needed for the next lot. Repeated for each lot of PBMC to be evaluated.

**Day 14, Measurement of Non-proliferation of PBMC**

Returned the duplicate T25 flasks to the BSC. For each flask, using a fresh 10 mL serological pipet, removed ~17 mL from each of the flasks, then carefully pulled up the remaining media to measure the volume remaining in the flasks. Recorded volume.

Mixed sample well by pipetting up and down using the same serological pipet.

Removed a 200 μL sample from each flask for counting. Counted cells using an automated cell counter. Repeated steps for each lot of PBMC being evaluated.

**RESULTS AND ACCEPTANCE CRITERION**

**Results**

The dose of gamma irradiation was expected to be sufficient to render the feeder cells replication incompetent. All lots were expected to meet the evaluation criterion, demonstrating a reduction in the total viable number of feeder cells remaining on Day 14 of the REP culture compared to Day 0.

**Acceptance Criterion**

The following acceptance criterion were met for each irradiated donor PBMC lot tested: "No growth" - meant that the total number of viable cells on Day 14 was less than the initial viable cell number put into culture on Day 0 of the REP.

**Contingency Testing of PBMC lots which do not meet acceptance criterion.**

In the event than an irradiated donor PBMC lot did not meet the acceptance criterion above, the following steps were taken to retest the lot to rule out simple experimenter error as the cause of its failure. If there were two or more remaining satellite vials of the lot, then the lot was retested. If there are one or no remaining satellite vials of the lot, then the lot was failed according to the acceptance criterion above.

To be qualified, a PBMC lot going through contingency testing had both the control lot and both replicates of the lot in question achieve the acceptance criterion. Upon meeting this criterion, the lot was then released for use.

**EXAMPLE 5: PREPARATION OF IL-2 STOCK SOLUTION (CELLGENIX)**

This Example describes the process of dissolving purified, lyophilized recombinant human interleukin-2 into stock samples suitable for use in further tissue culture protocols, including all of those described in the present application and Examples, including those that involve using rhIL-2.

**Procedure**

Prepared 0.2% Acetic Acid solution (HAc). Transferred 29 mL sterile water to a 50 mL conical tube. Added 1 mL 1 N acetic acid to the 50 mL conical tube. Mixed well by inverting tube 2-3 times. Sterilized the HAc solution by filtration using a Steriflip filter

Prepare 1% HSA in PBS. Added 4 mL of 25% HSA stock solution to 96 mL PBS in a 150 mL sterile filter unit. Filtered solution. Stored at 4°C. For each vial of rhIL-2 prepared, fill out forms.

Prepared rhIL-2 stock solution ($6 \times 10^6$ IU/mL final concentration). Each lot of rhIL-2 was different and required information found in the manufacturer's Certificate of Analysis (COA), such as: 1) Mass of rhIL-2 per vial (mg), 2) Specific activity of rhIL-2 (IU/mg) and 3) Recommended 0.2% HAc reconstitution volume (mL).

Calculated the volume of 1% HSA required for rhIL-2 lot by using the equation below:

$$\left(\frac{Vial\ Mass\ (mg)\ x\ Biological\ Activity\ (\frac{IU}{mg})}{6x10^6\ \frac{IU}{mL}}\right) - HAc\ vol\ (mL) = 1\%\ HSA\ vol\ (mL)$$

For example, according to CellGenix's rhIL-2 lot 10200121 COA, the specific activity for the 1 mg vial is $25 \times 10^6$ IU/mg. It recommends reconstituting the rhIL-2 in 2 mL 0.2% HAc.

$$\left(\frac{1mg\ x\ 25x10^6\ \frac{IU}{mg}}{6x10^6\ \frac{IU}{mL}}\right) - 2mL = 2.167mL\ HSA$$

Wiped rubber stopper of IL-2 vial with alcohol wipe. Using a 16G needle attached to a 3 mL syringe, injected recommended volume of 0.2% HAc into vial. Took care to not dislodge the stopper as the needle is withdrawn. Inverted vial 3 times and swirled until all powder is dissolved. Carefully removed the stopper and set aside on an alcohol wipe. Added the calculated volume of 1% HSA to the vial.

Storage of rhIL-2 solution. For short-term storage (<72 hrs), stored vial at 4°C. For long-term storage (>72 hrs), aliquoted vial into smaller volumes and stored in cryovials at - 20°C until ready to use. Avoided freeze/thaw cycles. Expired 6 months after date of preparation. Rh-IL-2 labels included vendor and catalog number, lot number, expiration date, operator initials, concentration and volume of aliquot.

## EXAMPLE 6: CRYOPRESERVATION PROCESS

This example describes the cryopreservation process method for TILs prepared with the abbreviated, closed procedure described in Example G using the CryoMed Controlled Rate Freezer, Model 7454 (Thermo Scientific).

The equipment used was as follows: aluminum cassette holder rack (compatible with CS750 freezer bags), cryostorage cassettes for 750 mL bags, low pressure (22 psi) liquid nitrogen tank, refrigerator, thermocouple sensor (ribbon type for bags), and CryoStore CS750 Freezing bags (OriGen Scientific).

The freezing process provides for a 0.5 °C rate from nucleation to -20 °C and 1 °C per minute cooling rate to -80 °C end temperature. The program parameters are as follows: Step 1 - wait at 4 °C; Step 2: 1.0 °C/min (sample temperature) to -4 °C; Step 3: 20.0 °C/min (chamber temperature) to -45 °C; Step 4: 10.0 °C/min (chamber temperature) to -10.0 °C; Step 5: 0.5 °C/min (chamber temperature) to -20 °C; and Step 6: 1.0 °C/min (sample temperature) to -80 °C.

## EXAMPLE 7: USE OF IL-2, IL-15, AND IL-21 CYTOKINE COCKTAIL

This example describes the use of IL-2, IL-15, and IL-21 cytokines, which serve as additional T cell growth factors, in combination with the TIL process of Examples 1 to 6.

Using the process of Examples 1 to 9, TILs were grown from colorectal, melanoma, cervical, triple negative breast, lung and renal tumors in presence of IL-2 in one arm of the experiment and, in place of IL-2, a combination of IL-2, IL-15, and IL-21 in another arm at the initiation of culture. At the completion of the pre-REP, cultures were assessed for expansion, phenotype, function (CD107a+ and IFNγ) and TCR Vβ repertoire. IL-15 and IL-21 are described elsewhere herein and in Gruijl, et al., IL-21 promotes the expansion of CD27+CD28+ tumor infiltrating lymphocytes with high cytotoxic potential and low collateral expansion of regulatory T cells, Santegoets, S. J., J Transl Med., 2013, 11:37 (https://www.ncbi.nlm.nih.gov/pmc/articles/PMC3626797/).

The results showed that enhanced TIL expansion (>20%), in both $CD4^+$ and $CD8^+$ cells in the IL-2, IL-15, and IL-21 treated conditions were observed in multiple histologies relative to the IL-2 only conditions. There was a skewing towards a predominantly $CD8^+$ population with a skewed TCR Vβ repertoire in the TILs obtained from the IL-2, IL-15, and IL-21 treated cultures relative to the IL-2 only cultures. IFNγ and CD107a were elevated in the IL-2, IL-15, and IL-21 treated TILs, in comparison to TILs treated only IL-2.

## EXAMPLE 8: TUMOR INFILTRATING LYMPHOCYTE (TIL) EXPANSION FROM FINE NEEDLE ASPIRATES (FNAS) AND CORE NEEDLE BIOPSIES

### I. Background

This example provides data related to expanding tumor infiltrating lymphocytes (TILs) from tumor samples obtained by fine needle aspirations or core needle biopsies.

The goal of the study was to develop a method of expanding TILs from either core needle biopsies or fine needle aspirates. In some cases, 3 to 5 tumor samples were obtained from patients with cancer such as lung cancer (n=3), melanoma (n=1), head and neck cancer (n=1), cervical cancer (n=1), ovarian cancer (n=3), pancreatic cancer (n=2), glioblastoma (GBM), and colorectal cancer (n=1).

### II. Expanded TILs from core biopsies from a patient with pancreatic cancer (P7015).

Pancreatic core biopsies were received for the study. One core was placed into one well of a 24-well plate for a total of 5 wells. When two cores were place into two wells, little to no growth was observed in these wells. Three wells were treated with culture medium supplemented with IL-2 and two wells were treated with culture medium supplemented with a cytokine cocktail containing IL-2, IL-15, and IL-21. Viable cells were observed in the wells treated with IL-2 alone (Figure 8A) and in the wells treated with the cytokine cocktail (Figure 8B). About $1.3 \times 10^6$ viable cells/well ($1.2 \times 10^6$ viable cells/well on average) were obtained with the IL-2 treatment. About $2.3 \times 10^6$ viable cells/well ($2.0 \times 10^6$ viable cells/well on average) were obtained with the cytokine cocktail treatment.

Figures 17A, 17B, and 17C show phenotyping and functional status of TILs derived from a core biopsy of pancreatic tumors. The TILs were expanded from pancreatic tumors and culture in medium containing IL-2 or medium supplemented with cell culture additives. TILs were expanded from pancreatic tumors in IL-2 supplemented culture medium and in culture medium containing cell culture additives (Figure 17D). Figure 17A shows the percentage of cells that were CD3+, CD4+, CD8+, CD19+, CD3+/CD56+, or CD3-/CD56+. Figure 17B shows that the percentage of CD107a cells in the CD4+ subpopulation after stimulation with phorbol 12-myristate 13-acetate (PMA) compared to an unstimulated condition. Figure 17C shows functional analysis of the expanded TILs from the pancreatic core biopsy. The graph shows the percentage of CD107a cells in the CD8+ subpopulation after stimulation with phorbol 12-myristate 13-acetate (PMA) compared to an unstimulated condition.

The majority of cells expanded from the pancreatic core biopsies are T cells (CD4+ cells and CD8+ cells). Stimulation with PMA demonstrated the ability of the CD8+ to degranulate as indicated by expression of CD107a+, thereby suggesting that these cells are functional. The % of CD8+ expressing CD107a+ was higher in the wells treated with the culture additive wells compared to IL-2 alone (Figure 17C).

III. **Expanded TILs from fine needle aspirates from a patient with cervical carcinoma (C2005).**

A cervical tumor was received for the study. Five fine needle aspirates were isolated from the tumor using a 22 gauge needle and syringe. Each FNA sample was placed into at least three wells of a 24-well plate. Two wells of each sample were treated with IL-2 and one well of each sample was treated with the cytokine cocktail (IL-2, IL-15, and IL-21).

Figure 10A shows that CD4+ and CD8+ T cells were present in the FNAs treated with IL-2. On average 3.45 $\times$ $10^5$ viable cells/well were present in the IL-2 treated wells. Figure 10B shows that CD4+ and CD8+ T cells were present in the FNAs treated with the cytokine cocktail. On average 8.69 $\times$ $10^5$ viable cells/well were present in these wells. The IL-2. IL-15, and IL-21 treated aspirates demonstrated more cells/well compared to the IL-2 treated aspirates. The percentage of CD8+ cells was greater in the cocktail treated wells (20.1%) compared to the IL-2 treated wells (8.58%).

IV. **Expanded TILs from fine needle aspirates from a patient with lung carcinoma (L4032)**.

Three lung tumors were received for the study. Fine needle aspirates were isolated from the tumors using a 22 gauge needle and syringe. One FNA sample included 1.6 $\times$ $10^6$ cells with 28% viable cells. Two wells (each condition) were treated with culture medium supplemented with (1) IL-2 alone or (2) the cytokine cocktail. In addition, lung tumor fragments were cultured in a 24-well plate (two wells each) as a control. Cells were harvested at day 13 and evaluated by FACS analysis.

Figure 11A shows that T cells expanded from an FNA sample treated with IL-2. There were on average about 4.64 $\times$ $10^5$ viable cells/well. Figure 11B shows that T cells expanded from an FNA sample treated with the cocktail containing IL-2, IL-15, and IL-21. There were on average about 7.75 $\times$ $10^5$ viable cells/well. Figure 11C shows that T cells expanded from a lung tumor fragment treated with IL-2. There were on average about 4.28 $\times$ $10^5$ viable cells/well.

Next, the TILs cultured from the FNA samples were expanded using a rapid expansion protocol *(e.g.,* second expansion protocol or step D). The cells were harvested at day 14 and evaluated by FACS analysis. Figure 12A shows the total cell count of the expanded TILs from the FNA samples treated with (i) IL-2, (ii) IL-2, IL-15, and IL-21, and (iii) IL-2, IL-15, and IL-21 into IL-2. Figure 12A shows that after the second expansion, the TILs included CD4+ and CD8+ T cells (84.1% and 10.3%, respectively).

V. **Expanded TILs from fine needle aspirates from a patient with lung carcinoma (L4033).**

A fine needle aspirate was isolated from a lung tumor using a 22 gauge needle and syringe. The FNA sample included 5.5 $\times$ $10^6$ cells with 76% viable cells. One G-Rex 10 flask (each condition) was treated with culture media containing (1) IL-2 alone or (2) the cytokine cocktail. In addition, four lung tumor fragments were cultured in a G-Rex 10 flask as a control. Cells were harvested at day 12 and evaluated by FACS analysis. Figure 13A shows that CD4+ T cells (4.25%) and CD8+ T cells (55.1%) were obtained from the FNA sample treated with IL-2. Figure 13B shows that CD4+ T cells (7.76%) and CD8+ T cells (54.5%) were obtained from the tumor fragments treated with IL-2.

Next, the TILs cultured from the FNA samples were expanded using a rapid expansion protocol *(e.g.,* second expansion protocol or step D). The aspirates were incubated for the first 6 days in a conical tube. The cells were harvested at day 14 and evaluated by FACS analysis. Figure 14 shows the total cell count of the expanded TILs from the FNA samples and the tumor fragments treated with IL-2. The graph shows that TILs from the FNA samples expanded similarly to the TILs from the tumor fragments.

In a similar experiment, FNA samples were isolated from three lung tumor samples from a patient with lung carcinoma (L4034). About 1.0 $\times$ $10^6$ cells from an FNA sample were cultured in a G-Rex 10 flask for 15 days in culture medium supplemented with IL-2 alone. As a control, four tumor fragments were cultured in a G-Rex 10 flask culture medium supplemented with IL-2 alone. Few to no detectable cells were harvested from the FNA culture.

VI. **Expanded TILs from fine needle aspirates from patients with ovarian carcinoma (OV8011, OV8012, and OV8013)**.

Three ovarian tumors (OV8011, OV8012, and OV8013) were received for the study. Fine needle aspirates were isolated from the tumors using a 22 gauge needle and syringe.

The FNA sample from OV8011 included 3.19 $\times$ $10^5$ cells with 28% viable cells. This FNA sample was divided into four wells; two wells were cultured in culture medium containing IL-2 and two wells were cultured in culture medium containing IL-2, IL-15, and IL-21. The FNA sample from OV8012 included 8.3 $\times$ $10^5$ cells with 11% viable cells. This FNA sample was divided into two wells; one well was cultured in culture medium containing IL-2 and the other well was cultured in culture medium containing IL-2, IL-15, and IL-21. The FNA sample from OV8013 included 1.5 $\times$ $10^5$ cells with 7% viable cells. This FNA sample was divided into two wells; one well was cultured in culture medium containing IL-2 and the other well was cultured in culture medium containing IL-2, IL-15, and IL-21. No expansion was observed from the FNA samples.

As a control, four tumor fragments from each tumor were cultured in a G-Rex 10 flask. Some flasks received culture medium containing IL-2 alone and other flasks received culture medium containing IL-2, Il-15, and IL-21. Figure 15 shows the total cell counts of the cells derived from the ovarian tumor fragments cultured in culture medium containing (1) IL-2 alone or (2) the cytokine cocktail. The results show that the ovarian tumors expanded poorly in the first expansion.

**VII. Expanded TILs from fine needle aspirates from a patient with melanoma (M1101).**

A melanoma tumor (M1101) was received for the study. An FNA sample was obtained from the tumor. The sample included 1.96 $\times$ $10^6$ cells with 65% viable cells. The sample was place in a G-Rex 10 flask and treated with culture medium supplemented with IL-2. As a control, four melanoma tumor fragments were placed in a G-Rex 10 flask and treated with culture medium supplemented with IL-2. The cells were harvested at Day 12. Figure 16A shows that CD4+ T cells (21.4%) and CD8+ T cells (52.8%) expanded from the FNA sample treated with IL-2. Figure 16B shows that CD4+ T cells (18.0%) and CD8+ T cells (71.5%) expanded from the tumor fragments treated with IL-2.

Figures 18A and 18B provides a summary of the results obtained from the core biopsies and fine needle aspirates.

VIII. **Expanded TILs from fine needle aspirates from a patient with melanoma (M1101).**

On Day 11 of the pre-REP, cell counts were performed on the flasks containing the aspirate and fragments (n=2). The pre-REP cultures containing the fragments were harvested at Day 11 (Gen2). The pre-REP culture containing the aspirate was continued due to low cell count at Day 11. The aspirate was recounted at Day 18 and then again at Day 21 (Figure 19A). At Day 21, the culture had reached almost 60 $\times$ $10^6$ cells, the cells were harvested at this time and assessed via flow cytometry. The phenotype (Figure 19B) and function (CD107a expression, Figure 19C) of the pre-REP TIL from the fragments and aspirates were shown to be comparable.

Figures 19A, 19B and 19C show the expansion of TILs from an FNA sample from another patient with melanoma (M1107). Figure 19A shows the total cell count at different time during the expansion. Figure 19B shows the phenotype of the expanded TILs. Figure 19C shows functional analysis of the expanded TILs from fragments and aspirates. The graph shows the percentage of CD107a cells in the CD4+ and CD8+ subpopulations after stimulation with PMA compared to an unstimulated condition.

**EXAMPLE 9: TUMOR INFILTRATING LYMPHOCYTE (TIL) EXPANSION FROM FINE NEEDLE ASPIRATES (FNAS) AND CORE NEEDLE BIOPSIES**

**I. Pancreatic cores: Data shows in Figures 20-29.**

Fifteen tumors (Pancreatic, Lung, Cervical, Mesothelioma, Colorectal) was reported. To date, 37 tumor samples from different histology including pancreatic, cervical, lung, oral and esophageal, sarcoma cancers have been received.

Upon arrival the tumors were fragmented and placed in flasks/plate with IL-2 for a pre-Rapid Expansion Protocol (REP). pre-REP TIL were further propagated in a REP protocol in the presence of irradiated PBMCs, anti-CD3 antibody (30 ng/mL), and IL-2 (3000 IU/mL). Lung and Sarcoma pre-REP TIL were expanded from 4 tumor fragments in 24-well-plate format for up 22 days. Other cancers were expanded from 4 tumor fragments in G-REX TIL for up to 22 days.

II. **TIL can be expanded from Core Biopsies from Pancreatic Cancer: data shown in Figures 30-37.**

**General conclusions**

The kinetics of growth demonstrated very few cells at Day 11-12 in both P7028 and P7031, however significant expansion was demonstrated between Day 21-28.

The majority of cells expanded from the pancreatic core biopsies are T cells and phenotypically CD4+. TIL isolated from pancreatic tumors are typically CD4+.

Stimulation with PMA demonstrated the ability of the CD4+ to degranulate as indicated by expression of CD107a+, thereby suggesting that these cells are functional, as was shown with the earlier studies with P7015. The expression of CD107a was similar in TIL derived from fragments.

The TIL derived from cores secreted significant levels of IFNγ in a restimulation assay with plate-bound anti-CD3, similar to TIL derived from fragments.

Extensive phenotyping demonstrated similar expression of the majority of markers assessed in TIL derived from tumor fragments, suggesting that the outcome of growing TIL from cores is similar to fragments.

The exhaustion/activation panel demonstrated significant differences in PD-1, Tim3 and KLRG1 in the CD4+ cells and LAG3, Tim3 and KLRG1 in the CD8+ cells. Additional pancreatic core studies are required to validate these findings.

**General Proposals:**

These results suggest that the process for growing TIL successfully from pancreatic cores (and other histologies) will likely require additional time in culture (beyond the pre-REP requirements for Gen2), unless additional "tweaking strategies" are utilized to enhance growth (see below).

Based upon the preliminary growth data in pancreatic cores, it is recommended that the pre-REP process be between Day 21-28, in order to obtain sufficient cells .

For this process the pre-REP should be initiated in G-Rex 10 or G-Rex 10M's will CM1 media and IL-2/IL-15/IL-21.

The REP should be initiated either in a G-Rex 100M or G-Rex 500M, depending on the pre-REP cell count.

G-Rex 500M: if the cell count is $5 \times 10^7$.

G-Rex 100M: if cell count is between $1\text{-}5 \times 10^7$.

Future experiments will be used to determine the minimum number of cells required for sufficient cell growth in each vessel.

Future experiments will compare the growth, phenotype and function of pancreatic cores expanded in IL-2/IL-15/IL-21 alone or with IL-2/IL-15/IL-21 with OKT3 and feeders.

**EXAMPLE 10: EXPANDING TIL USING CORE BIOPSIES**

Rationale:

Tumor removal/resection is not appropriate or feasible for all patients. In this cohort of patients, core biopsies may be a source of TIL for ACT (adoptive cellular therapy). Such core biopsies allow to sample multiple lesions and potentially obtain a broader TIL repertoire.

**Strategy:**

The present example provides a method to expand TIL from core biopsies, from multiple histologies and develop a defined process that is feasible for TIL production from core biopsies. See, for example Figure 38 which provides a diagram of two exemplary processes.

Data

The ability to expand TIL form biopsies has been demonstrated for pancreatic cores using Gen2, which also showed that TIL obtained from biopsies are phenotypically and functionally comparable to TIL obtained from fragments.

Seven sets of pancreatic cores (between 7-10 cores) were treated with IL-2/IL-15/IL-21 and pre-REP'ed and assessed for expansion. Three successfully expanded, however the growth was extremely variable. Pre-REP cultures required at least 21-28 days to acquire sufficient numbers of cells *(e.g.,* 21-28 days for Steps A through E of Figure 7). Extensive phenotyping demonstrated similar expression of the majority of markers assessed in TIL derived from pancreatic tumor fragment. The expression of CD107a and IFNγ was similar in TIL derived from fragments

Current protocol:

Addition of OKT3 & feeders at Day 3 of culture enhanced TIL yield. Cultured fewer cores/flask and assess growth (for example, cultured 1-2 cores).

1-2 core biopsies (3 pancreas, 4 melanoma, 1 breast, and 1 ovarian) were subjected to a modified research-scale process that included the addition of OKT3 + feeders at Day 3 (See, Figures 39-40).

- TIL were subjected to a second REP *(e.g.,* a second expansion).
- Pancreatic cores were expanded +/- IL15 & 21 (the triple cocktail; REP1 & REP2; *e.g.,* a first expansion and a second expansion)
- Ovarian cores were treated +/- OX40 (BMS) (n=1, preliminary data).
- All TIL were subjected to a second REP.

**PANCREATIC**

TIL from pancreatic cores were successfully expanded in the presence of OKT3 + feeders. >50e6 cells were acquired between Day 11-17 of the first expansion.

Two cores were generally required to acquire the appropriate cell numbers.

The addition of the triple cocktail (TC: IL-2, IL-15/IL-21) to the $2^{nd}$ REP (either previously cultured in IL-2 or the TC) did not significantly alter the expansion of TIL in comparison to IL-2 alone.

The addition of OKT3 + feeders at Day 3 to core biopsies does not significantly alter the phenotype (memory, CD27/CD28, exhaustion, activation) of TIL in comparison to cores grown using Gen2 like conditions. Phenotype (memory, CD27/CD28, exhaustion, activation) of TIL expanded through 2 REPs did not significantly differ from that of TIL grown using Gen2 conditions.

CD107a expression did not vary significantly with the addition of the OKT3 and feeders. TIL expanded through 2 REPs maintained the ability to respond to non-specific stimulus, as assessed by CD107a induction.

TIL expanded through 2 REPs maintained the ability to respond to non-specific stimulus, as assessed by CD107a induction.

IFNγ secretion was evaluated. TIL produce IFNγ upon stimulation with OKT3.

Pancreatic TILs can be expanded from Core Biopsies from Pancreatic Cancer. **P7028 and P7031 pre-REP Experimental Summary and Results**

Core biopsies were place into a G-Rex 10 with the triple cocktail (IL-2/IL-15/IL-21)

P7028 TIL were assessed at Day 12 and kept in culture until Day 19

Cell counts: Day 12= $1.61 \times 10^6$ viable cells; Day 21 $3.49 \times 10^7$ viable cells

P7030 TIL were assessed at Day 12 and kept in culture until Day 27

Cell counts: Day 12= $1.52 \times 10^5$ viable cells; Day 27: $1.14 \times 10^7$ viable cells

**MELANOMA**

TIL derived from melanoma cores were successfully expanded in the presence of OKT3 + feeders (n=4). >50 $\times 10^6$ cells were acquired by Day 12 of the first expansion.

One to two cores were generally required to acquire the appropriate cell numbers. The addition of OKT3 + feeders at Day 3 to core biopsies, does not significantly alter the phenotype (memory, CD27/CD28, exhaustion, activation) of TIL in comparison to fragments using Gen2 like conditions.

TIL expanded through 2 REPs responded to non-specific stimuli, as assessed by CD107a mobilization and IFNγ secretion.

**LUNG**

TILs from 1 core from lung were successfully expanded in the presence of IL-2+ OKT3 + feeders at Day 0. >50 $\times 10^6$ cells were acquired at Day 11.

Phenotype (memory, CD27/CD28, exhaustion, activation) of TIL expanded through 2-REPs varied slightly from TIL expanded from cores treated with OKT3 + feeders at Day 3 of culture initiation. There was an increase in the ratio of CD8+/CD4+ in the Day 3 treated cores.

There were slight differences in CD28, CD69, CD39, CD49a, Tim3 and CD101 expression were observed between the Day 0 and Day 3 culture conditions.

The TILs produced IFNγ upon stimulation with anti-CD3. The Day 0 OKT3 + feeders treated lung cores had greater levels of IFNγ in comparison to the Day 3 OKT3 + feeders treated lung cores.

The TILs expanded through 2 REPs maintained the ability to respond to non-specific stimulus, as assessed by CD107a induction.

The CD8+ TILs from Day 3 OKT3 + feeders treated lung cores were oligoclonal compared to the Day 0 OKT3 + feeders treated lung core TILs, indicating a different TCR repertoire depending on the timing of the addition of OKT3 + feeders.

**Conclusions**

The addition of OKT3 + feeders to cores at Day 3 of culture significantly improved TIL yield. >50 $\times$ $10^6$ cells (threshold for REP2 initiation) was acquired within 11-15 days of the first expansion in all histologies assessed (pancreatic, melanoma, breast and ovarian). Two cores (in the histologies assayed) were sufficient to acquire the number of cells required.

TILs from at least 2 cores from breast were successfully expanded in the presence of IL-2+ OKT3 + feeders, but not IL-2 alone. >50 $\times$ $10^6$ cells were acquired at Day 11. One to two cores are sufficient to acquire the number of cells required.

Data suggests that the phenotype and function of TIL expanded from cores (with early OKT3+ feeders) is similar to cores/fragments expanded under Gen2 conditions (IL-2 alone). Addition of OX40 in ovarian cores improved the expansion of TILs in comparison to IL-2 alone.

1-2 pancreatic cores failed to expand in IL-2 alone.

Side to side experiments will be performed with fragments +/- OKT3+ feeders at Day 3. TILs produced IFNγ upon stimulation with OKT3 and further experiments are ongoing. Differences in KLRG1, CD25 and PD1 were observed in ovarian cores treated +/-OX40 (therapeutic TIL population).

Phenotype, function (potency) and TCR repertoire were and continue to be assessed (currently in progress). The addition of OKT3 + feeders at Day 3 to core biopsies, did not significantly alter the phenotype (memory, CD27/CD28, exhaustion, activation) of TIL in comparison to fragments using Gen2 like conditions. TIL expanded through 2 REPs responded to non-specific stimuli, as assessed by CD107a mobilization and IFNγ secretion. Phenotype (memory, CD27/CD28, exhaustion, activation) of TILs expanded through 2-REPs did not significantly differ from that of TILs expanded using a 1-REP process (ie. no OKT3+feeders during 1st expansion) in breast cores.

Slight differences in KLRG1 and PD1 expression were observed.

TILs expanded through 2 REPs maintained the ability to respond to non-specific stimulus, as assessed by CD107a induction.

Further assessment will include +/- removal of cores at Day 3 as well as further assessment of the effect of anti-OX40 on expanded TILs will be performed.

**EXAMPLE 11: EXPANDING TIL FROM BREAST AND OVARIAN CORE BIOPSIES USING OKT3+ FEEDERS: SUMMARY OF EXAMPLES 9-10**

TIL from at least 2 cores from breast were successfully expanded in the presence of IL-2+ OKT3 + feeders, but not IL-2 alone. >50 $\times$ $10^6$ cells were acquired at Day 11.

Addition of OX40 in ovarian cores improved the expansion of TIL in comparison to IL-2 alone.

Phenotype (memory, CD27/CD28, exhaustion, activation) of TIL expanded through 2-REPs did not significantly differ from that of TIL expanded using a 1-REP process (*i.e.* no OKT3+feeders during 1st expansion) in breast cores.

Slight differences in KLRG1 and PD1 expression were observed.

Differences in KLRG1, CD25 and PD1 were observed in ovarian cores treated +/-OX40 (final product).

TIL produce IFNγ upon stimulation with OKT3.

TIL expanded through 2 REPs maintained the ability to respond to non-specific stimulus, as assessed by CD107a induction.

The addition of OKT3 + feeders to cores at Day 3 significantly improved TIL yield. >50 $\times$ $10^6$ cells (threshold for REP initiation under Gen2 conditions) was acquired within 11-15 days in all histologies assessed (pancreatic, melanoma, breast, and ovarian). Two cores (in the histologies assayed) are sufficient to acquire the number of cells required. Preliminary data suggest that the phenotype and function of TIL expanded from cores (with early OKT3+ feeders) is similar to cores/fragments expanded under control conditions.

**EXAMPLE 12: EXPANDING TIL FROM NEEDLE ASPIRATES**

**Strategy:**

To expand TIL from needle aspirates, from multiple histologies and develop a defined process that is feasible for TIL production. Preliminary studies demonstrated that TIL could be expanded from "some" aspirates performed under optimal conditions (i.e., taken directly from a tumor biopsies).

22 aspirates were isolated from tumor biopsies of various histologies at Iovance using Gen2 conditions (i.e., no OKT3 and feeders at Day 3). Growth was observed in 6 tumors (1 cervical, 2 lung and 3 melanoma). The number of cells isolated during the pre-REP was well below the $50 \times 10^6$ cell threshold.

Additionally, 10 needle aspirates have been received, including three from melanoma (n-3), and seven from breast (n-7). The aspirates were treated with OKT3 and feeders.

Due to small initial cell numbers, most of the experiments were initiated in a 96 well plate. $<50 \times 10^6$ cells were obtained at Day 11 in all aspirates. Several sub-cultures would be required to acquire the necessary number of cells.

## EXAMPLE 13: EXPANDING TIL FROM TUMOR CORE BIOPSIES

### INTRODUCTION

Adoptive T cell therapy (ACT) with autologous tumor infiltrating lymphocytes (TIL) has demonstrated clinical efficacy in patients with metastatic melanoma and cervical carcinoma. TIL are currently expanded from fragmented tumor that is removed surgically. Unfortunately, patients with unresectable tumors do not currently qualify for TIL therapy. If TIL expansion were feasible from the type of small tumor samples used by physicians for diagnostics and staging purposes, correlative studies, and biomarker analyses, TIL therapy would become available to a greater number of cancer patients.

A core needle biopsy is an example of such small tumor samples. It is an inexpensive, safe, simple and less invasive procedure than surgical resection. A core biopsy removes a small cylinder of tumor tissue using a small incision and needle. Typically, a needle can be inserted into the incision up to 5 times, to excise an adequate amount of tissue. Core biopsies are small samplings of the intact tumor microenvironment, and therefore contain tumor cells, stroma and immune cells, including TIL. To date, there is one clinical study, in melanoma, that used tissue derived from core needle biopsies to expand TIL (Ullenhag, G.J., et al., Cancer Immunol Immunother, 61(5):725-732 (2014)). The absolute cell numbers and fold expansion were similar between TIL derived from surgical resection versus TIL derived from core biopsies. Interestingly, most of the objective responses (4/5) in this study were treated with cells derived from core biopsies. No further studies have corroborated these findings.

In some embodimetns, a 2-REP process is employed to expand TIL derived from core biopsies, as described above and in the present example.

### PURPOSE

The present example provides a protocol to expand TIL to clinically appropriate numbers from small tumor samples for ACT (adoptive cell transfer). This protocol was developed using tumor core biopsies from pancreatic, melanoma, breast, and ovarian tumors. The expanded TIL were assessed for growth, viability, phenotype, function (IFN$\gamma$ secretion, CD107a mobilization), and TCR V$\beta$ repertoire (RNA-sequencing). Materials

Tumor Core Tissue. Tumor cores from pancreas, melanoma, breast, and ovarian are received from UPMC, Biotheme and MT group.

### PROCEDURE

#### Core Processing and Culture Initiation

Freshly resected tumor core biopsies are received from research alliances (UPMC) and tissue procurement vendors (Biotheme and MTG group). The tumor core biopsies are shipped overnight in HypoThermosol (Biolife Solutions, Washington, Cat # 101104) (with antibiotic) or RPMI 1640 (Fisher Scientific, Pennsylvania, Cat # 11875-085) + male human AB serum (Access Biologicals, California, A13012).

Using a 0.22-$\mu$M filter on top of 50-mL conical tube, carefully and slowly pour the contents of the shipping container comprising the tumor core biopsies through the filter. The cores are retained in the filter. Wash the cores by gently adding 10-20 mL of HBSS over the cores.

Gently remove each core from the filter membrane, while attempting to keep the core intact. Using either sterile plastic forceps or gently pipetting (with approximately 200 $\mu$L of HBSS in a 1-mL pipette), transfer the core to a G-Rex 10 with 40 mL of CM2 and 6000 IU/mL of IL-2.

#### REP1 initiation (*e.g.*, priming first expansion)

On Day 3 post-culture, OKT3 (30 ng/mL) (Miltenyi Biotec, Germany, Cat # 170-076-116) and Feeders (1:100) are added to the G-Rex 10 without disturbing the culture upon addition.

Assess the culture daily for growth, by microscopy. REP1 is conducted for 8 days after the addition of OKT3 + Feeders and ends at Day 11. The cells remain untouched for the entire length of the culture.

To count, remove approximately 30 mL of media and resuspend the cells in the remaining 10 mL of media, by pipetting up and down. Place cells in a 50-mL conical tube and centrifuge at 1500 rpm at RT for 5 min in a Sorvell Legend XFR centrifuge and swinging bucket (ThermoScientific, CA, Cat #75003180).

Determine cell and viability counts, using the Nexcelom Cellometer K2 (Nexcelom, MA).

If after centrifugation there is no pellet, or the pellet is < 200 μL, resuspend the pellet in 2 mL of media for counting. If the pellet is > 200 μL resuspend in 5 mL of CM2 media for counting.

Experiments are harvested at Day 11 post-culture initiation (8 days post-OKT3 + Feeders addition). REP1 product is assessed for cell count, viability, phenotype (TIL1, TIL2 and TIL3 (TIL2 and TIL3 panel for Surface Antigen Staining of TIL), TIL3), and function (CD107a (Assessing TIL function by CD107a mobilization), IFNγ (WRK LAB-059)). The remaining cells are frozen and stored in liquid nitrogen.

REP2 Initiation (*e.g.*, second rapid expansion)

For mini-REP2 initiation, $1 \times 10^5$ cells are placed into a G-Rex 10 with 40 mL of CM2 media and 3000 IU/mL of IL-2. Anti-CD3 (clone: OKT3, 30 ng/mL) and Feeders (1:100 ratio, TIL: Feeders) are added at culture initation. All flasks are treated with OKT3 + Feeders in REP2, even if OKT3 + feeders were not added during REP1.

A media change is performed at Day 5-7 of REP2 (Day 16-18 of process). The media in the G-Rex 10 flask is volume reduced to approximately 10 mL and supplemented to 40 mL with either CM2 or AimV + 3000 IU/mL IL-2.

At Day 11 of REP2, volume is reduced as indicated above and cells centrifuged at 1500 rpm at RT for 5 min.

The final product is assessed for cell count, viability, phenotype (TIL1, TIL2 (TIL2 panel for Surface Antigen Staining of TIL, v1 and v2), TIL3 and function (CD107a (Assessing TIL function by CD107a mobilization, IFNγ). Additional cells ($1 \times 10^6$ - $5 \times 10^6$ cells) are pelleted and frozen for RNA sequencing and analysis. The remaining cells are frozen and stored in liquid nitrogen.

RESULTS AND ACCEPTANCE CRITERIA

The REP1 yield is expected to be > $50 \times 10^6$ by D11 in flasks that were treated with OKT3 + Feeders. The fold expansion during REP2 is expected to be unaffected by the addition of OKT3 and feeders during REP1 and to be consistent with Gen2 REP fold expansion numbers. The phenotype of TIL derived from tumor core biopsies and exposed early to OKT3 + Feeders is expected to be similar to that of TIL derived from surgical resections (or small biopsies) and expanded according to the Gen 2 process.

**EXAMPLE 14: GEN 3 EXEMPLARY PROCESS**

The example provides a comparison between the Gen 2 and Gen 3 processes. This example describes the development of a robust TIL expansion platform. The modifications to the Gen 2 process reduce risk and streamline the manufacturing process by reducing the number of operator interventions, reduce the overall time of manufacturing, optimize the use of reagents, and facilitate a flexible semi-closed and semi-automated cell production process amenable to high-throughput manufacturing on a commercial scale.

Process Gen 2 and Gen 3 TILs are composed of autologous TIL derived from an individual patient through surgical resection of a tumor and then expanded *ex vivo.* The Priming First Expansion step of the Gen 3 process was a cell culture in the presence of interleukin-2 (IL-2) and the monoclonal antibody OKT3, which targets the T-cell co-receptor CD3 on a scaffold of irradiated peripheral blood mononuclear cells (PBMCs).

The manufacture of Gen 2 TIL products consisted of two phases: 1) pre-Rapid Expansion (Pre-REP) and 2) Rapid Expansion Protocol (REP). During the Pre-REP resected tumors were cut up into ≤ 50 fragments 2-3 mm in each dimension which were cultured with serum-containing culture medium (RPMI 1640 media containing 10% HuSAB supplemented) and 6,000 IU/mL of Interleukin-2 (IL-2) for a period of 11 days. On day 11 TIL were harvested and introduced into the large-scale secondary REP expansion. The REP consists of activation of $\leq 200 \times 10^6$ of the viable cells from pre-REP in a co-culture of $5 \times 10^9$ irradiated allogeneic PBMCs feeder cells loaded with 150 μg of monoclonal anti-CD3 antibody (OKT3) in a 5L volume of CM2 supplemented with 3000 IU/mL of rhIL-2 for 5 days. On day 16 the culture is volume reduced 90% and the cell fraction is split into multiple G-REX-500 flasks at $\geq 1 \times 10^9$ viable lymphocytes/flask and QS to 5L with CM4. TIL are incubated an additional 6 days. The REP is harvested on day 22, washed, formulated, and cryo-preserved prior to shipping at -150°C to the clinical site for infusion.

The manufacture of Gen 3 TIL products consisted of three phases: 1) Priming First Expansion Protocol 2) Rapid Second Expansion Protocol (also referred to as rapid expansion phase or REP) and 3) Subculture Split. To effect the Priming First Expansion TIL propagation, resected tumor was cut up into ≤ 120 fragments 2-3 mm in each dimension. On day 0 of the Priming First Expansion, a feeder layer of approximately 2.5 × $10^8$ allogeneic irradiated PBMCs feeder cells loaded with OKT-3 was established on a surface area of approximately 100 $cm^2$ in each of 3 100 MCS vessels. The tumor fragments were distributed among and cultured in the 3 100 MCS vessels each with 500 mL serum-containing CM1 culture medium and 6,000 IU/mL of Interleukin-2 (IL-2) and 15 ug OKT-3 for a period of 7 days. On day 7, REP was initiated by incorporating an additional feeder cell layer of approximately 5 × $10^8$ allogeneic irradiated PBMCs feeder cells loaded with OKT-3 into the tumor fragmented culture phase in each of the 3 100 MCS vessels and culturing with 500 mL CM2 culture medium and 6,000 IU/mL IL-2 and 30 ug OKT-3. The REP initiation was enhanced by activating the entire Priming First Expansion culture in the same vessel using closed system fluid transfer of OKT3 loaded feeder cells into the 100MCS vessel. For Gen 3, the TIL scale up or split involved process steps where the whole cell culture was scaled to a larger vessel through closed system fluid transfer and was transferred (from 100 M flask to a 500 M flask) and additional 4L of CM4 media was added. The REP cells were harvested on day 16, washed, formulated, and cryo-preserved prior to shipping at -150°C to the clinical site for infusion.

Overall, the Gen 3 process is a shorter, more scalable, and easily modifiable expansion platform that will accommodate to fit robust manufacturing and process comparability.

**Table 36:** Comparison of Exemplary Gen 2 and Exemplary Gen 3 manufacturing process.

| Step | Process (Gen 2) | Process (Gen 3) |
|---|---|---|
| Pre REP- day 0 | Up to 50 fragments/ 1-G-Rex 100MCS - 11 days<br>In 1 L of CM1 media<br>+ IL-2 (6000 IU/mL) | Whole tumor up to 120 fragments divided evenly among up to 3 flasks. 1 flask: 1-60 fragments<br>2 flasks: 61-89 fragments<br>3 flasks 90-120 fragments<br>7 days in 500 mL of CM1 media<br>+ IL-2 (6000 IU/mL)<br>2.5 × $10^8$ feeder cells/flask<br>15ug OKT-3/flask |

| | | |
|---|---|---|
| REP Initiation | Direct to REP- Day 11-<br><200 × $10^6$ TIL<br>(1)G-Rex 500MCS in 5L CM2 media<br>IL-2 (3000 IU/mL)<br>5 × $10^9$ feeder cells<br>150ug OKT-3 | Direct to REP- Day 7-all cells TIL- same G-Rex 100MCS<br>Add 500 CM2 media<br>IL-2 (6000 IU/mL)<br>5 × $10^8$ feeder cells/flask<br>30ug OKT-3/flask |
| TIL propagation or Scale up | Volume reduce and split cell fraction in up to 5 G-REX 500MCS<br>4.5L CM4 media + IL-2 (3000 IU/mL)<br>≥ 1 × $10^9$ TVC / flask<br>Split day 16 | Each G-REX 100MCS(1L) transfers to 1 G-REX 500MCS<br>Add 4L CM4 media +IL-2 (3000 IU/mL)<br>Scale up on day 9 to 11 |
| Harvest | Harvest day 22,<br>LOVO-automated cell washer | Harvest day 16<br>LOVO- automated cell washer |
| Final formulation | Cryopreserved Product<br>300IU/mL IL2- CS10 in $LN_2$,<br>multiple aliquots | Cryopreserved product<br>300IU/mL IL-2-CS10 in $LN_2$,<br>multiple aliquots |
| **Process time** | **22 days** | **16 days** |

On day 0, for both processes, the tumor was washed 3 times and the fragments were randomized and divided into two pools; one pool per process. For the Gen 2 Process, the fragments were transferred to one GREX 100MCS flask with 1 L of CM1 media containing 6,000IU/mL rhIL-2. For the Gen 3 Process, fragments were transferred to one GRE × 100MCS flask with 500 mL of CM1 containing 6,000IU/mL rhIL-2, 15 μg OKT-3 and 2.5 × $10^8$ feeder cells. Seeding of TIL for Rep initiation day occurred on different days according to each process. For the Gen 2 Process, in which the G-REX 100MCS flask was 90% volume reduced, collected cell suspension was transferred to a new G-REX 500MCS to start REP initiation on day 11 in CM2 media containing IL-2 (3000 IU/mL), plus 5 × $10^9$ feeder cells and OKT-3 (30 ng/mL). Cells were expanded and split on day 16 into multiple GREX 500 MCS flasks with CM4 media with IL-2 (3000 IU/mL) per protocol. The culture was then harvested and cryopreserved on day 22 per protocol. For the Gen 3 process, the REP initiation occurred on day 7, in which the same G-REX 100MCS used for REP initiation. Briefly, 500 mL of CM2 media containing IL-2 (6000 IU/mL) and 5 × $10^8$ feeder cells with 30 μg OKT-3 was added to each flask. On day 9-11 the culture was scaled up. The entire volume of the G-Re × 100M (1L) was transferred to a G-REX 500MCS and 4L of CM4 containing IL-2 (3000 IU/mL) was added. Flasks were incubated 5 days. Cultures were harvested and cryopreserved on Day 16.

Three different tumors were included in the comparison, two lung tumors (L4054 and L4055) and one melanoma tumor (M1085T).

CM1 (culture media 1), CM2 (culture media 2), and CM4 (culture media 4) media were prepared in advance and held at 4°C for L4054 and L4055. CM1 and CM2 media were prepared without filtration to compare cell growth with and without filtration of media.

Media was warmed at 37°C up to 24 hours in advance for L4055 tumor on REP initiation and scale-up.

**Results Summary**

Gen 3 fell within 30% of Gen 2 for total viable cells achieved. Gen 3 final product exhibited higher production of INF-γ after restimulation. Gen 3 final product exhibited increased clonal diversity as measured by total unique CDR3 sequences present. Gen 3 final product exhibited longer mean telomere length.

**Results achieved**

**Cell count and % viability:**

Pre REP and REP expansion on Gen 2 and Gen 3 processes followed details described above.

**Table 37: Pre-REP cell counts.** For each tumor, the two pools contained equal number of fragments. Due to the small size of tumors, the maximum number of fragments per flask was not achieved. Total pre-REP cells (TVC) were harvested and counted at day 11 for the Gen 2 process and at day 7 for the Gen 3 process. To compare the two pre-REP arms, the cell count was divided over the number of fragments provided in the culture in order to calculate an average of viable cells per fragment. As indicated in the table below, the Gen 2 process consistently grew more cells per fragment compared to the Gen 3 Process. An extrapolated calculation of the number of TVC expected for Gen 3 process at day 11, which was calculated dividing the pre-REP TVC by 7 and then multiply by 11.

**Table 37: pre-REP cell counts**

| **Tumor ID** | **L4054** | | **L4055*** | | **M1085T** | |
|---|---|---|---|---|---|---|
| Process | **Gen 2** | **Gen 3** | **Gen 2** | **Gen 3** | **Gen 2** | **Gen 3** |
| pre-REP TVC | 1.42E+08 | 4.32E+07 | 2.68E+07 | 1.38E+07 | 1.23E+07 | 3.50E+06 |
| Number of fragments | 21 | 21 | 24 | 24 | 16 | 16 |
| Average TVC per fragment at pre-REP | 6.65E+06 | 2.06E+06 | 1.12E+06 | 5.75E+05 | 7.66E+05 | 2.18E+05 |
| Gen 3 extrapolated value at pre REP day 11 | N/A | 6.79E+07 | N/A | 2.17E+07 | N/A | 5.49E+06 |
| *** L4055, unfiltered media.** | | | | | | |

**Table 38: Total viable cell count and fold expansion on TIL final product:** For the Gen 2 and Gen 3 processes, TVC was counted per process condition and percent viable cells was generated for each day of the process. On harvest, day 22 (Gen 2) and day 16 (Gen 3) cells were collected and the TVC count was established. The TVC was then divided by the number of fragments provided on day 0, to calculate an average of viable cells per fragment. Fold expansion was calculated by dividing harvest TVC by over the REP initiation TVC. As exhibited in the table, comparing Gen 2 and the Gen 3, fold expansions were similar for L4054; in the case of L4055, the fold expansion was higher for the Gen 2 process. Specifically, in this case, the media was warmed up 24 in advance of REP initiation day. A higher fold expansion was also observed in Gen 3 for M1085T. An extrapolated calculation of the number of TVC expected for Gen 3 process at day 22, which was calculated dividing the REP TVC by 16 and then multiply by 22.

**Table 38: Total viable cell count and fold expansion on TIL final product**

| **Tumor ID** | **L4054** | | **L4055** | | **M1085T** | |
|---|---|---|---|---|---|---|
| **Process** | **Gen 2** | **Gen 3** | **Gen 2** | **Gen 3** | **Gen 2** | **Gen 3** |
| # Fragments | **21** | **21** | **24** | **24** | **16** | **16** |
| TVC/fragment (at Harvest) | 3.18E+09 | 8.77E+08 | 2.30E+09 | 3.65E+08 | 7.09E+08 | 4.80E+08 |
| REP initiation | 1.42E+08 | 4.32E+07 | 2.68E+07 | 1.38E+07 | 1.23E+07 | 3.50E+06 |
| Scale up | 3.36E+09 | 9.35E+08 | 3.49E+09 | 8.44E+08 | 1.99E+09 | 3.25E+08 |
| Harvest | 6.67E+10 | 1.84E+10 | 5.52E+10 | 8.76E+09 | 1.13E+10 | 7.68E+09 |
| Fold Expansion Harvest/ REP initiation | **468.4** | **425.9** | **2056.8** | **634.6** | **925.0** | **2197.2** |
| Gen 3 extrapolated value at REP harvest day 22 | **N/A** | **2.53E+10** | **N/A** | **1.20E+10** | **N/A** | **1.06E+10** |
| *** L4055, unfiltered media.** | | | | | | |

**Table 39: % Viability of TIL final product:** Upon harvest, the final TIL REP products were compared against release criteria for % viability. All of the conditions for the Gen 2 and Gen 3 processes surpassed the 70% viability criterion and were comparable across processes and tumors.

**Table 39: % Viability of REP**

| Tumor ID | L4054 | | L4055 | | M1085T | |
|---|---|---|---|---|---|---|
| Process | Gen 2 | Gen 3 | Gen 2 | Gen 3 | Gen 2 | Gen 3 |
| REP initiation | 98.23% | 97.97% | 97.43% | 92.03% | 81.85% | 68.27% |
| Scale up | 94.00% | 93.57% | 90.50% | 95.93% | 78.55% | 71.15% |
| Harvest | 87.95% | 89.85% | 87.50% | 86.70% | 86.10% | 87.45% |

**Table 40: Estimate cell count per additional flask for Gen** 3 **process.** Due to the number of fragments per flask below the maximum required number, an estimated cell count at harvest day was calculated for each tumor. The estimation was based on the expectation that clinical tumors were large enough to seed 2 or 3 flasks on day 0.

**Table 40: Extrapolated estimate cell count calculation to full scale 2 and 3 flask on Gen 3 Process**

| Tumor ID | L4054 | | L4055 | | M1085T | |
|---|---|---|---|---|---|---|
| Gen 3 Process | 2 flasks | 3 Flasks | 2 flasks | 3 Flasks | 2 flasks | 3 Flasks |
| Estimate Harvest | 3.68E+10 | 5.52E+10 | 1.75E+10 | 2.63E+10 | 1.54E+10 | 2.30E+10 |

**IMMUNOPHENOTYPING:**

**Phenotypic markers comparison on TIL final product:**

Three tumors L4054, L4055, and M1085T underwent TIL expansion in both the Gen 2 and Gen 3 processes. Upon harvest, the REP TIL final products were subjected to flow cytometry analysis to test purity, differentiation, and memory markers. For all the conditions the percentage of TCR a/b+ cells was over 90%.

TIL harvested from the Gen 3 process showed a higher expression of CD8 and CD28 compared to TIL harvested from the Gen 2 process. The Gen 2 process showed a higher percentage of CD4+. **See, Figure 3 (A, B, C).**

**Memory markers comparison on TIL final product:**

TIL harvested from the Gen 3 process showed a higher expression on central memory compartments compared to TIL from the Gen 2 process. **See, Figure 4 (A, B, C).**

**Activation and exhaustion markers comparison on TIL final product:**

Activation and exhaustion marker were analyzed in TIL from two, tumors L4054 and L4055 to compare the final TIL product by from the Gen 2 and Gen 3 TIL expansion processes. Activation and exhaustion markers were comparable between the Gen 2 and Gen 3 processes. **See, Figure 5 (A, B); Figure 6 (A, B).**

**INTERFERON GAMMA SECRETION UPON RESTIMULATION:**

On harvest day, day 22 for Gen 2 and day 16 for Gen 3, TIL underwent an overnight restimulation with coated anti -CD3 plates for L4054 and L4055. The restimulation on M1085T was performed using anti-CD3, CD28, and CD137 beads. Supernatant was collected after 24 hours of the restimulation in all conditions and the supernatant was frozen. IFNγ analysis by ELISA was assessed on the supernatant from both processes at the same time using the same ELISA plate. Higher production of IFNγ from the Gen 3 process was observed in the three tumors analyzed. **See, Figure 7 (A, B, C).**

**MEASUREMENT OF IL-2 LEVELS IN CULTURE MEDIA:**

To compare the IL-2 consumption between Gen 2 and Gen 3 process, cell supernatant was collected on REP initiation, scale up, and harvest day, on tumor L4054 and L4055. The quantity of IL-2 in cell culture supernatant was measured by Quantitate ELISA Kit from R&D. The general trend indicates that the IL-2 concentration remains higher in the Gen 3 process when compared to the Gen 2 process. This is likely due to the higher concentration of IL-2 on REP initiation (6000 IU/mL) for Gen 3 coupled with the carryover of the media throughout the process. **See, Figure 8 (A, B).**

## METABOLIC SUBSTRATE AND METABOLITE ANALYSIS

The levels of metabolic substrates such as D-glucose and L-glutamine were measured as surrogates of overall media consumption. Their reciprocal metabolites, such lactic acid and ammonia, were measured. Glucose is a simple sugar in media that is utilized by mitochondria to produce energy in the form of ATP. When glucose is oxidized, lactic acid is produced (lactate is an ester of lactic acid). Lactate is strongly produced during the cells exponential growth phase. High levels of lactate have a negative impact on cell culture processes. **See, Figure 9 (A, B).**

Spent media for L4054 and L4055 was collected at REP initiation, scale up, and harvest days for both process Gen 2 and Gen 3. The spent media collection was for Gen 2 on Day 11, day 16 and day 22; for Gen 3 was on day 7, day 11 and day 16. Supernatant was analyzed on a CEDEX Bio-analyzer for concentrations of glucose, lactic acid, glutamine, glutamax, and ammonia.

L- glutamine is an unstable essential amino acid required in cell culture media formulations. Glutamine contains an amine, and this amide structural group can transport and deliver nitrogen to cells. When L-glutamine oxidizes, a toxic ammonia by-product is produced by the cell. To counteract the degradation of L-glutamine the media for the Gen 2 and Gen 3 processes was supplemented with Glutamax, which is more stable in aqueous solutions and does not spontaneously degrade. In the two tumor lines, the Gen 3 arm showed a decrease in L-glutamine and Glutamax during the process and an increase in ammonia throughout the REP. In the Gen 2 arm a constant concentration of L-glutamine and Glutamax, and a slight increase in the ammonia production was observed. The Gen 2 and Gen 3 processes were comparable at harvest day for ammonia and showed a slight difference in L-glutamine degradation. **See, Figure 10 (A, B, C).**

## TELOMERE REPEATS BY FLOW - FISH:

Flow-FISH technology was used to measure the average length of the telomere repeat on L4054 and L4055 under Gen 2 and Gen 3 process. The determination of a relative telomere length (RTL) was calculated using Telomere PNA kit/FITC for flow cytometry analysis from DAKO. Gen 3 showed comparable telomere length to Gen 2.

## CD3 Analysis

To determine the clonal diversity of the cell products generated in each process, TIL final product harvested for L4054 and L4055, were sampled and assayed for clonal diversity analysis through sequencing of the CDR3 portion of the T-cell receptors.

Table 41: Comparison of Gen 2 and Gen3 of percentage shared unique CDR3 sequences on L4054 on TIL harvested cell product. 199 sequences are shared between Gen 3 and Gen 2 final product, corresponding to 97.07% of top 80% of unique CDR3 sequences from Gen 2 shared with Gen 3 final product.

**Table 41: Comparison of shared uCDR3 sequences between Gen 2 and Gen 3 processes on L4054.**

| | All uCDR3's | | Top 80% uCDR3's | |
|---|---|---|---|---|
| **# uCDR3 (% Overlap)** | **Gen 2** | **Gen 3** | **Gen 2** | **Gen 3** |
| **Gen 2-L4054** | 8915 | 4355 **(48.85%)** | 205 | 199 **(97.07%)** |
| **Gen 3-L4054** | - | 18130 | - | 223 |

**Table 42: Comparison of Gen 2 and Gen3 of percentage shared unique CDR3 sequences on L4055 on TIL harvested cell product.** 1833 sequences are shared between Gen 3 and Gen 2 final product, corresponding to 99.45% of top 80% of unique CDR3 sequences from Gen 2 shared with Gen 3 final product.

**Table 42: Comparison of shared uCDR3 sequences between Gen 2 and Gen 3 processes on L4055.**

| | All uCDR3's | | Top 80% uCDR3's | |
|---|---|---|---|---|
| **# uCDR3 (% Overlap)** | **Gen 2** | **Gen 3** | **Gen 2** | **Gen 3** |
| **Gen 2-L4055** | 12996 | 6599 **(50.77%)** | 1843 | 1833 **(99.45%)** |
| **Gen 3-L4055** | - | 27246 | - | 2616 |

CM1 and CM2 media was prepared in advanced without filtration and held at 4°C until use for tumor L4055 to use on Gen 2 and Gen 3 process.

Media was warmed up at 37°C for 24 hours in advance for tumor L4055 on REP initiation day for Gen 2 and Gen 3 process.

LDH was not measured in the supernatants collected on the processes.

M1085T TIL cell count was executed with K2 cellometer cell counter.

On tumor M1085T, samples were not available such as supernatant for metabolic analysis, TIL product for activation and exhaustion markers analysis, telomere length and CD3 - TCR vb Analysis.

## CONCLUSIONS

This example compares 3 independent donor tumors tissue in terms of functional quality attributes, plus extended phenotypic characterization and media consumption among Gen 2 and Gen 3 processes.

Gen 2 and Gen 3 pre-REP and REP expansion comparison were evaluated in terms of total viable cells generated and viability of the total nucleated cell population. TVC cell doses at harvest day was not comparable between Gen 2 (22 days) and Gen 3 (16 days). Gen 3 cell doses were lower than Gen 2 at around 40% of total viable cells collected at harvest.

An extrapolated cell number was calculated for Gen 3 process assuming the pre-REP harvest occurred at day 11 instead day 7 and REP Harvest at Day 22 instead day 16. In both cases shows closer number on TVC compared to Gen 2 process, indicating that the early activation could allow an overall better performance on TIL growth. Table 4 and 5 bottom row.

In the case of extrapolated value for extra flasks (2 or 3) on Gen 3 process assuming a bigger size of tumor processed, and reaching the maximum number of fragments required per process as described. It was observed that a similar dose can be reachable on TVC at Day 16 Harvest for Gen 3 process compared to Gen 2 process at Day 22. This observation is important and indicates an early activation of the culture can allow better performance of TIL in less processing time.

Gen 2 and Gen 3 pre-REP and REP expansion comparison were evaluated in terms of total viable cells generated and viability of the total nucleated cell population. TVC cell doses at harvest day was not comparable between Gen 2 (22 days) and Gen 3 (16 days). Gen 3 cell doses were lower than Gen 2 at around 40% of total viable cells collected at harvest.

In terms of phenotypic characterization, a higher CD8+ and CD28+ expression was observed on three tumors on Gen 3 process compared to Gen 2 process. This data indicates the Gen 3 process has improved attributes of final TIL product compared to Gen 2.

Gen 3 process showed slightly higher central memory compartments compared to Gen 2 process.

Gen 2 and Gen 3 process showed comparable activation and exhaustion markers, despite the shorter duration of the Gen 3 process.

IFN gamma (IFNy) production was 3 times higher on Gen 3 final product compared to Gen 2 in the three tumors analyzed. This data indicates the Gen 3 process generated a highly functional and more potent TIL product as compared to the Gen 2 process, possibly due to the higher expression of CD8 and CD28 expression on Gen 3. Phenotypic characterization suggested positive trends in Gen 3 toward CD8+, CD28+ expression on three tumors compared to Gen 2 process.

Telomere length on TIL final product between Gen 2 and Gen 3 were comparable.

Glucose and Lactate levels were comparable between Gen 2 and Gen 3 final product, suggesting the levels of nutrients on the media of Gen 3 process were not affected due to the non-execution of volume reduction removal in each day of the process and less volume media overall in the process, compared to Gen 2.

Overall Gen 3 process showed a reduction almost two times of the processing time compared to Gen 2 process, which would yield a substantial reduction on the cost of goods (COGs) for TIL product expanded by the Gen 3 process.

IL-2 consumption indicates a general trend of IL-2 consumption on Gen 2 process, and in Gen 3 process IL-2 was higher due to the non-removal of the old media.

The Gen 3 process showed a higher clonal diversity measured by CDR3 TCRab sequence analysis.

The addition of feeders and OKT-3 on day 0 of the pre-REP allowed an early activation of TIL and overall a better growth TIL performance using the Gen 3 process.

Table 43 describes various embodiments and outcomes for the Gen 3 process as compared to the current Gen 2 process:

**Table 43: Exemplary Gen 3 process.**

| Step | Process Gen 2 | Process Gen 3 |
|---|---|---|
| **Pre REP-day 0** | ≤50 fragments<br>1X G-Rex 100MCS<br>1L media<br>IL-2 (6000IU/mL)<br>11 days | ≤240 fragments<br>≤60 fragments/flask<br>≤4 flasks<br>≤2L media (500mL/flask)<br>IL-2 (6000IU/mL)<br>$2.5 \times 10^8$ feeder cells/flask<br>15μg OKT3/flask |
| **REP Initiation** | Fresh TIL direct to REP<br>Day 11<br>≤$200 \times 10^6$ viable cells<br>$5 \times 10^9$ feeder cells<br>G-Rex 500MCS<br>5L CM2 media + IL-2 (3000 IU/mL)<br>150μg OKT3 | Fresh TIL direct to REP<br>Day 7<br>Activate entire culture<br>$5 \times 10^8$ feeder cells<br>30 μg OKT3/flask<br>G-Rex 100MCS<br>500mL media+ IL-2(6000IU/mL) |
| **TIL Subculture or Scale up** | ≤5 G-REX 500MCS<br>≤1 × 10 viable cells/ flask<br>SL/flask<br>Day 16 | ≤4 G-REX 500MCS<br>Scale up entire culture<br>4L/flask<br>Day 10-11 |
| **Harvest** | Harvest Day 22, LOVO-automated cell washer 2 wash cycles | Harvest Day 16 LOVO- automated cell washer 5 wash cycles |
| **Final formulation** | Cryopreserved Product 300IU/mL IL2- CS10 in $LN_2$, multiple aliquots | Cryopreserved product 300IU/mL IL-2-CS10 in $LN_2$, multiple aliquots |
| **Process time** | **22 days** | **16 days** |

TILs have been successfully expanded from core biopsies isolated from pancreatic tumors. TILs were also expanded from FNA's from certain tumor types. In the study no TIL growth was observed from ovarian tumors (n=3) and a colorectal tumor (n=1). TIL growth was observed from FNA's obtained from a lung tumor, melanoma, and a cervical tumor. Phenotyping and functional assays of the population of TILs from the first expansion and from the second expansion are currently in progress. The data from the study shows that a significant population of CD4+ and CD8+ T cells are present in the pre-REP aspirate.

## Claims

**1.** A method for expanding tumor infiltrating lymphocytes (TILs) into a therapeutic population of TILs comprising:

(i) performing a first expansion by culturing a first population of TILs from at least one fine needle aspirate (FNA) or at least one small biopsy from a tumor in a patient in a cell culture medium comprising IL-2 to produce a second population of TILs, wherein

the cell culture medium is supplemented with OKT-3 at any one of days 1-3, wherein the first expansion is performed for 3 days to 12 days in order
to obtain the second population of TILs, wherein the second population of TILs comprises at least $5 \times 10^7$ TILs by 3 days to 12 days when the first population of TILs is from a small biopsy; and

(ii) performing a second expansion by supplementing the cell culture medium of the second population of TILs with additional IL-2, OKT-3, and antigen presenting cells (APCs), to produce a third population of TILs, wherein the third population of TILs is at least 25-fold greater in number than the second population of TILs, and wherein the second expansion is performed for 3 days to 12 days in order to obtain the third population of TILs, wherein the third population of TILs is a therapeutic population of TILs.

2. The method according to claim 1, wherein the cell culture medium comprising IL-2 in step (i) further comprises OKT-3 and is not optionally supplemented with OKT-3 at any one of days 1-3, wherein step (i) is a priming first expansion step and step (ii) is a rapid second expansion, wherein optionally after step (ii), the cells are removed from the cell culture and cryopreserved in a storage medium.

3. The method of according to any of the preceding claims, wherein steps (i) through (ii) are performed within a period of 17 days to 24 days, optionally within a period of 18 days to 22 days, further optionally within a period of 20 days to 22 days, and further optionally within a period of 22 days.

4. The method according to any of the preceding claims, wherein the cells in the therapeutic population of TILs from step (ii) express CD4, CD8, and TCR $\alpha$ $\beta$ at levels similar to freshly harvested cells.

5. The method according to claim 1, wherein the APCs are peripheral blood mononuclear cells (PBMCs), wherein optionally the PBMCs are added to the cell culture in step (i) on any of days 3 through 12 after initiation of the culture in step (i) and/or in step (ii) on any of days 11 through 14 after initiation of the culture in step (i), further optionally wherein the APCs are artificial APCs (aAPCs) or autologous APCs.

6. The method according to claims 2 to 5, wherein the third population of TILs comprises an increased subpopulation of effector T cells and/or central memory T cells relative to the second population of TILs, wherein the effector T cells and/or central memory T cells in the therapeutic population of TILs in step (ii) exhibit one or more characteristics selected from the group consisting of expression of CD27, expression of CD28, longer telomeres, increased CD57 expression, and decreased CD56 expression, relative to effector T cells and/or central memory T cells in the second population of cells, wherein optionally the effector T cells and/or central memory T cells exhibit increased CD57 expression and decreased CD56 expression.

7. The method according to any of the preceding claims, wherein the therapeutic population of TILs are for infusion into a patient.

8. The method according to claim 1, wherein the first expansion in step (i) is performed by further supplementing the cell culture medium of the second population of TILs with OKT-3, IL-15, OX40 agonistic antibody and/or 4-1BB agonistic antibody and/or the second expansion in step (ii) is performed by further supplementing the cell culture medium of the second population of TILs with IL-15, OX40 agonistic antibody and/or 4-1BB agonistic antibody.

9. The method according to claim 1, wherein the FNA in step (i) comprises at least 400,000 TILs.

10. The method according to claim 1, wherein the small biopsy is obtained from a tumor selected from the group consisting of pancreatic, melanoma, breast, and ovarian and optionally wherein the FNA is obtained from a tumor selected from the group consisting of lung, melanoma, head and neck, cervical, ovarian, pancreatic, glioblastoma, colorectal, and sarcoma, wherein optionally the lung tumor is a non-small cell lung carcinoma (NSCLC), and optionally wherein the patient has previously undergone surgical treatment.

11. The method according to claim 1, wherein the first population of TILs in step (i) are obtained from a FNA, wherein optionally the FNA is obtained using a 25-18 gauge needle.

12. The method according to claim 1, wherein the first population of TILs in step (i) are obtained from a small biopsy, and are optionally obtained from a core biopsy, and further optionally wherein the small biopsy is obtained using a 16-11 gauge needle.

13. The method according to claim 1, wherein step (ii) is repeated one to four times in order to obtain sufficient TILs in the therapeutic population of TILs for a therapeutically effective dosage of the TILs, optionally where the number of TILs sufficient for a therapeutically effective dosage is from $2.3\times10^{10}$ to $13.7\times10^{10}$.

14. The method according to any preceding claim, wherein the first expansion is performed for 3 days to 10 days.

15. The method according to claim 14, which further comprises an additional second expansion in step (ii) which is performed by further supplementing the cell culture medium of the third population of TILs with IL-15, OX40 agonistic antibody and/or 4-IBB agonistic antibody.

16. The method according to any of claims 1 to 15, which comprises:

(a) adding the first population of TILs into a closed system;
(b) performing the first expansion by culturing the first population of TILs in a cell culture medium comprising IL-2 to produce a second population of TILs, wherein the cell culture medium is supplemented with OKT-3 at any one of days 1-3, wherein the first expansion is performed for 3 days to 12 days in order to obtain the second population of TILs, wherein the second population of TILs comprises at least $5 \times 10^7$ TILs by 3 days to 12 days when the first population of TILs is from a small biopsy, wherein the first expansion is performed in a closed container providing a first gas-permeable surface area, wherein the second population of TILs is at least 25-fold greater in number than the first population of TILs, and wherein the transition from step (a) to step (b) occurs without opening the system;
(c) performing the second expansion by supplementing the cell culture medium of the second population of TILs with additional IL-2, OKT-3, and antigen presenting cells (APCs), to produce a third population ofTILs, wherein the second expansion is performed for 3 days to 12 days to obtain the third population of TILs, wherein the third population of TILs is a therapeutic population ofTILs, wherein the second expansion is performed in a closed container providing a second gas-permeable surface area, and wherein the transition from step (b) to step (c) occurs without opening the system;
(d) harvesting the therapeutic population of TILs obtained from step (c), wherein the transition from step (c) to step (d) occurs without opening the system; and
(e) transferring the harvested TIL population from step (d) to an infusion bag, wherein the transfer from step (d) to (e) occurs without opening the system.

17. The method according to claim 16, wherein the cell culture medium comprising IL-2 in step (b) further comprises OKT-3 and is not optionally supplemented with OKT-3 at any one of days 1-3, and wherein step (b) is a priming first expansion step and step (c) is a rapid second expansion step, wherein optionally the method further comprises the step of cryopreserving the infusion bag comprising the harvested TIL population in step (e) using a cryopreservation process.

18. The method according to any one of claims 16 or 17, wherein the APCs are peripheral blood mononuclear cells (PBMCs) wherein optionally the PBMCs are irradiated and allogeneic and optionally, wherein the PBMCs are added to the cell culture on any of days 3 through 12 of the first period in step (b) and/or any of days 3 through 12 of the second period in step (c).

19. The method according to claim 16, wherein the first expansion in step (b) is performed by further supplementing the cell culture medium of the second population of TILs with OKT-3, IL-15, OX40 agonistic antibody and/or 4-1BB agonistic antibody and/or wherein the second expansion in step (c) is performed by further supplementing the cell culture medium of the second population of TILs with IL-15, OX40 agonistic antibody and/or 4-1BB agonistic antibody.

20. The method according to claim 16, wherein the TILs in step (a) are obtained from a FNA, and optionally wherein the FNA in step (a) comprises at least 400,000 TILs further optionally wherein the FNA is obtained using a 25-18 gauge needle, and further optionally wherein the FNA is obtained from a tumor selected from the group consisting of lung, melanoma, head and neck, cervical, ovarian, pancreatic, glioblastoma, colorectal, and sarcoma, wherein optionally the lung tumor is a non-small cell lung carcinoma (NSCLC), and optionally wherein the subject has previously undergone surgical treatment.

21. The method according to claim 16, wherein the first population of TILs in step (a) are obtained from a small biopsy and optionally wherein the small biopsy is obtained from a tumor selected from the group consisting of pancreatic, melanoma, breast and ovarian, and further optionally wherein the small biopsy is obtained using a 16-11 gauge needle.

22. The method according to claim 16, wherein the harvesting in step (d) is performing using a LOVO® cell processing system, wherein optionally the cell culture medium is provided in a container selected from the group consisting of a G-container™ and a Xuri™ cellbag and further optionally wherein the infusion bag in step (e) is a hypothermosol infusion bag.

23. The method according to claim 16, wherein steps (a) through (e) are performed within a period of 17 days to 24 days, optionally wherein steps (a) through (e) are performed within a period of 18 days to 22 days, further optionally wherein steps (a) through (e) are performed within a period of 20 days to 22 days.

**24.** The method according to claim 16, wherein steps (a) through (e) and optionally cryopreservation are performed in 22 days or less.

**25.** The method according to any one of claims 16 to 24, wherein the therapeutic population of TILs harvested in step (d) comprises sufficient TILs for a therapeutically effective dosage of the TILs, optionally wherein the number of TILs sufficient for a therapeutically effective dosage is from $2.3 \times 10^{10}$ to $13.7 \times 10^{10}$.

**26.** The method according to any one of claims 16 to 25, wherein steps (a) through (d) are performed in a single container, wherein performing steps (a) through (d) in a single container results in an increase in TIL yield per resected tumor as compared to performing steps (a) through (d) in more than one container,
optionally wherein the antigen-presenting cells are added to the TILs during the second expansion in step (c) without opening the system, wherein optionally the third population of TILs comprises an increased subpopulation of effector T cells and/or central memory T cells relative to the second population of TILs, wherein the effector T cells and/or central memory T cells in the therapeutic population of TILs exhibit one or more characteristics selected from the group consisting of expressing CD27+, expressing CD28+, longer telomeres, increased CD57 expression, and decreased CD56 expression relative to effector T cells, and/or central memory T cells obtained from the second population of cells, and further optionally wherein the effector T cells and/or central memory T cells obtained from the third population of TILs exhibit increased CD57 expression and decreased CD56 expression relative to effector T cells and/or central memory T cells obtained from the second population of cells.

## Patentansprüche

**1.** Verfahren zum Expandieren von tumorinfiltrierenden Lymphozyten (TILs) in eine therapeutische Population von TILs, umfassend:

(i) Durchführen einer ersten Expansion durch Kultivieren einer ersten Population von TILs aus mindestens einem Feinnadelaspirat (FNA) oder mindestens einer kleinen Biopsie aus einem Tumor bei einem Patienten in einem Zellkulturmedium, umfassend IL-2, um eine zweite Population von TILs zu erzeugen, wobei das Zellkulturmedium an einem der Tage 1-3 mit OKT-3 ergänzt wird, wobei die erste Expansion über 3 Tage bis 12 Tage durchgeführt wird, um die zweite Population von TILs zu erlangen, wobei die zweite Population von TILs nach 3 Tagen bis 12 Tagen mindestens $5 \times 10^7$ TILs umfasst, wenn die erste Population von TILs aus einer kleinen Biopsie ist; und
(ii) Durchführen einer zweiten Expansion durch Ergänzen des Zellkulturmediums der zweiten Population von TILs mit zusätzlichem IL-2, OKT-3 und antigenpräsentierenden Zellen (APCs), um eine dritte Population von TILs zu erzeugen, wobei die dritte Population von TILs in ihrer Anzahl mindestens 25-fach größer ist als die zweite Population von TILs, und wobei die zweite Expansion über 3 Tage bis 12 Tage durchgeführt wird, um die dritte Population von TILs zu erlangen, wobei die dritte Population von TILs eine therapeutische Population von TILs ist.

**2.** Verfahren nach Anspruch 1, wobei das Zellkulturmedium, das IL-2 umfasst, in Schritt (i) ferner OKT-3 umfasst und an einem der Tage 1-3 nicht optional mit OKT-3 ergänzt wird, wobei Schritt (i) ein erster Priming-Expansionsschritt ist und Schritt (ii) eine schnelle zweite Expansion ist, wobei optional nach Schritt (ii) die Zellen aus der Zellkultur entfernt und in einem Speichermedium kryokonserviert werden.

**3.** Verfahren nach einem der vorhergehenden Ansprüche, wobei Schritt (i) bis (ii) innerhalb eines Zeitraums von 17 Tagen bis 24 Tagen, optional innerhalb eines Zeitraums von 18 Tagen bis 22 Tagen, ferner optional innerhalb eines Zeitraums von 20 Tagen bis 22 Tagen und ferner optional innerhalb eines Zeitraums von 22 Tagen durchgeführt werden.

**4.** Verfahren nach einem der vorhergehenden Ansprüche, wobei die Zellen in der therapeutischen Population von TILs aus Schritt (ii) CD4, CD8 und TCR $\alpha\beta$ in ähnlichen Mengen exprimieren wie frisch geerntete Zellen.

**5.** Verfahren nach Anspruch 1, wobei die APCs periphere mononukleäre Blutzellen (PBMCs) sind, wobei optional die PBMCs der Zellkultur in Schritt (i) an einem der Tage 3 bis 12 nach Beginn der Kultur in Schritt (i) und/oder in Schritt (ii) an einem der Tage 11 bis 14 nach Beginn der Kultur in Schritt (i) hinzugefügt werden, ferner optional wobei die APCs künstliche APCs (aAPCs) oder autologe APCs sind.

6. Verfahren nach einem der Ansprüche 2 bis 5, wobei die dritte Population von TILs eine erhöhte Subpopulation von Effektor-T-Zellen und/oder zentralen Gedächtnis-T-Zellen in Bezug auf die zweite Population von TILs umfasst, wobei die Effektor-T-Zellen und/oder zentralen Gedächtnis-T-Zellen in der therapeutischen Population von TILs in Schritt (ii) ein oder mehrere Merkmale aufweisen, die ausgewählt sind aus der Gruppe, bestehend aus Expression von CD27, Expression von CD28, längeren Telomeren, erhöhter CD57-Expression und verringerter CD56-Expression, in Bezug auf Effektor-T-Zellen und/oder zentrale Gedächtnis-T-Zellen in der zweiten Zellpopulation, wobei optional die Effektor-T-Zellen und/oder zentralen Gedächtnis-T-Zellen eine erhöhte CD57-Expression und eine verringerte CD56-Expression aufweisen.

7. Verfahren nach einem der vorhergehenden Ansprüche, wobei die therapeutische Population von TILs zur Infusion bei einem Patienten bestimmt ist.

8. Verfahren nach Anspruch 1, wobei die erste Expansion in Schritt (i) durch weiteres Ergänzen des Zellkulturmediums der zweiten Population von TILs mit OKT-3, IL-15, agonistischen OX40-Antikörper und/oder agonistischen 4-1BB-Antikörper durchgeführt wird und/oder die zweite Expansion in Schritt (ii) durch weiteres Ergänzen des Zellkulturmediums der zweiten Population von TILs mit IL-15, agonistischen OX40-Antikörper und/oder agonistischen 4-1BB-Antikörper durchgeführt wird.

9. Verfahren nach Anspruch 1, wobei das FNA in Schritt (i) mindestens 400.000 TILs umfasst.

10. Verfahren nach Anspruch 1, wobei die kleine Biopsie aus einem Tumor erlangt wird, der ausgewählt ist aus der Gruppe, bestehend aus Pankreas, Melanom, Brust und Eierstock, und optional wobei das FNA aus einem Tumor erlangt wird, der ausgewählt ist aus der Gruppe, bestehend aus Lunge, Melanom, Kopf und Hals, Gebärmutterhals, Eierstock, Pankreas, Glioblastom, kolorektal und Sarkom, wobei optional der Lungentumor ein nicht-kleinzelliges Lungenkarzinom (NSCLC) ist, und optional wobei sich der Patient zuvor einer chirurgischen Behandlung unterzogen hat.

11. Verfahren nach Anspruch 1, wobei die erste Population von TILs in Schritt (i) aus einem FNA erlangt wird, wobei optional das FNA unter Verwendung einer 25-18 Gauge-Nadel erlangt wird.

12. Verfahren nach Anspruch 1, wobei die erste Population von TILs in Schritt (i) aus einer kleinen Biopsie erlangt wird und optional aus einer Kernbiopsie erlangt wird, und ferner optional wobei die kleine Biopsie unter Verwendung einer 16-11 Gauge-Nadel erlangt wird.

13. Verfahren nach Anspruch 1, wobei Schritt (ii) ein- bis viermal wiederholt wird, um ausreichende TILs, in der therapeutischen Population von TILs, für eine therapeutisch wirksame Dosierung der TILs, zu erlangen, optional wobei die Anzahl von TILs, die für eine therapeutisch wirksame Dosierung ausreichend ist, $2,3 \times 10^{10}$ bis $13,7 \times 10^{10}$ ist.

14. Verfahren nach einem der vorhergehenden Ansprüche, wobei die erste Expansion über 3 Tage bis 10 Tage durchgeführt wird.

15. Verfahren nach Anspruch 14, das ferner eine zusätzliche zweite Expansion in Schritt (ii) umfasst, die durch weiteres Ergänzen des Zellkulturmediums der dritten Population von TILs mit IL-15, einem agonistischen OX40-Antikörper und/oder einem agonistischen 4-1BB-Antikörper durchgeführt wird.

16. Verfahren nach einem der Ansprüche 1 bis 15, das Folgendes umfasst:

(a) Hinzufügen der ersten Population von TILs in ein geschlossenes System;
(b) Durchführen der ersten Expansion durch Kultivieren der ersten Population von TILs in einem Zellkulturmedium, umfassend IL-2, um eine zweite Population von TILs zu erzeugen, wobei das Zellkulturmedium an einem der Tage 1-3 mit OKT-3 ergänzt wird, wobei die erste Expansion über 3 Tage bis 12 Tage durchgeführt wird, um die zweite Population von TILs zu erlangen, wobei die zweite Population von TILs nach 3 Tagen bis 12 Tagen mindestens $5 \times 10^7$ TILs umfasst, wenn die erste Population von TILs aus einer kleinen Biopsie ist, wobei die erste Expansion in einem geschlossenen Behälter durchgeführt wird, der einen ersten gasdurchlässigen Oberflächenbereich bereitstellt, wobei die zweite Population von TILs mindestens 25-mal größer ist als die erste Population von TILs, und wobei der Übergang von Schritt (a) zu Schritt (b) ohne Öffnen des Systems erfolgt;
(c) Durchführen der zweiten Expansion durch Ergänzen des Zellkulturmediums der zweiten Population von TILs mit zusätzlichem IL-2, OKT-3 und antigenpräsentierenden Zellen (APCs), um eine dritte Population von TILs zu erzeugen, wobei die zweite Expansion über 3 Tage bis 12 Tage durchgeführt wird, um die dritte Population von TILs zu erlangen, wobei die dritte Population von TILs eine therapeutische Population von TII,s ist, wobei die zweite Expansion in einem geschlossenen Behälter durchgeführt wird, der eine zweite gasdurchlässige Oberfläche bereitstellt, und wobei der Übergang von Schritt (b) zu Schritt (c) ohne Öffnen des Systems erfolgt;
(d) Ernten der therapeutischen Population von TILs, die von Schritt (c) erlangt werden, wobei der Übergang von Schritt (c) zu Schritt (d) ohne Öffnen des Systems erfolgt; und
(e) Übertragen der geernteten Population von TIL von Schritt (d) in einen Infusionsbeutel, wobei der Übergang von Schritt (d) zu (e) ohne Öffnen des Systems erfolgt.

**17.** Verfahren nach Anspruch 16, wobei das Zellkulturmedium, umfassend IL-2, in Schritt (b) ferner OKT-3 umfasst und an einem der Tage 1-3 nicht optional mit OKT-3 ergänzt wird, und wobei Schritt (b) ein erster Priming-Expansionsschritt ist und Schritt (c) ein schneller zweiter Expansionsschritt ist, wobei optional das Verfahren ferner den Schritt eines Kryokonservierens des Infusionsbeutels, umfassend die geerntete Population von TIL, in Schritt (e) unter Verwendung eines Kryokonservierungsvorgangs umfasst.

**18.** Verfahren nach einem der Ansprüche 16 oder 17, wobei die APCs periphere mononukleäre Blutzellen (PBMCs) sind, wobei optional die PBMCs bestrahlt und allogen sind und optional wobei die PBMCs der Zellkultur an einem der Tage 3 bis 12 des ersten Zeitraums in Schritt (b) und/oder an einem der Tage 3 bis 12 des zweiten Zeitraums in Schritt (c) hinzugefügt werden.

**19.** Verfahren nach Anspruch 16, wobei die erste Expansion in Schritt (b) durch weiteres Ergänzen des Zellkulturmediums der zweiten Population von TILs mit OKT-3, IL-15, agonistischen OX40-Antikörper und/oder agonistischen 4-1BB-Antikörper durchgeführt wird und/oder wobei die zweite Expansion in Schritt (c) durch weiteres Ergänzen des Zellkulturmediums der zweiten Population von TILs mit IL-15, agonistischen OX40-Antikörper und/oder agonistischen 4-1BB-Antikörper durchgeführt wird.

**20.** Verfahren nach Anspruch 16, wobei die TILs in Schritt (a) aus einem FNA erlangt werden, und optional wobei das FNA in Schritt (a) mindestens 400.000 TILs umfasst, ferner optional wobei das FNA unter Verwendung einer 25-18 Gauge-Nadel erlangt wird, und ferner optional wobei das FNA aus einem Tumor erlangt wird, der ausgewählt ist aus der Gruppe, bestehend aus Lunge, Melanom, Kopf und Hals, Gebärmutterhals, Eierstock, Pankreas, Glioblastom, kolorektal und Sarkom, wobei optional der Lungentumor nicht-kleinzelliges Lungenkarzinom (NSCLC) ist, und optional wobei sich das Subjekt zuvor einer chirurgischen Behandlung unterzogen hat.

**21.** Verfahren nach Anspruch 16, wobei die erste Population von TILs in Schritt (a) aus einer kleinen Biopsie erlangt wird und optional wobei die kleine Biopsie aus einem Tumor erlangt wird, der ausgewählt ist aus der Gruppe, bestehend aus Pankreas, Melanom, Brust und Eierstock, und ferner optional wobei die kleine Biopsie unter Verwendung einer 16-11 Gauge-Nadel erlangt wird.

**22.** Verfahren nach Anspruch 16, wobei das Ernten in Schritt (d) unter Verwendung eines Zellverarbeitungssystems LOVO® durchgeführt wird, wobei optional das Zellkulturmedium in einem Behälter bereitgestellt ist, der ausgewählt ist aus der Gruppe, bestehend aus einem G-Container™ und einem Zellbeutel Xuri™, und ferner optional wobei der Infusionsbeutel in Schritt (e) ein Hypothermosol-Infusionsbeutel ist.

**23.** Verfahren nach Anspruch 16, wobei die Schritte (a) bis (e) innerhalb eines Zeitraums von 17 Tagen bis 24 Tagen durchgeführt werden, optional wobei die Schritte (a) bis (e) innerhalb eines Zeitraums von 18 Tagen bis 22 Tagen durchgeführt werden, ferner optional wobei die Schritte (a) bis (e) innerhalb eines Zeitraums von 20 Tagen bis 22 Tagen durchgeführt werden.

**24.** Verfahren nach Anspruch 16, wobei die Schritte (a) bis (e) und optional eine Kryokonservierung in 22 Tagen oder weniger durchgeführt werden.

**25.** Verfahren nach einem der Ansprüche 16 bis 24, wobei die therapeutische Population von TILs, die in Schritt (d) geerntet wird, ausreichend TILs für eine therapeutisch wirksame Dosierung der TILs umfasst, optional wobei die Anzahl an TILs, die für eine therapeutisch wirksame Dosierung ausreichend ist, von $2{,}3\times10^{10}$ bis $13{,}7\times10^{10}$ ist.

**26.** Verfahren nach einem der Ansprüche 16 bis 25, wobei die Schritte (a) bis (d) in einem einzigen Behälter durchgeführt werden, wobei ein Durchführen von Schritt (a) bis (d) in einem einzigen Behälter in einer Erhöhung von TIL-Ertrag pro reseziertem Tumor resultiert, im Vergleich zu einem Durchführen von Schritt (a) bis (d) in mehr als einem Behälter, optional wobei die antigenpräsentierenden Zellen während der zweiten Expansion in Schritt (c) zu den TILs hinzugefügt werden, ohne das System zu öffnen, wobei optional die dritte Population von TILs eine erhöhte Subpopulation von Effektor-T-Zellen und/oder zentralen Gedächtnis-T-Zellen in Bezug auf die zweite Population von TILs umfasst, wobei die Effektor-T-Zellen und/oder zentralen Gedächtnis-T-Zellen in der therapeutischen Population von TILs eine oder mehrere Eigenschaften aufweisen, die ausgewählt sind aus der Gruppe, bestehend aus der Expression von CD27+, der Expression von CD28+, längeren Telomeren, erhöhter CD57-Expression und verringerter CD56-Expression in Bezug auf Effektor-T-Zellen und/oder zentrale Gedächtnis-T-Zellen, die aus der zweiten Population von Zellen erlangt werden, und ferner optional wobei die Effektor-T-Zellen und/oder zentralen Gedächtnis-T-Zellen, die aus der dritten Population von TILs erlangt werden, eine erhöhte CD57-Expression und eine verringerte CD56-Expression in Bezug auf Effektor-T-Zellen und/oder zentrale Gedächtnis-T-Zellen, die aus der zweiten Population von Zellen erlangt werden, aufweisen.

**Revendications**

**1.** Méthode permettant l'expansion des lymphocytes infiltrant les tumeurs (TIL) dans une population thérapeutique de TIL comprenant :

(i) la réalisation d'une première expansion en cultivant une première population de TIL provenant d'au moins un produit d'aspiration d'aiguille fine (FNA) ou d'au moins une petite biopsie provenant d'une tumeur chez un patient dans un milieu de culture cellulaire comprenant IL-2 pour produire une deuxième population de TIL, ledit milieu de culture cellulaire étant complémenté avec OKT-3 à l'un quelconque des jours 1 à 3, ladite première expansion étant effectuée pendant 3 jours à 12 jours afin d'obtenir la deuxième population de TIL, ladite deuxième population de TIL comprenant au moins $5 \times 10^7$ TIL en 3 jours à 12 jours lorsque la première population de TIL provient d'une petite biopsie ; et
(ii) la réalisation d'une seconde expansion par complémentation du milieu de culture cellulaire de la deuxième population de TIL avec un supplément d'IL-2, de l'OKT-3 et des cellules présentatrices d'antigène (APC), pour produire une troisième population de TIL, ladite troisième population de TIL étant au moins 25 fois plus grande en nombre que la deuxième population de TIL, et ladite seconde expansion étant effectuée pendant 3 jours à 12 jours afin d'obtenir la troisième population de TIL, ladite troisième population de TIL étant une population thérapeutique de TIL.

**2.** Méthode selon la revendication 1, ledit milieu de culture cellulaire comprenant de l'IL-2 à l'étape (i) comprenant en outre de l'OKT-3 et n'étant éventuellement pas complémenté avec de l'OKT-3 à l'un quelconque des jours 1 à 3, ladite étape (i) étant une étape de première expansion d'amorçage et ladite étape (ii) étant une seconde expansion rapide, éventuellement après l'étape (ii), lesdites cellules étant retirées de la culture cellulaire et cryoconservées dans un milieu de stockage.

**3.** Méthode selon l'une quelconque des revendications précédentes, lesdites étapes (i) à (ii) étant effectuées dans un délai de 17 jours à 24 j ours, éventuellement dans un délai de 18 jours à 22 jours, en outre éventuellement dans un délai de 20 jours à 22 jours, et éventuellement en outre dans un délai de 22 jours.

**4.** Méthode selon l'une quelconque des revendications précédentes, lesdites cellules dans la population thérapeutique de TIL de l'étape (ii) exprimant CD4, CD8 et TCR $\alpha \beta$ à des niveaux similaires à des cellules fraîchement récoltées.

**5.** Méthode selon la revendication 1, lesdites APC étant des cellules mononucléaires de sang périphérique (PBMC), éventuellement lesdites PBMC étant ajoutées à la culture cellulaire à l'étape (i) à l'un quelconque des jours 3 à 12 après initiation de la culture à l'étape (i) et/ou à l'étape (ii) à l'un quelconque des jours 11 à 14 après initiation de la culture à l'étape (i), en outre éventuellement lesdites APC étant des APC artificielles (aAPC) ou des APC autologues.

**6.** Méthode selon les revendications 2 à 5, ladite troisième population de TIL comprenant une sous-population accrue de lymphocytes T effecteurs et/ou de lymphocytes T à mémoire centrale par rapport à la deuxième population de TIL, lesdits lymphocytes T effecteurs et/ou lymphocytes T à mémoire centrale dans la population thérapeutique de TIL à l'étape (ii) présentant une ou plusieurs caractéristiques choisies dans le groupe constitué par l'expression de CD27, l'expression de CD28, des télomères plus longs, l'expression de CD57 accrue et l'expression de CD56 réduite, par rapport aux lymphocytes T effecteurs et/ou aux lymphocytes T à mémoire centrale dans la deuxième population de cellules, éventuellement lesdits lymphocytes T effecteurs et/ou lymphocytes T à mémoire centrale présentant une expression de CD57 accrue et une expression de CD56 réduite.

**7.** Méthode selon l'une quelconque des revendications précédentes, ladite population thérapeutique de TIL étant destinée à une perfusion à un patient.

**8.** Méthode selon la revendication 1, ladite première expansion à l'étape (i) étant effectuée par complémentation supplémentaire du milieu de culture cellulaire de la deuxième population de TIL avec OKT-3, IL-15, un anticorps agoniste d'OX40 et/ou un anticorps agoniste de 4-1BB et/ou ladite seconde expansion à l'étape (ii) étant effectuée par complémentation supplémentaire du milieu de culture cellulaire de la deuxième population de TIL avec IL-15, un anticorps agoniste d'OX40 et/ou un anticorps agoniste de 4-1BB.

**9.** Méthode selon la revendication 1, ledit FNA à l'étape (i) comprenant au moins 400 000 TIL.

**10.** Méthode selon la revendication 1, ladite petite biopsie étant obtenue à partir d'une tumeur choisie dans le groupe constitué par les tumeurs du pancréas, de mélanome, du sein et de l'ovaire et éventuellement ledit FNA étant obtenu à partir d'une tumeur choisie dans le groupe constitué par les tumeurs du poumon, de mélanome, de la tête et du cou, du col de l'utérus, de l'ovaire, du pancréas, de glioblastome, colorectales et de sarcome, éventuellement ladite tumeur du poumon étant un carcinome pulmonaire non à petites cellules (NSCLC), et éventuellement ledit patient ayant précédemment subi un traitement chirurgical.

**11.** Méthode selon la revendication 1, ladite première population de TIL à l'étape (i) étant obtenue à partir d'un FNA, éventuellement ledit FNA étant obtenu en utilisant une aiguille de calibre 25 à 18.

**12.** Méthode selon la revendication 1, ladite première population de TIL à l'étape (i) étant obtenue à partir d'une petite biopsie, et étant éventuellement obtenue à partir d'une biopsie au trocart, et en outre éventuellement ladite petite biopsie étant obtenue en utilisant une aiguille de calibre 16 à 11.

**13.** Méthode selon la revendication 1, ladite étape (ii) étant répétée une à quatre fois afin d'obtenir suffisamment de TIL dans la solution thérapeutique de TIL pour un dosage thérapeutiquement efficace des TIL, éventuellement le nombre de TIL suffisant pour un dosage thérapeutiquement efficace allant de $2{,}3 \times 10^{10}$ à $13{,}7 \times 10^{10}$.

**14.** Méthode selon l'une quelconque des revendications précédentes, ladite première expansion étant effectuée pendant 3 jours à 10 jours.

**15.** Méthode selon la revendication 14, qui comprend en outre une seconde expansion supplémentaire à l'étape (ii) qui est effectuée par complémentation supplémentaire du milieu de culture cellulaire de la troisième population de TIL avec IL-15, un anticorps agoniste d'OX40 et/ou un anticorps agoniste de 4-1BB.

**16.** Méthode selon l'une quelconque des revendications 1 à 15, qui comprend :

(a) l'ajout de la première population de TIL dans un système fermé ;
(b) la réalisation de la première expansion en cultivant la première population de TIL dans un milieu de culture cellulaire comprenant IL-2 pour produire une deuxième population de TIL, ledit milieu de culture cellulaire étant complémenté avec OKT-3 à l'un quelconque des jours 1 à 3, ladite première expansion étant effectuée pendant 3 jours à 12 jours afin d'obtenir la deuxième population de TIL, ladite deuxième population de TIL comprenant au moins $5 \times 10^7$ TIL en 3 jours à 12 jours lorsque la population de TIL provient d'une petite biopsie, ladite première expansion étant effectuée dans un récipient fermé fournissant une première surface perméable aux gaz, ladite deuxième population de TIL étant au moins 25 fois plus grande en nombre que la première population de TIL, et ladite transition de l'étape (a) à l'étape (b) ayant lieu sans ouvrir le système ;
(c) la réalisation de la seconde expansion par complémentation du milieu de culture cellulaire de la deuxième population de TIL avec un supplément d'IL-2, de l'OKT-3 et des cellules présentatrices d'antigène (APC), pour produire une troisième population de TIL, ladite seconde expansion étant effectuée pendant 3 jours à 12 jours pour obtenir la troisième population de TIL, ladite troisième population de TIL étant une population thérapeutique de TIL, ladite seconde expansion étant effectuée dans un récipient fermé fournissant une seconde surface perméable aux gaz, et la transition de l'étape (b) à l'étape (c) ayant lieu sans ouvrir le système ;
(d) la récolte de la population thérapeutique de TIL obtenue à partir de l'étape (c), la transition de l'étape (c) à l'étape (d) ayant lieu sans ouvrir le système ; et

(e) le transfert de la population de TIL récoltée à partir de l'étape (d) dans une poche de perfusion, le transfert de l'étape (d) à l'étape (e) ayant lieu sans ouvrir le système.

**17.** Méthode selon la revendication 16, ledit milieu de culture cellulaire comprenant de l'IL-2 à l'étape (b) comprenant en outre de l'OKT-3 et n'étant pas éventuellement complémenté avec de l'OKT-3 à l'un quelconque des jours 1 à 3, et ladite étape (b) étant une étape de première expansion d'amorçage et ladite étape (c) étant une étape de seconde expansion rapide, éventuellement ladite méthode comprenant en outre l'étape de cryoconservation de la poche de perfusion comprenant la population de TIL récoltée à l'étape (e) en utilisant un processus de cryoconservation.

**18.** Méthode selon l'une quelconque des revendications 16 ou 17, lesdites APC étant des cellules mononucléaires de sang périphérique (PBMC), éventuellement lesdites PBMC étant irradiées et allogènes et éventuellement, lesdites PBMC étant ajoutées à la culture cellulaire à l'un quelconque des jours 3 à 12 de la première période à l'étape (b) et/ou à l'un quelconque des jours 3 à 12 de la seconde période à l'étape (c).

**19.** Méthode selon la revendication 16, ladite première expansion à l'étape (b) étant effectuée par complémentation supplémentaire du milieu de culture cellulaire de la deuxième population de TIL avec OKT-3, IL-15, un anticorps agoniste d'OX40 et/ou un anticorps agoniste de 4-1BB et/ou ladite seconde expansion à l'étape (c) étant effectuée par complémentation supplémentaire du milieu de culture cellulaire de la deuxième population de TIL avec IL-15, un anticorps agoniste d'OX40 et/ou un anticorps agoniste de 4-1BB.

**20.** Méthode selon la revendication 16, lesdits TIL à l'étape (a) étant obtenus à partir d'un FNA, et éventuellement ledit FNA à l'étape (a) comprenant au moins 400 000 TIL, en outre éventuellement ledit FNA étant obtenu en utilisant un aiguille de calibre 25 à 18, et en outre éventuellement ledit FNA étant obtenu à partir d'une tumeur choisie dans le groupe constitué par les tumeurs du poumon, de mélanome, de la tête et du cou, du col de l'utérus, de l'ovaire, du pancréas, de glioblastome, colorectales et de sarcome, éventuellement ladite tumeur du poumon étant un carcinome pulmonaire non à petites cellules (NSCLC), et éventuellement ledit sujet ayant précédemment subi un traitement chirurgical.

**21.** Méthode selon la revendication 16, ladite première population de TIL à l'étape (a) étant obtenue à partir d'une petite biopsie, et éventuellement ladite petite biopsie étant obtenue à partir d'une tumeur choisie dans le groupe constitué par les tumeurs du pancréas, de mélanome, du sein et de l'ovaire, et en outre éventuellement ladite petite biopsie étant obtenue en utilisant une aiguille de calibre 16 à 11.

**22.** Méthode selon la revendication 16, ladite récolte à l'étape (d) étant effectuée en utilisant un système de traitement de cellules LOVO®, éventuellement ledit milieu de culture cellulaire étant fourni dans un récipient choisi dans le groupe constitué par un récipient G-container™ et une poche à cellules Xuri™ et en outre éventuellement ladite poche de perfusion à l'étape (e) étant une poche de perfusion Hypothermosol.

**23.** Méthode selon la revendication 16, lesdites étapes (a) à (e) étant effectuées dans un délai de 17 jours à 24 jours, éventuellement lesdites étapes (a) à (e) étant effectuées dans un délai de 18 jours à 22 jours, en outre éventuellement lesdites étapes (a) à (e) étant effectuées dans un délai de 20 jours à 22 jours.

**24.** Méthode selon la revendication 16, lesdites étapes (a) à (e) et éventuellement ladite cryoconservation étant effectuées en 22 jours ou moins.

**25.** Méthode selon l'une quelconque des revendications 16 à 24, ladite population thérapeutique de TIL récoltée à l'étape (d) comprenant suffisamment de TIL pour un dosage thérapeutiquement efficace des TIL, éventuellement ledit nombre de TIL suffisant pour un dosage thérapeutiquement efficace allant de $2,3 \times 10^{10}$ à $13,7 \times 10^{10}$.

**26.** Méthode selon l'une quelconque des revendications 16 à 25, lesdites étapes (a) à (d) étant effectuées dans un récipient unique, ladite réalisation des étapes (a) à (d) dans un récipient unique conduisant à une augmentation du rendement en TIL par tumeur réséquée comparée à la réalisation des étapes (a) à (d) dans plus d'un récipient, éventuellement lesdites cellules présentatrices d'antigène étant ajoutées aux TIL pendant la seconde expansion à l'étape (c) sans ouvrir le système, éventuellement ladite troisième population de TIL comprenant une sous-population accrue de lymphocytes T effecteurs et/ou de lymphocytes T à mémoire centrale par rapport à la deuxième population de TIL, lesdits lymphocytes T effecteurs et/ou lymphocytes T à mémoire centrale dans la population thérapeutique de TIL présentant une ou plusieurs caractéristiques choisies dans le groupe constitué par l'expression de CD27+,

l'expression de CD28+, des télomères plus longs, l'expression de CD57 accrue et l'expression de CD56 réduite, par rapport aux lymphocytes T effecteurs et/ou aux lymphocytes T à mémoire centrale obtenus à partir de la deuxième population de cellules, et en outre éventuellement lesdits lymphocytes T effecteurs et/ou lymphocytes T à mémoire centrale obtenus à partir de la troisième population de TIL présentant une expression de CD57 accrue et une expression de CD56 réduite par rapport aux lymphocytes T effecteurs et/ou aux lymphocytes T à mémoire centrale obtenus à partir de la deuxième population de cellules.

# Figure 1

**Process 2A: about 22 days from Steps A - E**

1. **STEP A**

Obtain Patient Tumor Sample

2. **STEP B**

Fragmentation and First Expansion

3 days to 14 days

3. **STEP C**

First Expansion to Second Expansion Transition

No Storage and Closed System

4. **STEP D**

Second Expansion

IL-2, OKT-3, and antigen-presenting feeder cells

Closed System

5. **STEP E**

Harvest TILS from Step D

Closed System

6. **STEP F**

Final Formulation and/or Transfer to Infusion Bag

(optionally cryopreserve)

*Figure 2A*

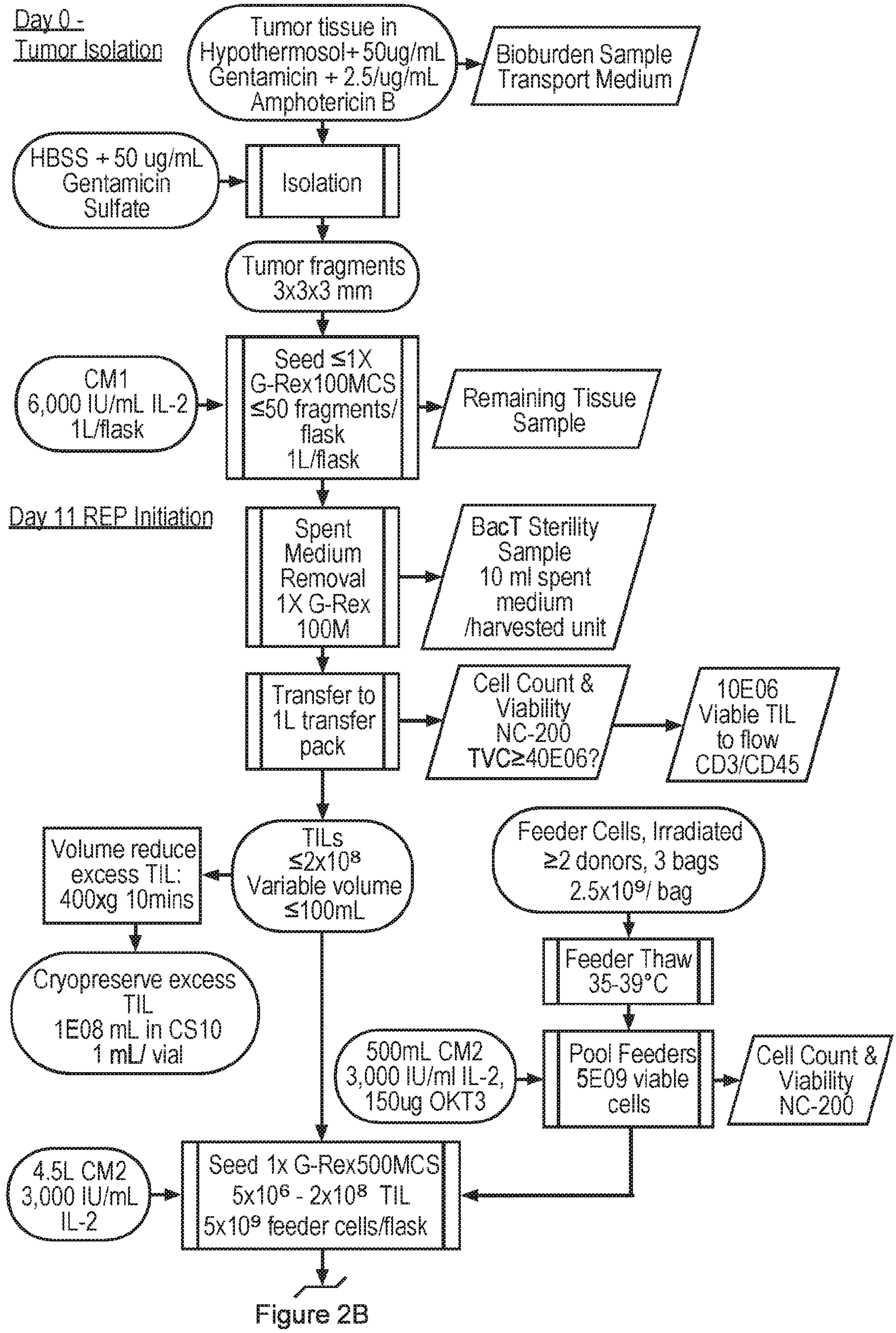
Day 0 - Tumor Isolation
Tumor tissue in Hypothermosol+50ug/mL Gentamicin + 2.5/ug/mL Amphotericin B
Bioburden Sample Transport Medium
HBSS + 50 ug/mL Gentamicin Sulfate
Isolation
Tumor fragments 3x3x3 mm
CM1 6,000 IU/mL IL-2 1L/flask
Seed ≤1X G-Rex100MCS ≤50 fragments/ flask 1L/flask
Remaining Tissue Sample
Day 11 REP Initiation
Spent Medium Removal 1X G-Rex 100M
BacT Sterility Sample 10 ml spent medium /harvested unit
Transfer to 1L transfer pack
Cell Count & Viability NC-200 TVC≥40E06?
10E06 Viable TIL to flow CD3/CD45
Volume reduce excess TIL: 400xg 10mins
TILs ≤2x10⁸ Variable volume ≤100mL
Feeder Cells, Irradiated ≥2 donors, 3 bags 2.5x10⁹/ bag
Cryopreserve excess TIL 1E08 mL in CS10 1 mL/ vial
Feeder Thaw 35-39°C
500mL CM2 3,000 IU/ml IL-2, 150ug OKT3
Pool Feeders 5E09 viable cells
Cell Count & Viability NC-200
4.5L CM2 3,000 IU/mL IL-2
Seed 1x G-Rex500MCS 5x10⁶ - 2x10⁸ TIL 5x10⁹ feeder cells/flask
Figure 2B

## *Figure 2B*

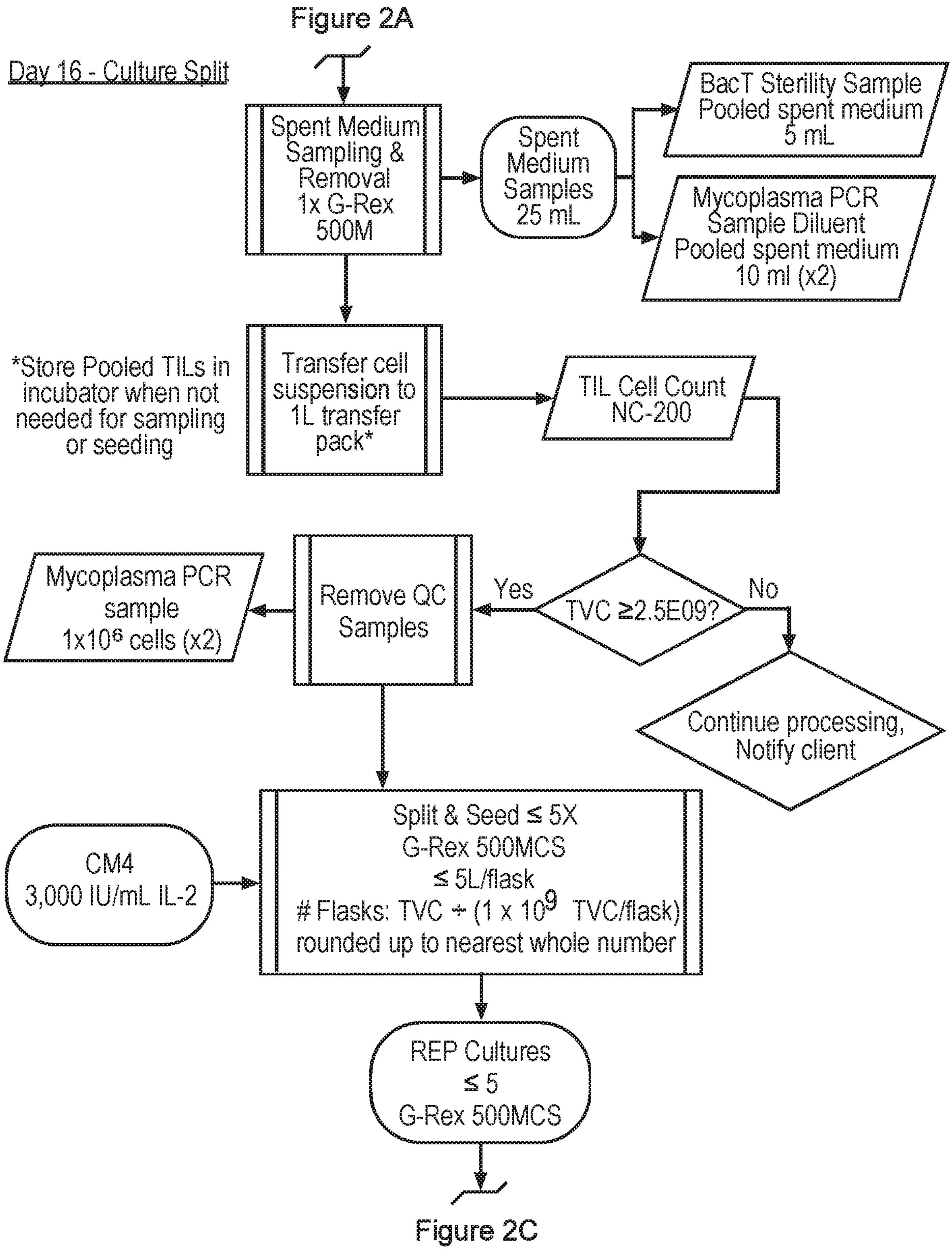

Figure 2A
Day 16 - Culture Split
Spent Medium Sampling & Removal 1x G-Rex 500M
Spent Medium Samples 25 mL
BacT Sterility Sample Pooled spent medium 5 mL
Mycoplasma PCR Sample Diluent Pooled spent medium 10 ml (x2)
*Store Pooled TILs in incubator when not needed for sampling or seeding
Transfer cell suspension to 1L transfer pack*
TIL Cell Count NC-200
Mycoplasma PCR sample $1x10^6$ cells (x2)
Remove QC Samples
Yes
TVC ≥2.5E09?
No
Continue processing, Notify client
CM4 3,000 IU/mL IL-2
Split & Seed ≤ 5X G-Rex 500MCS ≤ 5L/flask # Flasks: TVC ÷ ($1 x 10^9$ TVC/flask) rounded up to nearest whole number
REP Cultures ≤ 5 G-Rex 500MCS
Figure 2C

***Figure 2C***

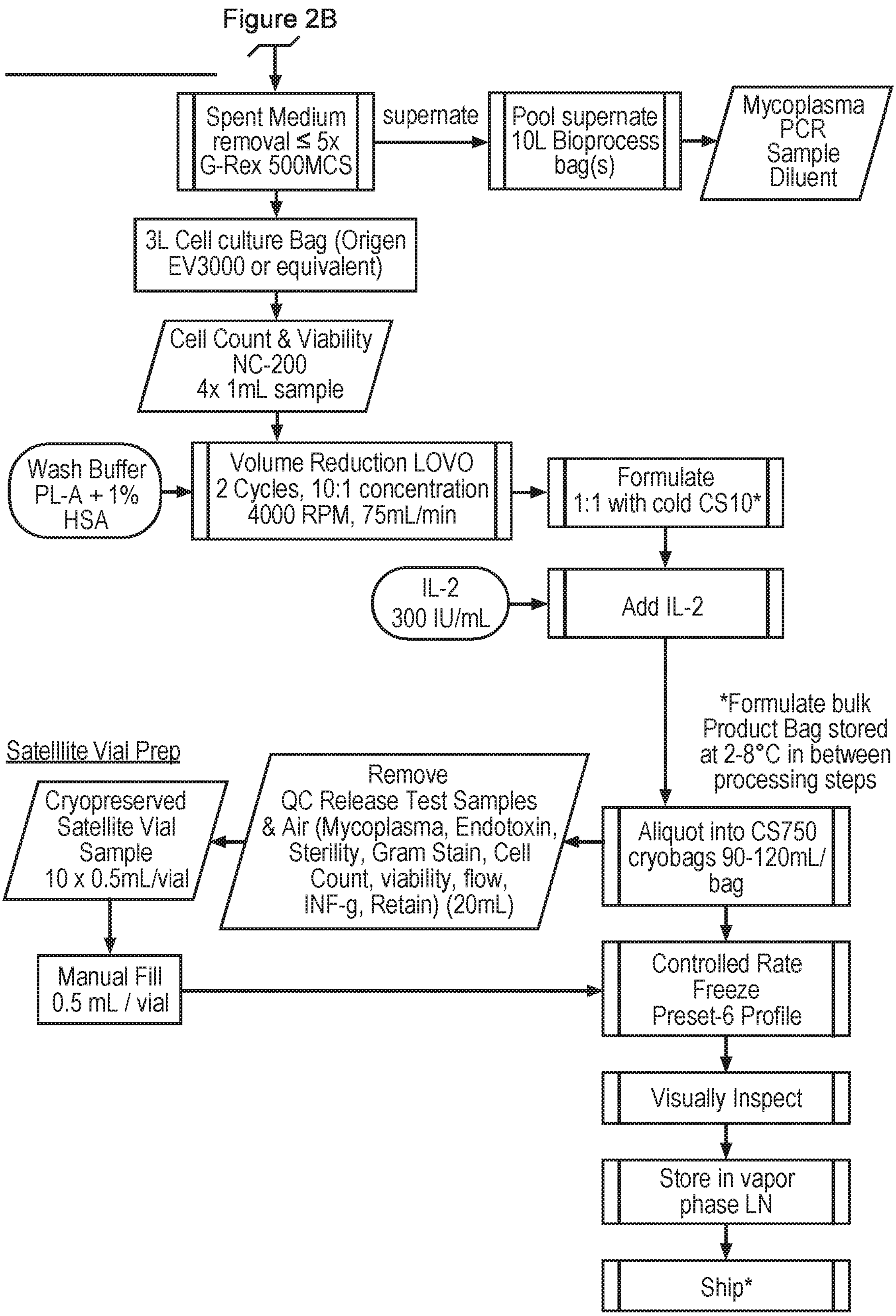
Figure 2B
Spent Medium removal ≤ 5x G-Rex 500MCS
supernate
Pool supernate 10L Bioprocess bag(s)
Mycoplasma PCR Sample Diluent
3L Cell culture Bag (Origen EV3000 or equivalent)
Cell Count & Viability NC-200 4x 1mL sample
Wash Buffer PL-A + 1% HSA
Volume Reduction LOVO 2 Cycles, 10:1 concentration 4000 RPM, 75mL/min
Formulate 1:1 with cold CS10*
IL-2 300 IU/mL
Add IL-2
*Formulate bulk Product Bag stored at 2-8°C in between processing steps
Satelllite Vial Prep
Cryopreserved Satellite Vial Sample 10 x 0.5mL/vial
Remove QC Release Test Samples & Air (Mycoplasma, Endotoxin, Sterility, Gram Stain, Cell Count, viability, flow, INF-g, Retain) (20mL)
Aliquot into CS750 cryobags 90-120mL/ bag
Manual Fill 0.5 mL / vial
Controlled Rate Freeze Preset-6 Profile
Visually Inspect
Store in vapor phase LN
Ship*

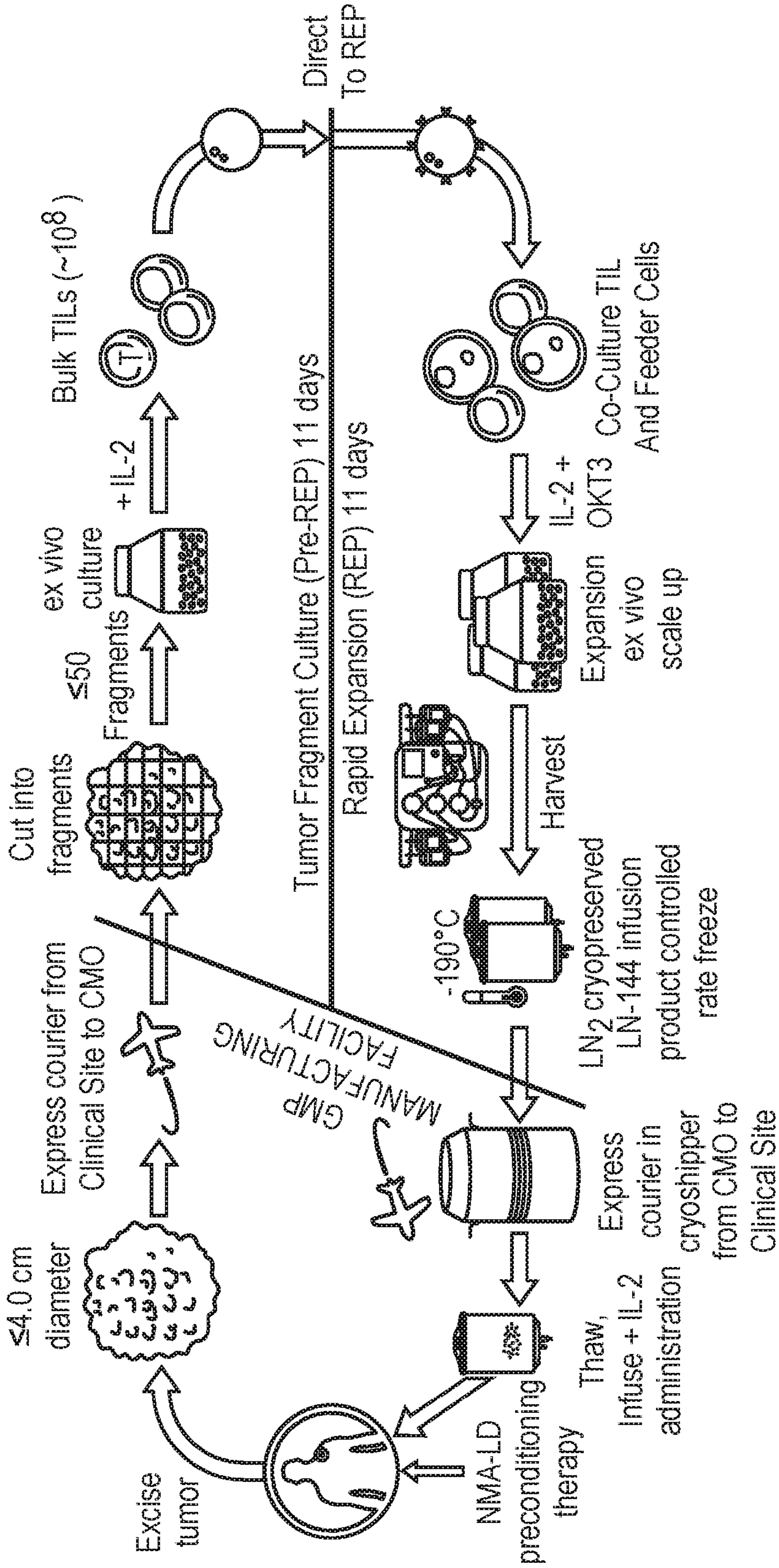
Excise
tumor
≤4.0 cm
diameter
Express courier from
Clinical Site to CMO
Cut into
fragments
≤50
Fragments
ex vivo
culture
+ IL-2
Bulk TILs (~$10^8$)
T
Direct
To REP
Tumor Fragment Culture (Pre-REP) 11 days
Rapid Expansion (REP) 11 days
GMP
MANUFACTURING
FACILITY
Co-Culture TIL
And Feeder Cells
IL-2 +
OKT3
Expansion
ex vivo
scale up
Harvest
-190°C
$LN_2$ cryopreserved
LN-144 infusion
product controlled
rate freeze
Express
courier in
cryoshipper
from CMO to
Clinical Site
Thaw,
Infuse + IL-2
administration
NMA-LD
preconditioning
therapy

***Figure 3***

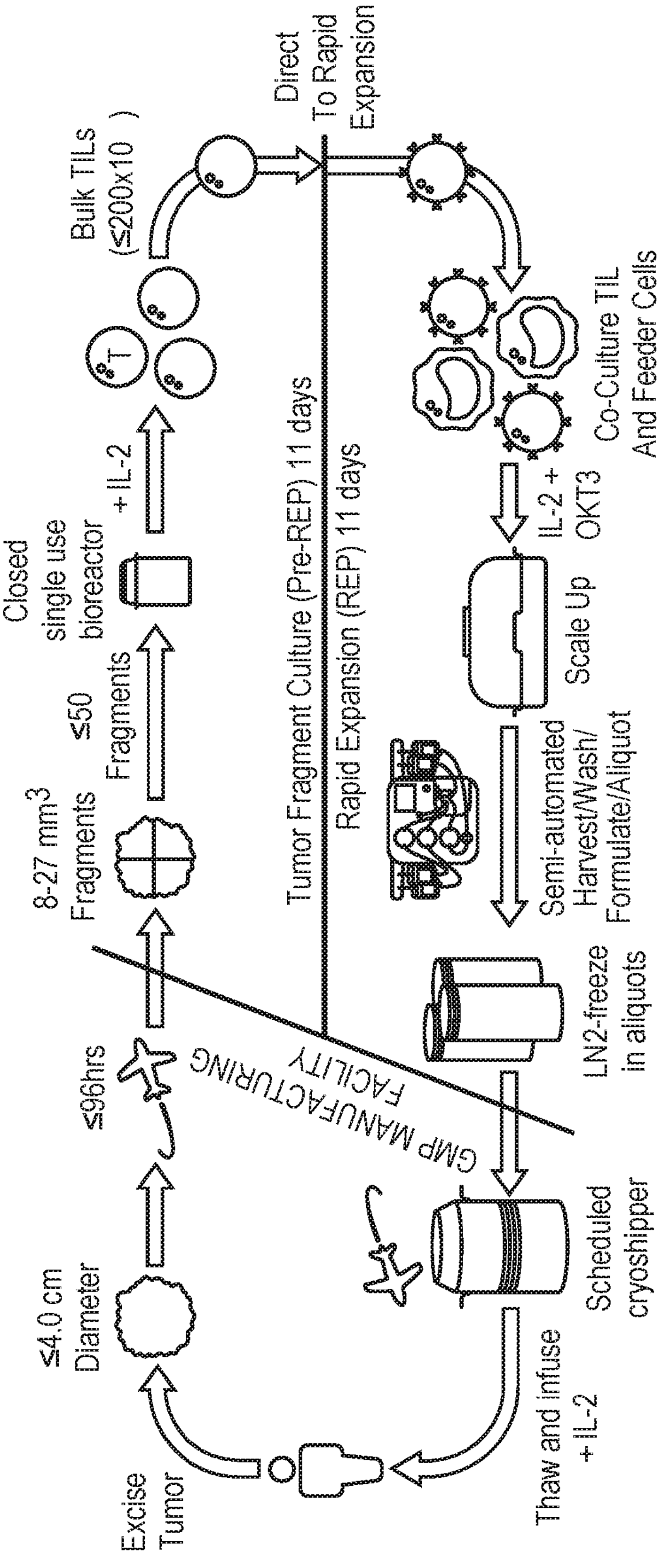
Excise Tumor
≤4.0 cm Diameter
≤96hrs
GMP MANUFACTURING FACILITY
8-27 mm3 Fragments
≤50 Fragments
Closed single use bioreactor
+ IL-2
T
Bulk TILs (≤200x10 )
Direct To Rapid Expansion
Tumor Fragment Culture (Pre-REP) 11 days
Rapid Expansion (REP) 11 days
Co-Culture TIL And Feeder Cells
IL-2 + OKT3
Scale Up
Semi-automated Harvest/Wash/Formulate/Aliquot
LN2-freeze in aliquots
Scheduled cryoshipper
Thaw and infuse + IL-2

*Figure 4*

# *Figure 5*

| Process 1C: 43-55 Days for Steps A - E | Process 2A: about 22 days from Steps A - E |
|---|---|
| 1. <u>STEP A</u><br>Obtain Patient Tumor Sample | 1. <u>STEP A</u><br>Obtain Patient Tumor Sample |
| 2. <u>STEP B</u><br>Fragmentation and First Expansion<br>11 days to 21 days | 2. <u>STEP B</u><br>Fragmentation and First Expansion<br>3 days to 14 days |
| 3. <u>STEP C</u><br>First Expansion to Second Expansion Transition<br>Optional Storage until Selection | 3. <u>STEP C</u><br>First Expansion to Second Expansion Transition<br>No Storage and Closed System |
| 4. <u>STEP D</u><br>Second Expansion<br>IL-2, OKT-3, antigen-presenting feeder cells<br>Optionally repeat one or more times | 4. <u>STEP D</u><br>Second Expansion<br>IL-2, OKT-3, and antigen-presenting feeder cells<br>Closed System |
| 5. <u>STEP E</u><br>Harvest TILS from Step D | 5. <u>STEP E</u><br>Harvest TILS from Step D<br>Closed System |
| 6. <u>STEP F</u><br>Final Formulation and/or Transfer to Infusion Bag | 6. <u>STEP F</u><br>Final Formulation and/or Transfer to Infusion Bag<br>(optionally cryopreserve) |

# Figure 6

| Process Step | Process 1C Embodiment | Process 2A Embodiment | Advantages |
|---|---|---|---|
| Pre-REP | • 4 fragments per 10 GREX-10 flasks<br>• 11-21 day duration | • 40 fragments per 1 GREX-100M flask<br>• 11 day duration | • Increased tumor fragments per flask<br>• Shortened culture time<br>• Reduced number of steps<br>• Amenable to closed system |
| Pre-REP to REP Transition | • Pre-REP TIL are frozen until phenotyped for selection then thawed to proceed to the REP (~day 30)<br>• REP requires >$40\times10^6$ TIL | • Pre-REP TIL directly move to REP on day 11<br>• REP requires 25-$200\times10^6$ TIL | • Shortened pre-REP-to-REP process<br>• Reduced number of steps<br>• Eliminated phenotyping selection<br>• Amenable to closed system |
| REP | • 6 GREX-100M flasks on REP day 0<br>• $5\times10^6$ TIL and $5\times10^8$ PBMC feeders per flask on REP day 0<br>• Split to 18-36 flasks on REP day 7<br>• 14 day duration | • 1 GREX-500M flask on day 11<br>• 25-$200\times10^6$ TIL and $5x10^9$ PBMC feeders on day 11<br>• Split to ≤ 6 GREX-500M flasks on day 16<br>• 11 day duration | • Reduced number of steps<br>• Shorter REP duration<br>• Closed system transfer of TIL between flasks<br>• Closed system media exchanges |
| Harvest | • TIL harvested via centrifugation | • TIL harvested via LOVO automated cell washing system' | • Reduced number of steps<br>• Automated cell washing<br>• Closed system<br>• Reduced loss of product during wash |
| Final Formulation | • Fresh product in Hypothermosol<br>• Single infusion bag<br>• Limited shipping stability | • Cyropreserved product in PlasmaLyte-A + 1% HSA and CS10 stored in $LN_2$<br>• Multiple aliquots<br>• Longer shipping stability | • Shipping flexibility<br>• Flexible patient scheduling<br>• More timely release testing |
| Overall Estimated Process Time | • 43-55 days | • 22 days | • Faster turnaround to patient |

*Figure 7A*

| Small Biopsy Process: Version 1<br>About 21-33 days from Steps A - E |
|---|
| **1. STEP A**<br>Obtain Patient Tumor Sample |
| **2. STEP B**<br>First Expansion<br>1 days to 19 days (with OKT-3 and antigen-presenting feeder cells at day 3) |
| **3. STEP C**<br>First Expansion to Second Expansion Transition<br>No Storage and Closed System |
| **4. STEP D**<br>Second Expansion<br>IL-2, OKT-3, and antigen-presenting feeder cells<br>11 to 14 days<br>Closed System |
| **5. STEP E**<br>Harvest TILS from Step D<br>Closed System |
| **6. STEP F**<br>Final Formulation and/or Transfer to Infusion Bag<br>(optionally cryopreserve) |

*Figure 7B*

**Small Biopsy Process: Version 2**

**About 17-24 days from Steps A - E**

1. **STEP A**

Obtain Patient Tumor Sample

2. **STEP B**

First Expansion

3 days to 12 days (with OKT-3 and antigen-presenting feeder cells at any one of days 1-3)

3. **STEP C**

First Expansion to Second Expansion Transition

No Storage and Closed System

4. **STEP D**

Second Expansion

IL-2, OKT-3, and antigen-presenting feeder cells

3 to 12 days

Closed System

5. **STEP E**

Harvest TILS from Step D

Closed System

6. **STEP F**

Final Formulation and/or Transfer to Infusion Bag

(optionally cryopreserve)

*Figure 7C*

| Small Biopsy Process: Version 1<br>About 21-33 days from Steps A - E | Small Biopsy Process: Version 2 (GEN3 based process)<br>About 17-24 days from Steps A - E | Process 2A:<br>About 22 days from Steps A - E |
|---|---|---|
| 1. **STEP A**<br>Obtain Patient Tumor Sample | 1. **STEP A**<br>1. Obtain Patient Tumor Sample | 1. **STEP A**<br>Obtain Patient Tumor Sample |
| 2. **STEP B**<br>First Expansion<br>1 days to 19 days (with OKT-3 and antigen-presenting feeder cells at day 3) | 2. **STEP B**<br>First Expansion<br>3 days to 12 days (with OKT-3 and antigen-presenting feeder cells at any one of days 1-3) | 2. **STEP B**<br>Fragmentation and First Expansion<br>3 days to 14 days |
| 3. **STEP C**<br>First Expansion to Second Expansion Transition<br>No Storage and Optionally Closed System | 3. **STEP C**<br>First Expansion to Second Expansion Transition<br>No Storage and optionally Closed System | 3. **STEP C**<br>First Expansion to Second Expansion Transition<br>No Storage and Closed System |
| 4. **STEP D**<br>Second Expansion<br>IL-2, OKT-3, and antigen-presenting feeder cells<br>11 to 14 days<br>Optionally Closed System | 4. **STEP D**<br>Second Expansion<br>IL-2, OKT-3, and antigen-presenting feeder cells<br>3 to 12 days<br>Optionally Closed System | 4. **STEP D**<br>Second Expansion<br>IL-2, OKT-3, and antigen-presenting feeder cells<br>Closed System |
| 5. **STEP E**<br>Harvest TILS from Step D<br>Optionally Closed System | 5. **STEP E**<br>Harvest TILS from Step D<br>Closed System | 5. **STEP E**<br>Harvest TILS from Step D<br>Closed System |
| 6. **STEP F**<br>Final Formulation and/or Transfer to Infusion Bag<br>(optionally cryopreserve) | 6. **STEP F**<br>Final Formulation and/or Transfer to Infusion Bag<br>(optionally cryopreserve) | 6. **STEP F**<br>Final Formulation and/or Transfer to Infusion Bag<br>(optionally cryopreserve) |

*Figures 8A and 8B*

Figure 8A

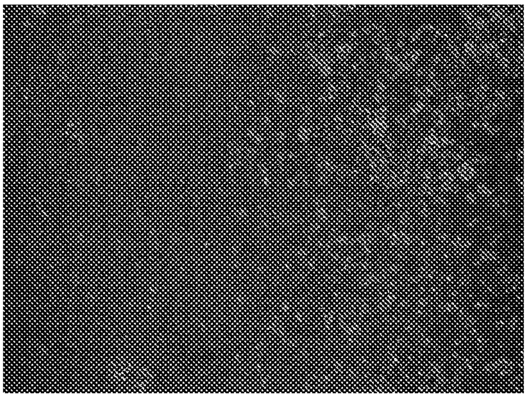

Figure 8B

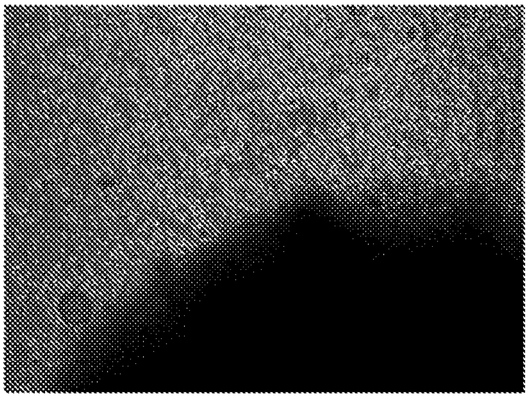

*Figures 9A and 9B*

## Figure 9A

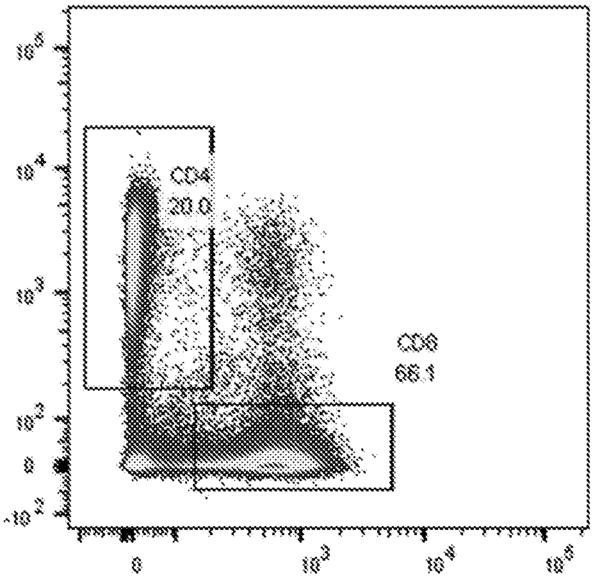


## Figure 9B

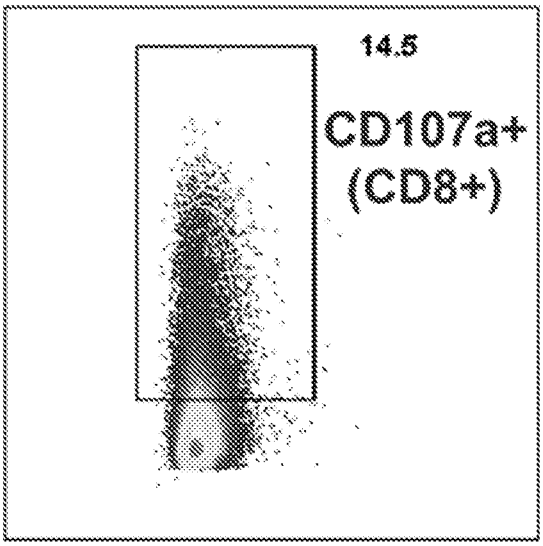

*Figures 9C and 9D*

Figure 9C

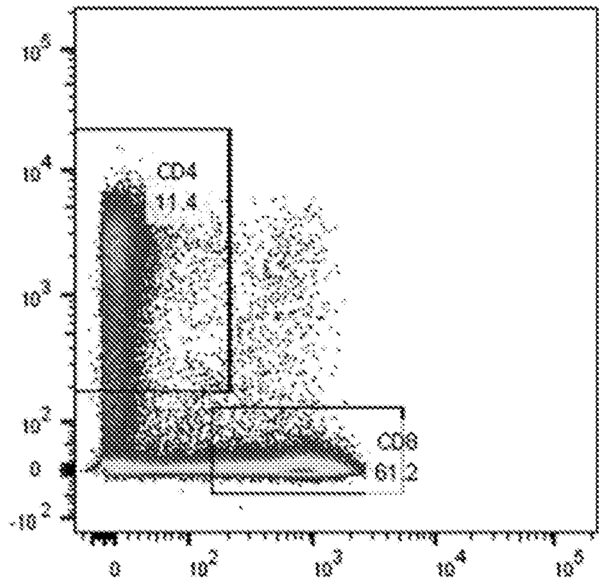


Figure 9D

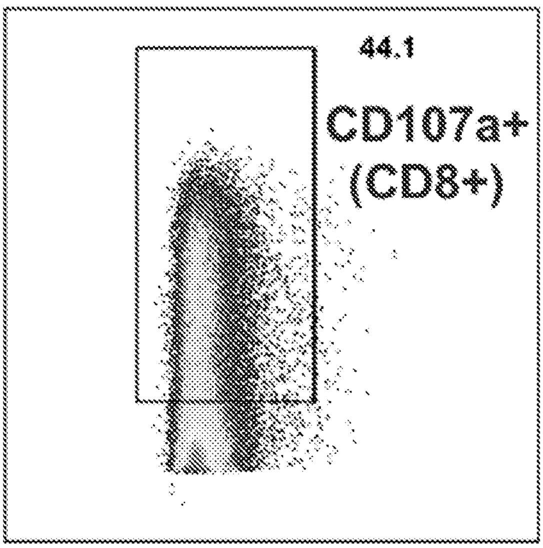

*Figures 10A and 10B*

Figure 10A

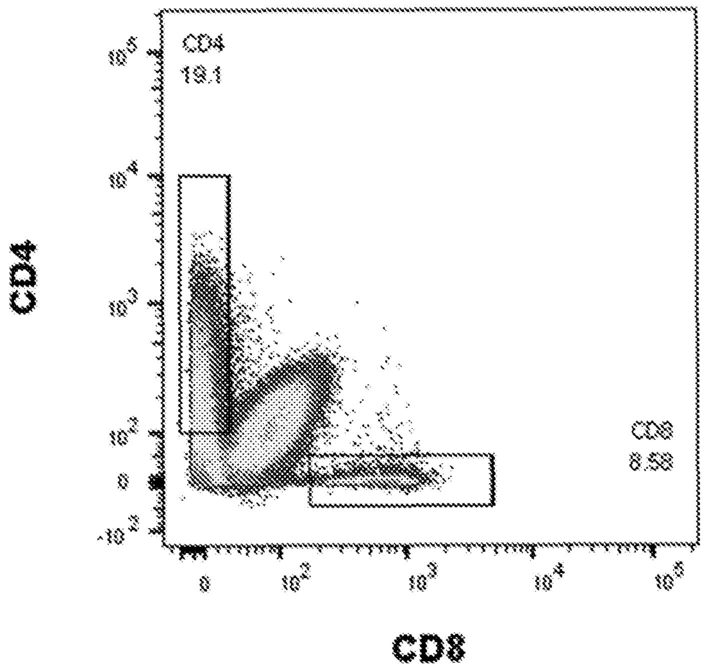


Figure 10B

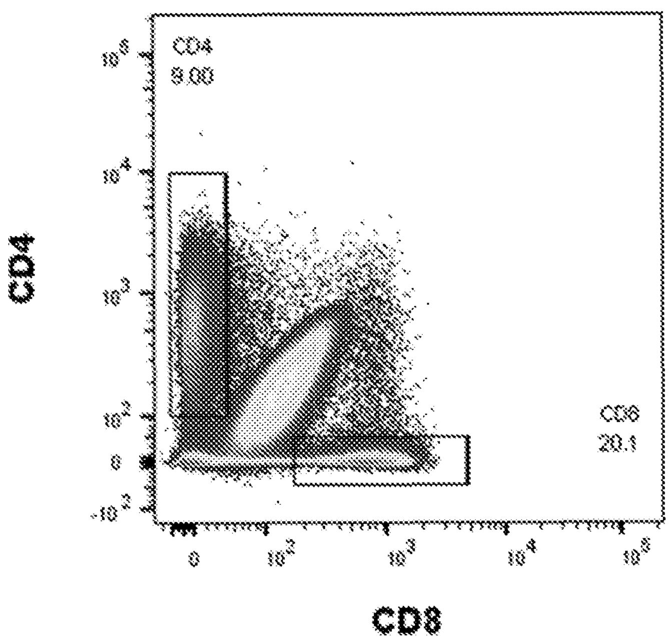

*Figures 11A and 11B*

## Figure 11A

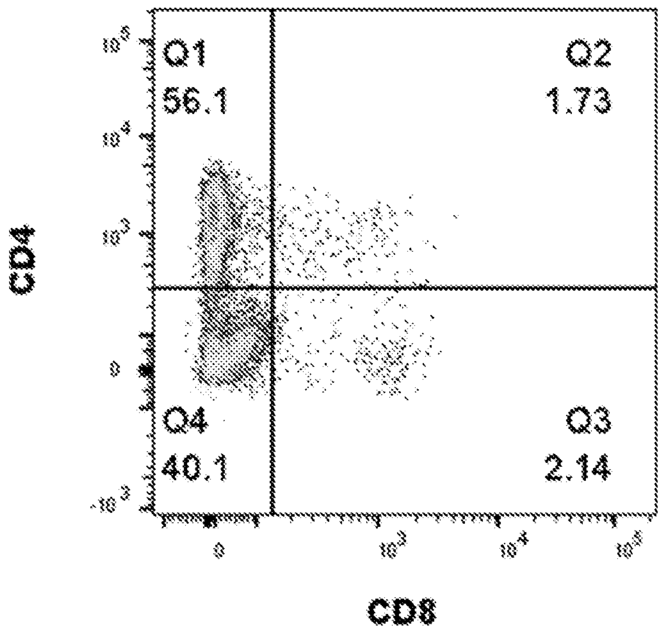


## Figure 11B

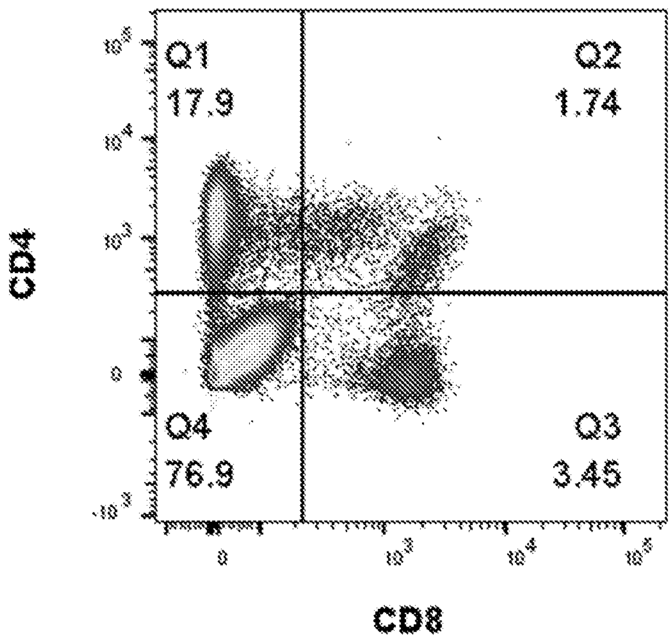

*Figure 11C*

Figure 11C

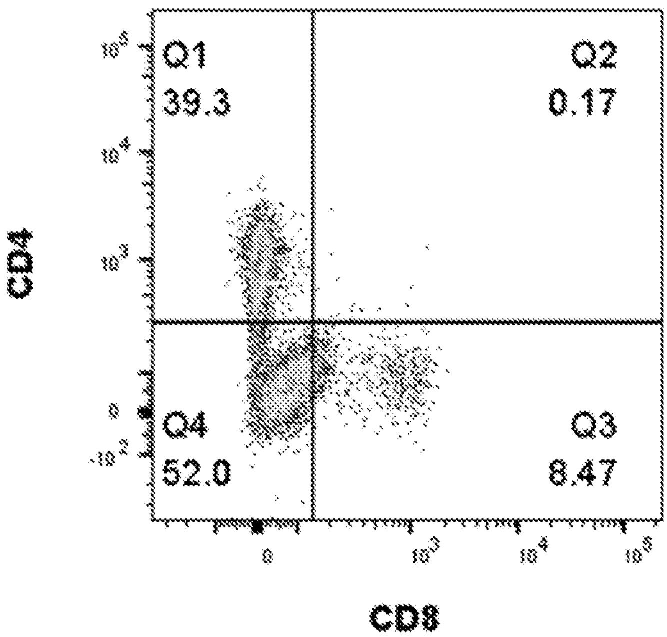

*Figures 12A and 12B*

## Figure 12A

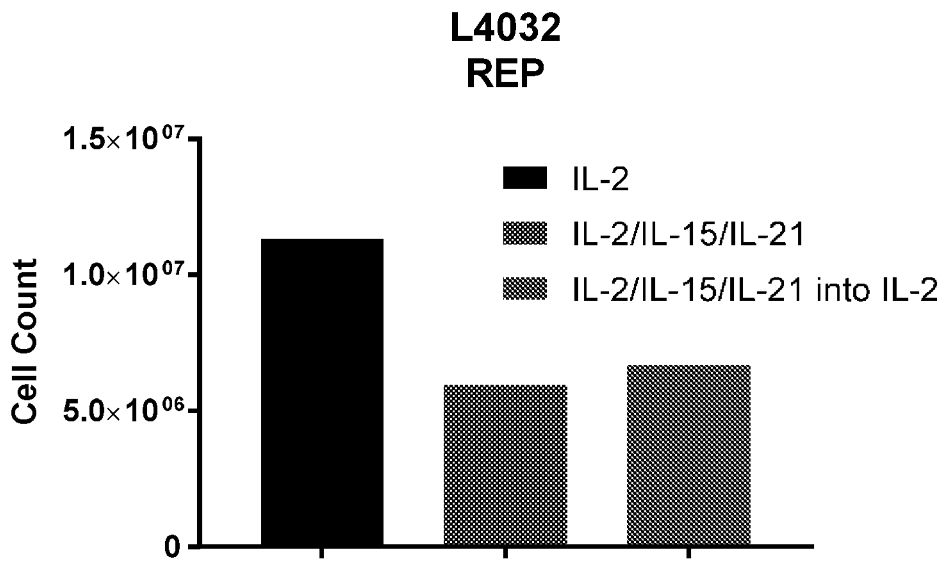


## Figure 12B

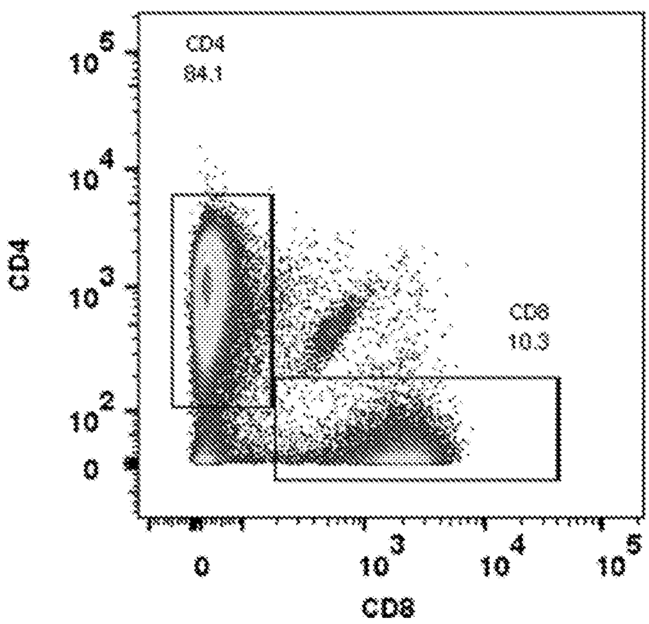

*Figures 13A and 13B*

Figure 13A

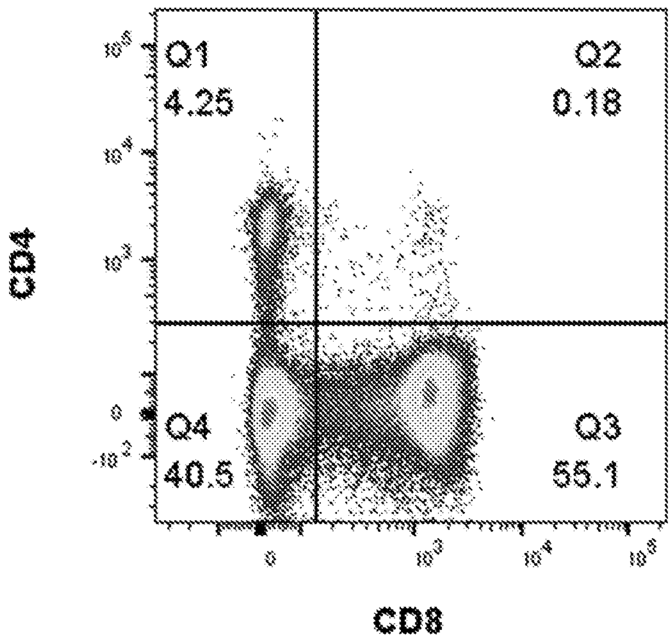


Figure 13B

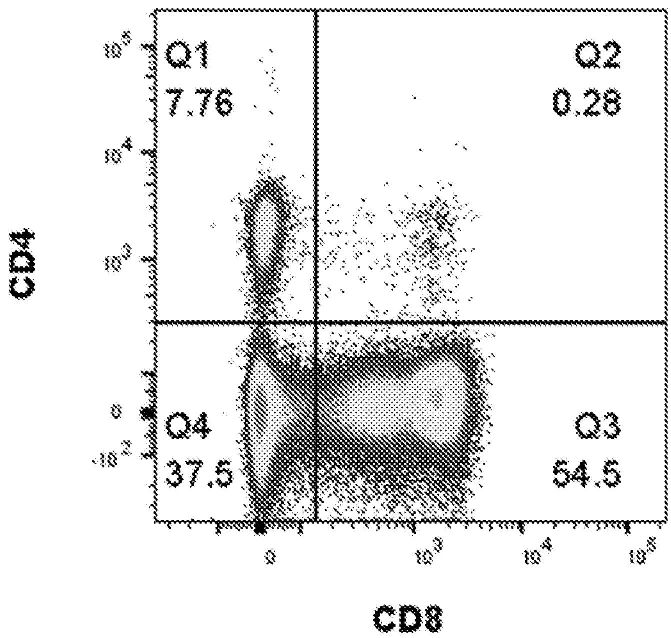

*Figure 14*

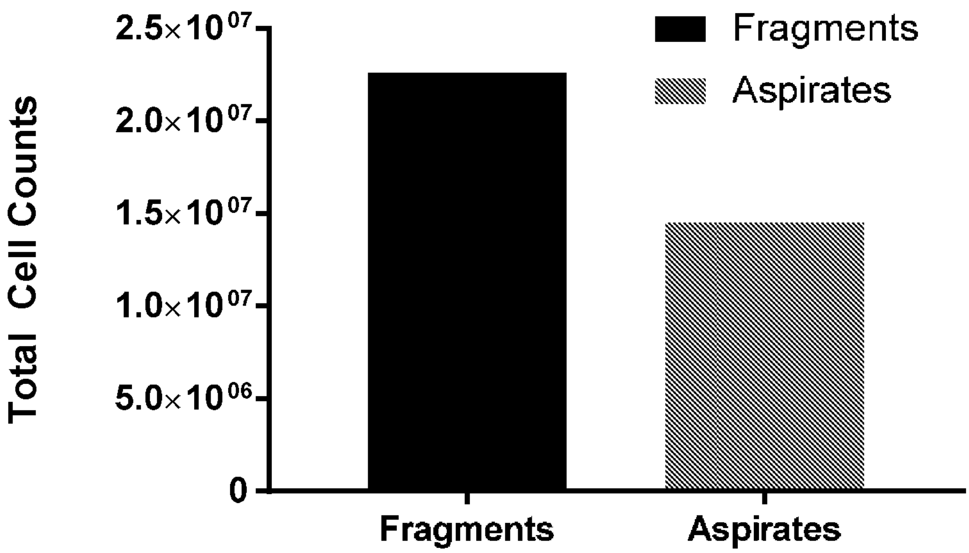
Total Cell Counts
2.5×10^07
2.0×10^07
1.5×10^07
1.0×10^07
5.0×10^06
0
Fragments
Aspirates
Fragments
Aspirates

*Figure 15*

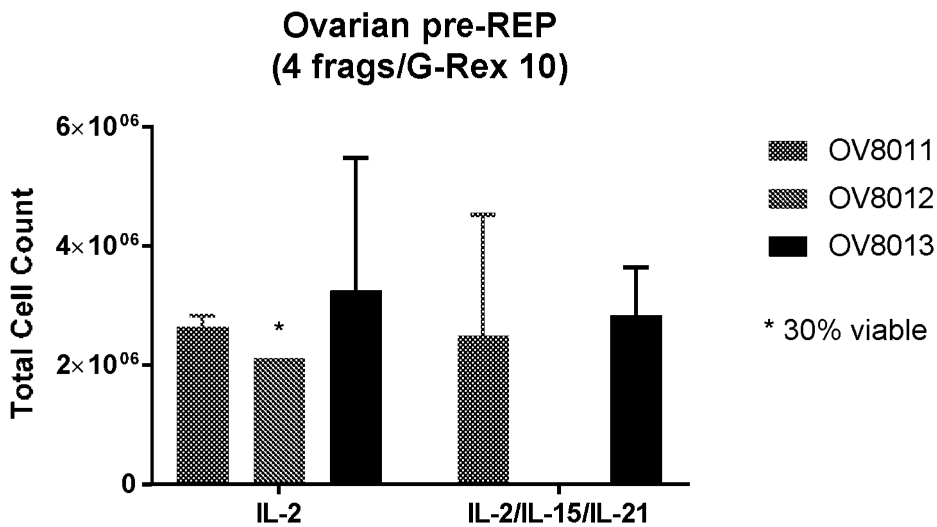
Ovarian pre-REP
(4 frags/G-Rex 10)
Total Cell Count
6×10⁰⁶
4×10⁰⁶
2×10⁰⁶
0
IL-2
IL-2/IL-15/IL-21
OV8011
OV8012
OV8013
* 30% viable

*Figures 16A and 16B*

Figure 16A

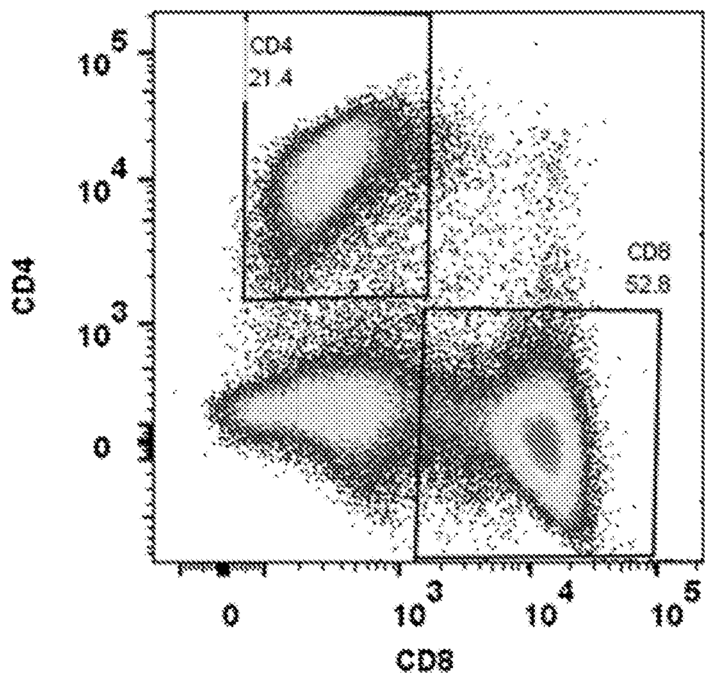


Figure 16B

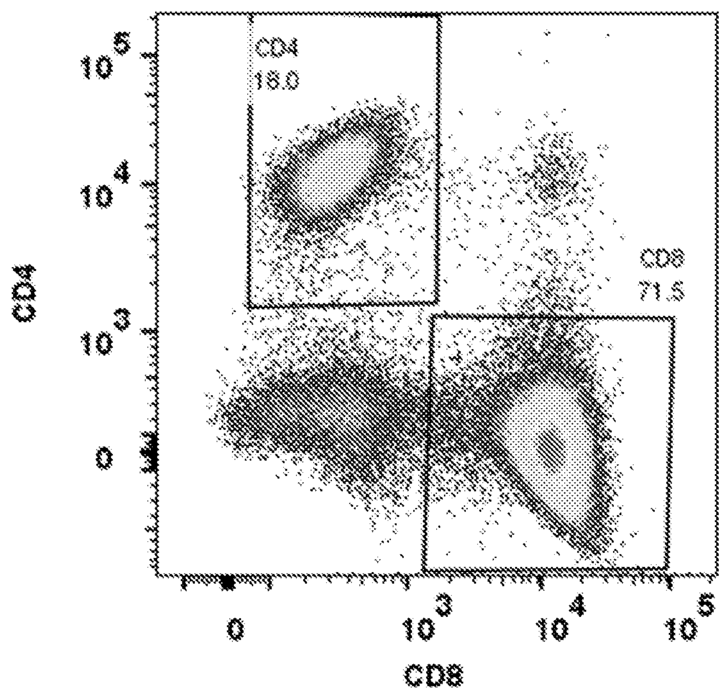

*Figures 17A and 17B*

## Figure 17A

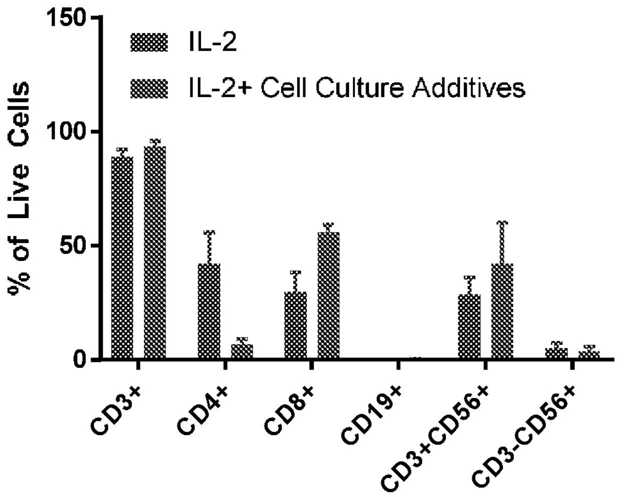


## Figure 17B

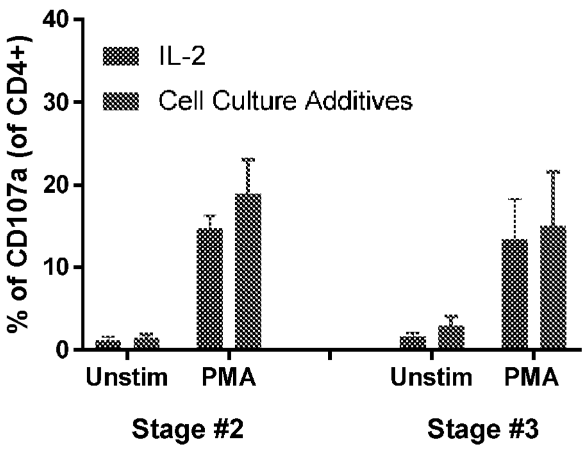

*Figures 17C and 17D*

## Figure 17C

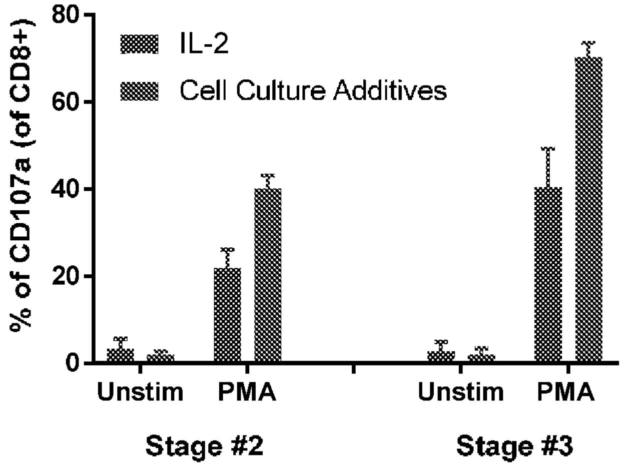


## Figure 17D

| Tumor ID | Cytokines | Days to reach Stage #1 | Acceptance Criteria for Stage #2 | Assayed Cells for Stage #2 | Acceptance Criteria for Stage #3 | Assayed Cells for Stage #3 |
|---|---|---|---|---|---|---|
| Pan-17-004 | IL-2 #1 | 27 | | $116x10^6$ | | Media Failure |
| | IL-2 #2 | 27 | | $130x10^6$ | | Media Failure |
| | Cell Culture Additives #1 | 14 | | Media Failure | | N/A |
| | Cell Culture Additives #2 | 32 | $20x10^6$ | $102x10^6$ | $100x10^6$ | $148x10^6$ |
| Pan-17-007 | IL2 | 19 | | Did not meet criteria to progress to REP | | N/A |
| | Cell Culture Additives | 25 | | $118x10^6$ | | $244x10^6$ |
| Pan-17-008 | IL2 | Did not meet criteria for G-Rex10 | | N/A | | N/A |
| | Cell Culture Additives | 21 | | $106x10^6$ | | $144x10^6$ |

## *Figure 18A*

| Histology | FNA/Biopsy | Initial Cell Count (FNA's) | Conditions | Days in Culture | Final Cell Counts | Assays |
|---|---|---|---|---|---|---|
| Pancreatic (P7015) | Cores 24 well plate | N/A | **ONE CORE**<br>1) IL-2 (3 wells)<br>2) IL-2/IL-15/IL-21 (2 wells)<br><br>**TWO CORES**<br>1) IL-2 (1 well)<br>2) IL-2/IL-15/IL-21 (1 wells) | 28 days | **ONE CORE**<br>IL-2<br>1.2e6 viable cells/well average<br><br>IL-2/IL-15/IL-21<br>2e6 viable cells/well average<br><br>**TWO CORES**<br>No growth | **Phenotypic**<br>FACS<br>**Functional**<br>CD107a |
| Pancreatic (P7017) | Cores 24 well plate | N/A | **ONE CORE** | N/A | No TIL growth | |
| Pancreatic (P7019) | Cores G-Rex 10 | N/A | **6 CORES** | In progress | | |
| Lung (L4032) | FNA 24 well plate | 1.6e6 cells (28% viable) | 1) IL-2 (2 wells)<br>2) IL-2/IL-15/IL-21 (2 wells)<br><br>+ Fragments | 13 days | IL-2<br>4.64e5cells/well average, 76 % viable<br><br>IL-2/IL-15/IL-21<br>7.75e5 cells/well average, 94% viable | **Phenotypic**<br>FACS<br>**Functional**<br>CD107a (N/A)<br>**REP**<br>(recently harvested) |
| Lung (L4033) | **FNA G-Rex 10** | 5.5e6 cells (76% viable) | 1) IL-2<br>2) IL-2/IL-15/IL-21<br><br>+ Fragments | 12 days | IL-2<br>6.e6, 73 % viable<br><br>IL-2/IL-15/IL-21<br>No detectable cells (no assay were run on this sample) | **Phenotypic**<br>FACS<br>**Functional**<br>CD107a<br>**REP**<br>(recently harvested) |
| Lung (L4034) | FNA G-Rex 10 | N/A | | | No TIL growth | No assays |

## *Figure 18B*

| Histology | FNA/Biopsy | Initial Cell Count (FNA's) | Conditions | Days in Culture | Final Cell Counts | Assays |
|---|---|---|---|---|---|---|
| Colorectal (CC10024) | FNA 24 well plate | 8.0e5 viable cells, 42% viable | 1) IL-2 (2 wells) 2) IL-2/IL-15/IL-21 (2 wells)<br><br>Fragments as a control | 27 days | No TIL growth | No assays |
| Ovarian (OV8012) | FNA 24 well plate | 3.19e5 viable cells, 28% viable | 1) IL-2 (2 wells) 2) IL-2/IL-15/IL-21 (2 wells) | | No TIL growth | No assays |
| Ovarian (OV8013) | FNA 24 well plate | 8.3e5 viable cells, 11% viable | 1) IL-2 (2 wells) 2) IL-2/IL-15/IL-21 (2 wells) | | No TIL growth | No assays |
| Ovarian (OV8014) | FNA 24 well plate | 1.0e5 viable cells, 7% viable | 1) IL-2 (1 well) 2) IL-2/IL-15/IL-21 (1 well) | | No TIL growth | No assays |
| Cervical (C2005) | FNA 24 well plate | 6.84e5 (13% viable) | 1) IL-2 (2 wells) 2) IL-2/IL-15/IL-21 (2 wells) + Fragments | 21 days | IL-2 3.45e5 viable cells/well average, 39% viable IL-2/IL-15/IL-21 8.69e5 viable cells/well average, 40% viable | **Phenotypic** FACS **Functional** CD107a |
| Melanoma (M1101) | FNA G-Rex 10 | 1.96e6 (65% viable) | IL-2 (1 G-Rex 10) + Fragments | 12 days | IL-2 3.0e6, 78 % viable | **Phenotypic** FACS **Functional** CD107a **REP** (recently harvested) |
| Melanoma (M1107) | FNA G-Rex 10 | 2.25e6 (69% viable) | IL-2 (2 G-Rex 10) IL-2/IL-15/IL-21 (2 G-Rex) + Fragments | | | Pre-REP in progress |

*Figures 19A and 19B*

Figure 19A

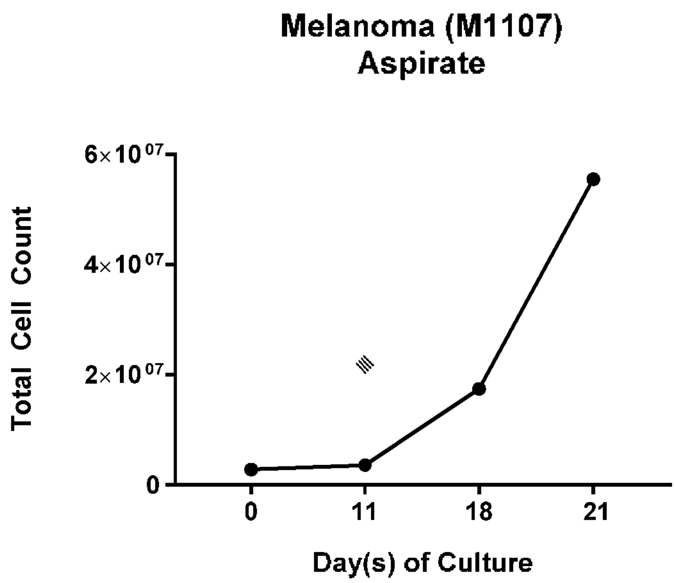


Figure 19B

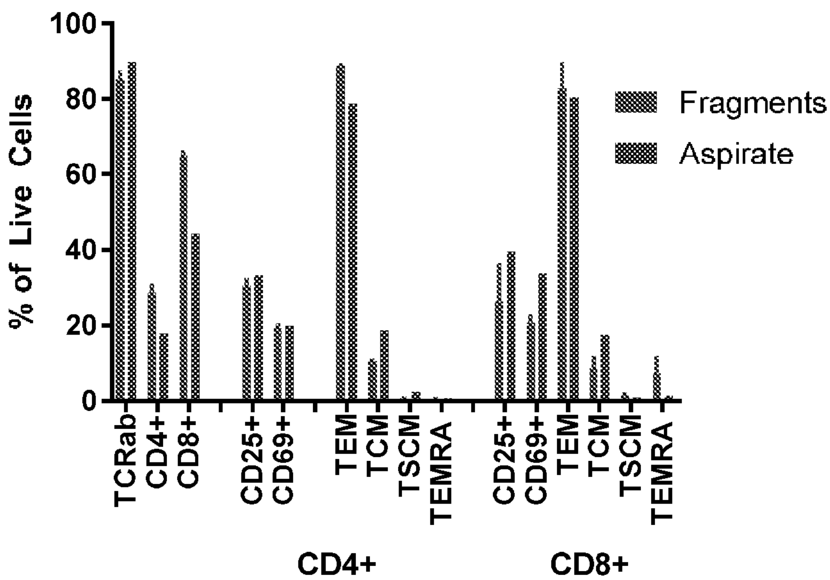

*Figure 19C*

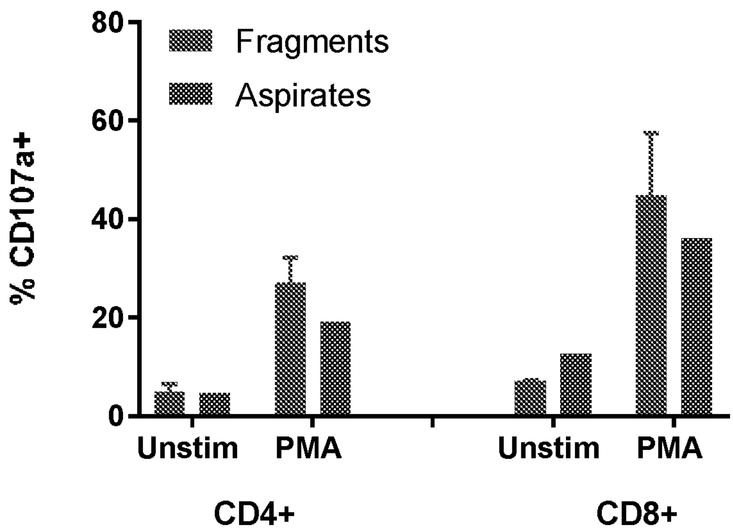

80
60
40
20
0
% CD107a+
Fragments
Aspirates
Unstim
PMA
Unstim
PMA
CD4+
CD8+

*Figure 20*

| Tumor Name | Cell Growth | Analysis |
|---|---|---|
| UPMC-3 Reported | 24 well plate: 1) IL-2 versus 2) Cytokine Additives | Cell growth, viability, phenotyping, CD107a |
| UPMC-9 Reported | Expanded poorly | N/A |
| UPMC-17 Reported | Contaminated culture | N/A |
| UPMC-26 | Expanded poorly | N/A |
| UPMC-30 | pre-REP Day 11: 1.6e6, 40% viable<br>pre-REP Day 18: 3.49e7, 89% viable | Cell growth, viability, phenotyping, CD107a, IFNγ, REP initiation and analysis |
| UPMC-61 | pre-REP Day 11: 1.52e5, 66% viable<br>pre-REP Day 18: 1.14e7, 1.14e7, 40% viable | Cell growth, viability, phenotyping, CD107a, IFNγ, REP initiation and analysis |
| UPMC-59 | Expanded poorly: | N/A |
| UPMC-61 | pre-REP: In progress | |

*Figure 21*

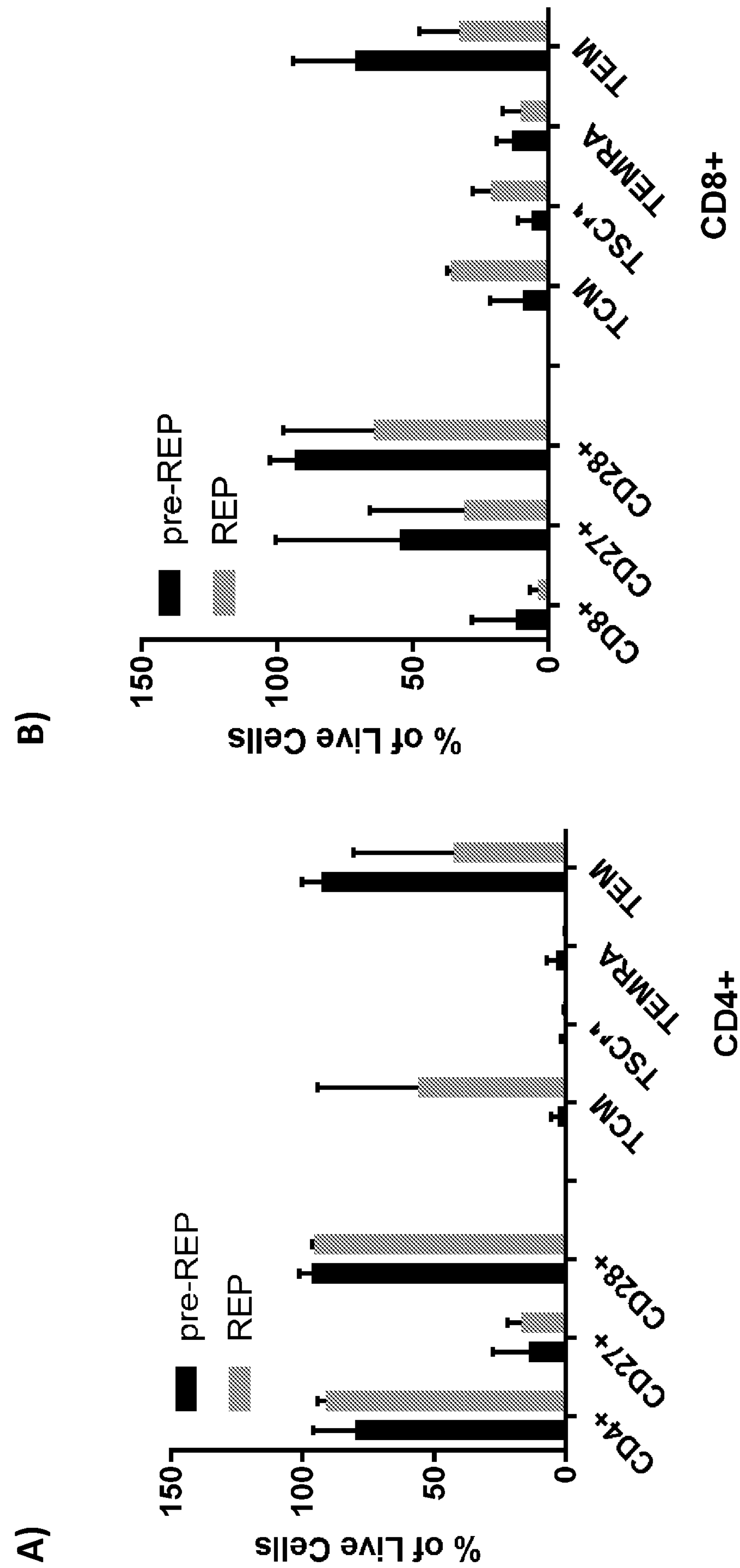
A)
% of Live Cells
150
100
50
0
pre-REP
REP
CD4+
CD27+
CD28+
TCM
TSCM
TEMRA
TEM
CD4+
B)
% of Live Cells
150
100
50
0
pre-REP
REP
CD8+
CD27+
CD28+
TCM
TSCM
TEMRA
TEM
CD8+

*Figure 22*

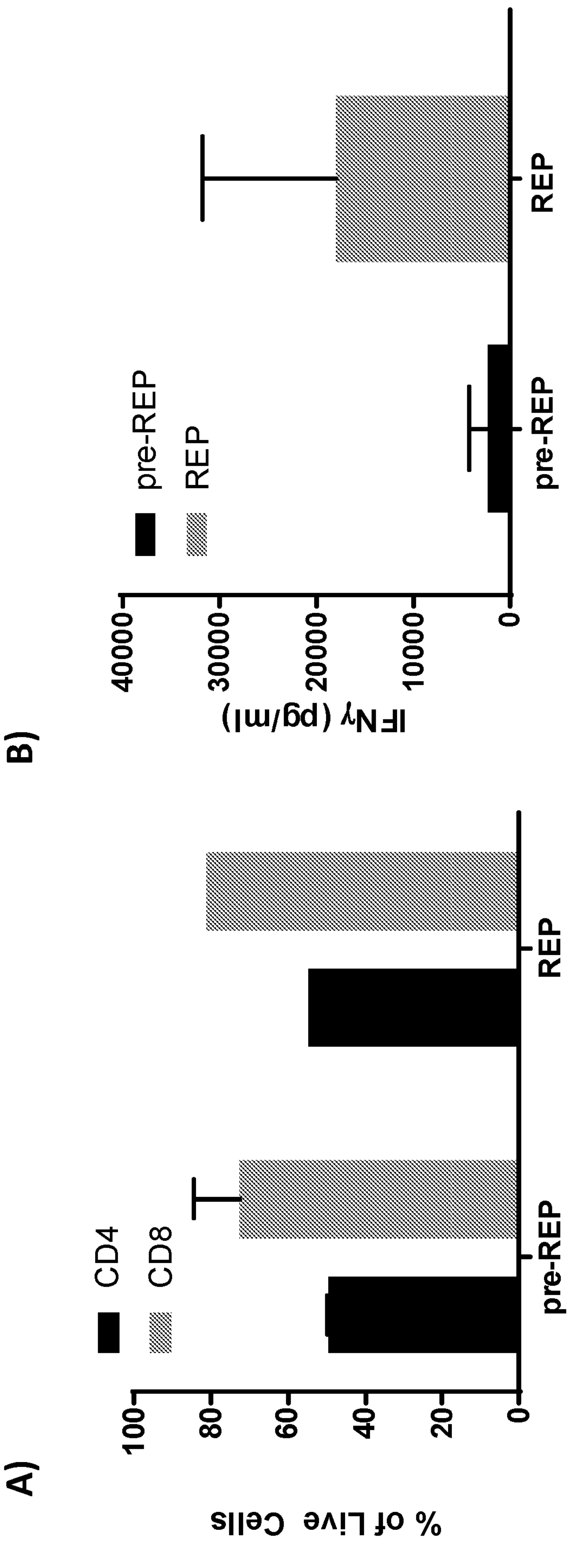

A)
% of Live Cells
100
80
60
40
20
0
CD4
CD8
pre-REP
REP
B)
IFNγ (pg/ml)
40000
30000
20000
10000
0
pre-REP
REP
pre-REP
REP

*Figure 23*

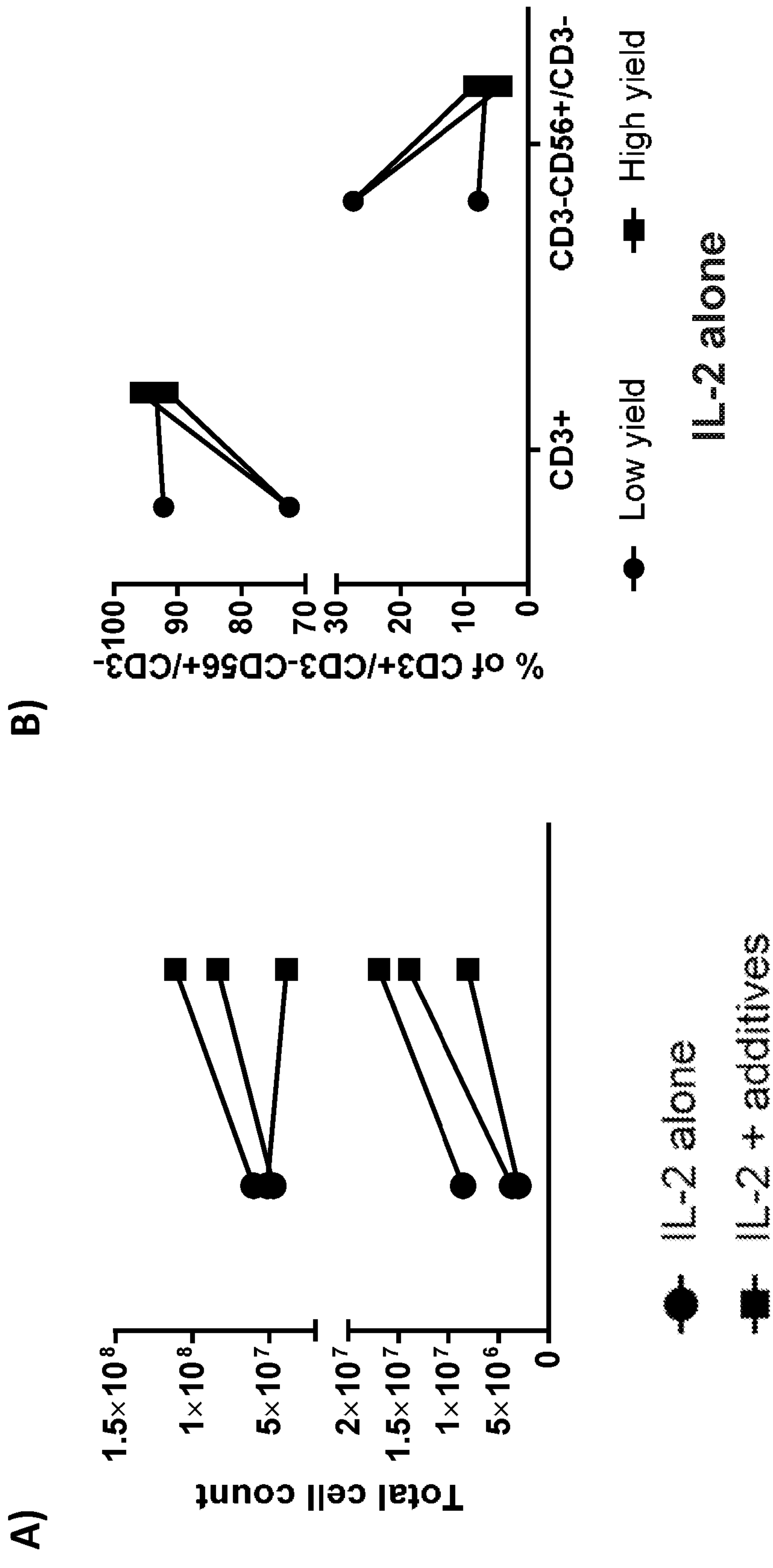
A)
Total cell count
1.5×10^8
1×10^8
5×10^7
2×10^7
1.5×10^7
1×10^7
5×10^6
0
IL-2 alone
IL-2 + additives
n=6
B)
% of CD3+/CD3-CD56+/CD3-
100
90
80
70
30
20
10
0
CD3+
CD3-CD56+/CD3-
Low yield
High yield
IL-2 alone

*Figure 24*

*A)*

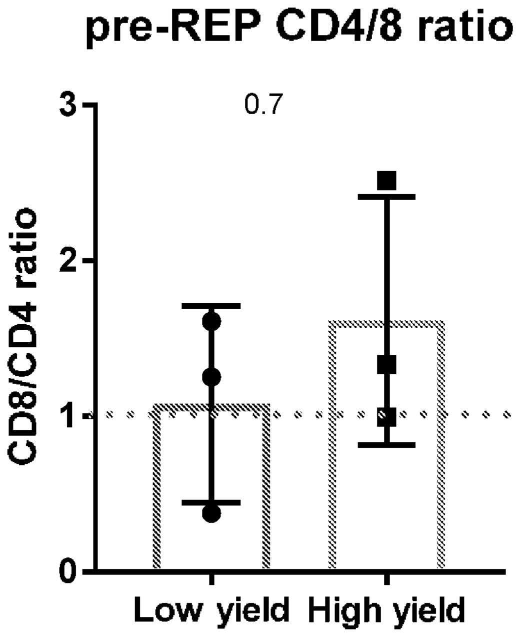
pre-REP CD4/8 ratio
0.7
CD8/CD4 ratio
3
2
1
0
Low yield
High yield

*B)*

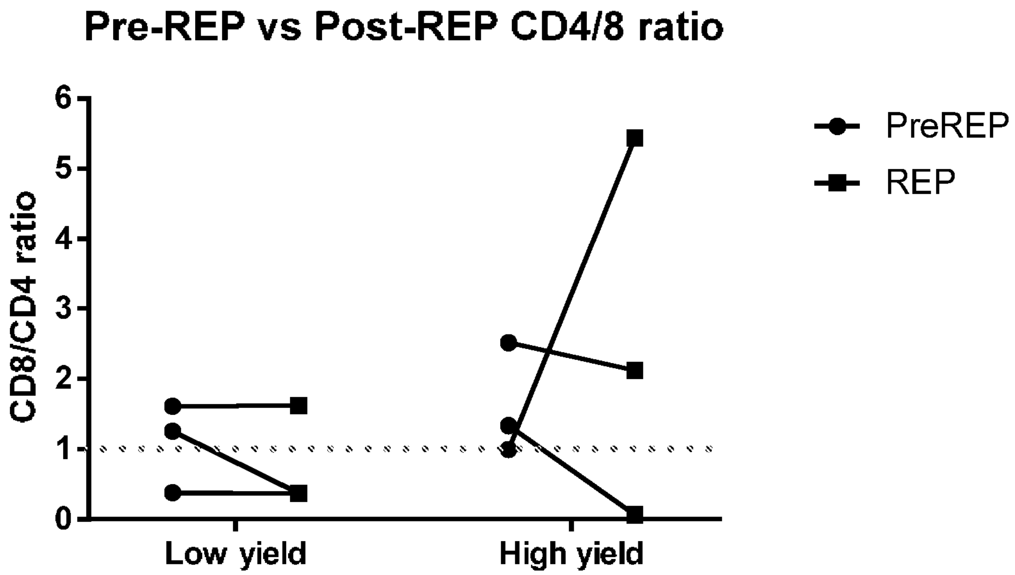
Pre-REP vs Post-REP CD4/8 ratio
CD8/CD4 ratio
6
5
4
3
2
1
0
Low yield
High yield
PreREP
REP

*Figure 25*

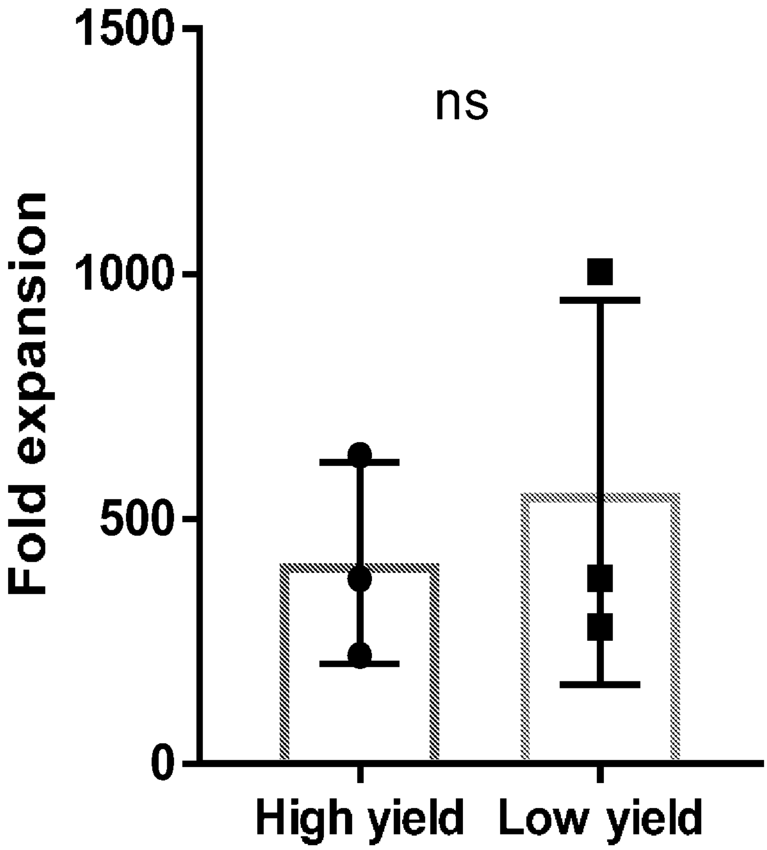


n=6

*Figure 26*

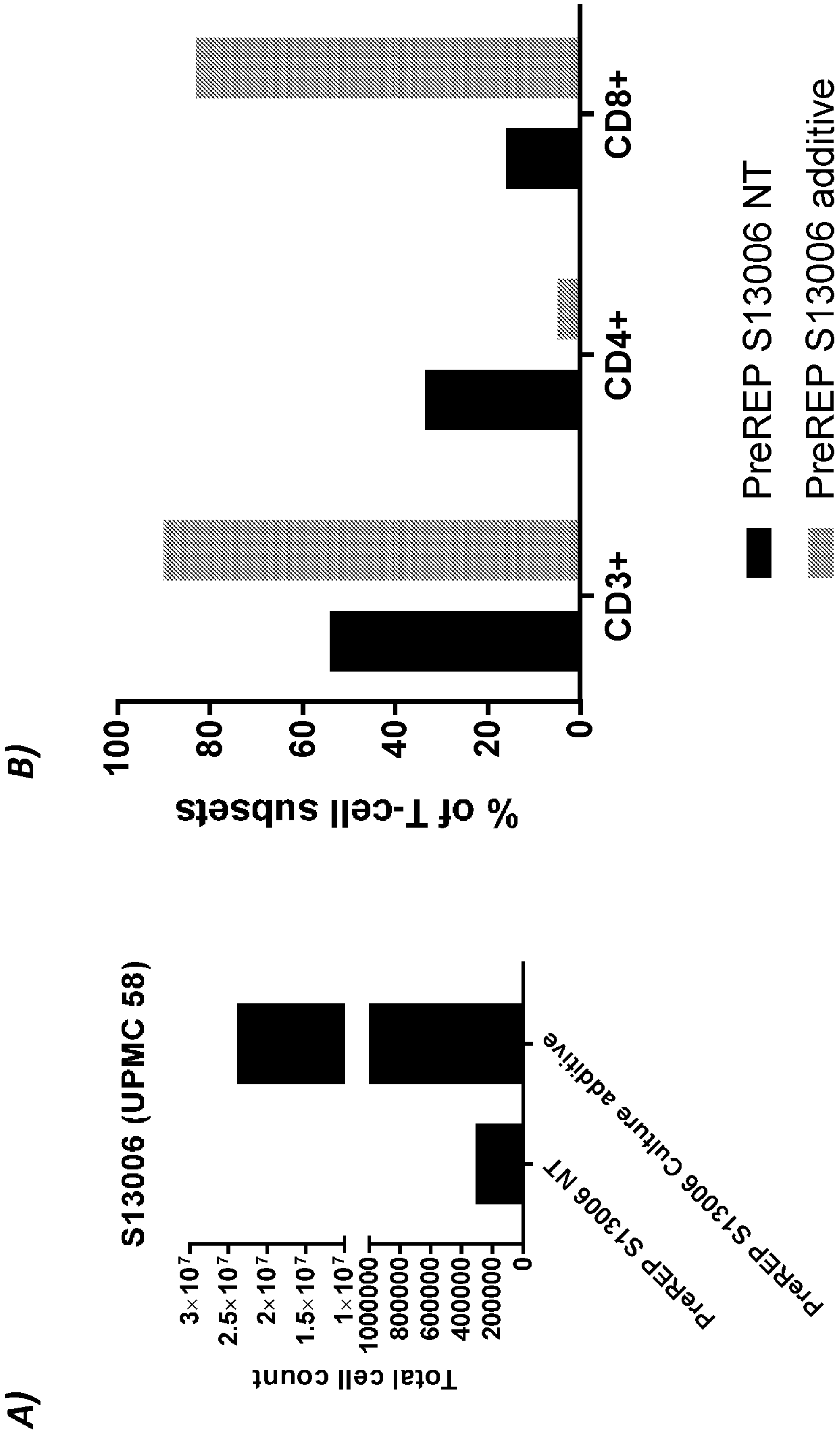
A)
S13006 (UPMC 58)
Total cell count
3×10⁷
2.5×10⁷
2×10⁷
1.5×10⁷
1×10⁷
1000000
800000
600000
400000
200000
0
PreREP S13006 NT
PreREP S13006 Culture additive
B)
% of T-cell subsets
100
80
60
40
20
0
CD3+
CD4+
CD8+
PreREP S13006 NT
PreREP S13006 additive

*Figure 27*

*A)*

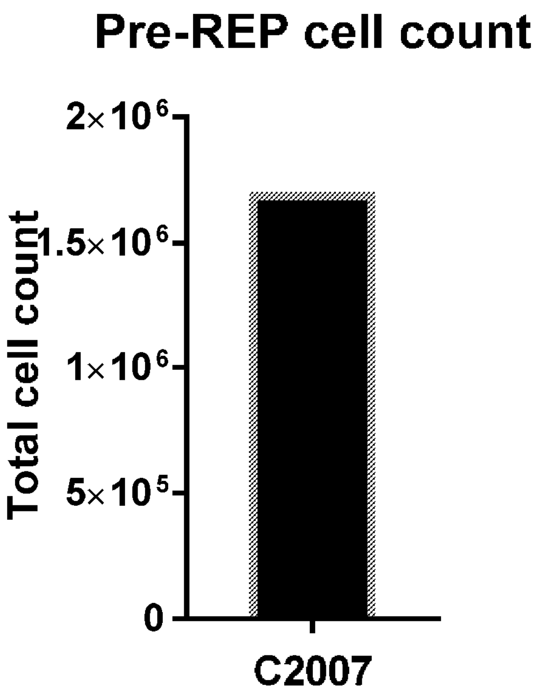
Pre-REP cell count
Total cell count
2×10^6
1.5×10^6
1×10^6
5×10^5
0
C2007

*B)*

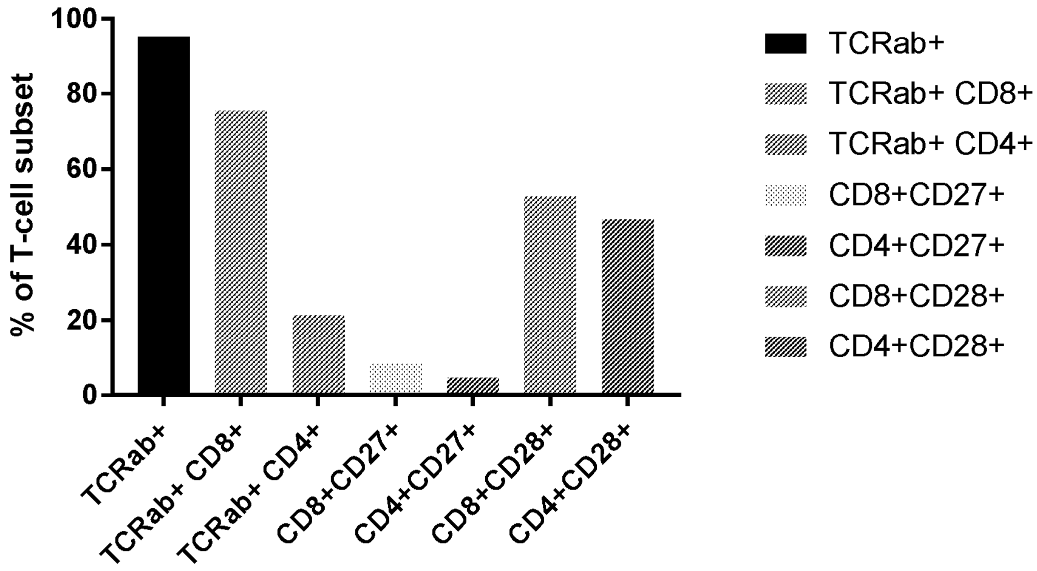
% of T-cell subset
100
80
60
40
20
0
TCRab+
TCRab+ CD8+
TCRab+ CD4+
CD8+CD27+
CD4+CD27+
CD8+CD28+
CD4+CD28+

*Figure 28*

A) B)

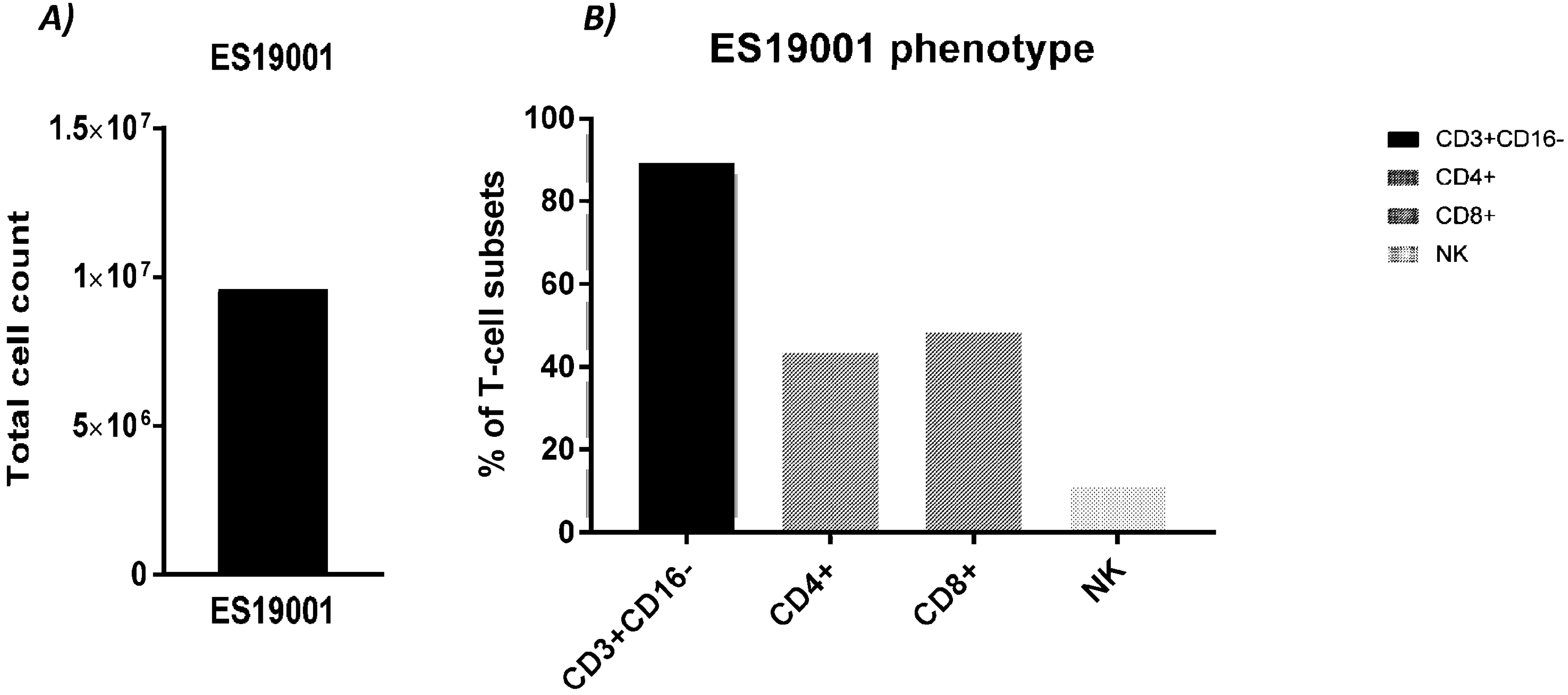
ES19001
Total cell count
1.5×10⁷
1×10⁷
5×10⁶
0
ES19001
ES19001 phenotype
% of T-cell subsets
100
80
60
40
20
0
CD3+CD16-
CD4+
CD8+
NK
CD3+CD16-
CD4+
CD8+
NK

*Figure 29*

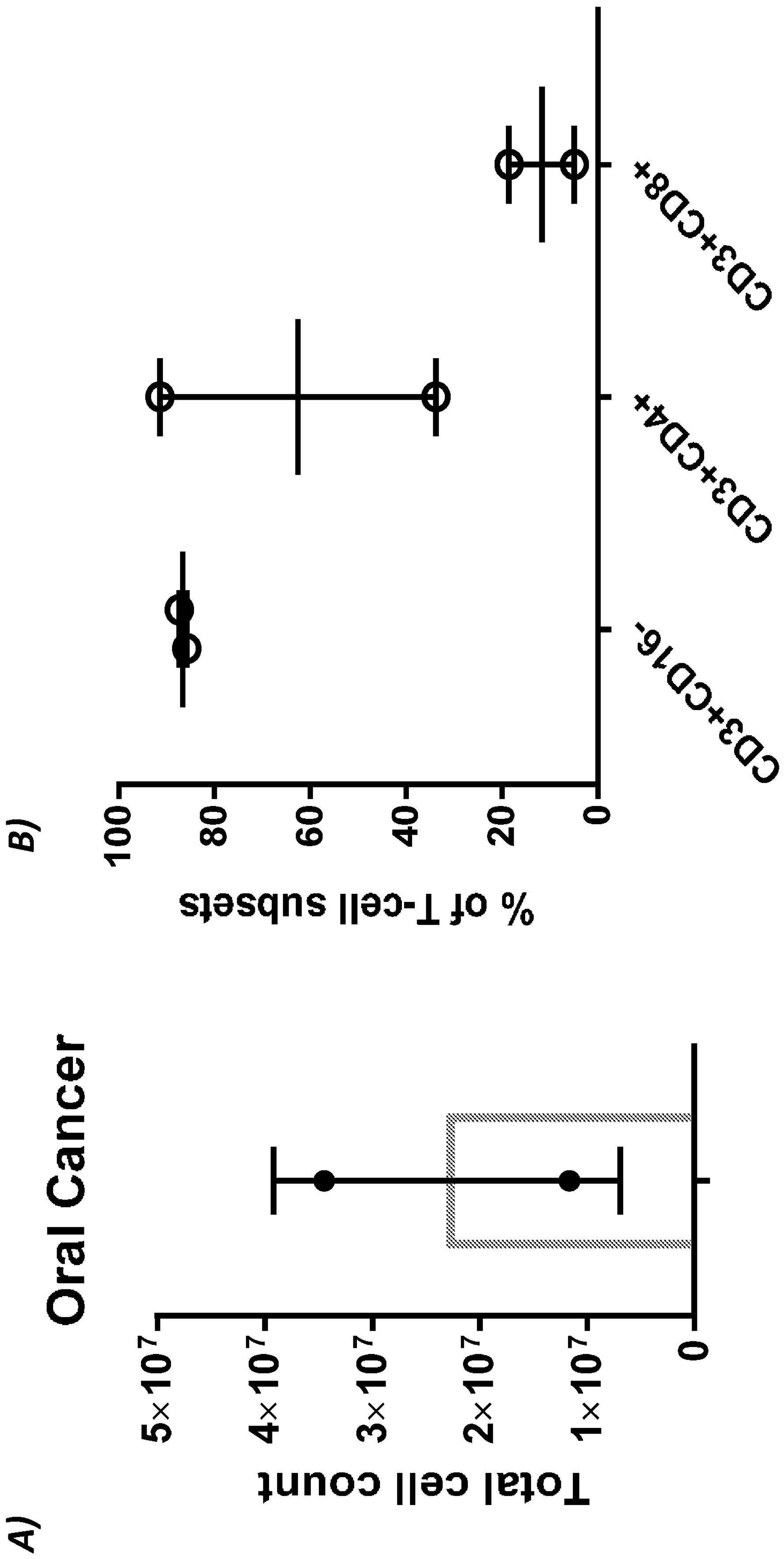
A)
Oral Cancer
Total cell count
5×10^7
4×10^7
3×10^7
2×10^7
1×10^7
0
B)
% of T-cell subsets
100
80
60
40
20
0
CD3+CD16-
CD3+CD4+
CD3+CD8+

*Figure 30*

| Tumor Name | Cell Growth | Analysis |
|---|---|---|
| UPMC-3 (P7011)<br>Reported | 24 well plate: 1) IL-2 versus 2) Cytokine Additives | Cell growth, viability, phenotyping, CD107a |
| UPMC-9 (P7017)<br>Reported | Expanded poorly | N/A |
| UPMC-17 (P7019)<br>Reported | Contaminated culture. Discarded. | N/A |
| UPMC-26 (P7022) | Expanded poorly | N/A |
| UPMC-30 (P7028) | pre-REP Day 12: 1.6e6, 40% viable<br>pre-REP Day 21: 3.49e7, 89% viable | Cell growth, viability, phenotyping, CD107a, IFNγ, REP initiation and analysis |
| UPMC-61 (P7031) | pre-REP Day 12: 1.52e5, 66% viable<br>pre-REP Day 28: 1.14e7, 1.14e7, 40% viable | Cell growth, viability, phenotyping, CD107a, IFNγ, REP initiation and analysis |
| UPMC-59 (P7033) | Expanded poorly | N/A |
| UPMC-61 (P7034) | pre-REP: In progress | |

Figure 31

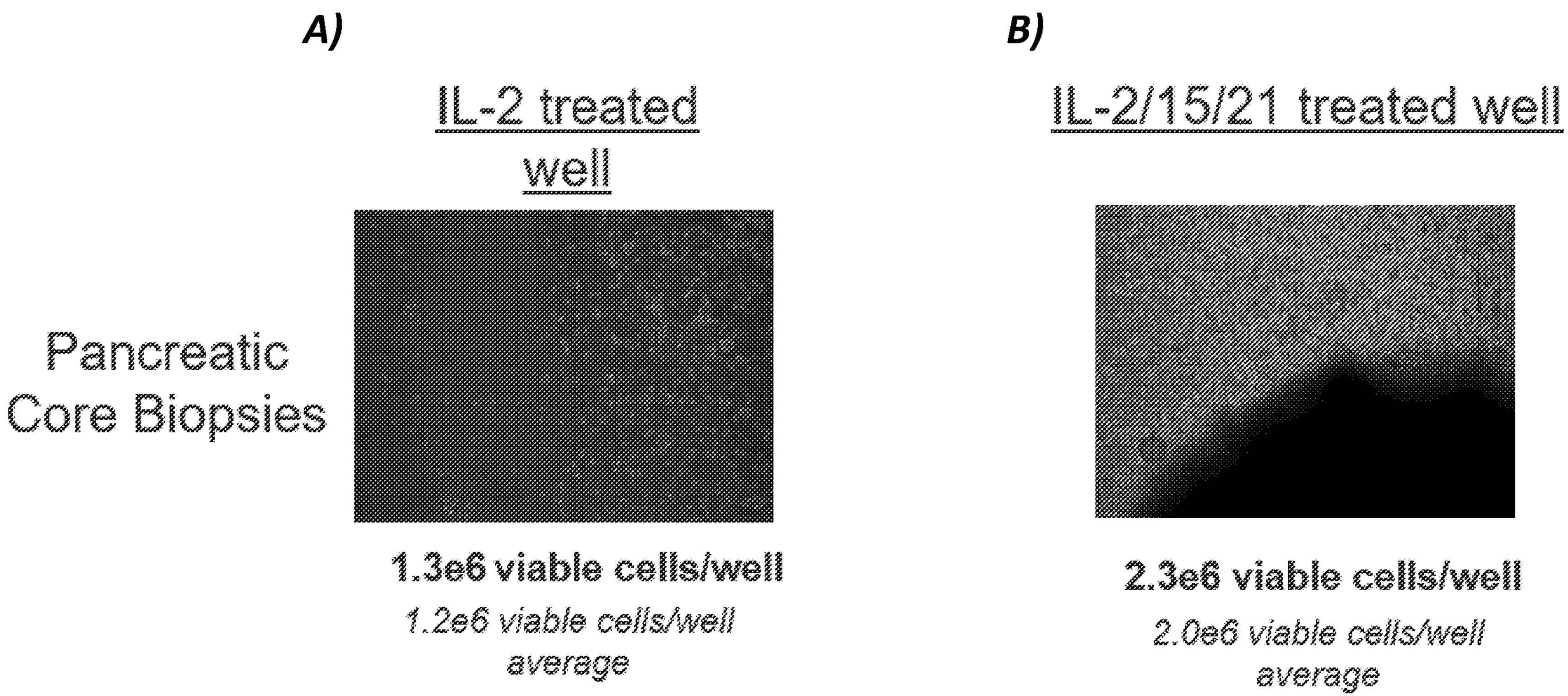
A)
IL-2 treated well
Pancreatic Core Biopsies
1.3e6 viable cells/well
1.2e6 viable cells/well average
B)
IL-2/15/21 treated well
2.3e6 viable cells/well
2.0e6 viable cells/well average

*Figure 32*

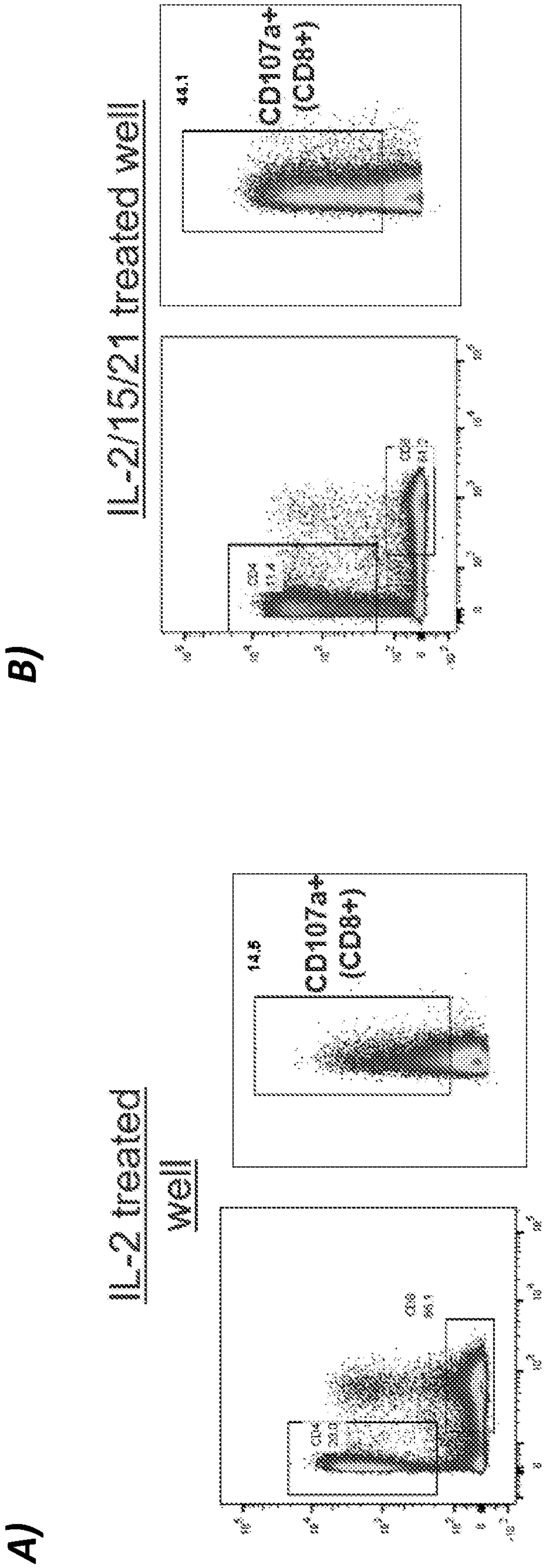
A)
IL-2 treated well
CD4
CD8
14.5
CD107a+ (CD8+)
B)
IL-2/15/21 treated well
CD4
11.4
CD8
44.1
CD107a+ (CD8+)

Figure 33

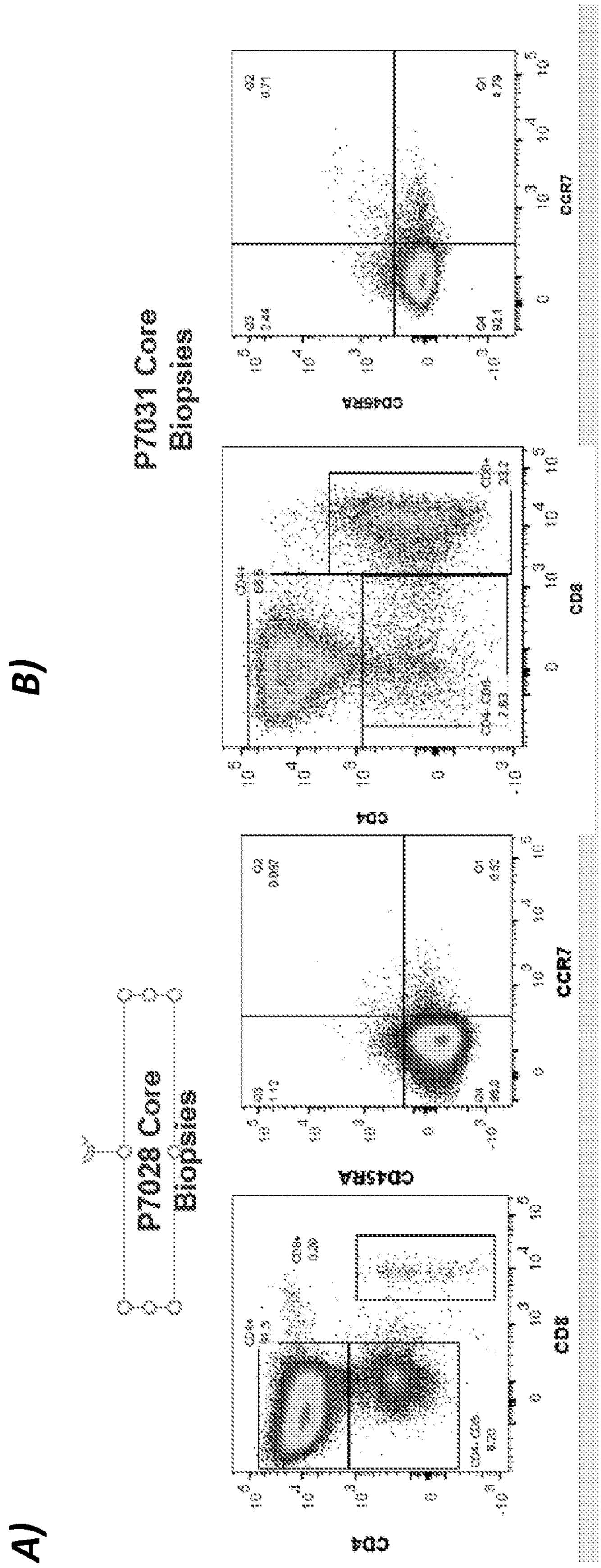
A)
P7028 Core Biopsies
CD4
CD8
CD45RA
CCR7
B)
P7031 Core Biopsies
CD4
CD8
CD45RA
CCR7

*Figure 34*

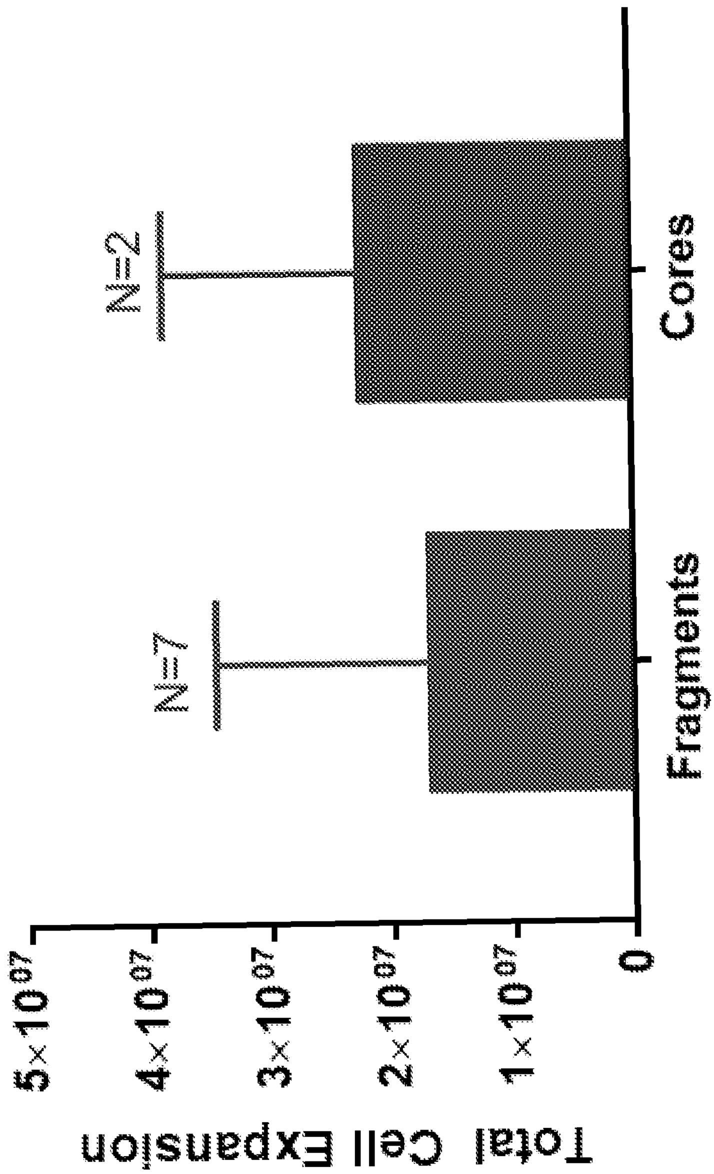
Total Cell Expansion
5×10⁰⁷
4×10⁰⁷
3×10⁰⁷
2×10⁰⁷
1×10⁰⁷
0
N=7
N=2
Fragments
Cores

*Figure 35*

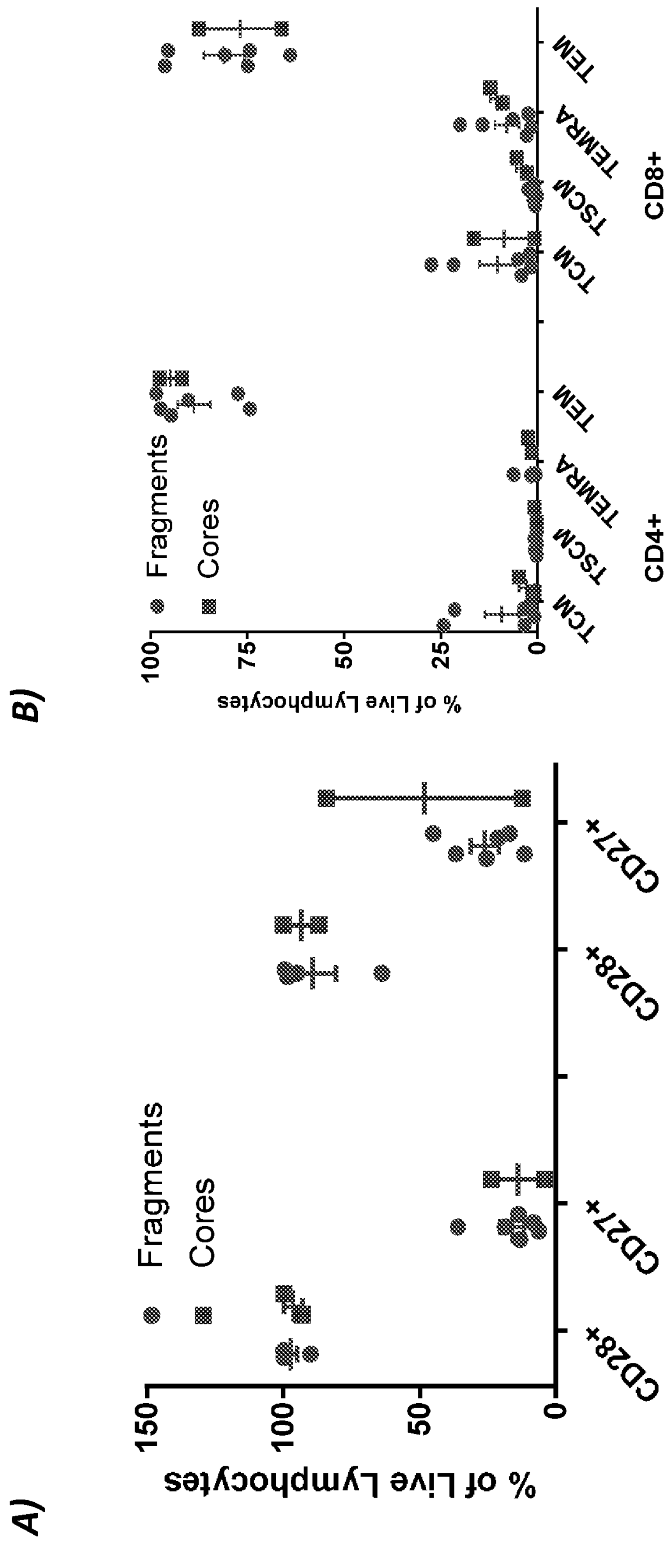
A)
% of Live Lymphocytes
150
100
50
0
Fragments
Cores
CD28+
CD27+
CD28+
CD27+
B)
% of Live Lymphocytes
100
75
50
25
0
Fragments
Cores
TCM
TSCM
TEMRA
TEM
TCM
TSCM
TEMRA
TEM
CD4+
CD8+

*Figure 36*

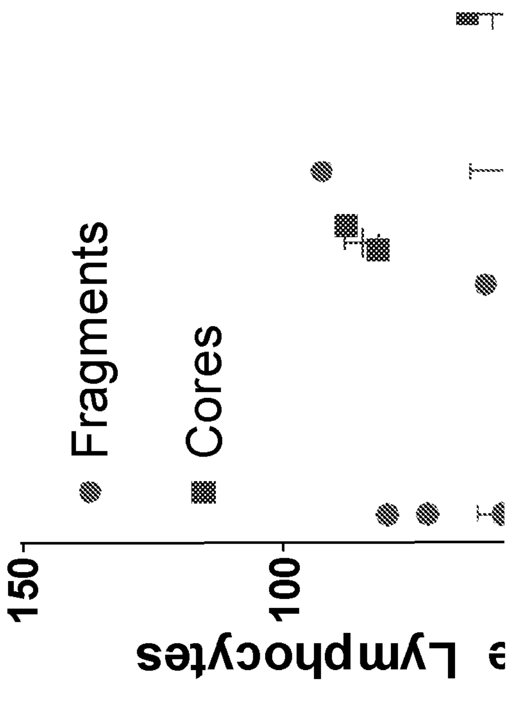

*Figure 37*

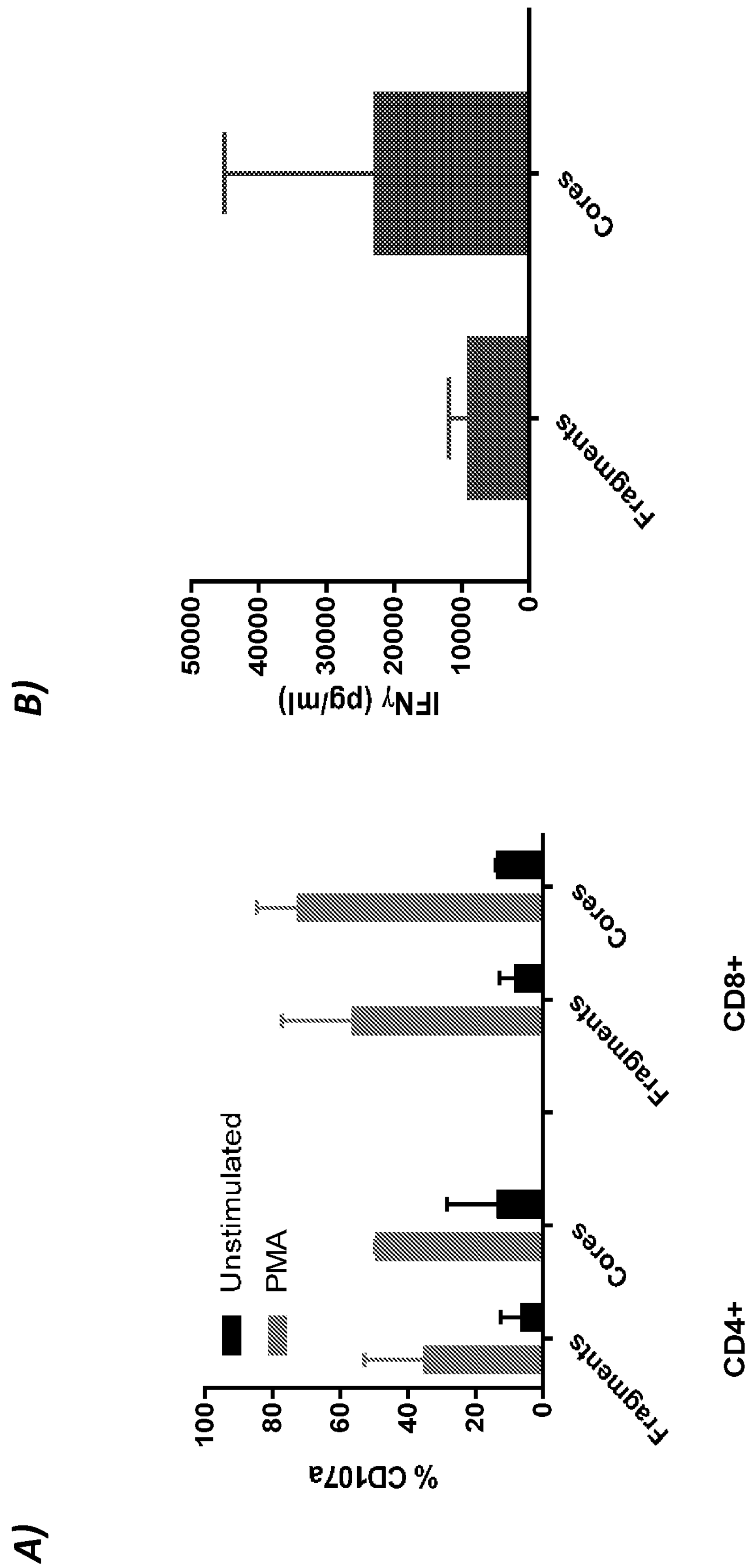
A)
% CD107a
100
80
60
40
20
0
Unstimulated
PMA
Fragments
Cores
Fragments
Cores
CD4+
CD8+
B)
IFNγ (pg/ml)
50000
40000
30000
20000
10000
0
Fragments
Cores

*Figure 38A*

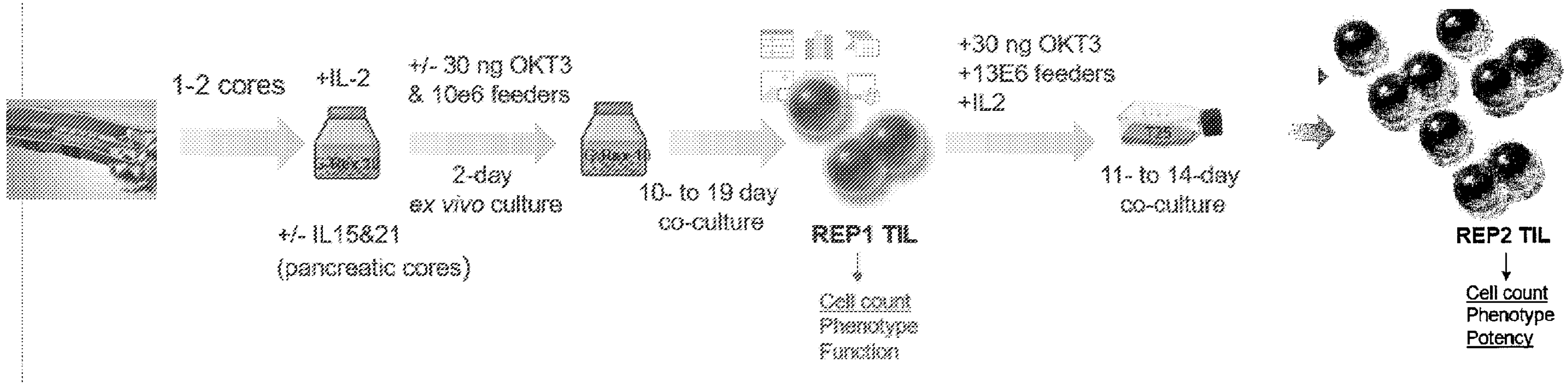
1-2 cores
+IL-2
+/- IL15&21
(pancreatic cores)
+/- 30 ng OKT3
& 10e6 feeders
2-day
*ex vivo* culture
10- to 19 day
co-culture
REP1 TIL
Cell count
Phenotype
Function
+30 ng OKT3
+13E6 feeders
+IL2
T25
11- to 14-day
co-culture
REP2 TIL
Cell count
Phenotype
Potency

*Figure 38B*

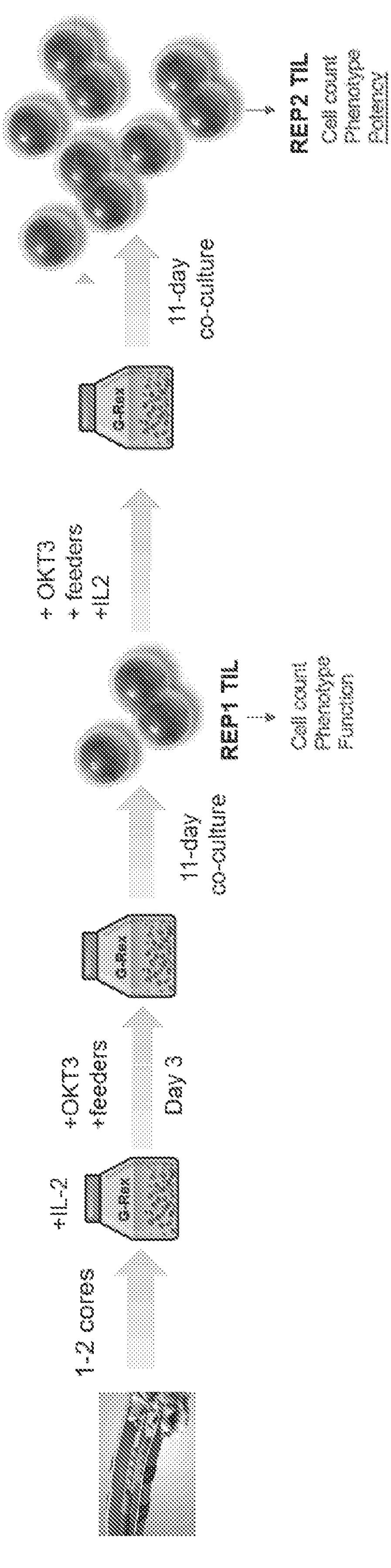
1-2 cores
+IL-2
G-Rex
+OKT3
+feeders
Day 3
G-Rex
11-day
co-culture
REP1 TIL
Cell count
Phenotype
Function
+ OKT3
+ feeders
+IL2
G-Rex
11-day
co-culture
REP2 TIL
Cell count
Phenotype
Potency

*Figure 39*

| | Culture conditions | Cores / cond. (#) | REP1 | REP2 |
|---|---|---|---|---|
| Pancreas (P7034) | REP1: + TC +OKT3 & feeders vs NO OKT3 & feeders<br>REP2: +/- triple cocktail | 2 (2 conditions) | **>50E6 cells** at Day 17 ONLY in the presence of OKT3 & feeders | **207- & 162-fold** expansion +/- triple cocktail **~3000 pg/ml IFNγ** +/- triple cocktail |
| Pancreas (P7037) | REP1: IL2 versus TC<br>REP2: +/- triple cocktail | 4 (2 conditions) | **>50E6 cells** at Day 11 only in the presence of OKT3 & feeders +/- TC | **207- & 231-fold** expansion +/- triple cocktail **~1000 pg/ml IFNγ**+/- triple cocktail |
| Pancreas (P7039) | REP1:<br>TC + OKT3& feeders, TC NO OKT3 & feeders<br>IL-2 + OKT3& feeders, IL-2 NO OKT3& feeders<br>REP2: +/- TC | 2 (4 conditions) | **>50E6 cells** at Day 12 (+/- TC) ONLY in the presence of OKT3 & feeders | **662- & 582-fold** expansion +/- triple cocktail **~10K & 3K pg/ml IFNγ**+/- triple cocktail |

*Figure 40*

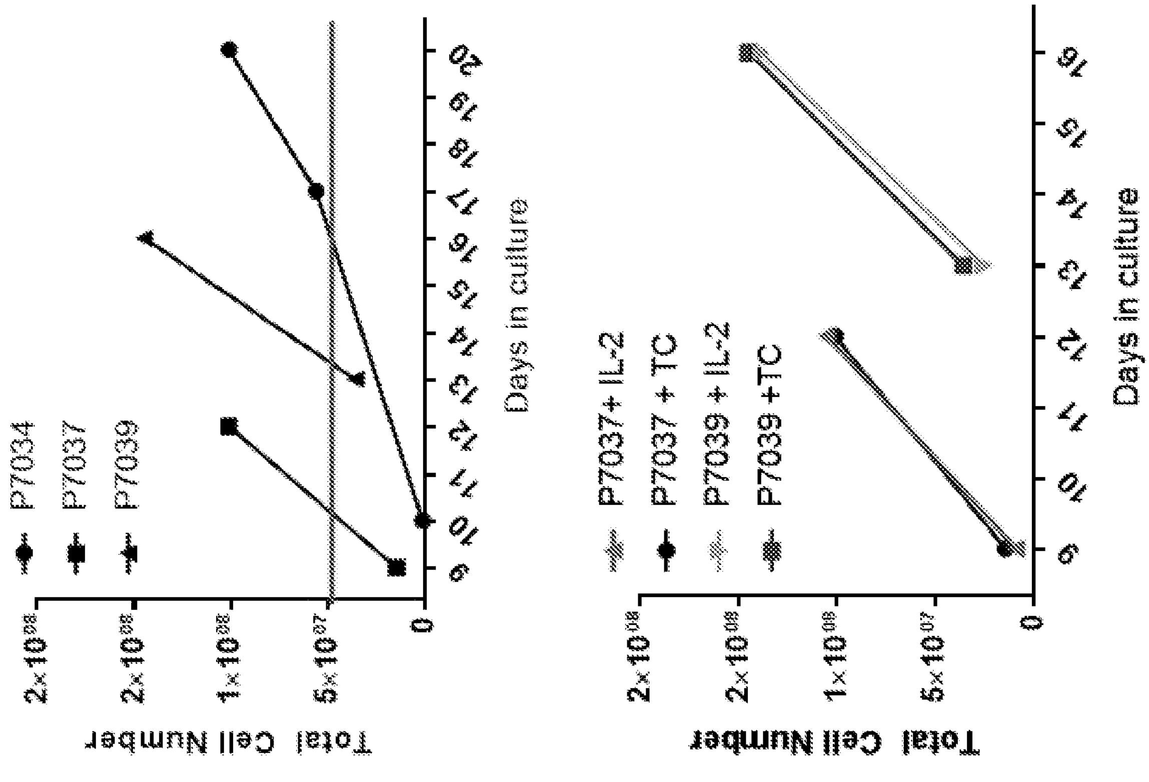
P7034
P7037
P7039
Total Cell Number
2×10^08
2×10^08
1×10^08
5×10^07
0
Days in culture
9
10
11
12
13
14
15
16
17
18
19
20
P7037+ IL-2
P7037 + TC
P7039 + IL-2
P7039 +TC
Total Cell Number
2×10^08
2×10^08
1×10^08
5×10^07
0
Days in culture
9
10
11
12
13
14
15
16

*Figure 41*

| | Culture conditions | Cores / cond. (#) | REP1 | REP2 |
|---|---|---|---|---|
| Melanoma (M1114) | REP1: IL2<br>REP2: IL-2 | 2 | **>50E6 cells** at Day 11 | **215-fold** expansion **~1000 pg/ml IFNγ** |
| Melanoma (M1115) | REP1: IL2<br>REP2: IL-2 | 2 | **>50E6 cells** at Day 12 | **662-fold** expansion **~3000 pg/ml IFNγ** |
| Melanoma (M1118) | REP1: IL-2<br>REP2: IL-2 | 1 | **>50E6 cells** at Day 11 | **215-fold** expansion **~10,000 pg/ml IFNγ** |
| ** Melanoma (M1121) | REP1: IL2<br>REP2: IL-2 | 1 | **50E6 cells** at Day 9 | **155-fold** expansion **~2000 pg/ml IFNγ** |

*Figure 42*

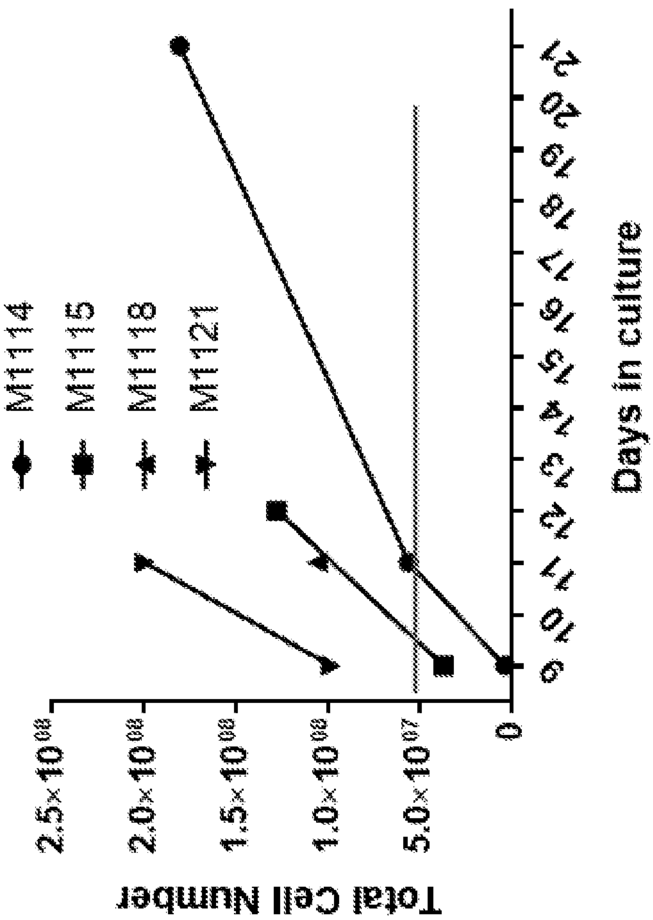
M1114
M1115
M1118
M1121
Total Cell Number
2.5×10^08
2.0×10^08
1.5×10^08
1.0×10^08
5.0×10^07
0
9
10
11
12
13
14
15
16
17
18
19
20
21
Days in culture

*Figure 43*

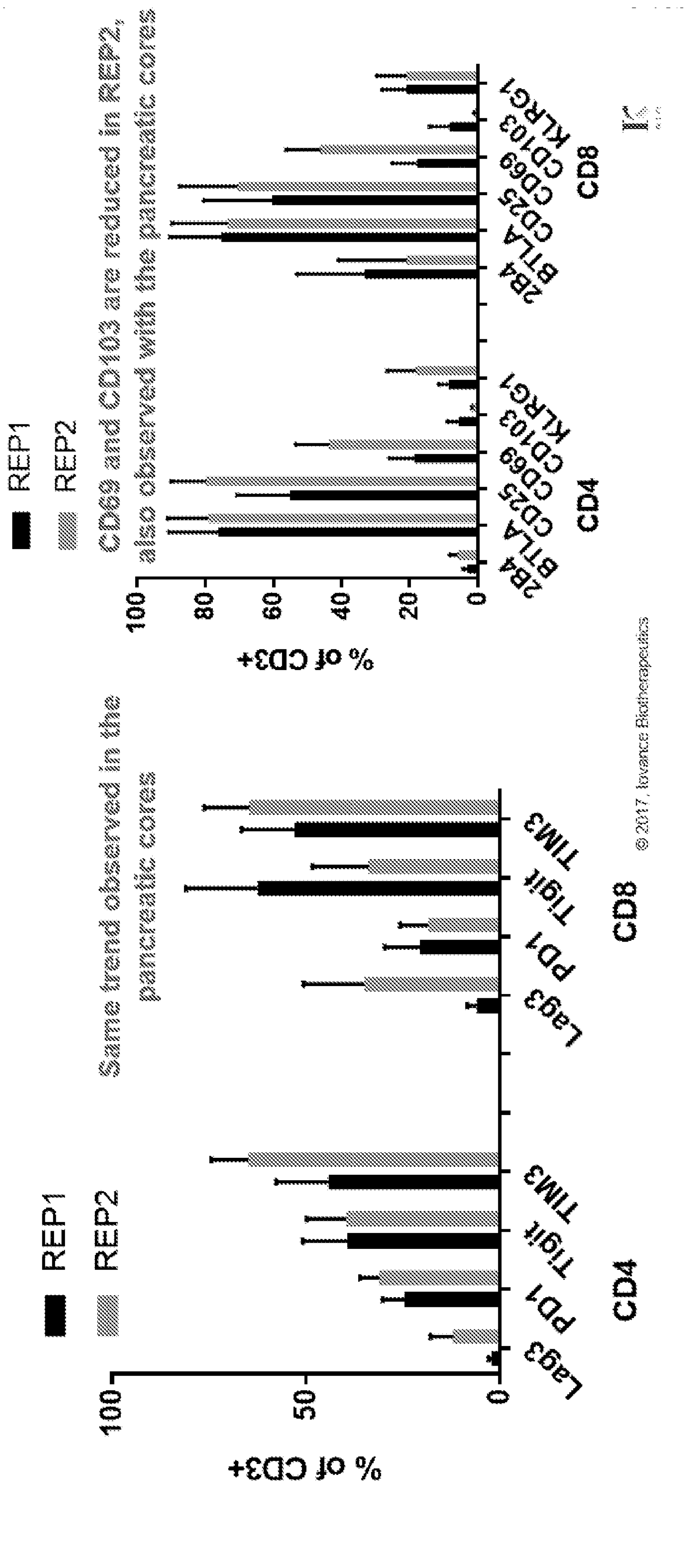
REP1
REP2
Same trend observed in the pancreatic cores
% of CD3+
100
50
0
Lag3
PD1
Tigit
TIM3
CD4
CD8
REP1
REP2
CD69 and CD103 are reduced in REP2, also observed with the pancreatic cores
% of CD3+
100
80
60
40
20
0
2B4
BTLA
CD25
CD69
CD103
KLRG1
CD4
CD8
© 2017, Iovance Biotherapeutics

*Figure 44*

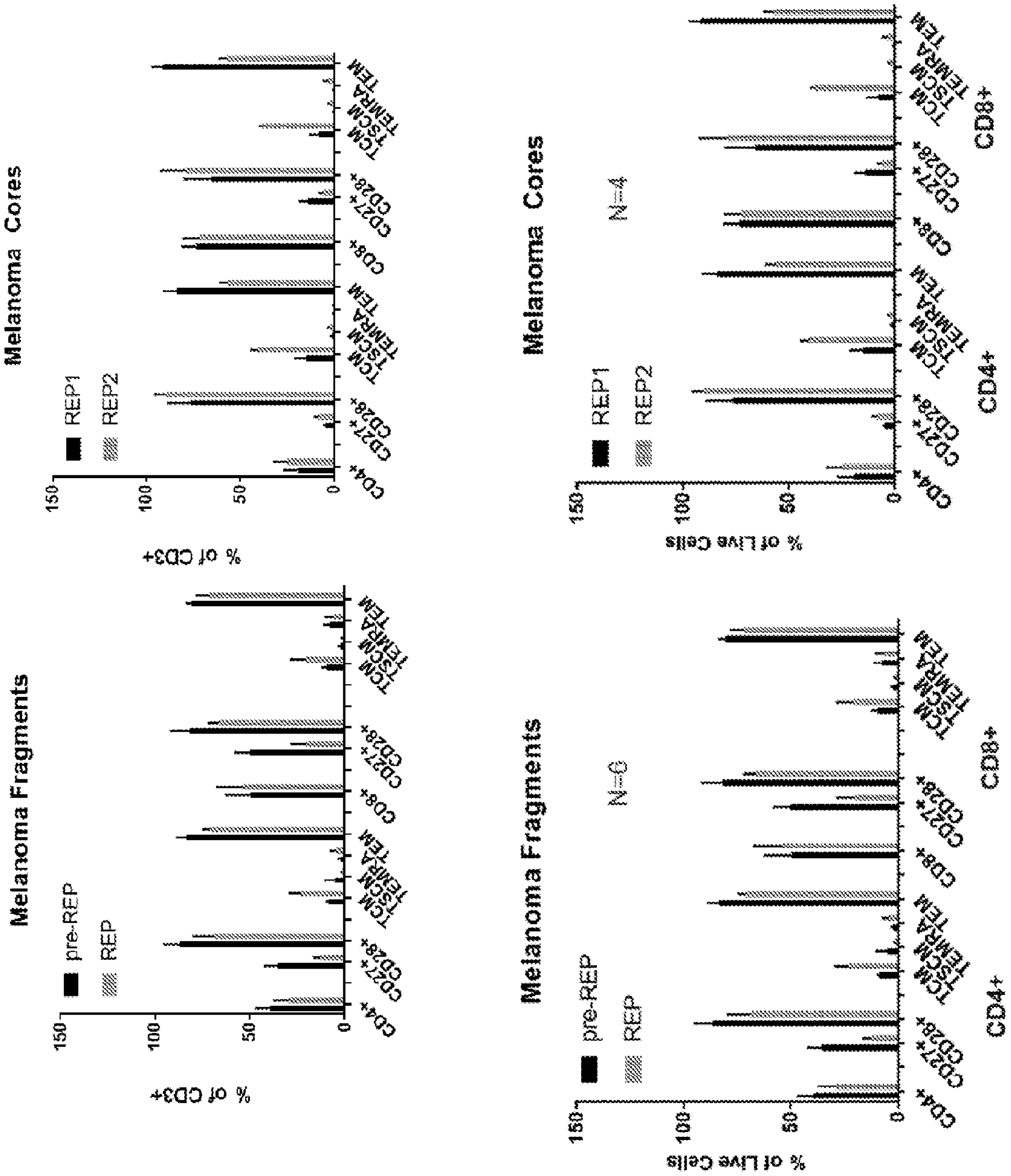
Melanoma Fragments
pre-REP
REP
% of CD3+
Melanoma Cores
REP1
REP2
% of CD3+
Melanoma Fragments
N=6
% of Live Cells
Melanoma Cores
N=4
% of Live Cells
CD4+
CD27+
CD28+
TCM
TSCM
TEMRA
TEM
CD8+

*Figure 45*

| | Culture conditions | Cores / cond. (#) | REP1 | REP2 |
|---|---|---|---|---|
| Breast (EP11032) | REP1: IL2<br>REP2: IL-2 | 1 vs 2 | **>50E6 cells** at Day 11 | **173 and 158-fold (1/2 cores) expansion ~ pg/ml IFNg** |
| Ovarian (OV8016) | REP1: IL2 +/- OX40<br>REP2: IL-2 +/- OX40 (in progress) | 2 | **>50E6 cells** at Day 11 (OX40 condition) | **In progress** |

*Figure 46*

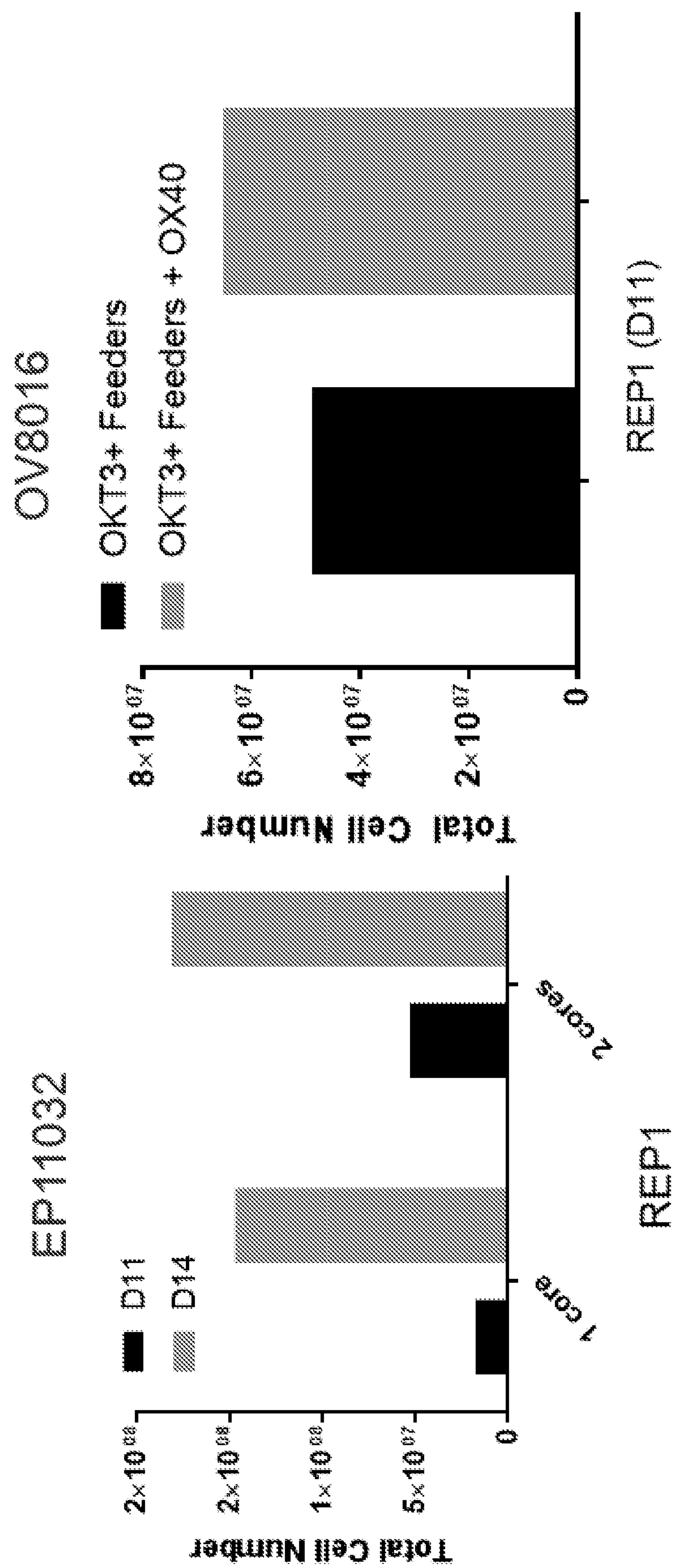
EP11032
D11
D14
Total Cell Number
2×10^08
2×10^08
1×10^08
5×10^07
0
1 core
2 cores
REP1
OV8016
OKT3+ Feeders
OKT3+ Feeders + OX40
Total Cell Number
8×10^07
6×10^07
4×10^07
2×10^07
0
REP1 (D11)

*Figure 47*

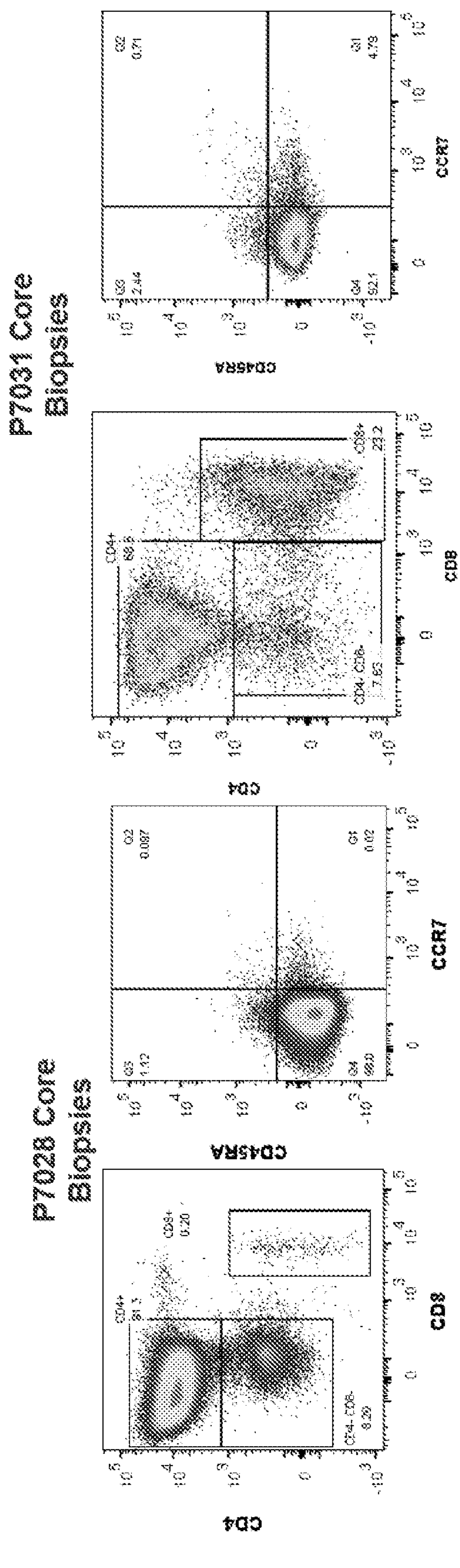
P7028 Core Biopsies
P7031 Core Biopsies
CD4
CD8
CD45RA
CCR7

*Figure 48*

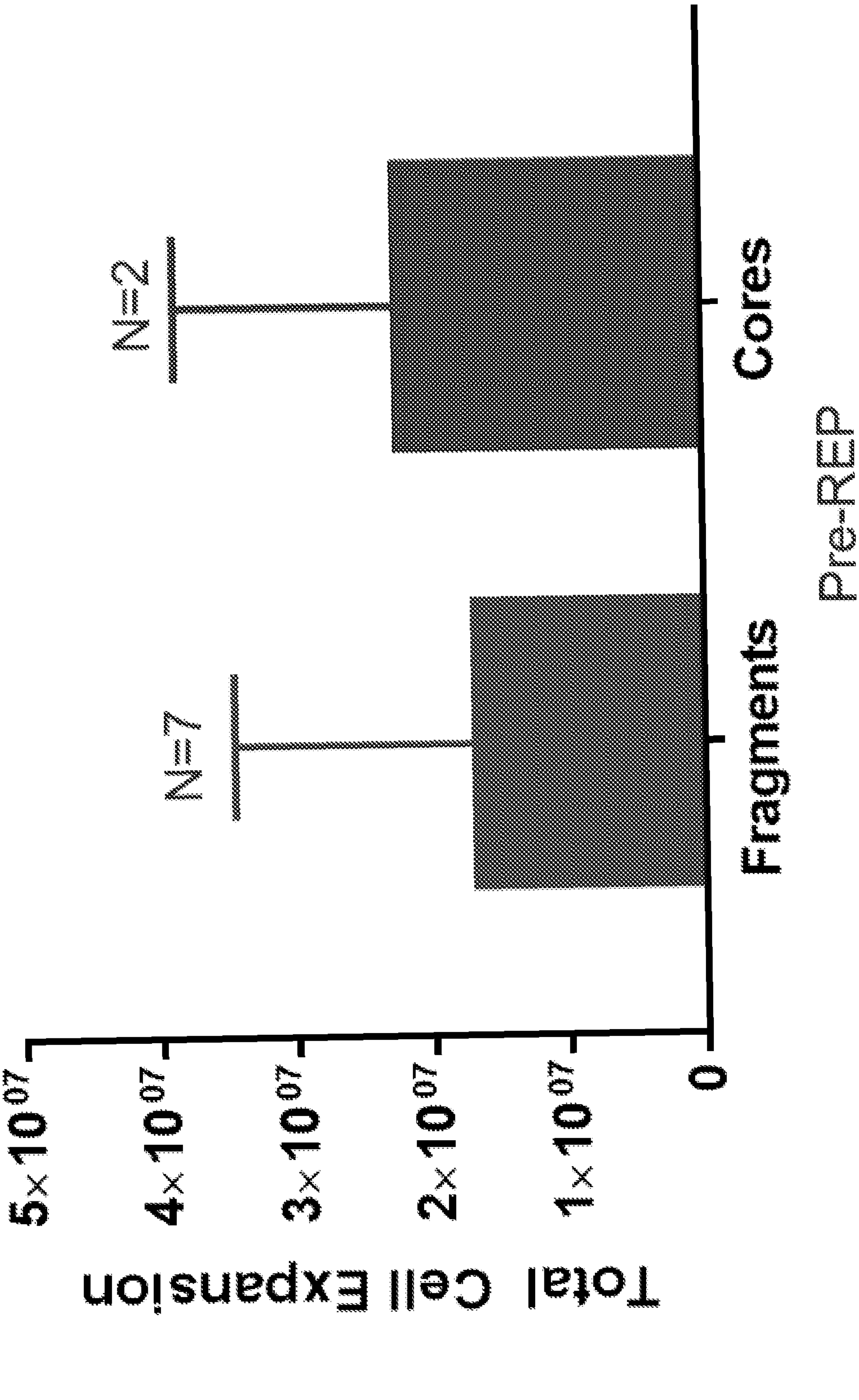
Total Cell Expansion
5×10⁰⁷
4×10⁰⁷
3×10⁰⁷
2×10⁰⁷
1×10⁰⁷
0
N=7
N=2
Fragments
Cores
Pre-REP

*Figure 49*

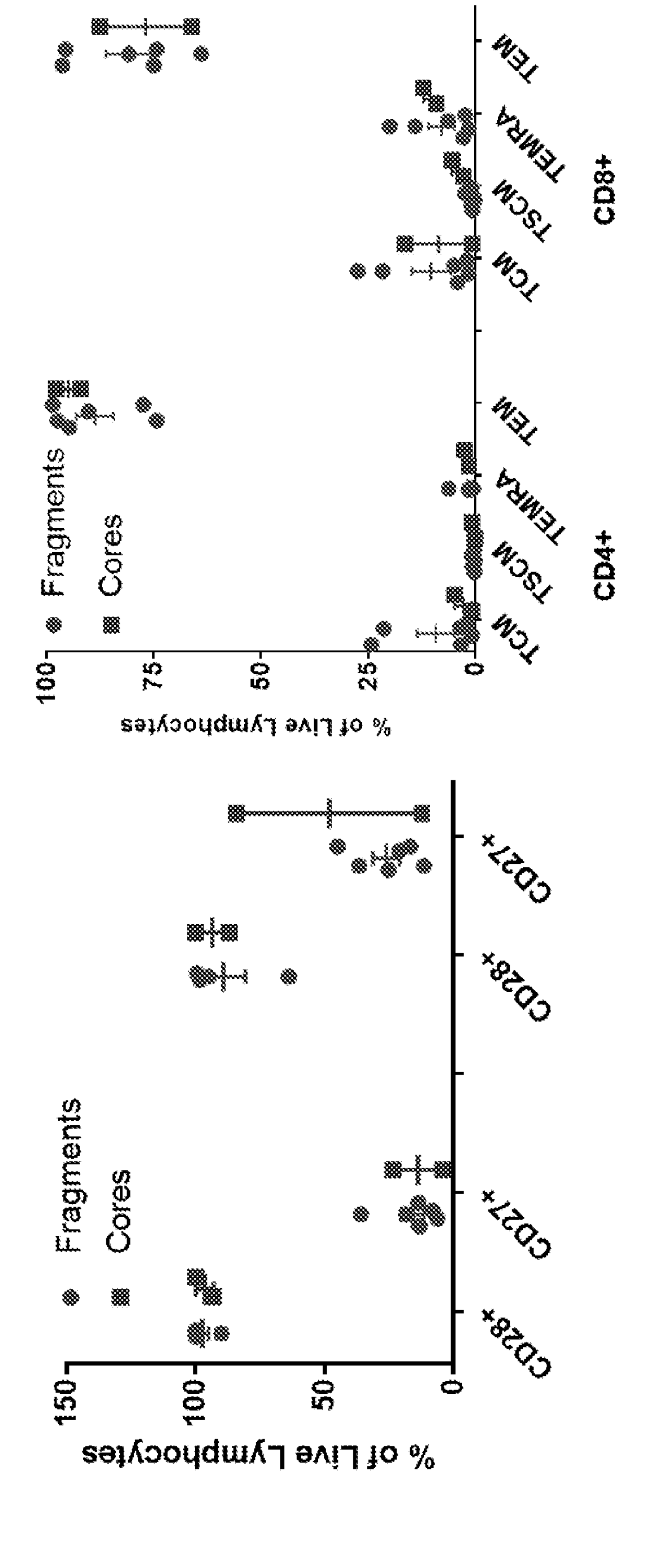
% of Live Lymphocytes
150
100
50
0
Fragments
Cores
CD28+
CD27+
CD28+
CD27+
% of Live Lymphocytes
100
75
50
25
0
Fragments
Cores
TCM
TSCM
TEMRA
TEM
TCM
TSCM
TEMRA
TEM
CD4+
CD8+

*Figure 50*

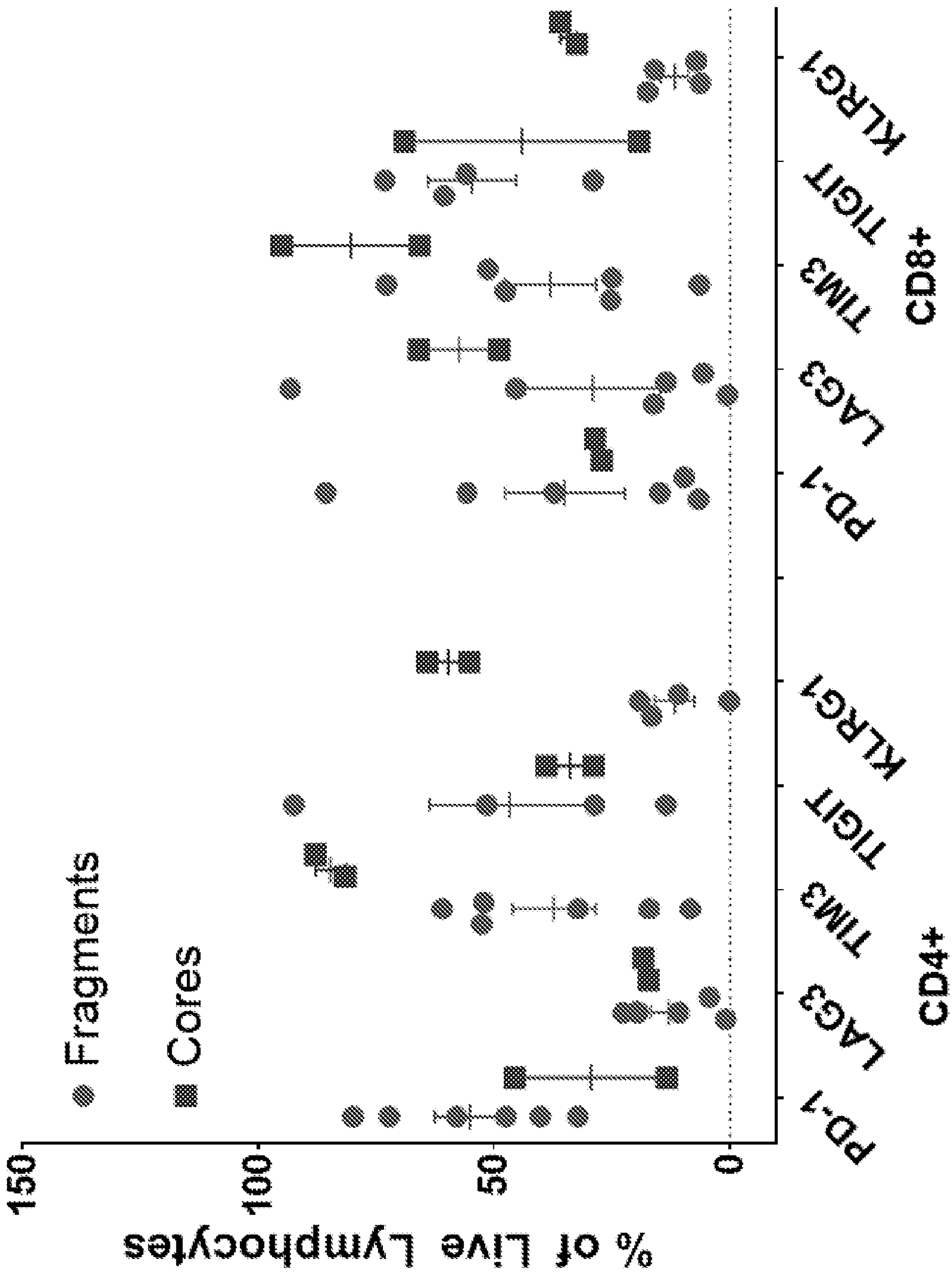
Fragments
Cores
% of Live Lymphocytes
150
100
50
0
PD-1
LAG3
TIM3
TIGIT
KLRG1
CD4+
PD-1
LAG3
TIM3
TIGIT
KLRG1
CD8+

*Figure 51*

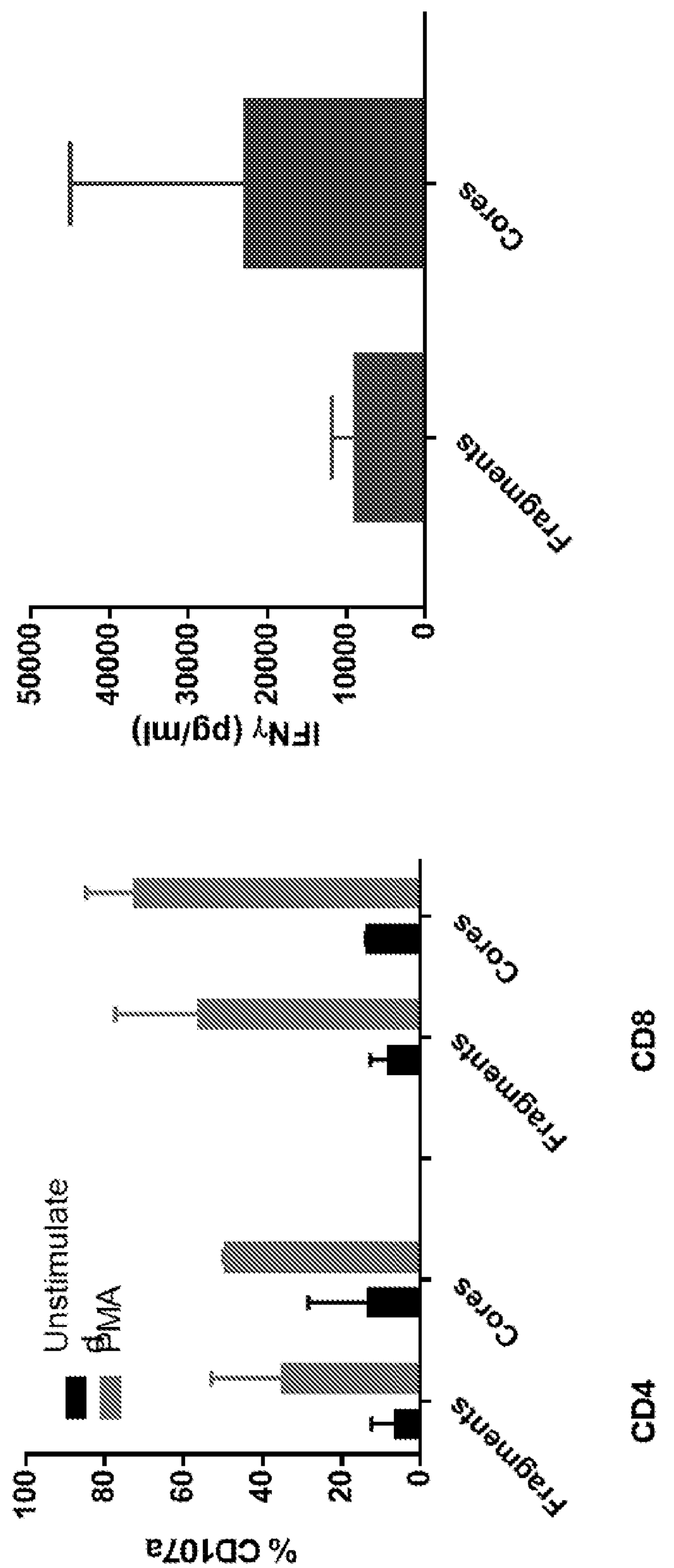
IFNγ (pg/ml)
50000
40000
30000
20000
10000
0
Fragments
Cores
% CD107a
100
80
60
40
20
0
Unstimulated
PMA
Fragments
Cores
Fragments
Cores
CD4
CD8

*Figure 52*

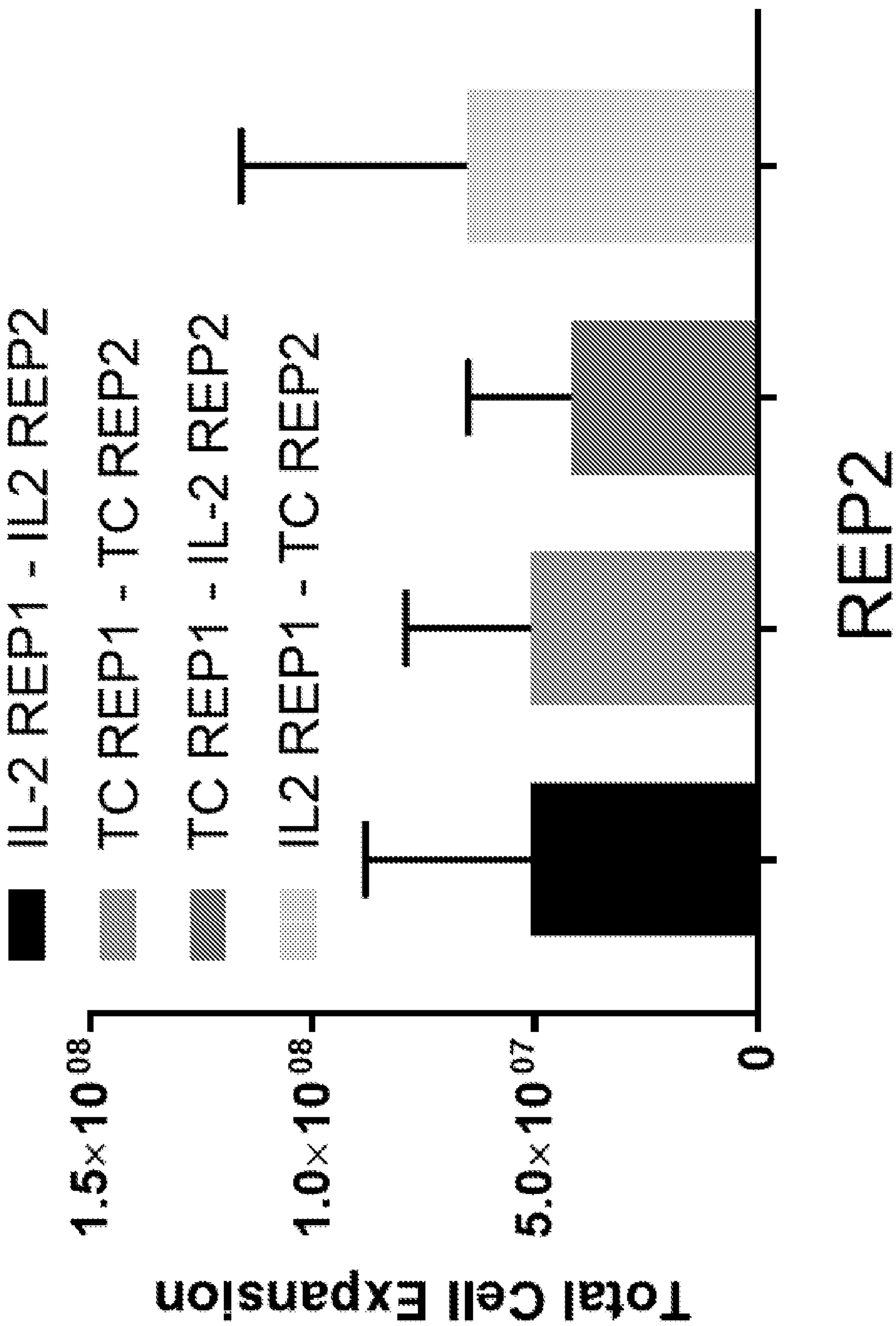
IL-2 REP1 - IL2 REP2
TC REP1 - TC REP2
TC REP1 - IL-2 REP2
IL2 REP1 - TC REP2
Total Cell Expansion
1.5×10⁰⁸
1.0×10⁰⁸
5.0×10⁰⁷
0
REP2

*Figure 53*

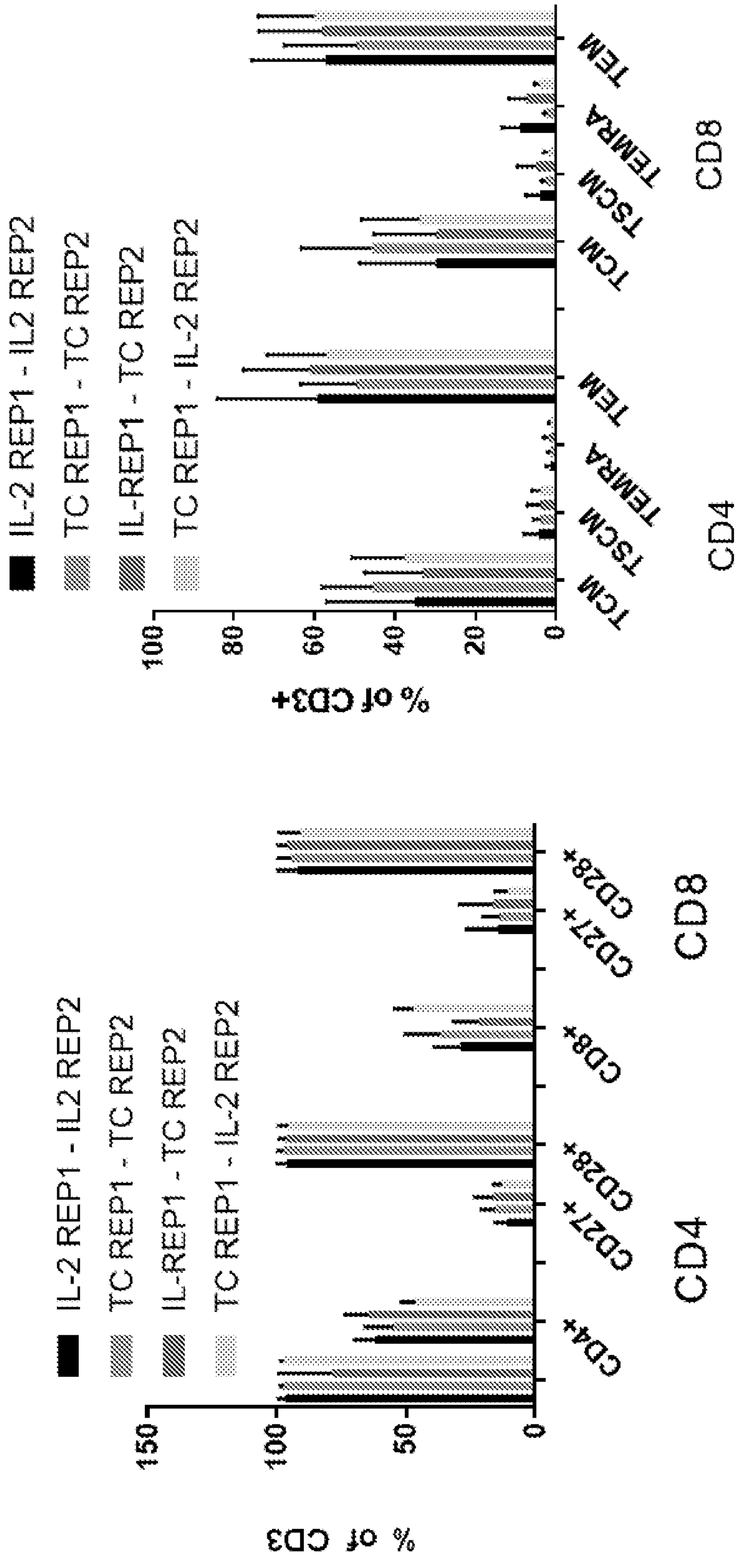
IL-2 REP1 - IL2 REP2
TC REP1 - TC REP2
IL-REP1 - TC REP2
TC REP1 - IL-2 REP2
% of CD3+
100
80
60
40
20
0
TCM
TSCM
TEMRA
TEM
CD4
CD8
% of CD3
150
100
50
CD4+
CD27+
CD28+
CD8+

*Figure 54*

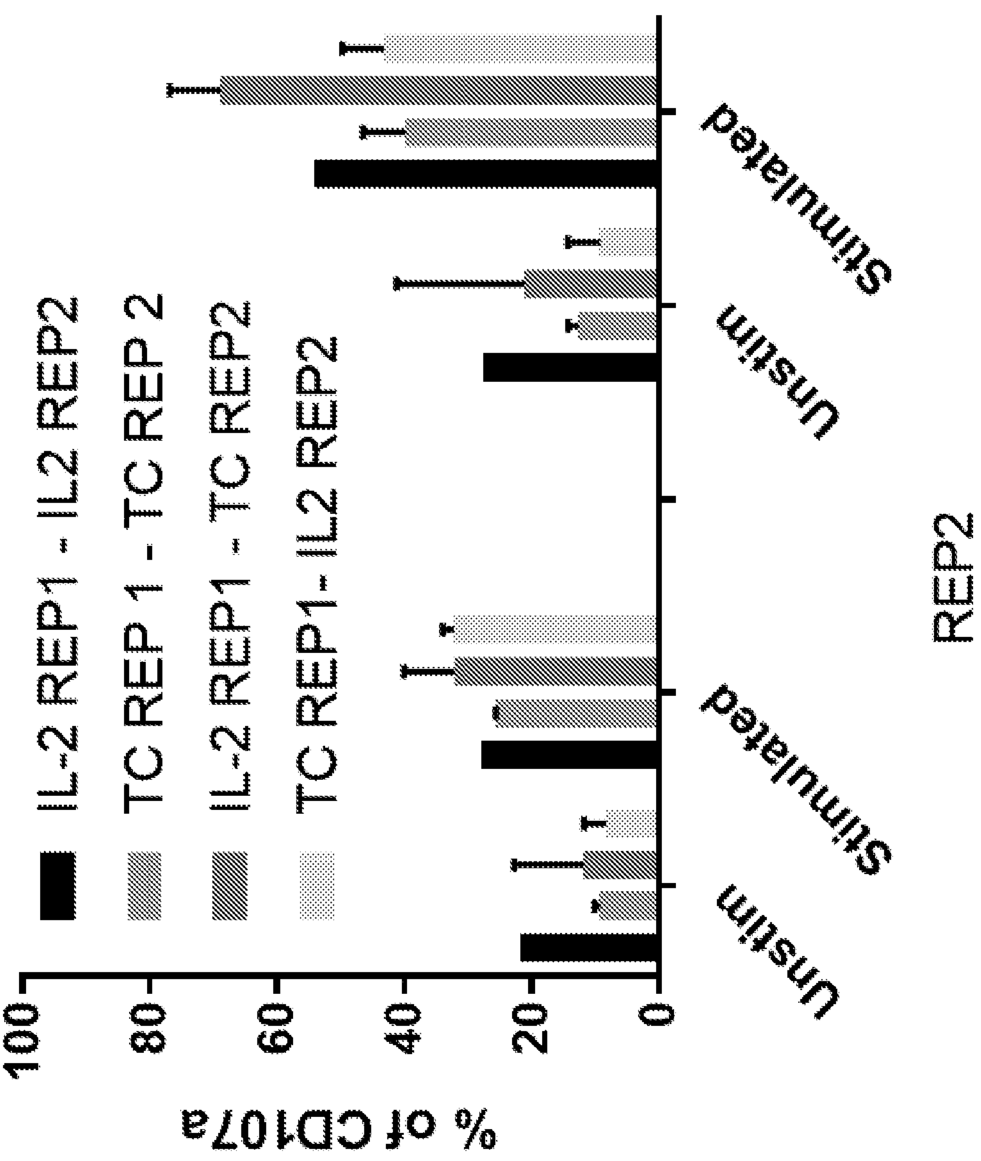
IL-2 REP1 - IL2 REP2
TC REP 1 - TC REP 2
IL-2 REP1 - TC REP2
TC REP1- IL2 REP2
% of CD107a
100
80
60
40
20
0
Unstim
Stimulated
Unstim
Stimulated
REP2

*Figure 55*

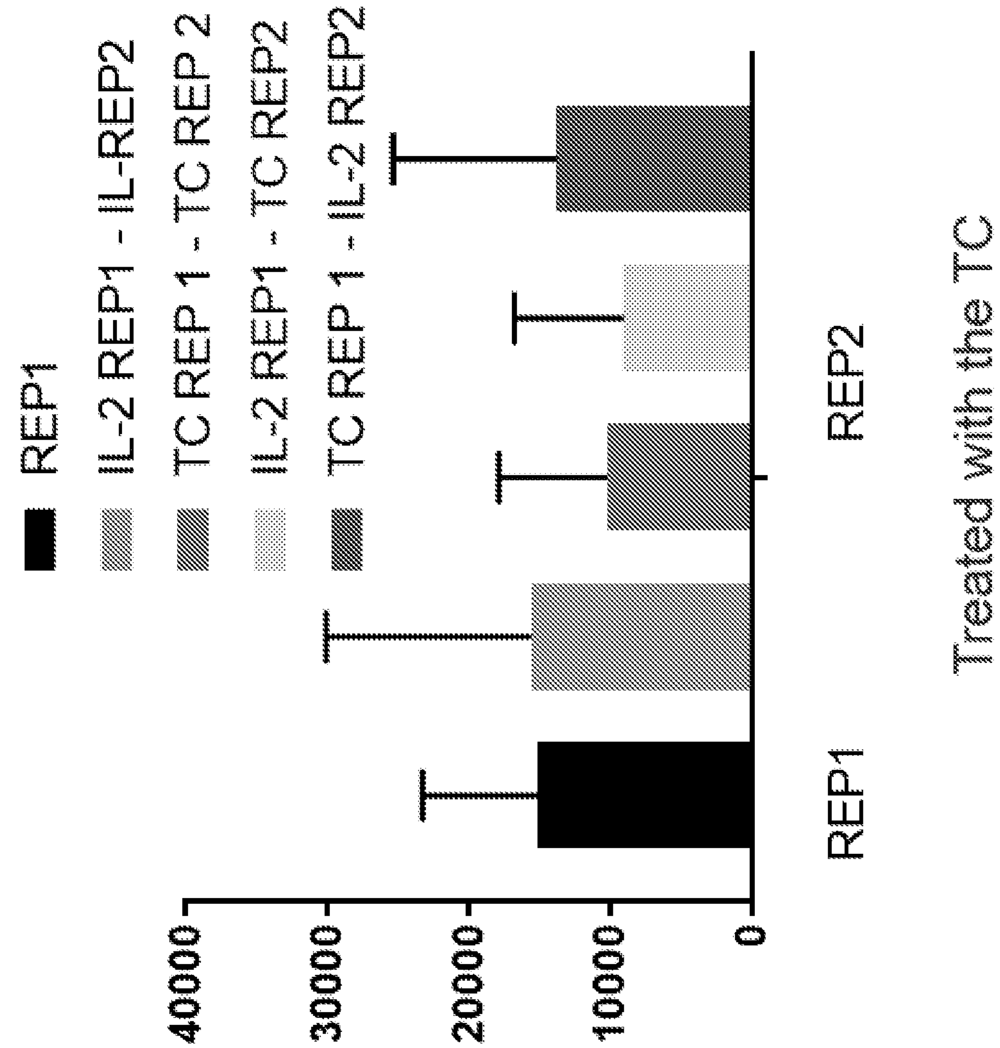
REP1
IL-2 REP1 - IL-REP2
TC REP 1 - TC REP 2
IL-2 REP1 - TC REP2
TC REP 1 - IL-2 REP2
IFNγ (pg/ml)
40000
30000
20000
10000
0
REP1
REP2
Treated with the TC

Figure 56

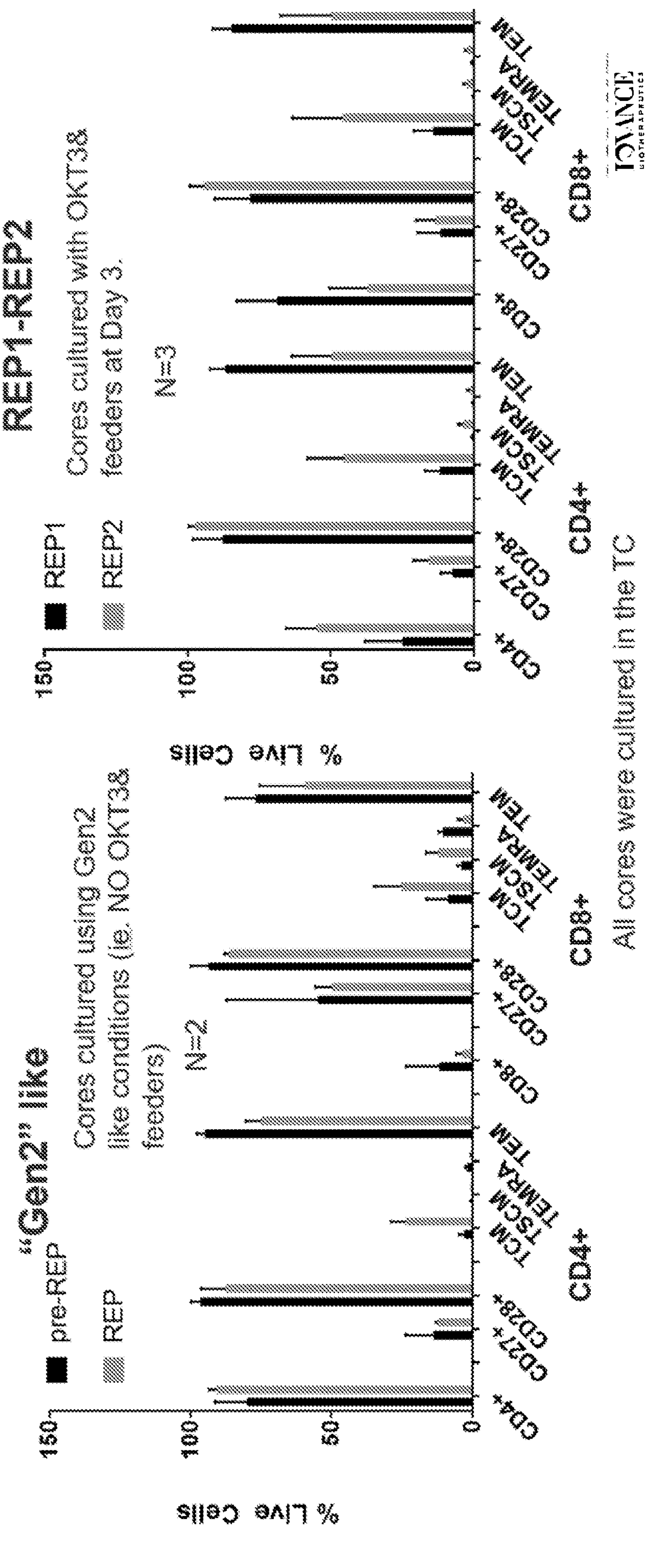
"Gen2" like
pre-REP
REP
Cores cultured using Gen2 like conditions (ie, NO OKT3& feeders)
N=2
REP1-REP2
REP1
REP2
Cores cultured with OKT3& feeders at Day 3.
N=3
% Live Cells
150
100
50
0
CD4+
CD27+
CD28+
TCM
TSCM
TEMRA
TEM
CD8+
CD4+
CD8+
All cores were cultured in the TC
IOVANCE BIOTHERAPEUTICS

*Figure 57*

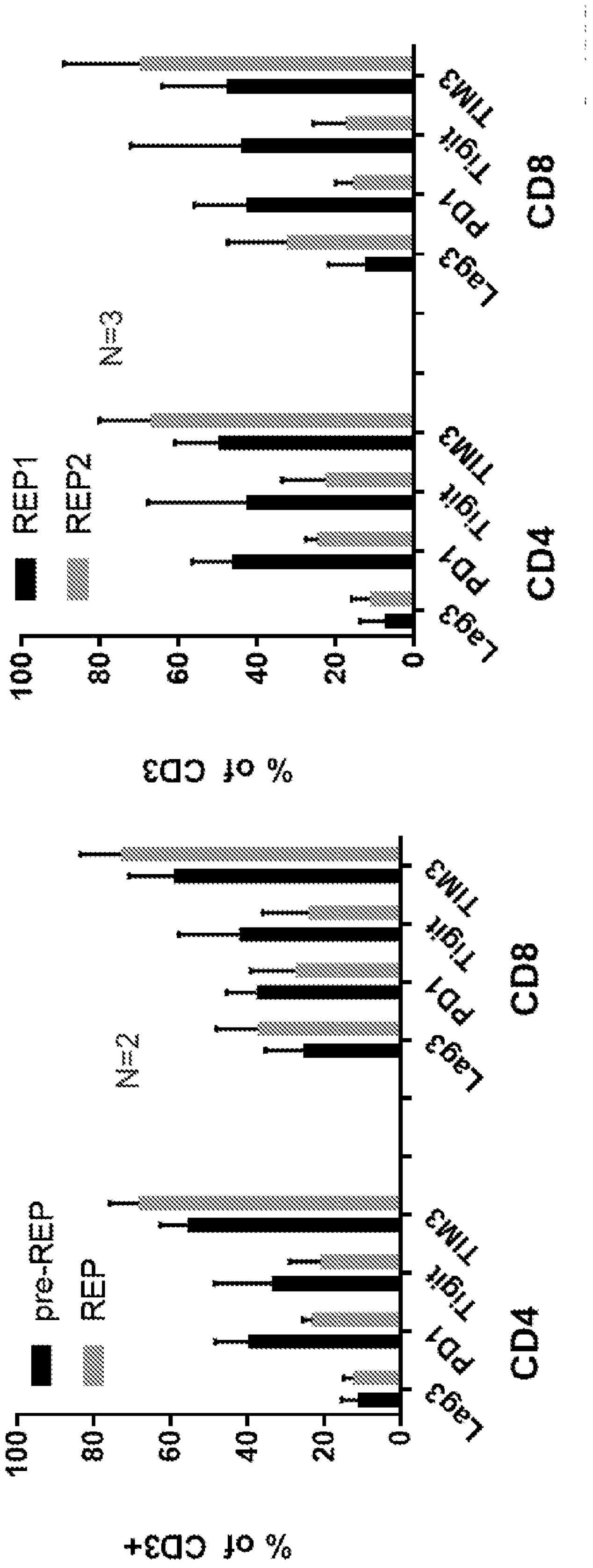
% of CD3+
pre-REP
REP
N=2
Lag3
PD1
Tigit
TIM3
CD4
CD8
100
80
60
40
20
0
% of CD3
REP1
REP2
N=3

*Figure 58*

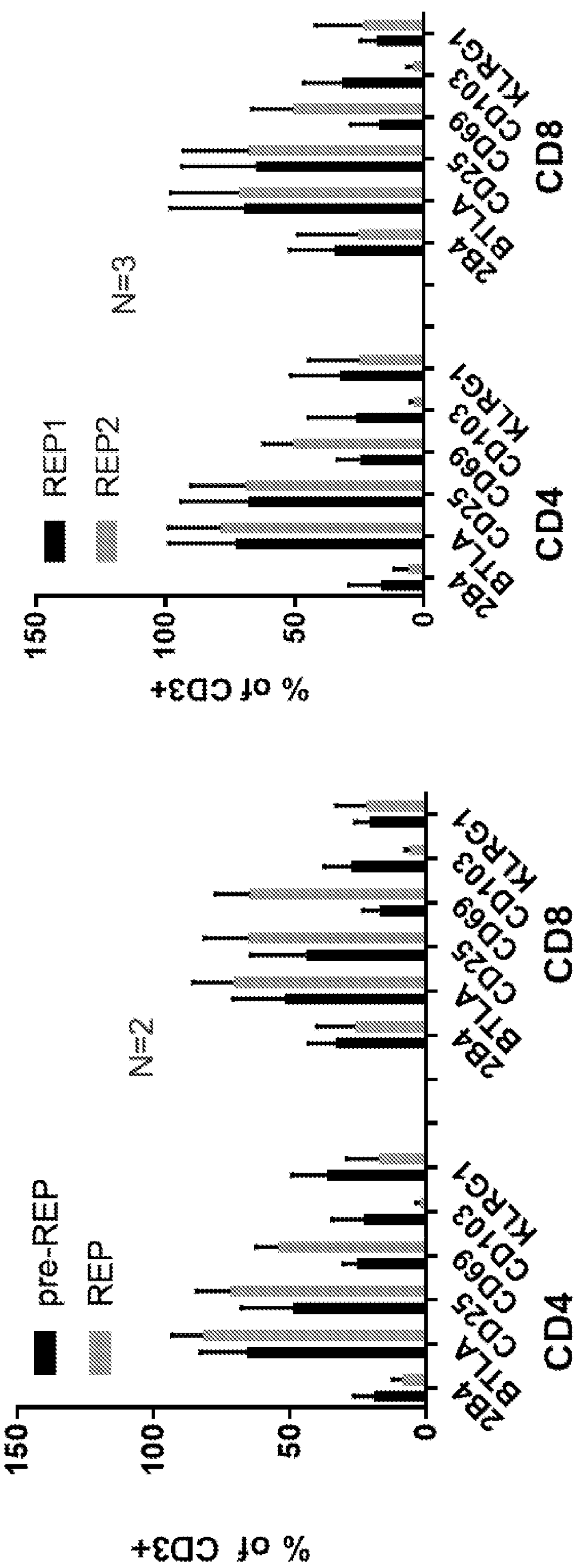

REP1
REP2
N=3
% of CD3+
150
100
50
0
2B4
BTLA
CD25
CD69
CD103
KLRG1
CD4
CD8
pre-REP
REP
N=2

*Figure 59*

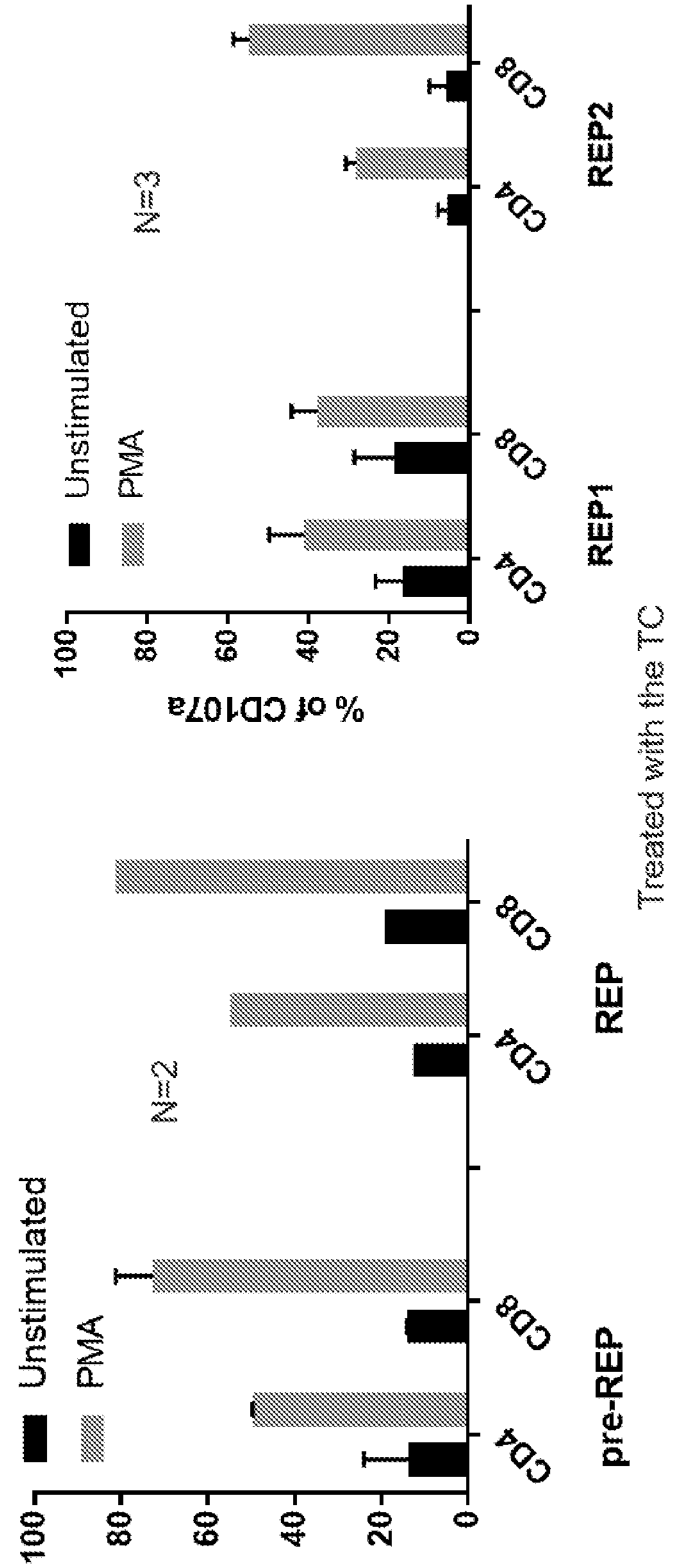
% of CD107a
100
80
60
40
20
0
Unstimulated
PMA
N=2
CD4
CD8
CD4
CD8
pre-REP
REP
% of CD107a
100
80
60
40
20
0
Unstimulated
PMA
N=3
CD4
CD8
CD4
CD8
REP1
REP2
Treated with the TC

*Figure 60*

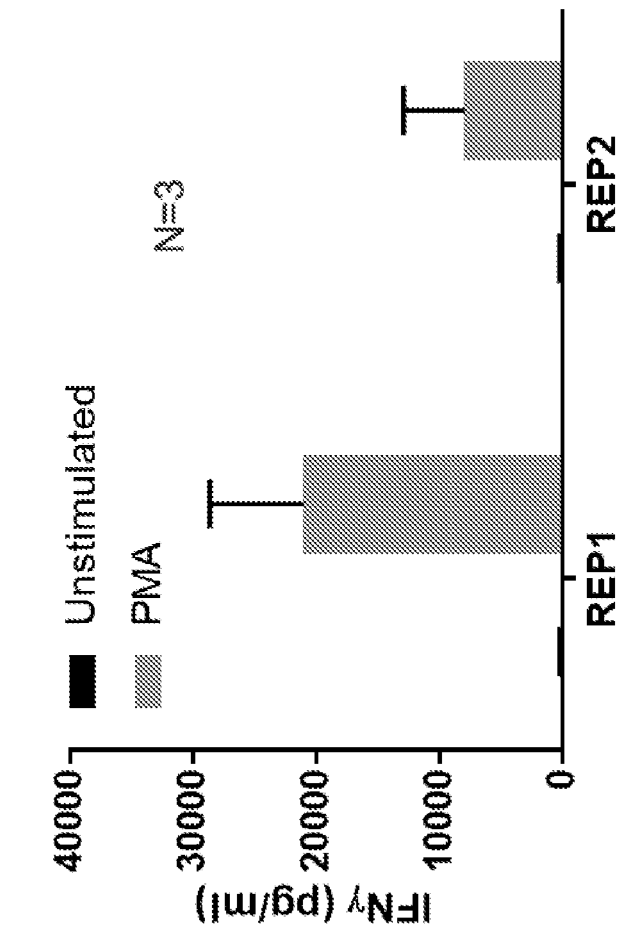
Unstimulated
PMA
N=3
IFNγ (pg/ml)
40000
30000
20000
10000
0
REP1
REP2

*Figure 61*

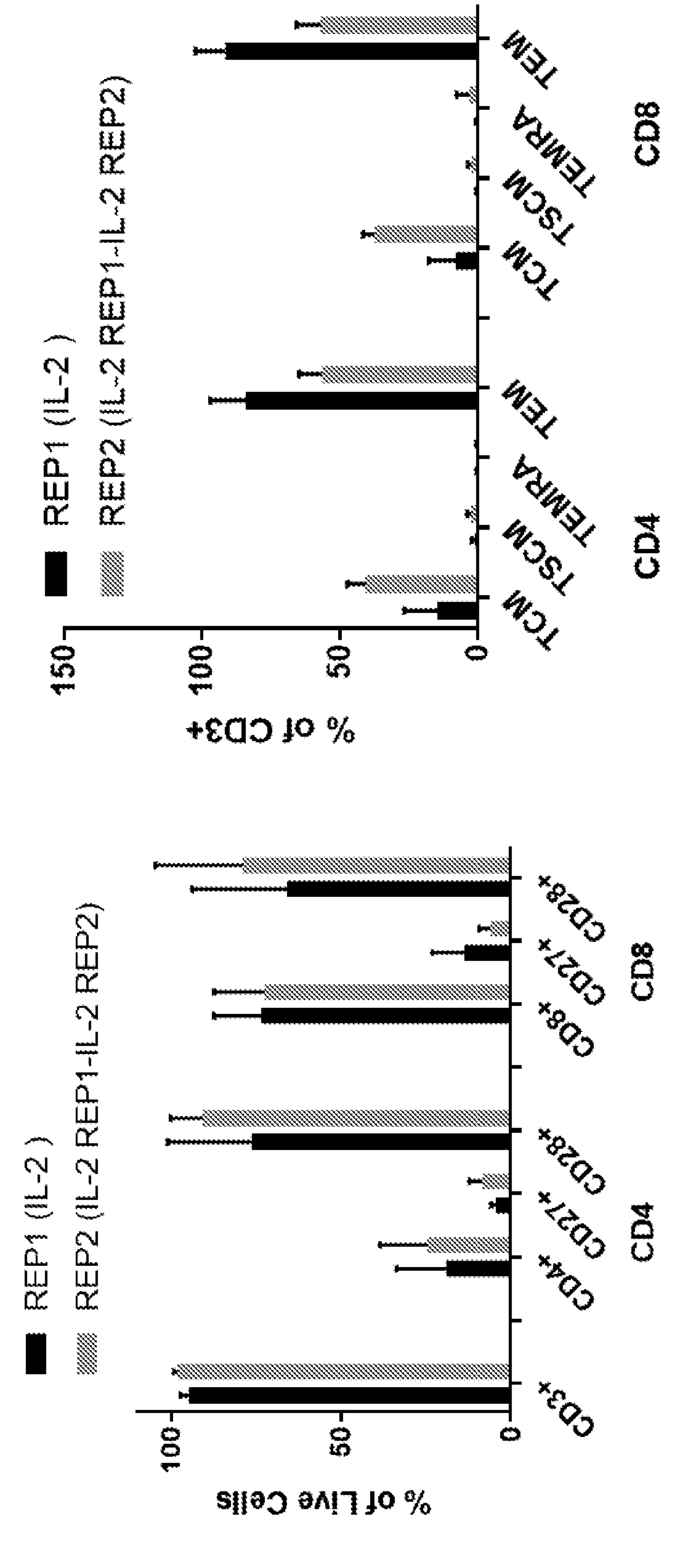

REP1 (IL-2 )
REP2 (IL-2 REP1-IL-2 REP2)
% of CD3+
150
100
50
0
TCM
TSCM
TEMRA
TEM
CD4
CD8
% of Live Cells
CD3+
CD4+
CD27+
CD28+
CD8+

*Figure 62*

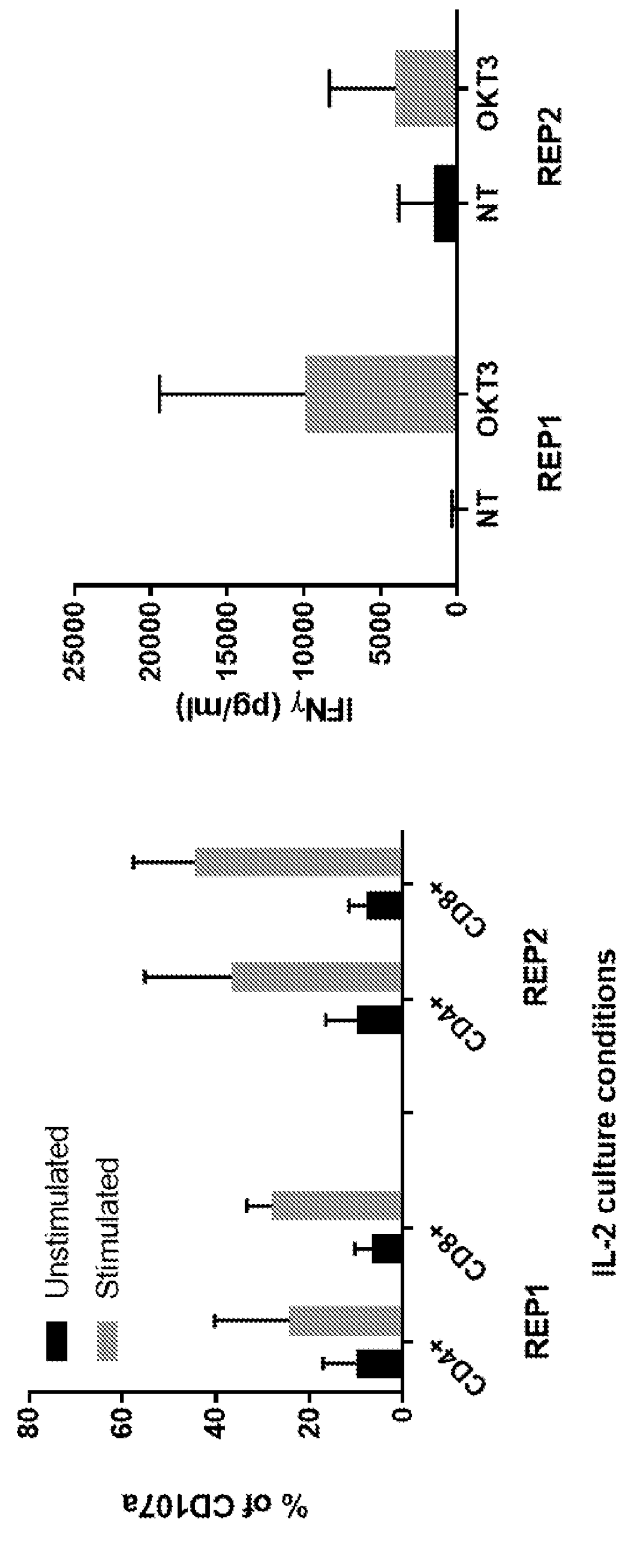
% of CD107a
80
60
40
20
0
Unstimulated
Stimulated
CD4+
CD8+
CD4+
CD8+
REP1
REP2
IL-2 culture conditions
IFNγ (pg/ml)
25000
20000
15000
10000
5000
0
NT
OKT3
NT
OKT3
REP1
REP2

*Figure 63*

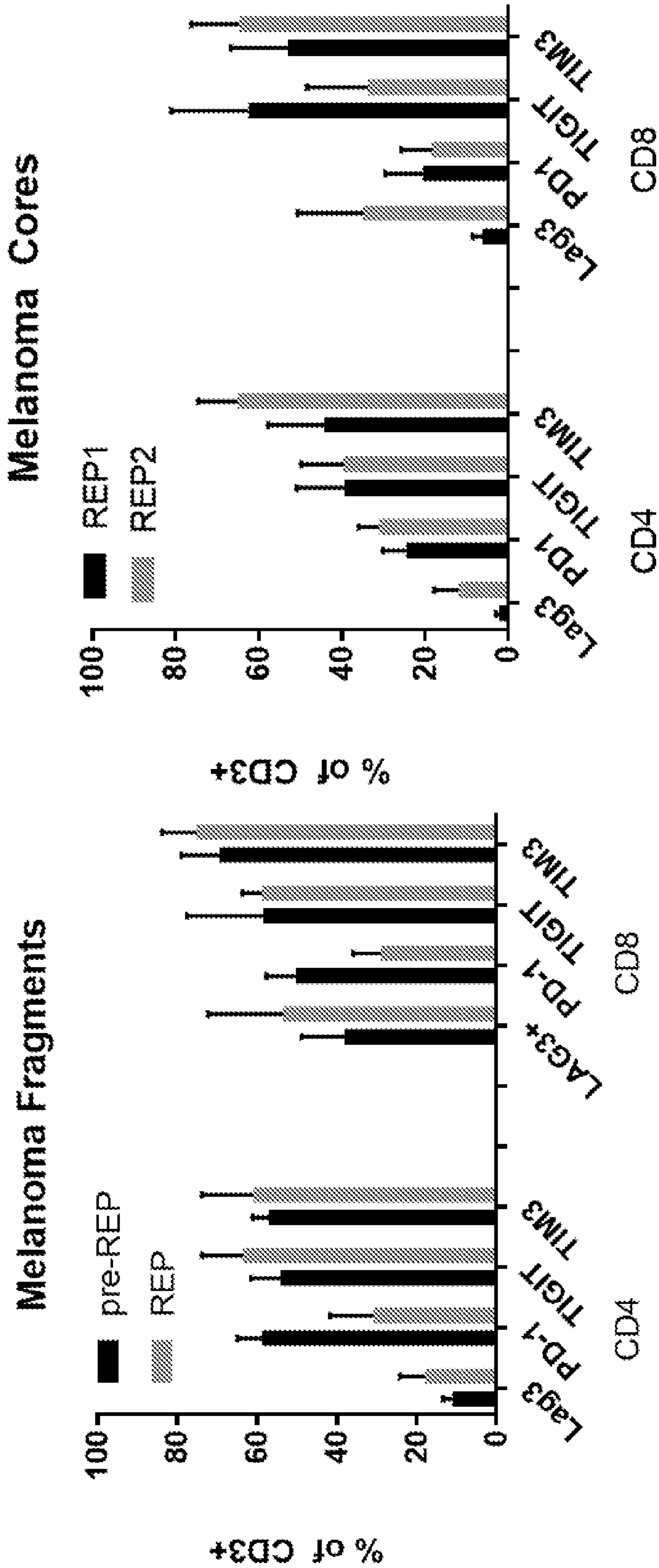
Melanoma Cores
REP1
REP2
% of CD3+
100
80
60
40
20
0
Lag3
PD1
TIGIT
TIM3
CD4
CD8
Melanoma Fragments
pre-REP
REP
% of CD3+
Lag3
PD-1
TIGIT
TIM3
LAG3+
CD4
CD8

Figure 64

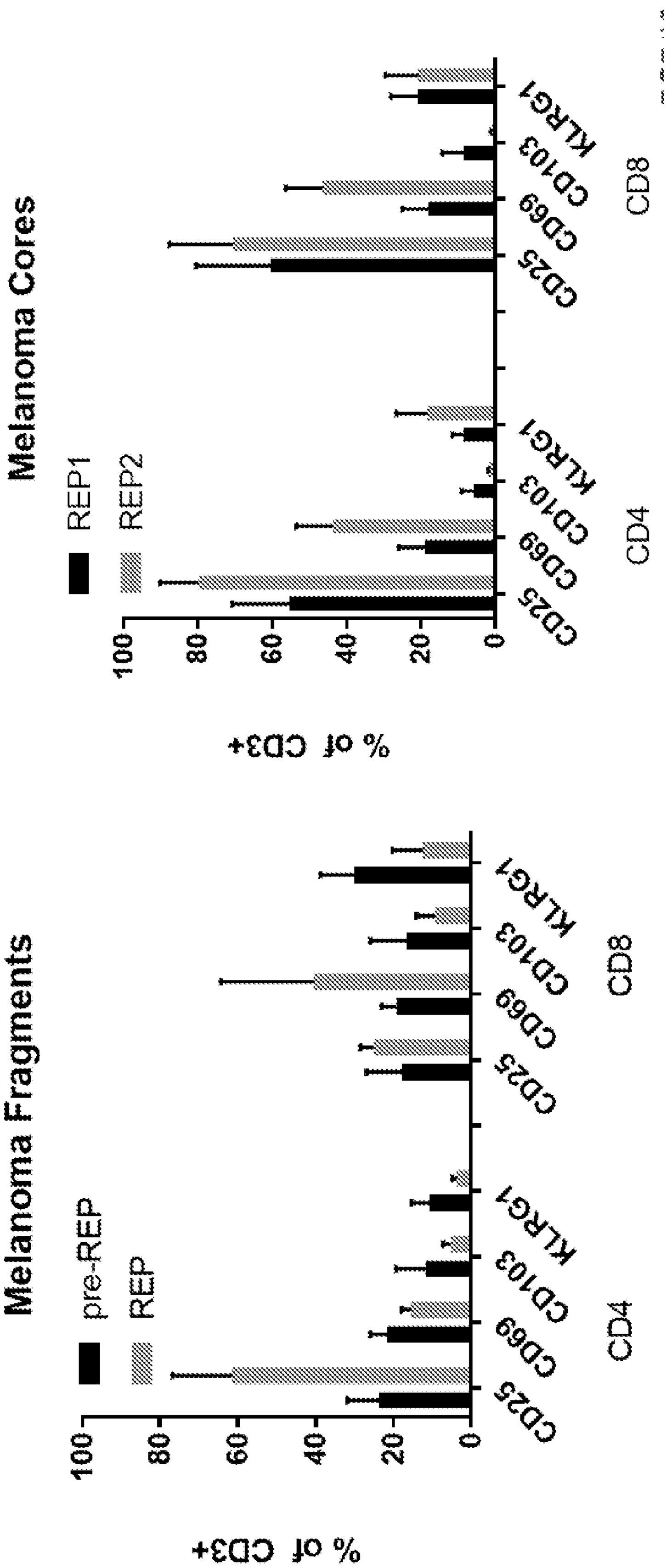
Melanoma Fragments
pre-REP
REP
% of CD3+
100
80
60
40
20
0
CD25
CD69
CD103
KLRG1
CD4
CD8
Melanoma Cores
REP1
REP2

*Figure 65*

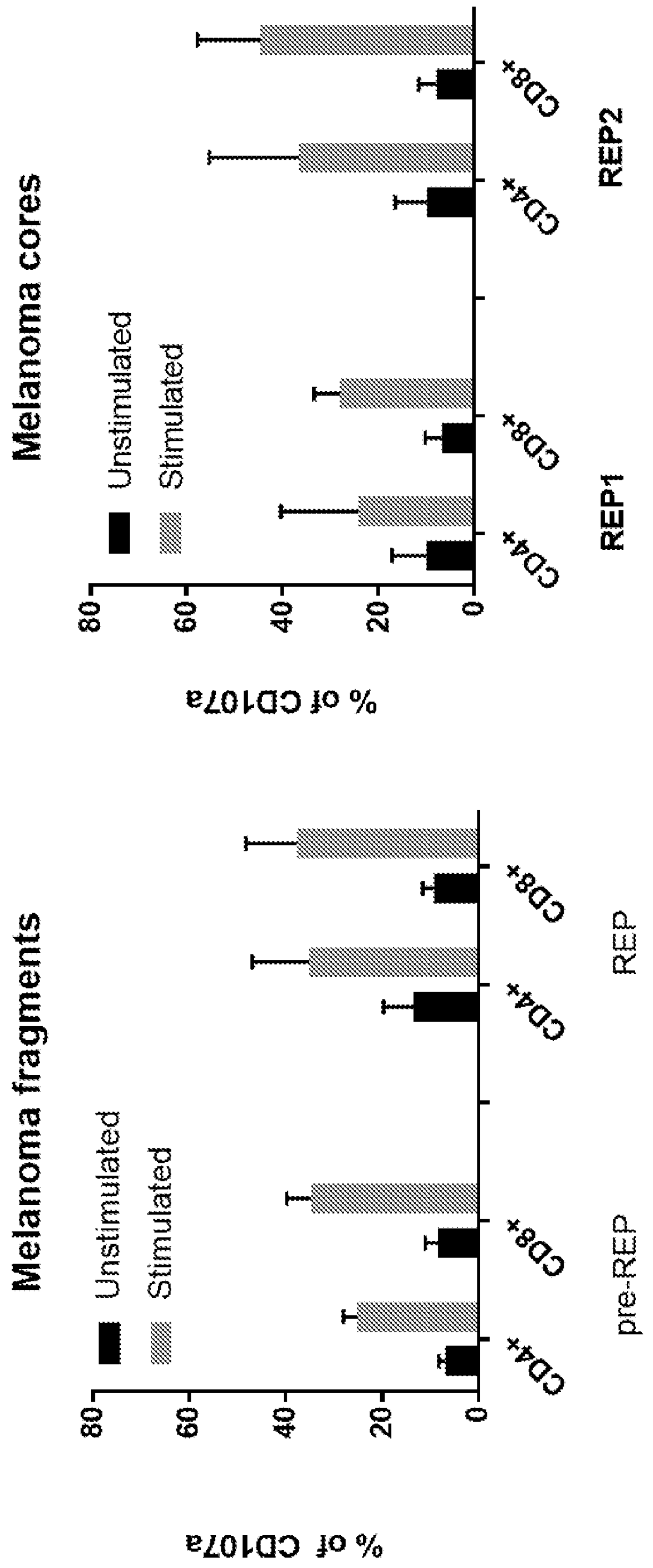
Melanoma fragments
Unstimulated
Stimulated
% of CD107a
80
60
40
20
0
CD4+
CD8+
CD4+
CD8+
pre-REP
REP
Melanoma cores
Unstimulated
Stimulated
% of CD107a
80
60
40
20
0
CD4+
CD8+
CD4+
CD8+
REP1
REP2

*Figure 66*

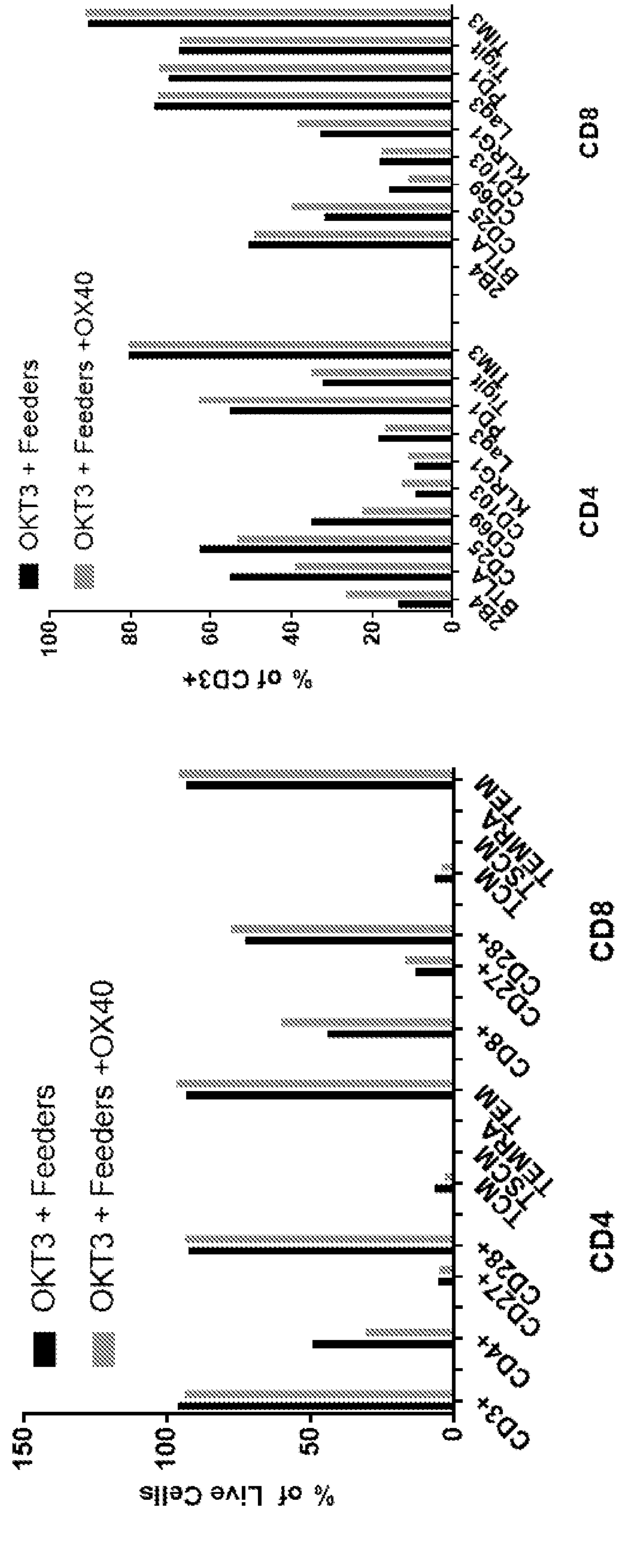
OKT3 + Feeders
OKT3 + Feeders +OX40
% of Live Cells
150
100
50
0
CD3+
CD4+
CD27+
CD28+
TCM
TSCM
TEMRA
TEM
CD8+
CD27+
CD28+
TCM
TSCM
TEMRA
TEM
CD4
CD8
OKT3 + Feeders
OKT3 + Feeders +OX40
% of CD3+
100
80
60
40
20
0
2B4
BTLA
CD25
CD69
CD103
KLRG1
Lag3
PD1
Tigit
TIM3
2B4
BTLA
CD25
CD69
CD103
KLRG1
Lag3
PD1
Tigit
TIM3
CD4
CD8

Figure 67

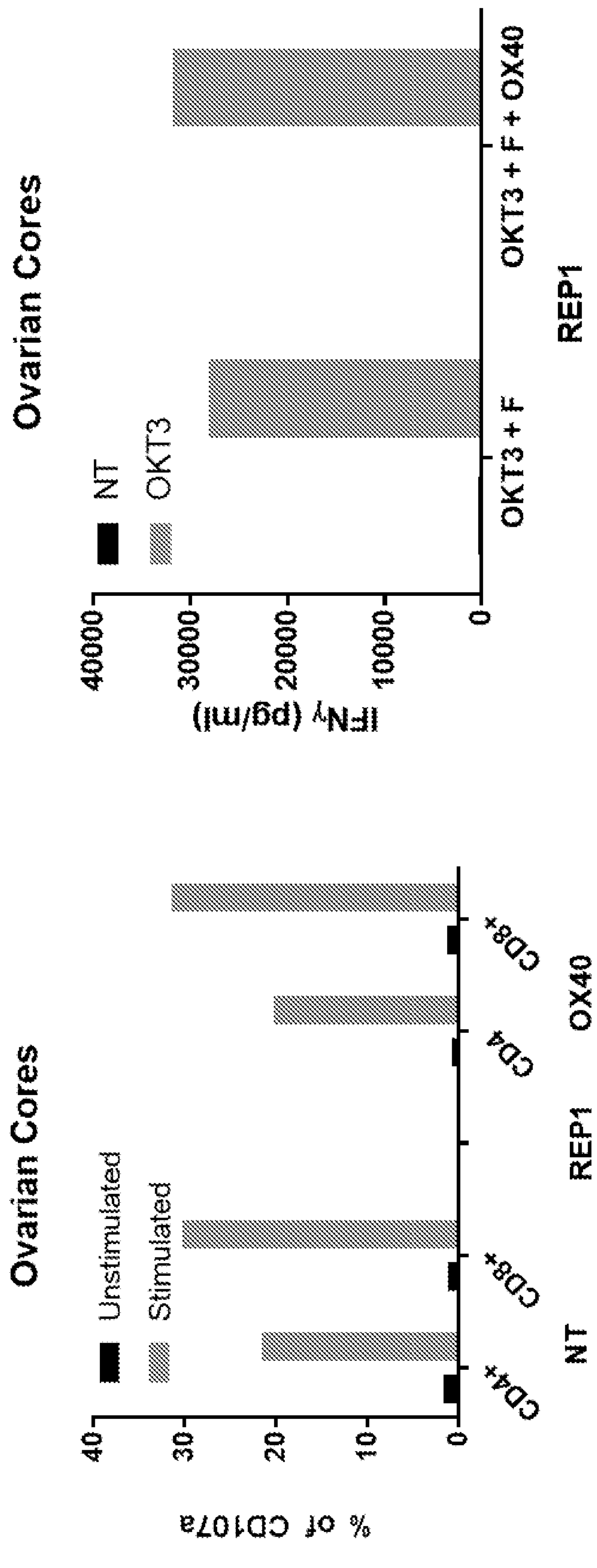
Ovarian Cores
Unstimulated
Stimulated
% of CD107a
40
30
20
10
0
CD4+
CD8+
CD4
CD8+
NT
REP1
OX40
Ovarian Cores
NT
OKT3
IFNγ (pg/ml)
40000
30000
20000
10000
0
OKT3 + F
OKT3 + F + OX40
REP1

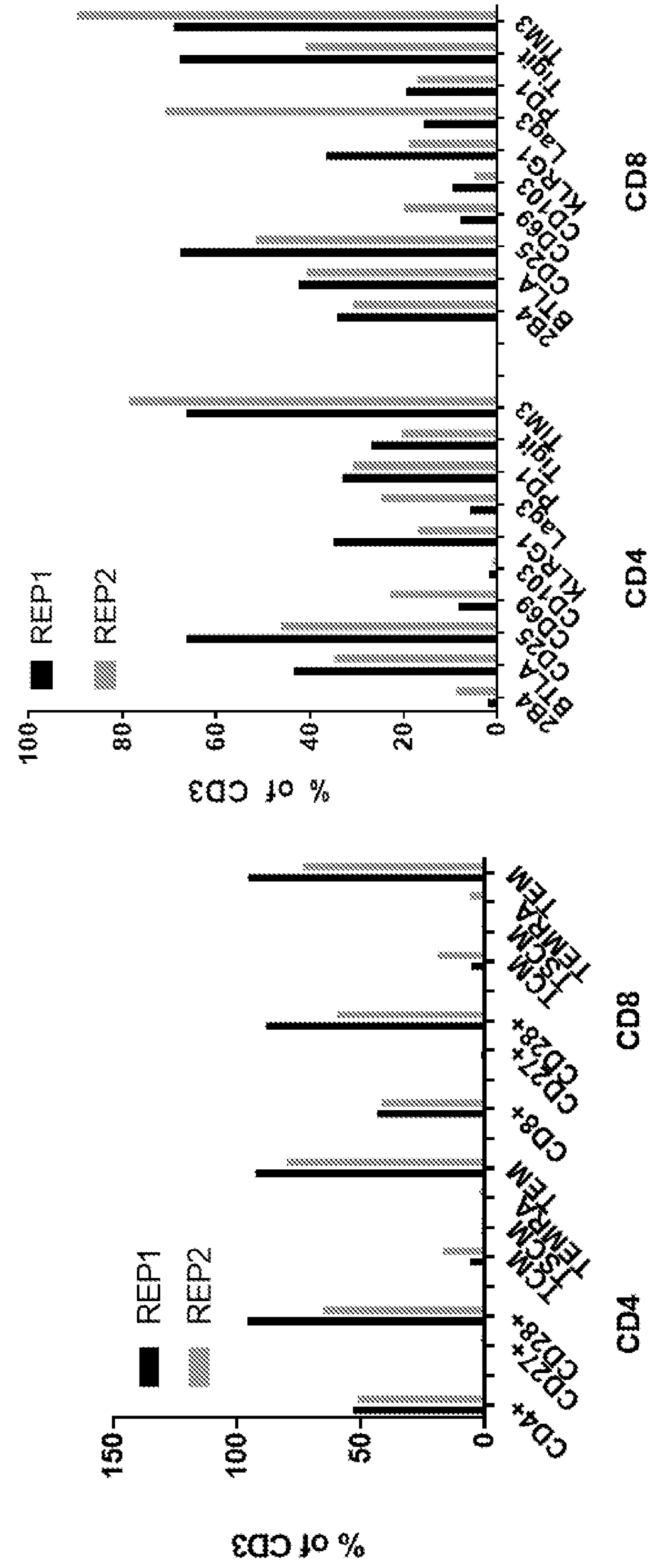
REP1
REP2
% of CD3
0
50
100
150
CD4+
CD27+
CD28+
TCM
TSCM
TEMRA
TEM
CD8+
CD4
CD8
0
20
40
60
80
100
2B4
BTLA
CD25
CD69
CD103
KLRG1
Lag3
PD1
Tigit
TIM3

*Figure 68*

*Figure 69 A & B*

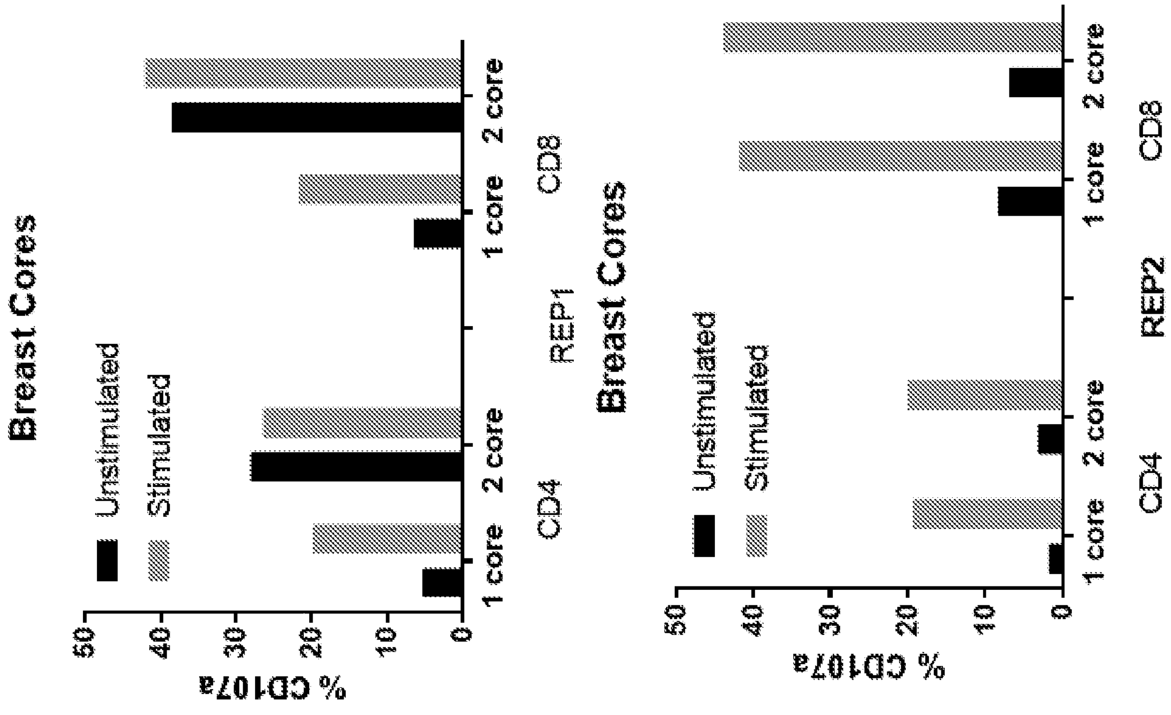
Breast Cores
Unstimulated
Stimulated
% CD107a
50
40
30
20
10
0
1 core
2 core
1 core
2 core
CD4
REP1
CD8
Breast Cores
Unstimulated
Stimulated
% CD107a
50
40
30
20
10
0
1 core
2 core
1 core
2 core
CD4
REP2
CD8

*Figure 69C*

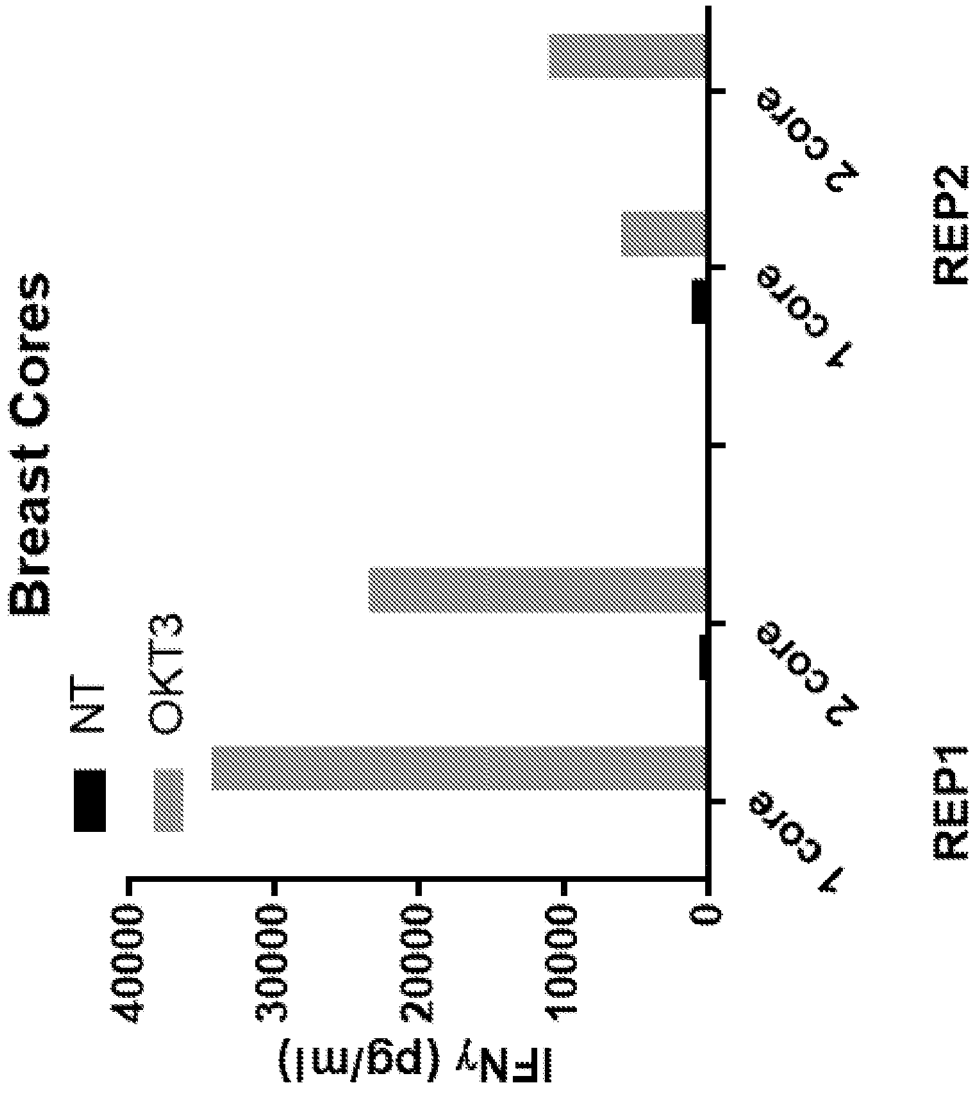
Breast Cores
NT
OKT3
IFNγ (pg/ml)
40000
30000
20000
10000
0
1 core
2 core
1 core
2 core
REP1
REP2

*Figure 70*

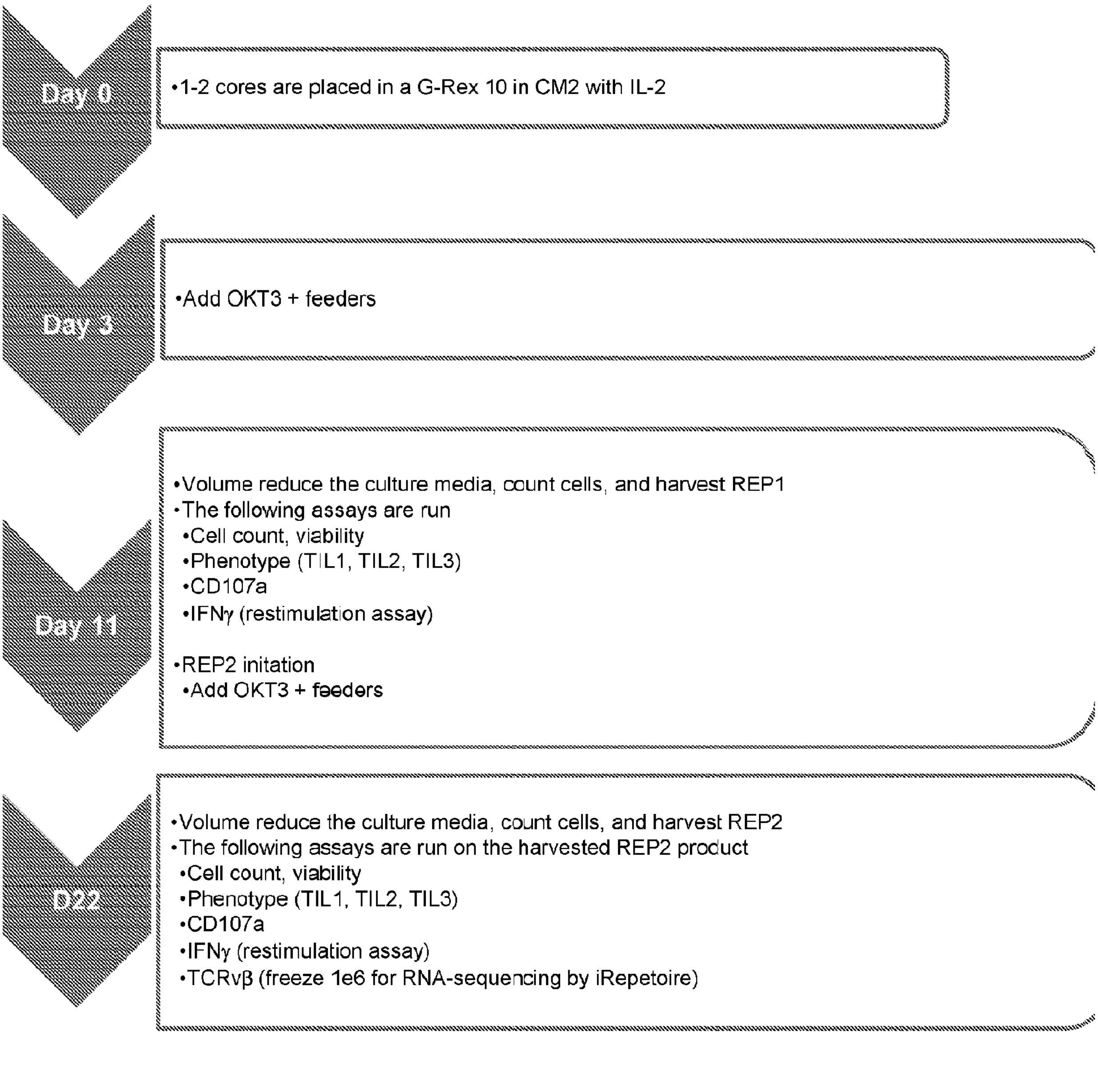
Day 0
•1-2 cores are placed in a G-Rex 10 in CM2 with IL-2
Day 3
•Add OKT3 + feeders
Day 11
•Volume reduce the culture media, count cells, and harvest REP1
•The following assays are run
•Cell count, viability
•Phenotype (TIL1, TIL2, TIL3)
•CD107a
•IFNγ (restimulation assay)
•REP2 initation
•Add OKT3 + feeders
D22
•Volume reduce the culture media, count cells, and harvest REP2
•The following assays are run on the harvested REP2 product
•Cell count, viability
•Phenotype (TIL1, TIL2, TIL3)
•CD107a
•IFNγ (restimulation assay)
•TCRvβ (freeze 1e6 for RNA-sequencing by iRepetoire)

*Figure 71*

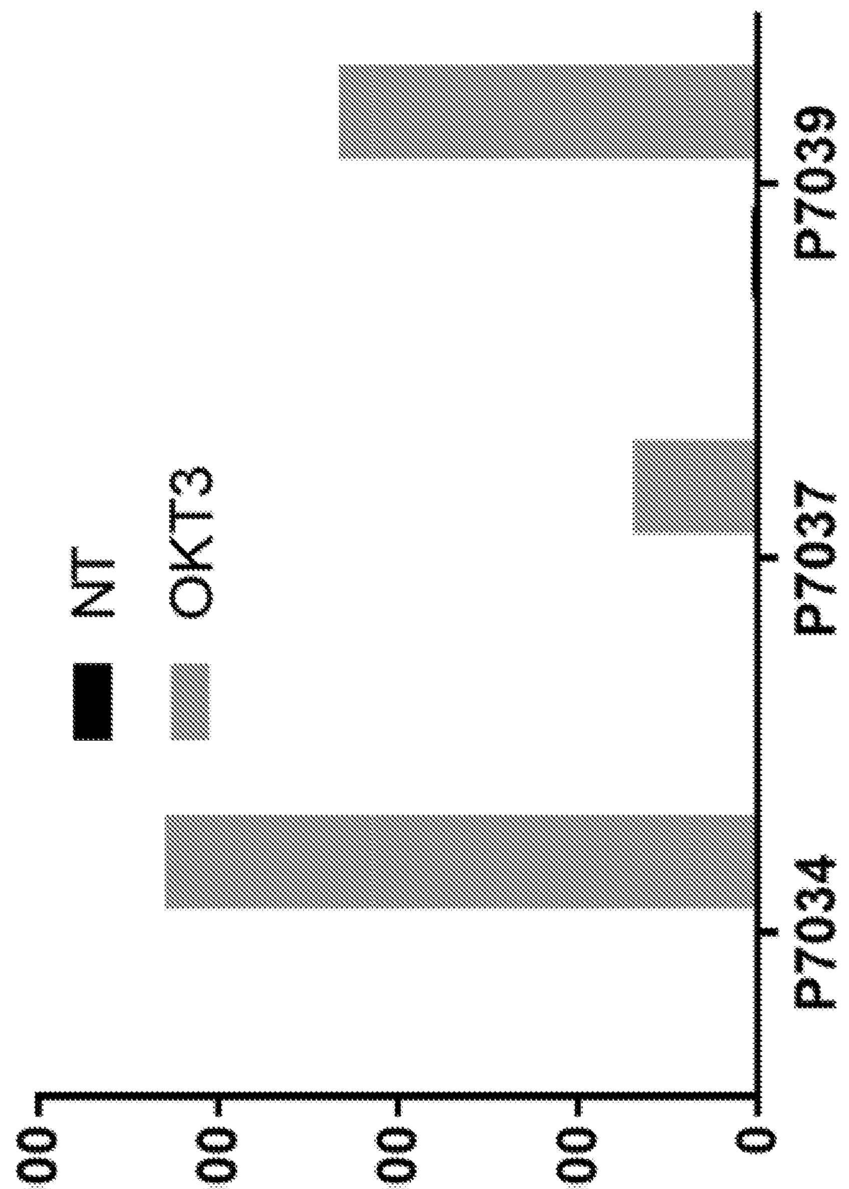
NT
OKT3
IFNγ (pg/ml)
40000
30000
20000
10000
0
P7034
P7037
P7039

Figure 72

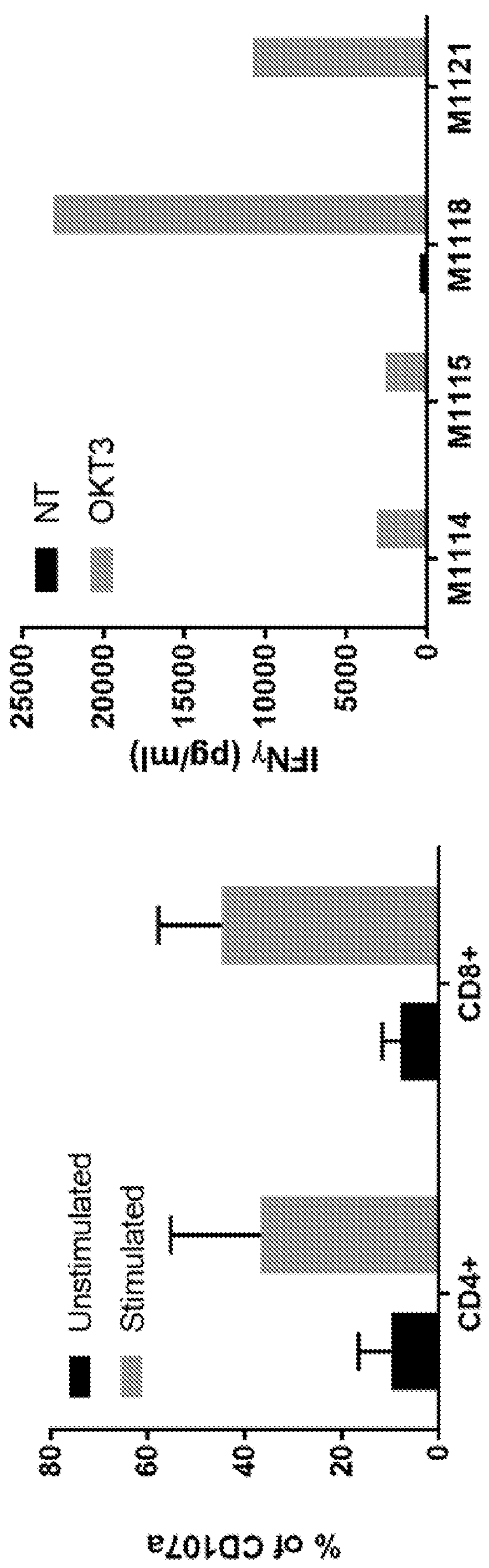
% of CD107a
80
60
40
20
0
Unstimulated
Stimulated
CD4+
CD8+
IFNγ (pg/ml)
25000
20000
15000
10000
5000
0
NT
OKT3
M1114
M1115
M1118
M1121

Figure 73A

| | Culture conditions | Cores / cond. (#) | REP1 | REP2 |
|---|---|---|---|---|
| Breast (EP11028) | REP1: IL2<br>REP2: IL-2 | 1 vs 2 | **>50E6 cells** at Day 11 (2 cores) | **173 and 158-fold (1/2 cores) expansion**<br>**~ 34,0000 and 23,000 pg/ml IFNγ** |
| Breast (EP11045) | REP1; IL-2 (+/- OKT3+feeders)<br>REP2: IL-2 (OKT3+feeders) | 2 | **>50e6 cells** at Day 11 (OKT3+ feeders) | **1178 and 1920-fold (+/- OKT3+feeders REP1)**<br>**~ 99,000 and 45,000 pg/ml IFNγ** |
| Ovarian (OV8016) | REP1: IL2 +/- OX40<br>REP2: IL-2 +/- OX40 | 2 | **>50E6 cells** at Day 11 (OX40 condition) | **1860 and 1540-fold (NO OX40/OX40) expansion**<br>**~ 10,000 and 20,000 pg/ml IFNγ** |
| Ovarian (OV8031) | REP1: IL2<br>REP2: IL-2 | 1(small core) | 1.3e6 cells at Day 11 | **400-fold expansion**<br>**~ ELISA is in progress** |

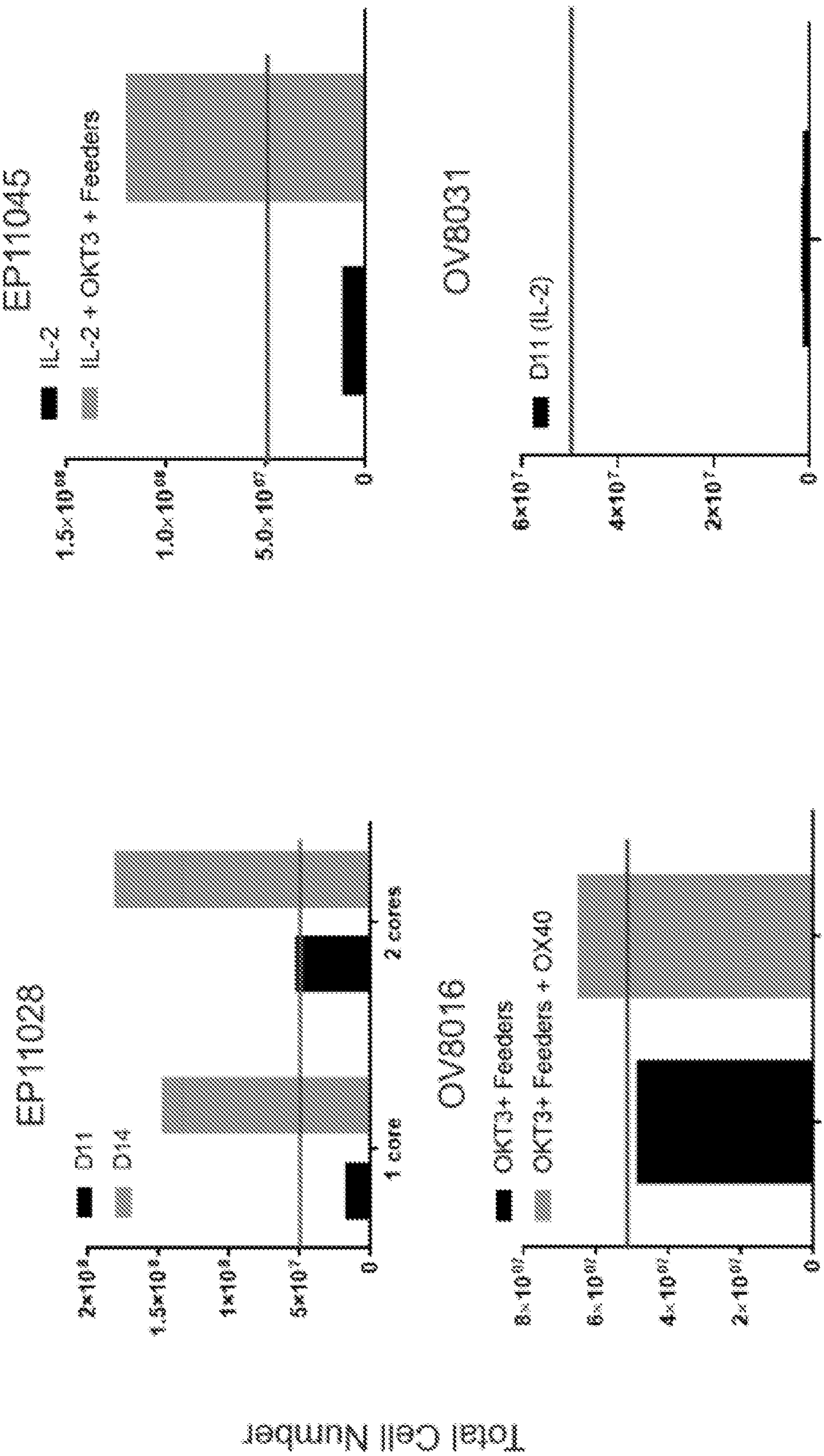
EP11028
D11
D14
1 core
2 cores
OV8016
OKT3+ Feeders
OKT3+ Feeders + OX40
EP11045
IL-2
IL-2 + OKT3 + Feeders
OV8031
D11 (IL-2)
Total Cell Number

*Figure 73B*

Figure 74

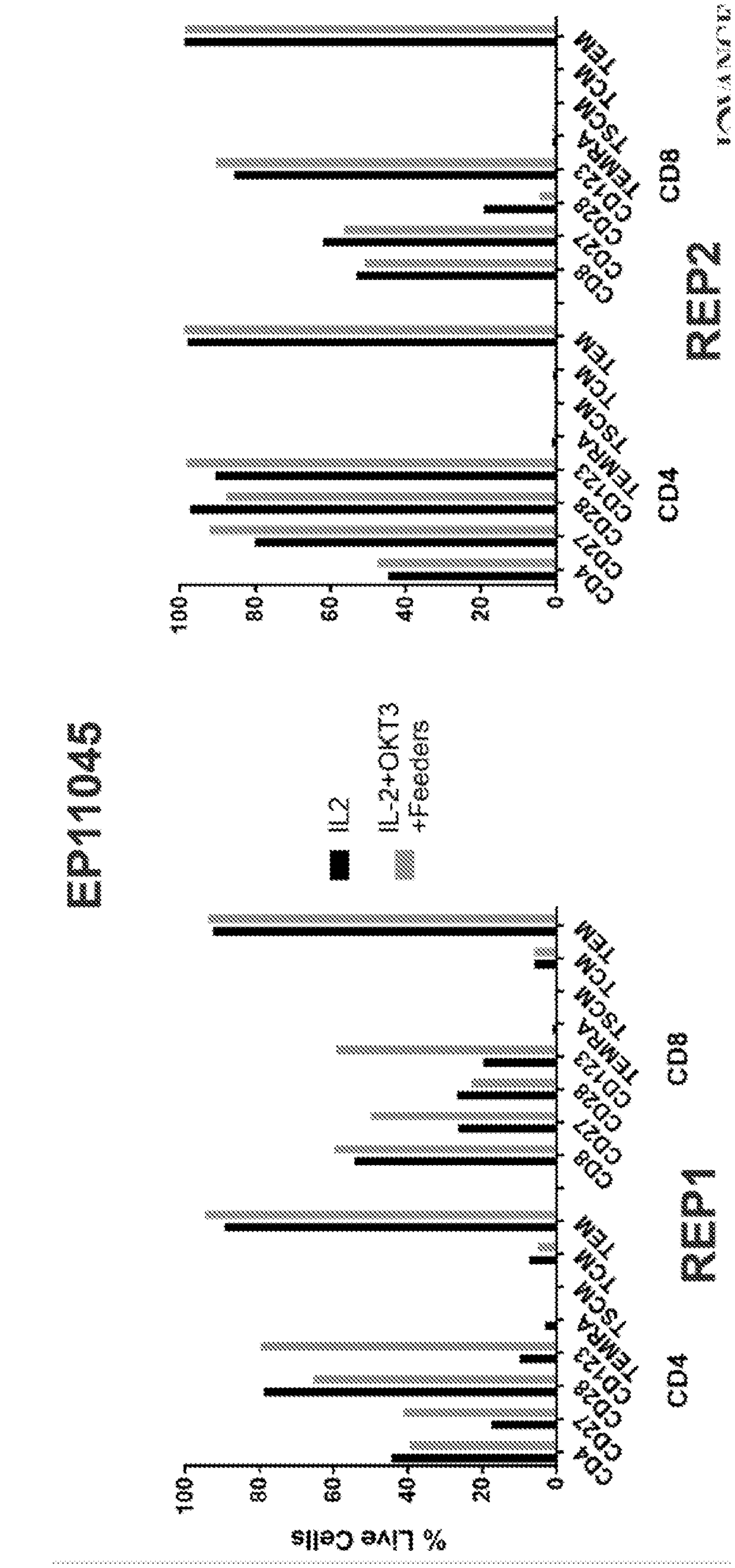
EP11045
IL2
IL-2+OKT3 +Feeders
% Live Cells
0
20
40
60
80
100
CD4
CD27
CD28
CD123
TEMRA
TSCM
TCM
TEM
CD8
CD27
CD28
CD123
TEMRA
TSCM
TCM
TEM
CD4
CD8
REP1
REP2

*Figure 75*

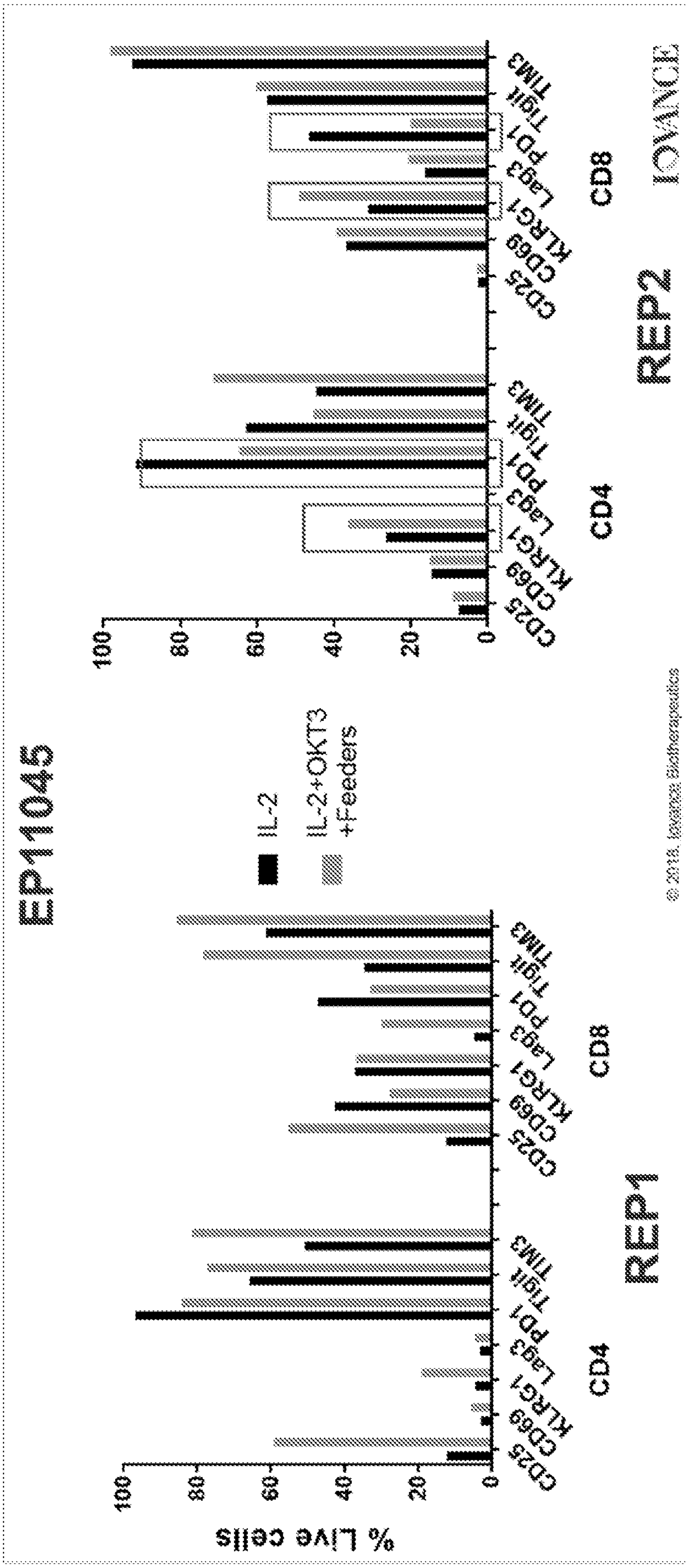
EP11045
% Live cells
0
20
40
60
80
100
CD25
CD69
KLRG1
Lag3
PD1
Tigit
TIM3
CD4
CD8
REP1
REP2
IL-2
IL-2+OKT3
+Feeders
© 2018. Iovance Biotherapeutics
IOVANCE

*Figure 76*

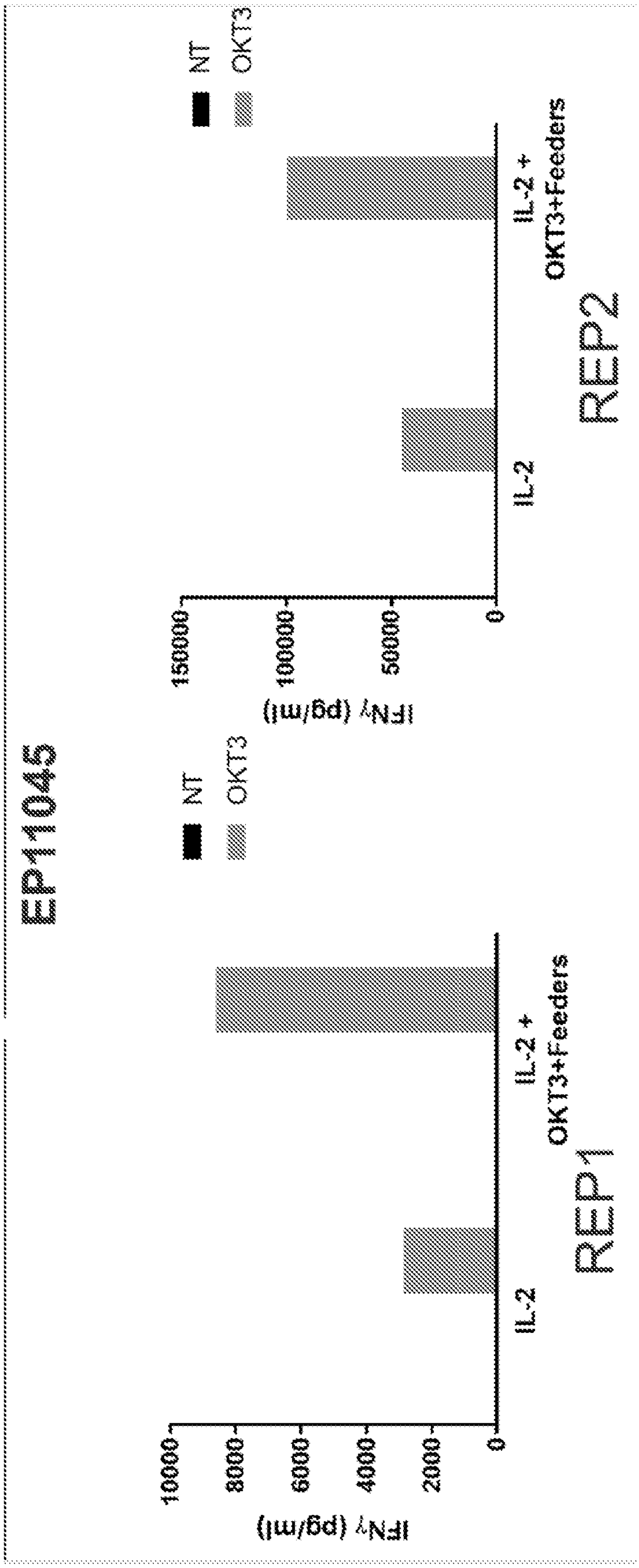
EP11045
REP1
REP2
IFNγ (pg/ml)
10000
8000
6000
4000
2000
0
150000
100000
50000
IL-2
IL-2 +
OKT3+Feeders
NT
OKT3

*Figure 77*

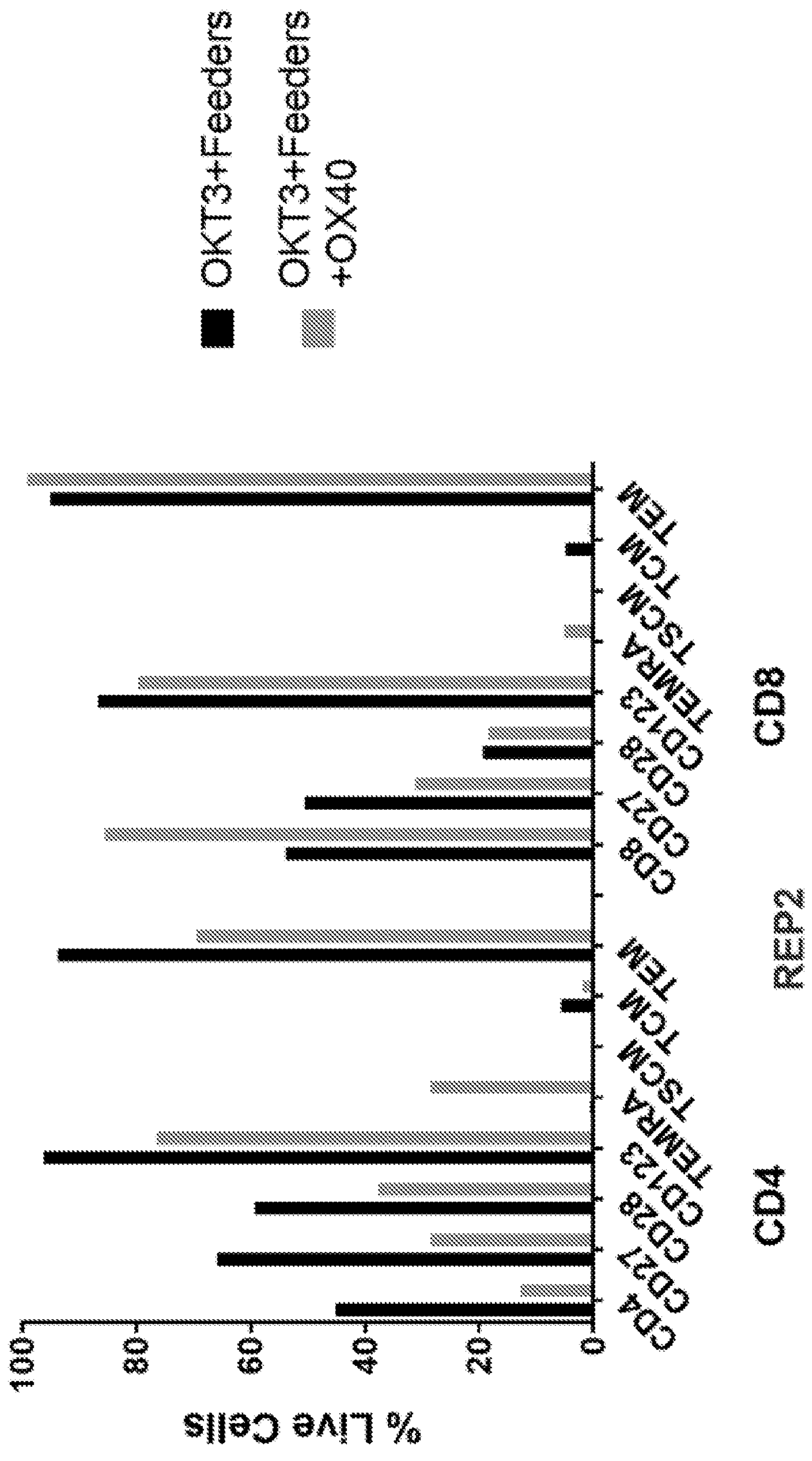

OKT3+Feeders
OKT3+Feeders +OX40
% Live Cells
100
80
60
40
20
0
CD4
CD27
CD28
CD123
TEMRA
TSCM
TCM
TEM
CD8
CD27
CD28
CD123
TEMRA
TSCM
TCM
TEM
CD4
REP2
CD8

*Figure 78*

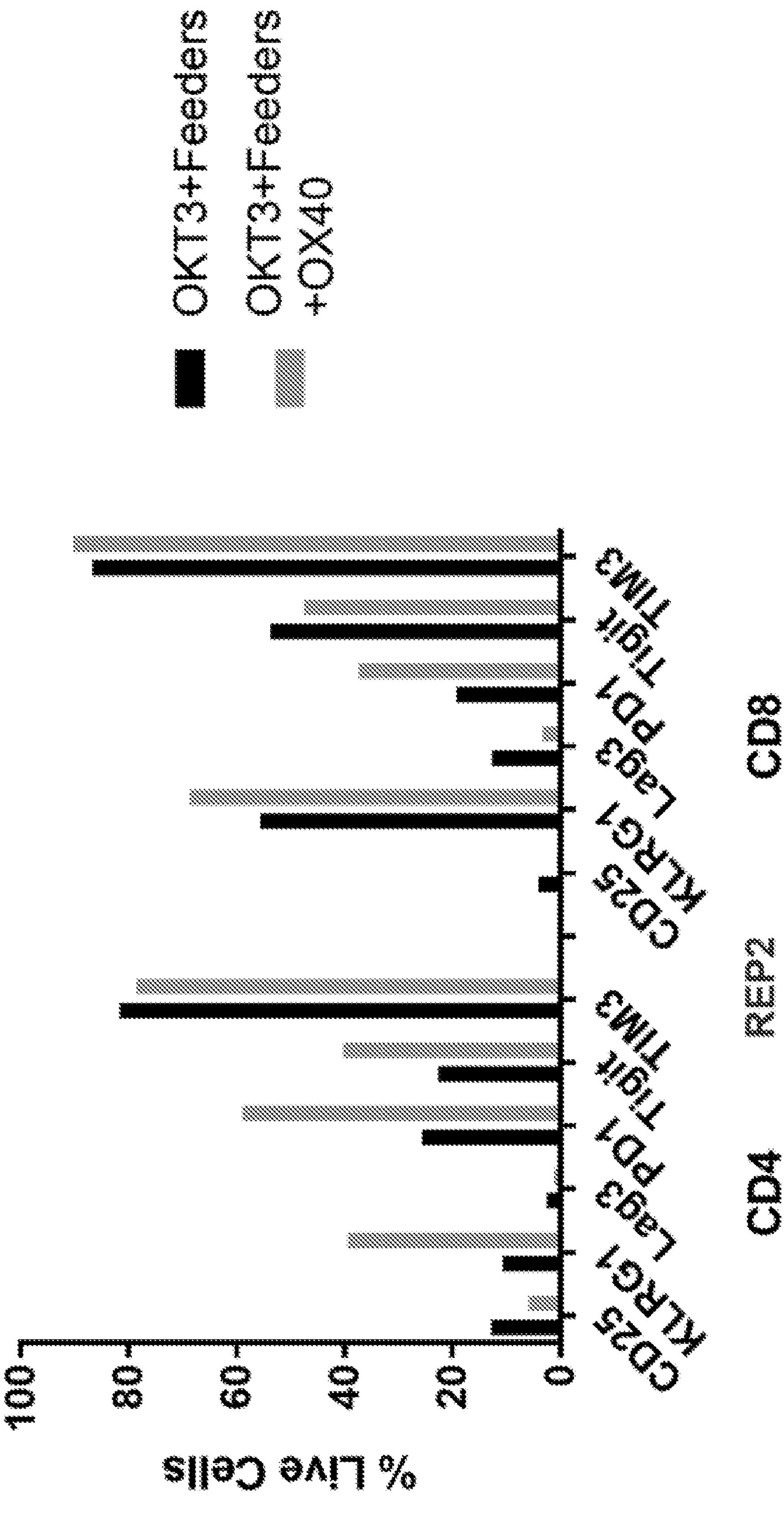
OKT3+Feeders
OKT3+Feeders +OX40
% Live Cells
100
80
60
40
20
0
CD25
KLRG1
Lag3
PD1
Tigit
TIM3
CD4
REP2
CD8

*Figure 79*

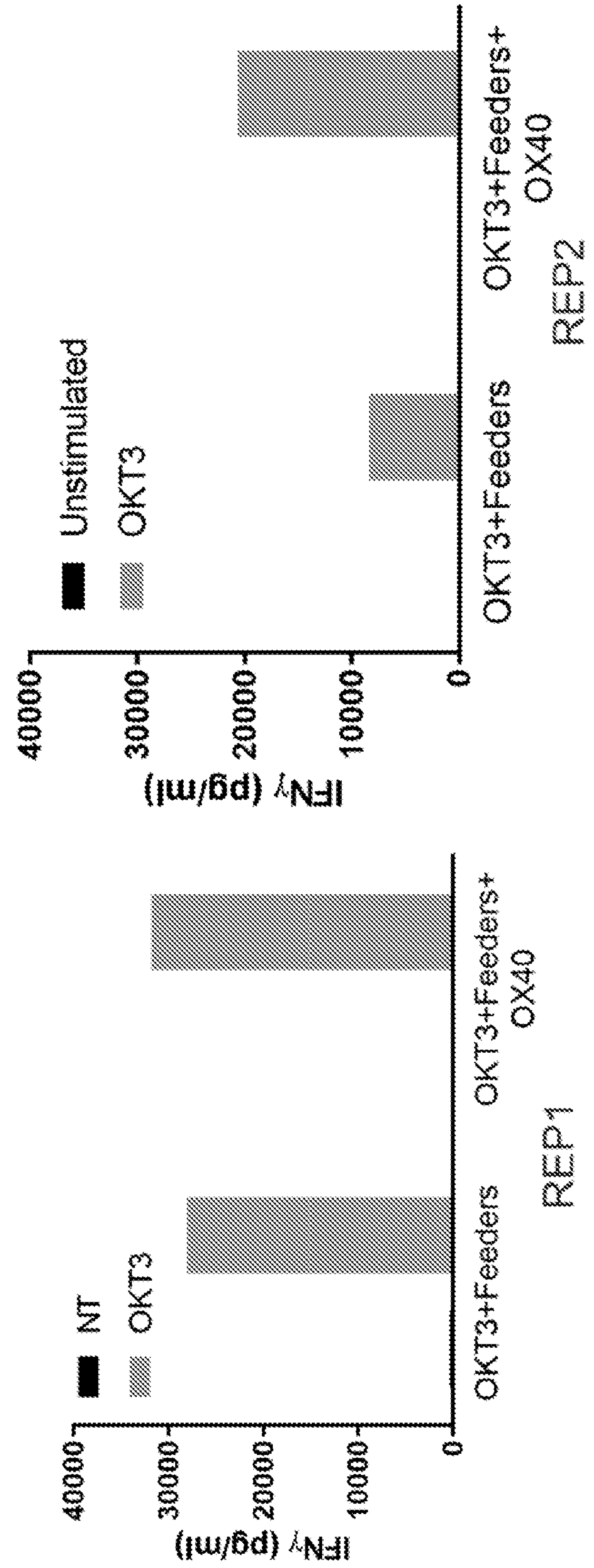
IFNγ (pg/ml)
40000
30000
20000
10000
0
NT
OKT3
OKT3+Feeders
OKT3+Feeders+ OX40
REP1
Unstimulated
OKT3
OKT3+Feeders
OKT3+Feeders+ OX40
REP2

*Figure 80*

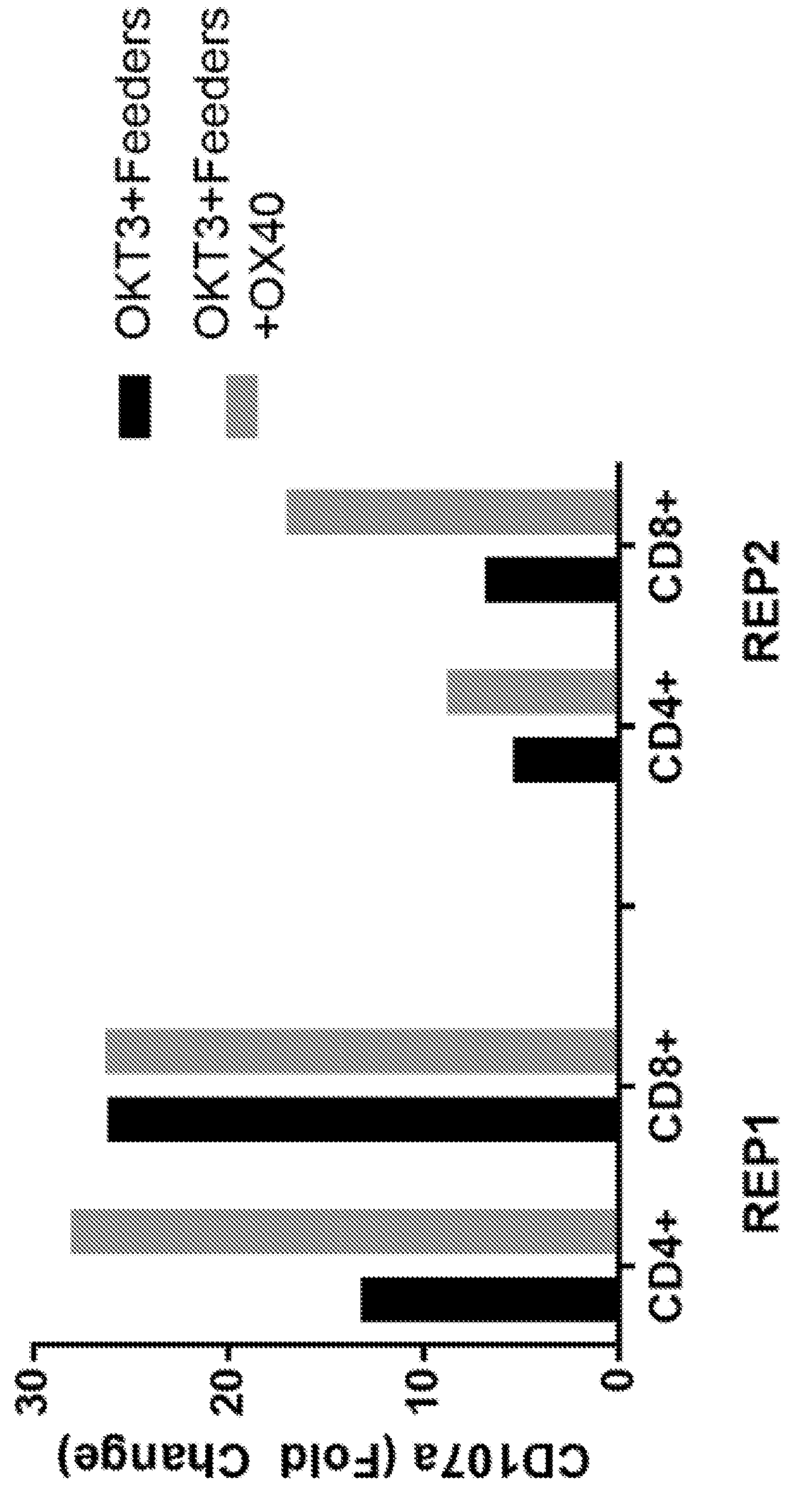
OKT3+Feeders
OKT3+Feeders +OX40
CD107a (Fold Change)
30
20
10
0
CD4+
CD8+
CD4+
CD8+
REP1
REP2

*Figure 81*

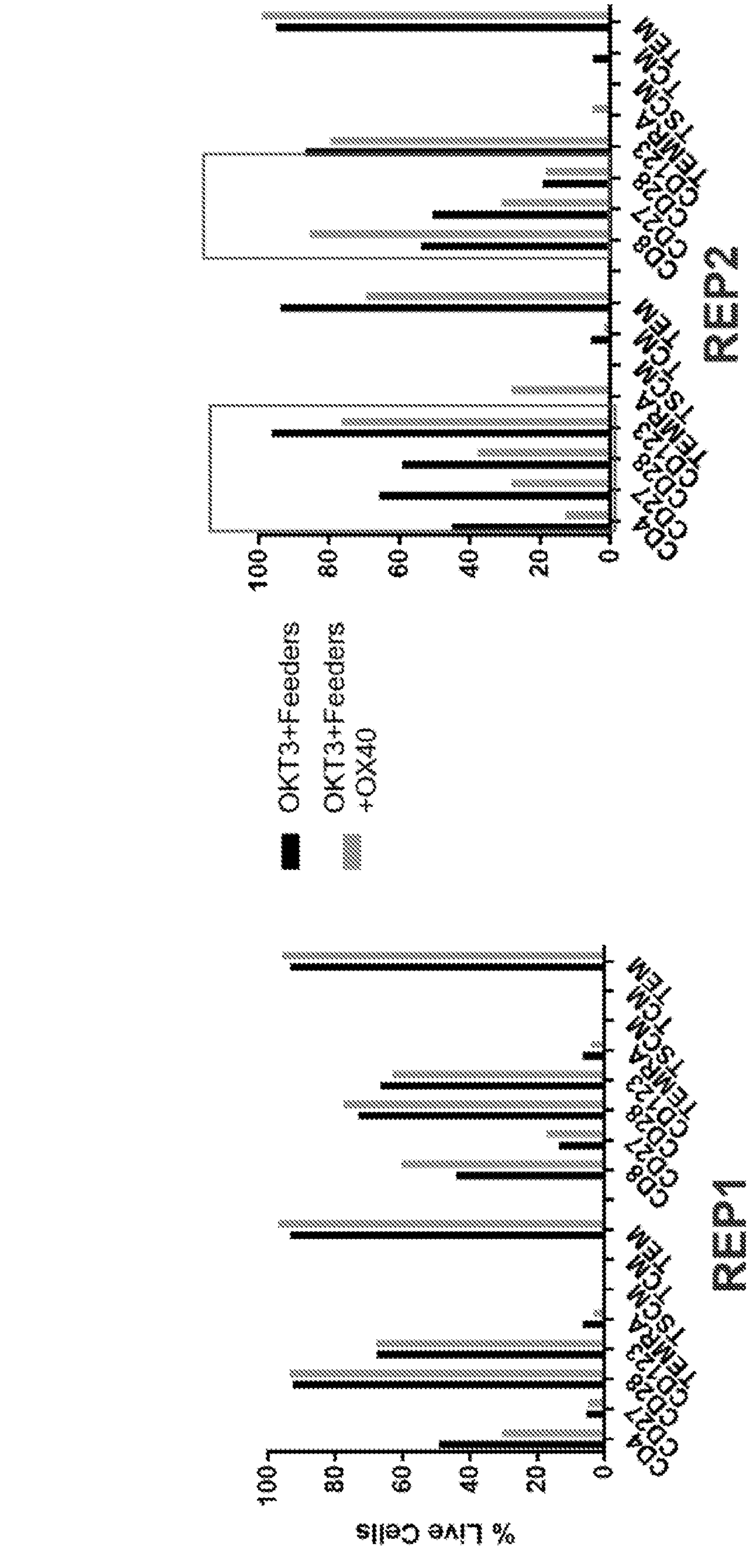
% Live Cells
100
80
60
40
20
0
CD4
CD27
CD28
CD123
TEMRA
TSCM
TCM
TEM
CD8
REP1
REP2
OKT3+Feeders
OKT3+Feeders
+OX40

*Figure 82*

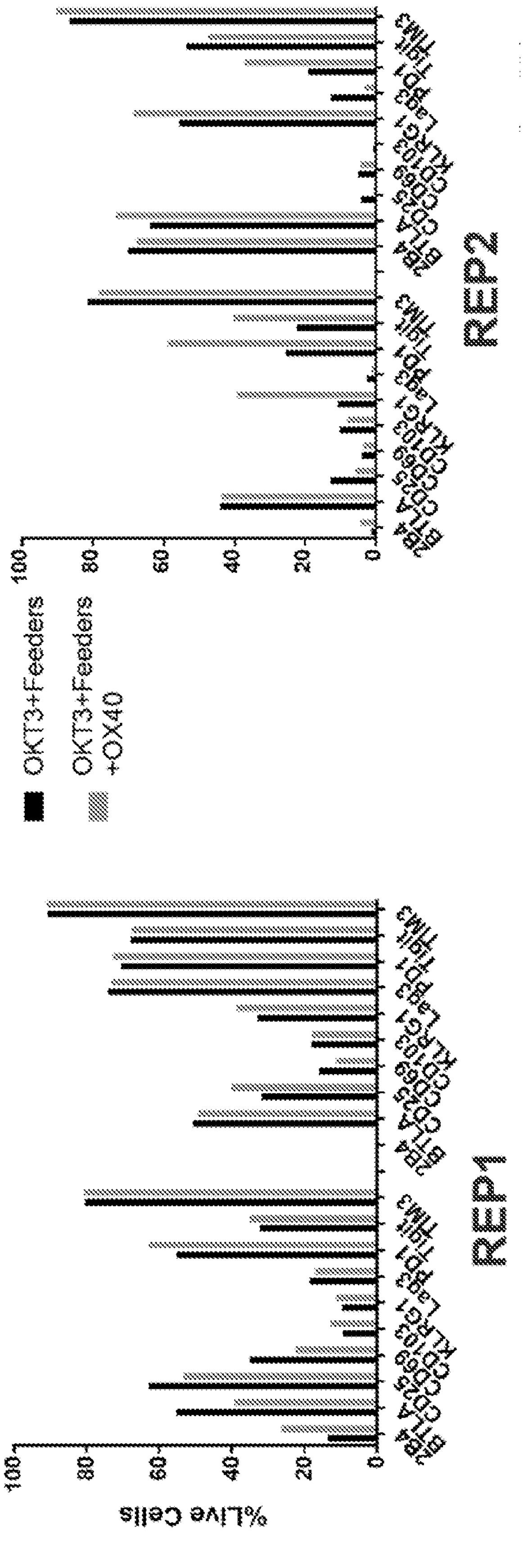

OKT3+Feeders
OKT3+Feeders +OX40
%Live Cells
100
80
60
40
20
0
2B4
BTLA
CD25
CD69
CD103
KLRG1
Lag3
PD1
Tigit
TIM3
REP1
REP2

*Figure 83*

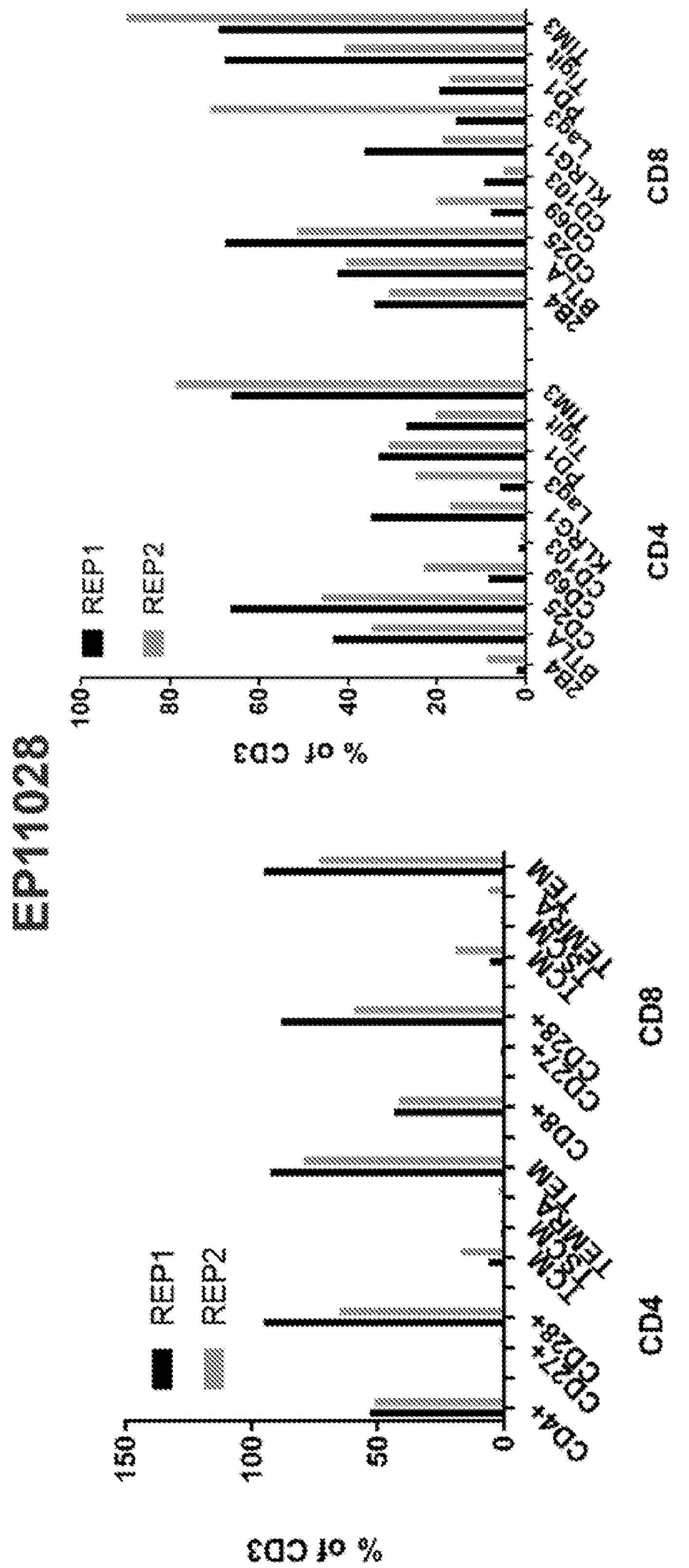
EP11028
% of CD3
150
100
50
0
REP1
REP2
CD4+
CD27+ CD28+
TCM
TSCM
TEMRA
TEM
CD8+
CD4
CD8
100
80
60
40
20
2B4
BTLA
CD25
CD69
CD103
KLRG1
Lag3
PD1
Tigit
TIM3

*Figure 84*

**EP11028**

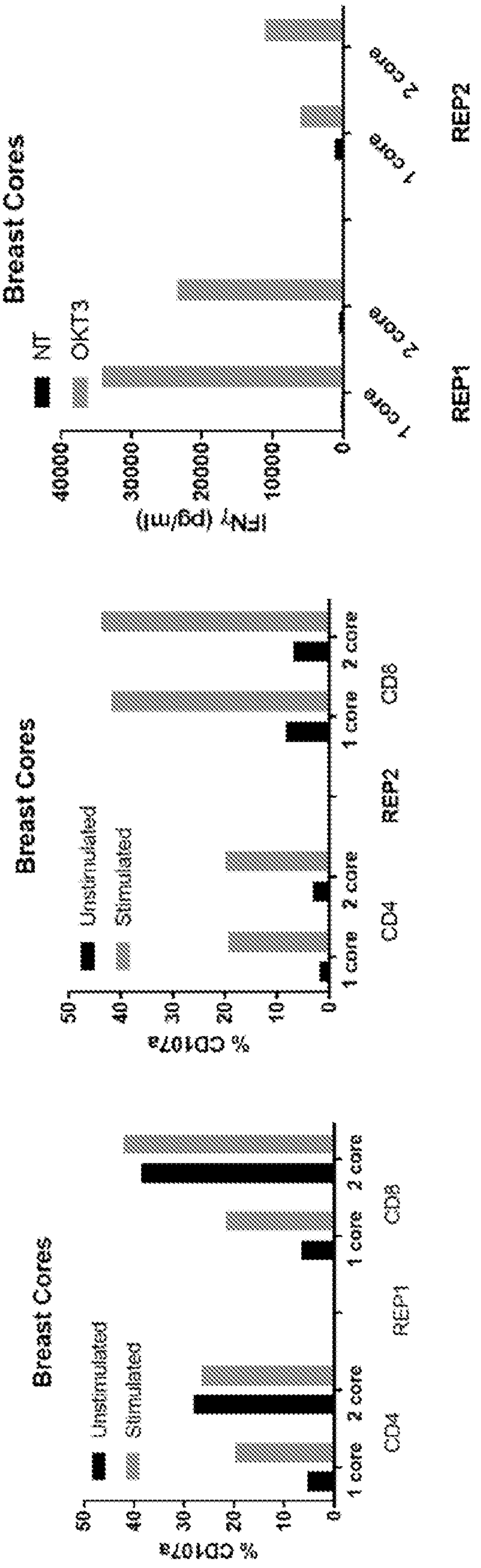

# *Figure 85A*

| Process 2A: about 22 days from Steps A - E | Process GEN 3: about 14-16 days from Steps A - E |
|---|---|
| **STEP A**<br>Obtain Patient Tumor Sample<br>(optionally can be frozen before Step B) | **STEP A**<br>Obtain Patient Tumor Sample<br>(optionally can be frozen before Step B) |
| **STEP B**<br>First Expansion<br>(physical fragmentation to at least 40 fragments per container grown for about 3 days to 14 days with media comprising IL-2) | **STEP B**<br>Priming First Expansion<br>(physical fragmentation of up to 60 fragments per container grown for about 1 days to 7 days with media comprising IL-2, OKT-3, and antigen-presenting feeder cells) |
| **STEP C**<br>First Expansion to Second Expansion Transition<br>(Step B TILs directly move to Step D, optionally on Step B day 11) | **STEP C**<br>Priming First Expansion to Rapid Second Expansion Transition<br>(Step B TILs directly move to Step D on day 7) |
| **STEP D**<br>Second Expansion<br>(TILs grown in growth media medium comprising IL-2, OKT-3, and antigen-presenting feeder cells in a closed container) | **STEP D**<br>Rapid Second Expansion<br>(TILs grown in growth media medium comprising IL-2, OKT-3, and 2X antigen-presenting feeder cells; Days 10-11 scale up and add additional IL-2) |
| **STEP E**<br>Harvest TILS from Step D<br>(TILs harvested via closed system) | **STEP E**<br>Harvest TILS from Step D |
| **STEP F**<br>Final Formulation and/or Transfer to Infusion Bag<br>(optionally cryopreserve) | **STEP F**<br>Final Formulation and/or Transfer to Infusion Bag<br>(optionally cryopreserve) |

# *Figure 85B*

**Small Biopsy GEN 3 Process: about 17-24 days from Steps A - E**

**STEP A**
Obtain Patient Tumor Sample
(optionally can be frozen before Step B)

**STEP B**
Priming First Expansion
(physical fragmentation of up to 60 fragments per container grown for about 3 days to 12 days with media comprising IL-2, OKT-3, and antigen-presenting feeder cells)

**STEP C**
Priming First Expansion to Rapid Second Expansion Transition
(Step B TILs directly move to Step D on day 7 to 12)

**STEP D**
Rapid Second Expansion
(TILs grown in growth media medium comprising IL-2, OKT-3, and 2X antigen-presenting feeder cells; Days 10-12 scale up and add additional IL-2)

**STEP E**
Harvest TILS from Step D

**STEP F**
Final Formulation and/or Transfer to Infusion Bag
(optionally cryopreserve)

# *Figure 85C*

| Small Biopsy Process: Version 2 (GEN3 based process)<br><br>About 17-24 days from Steps A - E |
|---|
| **STEP A**<br>Obtain Patient Tumor Sample |
| **STEP B**<br>First Expansion<br>3 days to 12 days (with OKT-3 and antigen-presenting feeder cells at any one of days 1-3) |
| **STEP C**<br>First Expansion to Second Expansion Transition<br>No Storage and optionally Closed System |
| **STEP D**<br>Second Expansion<br>IL-2, OKT-3, and antigen-presenting feeder cells<br>3 to 12 days<br>Optionally Closed System |
| **STEP E**<br>Harvest TILS from Step D<br>Closed System |
| **STEP F**<br>Final Formulation and/or Transfer to Infusion Bag<br>(optionally cryopreserve) |

*Figure 86*

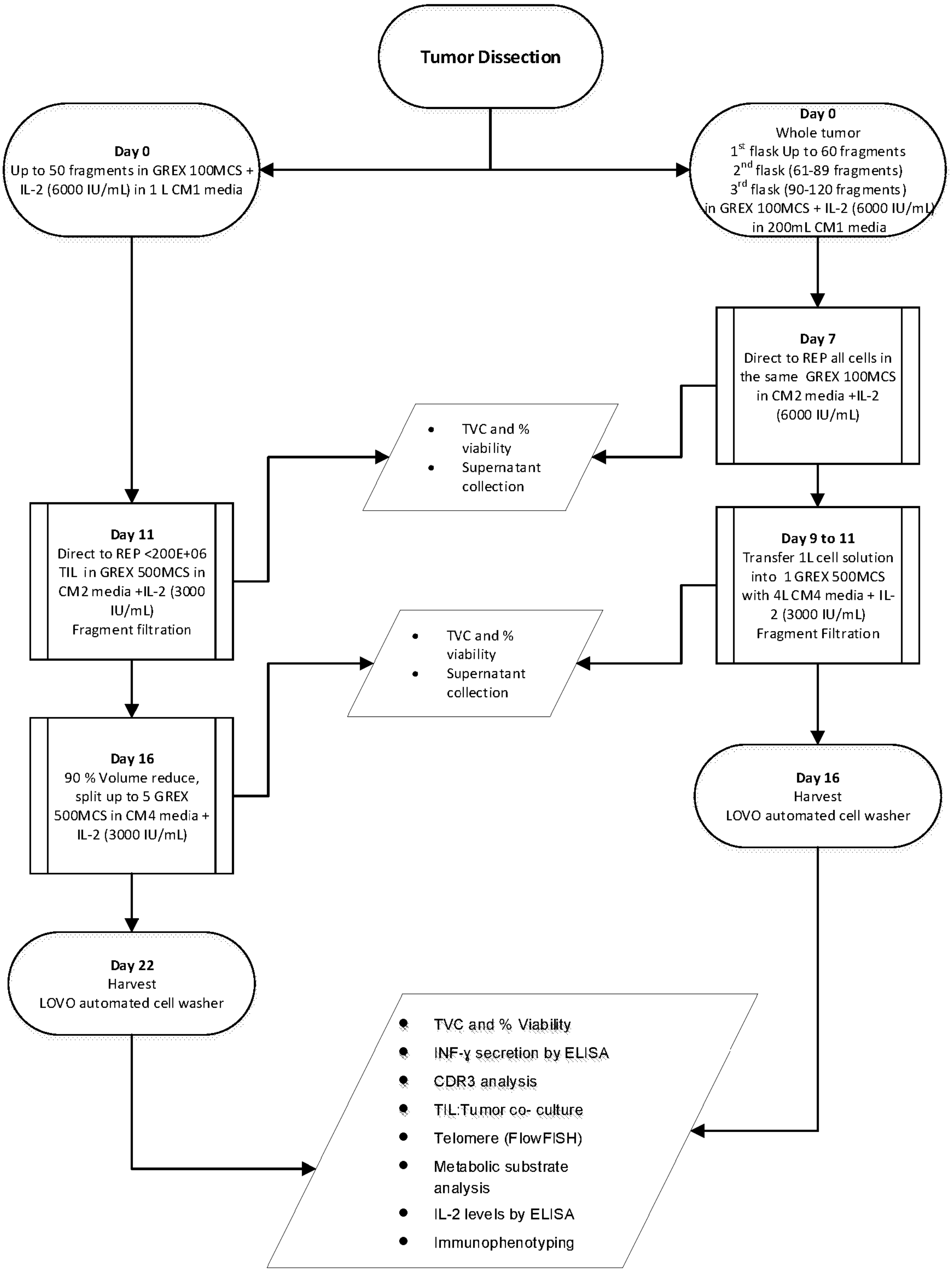
Tumor Dissection
Day 0
Up to 50 fragments in GREX 100MCS + IL-2 (6000 IU/mL) in 1 L CM1 media
Day 0
Whole tumor
1st flask Up to 60 fragments
2nd flask (61-89 fragments)
3rd flask (90-120 fragments)
in GREX 100MCS + IL-2 (6000 IU/mL)
in 200mL CM1 media
Day 7
Direct to REP all cells in the same GREX 100MCS in CM2 media +IL-2 (6000 IU/mL)
TVC and % viability
Supernatant collection
Day 11
Direct to REP <200E+06 TIL in GREX 500MCS in CM2 media +IL-2 (3000 IU/mL)
Fragment filtration
Day 9 to 11
Transfer 1L cell solution into 1 GREX 500MCS with 4L CM4 media + IL-2 (3000 IU/mL)
Fragment Filtration
TVC and % viability
Supernatant collection
Day 16
90 % Volume reduce, split up to 5 GREX 500MCS in CM4 media + IL-2 (3000 IU/mL)
Day 16
Harvest
LOVO automated cell washer
Day 22
Harvest
LOVO automated cell washer
TVC and % Viability
INF-γ secretion by ELISA
CDR3 analysis
TIL:Tumor co- culture
Telomere (FlowFISH)
Metabolic substrate analysis
IL-2 levels by ELISA
Immunophenotyping

*Figure 87*

A B C

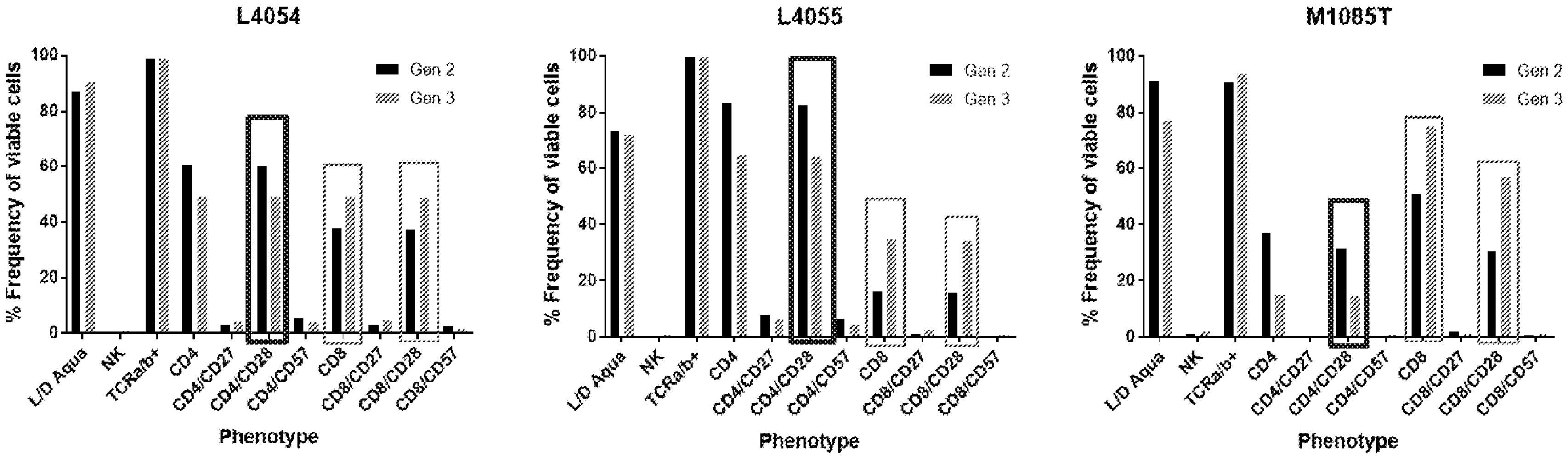


| Tumor ID | L4054 | | L4055 | | M1085T | |
|---|---|---|---|---|---|---|
| Process | Gen 2 | Gen 3 | Gen 2 | Gen 3 | Gen 2 | Gen 3 |
| # Fragments | 21 | 21 | 24 | 24 | 16 | 16 |

*Figure 88*

A B C

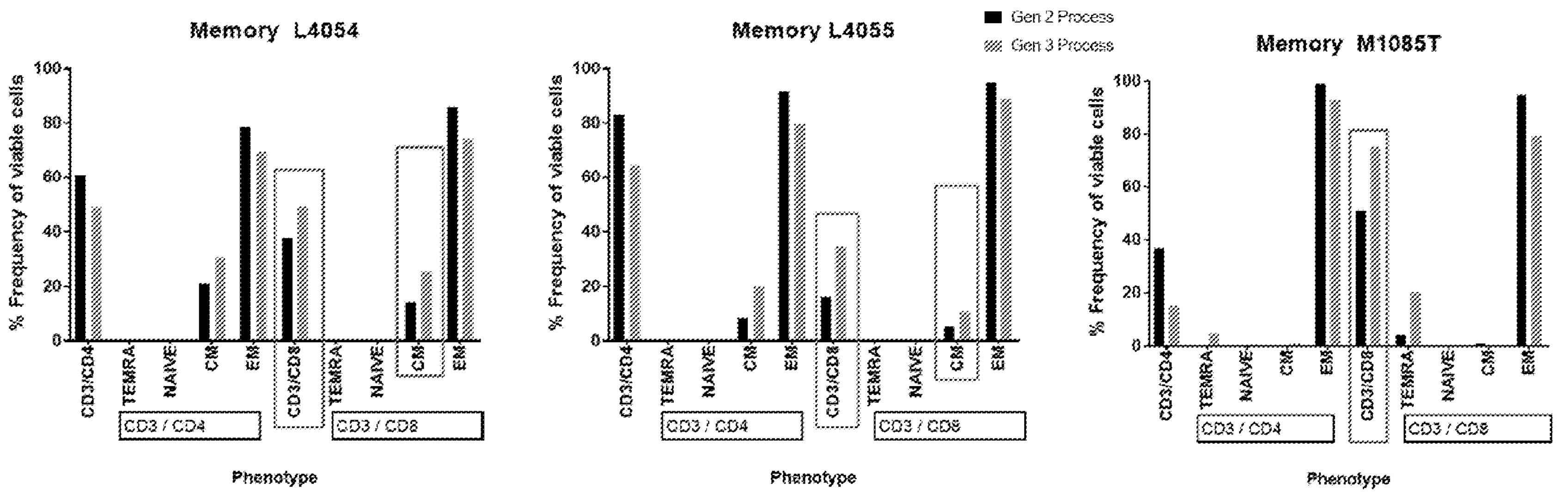


| Tumor ID | L4054 | | L4055 | | M1085T | |
|---|---|---|---|---|---|---|
| Process | Gen 2 | Gen 3 | Gen 2 | Gen 3 | Gen 2 | Gen 3 |
| # Fragments | 21 | 21 | 24 | 24 | 16 | 16 |

*Figure 89*

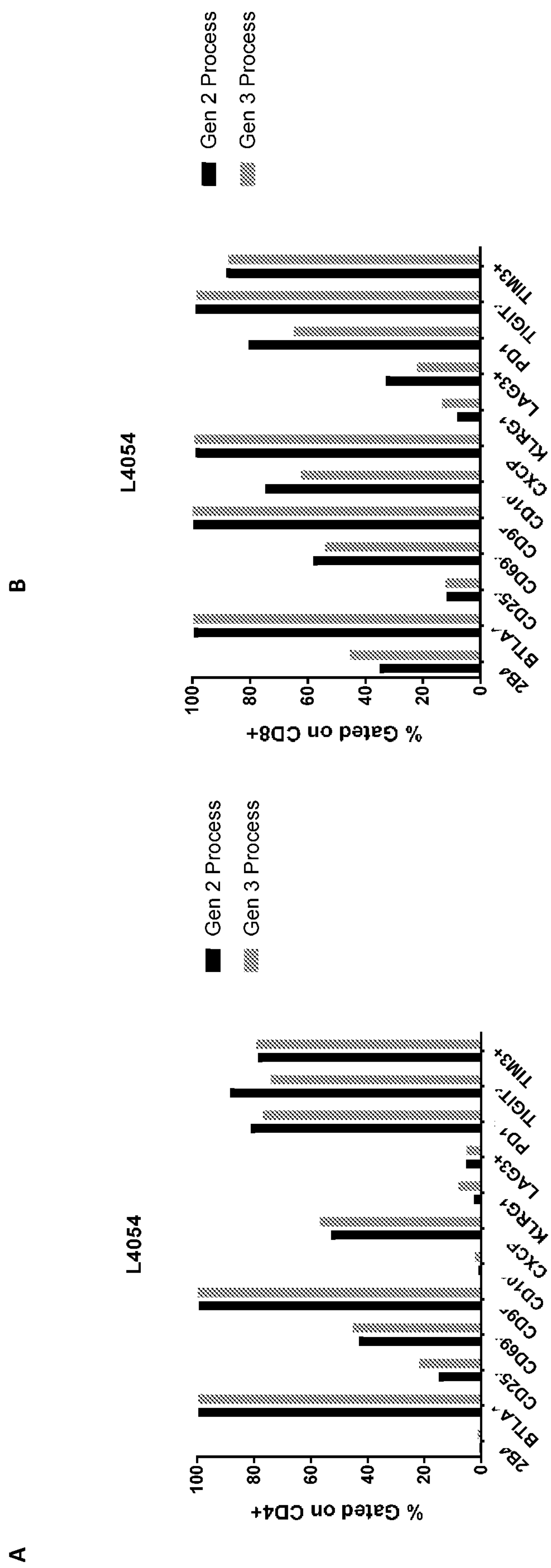
A
L4054
% Gated on CD4+
100
80
60
40
20
0
Gen 2 Process
Gen 3 Process
B
L4054
% Gated on CD8+
100
80
60
40
20
0
Gen 2 Process
Gen 3 Process

*Figure 90*

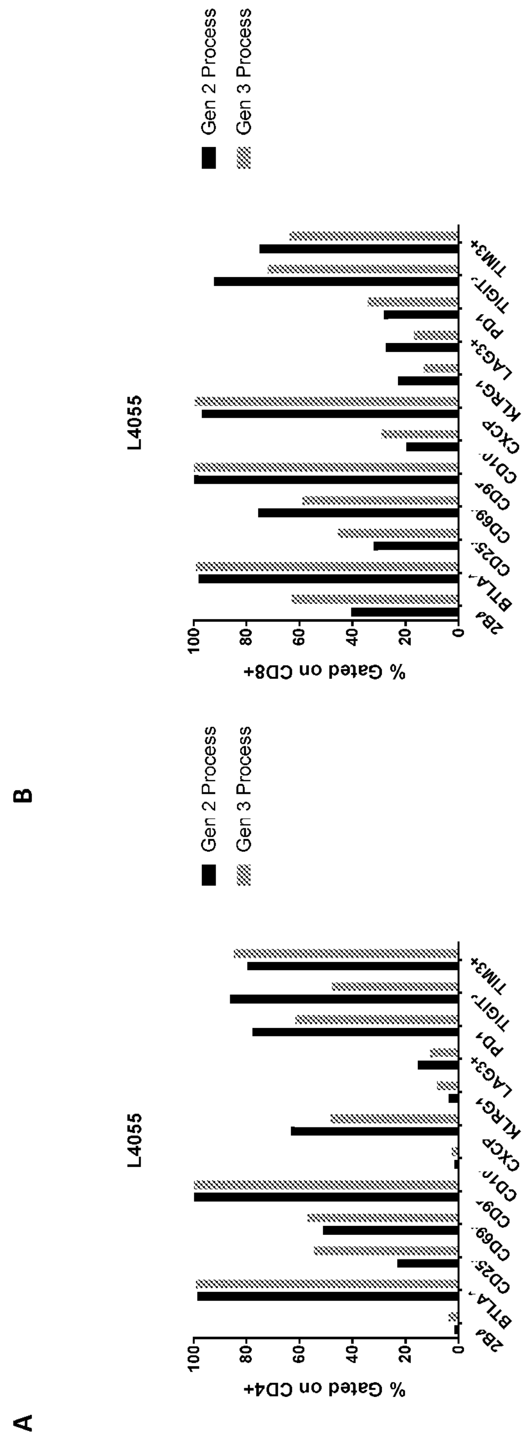
A
B
L4055
L4055
% Gated on CD4+
% Gated on CD8+
Gen 2 Process
Gen 3 Process
Gen 2 Process
Gen 3 Process

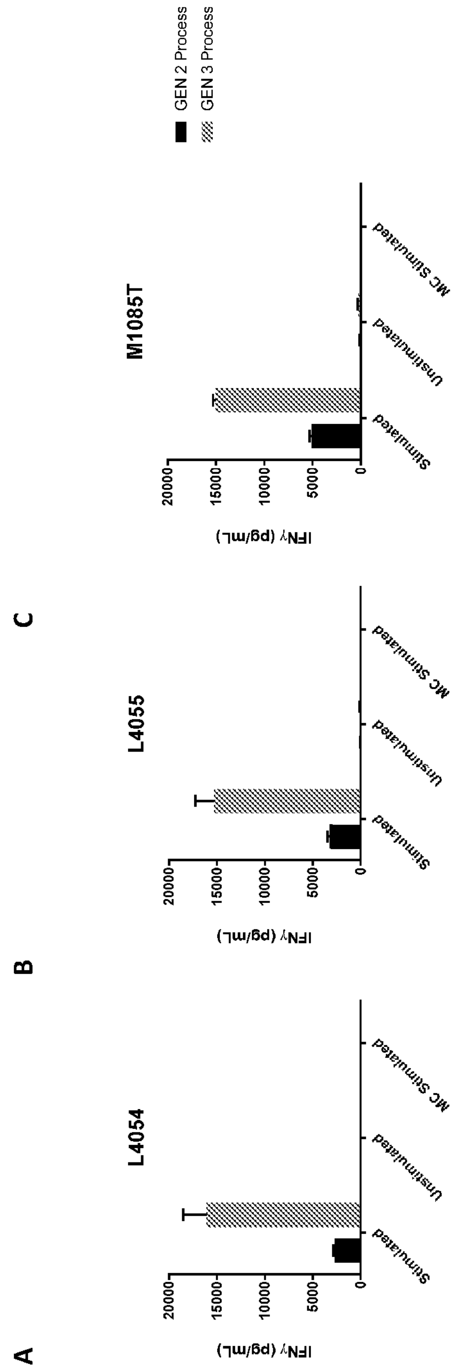
A
L4054
B
L4055
C
M1085T
IFNγ (pg/mL)
20000
15000
10000
5000
0
Stimulated
Unstimulated
MC Stimulated
GEN 2 Process
GEN 3 Process

*Figure 91*

*Figure 92*

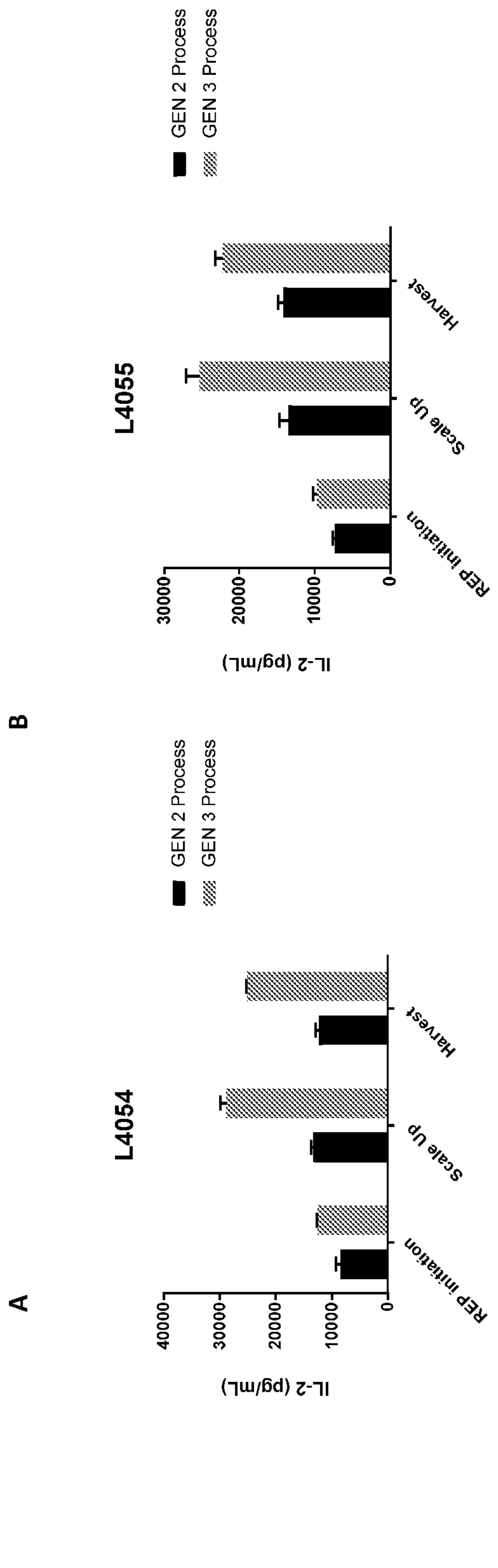
A
L4054
IL-2 (pg/mL)
40000
30000
20000
10000
0
REP Initiation
Scale Up
Harvest
GEN 2 Process
GEN 3 Process
B
L4055
IL-2 (pg/mL)
30000
20000
10000
0
REP Initiation
Scale Up
Harvest
GEN 2 Process
GEN 3 Process

*Figure 93*

A

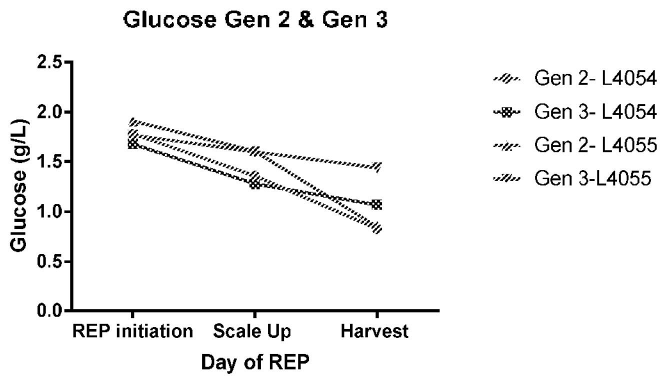
Glucose Gen 2 & Gen 3
Glucose (g/L)
2.5
2.0
1.5
1.0
0.5
0.0
REP initiation
Scale Up
Harvest
Day of REP
Gen 2- L4054
Gen 3- L4054
Gen 2- L4055
Gen 3-L4055

B

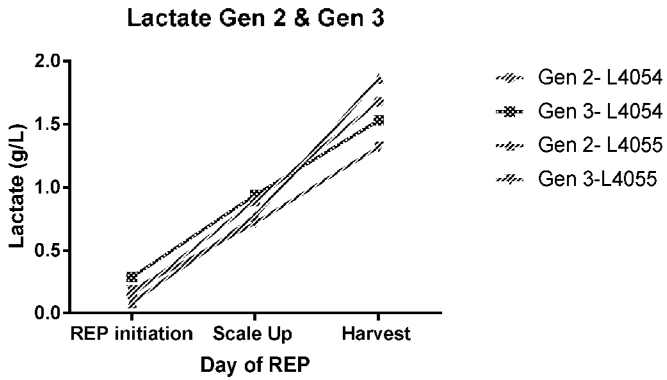
Lactate Gen 2 & Gen 3
Lactate (g/L)
2.0
1.5
1.0
0.5
0.0
REP initiation
Scale Up
Harvest
Day of REP
Gen 2- L4054
Gen 3- L4054
Gen 2- L4055
Gen 3-L4055

*Figure 94*

A

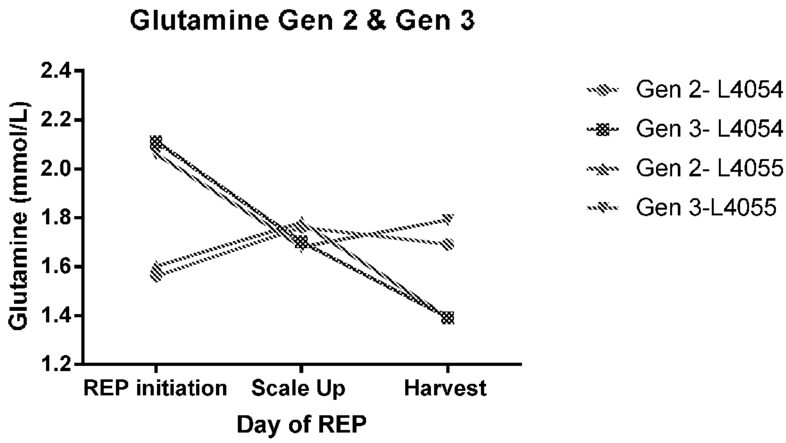
Glutamine Gen 2 & Gen 3
Glutamine (mmol/L)
2.4
2.2
2.0
1.8
1.6
1.4
1.2
REP initiation
Scale Up
Harvest
Day of REP
Gen 2- L4054
Gen 3- L4054
Gen 2- L4055
Gen 3-L4055

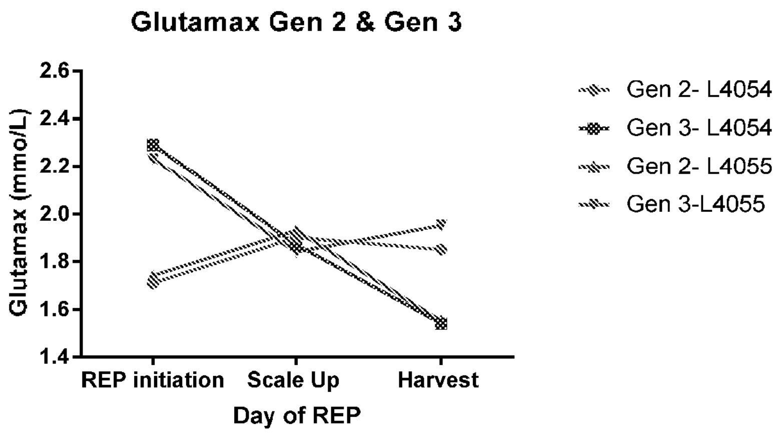
Glutamax Gen 2 & Gen 3
Glutamax (mmol/L)
2.6
2.4
2.2
2.0
1.8
1.6
1.4
REP initiation
Scale Up
Harvest
Day of REP
Gen 2- L4054
Gen 3- L4054
Gen 2- L4055
Gen 3-L4055

B

*Figure 94*

C

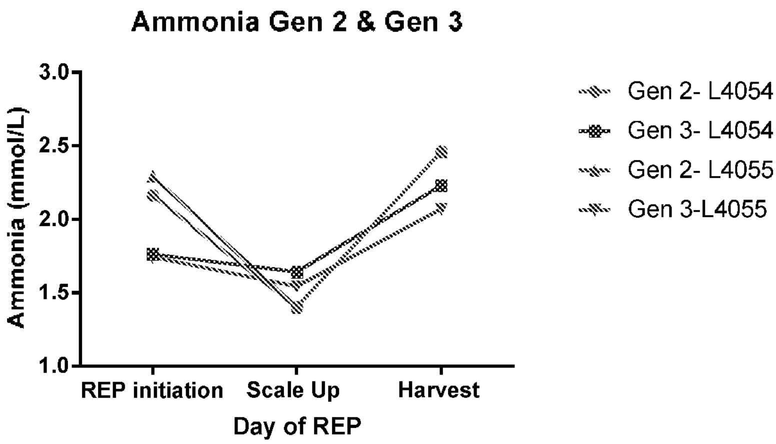
Ammonia Gen 2 & Gen 3
Ammonia (mmol/L)
3.0
2.5
2.0
1.5
1.0
REP initiation
Scale Up
Harvest
Day of REP
Gen 2- L4054
Gen 3- L4054
Gen 2- L4055
Gen 3-L4055

*Figure 95*

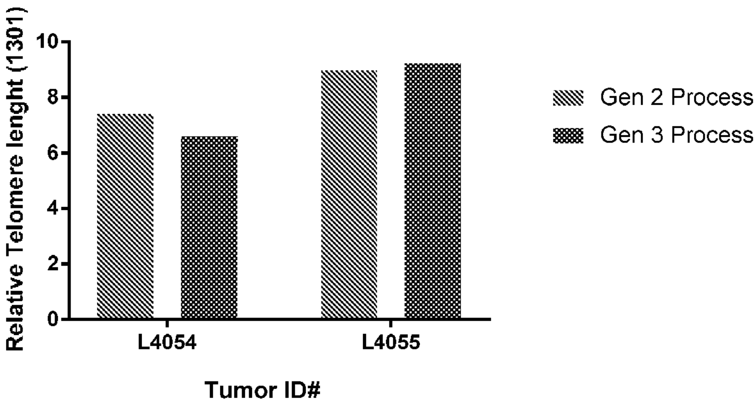
Relative Telomere lenght (1301)
10
8
6
4
2
0
L4054
L4055
Tumor ID#
Gen 2 Process
Gen 3 Process

*Figure 96*

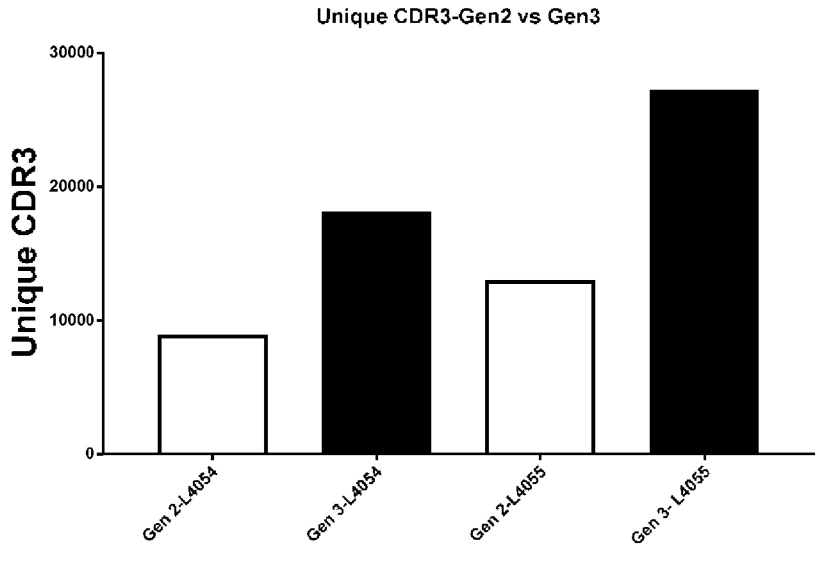
Unique CDR3-Gen2 vs Gen3
Unique CDR3
30000
20000
10000
0
Gen 2-L4054
Gen 3-L4054
Gen 2-L4055
Gen 3- L4055

*Figure 97*

| # uCDR3 (% Overlap) | All uCDR3's | | Top 80% uCDR3's | |
|---|---|---|---|---|
| | Gen 2 | Gen 3 | Gen 2 | Gen 3 |
| **Gen 2-L4054** | 8915 | 4355 **(48.85%)** | 205 | 199 **(97.07%)** |
| **Gen 3-L4054** | - | 18130 | - | 223 |

- 199 sequences are shared between Gen 3 and Gen 2 final product, corresponding to 97.07% of top 80% of unique CDR3 sequences from Gen 2 shared with Gen 3 final product.

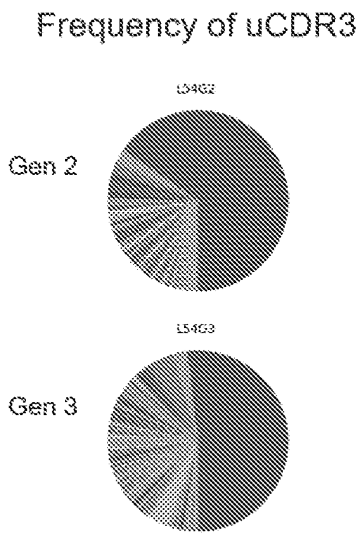

*Figure 98*

| # uCDR3 (% Overlap) | All uCDR3's | | Top 80% uCDR3's | |
|---|---|---|---|---|
| | Gen 2 | Gen 3 | Gen 2 | Gen 3 |
| Gen 2-L4055 | 12996 | 6599 **(50.77%)** | 1843 | 1833 **(99.45%)** |
| Gen 3-L4055 | - | 27246 | - | 2616 |

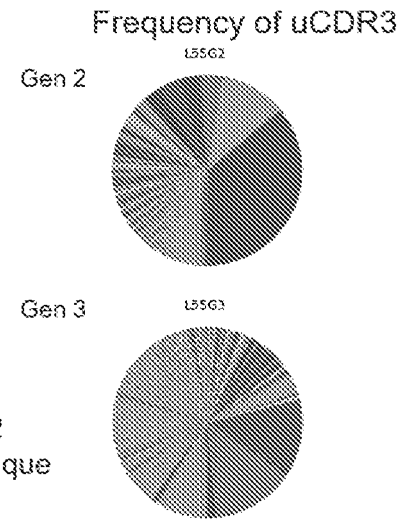


- 1833 sequences are shared between Gen 3 and Gen 2 final product, corresponding to 99.45% of top 80% of unique CDR3 sequences from Gen 2 shared with Gen 3 final product.

23

© 2018, Iovance Biotherapeutics

*Figure 99*

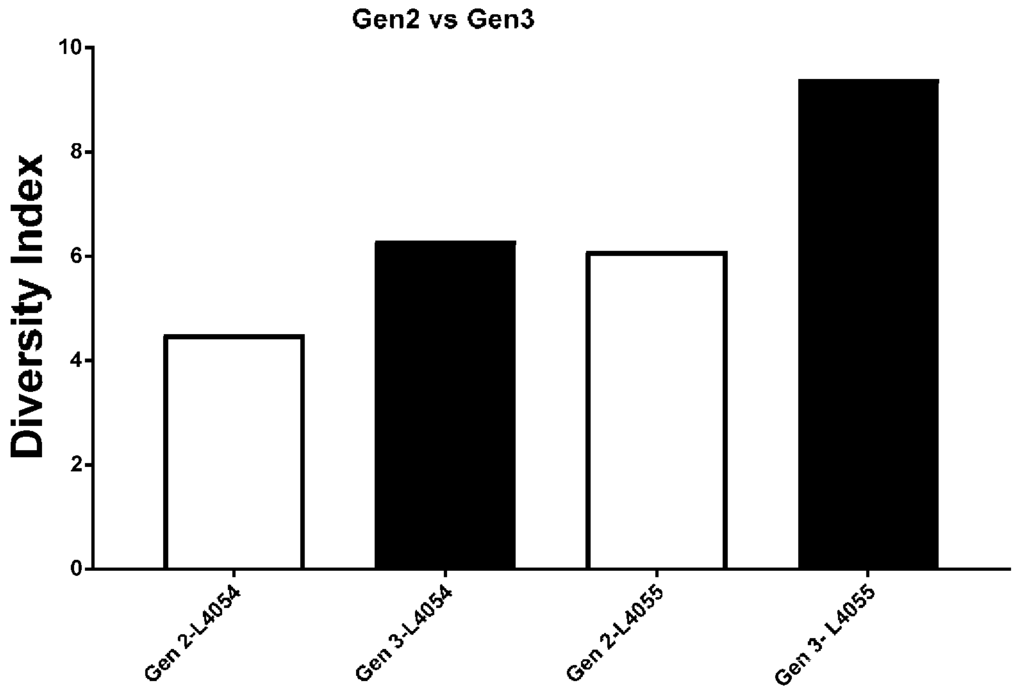
Gen2 vs Gen3
Diversity Index
10
8
6
4
2
0
Gen 2-L4054
Gen 3-L4054
Gen 2-L4055
Gen 3- L4055

*Figure 100*

| Volume (mL) | | Count 1 (cells/mL) | Count 2 (cells/mL) | Count 3 (cells/mL) | Average (cells/mL) | Total Cells Average*Volume |
|---|---|---|---|---|---|---|
| 188 | Total | 2.46E+05 | 2.28E+05 | 2.31E+05 | 2.35E+05 | 4.42E+07 |
| | Live | 2.37E+05 | 2.27E+05 | 2.26E+05 | 2.30E+05 | 4.32E+07 |
| | Dead | 9.26E+03 | 7.03E+02 | 4.63E+03 | 4.86E+03 | 9.14E+05 |
| | % Viability | 96.20% | 99.70% | 98.00% | 97.97% | |
| 188 | Total | 8.26E+04 | 8.00E+04 | 7.66E+04 | 7.97E+04 | 1.50E+07 |
| | Live | 7.60E+04 | 7.54E+04 | 6.89E+04 | 7.34E+04 | 1.38E+07 |
| | Dead | 6.61E+03 | 4.63E+03 | 7.73E+03 | 6.32E+03 | 1.19E+06 |
| | % Viability | 92.00% | 94.20% | 89.90% | 92.03% | |
| 200 | Total | 2.44E+04 | 4.20E+04 | 1.05E+04 | 2.56E+04 | 5.13E+06 |
| | Live | 1.74E+04 | 2.80E+04 | 7.03E+03 | 1.75E+04 | 3.50E+06 |
| | Dead | 7.03E+03 | 1.40E+04 | 3.48E+03 | 8.17E+03 | 1.63E+06 |
| | % Viability | 71.20% | 66.70% | 66.90% | 68.27% | |

*Figure 101*

| ID | Volume (mL) | | Count 1 (cells/mL) | Count 3 (cells/mL) | Count 2 (cells/mL) | Average (cells/mL) | Total Cells Average*Volume |
|---|---|---|---|---|---|---|---|
| Gen 3 #L4054 First Round | 1000 | Total | 9.11E+05 | 1.01E+06 | 1.08E+06 | 1.00E+06 | 1.00E+09 |
| | | Live | 8.60E+05 | 9.34E+05 | 1.01E+06 | 9.35E+05 | 9.35E+08 |
| | | Dead | 5.09E+04 | 7.49E+04 | 6.74E+04 | 6.44E+04 | 6.44E+07 |
| | | % Viability | 94.40% | 92.60% | 93.70% | 93.57% | |
| Gen 3 #L4055 Second Round | 1000 | Total | 8.56E+05 | 9.04E+05 | 8.81E+05 | 8.80E+05 | 8.80E+08 |
| | | Live | 8.24E+05 | 8.67E+05 | 8.42E+05 | 8.44E+05 | 8.44E+08 |
| | | Dead | 3.17E+04 | 3.70E+04 | 3.87E+04 | 3.58E+04 | 3.58E+07 |
| | | % Viability | 96.30% | 95.90% | 95.60% | 95.93% | |
| Gen 3 #M1085T | 200 | Total | 2.32E+06 | 2.25E+06 | | 2.29E+06 | 4.57E+08 |
| | | Live | 1.68E+06 | 1.57E+06 | | 1.63E+06 | 3.25E+08 |
| | | Dead | 6.41E+05 | 6.79E+05 | | 6.60E+05 | 1.32E+08 |
| | | % Viability | 72.40% | 69.90% | | 71.15% | |
| Gen 2 #L4054 First Round | 142 | Total | 1.08E+06 | 9.84E+05 | 1.00E+06 | 1.02E+06 | 1.45E+08 |
| | | Live | 1.06E+06 | 9.71E+05 | 9.79E+05 | 1.00E+06 | 1.42E+08 |
| | | Dead | 2.05E+04 | 1.26E+04 | 2.12E+04 | 1.81E+04 | 2.57E+06 |
| | | % Viability | 98.10% | 98.70% | 97.90% | 98.23% | |
| Gen 2 #L4055 Second Round | 96 | Total | 2.93E+05 | 3.05E+05 | 2.64E+05 | 2.87E+05 | 2.76E+07 |
| | | Live | 2.88E+05 | 2.96E+05 | 2.55E+05 | 2.80E+05 | 2.68E+07 |
| | | Dead | 4.72E+03 | 9.14E+03 | 8.26E+03 | 7.37E+03 | 7.08E+05 |
| | | % Viability | 98.40% | 97.00% | 96.90% | 97.43% | |
| Gen 2 #M1085T | 200 | Total | 9.10E+04 | 5.60E+04 | | 7.35E+04 | 1.47E+07 |
| | | Live | 8.05E+04 | 4.21E+04 | | 6.13E+04 | 1.23E+07 |
| | | Dead | 1.04E+04 | 1.39E+04 | | 1.22E+04 | 2.43E+06 |
| | | % Viability | 88.50% | 75.20% | | 81.85% | |

# *Figure 102*

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 3 #L4054 First Round -pre LOVO | 1243.1 | Total | 1.40E+07 | 1.46E+07 | | 1.43E+07 | 1.78E+10 |
| | | Live | 1.32E+07 | 1.35E+07 | | 1.34E+07 | 1.66E+10 |
| | | Dead | 8.47E+05 | 1.12E+06 | | 9.84E+05 | 1.22E+09 |
| | | % Viability | 94.00% | 92.40% | | 93.20% | |
| Gen 3 #L4054 First Round -post LOVO | 330 | Total | 6.35E+07 | 6.07E+07 | | 6.21E+07 | 2.05E+10 |
| | | Live | 5.72E+07 | 5.44E+07 | | 5.58E+07 | 1.84E+10 |
| | | Dead | 6.35E+06 | 6.27E+06 | | 6.31E+06 | 2.08E+09 |
| | | % Viability | 90.00% | 89.70% | | 89.85% | |

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 3 #L4055 Second Round -pre LOVO | 1032.6 | Total | 9.49E+06 | 1.05E+07 | | 1.00E+07 | 1.03E+10 |
| | | Live | 8.90E+06 | 9.79E+06 | | 9.35E+06 | 9.65E+09 |
| | | Dead | 5.85E+05 | 6.75E+05 | | 6.30E+05 | 6.51E+08 |
| | | % Viability | 93.80% | 93.60% | | 93.70% | |
| Gen 3 #L4055 Second Round -post LOVO | 330 | Total | 3.31E+07 | 2.82E+07 | 3.18E+07 | 3.07E+07 | 1.01E+10 |
| | | Live | 2.85E+07 | 2.46E+07 | 2.77E+07 | 2.66E+07 | 8.76E+09 |
| | | Dead | 4.56E+06 | 3.61E+06 | 4.12E+06 | 4.09E+06 | 1.35E+09 |
| | | % Viability | 86.20% | 87.20% | 87.10% | 86.70% | |

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 3 #M1085T pre-LOVO | 5000 | Total | 2.26E+06 | 2.64E+06 | | 2.45E+06 | 1.23E+10 |
| | | Live | 1.97E+06 | 2.31E+06 | | 2.14E+06 | 1.07E+10 |
| | | Dead | 2.85E+05 | 3.30E+05 | | 3.08E+05 | 1.54E+09 |
| | | % Viability | 87.40% | 87.50% | | 87.45% | |
| Gen 3 #M1085T - post LOVO pre CS10 addition | 150 | Total | 6.27E+07 | 5.44E+07 | | 5.86E+07 | 8.78E+09 |
| | | Live | 5.50E+07 | 4.74E+07 | | 5.12E+07 | 7.68E+09 |
| | | Dead | 7.70E+06 | 6.96E+06 | | 7.33E+06 | 1.10E+09 |
| | | % Viability | 87.70% | 87.20% | | 87.45% | |

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 2 #L4054 First Round | 913 | Total | 3.53E+06 | 4.30E+06 | | 3.92E+06 | 3.57E+09 |
| | | Live | 3.35E+06 | 4.00E+06 | | 3.68E+06 | 3.36E+09 |
| | | Dead | 1.75E+05 | 3.03E+05 | | 2.39E+05 | 2.18E+08 |
| | | % Viability | 95.00% | 93.00% | | 94.00% | |
| Gen 2 #L4055 Second Round | 292 | Total | 1.29E+07 | 1.36E+07 | | 1.33E+07 | 3.87E+09 |
| | | Live | 1.16E+07 | 1.23E+07 | | 1.20E+07 | 3.49E+09 |
| | | Dead | 1.27E+06 | 1.23E+06 | | 1.25E+06 | 3.65E+08 |
| | | % Viability | 90.10% | 90.90% | | 90.50% | |
| Gen 2 #M1085T | 369 | Total | 6.75E+06 | 6.98E+06 | | 6.87E+06 | 2.53E+09 |
| | | Live | 5.22E+06 | 5.58E+06 | | 5.40E+06 | 1.99E+09 |
| | | Dead | 1.54E+06 | 1.40E+06 | | 1.47E+06 | 5.42E+08 |
| | | % Viability | 77.20% | 79.90% | | 78.55% | |

*Figure 103*

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 2 #L4054 First Round -pre LOVO | 1385.7 | Total | 3.55E+07 | 4.12E+07 | 4.03E+07 | 3.90E+07 | 5.40E+10 |
| | | Live | 3.26E+07 | 3.73E+07 | 3.67E+07 | 3.55E+07 | 4.92E+10 |
| | | Dead | 2.93E+06 | 3.87E+06 | 3.59E+06 | 3.46E+06 | 4.80E+09 |
| | | % Viability | 91.70% | 90.60% | 91.10% | 91.13% | |
| Gen 2 #L4054 First Round -post LOVO | 330 | Total | 1.70E+08 | 1.79E+08 | 1.68E+08 | 1.72E+08 | 5.69E+10 |
| | | Live | 1.49E+08 | 1.58E+08 | 1.48E+08 | 1.52E+08 | 5.01E+10 |
| | | Dead | 2.16E+07 | 2.04E+07 | 2.00E+07 | 2.07E+07 | 6.82E+09 |
| | | % Viability | 87.30% | 88.60% | 88.10% | 87.95% | |

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 2 #L4055 First Round -pre LOVO | 1968.2 | Total | 3.15E+07 | 2.51E+07 | 2.97E+07 | 2.88E+07 | 5.66E+10 |
| | | Live | 2.89E+07 | 2.25E+07 | 2.72E+07 | 2.62E+07 | 5.16E+10 |
| | | Dead | 2.57E+06 | 2.61E+06 | 2.52E+06 | 2.57E+06 | 5.05E+09 |
| | | % Viability | 91.80% | 89.60% | 91.50% | 90.97% | |
| Gen 2 #L4055 First Round -post LOVO | 330 | Total | 2.33E+08 | 1.89E+08 | 1.53E+08 | 1.92E+08 | 6.33E+10 |
| | | Live | 2.03E+08 | 1.66E+08 | 1.33E+08 | 1.67E+08 | 5.52E+10 |
| | | Dead | 3.00E+07 | 2.24E+07 | 1.94E+07 | 2.39E+07 | 7.90E+09 |
| | | % Viability | 87.10% | 88.10% | 87.30% | 87.50% | |

| ID | Volume (mL) | | Count 1 (cells/mL) | Count 2 (cells/mL) | Count 3 (cells/mL) | Average (cells/mL) | Total Cells Average*Volume |
|---|---|---|---|---|---|---|---|
| Gen 2 #M1085T-pre LOVO | N/A | Total | | | | #DIV/0! | #DIV/0! |
| | | Live | | | | #DIV/0! | #DIV/0! |
| | | Dead | | | | #DIV/0! | #DIV/0! |
| | | % Viability | | | | #DIV/0! | |
| Gen 2 #M1085T - post LOVO pre CS10 addition | 150 | Total | 8.51E+07 | 9.05E+07 | | 8.78E+07 | 1.32E+10 |
| | | Live | 7.33E+07 | 7.79E+07 | | 7.56E+07 | 1.13E+10 |
| | | Dead | 1.18E+07 | 1.26E+07 | | 1.22E+07 | 1.83E+09 |
| | | % Viability | 86.10% | 86.10% | | 86.10% | |

**For L4054 Gen 2, post LOVO count was extrapolated to 4 flasks, because was the total number of the study. 1 flask was contaminated, and the extrapolation was done for total = 6.67E+10**

# *Figure 104*

| | S1/S2/All/Live \| Freq. of Parent (%) | S1/S2/All/Live/CD14+ \| Freq. of Parent (%) | S1/S2/All/Live/CD14-/CD3-/NK \| Freq. of null (%) | S1/S2/All/Live/CD14-/CD19 \| Freq. of null (%) | S1/S2/All/Live/CD14-/TCRab \| Freq. of null (%) | S1/S2/All/Live/CD14-/TCRab/CD4 \| Freq. of null (%) | S1/S2/All/Live/CD14-/TCRab/CD4/CD27 \| Freq. of null (%) | S1/S2/All/Live/CD14-/TCRab/CD4/CD28 \| Freq. of null (%) | S1/S2/All/Live/CD14-/TCRab/CD4/CD56 \| Freq. of null (%) | S1/S2/All/Live/CD14-/TCRab/CD4/CD57 \| Freq. of null (%) | S1/S2/All/Live/CD14-/TCRab/CD4/Q1: CCR7-PE-A-, CD45RA-AF700-A+ \| Freq. of Parent (%) | S1/S2/All/Live/CD14-/TCRab/CD4/Q2: CCR7-PE-A+, CD45RA-AF700-A+ \| Freq. of Parent (%) | S1/S2/All/Live/CD14-/TCRab/CD4/Q3: CCR7-PE-A+, CD45RA-AF700-A- \| Freq. of Parent (%) | S1/S2/All/Live/CD14-/TCRab/CD4/Q4: CCR7-PE-A-, CD45RA-AF700-A- \| Freq. of Parent (%) | S1/S2/All/Live/CD14-/TCRab/CD4/Q5: CD62L-BV421-A-, CD45RA-AF700-A+ \| Freq. of Parent (%) | S1/S2/All/Live/CD14-/TCRab/CD4/Q6: CD62L-BV421-A+, CD45RA-AF700-A+ \| Freq. of Parent (%) | S1/S2/All/Live/CD14-/TCRab/CD4/Q7: CD62L-BV421-A+, CD45RA-AF700-A- \| Freq. of Parent (%) | S1/S2/All/Live/CD14-/TCRab/CD4/Q8: CD62L-BV421-A-, CD45RA-AF700-A- \| Freq. of Parent (%) |
|---|---|---|---|---|---|---|---|---|---|---|---|---|---|---|---|---|---|---|
| | | | | | | | | | | | TEMRA | NAÏVE | CM | EM | TEMRA | NAÏVE | CM | EM |
| A1 gen2 1st.fcs | 87.2 | 1.77E-03 | 0.66 | 0 | 98.9 | 60.6 | 3.35 | 60.1 | 0.06 | 5.34 | 0.25 | 0.073 | 3.37 | 96.3 | 0.056 | 0.032 | 21.1 | 78.8 |
| A2 gen2 2nd.fcs | 73.5 | 0 | 0.12 | 0 | 99.7 | 83.1 | 7.94 | 82.6 | 0.14 | 6.18 | 0.16 | 0.047 | 2.39 | 97.4 | 0.036 | 0.014 | 8.3 | 91.6 |
| A3 gen3 1st.fcs | 90.4 | 0 | 0.97 | 1.72E-03 | 98.8 | 49.1 | 4.5 | 48.9 | 0.18 | 4.02 | 0.2 | 0.059 | 2.29 | 97.5 | 0.059 | 0.049 | 30.7 | 69.2 |
| A4 gen3 2nd.fcs | 72.1 | 2.56E-03 | 0.44 | 0.018 | 99.3 | 64.4 | 6.27 | 64 | 0.26 | 4.3 | 0.25 | 0.18 | 7.12 | 92.5 | 0.12 | 0.072 | 20.1 | 79.7 |
| A5 PBMC.fcs | 93.7 | 6.07 | 1.6 | 6.12 | 70.9 | 41.1 | 35.6 | 40.4 | 0.041 | 0.31 | 0.91 | 36.1 | 16.2 | 46.9 | 11.4 | 23.1 | 16.4 | 49.1 |

l area was include to make the graph
TCRab+ are = CD3+

| S1/S2/All/Live/CD14-/TCRab/CD8 \| Freq. of null (%) | S1/S2/All/Live/CD14-/TCRab/CD8/CD27 \| Freq. of null (%) | S1/S2/All/Live/CD14-/TCRab/CD8/CD28 \| Freq. of null (%) | S1/S2/All/Live/CD14-/TCRab/CD8/CD56 \| Freq. of null (%) | S1/S2/All/Live/CD14-/TCRab/CD8/CD57 \| Freq. of null (%) | S1/S2/All/Live/CD14-/TCRab/CD8/Q1: CCR7-PE-A-, CD45RA-AF700-A+ \| Freq. of Parent (%) | S1/S2/All/Live/CD14-/TCRab/CD8/Q2: CCR7-PE-A+, CD45RA-AF700-A+ \| Freq. of Parent (%) | S1/S2/All/Live/CD14-/TCRab/CD8/Q3: CCR7-PE-A+, CD45RA-AF700-A- \| Freq. of Parent (%) | S1/S2/All/Live/CD14-/TCRab/CD8/Q4: CCR7-PE-A-, CD45RA-AF700-A- \| Freq. of Parent (%) | S1/S2/All/Live/CD14-/TCRab/CD8/Q5: CD62L-BV421-A-, CD45RA-AF700-A+ \| Freq. of Parent (%) | S1/S2/All/Live/CD14-/TCRab/CD8/Q6: CD62L-BV421-A+, CD45RA-AF700-A+ \| Freq. of Parent (%) | S1/S2/All/Live/CD14-/TCRab/CD8/Q7: CD62L-BV421-A+, CD45RA-AF700-A- \| Freq. of Parent (%) | S1/S2/All/Live/CD14-/TCRab/CD8/Q8: CD62L-BV421-A-, CD45RA-AF700-A- \| Freq. of Parent (%) | S1/S2/All/Live/CD14-/TCRgd \| Freq. of null (%) |
|---|---|---|---|---|---|---|---|---|---|---|---|---|---|
| | | | | | TEMRA | NAÏVE | CM | EM | TEMRA | NAÏVE | CM | EM | |
| 37.9 | 3.25 | 37.3 | 0.41 | 2.31 | 0.028 | 0 | 4.24 | 95.7 | 0.033 | 9.37E-03 | 14.1 | 85.9 | 0 |
| 16.1 | 1.08 | 15.8 | 0.67 | 0.17 | 0.042 | 0.014 | 4.76 | 95.2 | 0.071 | 0.014 | 5.24 | 94.7 | 0 |
| 49.2 | 4.83 | 48.5 | 0.71 | 1.53 | 0.056 | 6.97E-03 | 3.11 | 96.8 | 0.077 | 0.017 | 25.5 | 74.4 | 0 |
| 34.6 | 2.46 | 34 | 1.57 | 0.5 | 0.18 | 0.13 | 10.2 | 89.5 | 0.29 | 0.044 | 10.6 | 89.1 | 0 |
| 29.6 | 25.8 | 27.4 | 0.75 | 2.81 | 8.05 | 40.9 | 3.02 | 48 | 22.3 | 27.1 | 6.23 | 44.3 | 0 |

*Figure 105*

| | CD3/CD4 CD38 | HLA-DR | CCR7- CD45RA+ | CCR7+CD45RA+ | CCR7+ CD45RA- | CCR7-CD45RA- |
|---|---|---|---|---|---|---|
| GEN 3-Day 16-Harvest | 42.4 | 95.2 | 4.96 | 0.7 | 1.22 | 93.1 |
| GEN 2-D22-Harvest | 50.4 | 98 | 0.61 | 0.13 | 0.3 | 99 |

| CD3 CD8 | CD3/CD8 CD38 | HLA-DR | CCR7-CD45RA+ | CCR7+CD45RA+ | CCR7+CD45RA- | CCR7-CD45RA- |
|---|---|---|---|---|---|---|
| 77.6 | 83.2 | 90.3 | 20.5 | 0.013 | 0.06 | 79.5 |
| 54.3 | 88 | 83.2 | 4.24 | 0.026 | 1.05 | 94.7 |

*Figure 106*

| | | | | | | | | | | | | | | |
|---|---|---|---|---|---|---|---|---|---|---|---|---|---|---|
| | | | **CD4** | | | | | | | | | | | |
| | CD3+ | CD4+ | 2B4+ | BTLA+ | CD103+ | CD25+ | CD69+ | CD95+ | CXCR3+ | KLRG1+ | LAG3+ | PD1+ | TIGIT+ | TIM3+ |
| Gen 2-L4054 | | | 0.61 | 99.50 | 1.05 | 14.80 | 43.20 | 99.40 | 52.90 | 2.65 | 5.36 | 81.20 | 88.40 | 78.60 |
| Gen 3-L4054 | | | 1.11 | 99.50 | 2.28 | 21.70 | 45.40 | 99.70 | 56.90 | 8.06 | 5.07 | 76.70 | 74.10 | 79.30 |
| Gen 2-L4055 | | | 1.61 | 98.60 | 1.56 | 23.10 | 51.10 | 99.80 | 63.30 | 3.86 | 15.30 | 77.60 | 86.00 | 79.50 |
| Gen 3- L4055 | | | 3.57 | 99.30 | 2.61 | 54.40 | 57.00 | 99.90 | 48.00 | 8.02 | 10.80 | 61.40 | 47.50 | 84.60 |
| | | | | | | | | | | | | | | |
| | | | | | | | | | | | | | | |
| | | | **CD8** | | | | | | | | | | | |
| Gen 2-L4054 | | CD8+ | 2B4+ | BTLA4+ | CD103+ | CD25+ | CD95+ | CD69+ | CXCR3+ | KLRG1+ | LAG3+ | PD1+ | TIGIT+ | TIM3+ |
| Gen 3-L4054 | | | 35.20 | 99.50 | 74.90 | 11.80 | 99.80 | 58.30 | 99.10 | 8.03 | 33.10 | 80.60 | 99.10 | 88.30 |
| Gen 2-L4055 | | | 45.50 | 99.60 | 62.40 | 12.10 | 99.90 | 54.00 | 99.30 | 13.40 | 22.10 | 64.90 | 98.50 | 87.50 |
| Gen 3- L4055 | | | 40.60 | 98.20 | 19.50 | 32.00 | 99.70 | 75.50 | 96.80 | 22.80 | 27.40 | 28.10 | 92.30 | 75.10 |
| | | | 62.80 | 99.20 | 29.20 | 45.50 | 99.70 | 58.80 | 99.60 | 13.10 | 16.60 | 34.10 | 71.70 | 63.60 |

*Figure 107*

| | 1 | 2 | 3 | 4 | 5 | 6 | 7 | 8 | 9 | 10 | 11 | 12 | |
|---|---|---|---|---|---|---|---|---|---|---|---|---|---|
| A | STD1 | STD1 | STD1 | TIL- Gen 2- stimulated | TIL- Gen 2- stimulated | TIL- Gen 2- stimulated | TIL- Gen 2- Unstimulated | TIL- Gen 2- Unstimulated | TIL- Gen 2- Unstimulated | MC- Stimulated xG2 | MC- Stimulated xG2 | MC- Stimulated xG2 | Well ID |
| | 1000 | 1000 | 1000 | 3 | 3 | 3 | 3 | 3 | 3 | 3 | 3 | 3 | Conc/Dil |
| B | STD2 | STD2 | STD2 | TIL- Gen 2- stimulated | TIL- Gen 2- stimulated | TIL- Gen 2- stimulated | TIL- Gen 2- Unstimulated | TIL- Gen 2- Unstimulated | TIL- Gen 2- Unstimulated | MC- Stimulated xG2 | MC- Stimulated xG2 | MC- Stimulated xG2 | Well ID |
| | 500 | 500 | 500 | 9 | 9 | 9 | 9 | 9 | 9 | 9 | 9 | 9 | Conc/Dil |
| C | STD3 | STD3 | STD3 | TIL- Gen 2- stimulated | TIL- Gen 2- stimulated | TIL- Gen 2- stimulated | TIL- Gen 2- Unstimulated | TIL- Gen 2- Unstimulated | TIL- Gen 2- Unstimulated | MC- Unstimulated xG2 | MC- Unstimulated xG2 | MC- Unstimulated xG2 | Well ID |
| | 250 | 250 | 250 | 27 | 27 | 27 | 27 | 27 | 27 | 3 | 3 | 3 | Conc/Dil |
| D | STD4 | STD4 | STD4 | TIL- Gen 2- stimulated | TIL- Gen 2- stimulated | TIL- Gen 2- stimulated | TIL- Gen 2- Unstimulated | TIL- Gen 2- Unstimulated | TIL- Gen 2- Unstimulated | MC- Unstimulated xG2 | MC- Unstimulated xG2 | MC- Unstimulated xG2 | Well ID |
| | 125 | 125 | 125 | 81 | 81 | 81 | 81 | 81 | 81 | 9 | 9 | 9 | Conc/Dil |
| E | STD5 | STD5 | STD5 | TIL- Gen 3- stimulated | TIL- Gen 3- stimulated | TIL- Gen 3- stimulated | TIL- Gen 3- Unstimulated | TIL- Gen 3- Unstimulated | TIL- Gen 3- Unstimulated | MC- Stimulated xG3 | MC- Stimulated xG3 | MC- Stimulated xG3 | Well ID |
| | 62.5 | 62.5 | 62.5 | 3 | 3 | 3 | 3 | 3 | 3 | 3 | 3 | 3 | Conc/Dil |
| F | STD6 | STD6 | STD6 | TIL- Gen 3- stimulated | TIL- Gen 3- stimulated | TIL- Gen 3- stimulated | TIL- Gen 3- Unstimulated | TIL- Gen 3- Unstimulated | TIL- Gen 3- Unstimulated | MC- Stimulated xG3 | MC- Stimulated xG3 | MC- Stimulated xG3 | Well ID |
| | 31.3 | 31.3 | 31.3 | 9 | 9 | 9 | 9 | 9 | 9 | 9 | 9 | 9 | Conc/Dil |
| G | STD7 | STD7 | STD7 | TIL- Gen 3- stimulated | TIL- Gen 3- stimulated | TIL- Gen 3- stimulated | TIL- Gen 3- Unstimulated | TIL- Gen 3- Unstimulated | TIL- Gen 3- Unstimulated | MC- Unstimulated xG3 | MC- Unstimulated xG3 | MC- Unstimulated xG3 | Well ID |
| | 15.6 | 15.6 | 15.6 | 27 | 27 | 27 | 27 | 27 | 27 | 3 | 3 | 3 | Conc/Dil |
| H | STD8 | STD8 | STD8 | TIL- Gen 3- stimulated | TIL- Gen 3- stimulated | TIL- Gen 3- stimulated | TIL- Gen 3- Unstimulated | TIL- Gen 3- Unstimulated | TIL- Gen 3- Unstimulated | MC- Unstimulated xG3 | MC- Unstimulated xG3 | MC- Unstimulated xG3 | Well ID |
| | 0 | 0 | 0 | 81 | 81 | 81 | 81 | 81 | 81 | 9 | 9 | 9 | Conc/Dil |

22.6
22.6

| | 1 | 2 | 3 | 4 | 5 | 6 | 7 | 8 | 9 | 10 | 11 | 12 | |
|---|---|---|---|---|---|---|---|---|---|---|---|---|---|
| A | 2.866 | 2.882 | 2.886 | 2.797 | 2.164 | 2.618 | 0.117 | 0.116 | 0.117 | 0.105 | 0.108 | 0.102 | 450 |
| | 0.05 | 0.05 | 0.05 | 0.05 | 0.048 | 0.051 | 0.041 | 0.043 | 0.041 | 0.041 | 0.043 | 0.041 | 570 |
| | 2.816 | 2.832 | 2.836 | 2.746 | 2.117 | 2.567 | 0.076 | 0.074 | 0.076 | 0.064 | 0.065 | 0.06 | Delta |
| | 994.773 | 1002.343 | 1004.079 | 963.405 | 694.982 | 1018.375 | 1.825 | 1.421 | 1.952 | <0.000 | <0.000 | <0.000 | [Concentration] |
| | | | | 2890.216 | 2084.946 | 3055.126 | 5.474 | 4.262 | 5.855 | <0.000 | <0.000 | <0.000 | x dil factor |
| B | 1.659 | 1.654 | 1.65 | 1.181 | 0.9 | 1.215 | 0.107 | 0.107 | 0.108 | 0.106 | 0.105 | 0.102 | 450 |
| | 0.046 | 0.046 | 0.046 | 0.045 | 0.047 | 0.045 | 0.042 | 0.041 | 0.042 | 0.043 | 0.042 | 0.042 | 570 |
| | 1.613 | 1.608 | 1.604 | 1.136 | 0.853 | 1.17 | 0.066 | 0.066 | 0.067 | 0.062 | 0.063 | 0.061 | Delta |
| | 499.866 | 497.826 | 496.528 | 329.765 | 235.273 | 341.438 | <0.000 | <0.000 | <0.000 | <0.000 | <0.000 | <0.000 | [Concentration] |
| | | | | 2967.887 | 2117.46 | 3072.942 | <0.000 | <0.000 | <0.000 | <0.000 | <0.000 | <0.000 | x dil factor |
| C | 0.952 | 0.966 | 0.94 | 0.494 | 0.391 | 0.505 | 0.106 | 0.107 | 0.107 | 0.107 | 0.105 | 0.103 | 450 |
| | 0.044 | 0.048 | 0.043 | 0.042 | 0.042 | 0.043 | 0.041 | 0.042 | 0.042 | 0.043 | 0.042 | 0.042 | 570 |
| | 0.908 | 0.919 | 0.896 | 0.452 | 0.349 | 0.462 | 0.065 | 0.065 | 0.065 | 0.063 | 0.063 | 0.062 | Delta |
| | 253.244 | 256.8 | 249.399 | 109.334 | 78.83 | 112.411 | <0.000 | <0.000 | <0.000 | <0.000 | <0.000 | <0.000 | [Concentration] |
| | | | | 2952.028 | 2128.412 | 3035.11 | <0.000 | <0.000 | <0.000 | <0.000 | <0.000 | <0.000 | x dil factor |
| D | 0.549 | 0.541 | 0.542 | 0.258 | 0.218 | 0.259 | 0.102 | 0.101 | 0.103 | 0.111 | 0.102 | 0.101 | 450 |
| | 0.044 | 0.044 | 0.043 | 0.042 | 0.041 | 0.047 | 0.041 | 0.042 | 0.042 | 0.05 | 0.042 | 0.043 | 570 |
| | 0.505 | 0.498 | 0.499 | 0.216 | 0.176 | 0.212 | 0.06 | 0.059 | 0.061 | 0.061 | 0.06 | 0.057 | Delta |
| | 125.458 | 123.174 | 123.508 | 40.262 | 29.15 | 39.101 | <0.000 | <0.000 | <0.000 | <0.000 | <0.000 | <0.000 | [Concentration] |
| | | | | 3261.251 | 2361.138 | 3167.212 | <0.000 | <0.000 | <0.000 | <0.000 | <0.000 | <0.000 | x dil factor |
| E | 0.334 | 0.332 | 0.332 | OVRFLW | OVRFLW | OVRFLW | 0.114 | 0.11 | 0.113 | 0.103 | 0.102 | 0.104 | 450 |
| | 0.043 | 0.042 | 0.042 | 0.07 | 0.068 | 0.069 | 0.041 | 0.041 | 0.042 | 0.042 | 0.042 | 0.043 | 570 |
| | 0.291 | 0.29 | 0.29 | ????? | ????? | ????? | 0.073 | 0.069 | 0.072 | 0.062 | 0.061 | 0.061 | Delta |
| | 61.826 | 61.537 | 61.594 | ????? | ????? | ????? | 1.145 | 0.285 | 0.822 | <0.000 | <0.000 | <0.000 | [Concentration] |
| | | | | ????? | ????? | ????? | 3.435 | 0.854 | 2.465 | <0.000 | <0.000 | <0.000 | x dil factor |
| F | 0.222 | 0.222 | 0.224 | 3.84 | [illegible] | 3.853 | 0.114 | 0.104 | 0.107 | 0.101 | 0.103 | 0.101 | 450 |
| | 0.042 | 0.043 | 0.042 | 0.058 | 0.056 | 0.057 | 0.041 | 0.041 | 0.042 | 0.042 | 0.041 | 0.042 | 570 |
| | 0.18 | 0.179 | 0.181 | 3.782 | [illegible] | 3.796 | 0.073 | 0.063 | 0.066 | 0.059 | 0.062 | 0.059 | Delta |
| | 30.212 | 29.82 | 30.519 | 1474.838 | >1500.000 | 1482.263 | 1.145 | <0.000 | <0.000 | <0.000 | <0.000 | <0.000 | [Concentration] |
| | | | | 13273.55 | >13500.000 | 13340.37 | 10.304 | <0.000 | <0.000 | <0.000 | <0.000 | <0.000 | x dil factor |
| G | 0.174 | 0.17 | 0.172 | 1.996 | 1.997 | 2.017 | 0.114 | 0.109 | 0.114 | 0.106 | 0.107 | 0.104 | 450 |
| | 0.041 | 0.041 | 0.041 | 0.048 | 0.047 | 0.049 | 0.042 | 0.042 | 0.041 | 0.042 | 0.041 | 0.042 | 570 |
| | 0.132 | 0.128 | 0.131 | 1.948 | 1.949 | 1.968 | 0.073 | 0.067 | 0.073 | 0.063 | 0.065 | 0.062 | Delta |
| | 16.911 | 15.874 | 16.447 | 627.703 | 628.253 | 635.61 | 1.12 | <0.000 | 1.17 | <0.000 | <0.000 | <0.000 | [Concentration] |
| | | | | 16947.99 | 16962.84 | 17161.47 | 30.239 | <0.000 | 31.588 | <0.000 | <0.000 | <0.000 | x dil factor |
| H | 0.117 | 0.111 | 0.114 | 0.84 | 0.867 | 0.857 | 0.11 | 0.11 | 0.115 | 0.109 | 0.11 | 0.113 | 450 |
| | 0.045 | 0.043 | 0.044 | 0.044 | 0.044 | 0.044 | 0.041 | 0.042 | 0.043 | 0.042 | 0.043 | 0.042 | 570 |
| | 0.072 | 0.068 | 0.07 | 0.797 | 0.822 | 0.813 | 0.069 | 0.068 | 0.073 | 0.067 | 0.067 | 0.071 | Delta |
| | 0.822 | 0.073 | 0.405 | 216.931 | 225.269 | 222.13 | 0.119 | 0.05 | 1.145 | <0.000 | <0.000 | 0.748 | [Concentration] |
| | | | | 17571.43 | 18246.77 | 17992.49 | 9.644 | 4.039 | 92.74 | <0.000 | <0.000 | 60.557 | x dil factor |

| | | | | Average | Average |
|---|---|---|---|---|---|
| Gen 2-Stimulated+TIL ' :3 | 2890.216 | 2084.946 | 3055.126 | 2676.76267 | 2757.81 |
| Gen 2-Stimulated+TIL ' :9 | 2967.887 | 2117.46 | 3072.942 | 2719.42967 | |
| Gen 2-Stimulated+TIL ' :27 | 2952.028 | 2128.412 | 3035.11 | 2705.18333 | |
| Gen 2-Stimulated+TIL ' :81 | 3261.251 | 2361.138 | 3167.212 | 2929.867 | |
| Gen 3-Stimulated+TIL ' :3 | ????? | ????? | ????? | #DIV/0! | #DIV/0! |
| Gen 3-Stimulated+TIL ' :9 | 13273.546 | >13500.0( | 13340.37 | 13306.9575 | 16089.32 |
| Gen 3-Stimulated+TIL ' :27 | 16947.994 | 16962.84 | 17161.47 | 17024.1 | |
| Gen 3-Stimulated+TIL ' :81 | 17571.427 | 18246.77 | 17992.49 | 17936.8973 | |
| Gen 2-Unstimulated 1:3 | 5.474 | 4.262 | 5.855 | 5.197 | 5.20 |
| Gen 2-Unstimulated 1:9 | <0.000 | <0.000 | <0.000 | #DIV/0! | |
| Gen 2-Unstimulated 1:27 | <0.000 | <0.000 | <0.000 | #DIV/0! | |
| Gen 2-Unstimulated 1:81 | <0.000 | <0.000 | <0.000 | #DIV/0! | |
| Gen 3-Unstimulated 1:3 | 3.435 | 0.854 | 2.465 | 2.25133333 | 19.74 |
| Gen 3-Unstimulated 1:9 | 10.304 | <0.000 | <0.000 | 10.304 | |
| Gen 3-Unstimulated 1:27 | 30.239 | <0.000 | 31.588 | 30.9135 | |
| Gen 3-Unstimulated 1:81 | 9.644 | 4.039 | 92.74 | 35.4743333 | |
| Gen 2-Media Control Stimulated 1:3 | <0.000 | <0.000 | <0.000 | #DIV/0! | 0.00 |
| Gen 2-Media Control Stimulated 1:9 | <0.000 | <0.000 | <0.000 | #DIV/0! | |
| Gen 3-Media Control Stimulated 1:3 | <0.000 | <0.000 | <0.000 | #DIV/0! | 0.00 |
| Gen 3-Media Control Stimulated 1:9 | <0.000 | <0.000 | <0.000 | #DIV/0! | |
| Gen 2-Media Control Unstimulated 1:3 | <0.000 | <0.000 | <0.000 | #DIV/0! | 0.00 |
| Gen 2-Media Control Unstimulated 1:9 | <0.000 | <0.000 | <0.000 | #DIV/0! | |
| Gen 3-Media Control Unstimulated 1:3 | <0.000 | <0.000 | <0.000 | 0 | 30.28 |
| Gen 3-Media Control Unstimulated 1:9 | <0.000 | <0.000 | 60.557 | 60.557 | |

# Figure 108

| | 1 | 2 | 3 | 4 | 5 | 6 | 7 | 8 | 9 | 10 | 11 | 12 | |
|---|---|---|---|---|---|---|---|---|---|---|---|---|---|
| A | STD1 | STD1 | STD1 | TIL- Gen 2-stimulated | TIL- Gen 2-stimulated | TIL- Gen 2-stimulated | TIL- Gen 2-Unstimulated | TIL- Gen 2-Unstimulated | TIL- Gen 2-Unstimulated | MC-Stimulated xG2 | MC-Stimulated xG2 | MC-Stimulated xG2 | Well ID |
| | 1000 | 1000 | 1000 | 3 | 3 | 3 | 3 | 3 | 3 | 3 | 3 | 3 | Conc/Dil |
| B | STD2 | STD2 | STD2 | TIL- Gen 2-stimulated | TIL- Gen 2-stimulated | TIL- Gen 2-stimulated | TIL- Gen 2-Unstimulated | TIL- Gen 2-Unstimulated | TIL- Gen 2-Unstimulated | MC-Stimulated xG2 | MC-Stimulated xG2 | MC-Stimulated xG2 | Well ID |
| | 500 | 500 | 500 | 9 | 9 | 9 | 9 | 9 | 9 | 9 | 9 | 9 | Conc/Dil |
| C | STD3 | STD3 | STD3 | TIL- Gen 2-stimulated | TIL- Gen 2-stimulated | TIL- Gen 2-stimulated | TIL- Gen 2-Unstimulated | TIL- Gen 2-Unstimulated | TIL- Gen 2-Unstimulated | MC-Unstimulated xG2 | MC-Unstimulated xG2 | MC-Unstimulated xG2 | Well ID |
| | 250 | 250 | 250 | 27 | 27 | 27 | 27 | 27 | 27 | 3 | 3 | 3 | Conc/Dil |
| D | STD4 | STD4 | STD4 | TIL- Gen 2-stimulated | TIL- Gen 2-stimulated | TIL- Gen 2-stimulated | TIL- Gen 2-Unstimulated | TIL- Gen 2-Unstimulated | TIL- Gen 2-Unstimulated | MC-Unstimulated xG2 | MC-Unstimulated xG2 | MC-Unstimulated xG2 | Well ID |
| | 125 | 125 | 125 | 81 | 81 | 81 | 81 | 81 | 81 | 9 | 9 | 9 | Conc/Dil |
| E | STD5 | STD5 | STD5 | TIL- Gen 3-stimulated | TIL- Gen 3-stimulated | TIL- Gen 3-stimulated | TIL- Gen 3-Unstimulated | TIL- Gen 3-Unstimulated | TIL- Gen 3-Unstimulated | MC-Stimulated xG3 | MC-Stimulated xG3 | MC-Stimulated xG3 | Well ID |
| | 62.5 | 62.5 | 62.5 | 3 | 3 | 3 | 3 | 3 | 3 | 3 | 3 | 3 | Conc/Dil |
| F | STD6 | STD6 | STD6 | TIL- Gen 3-stimulated | TIL- Gen 3-stimulated | TIL- Gen 3-stimulated | TIL- Gen 3-Unstimulated | TIL- Gen 3-Unstimulated | TIL- Gen 3-Unstimulated | MC-Stimulated xG3 | MC-Stimulated xG3 | MC-Stimulated xG3 | Well ID |
| | 31.3 | 31.3 | 31.3 | 9 | 9 | 9 | 9 | 9 | 9 | 9 | 9 | 9 | Conc/Dil |
| G | STD7 | STD7 | STD7 | TIL- Gen 3-stimulated | TIL- Gen 3-stimulated | TIL- Gen 3-stimulated | TIL- Gen 3-Unstimulated | TIL- Gen 3-Unstimulated | TIL- Gen 3-Unstimulated | MC-Unstimulated xG3 | MC-Unstimulated xG3 | MC-Unstimulated xG3 | Well ID |
| | 15.6 | 15.6 | 15.6 | 27 | 27 | 27 | 27 | 27 | 27 | 3 | 3 | 3 | Conc/Dil |
| H | STD8 | STD8 | STD8 | TIL- Gen 3-stimulated | TIL- Gen 3-stimulated | TIL- Gen 3-stimulated | TIL- Gen 3-Unstimulated | TIL- Gen 3-Unstimulated | TIL- Gen 3-Unstimulated | MC-Unstimulated xG3 | MC-Unstimulated xG3 | MC-Unstimulated xG3 | Well ID |
| | 0 | 0 | 0 | 81 | 81 | 81 | 81 | 81 | 81 | 9 | 9 | 9 | Conc/Dil |

22.9
22.9

| | 1 | 2 | 3 | 4 | 5 | 6 | 7 | 8 | 9 | 10 | 11 | 12 | |
|---|---|---|---|---|---|---|---|---|---|---|---|---|---|
| A | 2.875 | 2.759 | 2.877 | [illegible] | 2.823 | 2.8 | 0.128 | 0.126 | 0.132 | 0.11 | 0.111 | 0.107 | 450 |
| | 0.054 | 0.053 | 0.052 | 0.055 | 0.055 | 0.052 | 0.041 | 0.041 | 0.042 | 0.042 | 0.041 | 0.042 | 570 |
| | 2.821 | 2.807 | 2.825 | 3.129 | 2.768 | 2.748 | 0.087 | 0.085 | 0.09 | 0.069 | 0.069 | 0.065 | Delta |
| | 1001.719 | 995.223 | 1003.493 | 1145.286 | 977.713 | 968.66 | 2.922 | 2.418 | 3.712 | <0.000 | <0.000 | <0.000 | [Concentration] |
| | | | | 3435.858 | 2933.14 | 2905.979 | 8.766 | 7.254 | 11.136 | <0.000 | <0.000 | <0.000 | x dil factor |
| B | 1.669 | 1.635 | 1.668 | 1.411 | 1.199 | 1.177 | 0.119 | 0.118 | 0.126 | 0.112 | 0.112 | 0.114 | 450 |
| | 0.047 | 0.046 | 0.047 | 0.046 | 0.045 | 0.047 | 0.041 | 0.042 | 0.044 | 0.042 | 0.041 | 0.042 | 570 |
| | 1.622 | 1.589 | 1.621 | 1.365 | 1.154 | 1.13 | 0.078 | 0.076 | 0.082 | 0.07 | 0.07 | 0.072 | Delta |
| | 503.563 | 491.203 | 503.336 | 408.474 | 333.492 | 325.164 | 0.931 | 0.531 | 1.786 | <0.000 | <0.000 | <0.000 | [Concentration] |
| | | | | 3676.262 | 3001.429 | 2926.474 | 8.383 | 4.779 | 16.076 | <0.000 | <0.000 | <0.000 | x dil factor |
| C | 0.974 | 0.957 | 0.939 | 0.576 | 0.528 | 0.518 | 0.115 | 0.116 | 0.125 | 0.111 | 0.114 | 0.115 | 450 |
| | 0.045 | 0.043 | 0.045 | 0.045 | 0.043 | 0.045 | 0.041 | 0.044 | 0.042 | 0.041 | 0.043 | 0.043 | 570 |
| | 0.929 | 0.914 | 0.894 | 0.531 | 0.486 | 0.473 | 0.074 | 0.072 | 0.083 | 0.07 | 0.071 | 0.071 | Delta |
| | 256.406 | 251.388 | 244.654 | 128.566 | 114.706 | 110.92 | 0.209 | <0.000 | 1.988 | <0.000 | <0.000 | <0.000 | [Concentration] |
| | | | | 3471.282 | 3097.07 | 2994.844 | 5.632 | <0.000 | 53.672 | <0.000 | <0.000 | <0.000 | x dil factor |
| D | 0.577 | 0.551 | 0.56 | 0.301 | 0.286 | 0.27 | 0.115 | 0.11 | 0.119 | 0.111 | 0.112 | 0.11 | 450 |
| | 0.044 | 0.042 | 0.044 | 0.042 | 0.043 | 0.041 | 0.042 | 0.043 | 0.042 | 0.042 | 0.041 | 0.042 | 570 |
| | 0.533 | 0.509 | 0.516 | 0.259 | 0.243 | 0.229 | 0.073 | 0.067 | 0.077 | 0.069 | 0.07 | 0.068 | Delta |
| | 129.057 | 121.737 | 123.877 | 48.276 | 43.863 | 39.927 | <0.000 | <0.000 | 0.74 | <0.000 | <0.000 | <0.000 | [Concentration] |
| | | | | 3910.387 | 3552.941 | 3234.126 | <0.000 | <0.000 | 59.939 | <0.000 | <0.000 | <0.000 | x dil factor |
| E | 0.361 | 0.35 | 0.346 | OVRFLW | OVRFLW | OVRFLW | 0.143 | 0.135 | 0.145 | 0.115 | 0.112 | 0.11 | 450 |
| | 0.042 | 0.042 | 0.042 | [illegible] | [illegible] | [illegible] | 0.041 | 0.042 | 0.042 | 0.041 | 0.043 | 0.041 | 570 |
| | 0.319 | 0.309 | 0.304 | ????? | ????? | ????? | 0.102 | 0.093 | 0.103 | 0.074 | 0.069 | 0.068 | Delta |
| | 65.323 | 62.395 | 61.049 | ????? | ????? | ????? | 6.579 | 4.418 | 6.871 | 0.076 | <0.000 | <0.000 | [Concentration] |
| | | | | ????? | ????? | ????? | 19.738 | 13.255 | 20.614 | 0.228 | <0.000 | <0.000 | x dil factor |
| F | 0.237 | 0.233 | 0.239 | [illegible] | [illegible] | [illegible] | 0.122 | 0.144 | 0.125 | 0.114 | 0.112 | 0.111 | 450 |
| | 0.042 | 0.043 | 0.042 | 0.067 | 0.067 | 0.055 | 0.041 | [illegible] | 0.042 | 0.041 | 0.041 | 0.042 | 570 |
| | 0.195 | 0.19 | 0.197 | [illegible] | [illegible] | [illegible] | 0.081 | 0.089 | 0.084 | 0.073 | 0.071 | 0.069 | Delta |
| | 30.739 | 29.388 | 31.253 | 1464.87 | 1491.325 | 1424.683 | 1.542 | 3.455 | 2.191 | <0.000 | <0.000 | <0.000 | [Concentration] |
| | | | | 13183.83 | 13421.93 | 12822.14 | 13.875 | 31.096 | 19.718 | <0.000 | <0.000 | <0.000 | x dil factor |
| G | 0.182 | 0.173 | 0.183 | 1.922 | 1.967 | 1.765 | 0.115 | 0.115 | 0.121 | 0.114 | 0.111 | 0.114 | 450 |
| | 0.044 | 0.041 | 0.042 | 0.047 | 0.048 | 0.047 | 0.042 | 0.041 | 0.042 | 0.041 | 0.041 | 0.043 | 570 |
| | 0.138 | 0.132 | 0.141 | 1.875 | 1.919 | 1.718 | 0.073 | 0.073 | 0.079 | 0.073 | 0.07 | 0.07 | Delta |
| | 15.598 | 14.006 | 16.321 | 601.156 | 618.525 | 540.232 | 0.006 | <0.000 | 1.234 | <0.000 | <0.000 | <0.000 | [Concentration] |
| | | | | 16231.22 | 16700.18 | 14586.27 | 0.172 | <0.000 | 33.321 | <0.000 | <0.000 | <0.000 | x dil factor |
| H | 0.121 | 0.117 | 0.122 | 0.837 | 0.857 | 0.794 | 0.119 | 0.125 | 0.123 | 0.119 | 0.12 | 0.112 | 450 |
| | 0.045 | 0.042 | 0.047 | 0.043 | 0.045 | 0.043 | 0.041 | 0.041 | 0.043 | 0.045 | 0.044 | 0.042 | 570 |
| | 0.076 | 0.075 | 0.074 | 0.793 | 0.812 | 0.75 | 0.078 | 0.083 | 0.079 | 0.074 | 0.076 | 0.071 | Delta |
| | 0.635 | 0.408 | 0.17 | 211.613 | 217.755 | 197.622 | 0.953 | 2.123 | 1.256 | 0.151 | 0.531 | <0.000 | [Concentration] |
| | | | | 17140.68 | 17638.2 | 16007.4 | 77.18 | 171.968 | 101.73 | 1.357 | 4.779 | <0.000 | x dil factor |

| | | | | Average | Average |
|---|---|---|---|---|---|
| Gen 2-Stimulated+TIL 1:3 | 3435.858 | 2933.14 | 2905.979 | 3091.659 | 3261.65 |
| Gen 2-Stimulated+TIL 1:9 | 3676.262 | 3001.429 | 2926.474 | 3201.388333 | |
| Gen 2-Stimulated+TIL 1:27 | 3471.282 | 3097.07 | 2994.844 | 3187.732 | |
| Gen 2-Stimulated+TIL 1:81 | 3910.387 | 3552.941 | 3234.126 | 3565.818 | |
| Gen 3-Stimulated+TIL 1:3 | ????? | ????? | ????? | #DIV/0! | #DIV/0! |
| Gen 3-Stimulated+TIL 1:9 | 13183.826 | 13421.925 | 12822.143 | 13142.63133 | 15303.54 |
| Gen 3-Stimulated+TIL 1:27 | 16231.22 | 16700.175 | 14586.268 | 15839.221 | |
| Gen 3-Stimulated+TIL 1:81 | 17140.679 | 17638.195 | 16007.402 | 16928.75867 | |
| Gen 2-Unstimulated 1:3 | 8.766 | 7.254 | 11.136 | 9.052 | 27.10 |
| Gen 2-Unstimulated 1:9 | 8.383 | 4.779 | 16.076 | 9.746 | |
| Gen 2-Unstimulated 1:27 | 5.632 | <0.000 | 53.672 | 29.652 | |
| Gen 2-Unstimulated 1:81 | <0.000 | <0.000 | 59.939 | 59.939 | |
| Gen 3-Unstimulated 1:3 | 19.738 | 13.255 | 20.614 | 17.869 | 43.28 |
| Gen 3-Unstimulated 1:9 | 13.875 | 31.096 | 19.718 | 21.563 | |
| Gen 3-Unstimulated 1:27 | 0.172 | <0.000 | 33.321 | 16.7465 | |
| Gen 3-Unstimulated 1:81 | 77.18 | 171.968 | 101.73 | 116.9593333 | |
| Gen 2-Media Control Stimulated 1:3 | <0.000 | <0.000 | <0.000 | #DIV/0! | 0.00 |
| Gen 2-Media Control Stimulated 1:9 | <0.000 | <0.000 | <0.000 | #DIV/0! | |
| Gen 3-Media Control Stimulated 1:3 | 0.228 | <0.000 | <0.000 | 0.228 | 0.11 |
| Gen 3-Media Control Stimulated 1:9 | <0.000 | <0.000 | <0.000 | 0 | |
| Gen 2-Media Control Unstimulated 1:3 | <0.000 | <0.000 | <0.000 | 0 | 0.00 |
| Gen 2-Media Control Unstimulated 1:9 | <0.000 | <0.000 | <0.000 | 0 | |
| Gen 3-Media Control Unstimulated 1:3 | <0.000 | <0.000 | <0.000 | 0 | 1.53 |
| Gen 3-Media Control Unstimulated 1:9 | 1.357 | 4.779 | <0.000 | 3.068 | |

*Figure 109*

| | 1 | 2 | 3 | 4 | 5 | 6 | 7 | 8 | 9 | 10 | 11 | 12 | |
|---|---|---|---|---|---|---|---|---|---|---|---|---|---|
| A | STD1 | STD1 | STD1 | 2A-BEAD+TIL | 2A-BEAD+TIL | 2A-BEAD+TIL | 2A-MC | 2A-MC | 2A-MC | 2A-Unstimulated | 2A-Unstimulated | 2A-Unstimulated | Well ID |
| | 1000 | 1000 | 1000 | 3 | 3 | 3 | 3 | 3 | 3 | 3 | 3 | 3 | Conc/Dil |
| B | STD2 | STD2 | STD2 | 2A-BEAD+TIL | 2A-BEAD+TIL | 2A-BEAD+TIL | 2A-MC | 2A-MC | 2A-MC | 2A-Unstimulated | 2A-Unstimulated | 2A-Unstimulated | Well ID |
| | 500 | 500 | 500 | 9 | 9 | 9 | 9 | 9 | 9 | 9 | 9 | 9 | Conc/Dil |
| C | STD3 | STD3 | STD3 | 2A-BEAD+TIL | 2A-BEAD+TIL | 2A-BEAD+TIL | 2A-MC | 2A-MC | 2A-MC | 2A-Unstimulated | 2A-Unstimulated | 2A-Unstimulated | Well ID |
| | 250 | 250 | 250 | 27 | 27 | 27 | 27 | 27 | 27 | 27 | 27 | 27 | Conc/Dil |
| D | STD4 | STD4 | STD4 | 2A-BEAD+TIL | 2A-BEAD+TIL | 2A-BEAD+TIL | 2A-MC | 2A-MC | 2A-MC | 2A-Unstimulated | 2A-Unstimulated | 2A-Unstimulated | Well ID |
| | 125 | 125 | 125 | 81 | 81 | 81 | 81 | 81 | 81 | 81 | 81 | 81 | Conc/Dil |
| E | STD5 | STD5 | STD5 | 2B-BEAD+TIL | 2B-BEAD+TIL | 2B-BEAD+TIL | 2B-Unstimulated | 2B-Unstimulated | 2B-Unstimulated | 2B-MC | 2B-MC | 2B-MC | Well ID |
| | 62.5 | 62.5 | 62.5 | 3 | 3 | 3 | 3 | 3 | 3 | 3 | 3 | 3 | Conc/Dil |
| F | STD6 | STD6 | STD6 | 2B-BEAD+TIL | 2B-BEAD+TIL | 2B-BEAD+TIL | 2B-Unstimulated | 2B-Unstimulated | 2B-Unstimulated | 2B-MC | 2B-MC | 2B-MC | Well ID |
| | 31.3 | 31.3 | 31.3 | 9 | 9 | 9 | 9 | 9 | 9 | 9 | 9 | 9 | Conc/Dil |
| G | STD7 | STD7 | STD7 | 2B-BEAD+TIL | 2B-BEAD+TIL | 2B-BEAD+TIL | 2B-Unstimulated | 2B-Unstimulated | 2B-Unstimulated | 2B-MC | 2B-MC | 2B-MC | Well ID |
| | 15.6 | 15.6 | 15.6 | 27 | 27 | 27 | 27 | 27 | 27 | 27 | 27 | 27 | Conc/Dil |
| H | STD8 | STD8 | STD8 | 2B-BEAD+TIL | 2B-BEAD+TIL | 2B-BEAD+TIL | 2B-Unstimulated | 2B-Unstimulated | 2B-Unstimulated | 2B-MC | 2B-MC | 2B-MC | Well ID |
| | 0 | 0 | 0 | 81 | 81 | 81 | 81 | 81 | 81 | 81 | 81 | 81 | Conc/Dil |

24.1
24.1

| | 1 | 2 | 3 | 4 | 5 | 6 | 7 | 8 | 9 | 10 | 11 | 12 | |
|---|---|---|---|---|---|---|---|---|---|---|---|---|---|
| A | 2.119 | 2.231 | 2.274 | [illegible] | [illegible] | [illegible] | 0.079 | 0.079 | 0.081 | 0.18 | 0.198 | 0.191 | 450 |
| | 0.048 | 0.049 | 0.049 | 0.052 | 0.052 | 0.052 | 0.042 | 0.042 | 0.043 | 0.042 | 0.043 | 0.042 | 570 |
| | 2.071 | 2.182 | 2.224 | [illegible] | [illegible] | [illegible] | 0.037 | 0.037 | 0.037 | 0.138 | 0.156 | 0.149 | Delta |
| | 951.995 | 1012.581 | 1035.454 | 1479.442 | >1500.000 | >1500.000 | 0.379 | 0.287 | 0.472 | 37.604 | 44.509 | 41.927 | [Concentration] |
| | | | | 4438.326 | >4500.000 | >4500.000 | 1.137 | 0.861 | 1.417 | 112.812 | 133.527 | 125.78 | xdil factor |
| B | 1.221 | 1.22 | 1.252 | 1.265 | 1.361 | 1.352 | 0.077 | 0.077 | 0.077 | 0.109 | 0.115 | 0.114 | 450 |
| | 0.046 | 0.046 | 0.046 | 0.046 | 0.047 | 0.047 | 0.042 | 0.044 | 0.042 | 0.042 | 0.043 | 0.042 | 570 |
| | 1.175 | 1.174 | 1.206 | 1.219 | 1.314 | 1.304 | 0.035 | 0.033 | 0.035 | 0.067 | 0.072 | 0.072 | Delta |
| | 496.244 | 495.812 | 511.027 | 517.353 | 563.806 | 558.817 | <0.000 | <0.000 | <0.000 | 10.74 | 12.637 | 12.564 | [Concentration] |
| | | | | 4656.178 | 5074.253 | 5029.349 | <0.000 | <0.000 | <0.000 | 96.66 | 113.737 | 113.077 | xdil factor |
| C | 0.677 | 0.681 | 0.681 | 0.531 | 0.563 | 0.555 | 0.075 | 0.074 | 0.075 | 0.087 | 0.096 | 0.091 | 450 |
| | 0.043 | 0.044 | 0.044 | 0.044 | 0.045 | 0.043 | 0.042 | 0.042 | 0.042 | 0.042 | 0.045 | 0.042 | 570 |
| | 0.634 | 0.637 | 0.637 | 0.488 | 0.518 | 0.511 | 0.033 | 0.032 | 0.033 | 0.045 | 0.05 | 0.049 | Delta |
| | 245.876 | 247.384 | 247.428 | 182.102 | 195.039 | 192.221 | <0.000 | <0.000 | <0.000 | 2.846 | 4.883 | 4.221 | [Concentration] |
| | | | | 4916.763 | 5266.064 | 5189.955 | <0.000 | <0.000 | <0.000 | 76.848 | 131.834 | 113.974 | xdil factor |
| D | 0.406 | 0.405 | 0.407 | 0.251 | 0.257 | 0.25 | 0.075 | 0.075 | 0.077 | 0.082 | 0.081 | 0.084 | 450 |
| | 0.044 | 0.043 | 0.044 | 0.044 | 0.043 | 0.042 | 0.042 | 0.043 | 0.043 | 0.044 | 0.042 | 0.044 | 570 |
| | 0.363 | 0.362 | 0.363 | 0.207 | 0.214 | 0.208 | 0.033 | 0.032 | 0.035 | 0.039 | 0.039 | 0.04 | Delta |
| | 128.798 | 128.379 | 128.798 | 64.814 | 67.463 | 65.095 | <0.000 | <0.000 | <0.000 | 0.885 | 1.014 | 1.438 | [Concentration] |
| | | | | 5249.956 | 5464.506 | 5272.686 | <0.000 | <0.000 | <0.000 | 71.65 | 82.102 | 116.487 | xdil factor |
| E | 0.244 | 0.241 | 0.241 | OVRFLW | OVRFLW | OVRFLW | 0.35 | 0.356 | 0.275 | 0.078 | 0.079 | 0.079 | 450 |
| | 0.042 | 0.042 | 0.042 | [illegible] | [illegible] | [illegible] | 0.043 | 0.043 | 0.043 | 0.042 | 0.042 | 0.043 | 570 |
| | 0.201 | 0.199 | 0.199 | ????? | ????? | ????? | 0.307 | 0.314 | 0.232 | 0.036 | 0.037 | 0.036 | Delta |
| | 62.572 | 61.493 | 61.533 | ????? | ????? | ????? | 105.745 | 108.313 | 74.922 | 0.079 | 0.318 | <0.000 | [Concentration] |
| | | | | ????? | ????? | ????? | 317.235 | 324.939 | 224.765 | 0.237 | 0.953 | <0.000 | xdil factor |
| F | 0.166 | 0.165 | 0.161 | [illegible] | [illegible] | [illegible] | 0.165 | 0.17 | 0.146 | 0.078 | 0.079 | 0.078 | 450 |
| | 0.042 | 0.043 | 0.043 | 0.055 | 0.053 | 0.055 | 0.043 | 0.042 | 0.045 | 0.042 | 0.042 | 0.042 | 570 |
| | 0.124 | 0.122 | 0.119 | [illegible] | [illegible] | [illegible] | 0.123 | 0.128 | 0.101 | 0.036 | 0.037 | 0.035 | Delta |
| | 31.999 | 31.384 | 30.079 | >1500.000 | 1346.731 | >1500.000 | 31.769 | 33.579 | 23.374 | 0.051 | 0.472 | <0.000 | [Concentration] |
| | | | | >13500.000 | 12120.579 | >13500.000 | 285.917 | 302.211 | 210.37 | 0.457 | 4.25 | <0.000 | xdil factor |
| G | 0.122 | 0.119 | 0.117 | 1.371 | 1.194 | 1.443 | 0.102 | 0.097 | 0.092 | 0.072 | 0.072 | 0.07 | 450 |
| | 0.043 | 0.042 | 0.043 | 0.052 | 0.048 | 0.052 | 0.043 | 0.044 | 0.045 | 0.043 | 0.045 | 0.043 | 570 |
| | 0.078 | 0.076 | 0.074 | 1.319 | 1.146 | 1.396 | 0.058 | 0.054 | 0.046 | 0.029 | 0.028 | 0.027 | Delta |
| | 14.956 | 14.181 | 13.334 | 566.107 | 482.193 | 603.959 | 7.605 | 5.971 | 3.462 | <0.000 | <0.000 | <0.000 | [Concentration] |
| | | | | 15284.891 | 13019.2 | 16306.887 | 205.324 | 161.226 | 93.466 | <0.000 | <0.000 | <0.000 | xdil factor |
| H | 0.077 | 0.084 | 0.077 | 0.572 | 0.489 | 0.583 | 0.098 | 0.082 | 0.086 | 0.074 | 0.073 | 0.07 | 450 |
| | 0.042 | 0.042 | 0.042 | 0.046 | 0.045 | 0.046 | 0.049 | 0.043 | 0.043 | 0.043 | 0.043 | 0.043 | 570 |
| | 0.036 | 0.042 | 0.036 | 0.526 | 0.443 | 0.537 | 0.05 | 0.038 | 0.043 | 0.031 | 0.03 | 0.027 | Delta |
| | <0.000 | 2.136 | 0.023 | 198.6 | 163.034 | 203.299 | 4.534 | 0.756 | 2.305 | <0.000 | <0.000 | <0.000 | [Concentration] |
| | | | | 16086.64 | 13205.761 | 16467.237 | 367.254 | 61.271 | 186.666 | <0.000 | <0.000 | <0.000 | xdil factor |

| | | | | Average | Average |
|---|---|---|---|---|---|
| 2A-beads+TIL 1:3 | | | | #DIV/0! | |
| 2A-beads+TIL 1:9 | 4656.178 | 5074.253 | 5029.349 | 4919.927 | 5124.412 |
| 2A-beads+TIL 1:27 | 4916.763 | 5266.064 | 5189.955 | 5124.261 | |
| 2A-beads+TIL 1:81 | 5249.956 | 5464.506 | 5272.686 | 5329.049 | |
| 2B-beads+TIL 1:3 | ????? | ????? | ????? | #DIV/0! | |
| 2B-beads+TIL 1:9 | | | | #DIV/0! | |
| 2B-beads+TIL 1:27 | 15284.891 | 13019.2 | 16306.887 | 14870.33 | 15061.77 |
| 2B-beads+TIL 1:81 | 16086.64 | 13205.761 | 16467.237 | 15253.21 | |
| 2A-Unstimulated 1:3 | 112.812 | 133.527 | 125.78 | 124.0397 | 107.374 |
| 2A-Unstimulated 1:9 | 96.66 | 113.737 | 113.077 | 107.8247 | |
| 2A-Unstimulated 1:27 | 76.848 | 131.834 | 113.974 | 107.552 | |
| 2A-Unstimulated 1:81 | 71.65 | 82.102 | 116.487 | 90.07967 | |
| 2B-Unstimulated 1:3 | 317.235 | 324.939 | 224.765 | 288.9797 | 236.1614 |
| 2B-Unstimulated 1:9 | 285.917 | 302.211 | 210.37 | 266.166 | |
| 2B-Unstimulated 1:27 | 205.324 | 161.226 | 93.466 | 153.3387 | |
| 2B-Unstimulated 1:81 | | | | | |
| 2A-Media Control 1:3 | 1.137 | 0.861 | 1.417 | 1.138333 | 1.138778 |
| 2A-Media Control 1:9 | | | | | |
| 2A-Media Control 1:27 | | | | | |
| 2A-Media Control 1:81 | | | | | |
| 2B-Media Control 1:3 | 0.237 | 0.953 | <0.000 | 0.595 | 1.47425 |
| 2B-Media Control 1:9 | 0.457 | 4.25 | <0.000 | 2.3535 | |
| 2B-Media Control 1:27 | | | | | |
| 2B-Media Control 1:81 | | | | | |

*Figure 110*

| | 1 | 2 | 3 | 4 | 5 | 6 | 7 | 8 | 9 | 10 | 11 | 12 | |
|---|---|---|---|---|---|---|---|---|---|---|---|---|---|
| A | STD1<br>2000 | STD1<br>2000 | STD1<br>2000 | Gen 3-D7 1ST RUN | Gen 3-D7 1ST RUN | Gen 3-D7 1ST RUN | Gen 3-D7 2ND RUN | Gen 3-D7 2ND RUN | Gen 3-D7 2ND RUN | | | | Well ID<br>Conc/Dil |
| B | STD2<br>1000 | STD2<br>1000 | STD2<br>1000 | Gen 3-D11 1ST RUN | Gen 3-D11 1ST RUN | Gen 3-D11 1ST RUN | Gen 3-D11 2ND RUN | Gen 3-D11 2ND RUN | Gen 3-D11 2ND RUN | | | | Well ID<br>Conc/Dil |
| C | STD3<br>500 | STD3<br>500 | STD3<br>500 | Gen 3-D16 1ST RUN | Gen 3-D16 1ST RUN | Gen 3-D16 1ST RUN | Gen 3-D16 2ND RUN | Gen 3-D16 2ND RUN | Gen 3-D16 2ND RUN | | | | Well ID<br>Conc/Dil |
| D | STD4<br>250 | STD4<br>250 | STD4<br>250 | Gen 2-D11 1ST RUN | Gen 2-D11 1ST RUN | Gen 2-D11 1ST RUN | Gen 2-D11 2ND RUN | Gen 2-D11 2ND RUN | Gen 2-D11 2ND RUN | | | | Well ID<br>Conc/Dil |
| E | STD5<br>125 | STD5<br>125 | STD5<br>125 | Gen 2-D16 1ST RUN | Gen 2-D16 1ST RUN | Gen 2-D16 1ST RUN | Gen 2-D16 2ND RUN | Gen 2-D16 2ND RUN | Gen 2-D16 2ND RUN | | | | Well ID<br>Conc/Dil |
| F | STD6<br>62.5 | STD6<br>62.5 | STD6<br>62.5 | Gen 2-D22 1ST RUN | Gen 2-D22 1ST RUN | Gen 2-D22 1ST RUN | Gen 2-D22 2ND RUN | Gen 2-D22 2ND RUN | Gen 2-D22 2ND RUN | | | | Well ID<br>Conc/Dil |
| G | STD7<br>31.3 | STD7<br>31.3 | STD7<br>31.3 | Empty | Empty | Empty | Empty | Empty | Empty | | | | Well ID<br>Conc/Dil |
| H | STD8<br>0 | STD8<br>0 | STD8<br>0 | Empty | Empty | Empty | Empty | Empty | Empty | | | | Well ID<br>Conc/Dil |

22
22

| | 1 | 2 | 3 | 4 | 5 | 6 | 7 | 8 | 9 | 10 | 11 | 12 | |
|---|---|---|---|---|---|---|---|---|---|---|---|---|---|
| A | 2.223 | 2.297 | [illegible] | 0.218 | 0.22 | 0.215 | 0.187 | 0.187 | 0.184 | | | | 450 |
| | 0.052 | 0.052 | 0.051 | 0.043 | 0.042 | 0.043 | 0.044 | 0.043 | 0.051 | | | | 570 |
| | 2.171 | 2.245 | [illegible] | 0.175 | 0.177 | 0.172 | 0.143 | 0.145 | 0.133 | | | | Delta |
| | 1970.541 | 2046.905 | 1987.758 | 124.53 | 126.082 | 121.662 | 99.302 | 100.904 | 92.083 | | | | [Concentration] |
| B | 1.095 | 1.281 | 1.276 | 0.415 | 0.412 | 0.435 | 0.394 | 0.392 | 0.356 | | | | 450 |
| | 0.047 | 0.046 | 0.047 | 0.043 | 0.043 | 0.043 | 0.043 | 0.044 | 0.044 | | | | 570 |
| | 1.048 | 1.234 | 1.229 | 0.372 | 0.369 | 0.393 | 0.351 | 0.348 | 0.312 | | | | Delta |
| | 877.734 | 1051.728 | 1047.027 | 282.4 | 280.328 | 299.854 | 264.954 | 262.893 | 233.453 | | | | [Concentration] |
| C | 0.683 | 0.683 | 0.68 | 0.379 | 0.372 | 0.376 | 0.356 | 0.338 | 0.33 | | | | 450 |
| | 0.045 | 0.044 | 0.044 | 0.043 | 0.043 | 0.043 | 0.043 | 0.043 | 0.043 | | | | 570 |
| | 0.638 | 0.639 | 0.637 | 0.336 | 0.33 | 0.333 | 0.313 | 0.295 | 0.287 | | | | Delta |
| | 510.008 | 510.619 | 508.261 | 252.774 | 247.768 | 250.557 | 233.943 | 219.211 | 212.888 | | | | [Concentration] |
| D | 0.37 | 0.38 | 0.382 | 0.162 | 0.156 | 0.177 | 0.155 | 0.15 | 0.149 | | | | 450 |
| | 0.043 | 0.045 | 0.043 | 0.046 | 0.042 | 0.042 | 0.043 | 0.044 | 0.043 | | | | 570 |
| | 0.328 | 0.335 | 0.338 | 0.116 | 0.114 | 0.135 | 0.112 | 0.106 | 0.106 | | | | Delta |
| | 246.046 | 252.035 | 254.91 | 79.199 | 77.702 | 93.523 | 76.431 | 71.588 | 71.737 | | | | [Concentration] |
| E | 0.216 | 0.219 | 0.22 | 0.235 | 0.227 | 0.228 | 0.253 | 0.231 | 0.222 | | | | 450 |
| | 0.043 | 0.043 | 0.045 | 0.044 | 0.043 | 0.043 | 0.048 | 0.045 | 0.045 | | | | 570 |
| | 0.173 | 0.176 | 0.175 | 0.191 | 0.185 | 0.185 | 0.205 | 0.186 | 0.177 | | | | Delta |
| | 122.514 | 124.995 | 123.909 | 136.754 | 131.526 | 131.682 | 147.807 | 132.695 | 125.539 | | | | [Concentration] |
| F | 0.137 | 0.132 | 0.133 | 0.21 | 0.227 | 0.216 | 0.238 | 0.233 | 0.253 | | | | 450 |
| | 0.047 | 0.043 | 0.044 | 0.043 | 0.044 | 0.044 | 0.043 | 0.043 | 0.045 | | | | 570 |
| | 0.089 | 0.089 | 0.089 | 0.166 | 0.183 | 0.172 | 0.195 | 0.19 | 0.207 | | | | Delta |
| | 59.482 | 58.969 | 59.116 | 117.564 | 130.125 | 121.895 | 139.491 | 135.738 | 149.459 | | | | [Concentration] |
| G | 0.088 | 0.088 | 0.089 | 2.265 | 2.329 | 2.22 | 2.225 | 2.174 | 2.239 | | | | 450 |
| | 0.042 | 0.043 | 0.043 | 0.05 | 0.05 | 0.049 | 0.051 | 0.052 | 0.053 | | | | 570 |
| | 0.046 | 0.046 | 0.046 | 2.215 | 2.28 | 2.171 | 2.174 | 2.122 | 2.186 | | | | Delta |
| | 28.375 | 28.237 | 28.514 | 2014.9 | 2081.651 | 1970.439 | 1973.799 | 1920.845 | 1986.127 | | | | [Concentration] |
| H | 0.05 | 0.048 | 0.048 | 2.273 | 2.267 | 2.213 | 2.244 | 2.254 | 2.241 | | | | 450 |
| | 0.043 | 0.042 | 0.041 | 0.05 | 0.05 | 0.052 | 0.057 | 0.052 | 0.053 | | | | 570 |
| | 0.007 | 0.006 | 0.007 | 2.223 | 2.217 | 2.161 | 2.187 | 2.202 | 2.188 | | | | Delta |
| | 3.1 | 2.399 | 2.573 | 2023.073 | 2016.943 | 1960.16 | 1986.84 | 2002.036 | 1988.165 | | | | [Concentration] |

**Dilution Factor=100**

| | Reading A | Reading B | Reading C | Reading A*100 | Reading B*100 | Reading C*100 | Average |
|---|---|---|---|---|---|---|---|
| Gen 3 Rep initiation 1st run | 124.530 | 126.082 | 121.662 | 12453.0 | 12608.2 | 12166.2 | 12409.1 |
| Gen 3 Scale up 1st run | 282.400 | 280.328 | 299.854 | 28240.0 | 28032.8 | 29985.4 | 28752.7 |
| Gen 3 Harvest 1st run | 252.774 | 247.768 | 250.557 | 25277.4 | 24776.8 | 25055.7 | 25036.6 |
| Gen 2 Rep initiation 1st run | 79.199 | 77.702 | 93.523 | 7919.9 | 7770.2 | 9352.3 | 8347.5 |
| Gen 2 Scale up 1st run | 136.754 | 131.526 | 131.682 | 13675.4 | 13152.6 | 13168.2 | 13332.1 |
| Gen 2 Harvest 1st run | 117.564 | 130.125 | 121.895 | 11756.4 | 13012.5 | 12189.5 | 12319.5 |
| Gen 3 Rep initiation 2nd run | 99.302 | 100.904 | 92.083 | 9930.2 | 10090.4 | 9208.3 | 9743.0 |
| Gen 3 Scale up 2nd run | 264.954 | 262.893 | 233.453 | 26495.4 | 26289.3 | 23345.3 | 25376.7 |
| Gen 3 Harvest 2nd run | 233.943 | 219.211 | 212.888 | 23394.3 | 21921.1 | 21288.8 | 22201.4 |
| Gen 2 Rep initiation 2nd run | 76.431 | 71.588 | 71.737 | 7643.1 | 7158.8 | 7173.7 | 7325.2 |
| Gen 2 Scale up 2nd run | 147.807 | 132.695 | 125.539 | 14780.7 | 13269.5 | 12553.9 | 13534.7 |
| Gen 2 Harvest 2nd run | 139.491 | 135.738 | 149.459 | 13949.1 | 13573.8 | 14945.9 | 14156.3 |

*Figure 111*

| Sample number # | Description | Glucose (g/L) | Lactate (g/L) | Ammonia (mmol/L) | Glutamine (mmol/L) | Glutamax (mmol/L) | Glutamax-Glutamine (mmol/L) |
|---|---|---|---|---|---|---|---|
| 1 | Gen 2-Rep initiation First Round #L4054 | 1.78 | 0.14 | 2.16 | 1.56 | 1.71 | 0.16 |
| 2 | Gen 2-Scale Up First Round #L4054 | 1.36 | 0.89 | 1.40 | 1.76 | 1.90 | 0.15 |
| 3 | Gen 2-Harvest First Round #L4054 | 0.83 | 1.68 | 2.46 | 1.69 | 1.85 | 0.17 |
| 4 | Gen 3-REP initiation First Round #L4054 | 1.68 | 0.29 | 1.76 | 2.11 | 2.29 | 0.18 |
| 5 | Gen 3-Scale Up First Round #L4054 | 1.28 | 0.94 | 1.64 | 1.70 | 1.87 | 0.16 |
| 6 | Gen 3-Harvest First Round #L4054 | 1.07 | 1.53 | 2.23 | 1.39 | 1.54 | 0.14 |
| | | | | | | | |
| 7 | Gen 2-Rep initiation Second Round #L4055 | 1.91 | 0.08 | 2.29 | 1.60 | 1.74 | 0.14 |
| 8 | Gen 2-Scale up Second Round #L4055 | 1.61 | 0.77 | 1.40 | 1.78 | 1.93 | 0.15 |
| 9 | Gen 2-Harvest Second Round #L4055 | 0.85 | 1.86 | 2.46 | 1.39 | 1.55 | 0.16 |
| 10 | Gen 3-REP initiation Second Round #L4055 | 1.77 | 0.18 | 1.74 | 2.06 | 2.23 | 0.17 |
| 11 | Gen 3-Scale up Second Round #L4055 | 1.60 | 0.72 | 1.54 | 1.68 | 1.84 | 0.15 |
| 12 | Gen 3-Harvest Second Round #L4055 | 1.44 | 1.32 | 2.07 | 1.79 | 1.95 | 0.16 |

*Figure 112*

| | $FIL | S/L/1301 \| Geometr | S/L/Pt \| Geometric Mean (FL03-A) |
|---|---|---|---|
| A1 gen2L4054 ne | A1 gen2L4054 neg.fcs | 34.5 | 3.24 |
| A2 gen2L4054 ne | A2 gen2L4054 neg.fcs | 34 | 3.19 |
| A3 gen2L4054 po | A3 gen2L4054 pos.fcs | 1834 | 70.6 |
| A4 gen2L4054 po | A4 gen2L4054 pos.fcs | 1872 | 70.3 |
| A5 gen2L4055 ne | A5 gen2L4055 neg.fcs | 31.9 | 4.23 |
| A6 gen2L4055 ne | A6 gen2L4055 neg.fcs | 32.4 | 4.87 |
| A7 gen2L4055 po | A7 gen2L4055 pos.fcs | 1941 | 88.7 |
| A8 gen2L4055 po | A8 gen2L4055 pos.fcs | 1933 | 91.1 |
| B1 gen3L4054 ne | B1 gen3L4054 neg.fcs | 31.2 | 3.45 |
| B2 gen3L4054 ne | B2 gen3L4054 neg.fcs | 31.9 | 3.39 |
| B3 gen3L4054 po | B3 gen3L4054 pos.fcs | 2016 | 61.3 |
| B4 gen3L4054 po | B4 gen3L4054 pos.fcs | 2014 | 76.2 |
| B5 gen3L4055 ne | B5 gen3L4055 neg.fcs | 33.7 | 6.76 |
| B6 gen3L4055 ne | B6 gen3L4055 neg.fcs | 35.4 | 6.7 |
| B7 gen3L4055 po | B7 gen3L4055 pos.fcs | 2158 | 99.4 |
| B8 gen3L4055 po | B8 gen3L4055 pos.fcs | 1938 | 99.5 |
| Mean | - | 998 | 43.3 |
| SD | - | 999 | 41.3 |

*Figure 113*

| Study | Sample | Sample id | Species | Chain | Reads | CDR3 | Unique CDR3 | D50 |
|---|---|---|---|---|---|---|---|---|
| 20180511_Study1_FCA | Gen2-L4054 | 59990 | TRB | h | 1181732 | 1181732 | 8915 | 0 |
| 20180511_Study1_FCA | Gen3-L4054 | 59991 | TRB | h | 1145697 | 1145697 | 18130 | 0 |
| 20180511_Study1_FCA | Gen2-L4055 | 59987 | TRB | h | 1166465 | 1166465 | 12996 | 0.1 |
| 20180511_Study1_FCA | Gen3-L4055 | 59982 | TRB | h | 1059985 | 1059985 | 27246 | 0.9 |

*Figure 114*

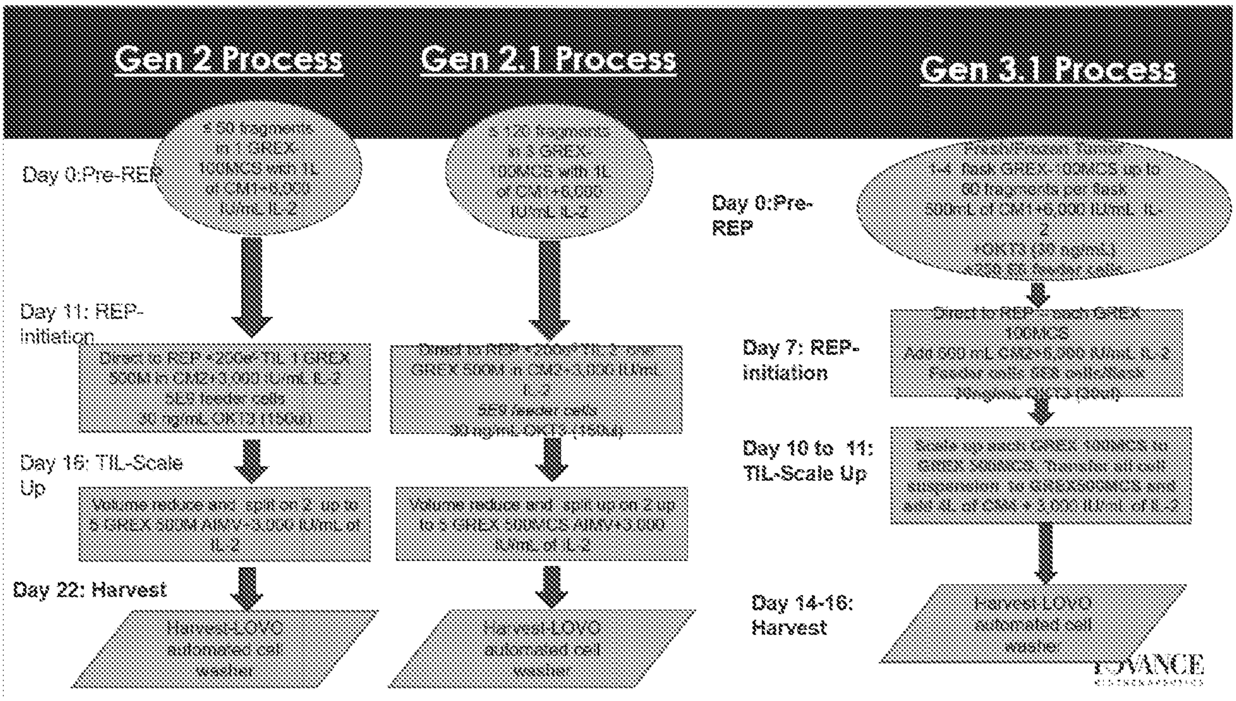
Gen 2 Process
Gen 2.1 Process
Gen 3.1 Process
Day 0:Pre-REP
Day 11: REP-initiation
Day 16: TIL-Scale Up
Day 22: Harvest
Harvest-LOVO automated cell washer
Harvest-LOVO automated cell washer
Day 0:Pre-REP
Day 7: REP-initiation
Day 10 to 11: TIL-Scale Up
Day 14-16: Harvest

*Figure 115*

| STEP | Gen 2 | Gen 2.1 | Gen 3.0 |
|---|---|---|---|
| Pre REP-<br>day 0 | ≤ 50 fragments/ 1 G-Rex<br>100MCS - **11 days** | ≤ 180 fragments/ 3 G-Rex<br>Pre-formulated CM1 warmed media<br>100MCS - **11 days** | *Fresh or Frozen Tumor*<br>Whole tumor with ≤ 30 fragments up to 60 fragments per 1 G-Rex<br>100MCS (up to 4 G-Rex),<br>preformulated warmed media - **7 days, Pre REP, Feeders 2.5 E8 cells + OKT-3 (30ng/mL)** |
| REP Initiation | Direct to REP- Day 11-<br>**<200 E6 TIL**<br>1 G-Rex 500MCS | Direct to REP- Day 11-<br>**<200 E6 TIL**<br>Pre-formulated CM2 warmed media in one G-Rex 500MCS | Direct to REP- Day **7-all cells TIL- same G-Rex 100MCS (100MCS up to 4 GREX), Standard media or Defined Media (Serum free). Addition Feeders 5 E8 cells +OKT-3 (30ng/mL)** |
| TIL propagation or Scale up | 1 to 5 G-REX 500MCS<br>**Split day 16** | 2 to 5 G-REX 500MCS<br>Pre-formulated CM4 warmed media<br>**Split day 16** | From G-REX 100MCS transfer TIL suspension to G-REX 500MCS , up to 4 GREX 500 MCS- Standard media or Defined Media (Serum Free)<br>**Scale up on day 10 or 11** |
| Harvest | **Harvest day 22,**<br>LOVO-automated cell washer | **Harvest day 22,**<br>LOVO-automated cell washer (5 wash cycle) | **Harvest day 14 or 16**<br>LOVO- automated cell washer (5 wash cycle) |
| Final formulation | Cryopreserved Product<br>300IU/ml IL2- CS10 in $LN_2$,<br>multiple aliquots | Cryopreserved Product<br>300IU/ml IL2- CS10 in $LN_2$,<br>multiple aliquots | Cryopreserved product<br>300IU/ml IL-2-CS10 in $LN_2$,<br>multiple aliquots |
| Process time | 22 days | 22 days | 16 days |

*Figure 116*

| Process Day | Conditions | Gen 3.1 |
|---|---|---|
| Day 0- pre REP initiation | Media CM1 | 500 mL |
| | IL-2 (6000 IU/mL) | + |
| | OKT-3 (30ng/mL) | + |
| | Feeders (250 E+06) | + |

| Process Day | Conditions | Gen 3.1 |
|---|---|---|
| Day 7- REP Initiation | Media CM2 | 500 mL |
| | IL-2 (6000 IU/mL) | + |
| | OKT-3 (30ng/mL) added on Day 7 | + |
| | Feeders Added on Day 7 | 500 E06 |
| | Total Feeders at Day | 750 E+06 |

| Process Day | Conditions | Gen 3.1 |
|---|---|---|
| Day 9-11 –Scale Up | From G-REX 100MCS transfer TIL suspension to 1 G-REX 500MCS ( up to 3 GREX 500MCS) | Yes |
| Day 16- Harvest | LOVO- automated cell washer | Yes |

*Figure 117*

| Process Comparison | Key Process Changes | Benefit |
|---|---|---|
| **Gen 2 : Gen 2.1** | • Initiate process with two flasks instead of one flask<br>• Divide REP initiation feeder layer between 2 G-Rex500MCS Flasks<br>• Pre-formulate media and warm prior to use | • Potential doubling of final cell count (dose) with increased TIL repertoire.<br>• Process redundancy throughout process |
| **Gen 2.1 : Gen 3.1** | • Fresh or Frozen tumor<br>• 14-16 day process (from 22 day)<br>• Reduce total feeder layer on process<br>• Feeder layer and OKT3 present at Day 0<br>• REP initiated with fragments<br>• 100MCS scales to 500MCS<br>• Scales to multiple pre-REP flasks<br>• Standard Media and Defined Media (Serum Free) | • Increased potency<br>• Improved phenotype<br>• Decreased process time<br>• Reduced reagent testing<br>• Decreased process variability<br>• Defined reagents<br>• Increased repertoire<br>• Reduce impurities (feeder)<br>• Comparable or Higher Dose. |

*Figure 118*

| Process Comparison | Key Process Changes | Desired Improvement | Criteria for Success | Outcome |
|---|---|---|---|---|
| **Gen 2 : Gen 3.0** | • 14-16 days<br>• Initiate REP with fragments up to 4 flask.<br>• 100MCS scales to 500MCS | • Increased potency<br>• Improved phenotype<br>• Decreased process time | • Increase potency as measured by INF-g ✓<br>• Comparable phenotype ✓<br>• Comparable Dose ✓<br>• Comparable purity ✓ ( feeder cell )<br>• Maintain clonal diversity ✓ | • Potency increased over Gen2<br>• Improved expression of CD28 on CD8 cells<br>• Maximum capacity of flask reached by day 16 on Gen 3.1<br>• Reduced feeder cell usage<br>• Increased diversity |

*Figure 119*

| Process | Gen 2 | Gen 3 |
|---|---|---|
| L4054 | Standard Media | Standard Media |
| L4055 | Standard Media | Standard Media |
| M1085T | Standard Media | Standard Media |

| Process | Gen 3 | Gen 3.1 control | Gen 3.1 |
|---|---|---|---|
| L4063 | Standard Media | Standard Media | Standard Media |
| L4064 | Defined Media | Defined Media | Defined Media |

**Standard Media:**
Pre REP: CM1
REP initiation : CM2
Split or Scale up : CM4

**Defined Media:**
CTS Optimizer (Serum Free Media) in each day of the process

*Figure 120*

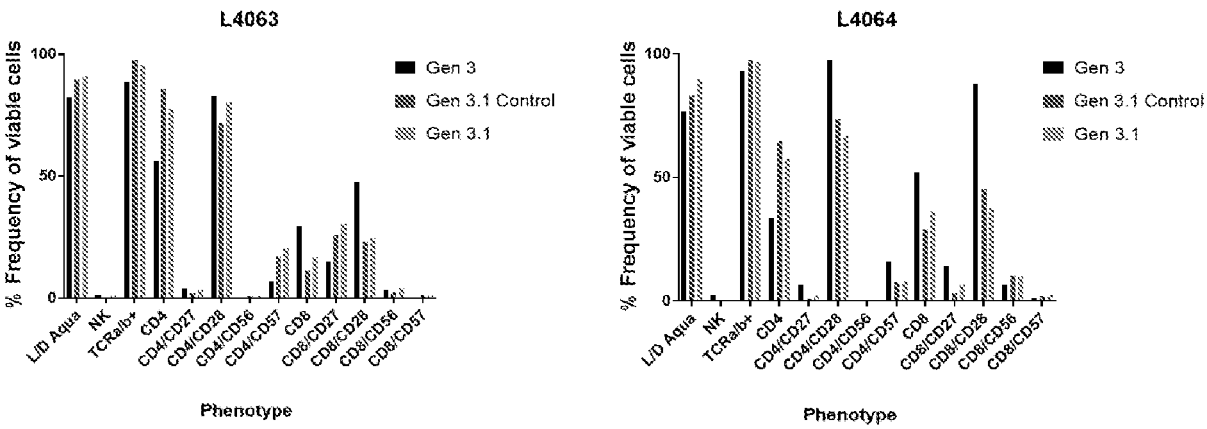


| Tumor ID | L4063 Standard media | | | L4064 Defined Media | | |
|---|---|---|---|---|---|---|
| Process | Gen 3.0 | Gen 3.1 Control | Gen 3.1 | Gen 3.0 | Gen 3.1 Control | Gen 3.1 |
| # Fragments | 53 | 53 | 53 | 21 | 21 | 21 |

*Figure 121*

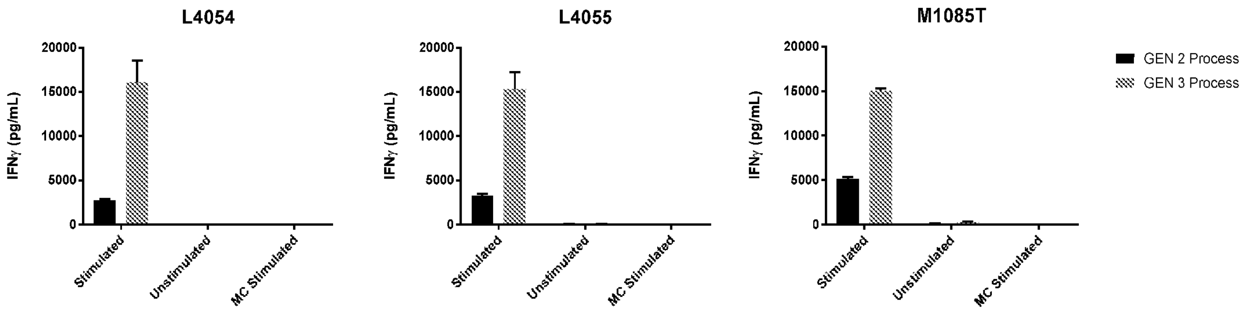


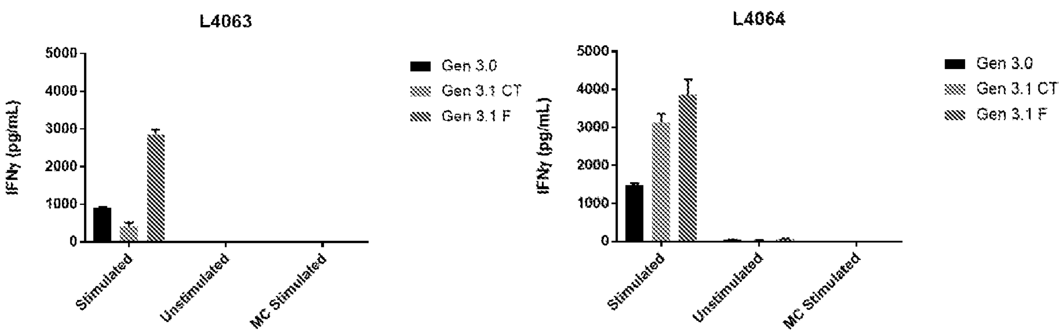


L4063- Standard Media:

L4064- Defined Media

| Process Day | Conditions | Gen 3.0 | Gen 3.1 CT (control) | Gen 3.1 F |
|---|---|---|---|---|
| Day 0- pre REP initiation | Media CM1 | 500 mL | 500 mL | 500 mL |
| | IL-2 (6000 IU/mL) | + | + | + |
| | OKT-3 (30ng/mL) | - | + | + |
| | Feeders (250 E+06) | - | - | + |

*Figure 122*

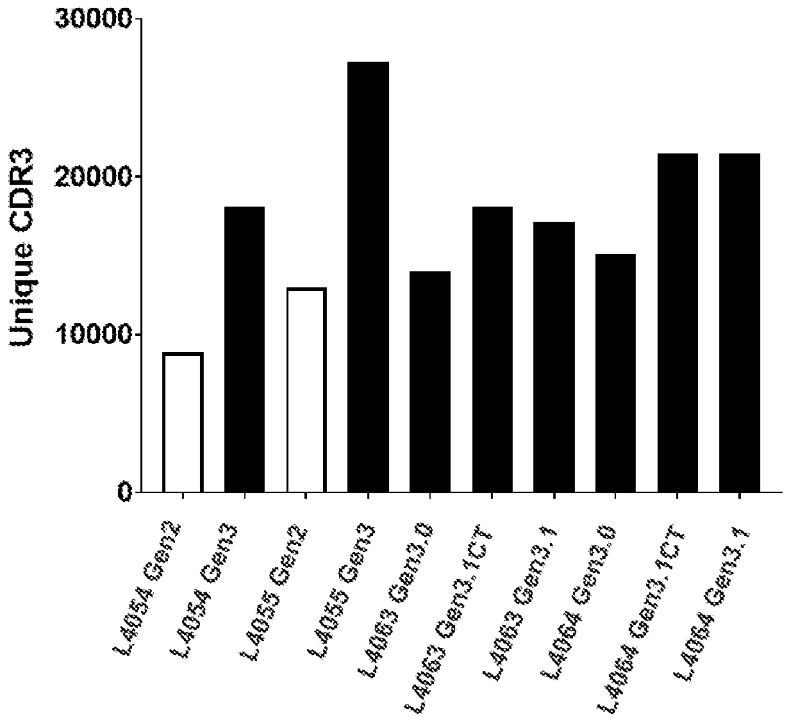
30000
20000
10000
0
Unique CDR3
L4054 Gen2
L4054 Gen3
L4055 Gen2
L4055 Gen3
L4063 Gen3.0
L4063 Gen3.1CT
L4063 Gen3.1
L4064 Gen3.0
L4064 Gen3.1CT
L4064 Gen3.1

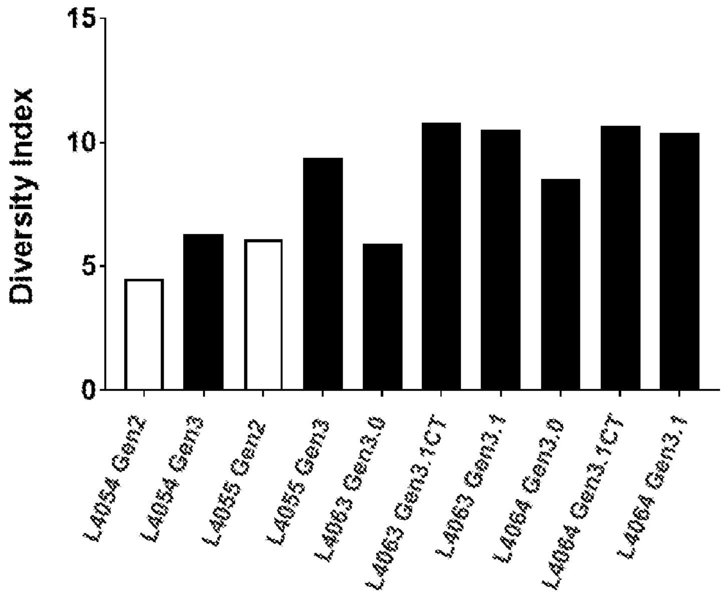
15
10
5
0
Diversity Index
L4054 Gen2
L4054 Gen3
L4055 Gen2
L4055 Gen3
L4063 Gen3.0
L4063 Gen3.1CT
L4063 Gen3.1
L4064 Gen3.0
L4064 Gen3.1CT
L4064 Gen3.1

*Figure 123*

| Conditions on Day 0 | Gen 3.0 | Gen 3.1 (control) | Gen 3.1 |
|---|---|---|---|
| Media | 500 mL | 500 mL | 500 mL |
| IL-2 (6E+06) | + | + | + |
| OKT-3 (30ng/mL) | - | + | + |
| Feeders (250 E+06) | - | - | + |

| Number of uCDR3 (% Overlap) | All uCDR3's | | | Top 80% uCDR3's | | |
|---|---|---|---|---|---|---|
| | Gen 3.0 | Gen 3.1 Control | Gen 3.1 | Gen 3.1 | Gen 3.1 control | Gen 3.1 |
| Gen 3.0 | 14005 | 3240 (23) | 2959 (21) | 1105 | 1000 (90) | 975 (88) |
| Gen 3.1 control | - | 18149 | - | - | 2438 | - |
| Gen 3.1 | - | - | 17181 | - | - | 2332 |

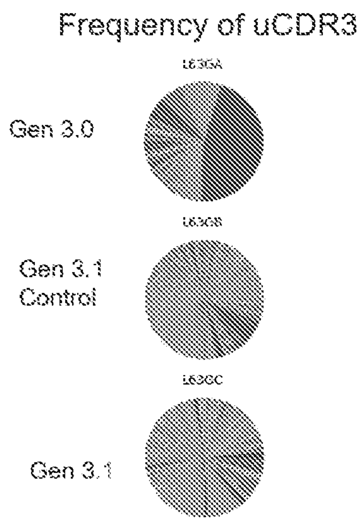

*Figure 124*

| Conditions on Day 0 | Gen 3.0 | Gen 3.1 (control) | Gen 3.1 |
|---|---|---|---|
| Media | 500 mL | 500 mL | 500 mL |
| IL-2 (6E+06) | + | + | + |
| OKT-3 (30ng/mL) | - | + | + |
| Feeders (250 E+06) | - | - | + |

| Number of uCDR3 (% Overlap) | All uCDR3's | | | Top 80% uCDR3's | | |
|---|---|---|---|---|---|---|
| | Gen 3.0 | Gen 3.1 Control | Gen 3.1 | Gen 3.0 | Gen 3.1 Control | Gen 3.1 |
| **Gen 3.0** | 15107 | 5486 **(36)** | 5541 **(37)** | 2479 | 2186 **(88)** | 2163 **(87)** |
| **Gen 3.1 Control** | - | 21460 | - | - | 3247 | - |
| **Gen 3.1** | - | - | 21503 | - | - | 3264 |

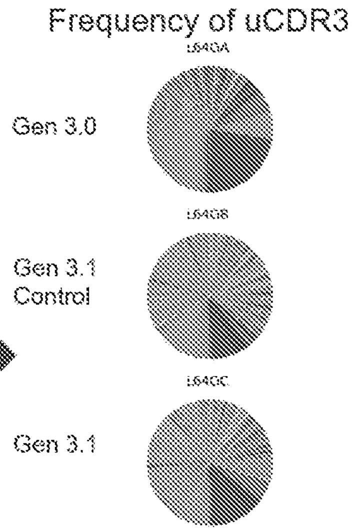

*Figure 125*

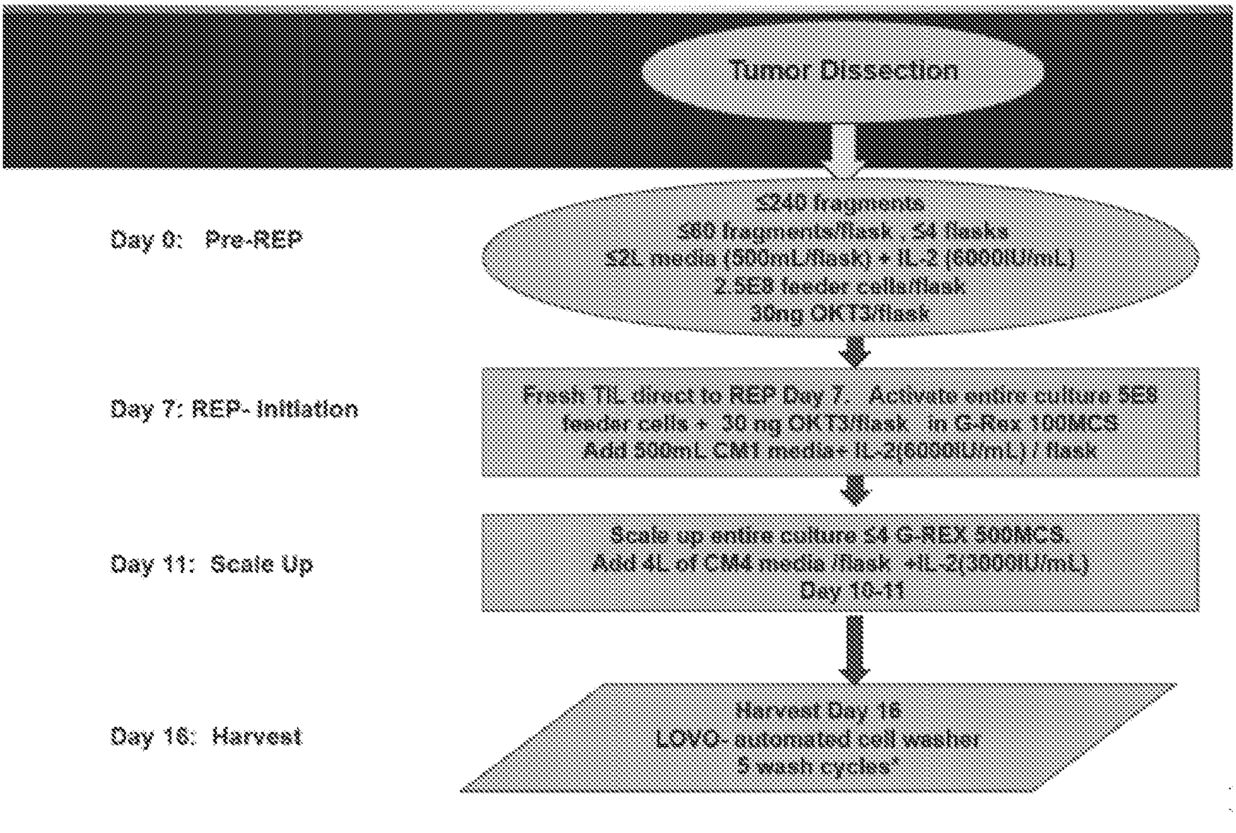
Tumor Dissection
Day 0: Pre-REP
≤240 fragments
≤60 fragments/flask , ≤4 flasks
≤2L media (500mL/flask) + IL-2 (6000IU/mL)
2.5E8 feeder cells/flask
30ng OKT3/flask
Day 7: REP- Initiation
Fresh TIL direct to REP Day 7. Activate entire culture 5E8 feeder cells + 30 ng OKT3/flask in G-Rex 100MCS
Add 500mL CM1 media+ IL-2(6000IU/mL) / flask
Day 11: Scale Up
Scale up entire culture ≤4 G-REX 500MCS
Add 4L of CM4 media /flask +IL-2(3000IU/mL)
Day 10-11
Day 16: Harvest
Harvest Day 16
LOVO- automated cell washer
5 wash cycles*

*Figure 126*

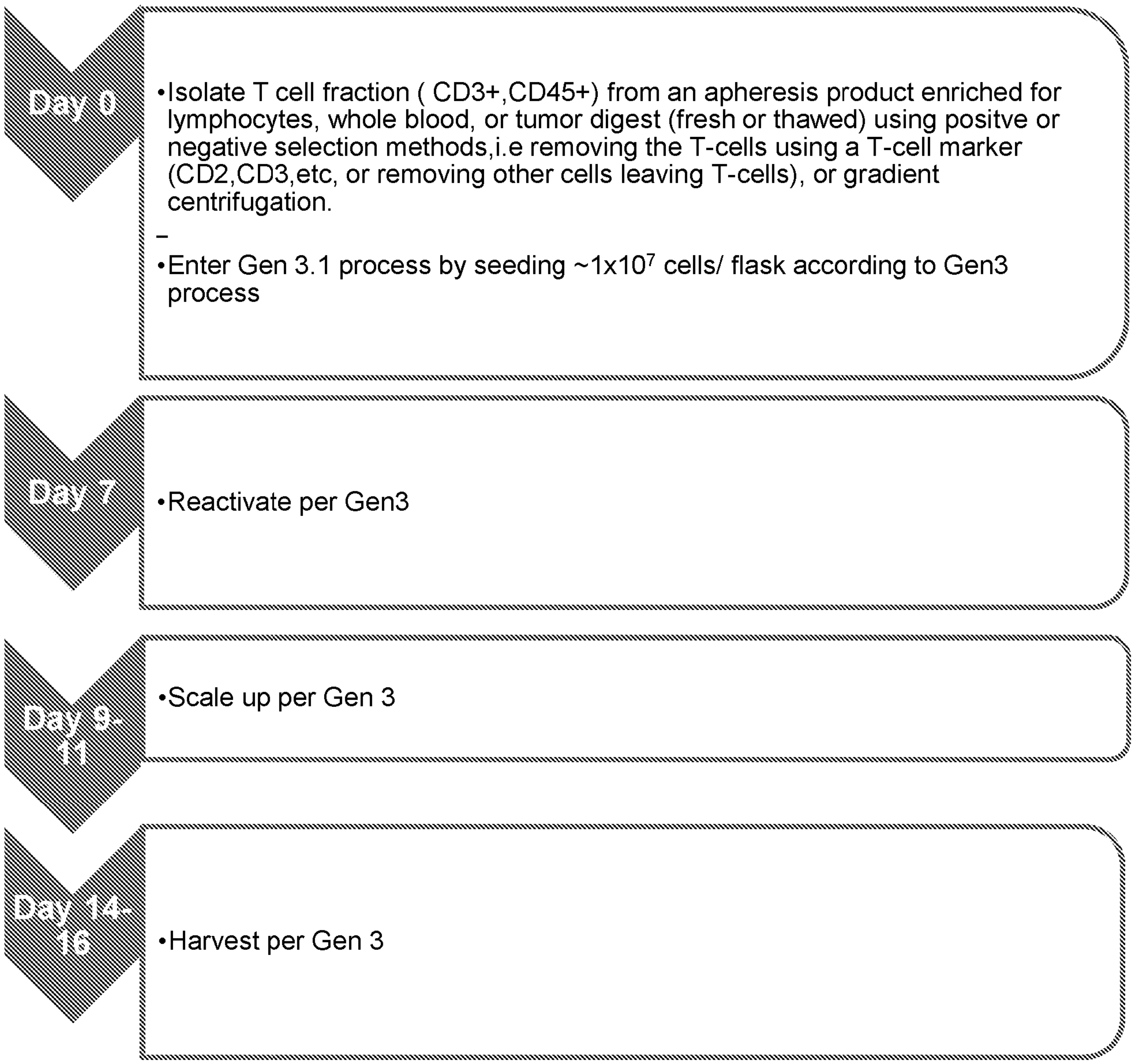
Day 0
•Isolate T cell fraction ( CD3+,CD45+) from an apheresis product enriched for lymphocytes, whole blood, or tumor digest (fresh or thawed) using positve or negative selection methods,i.e removing the T-cells using a T-cell marker (CD2,CD3,etc, or removing other cells leaving T-cells), or gradient centrifugation.
•Enter Gen 3.1 process by seeding ~1x10^7 cells/ flask according to Gen3 process
Day 7
•Reactivate per Gen3
Day 9-11
•Scale up per Gen 3
Day 14-16
•Harvest per Gen 3

*Figure 127*

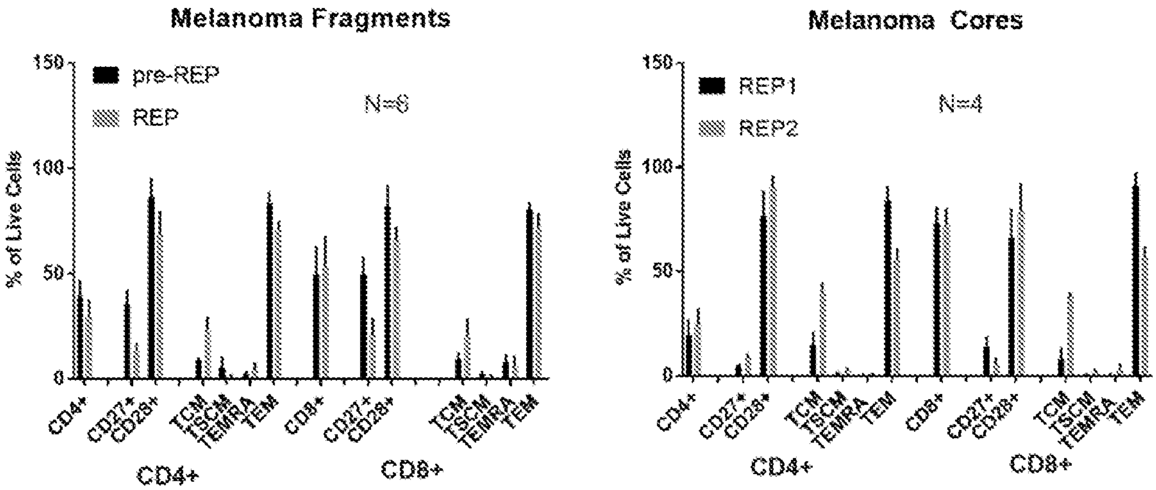
Melanoma Fragments
pre-REP
REP
N=6
% of Live Cells
150
100
50
0
CD4+
CD27+
CD28+
TCM
TSCM
TEMRA
TEM
CD8+
CD27+
CD28+
TCM
TSCM
TEMRA
TEM
CD4+
CD8+
Melanoma Cores
REP1
REP2
N=4
% of Live Cells
150
100
50
0
CD4+
CD27+
CD28+
TCM
TSCM
TEMRA
TEM
CD8+
CD27+
CD28+
TCM
TSCM
TEMRA
TEM
CD4+
CD8+

*Figure 128*

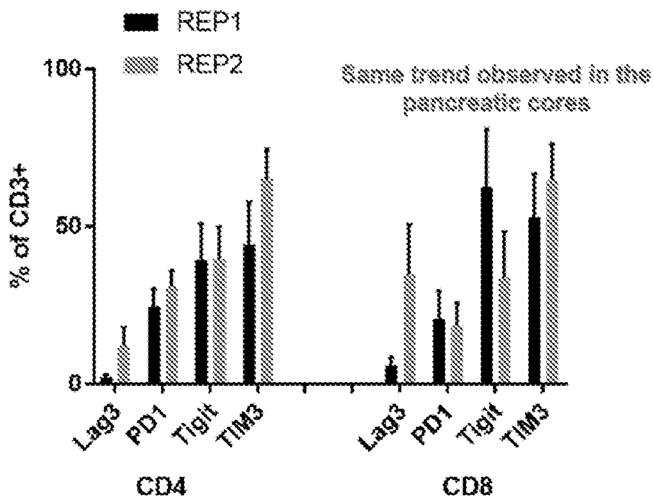
REP1
REP2
Same trend observed in the pancreatic cores
% of CD3+
100
50
0
Lag3
PD1
Tigit
TIM3
Lag3
PD1
Tigit
TIM3
CD4
CD8

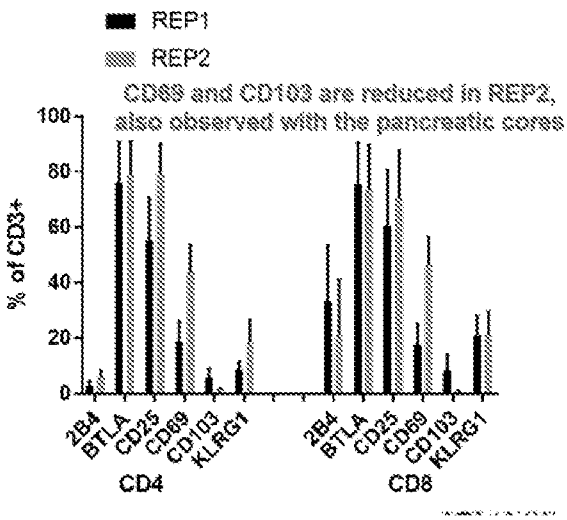
REP1
REP2
CD69 and CD103 are reduced in REP2, also observed with the pancreatic cores
% of CD3+
100
80
60
40
20
0
2B4
BTLA
CD25
CD69
CD103
KLRG1
2B4
BTLA
CD25
CD69
CD103
KLRG1
CD4
CD8

*Figure 129*

| | Culture conditions | Cores / cond. (#) | REP1 | REP2 |
|---|---|---|---|---|
| Lung (L4083) | Day 0: Addition of OKT3+feeders<br>Day 3: Addition of OKT3+feeders | 1 | >50E6 cells at Day 11 in Day 0 condition | 1157-fold (Day 0) and 925-fold (1/2 cores) expansion<br>~ 10,350 and 7,863 pg/ml IFNγ (respectively) |

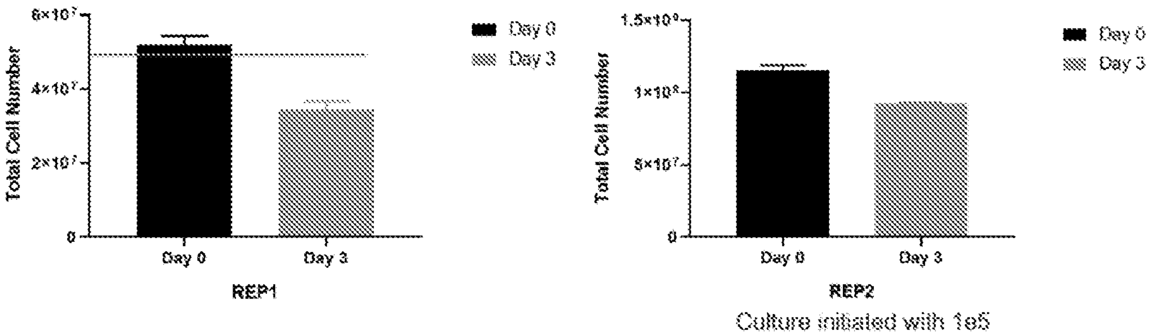

*Figure 130*

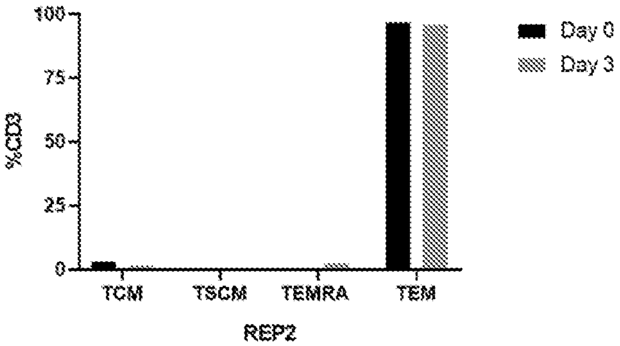
100
75
50
25
0
%CD3
TCM
TSCM
TEMRA
TEM
REP2
Day 0
Day 3

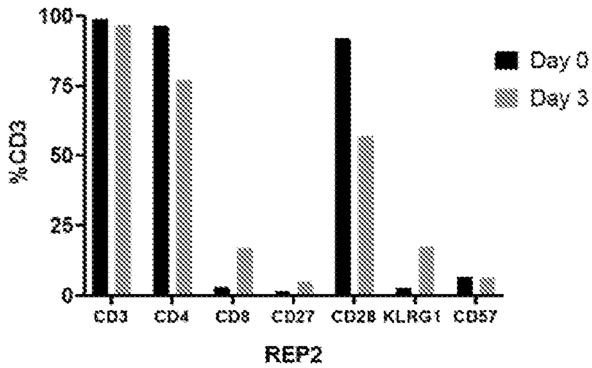
100
75
50
25
0
%CD3
CD3
CD4
CD8
CD27
CD28
KLRG1
CD57
REP2
Day 0
Day 3

*Figure 131*

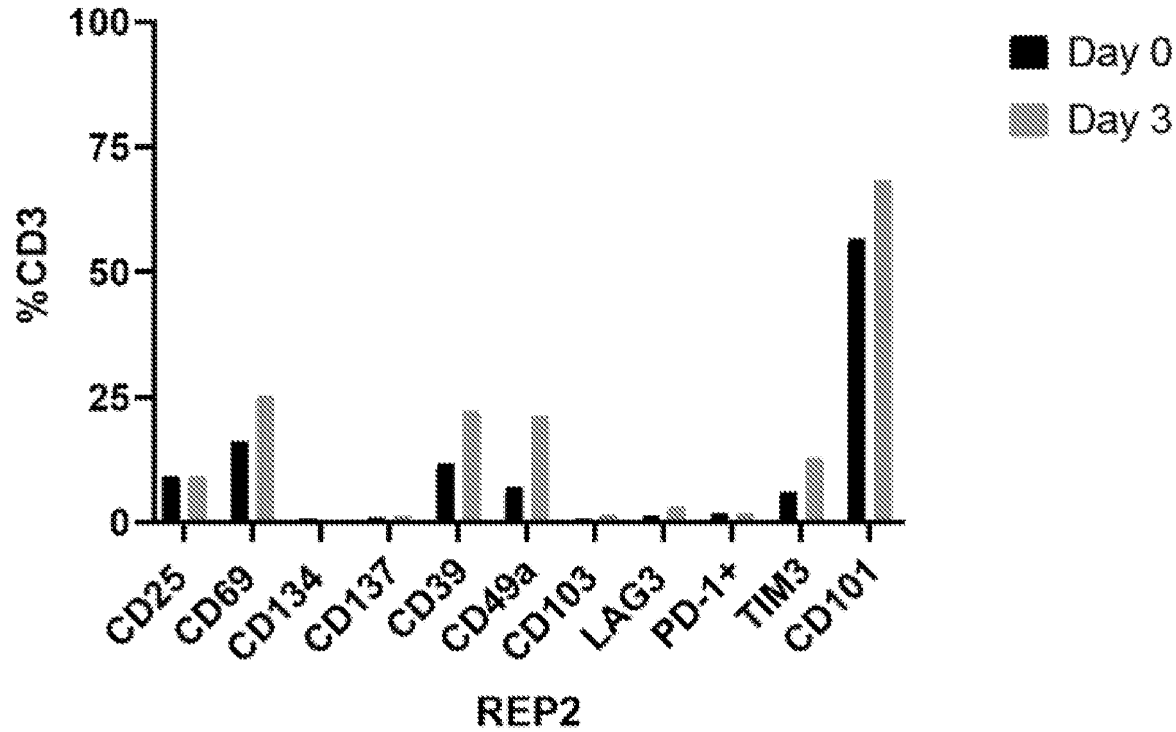
100
75
50
25
0
%CD3
Day 0
Day 3
CD25
CD69
CD134
CD137
CD39
CD49a
CD103
LAG3
PD-1+
TIM3
CD101
REP2

*Figure 132*

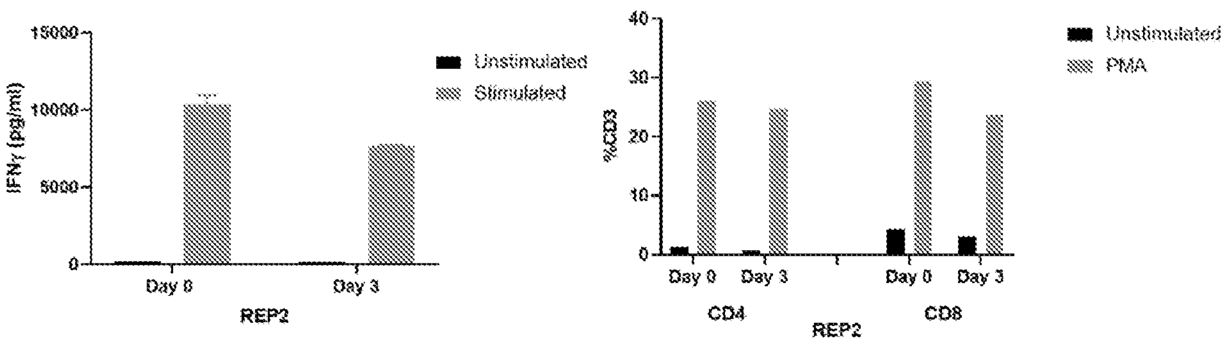
15000
10000
5000
0
IFNγ (pg/ml)
Unstimulated
Stimulated
Day 0
Day 3
REP2
40
30
20
10
0
%CD3
Unstimulated
PMA
Day 0
Day 3
Day 0
Day 3
CD4
REP2
CD8

*Figure 133*

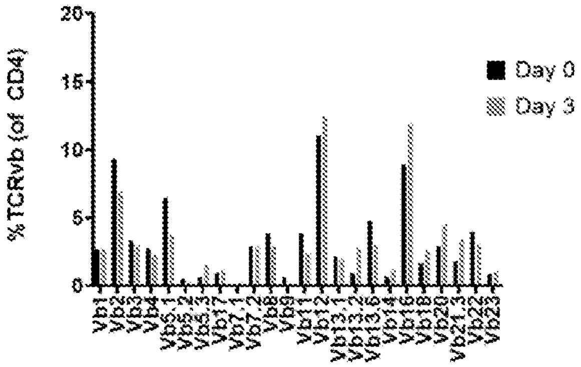
% TCRvb (of CD4)
Day 0
Day 3
Vb1
Vb2
Vb3
Vb4
Vb5.1
Vb5.2
Vb5.3
Vb17
Vb7.1
Vb7.2
Vb8
Vb9
Vb11
Vb12
Vb13.1
Vb13.2
Vb13.6
Vb14
Vb16
Vb18
Vb20
Vb21.3
Vb22
Vb23

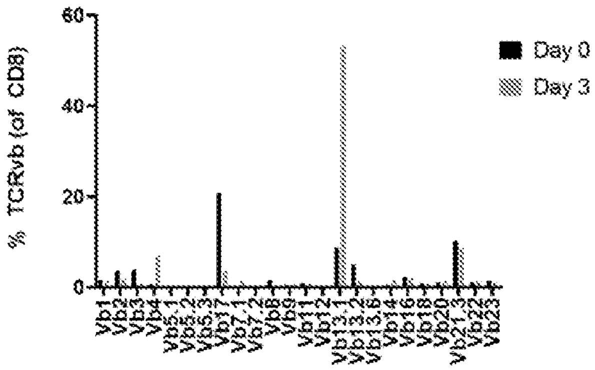
% TCRvb (of CD8)
Day 0
Day 3
Vb1
Vb2
Vb3
Vb4
Vb5.1
Vb5.2
Vb5.3
Vb17
Vb7.1
Vb7.2
Vb8
Vb9
Vb11
Vb12
Vb13.1
Vb13.2
Vb13.6
Vb14
Vb16
Vb18
Vb20
Vb21.3
Vb22
Vb23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140328791 A1
- WO 2012065086 A1
- US 4766106 A
- US 5206344 A
- US 5089261 A
- US 4902502 A
- US 6706289 B
- US 20160010058 A1
- WO 2015189356 A
- WO 2015189357 A
- US 20180280436 A
- US 8821867 B
- US 8337850 B
- US 9468678 B
- WO 2012032433 A1
- US 7288638 B
- US 8962804 B
- US 6974863 B
- US 20050095244 A
- US 6887673 B
- US 7214493 B
- US 6303121 B
- US 6569997 B
- US 6905685 B
- US 6362325 B
- US 6210669 B
- US 5928893 A
- WO 2012177788 A
- WO 2015119923 A
- WO 2010042433 A
- WO 2008025516 A1
- WO 2009007120 A1
- WO 2010003766 A1
- WO 2010010051 A1
- WO 2010078966 A1
- US 20110027218 A1
- US 20150126709 A1
- US 20110111494 A1
- US 20150110734 A1
- US 20150126710 A1
- US 9359420 B
- US 9340599 B
- US 8921519 B
- US 8450460 B
- US 7960515 B
- US 8236930 B
- US 9028824 B
- US 9006399 B
- US 9163085 B
- WO 2012027328 A
- WO 9512673 A
- WO 9521925 A
- WO 2006121810 A
- WO 2013028231 A
- WO 2013038191 A
- WO 2014148895 A
- EP 0672141 A
- US 2010136030 A
- US 2014377284 A
- US 2015190506 A
- US 2015132288 A
- US 7504101 B
- US 7550140 B
- US 7622444 B
- US 7696175 B
- US 7961515 B
- US 8133983 B
- US 6312700 B
- US 20050106717 A1
- US 20140377739 A1
- WO 2014210036 A1
- US 20130115617 A1
- WO 2013188427 A1
- US 20110136228 A1
- US 8809050 B2
- WO 2011072088 A2
- US 20160208216 A1
- US 20120244133 A1
- WO 2012129201 A1
- US 20130102075 A1
- US 8956860 B2
- WO 2013173835 A1
- US 20150175966 A1
- US 20140227237 A1
- US 5593875 A
- US 5279833 A
- US 5908635 A
- US 6056938 A
- US 6110490 A
- US 6534484 B
- US 7687070 B
- US 5766902 A
- US 6025337 A
- US 6410517 B
- US 6475994 B
- US 7189705 B
- WO 2013059343 A1
- WO 2017008063 A1

- WO 2017123663 A1
- US 6627442 B
- US 6489458 B
- US 20110039914 A1
- US 20130131141 A1
- US 20130131142 A1
- US 9080171 B
- US 20160304873 A
- WO 2010033246 A
- WO 2017070151 A
- WO 2009102427 A
- WO 2011119887 A
- WO 2010033247 A2
- WO 2009045457 A
- WO 2011119852 A
- US 5849902 A
- WO 9813526 A
- WO 9014074 A
- WO 9116024 A
- WO 9117424 A
- US 4897355 A
- US 5019034 A
- US 5128257 A
- US 5137817 A
- US 5173158 A
- US 5232856 A
- US 5273525 A
- US 5304120 A
- US 5318514 A
- US 6010613 A
- US 6078490 A
- US 2017058610 W
- US 2018012605 W
- US 2018012633 W
- US 8697359 B
- US 8993233 B
- US 8795965 B
- US 8771945 B
- US 8889356 B
- US 8865406 B
- US 8999641 B
- US 8945839 B
- US 8932814 B
- US 8871445 B
- US 8906616 B
- US 8895308 B
- US 9790490 B
- US 20110201118 A1
- US 20130117869 A1
- US 20130315884 A1
- US 20150203871 A1
- US 20160120906 A1
- US 8586526 B
- US 6534261 B
- US 6607882 B
- US 6746838 B
- US 6794136 B
- US 6824978 B
- US 6866997 B
- US 6933113 B
- US 6979539 B
- US 7013219 B
- US 7030215 B
- US 7220719 B
- US 7241573 B
- US 7241574 B
- US 7585849 B
- US 7595376 B
- US 6903185 B
- US 6479626 B
- US 8008449 B
- US 7943743 B
- US 20110052530 A

**Non-patent literature cited in the description**


- **GATTINONI et al.** *Nat. Rev. Immunol.,* 2006, vol. 6, 383-393
- **DUDLEY et al.** *Science,* 2002, vol. 298, 850-54
- **DUDLEY et al.** *J. Clin. Oncol.,* 2005, vol. 23, 2346-57
- **DUDLEY et al.** *J. Clin. Oncol.,* 2008, vol. 26, 5233-39
- **RIDDELL et al.** *Science,* 1992, vol. 257, 238-41
- **DUDLEY et al.** *J. Immunother.,* 2003, vol. 26, 332-42
- **CHANG et al.** *Nat. Immunol.,* 2016, vol. 17, 364
- **CHANG et al.** *Nat. Immunol.,* 2016, vol. 17 (364
- **NELSON.** *J. Immunol.,* 2004, vol. 172, 3983-88
- **MALEK.** *Annu. Rev. Immunol.,* 2008, vol. 26, 453-79
- **STEINKE ; BORISH.** *Respir. Res.,* 2001, vol. 2, 66-70
- **FRY ; MACKALL.** *Blood,* 2002, vol. 99, 3892-904
- **FEHNIGER ; CALIGIURI.** *Blood,* 2001, vol. 97, 14-32
- **SPOLSKI ; LEONARD.** *Nat. Rev. Drug. Disc.,* 2014, vol. 13, 379-95
- **ROSENBERG et al.** *New Eng. J. of Med.,* 1988, vol. 319, 1676
- **SWARTZ et al.** *Cancer Res.,* 2012, vol. 72, 2473

- **DONIA et al.** *Scandinavian Journal of Immunology,* 2012, vol. 75, 157-167
- **DUDLEY et al.** *Clin Cancer Res,* 2010, vol. 16, 6122-6131
- **HUANG et al.** *J Immunother,* 2005, vol. 28 (3), 258-267
- **BESSER et al.** *, Clin Cancer Res,* 2013, vol. 19 (17), OF1-OF9
- **BESSER et al.** *J Immunother,* 2009, vol. 32, 415-423
- **ROBBINS et al.** *J Immunol,* 2004, vol. 173, 7125-7130
- **SHEN et al.** *J Immunother,* 2007, vol. 30, 123-129
- **ZHOU et al.** *J Immunother,* 2005, vol. 28, 53-62
- **TRAN et al.** *J Immunother,* 2008, vol. 31, 742-751
- **TRAN et al.** *J. Immunother.,* 2008, vol. 31, 742-51
- **TRAN KQ ; ZHOU J ; DURFLINGER KH et al.** *J Immunother.,* 2008, vol. 31, 742-751
- **DUDLEY ME ; WUNDERLICH JR ; SHELTON TE et al.** *J Immunother.,* 2003, vol. 26, 332-342
- **DUDLEY et al.** *, Clin Cancer Res,* 2010, vol. 16, 6122-6131
- **TSOUKAS et al.** *J. Immunol.,* 1985, vol. 135, 1719
- **JIN, JIANJIAN.** Simplified Method of the Growth of Human Tumor Infiltrating Lymphocytes (TIL) in Gas-Permeable Flasks to Numbers Needed for Patient Treatment. *J Immunother.,* April 2012, vol. 35 (3), 283-292
- **JAEGER HM ; NAGEL SR.** Physics of the granular state. *Science,* 20 March 1992, vol. 255 (5051), 1523-31
- **O. R. MUSIN.** The problem of the twenty-five spheres. *Russ. Math. Surv.,* 2003, vol. 58 (4), 794-795
- **LEE et al.** *PLOS One,* 2013, vol. 8, e69677
- **GIEFFERS et al.** *Mol. Cancer Therapeutics,* 2013, vol. 12, 2735-47
- **FISHER et al.** *Cancer Immunolog. & Immunother,* 2012, vol. 61, 1721-33
- **SEGAL et al.** *Clin. Cancer Res.,* 2016, htp:/dx.doi.org/ 10.1158/1078-0432.CCR-16-1272
- **MARCO.** *Microbial Cell Factories,* 2011, vol. 10, 44
- **AHMAD et al.** *Clin. & Dev. Immunol.,* 2012, 980250
- **MONNIER et al.** *Antibodies,* 2013, vol. 2, 193-208
- **SADUN et al.** *J. Immunother.,* 2009, vol. 182, 1481-89
- **CURTI et al.** *Cancer Res.,* 2013, vol. 73, 7189-98
- **WEINBERG et al.** *J. Immunother.,* 2006, vol. 29, 575-585
- **JIN et al.** *J. Immunotherapy,* 2012, vol. 35, 283-292
- **TSENG.** *Biophys. J.,* 1991, vol. 60, 297-306
- **GRAHAM ; VAN DER EB.** *Virology,* 1973, vol. 52, 456-467
- **WIGLER et al.** *Proc. Natl. Acad. Sci.,* 1979, vol. 76, 1373-1376
- **CHEN ; OKAYAREA.** *Mol. Cell. Biol.,* 1987, vol. 7, 2745-2752
- **ROSE et al.** *Biotechniques,* 1991, vol. 10, 520-525
- **FELGNER et al.** *Proc. Natl. Acad. Sci. USA,* 1987, vol. 84, 7413-7417
- **GATTINONI et al.** *Nat Med,* 2009, 2011
- **GATTINONI.** *Nature Rev. Cancer,* 2012
- **CIERI et al.** *Blood,* 2013
- **SHAREI et al.** *PNAS,* 2013
- **SHAREI et al.** *PLOS ONE,* 2015
- **GREISBECK et al.** *J. Immunology,* 2015, vol. 195
- **LEVINE et al.** *Proc. Nat'l Acad. Sci.,* 2006, vol. 103, 17372-77
- **ZUFFEREY et al.** *Nat. Biotechnol.,* 1997, vol. 15, 871-75
- **DULL et al.** *J. Virology,* 1998, vol. 72, 8463-71
- **CEPKO ; PEAR.** *Cur. Prot. Mol. Biol.,* 1996, 9.9.1-9.9.16
- **HACKETT et al.** *Mol. Therapy,* 2010, vol. 18, 674-83
- **KHVOROVA ; WATTS.** *Nat. Biotechnol.,* 2017, vol. 35, 238-248
- **BYRNE et al.** *J. Ocul. Pharmacol. Ther.,* 2013, vol. 29, 855-864
- **LIGTENBERG et al.** *Mol. Therapy,* 2018
- **AUGUSTYNS, K. et al.** *Nucl. Acids. Res.,* 1992, vol. 18, 4711
- **BYRNE et al.** *J. Ocular Pharmacology and Therapeutics,* December 2013, vol. 29 (10), 855-864
- **BERGAN.** *Nucleic Acids Research.,* 1993, vol. 21, 3567
- **COX et al.** *Nature Medicine,* 2015, vol. 21 (2
- **BEANE et al.** *Mol. Therapy,* 2015, vol. 23, 1380-1390
- **SADEGHI et al.** *Acta Oncologica,* 2013, vol. 52, 978-986
- **JIN et al.** *J. Immunotherapy,* 2012, vol. 35 (3), 283-292
- **GOFF et al.** *J. Clinical Oncology,* 2016, vol. 34 (20), 2389-239
- **DUDLEY et al.** *J Immunother.,* 2003, vol. 26, 332-342
- **GOFF et al.** *J Immunother.,* 2010, vol. 33, 840-847
- **CAMPBELL et al.** *Cell,* 2017, vol. 171, 1042-1056

- **PFEIFER, G.P. ; YOU, Y.H. ; BESARATINIA, A.** *Mutat. Res.,* 2005, vol. 571, 19-31
- **SAGE, E.** *Photochem. Photobiol.,* 1993, vol. 57, 163-174
- **PLEASANCE, E.D. ; STEPHENS, P.J. ; O'MEARA, S. ; MCBRIDE, D.J. ; MEYNERT, A. ; JONES, D. ; LIN, M.L. ; BEARE, D. ; LAU, K.W. ; GREENMAN, C. et al.** *Nature,* 2010, vol. 463, 184-190
- **ROBERTS, S.A. ; LAWRENCE, M.S. ; KLIMCZAK, L.J. ; GRIMM, S.A. ; FARGO, D. ; STOJANOV, P. ; KIEZUN, A. ; KRYUKOV, G.V. ; CARTER, S.L. ; SAKSENA, G. et al.** *Nat. Genet.,* 2013, vol. 45, 970-976
- **KANDOTH, C. ; SCHULTZ, N. ; CHERNIACK, A.D ; AKBANI, R. ; LIU, Y. ; SHEN, H. ; ROBERTSON, A.G. ; PASHTAN, I. ; SHEN, R. ; BENZ, C.C. et al.** *Nature,* 2013, vol. 497, 67-73
- **MUZNY, D.M. ; BAINBRIDGE, M.N. ; CHANG, K. ; DINH, H.H. ; DRUMMOND, J.A. ; FOWLER, G. ; KOVAR, C.L. ; LEWIS, L.R. ; MORGAN, M.B. ; NEWSHAM, I.F. et al.** *Nature,* 2012, vol. 487, 330-337
- **MULLANY et al.** *Endocrinology,* 2012, vol. 153, 1585-92
- **FONG et al.** *J. Ovarian Res.,* 2009, vol. 2, 12
- **HERREROS-VILLANUEVA et al.** *World J. Gastroenterol.,* 2012, vol. 18, 1286-1294
- **FANTOZZI.** *Breast Cancer Res.,* 2006, vol. 8, 212
- **DAMSKY et al.** *Pigment Cell & Melanoma Res.,* 2010, vol. 23, 853-859
- **MEUWISSEN et al.** *Genes & Development,* 2005, vol. 19, 643-664
- **KIM.** *Clin. Exp. Otorhinolaryngol.,* 2009, vol. 2, 55-60
- **SANO.** *Head Neck Oncol.,* 2009, vol. 1, 32
- **GASSNER et al.** *Cancer Immunol. Immunother.,* 2011, vol. 60, 75-85
- **MURANSKI et al.** *Nat. Clin. Pract. Oncol.,* 2006, vol. 3, 668-681
- **DUDLEY et al.** *J. Clin. Oncol.,* 2008, vol. 26, 5233-5239
- **DUDLEY et al.** *J. Clin. Oncol.,* 2005, vol. 23, 2346-2357
- **O'DAY et al.** *J. Clin. Oncol.,* 1999, vol. 17, 2752-61
- **ETON et al.** *Cancer,* 2000, vol. 88, 1703-9
- **SANTEGOETS, S. J.,.** *J Transl Med.,* 2013, vol. 11, 37, https://www.ncbi.nlm.nih.gov/pmc/articles/PMC3626797
- **SANTEGOETS, S. J.** *J Transl Med.,* 2013, vol. 11, 37, https://www.ncbi.nlm.nih.gov/pmc/articles/PMC3626797
- **ULLENHAG, G.J. et al.** *Cancer Immunol Immunother,* 2014, vol. 61 (5), 725-732